# EUROPEAN PATENT APPLICATION

(11) **EP 2 067 766 A1**
(43) Date of publication of application: **10.06.2009**
(21) Application number: 08253755.6
(22) Date of filing: 19.11.2008
(51) Int. Cl.: C07C 211/61, C09K 11/06, H05B 33/14, H01L 51/00

(54) **Green electroluminescent compounds and organic electroluminescent device using the same**

(30) Priority: 19.11.2007 KR 20070118130
(71) Applicant: Gracel Display Inc., Seoul 133-833 (KR)
(72) Inventor: Kim, Bo, Ra, Uijeongbu-si, Gyeonggi-do, 480-020 (KR); Lee, Mi, Ae, Seoul, 121-190 (KR); Kim, Chi, Sik, Seoul 156-825 (KR); Lee, Soo, Yong, Namyangju-si, Gyeonggi-do 472-120 (KR); Cho, Young, Jun, Seoul 136-060 (KR); Kwon, Hyuck, Joo, Seoul 130-100 (KR); Kim, Bong, Ok, Seoul 135-090 (KR); Kim, Sung, Min, Seoul-city 157-886 (KR); Yoon, Seung, Soo, Seoul 135-884 (KR)
(74) Representative: Kent, Venetia Katherine

(57) **Abstract**

The present invention relates to novel organic electroluminescent compounds, organic electroluminescent devices and organic solar cells comprising the same. Specifically, the organic electroluminescent compounds according to the invention are represented by Chemical Formula (1) : 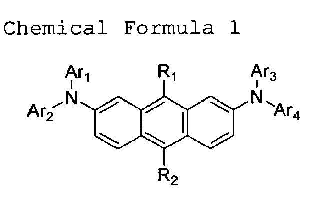 wherein, R₁ and R₂ independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R₁ and R₂ may be further substituted by one or more substituent(s) selected from deuterium, linear or branched (C1-C20)alkyl, (Cl-C20)alkenyl, (C1-C20)alkynyl, halogen, phenyl, fluorenyl, naphthyl and anthryl.

The electroluminescent compounds according to the present invention are green electroluminescent compounds, of which the luminous efficiency and device lifetime have been maximized.

## Description

### FIELD OF THE INVENTION

The present invention relates to novel organic electroluminescent compounds, and organic electroluminescent devices employing the same in the electroluminescent layer and organic solar cells employing the same. Specifically, the organic electroluminescent compounds according to the present invention are characterized in that they are compounds represented by Chemical Formula (1): 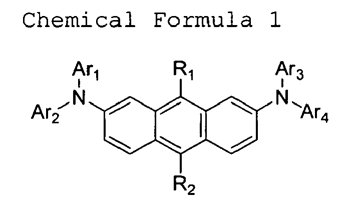 wherein, R₁ and R₂ independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R₁ and R₂ may be further substituted by one or more substituent(s) selected from deuterium, linear or branched (C1-C20)alkyl, (C1-C20)alkenyl, (C1-C20)alkynyl, halogen, phenyl, fluorenyl, naphthyl and anthryl; and

Ar₁ through Ar₄ independently represent (C6-C20)aryl, (C4-C20)heteroaryl, morpholino or thiomorpholino, and the aryl or heteroaryl of Ar₁ through Ar₄ may be further substituted by one or more substituent(s) selected from a group consisting of (C6-C20)aryl or (C4-C20)heteroaryl containing one or more substituent(s) selected from a group consisting of deuterium, linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl; linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl.

### BACKGROUND OF THE INVENTION

The most important matter in developing an OLED having high efficiency and long life is development of electroluminescent material of high performance. In view of current development of electroluminescent material, green electroluminescent materials show superior electroluminescent property to red or blue electroluminescent materials. However, conventional green electroluminescent materials still have many problems to achieve manufacturing panels of large scale with low power consumption. In view of practical efficiency and life, various kinds of materials for green have been reported up to now. Though they show from 2 to 5 times of electroluminescent property as compared to red or blue electroluminescent materials, development of green electroluminescent material is getting challenged by the improvement of properties of red or blue electroluminescent material. In the meanwhile, enhancement of device life of the green material is still insufficient, so that a green electroluminescent material providing long life is seriously required.

As green fluorescent material, a coumarin derivative (Compound D), a quinacrydone derivative (Compound E), DPT (Compound F) and the like have been known. Compound D is the structure of C545T that is the most widely used coumarin derivative up to the present. In general, those materials are doped by using Alq as the host, at a concentration of several % to about several ten %, to form an electroluminescent device.

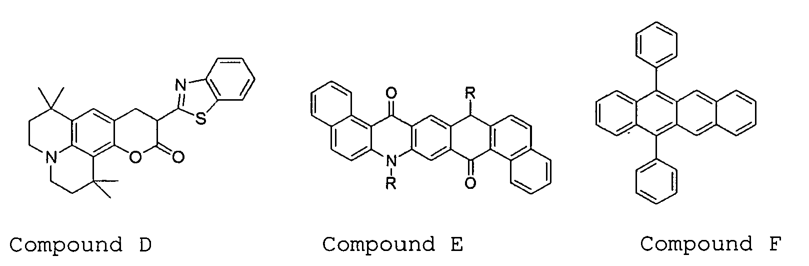

Korean Patent Registration No. 0736619 discloses only the symmetric compounds wherein substituent(s) such as methyl and t-butyl are substituted at 9- and 10-position of anthracene, and diarylamino groups are directly substituted at the 2- and 6-position, but not the asymmetric compound wherein diarylamino groups are directly substituted at 2- and 7-position. Though it was observed in Korean Patent Registration No. 0736619 that the symmetric compounds wherein diarylamino groups are directly substituted at 2- and 6-position of anthracene ring, respectively, with containing methyl or t-butyl substituents at 9- and 10-position (G-36, G-42, G-64) exhibited lowered luminous efficiency, the inventors did not recognized at all, in order to solve the problem, that the asymmetric compounds wherein alkyl groups such as methyl and t-butyl are substituted at 9- and 10-position of anthracene, and diarylamino groups are directly substituted at 2- and 7-position.

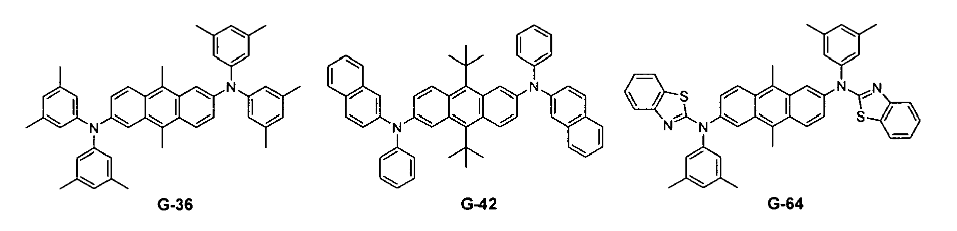

In the meanwhile, U.S. Patent Publication No. 2005/0260442 describes the compounds wherein two diarylamino groups are directly substituted on anthracene ring while containing aryl, heteroaryl or alkyl groups at 9- and 10-position. But the Patent Publication describes only the compounds substituted by aryl (such as phenyl and naphthyl) groups, not alkyl groups, at 9- and 10-position, while it does not mention at all an asymmetric compound wherein diarylamino groups are directly substituted at 2- and 7-position of anthracene. This indicates that U.S. Patent Publication No. 2005/0260442 did not specifically practiced the asymmetric compounds wherein diarylamino groups are directly substituted at 2- and 7-position of anthracene, respectively, and alkyl groups substituted at 9- and 10-position.

### SUMMARY OF THE INVENTION

The present inventors confirmed that the compounds having asymmetric structure wherein diarylamino groups are directly substituted at 2- and 7-position of anthracene, respectively, and substituents selected from alkyl, alkenyl, alkynyl, cycloalkyl, alkylsilyl, arylsilyl, adamantyl, bicycloalkyl and heteroaryl, such as methyl, t-butyl, phenylethenyl, phenylethynyl, cyclohexyl, trimethylsilyl, triphenylsilyl, adamantyl, 4-pentylbicyclo[2.2.2]octyl and benzothiazolyl are substituted at 9- and 10-position, could noticeably improve the electroluminescent properties, and completed the invention.

The present inventors surprisingly found that the problems of conventional hole transport materials such as lowered luminous efficiency, shortened operation life and raised ionization potential can be overcome when substituents selected from alkyl, alkenyl, alkynyl, cycloalkyl, alkylsilyl, arylsilyl, adamantyl, bicycloalkyl and heteroaryl, such as methyl, t-butyl, phenylethenyl, phenylethynyl, cyclohexyl, trimethylsilyl, triphenylsilyl, adamantyl, 4-pentylbicyclo[2.2.2]octyl and benzothiazolyl are substituted at 9- and 10-position, even though diarylamino groups are directly substituted at 2- and 7-position of said anthracene ring. Thus, they completed the present invention by incorporating the structure to electroluminescent materials, which was not recognized by any prior inventions including Korean Patent Registration No. 0736619 and U.S. Patent Publication No. 2005/0260442.

Further, the inventors found that the device life could be increased as well as noticeable increase in color reproducibility due to improvement of color purity and enhanced luminous efficiency, when one or more organic electroluminescent compound(s) according to the invention is(are) employed together with one or more compound(s) selected from certain anthracene derivatives and benz[a]anthracene derivatives as electroluminescent host(s) in the electroluminescent region.

The object of the present invention is to provide novel organic electroluminescent compounds wherein substituents such as alkyl, alkenyl, alkynyl, cycloalkyl, alkylsilyl, arylsilyl, adamantyl, bicycloalkyl and heteroaryl are substituted at 9-and 10-position of anthracene, and diarylamino groups are directly substituted at 2- and 7-position of the anthracene ring. Another object of the present invention is to provide organic electroluminescent devices which comprise an electroluminescent region employing one or more organic electroluminescent compound(s) as described above and one or more compound(s) selected from certain anthracene derivatives and benz[a]anthracene derivatives as an electroluminescent host. Still another object of the present invention is to provide organic electroluminescent compounds exhibiting excellent color purity, high luminous efficiency and much improved device lifetime, and to provide organic electroluminescent devices and organic solar cells comprising the novel organic electroluminescent compound as described above.

The present invention relates to novel organic electroluminescent compounds and organic electroluminescent devices and organic solar cells comprising the same. Specifically, the organic electroluminescent compounds according to the invention are characterized in that they are represented by Chemical Formula (1): 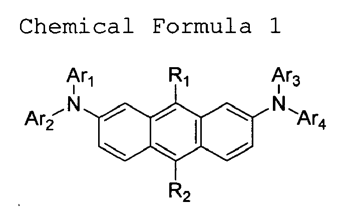 wherein, R₁ and R₂ independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R₁ and R₂ may be further substituted by one or more substituent(s) selected from deuterium, linear or branched (C1-C20)alkyl, (C1-C20)alkenyl, (C1-C20)alkynyl, halogen, phenyl, fluorenyl, naphthyl and anthryl; and

Ar₁ through Ar₄ independently represent (C6-C20)aryl, (C4-C20)heteroaryl, morpholino or thiomorpholino, and the aryl or heteroaryl of Ar₁ through Ar₄ may be further substituted by one or more substituent(s) selected from a group consisting of (C6-C20)aryl or (C4-C20)heteroaryl containing one or more substituent(s) selected from a group consisting of deuterium, linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl; linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl.

### BRIEF DESCRIPTION OF THE DRAWINGS

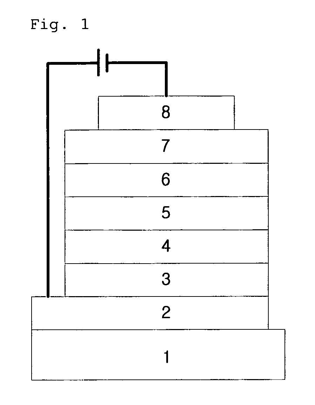

Fig. 1 is a cross-sectional view of an OLED.

### DETAILED DESCRIPTION OF THE INVENTION

Referring now to the Drawings, Fig. 1 illustrates a cross-sectional view of an OLED comprising a Glass 1, a Transparent electrode 2, a Hole injection layer 4, an Electroluminescent layer 5, an Electron transport layer 6, an Electron injection layer 7 and an Al cathode 8.

The term "alkyl", "alkoxy" described herein and any substituents comprising "alkyl" moiety include both linear and branched species.

The term "aryl" described herein means an organic radical derived from aromatic hydrocarbon via elimination of one hydrogen atom. Each ring comprises a monocyclic or fused ring system containing from 4 to 7, preferably from 5 to 6 cyclic atoms. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S as the aromatic cyclic backbone atom(s), and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl group may comprise a bivalent aryl group, of which the heteroatoms may be oxidized or quaternized to form N-oxide and quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The term "saturated 5- or 6-membered heterocyclic amino" described herein refers a compound containing nitrogen as a cyclic atom of a 5- or 6-membered ring comprised of saturated bonding, which may further comprise one or more heteroatom(s) selected from N, O and S.

The compounds of Chemical Formula (1) are characterized by their structure of novel concept which maximizes luminous efficiency of green electroluminescent devices resulted from those compounds and their device life, being unexpected by conventional inventions. The organic electroluminescent compounds of Chemical Formula (1) according to the invention adopted a structure showing an efficient energy transfer mechanism between the host and the dopant, which can realize electroluminescent property with a reliably high efficiency on the basis of improvement in electron density distribution. The structure of the novel compounds according to the present invention can provide a skeletal which can also tune an electroluminescent property with high efficiency in the range from blue to red, not only for green electroluminescence. Beyond the concept of using a host material with high electron conductivity such as Alq, the invention applies a host having appropriate balance of hole conductivity and electron conductivity, thereby overcoming the problems of conventional materials including low initial efficiency and short lifetime, and ensures electroluminescent properties with high performance having high efficiency and long life for each color.

The organic electroluminescent compounds according to the present invention include those represented by Chemical Formula (2): 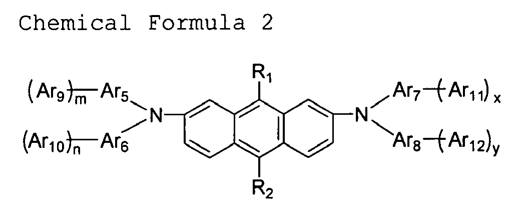 wherein, R₁ and R₂ are defined as in Chemical Formula (1); Ar₅ through Ar₈ independently represent (C6-C20)aryl, (C4-C20)heteroaryl, morpholino, thiomorpholino, (C6-C20)arylene or (C4-C20)heteroarylene, and the aryl or heteroaryl of Ar₅ through Ar₈ may be further substituted by one or more substituent(s) selected from deuterium, linear or branched (C1-C20)alkyl with or without halogen substituent(s), (C1-C20)alkoxy, (C3-C15)cycloalkyl, halogen, cyano, (C6-C20)aryl, tri(C1-C20)alkylsilyl, di(C1-C20)alkyl(C6-C20)arylsilyl and tri(C6-C20)arylsilyl;

Ar₉ through Ar₁₂ independently represent (C6-C20)aryl or (C4-C20)heteroaryl, and the aryl or heteroaryl of Ar₉ through Ar₁₂ may be further substituted by one or more substituent(s) selected from deuterium, linear or branched (C1-C20)alkyl, (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl;
provided that m is 0 when Ar₅ represents (C6-C20)aryl, (C4-C20)heteroaryl, morpholino or thiomorpholino, while m is an integer from 1 to 4 when Ar₅ represents (C6-C20)arylene or (C4-C20)heteroarylene;
x is 0 when Ar₁₁ represents (C6-C20)aryl, (C4-C20)heteroaryl, morpholino or thiomorpholino, while x is an integer from 1 to 4 when Ar₁₁ represents (C6-C20)arylene or (C4-C20)heteroarylene;
n is 0 when Ar₆ represents (C6-C20)aryl, (C4-C20)heteroaryl, morpholino or thiomorpholino, while n is an integer from 1 to 4 when Ar₆ represents (C6-C20)arylene or (C4-C20)heteroarylene; and
y is 0 when Ar₁₂ represents (C6-C20)aryl, (C4-C20)heteroaryl, morpholino or thiomorpholino, while y is an integer from 1 to 4 when Ar₁₂ represents (C6-C20)arylene or (C4-C20)heteroarylene.

In Chemical Formulas. (1) and (2), R₁ and R₂ are independently selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, ethenyl, phenylethenyl, ethynyl, phenylethynyl, cyclopentyl, cyclohexyl, cycloheptyl, cyclooctyl, trimethylsilyl, triethylsilyl, tripropylsilyl, tri(t-butyl)silyl, t-butyldimethylsilyl, dimethylphenylsilyl, triphenylsilyl, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, pyridyl, pyrrolyl, furanyl, thiophenyl, imidazolyl, benzimidazolyl, tetrazolyl, pyrazinyl, pyrimidyl, quinolyl, benzofuranyl, benzothiophenyl, pyrazolyl, indolyl, carbazolyl, thiazolyl, oxazolyl, benzothiazolyl and benzoxazolyl.

In Chemical Formula (2), Ar₅ through Ar₈ are independently selected from phenyl, biphenyl, naphthyl, anthryl, fluorenyl, phenanthryl, pyrenyl, perylenyl, fluoranthenyl, pyridyl, quinolyl, furanyl, thiophenyl, thiazolyl, imidazolyl, oxazolyl, benzofuranyl, benzothiazolyl, benzimidazolyl, benzoxazolyl, morpholino, thiomorpholino, 1,2-phenylene, 1,3-phenylene, 1,4-phenylene, 1,3,4-phenylene, 1,3,5-phenylene, and the following structures: 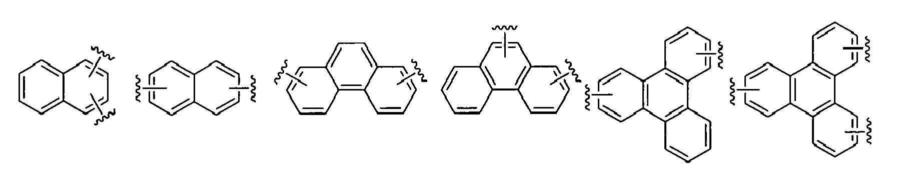 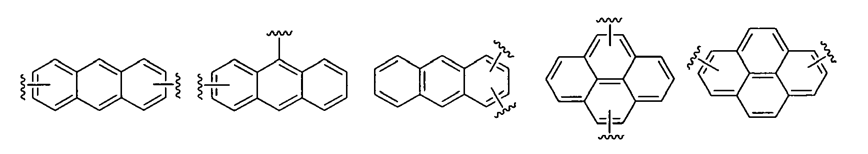 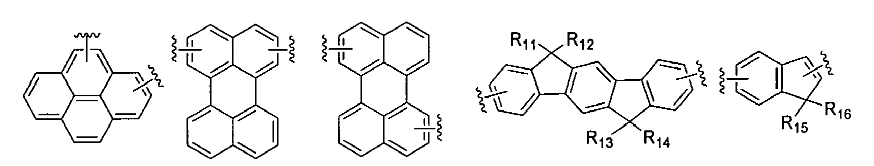 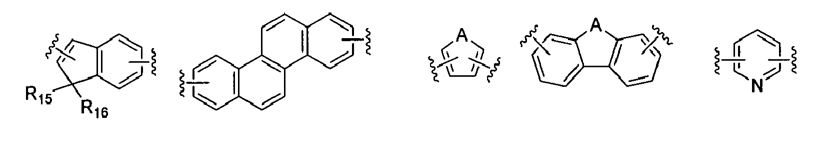 the phenyl, biphenyl, naphthyl, fluorenyl or benzimidazolyl of Ar₅ through Ar₈ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl;
R₁₁ through R₁₆ independently represent hydrogen, (C1-C20)alkyl or (C6-C20)aryl;
A represents CR₂₁C₂₂, NR₂₃, O or S; and
R₂₁ through R₂₃ independently represent hydrogen, (C1-C20)alkyl or (C6-C20)aryl.

In Chemical Formula (2), Ar₉ through Ar₁₂ independently represent phenyl, biphenyl, naphthyl, anthryl, fluorenyl, phenanthryl, pyrenyl, perylenyl, fluoranthenyl, pyridyl or quinolyl; and
the phenyl, naphthyl, anthryl, fluorenyl, phenanthryl, pyrenyl, perylenyl, fluoranthenyl, pyridyl or quinolyl of Ar₉ through Ar₁₂ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl and triphenylsilyl.

The organic electroluminescent compounds according to the present invention can be specifically exemplified by the following compounds, but they are not restricted thereto: 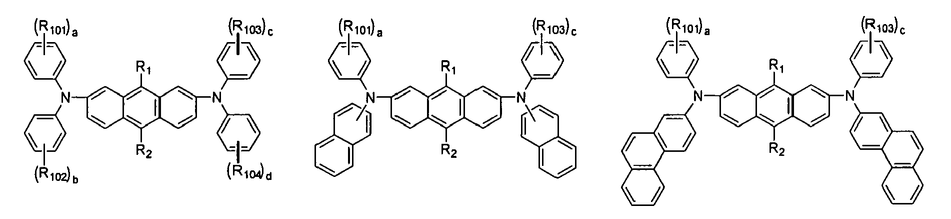 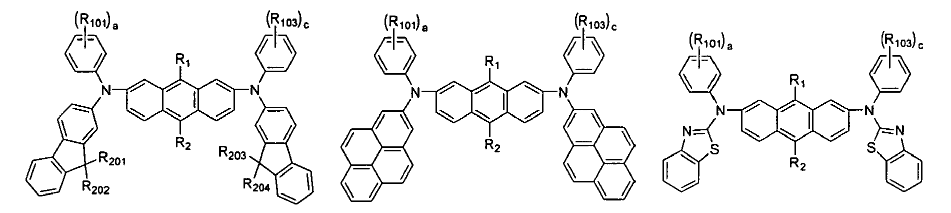 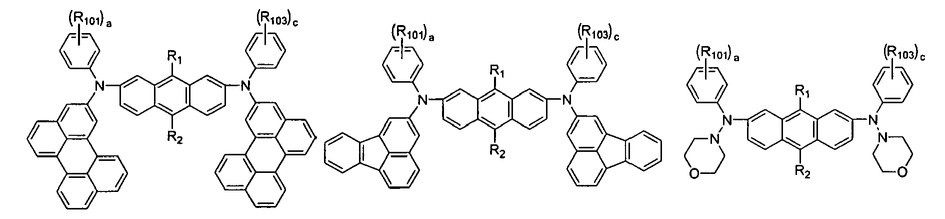 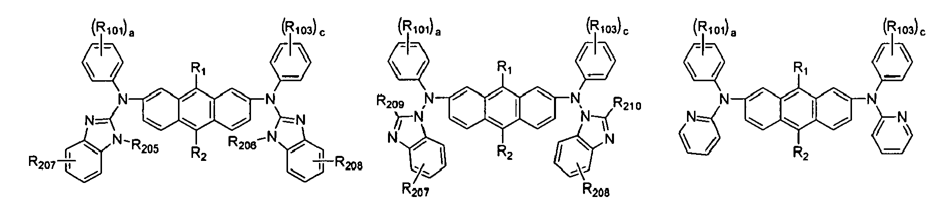 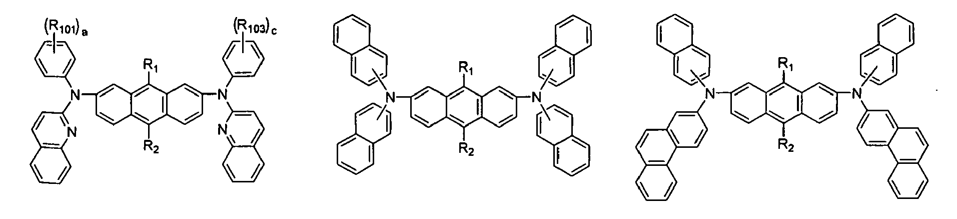 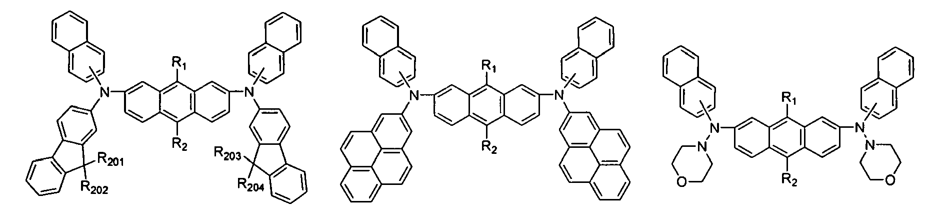 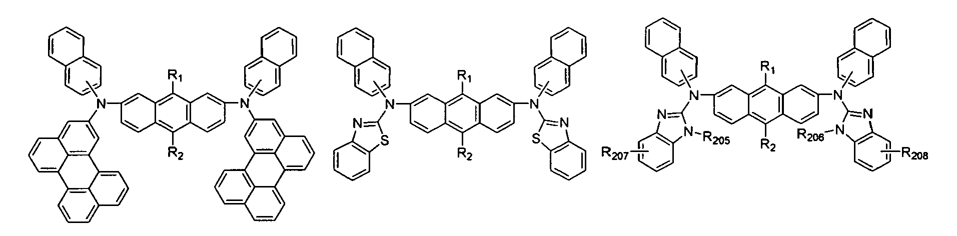 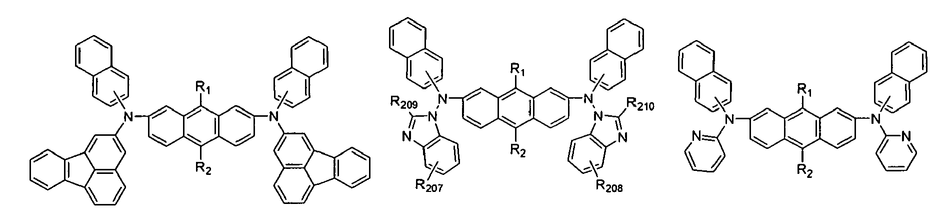 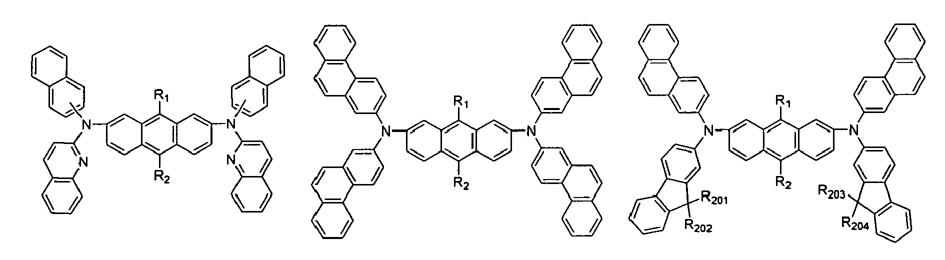 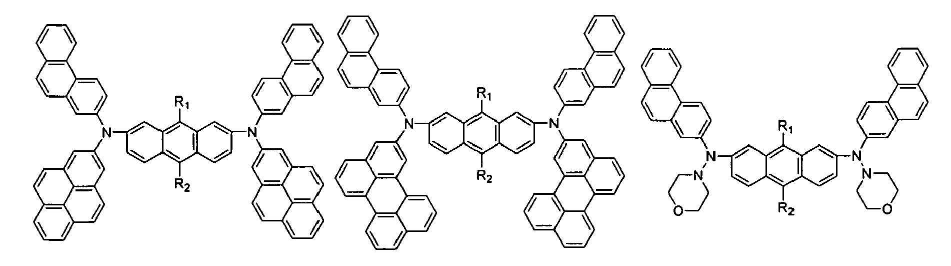 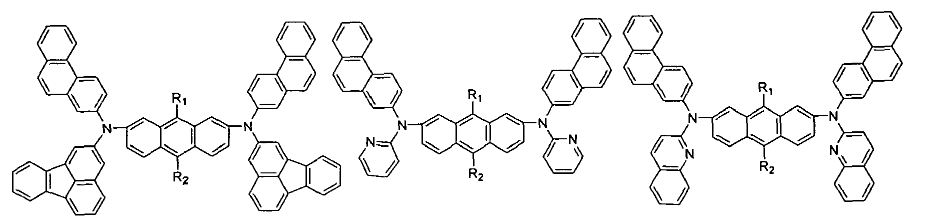 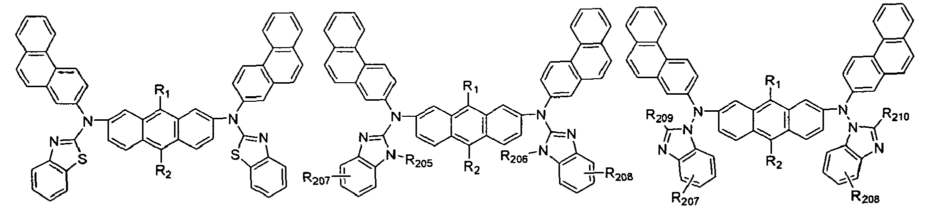 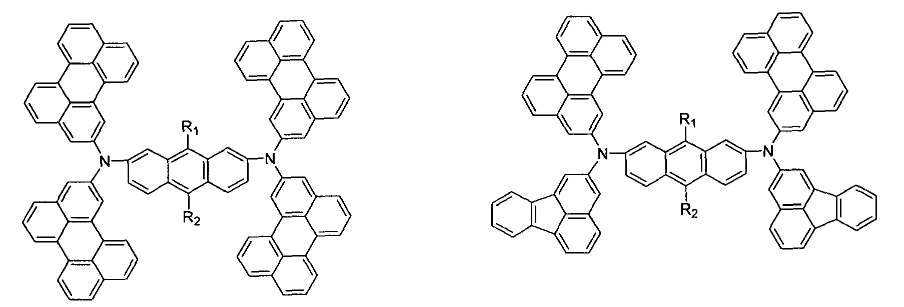 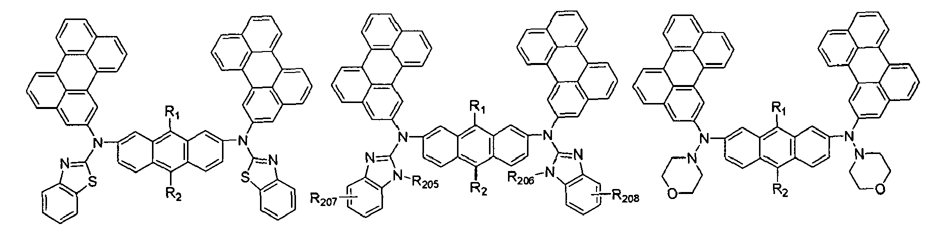 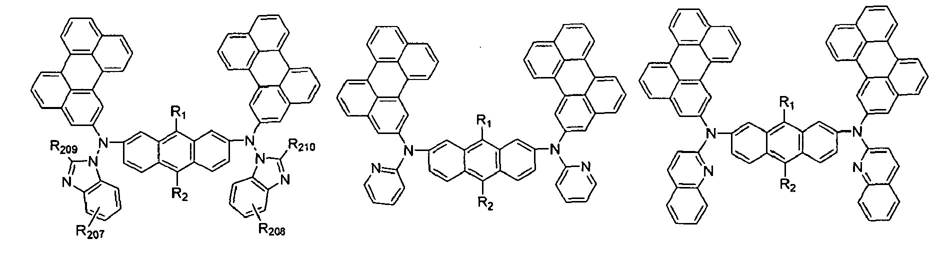 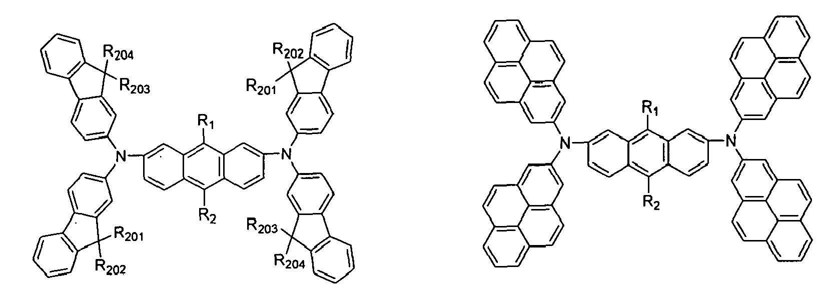 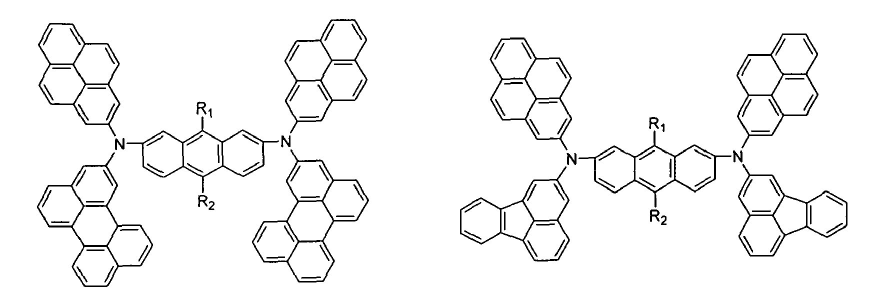 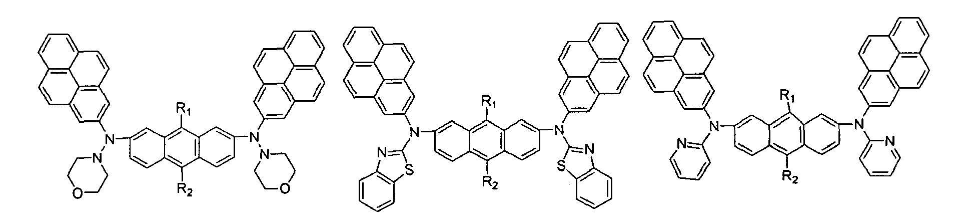 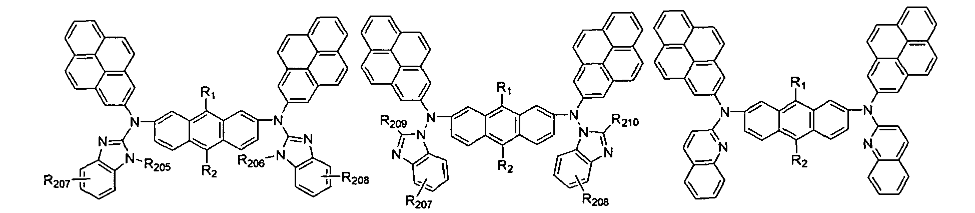 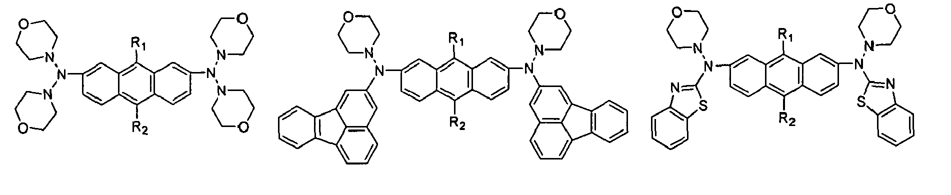 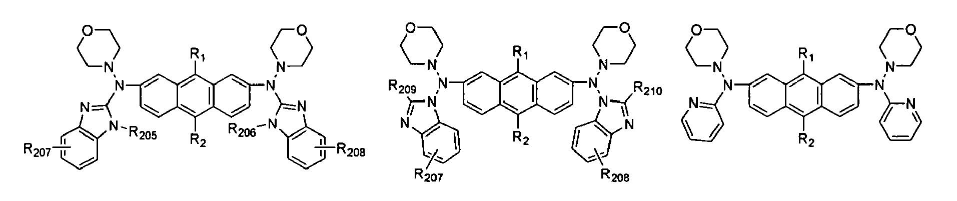 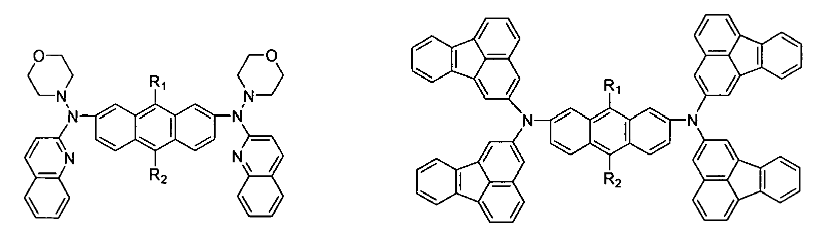 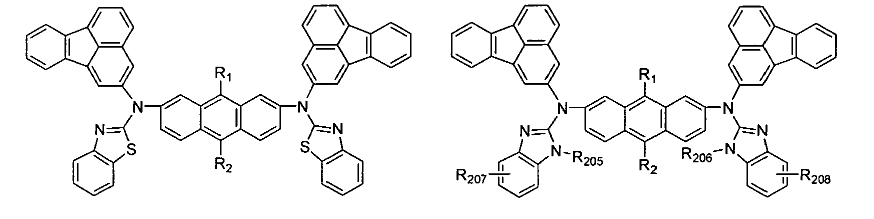 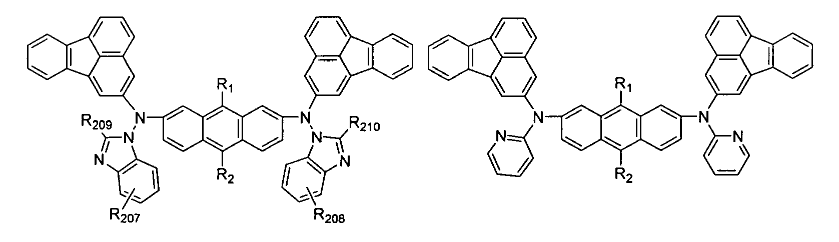 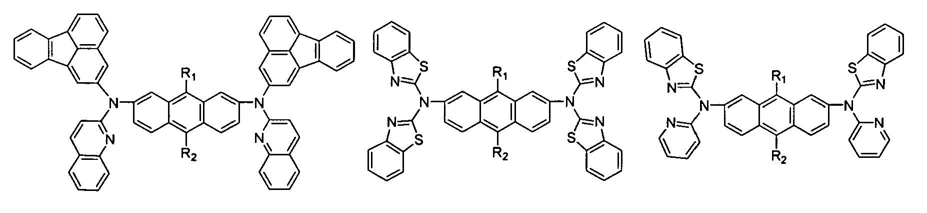 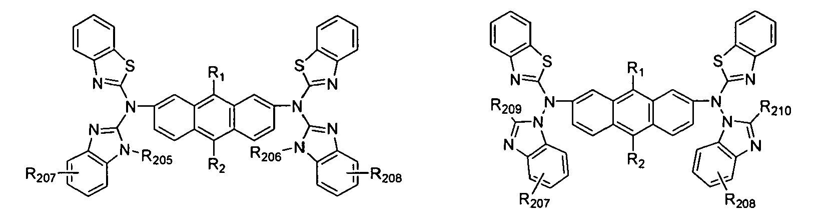 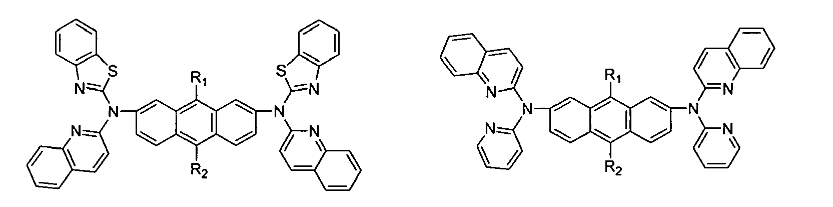 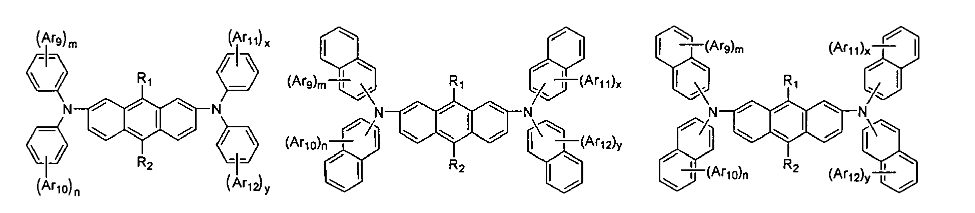 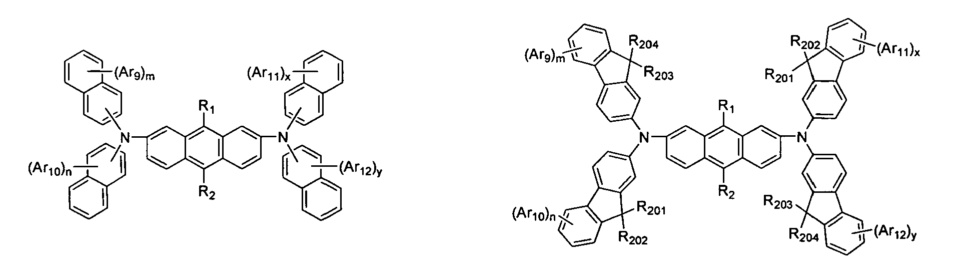 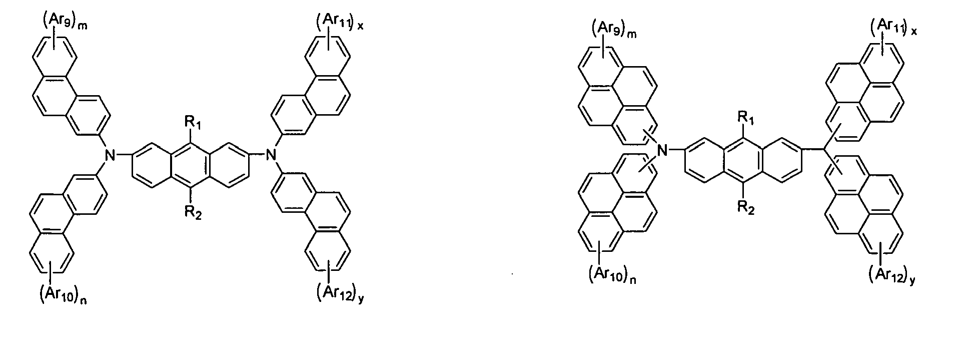 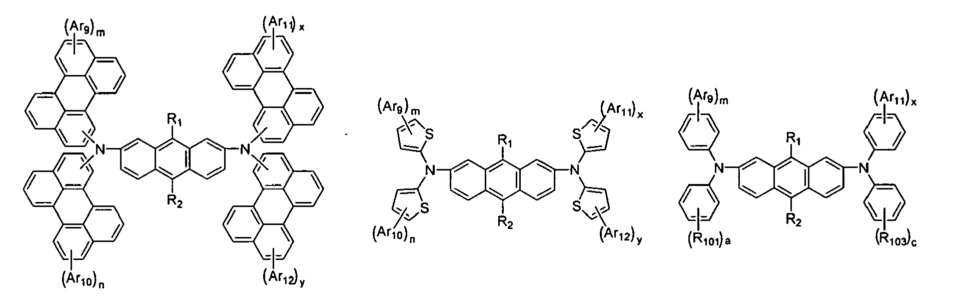 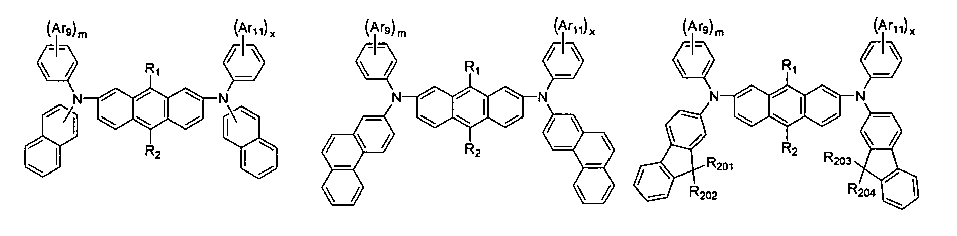 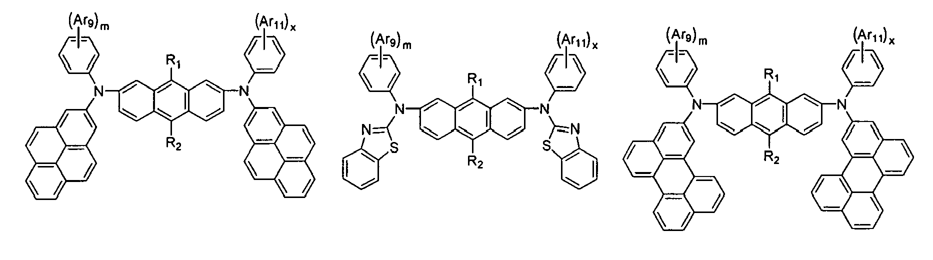 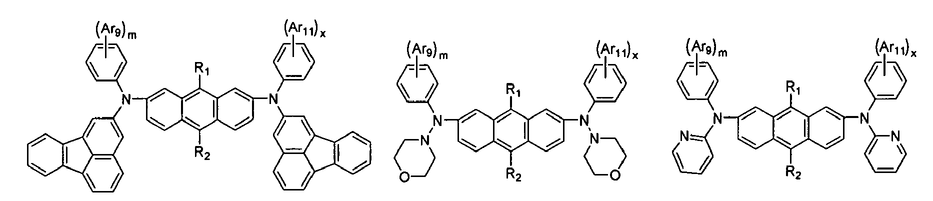 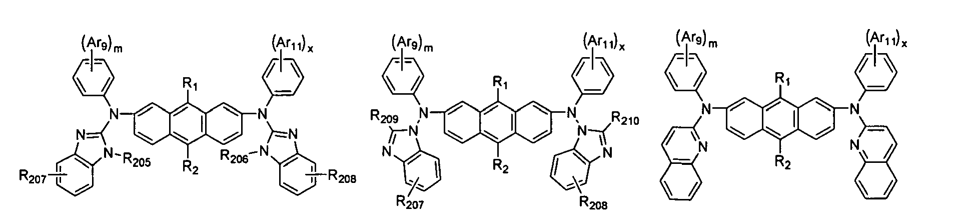 wherein, R₁ and R₂ independently represent methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoroethyl, perfluoropropyl, perfluorobutyl, benzyl, ethenyl, phenylethenyl, ethynyl, phenylethynyl, cyclopentyl, cyclohexyl, cycloheptyl, cyclooctyl, trimethylsilyl, triethylsilyl, tripropylsilyl, tri(t-butyl)silyl, t-butyldimethylsilyl, dimethylphenylsilyl, triphenylsilyl, adamantyl, bicyclo[2.2.1]heptyl, bicyclo[2.2.2]octyl, bicyclo[3.2.1]octyl, bicyclo[5.2.0]nonyl, bicyclo[4.2.2]decyl, bicyclo[2.2.2]octyl, 4-pentylbicyclo[2.2.2]octyl, pyridyl, pyrrolyl, furanyl, thiophenyl, imidazolyl, benzimidazolyl, tetrazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl;
R₁₀₁, R₁₀₂, R₁₀₃ and R₁₀₄ independently represent methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri(t-butyl)silyl, t-butyldimethylsilyl, dimethylphenylsilyl or triphenylsilyl;
R₂₀₁, R₂₀₂, R₂₀₃ and R₂₀₄ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
R₂₀₅ and R₂₀₆ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
R₂₀₇ and R₂₀₈ independently represent hydrogen, methyl, ethyl, n-propyl, i-propyl, n-butyl, i-butyl, t-butyl, n-pentyl, i-pentyl, n-hexyl, n-heptyl, n-octyl, fluoro or cyano;
R₂₀₉ and R₂₁₀ independently represent methyl, ethyl, n-propyl, i-propyl, n-butyl, i-butyl, t-butyl, n-pentyl, i-pentyl, n-hexyl, n-heptyl, n-octyl, 2-ethylhexyl, phenyl or naphthyl;
Ar₉, Ar₁₀, Ar₁₁ and Ar₁₂ independently represent phenyl, biphenyl, naphthyl, anthryl, fluorenyl, phenanthryl, pyrenyl, perylenyl, fluoranthenyl, pyridyl or quinolyl; and the phenyl, naphthyl, anthryl, fluorenyl, phenanthryl, pyrenyl, perylenyl, fluoranthenyl, pyridyl or quinolyl of Ar₉ through Ar₁₂ may be further substituted by one or more substituent(s) selected from methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopropyl, cyclopentyl, cyclohexyl, fluoro, cyano, phenyl, naphthyl, anthryl, trimethylsilyl, triethylsilyl, tripropylsilyl, tri (t-butyl) silyl, t-butyldimethylsilyl, dimethylphenylsilyl and triphenylsilyl;
a, b, c and d independently represent an integer from 0 to 4; and
m, n, x and y independently represent an integer from 1 to 3.

The organic electroluminescent compounds according to the present invention can be prepared according to the process illustrated by Reaction Schemes (1) shown below: 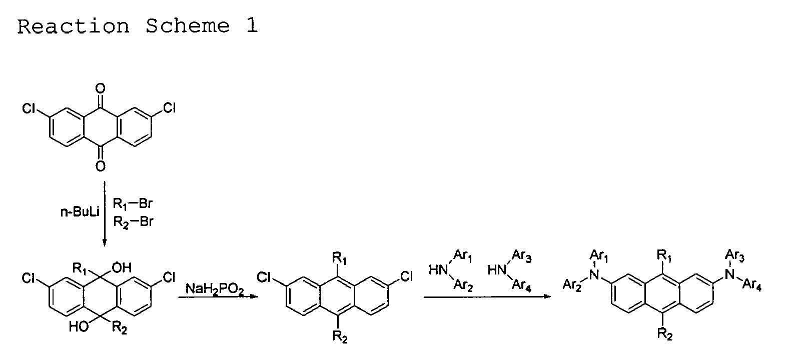 wherein, R₁, R₂, Ar₁, Ar₂, Ar₃ and Ar₄ are defined as in Chemical Formula (1).

The present invention also provides organic solar cells, which comprises one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more electroluminescent compound(s) represented by Chemical Formula (1).

The organic electroluminescent device according to the present invention is characterized in that the organic layer comprises an electroluminescent layer, which comprises one or more organic electroluminescent compound(s) represented by Chemical Formula (1) as electroluminescent dopant, and one or more host(s). The host applied to the organic electroluminescent device according to the invention is not particularly restricted, but is preferably selected from the compounds by one of Chemical Formulas (3) and (4):
Chemical Formula 3 (Ar₂₁)ₑ-L₁-(Ar₂₂)_{f}
Chemical Formula 4 (Ar₂₃)-L₂-(Ar₂₄)ₕ
wherein, L₁ represents (C6-C60)arylene or (C4-C60)heteroarylene;
L₂ represents anthracenylene;
Ar₂₁ through Ar₂₄ are independently selected from hydrogen, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₂₁ through Ar₂₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
e, f, g and h independently represent an integer from 0 to 4.

The hosts represented by Chemical Formula (3) or (4) can be exemplified by anthracene derivatives or benz[a]anthracene derivatives represented by one of Chemical Formulas (5) to (8).

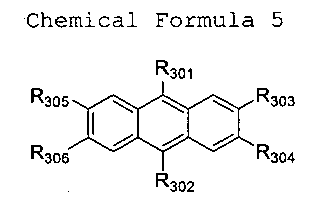

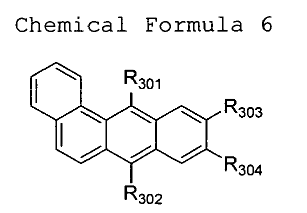

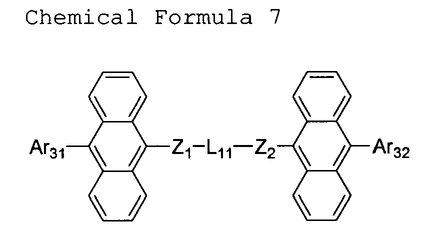

In Chemical Formulas (5) to (7),
R₃₀₁ and R₃₀₂ independently represent hydrogen, deuterium, (C1-C60)alkyl, halogen, (C6-C60)aryl, (C4-C60)heteroaryl, a 5-or 6-membered heterocycloalkyl containing one or more heteroatom(s) selected from N, O and S, or (C3-C60)cycloalkyl, and the aryl or heteroaryl of R₃₀₁ and R₃₀₂ may be further substituted by one or more substituent(s) selected from deuterium, (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₃₀₃ through R₃₀₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₃₀₃ through R₃₀₆ may be further substituted by one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
Z₁ and Z₂ independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₃₁ and Ar₃₂ represent aryl selected from the following structures, or (C4-C60)heteroaryl: 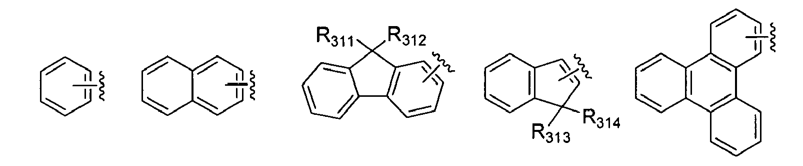 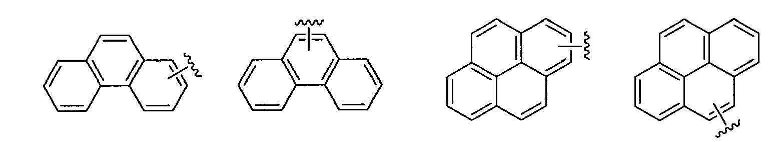 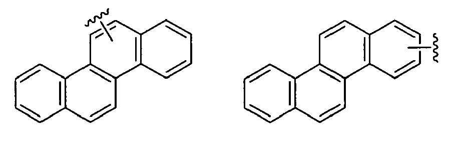 the aryl or heteroaryl of Ar₃₁ and Ar₃₂ may be substituted by one or more substituent(s) selected from deuterium, (C1-C60)alkyl, (C1-C60)alkoxy, (C6-C60)aryl and (C4-C60)heteroaryl;
L₁₁ represents (C6-C60)arylene, (C4-C60)heteroarylene or a compound represented by the following structure: 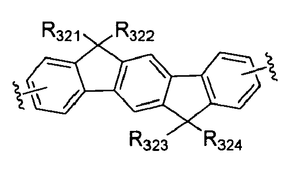 the arylene or heteroarylene of L₁₁ may be substituted by one or more substituent(s) selected from deuterium, (C1-C60)alkyl, (C1-C60)alkoxy, (C6-C60)aryl, (C4-C60)heteroaryl and halogen;
R₃₁₁, R₃₁₂, R₃₁₃ and R₃₁₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₃₂₁, R₃₂₂, R₃₂₃ and R₃₂₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.
Chemical Formula 8

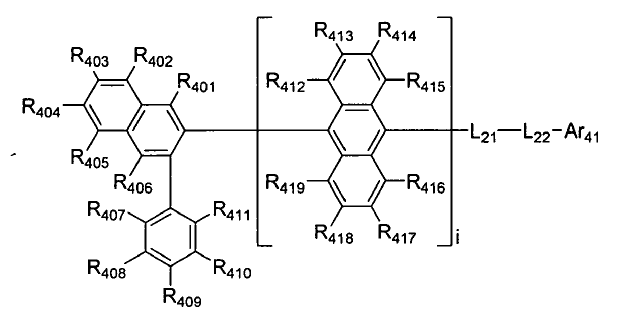

In Chemical Formula 8,
L₂₁ and L₂₂ independently represent a chemical bond, (C6-C60)arylene or (C3-C60)heteroarylene, and the arylene or heteroarylene of L₂₁ and L₂₂ may be further substituted by one or more substituent(s) selected from deuterium, (C1-C60)alkykl, halogen, cyano, (C1-C60)alkoxy, (C3-C60)cycloalkyl, (C6-C60)aryl, (C3-C60)heteroaryl, tri(C1-C30)alkylsilyl, di(C1-C30)alkyl(C6-C30)arylsilyl and tri(C6-C30)arylsilyl;
R₄₀₁ through R₄₁₉ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₄₀₁ through R₄₁₉ may be linked to an adjacent substituent via (C3-C60)alkylene or (C3-C60)alkenylene with or without a fused ring to form an alicyclic ring, or a monocyclic or polycyclic aromatic ring;
Ar₄₁ represents (C6-C60)aryl, (C4-C60)heteroaryl, a 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 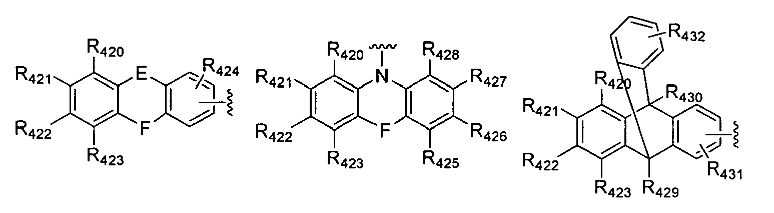 wherein, R₄₂₀ through R₄₃₂ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl;
E and F independently represent a chemical bond, - (CR₄₃₃R₄₃₄)₁-, -N(R₄₃₅)-, -S-, -O-, -Si(R₄₃₆)(R₄₃₇)-, -P(R₄₃₈)-, C(=O)-, -B(R₄₃₉)-, -In(R₄₄₀)-, -Se-, -Ge(R₄₄₁)(R₄₄₂)-, - Sn(R₄₄₃)(R₄₄₄)-, -Ga(R₄₄₅)- or -C(R₄₄₆)=C(R₄₄₇)-;
R₄₃₃ through R₄₄₇ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₄₃₃ through R₄₄₇ may be linked to an adjacent substituent via (C3-C60)alkylene or (C3-C60)alkenylene with or without a fused ring to form an alicyclic ring, or a monocyclic or polycyclic aromatic ring;
the aryl, heteroaryl, heterocycloalkyl, adamantyl or bicycloalkyl of Ar₄₁, or the alkyl, alkenyl, alkynyl, cycloalkyl, heterocycloalkyl, aryl, heteroaryl, arylsilyl, alkylsilyl, alkylamino or arylamino of R₄₀₁ through R₄₃₂ may be further substituted by one or more substituent(s) selected from deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
i is an integer from 1 to 4; and
l is an integer from 0 to 4.

The electroluminescent layer means the layer where electroluminescence occurs, and it may be a single layer or a multi-layer consisting of two or more layers laminated. When a mixture of host-dopant is used according to the construction of the present invention, noticeable improvement in luminous efficiency could be confirmed. This can be achieved by the doping concentration of 0.5 to 10% by weight. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (5) to (8) as an electroluminescent host supplements electronic drawback of the organic electroluminescent compounds of Chemical Formula (1) according to the present invention.

The host compounds represented by one of Chemical Formulas (5) to (8) can be exemplified by the following compounds, but are not restricted thereto.

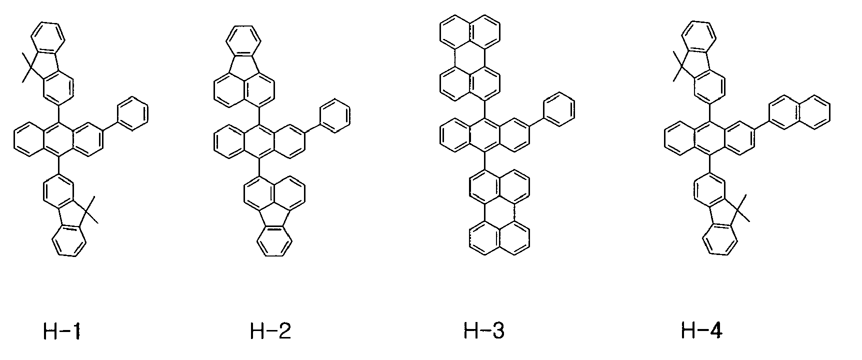

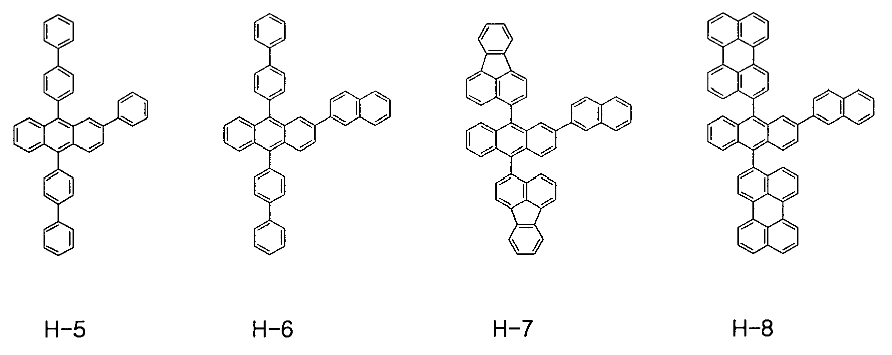

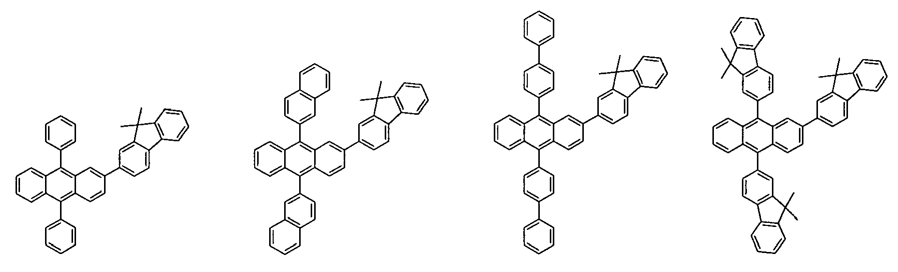

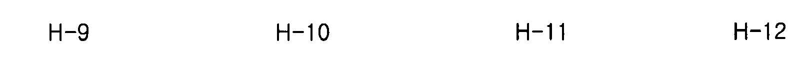

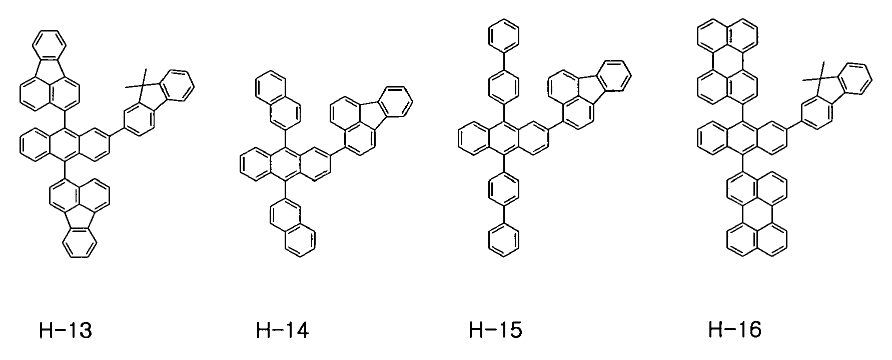

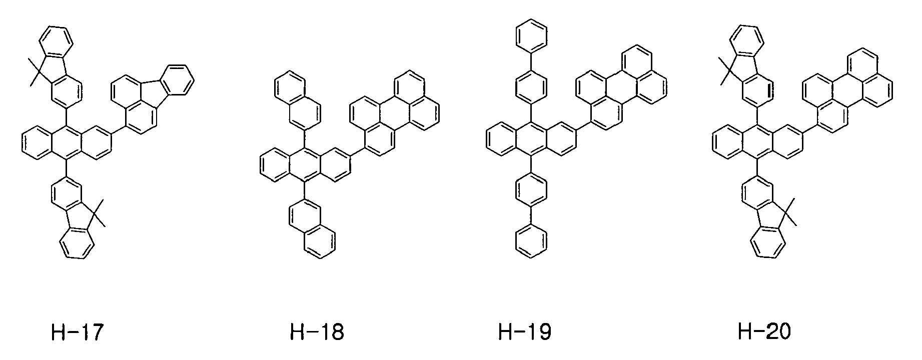

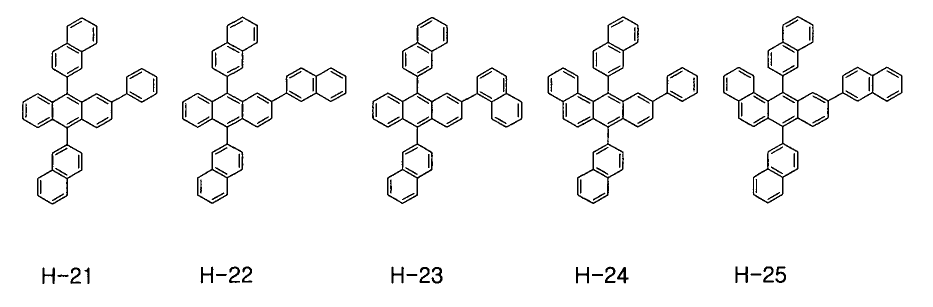

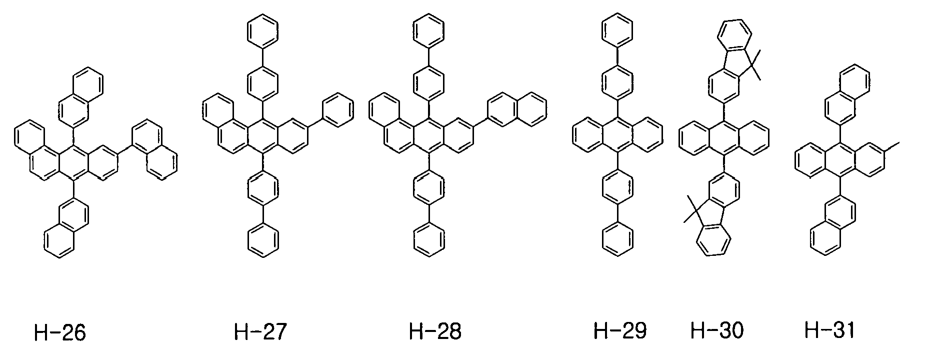

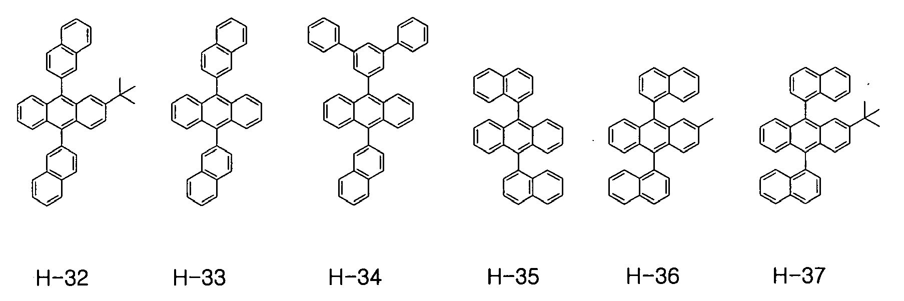

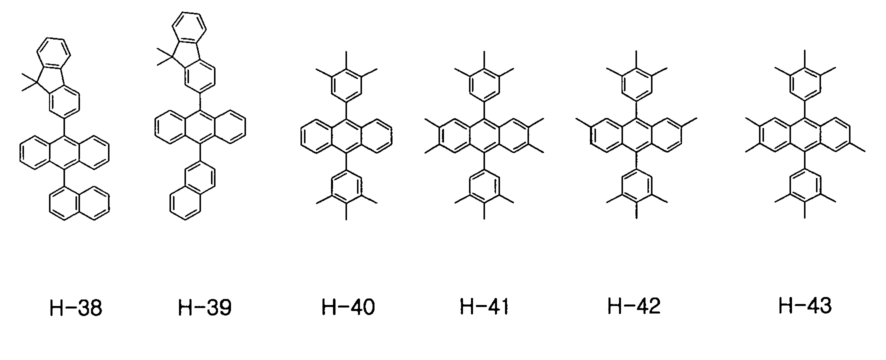

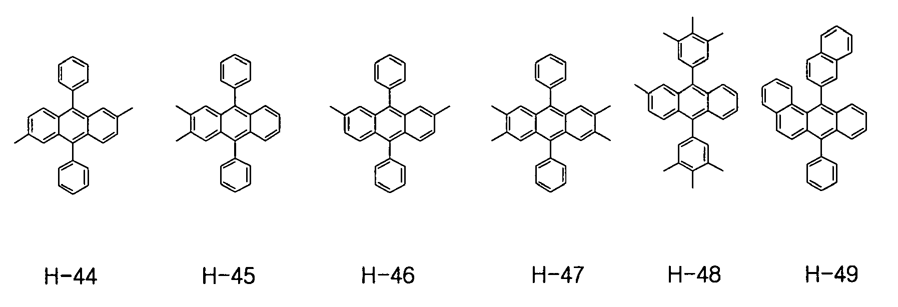

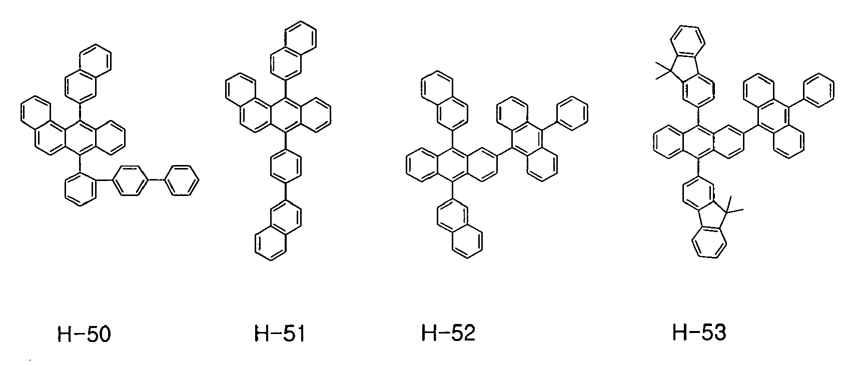

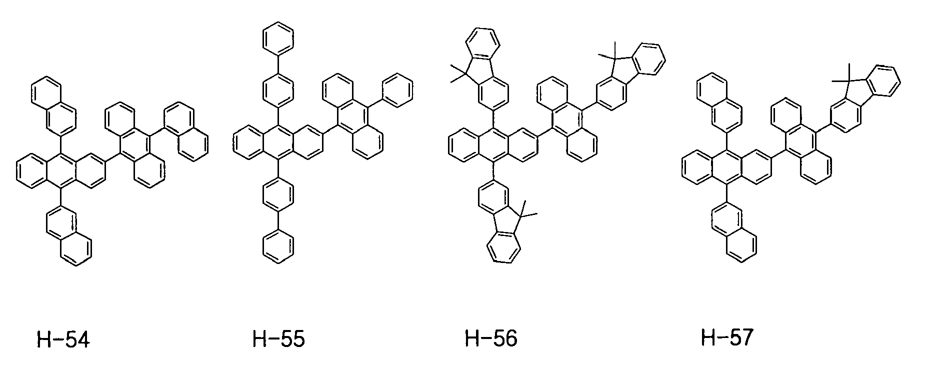

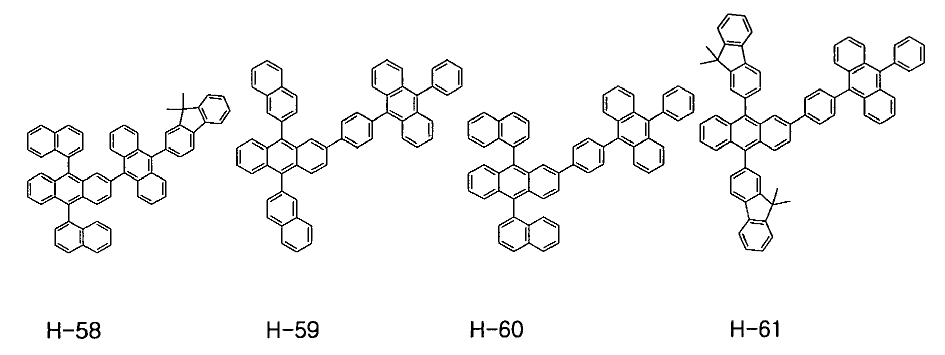

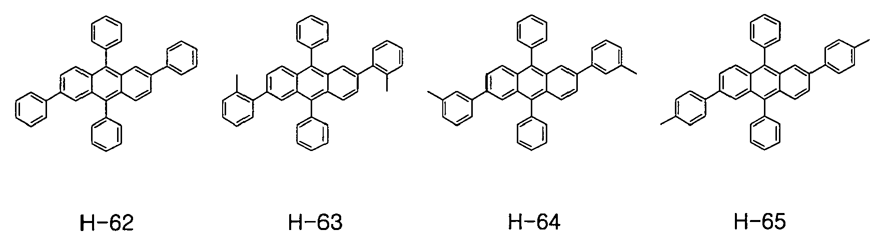

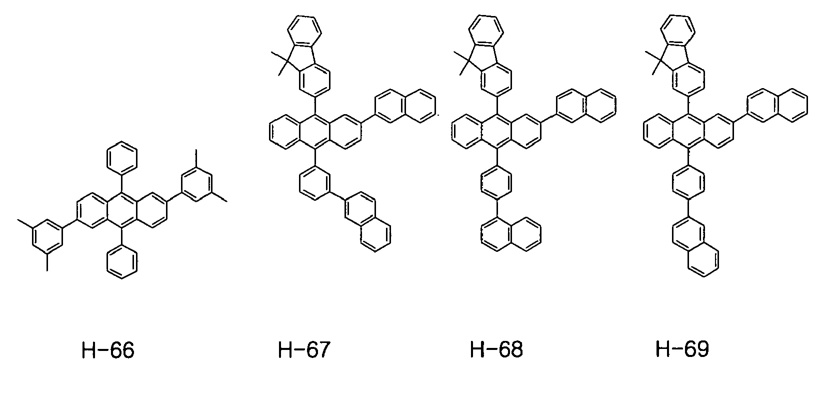

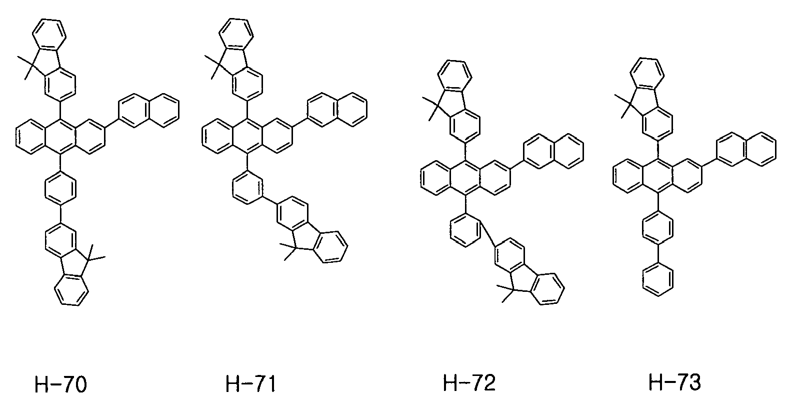

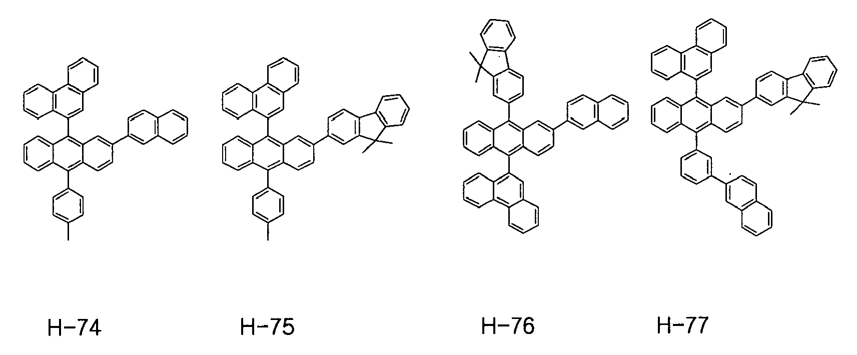

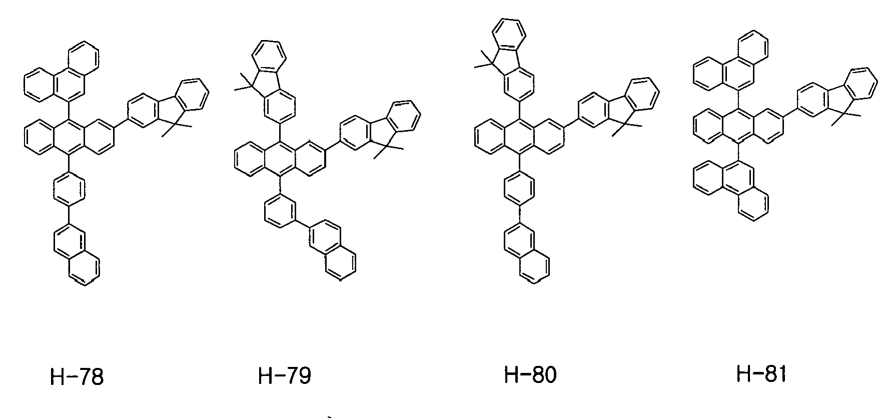

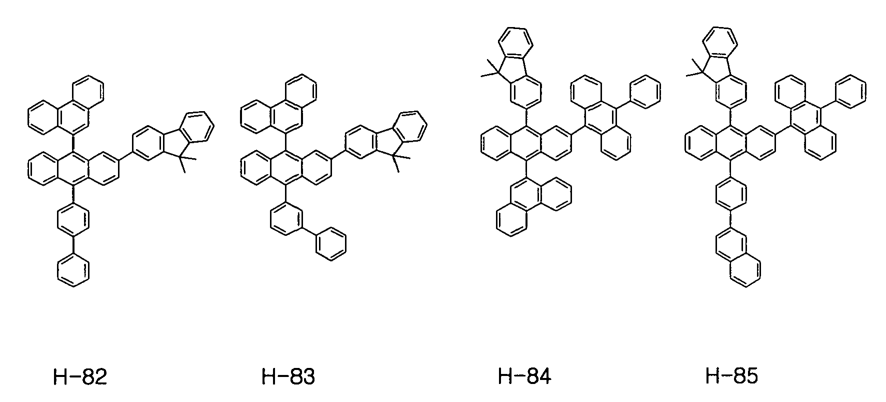

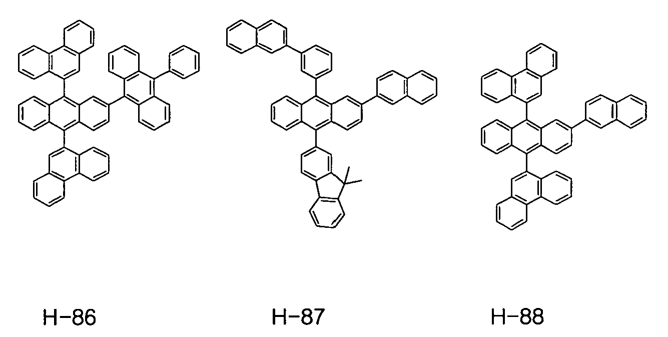

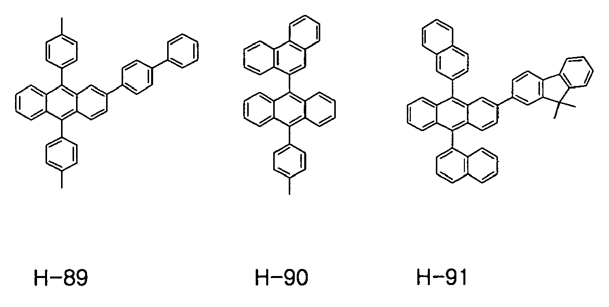

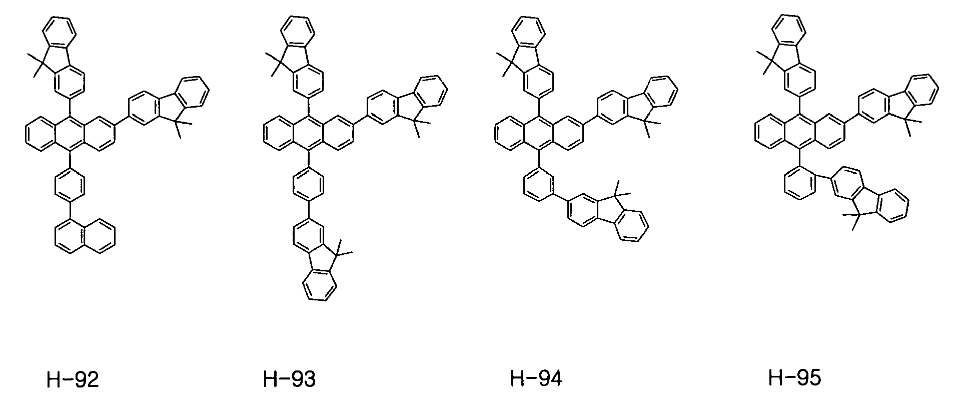

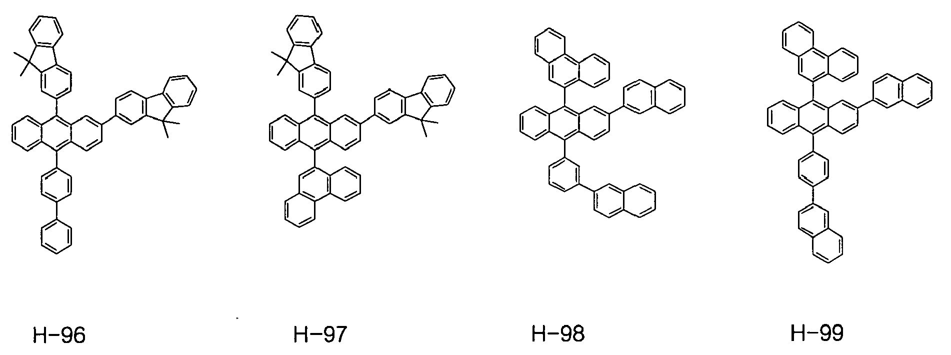

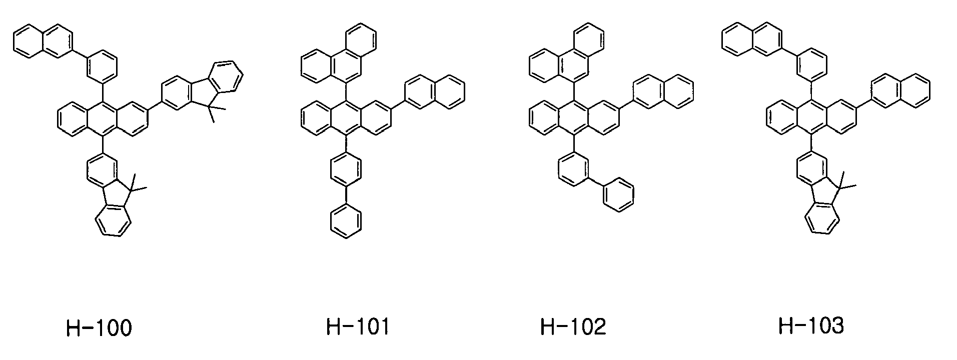

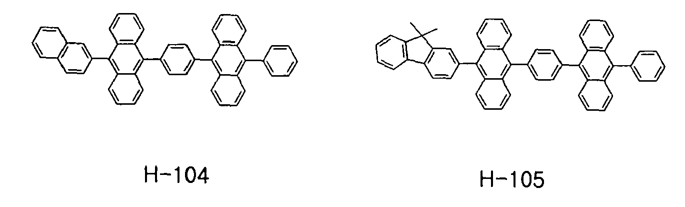

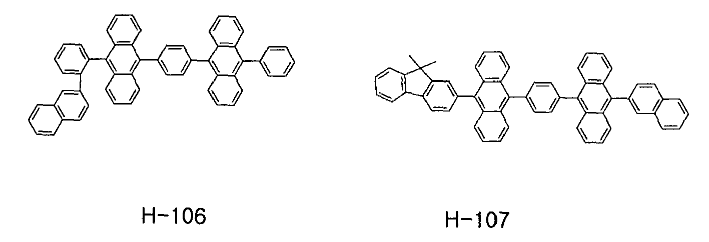

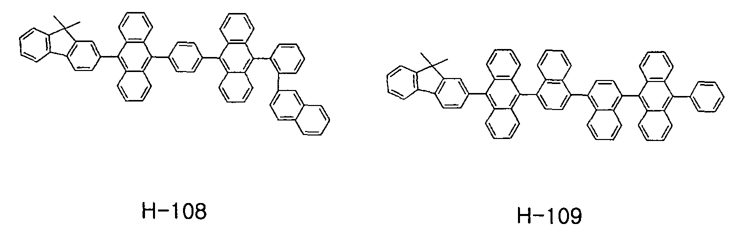

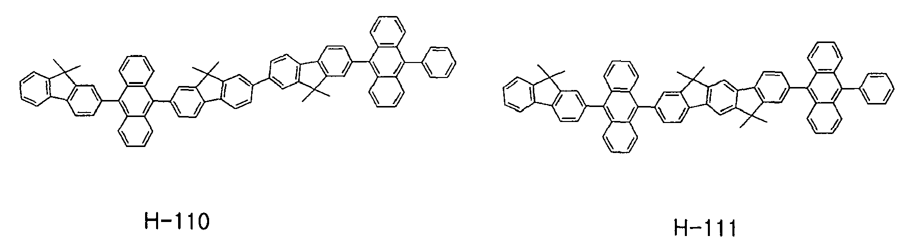

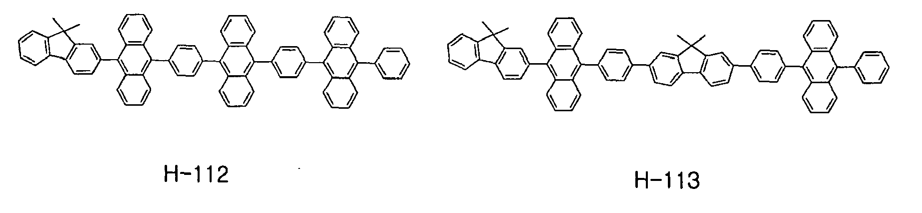

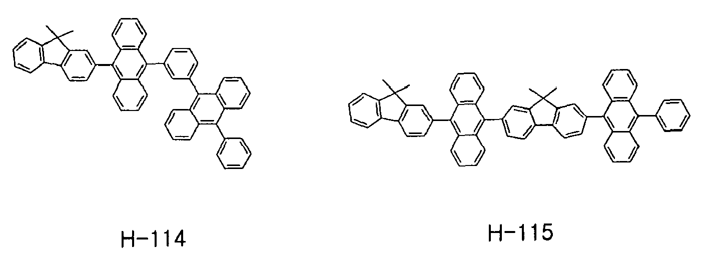

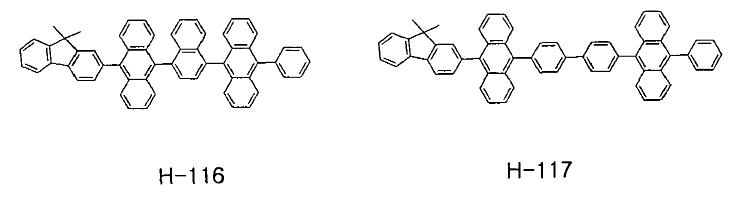

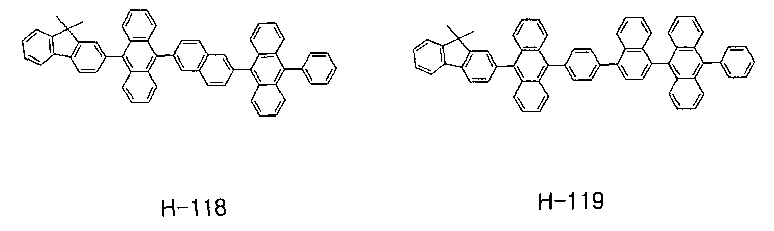

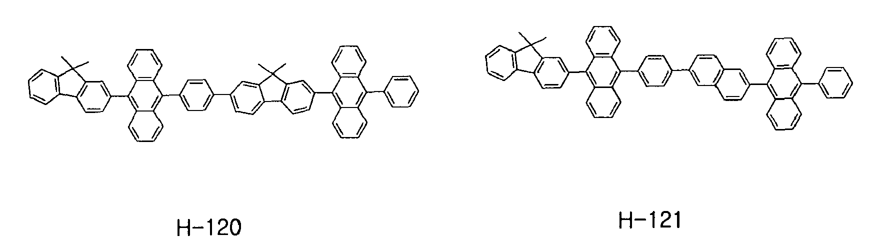

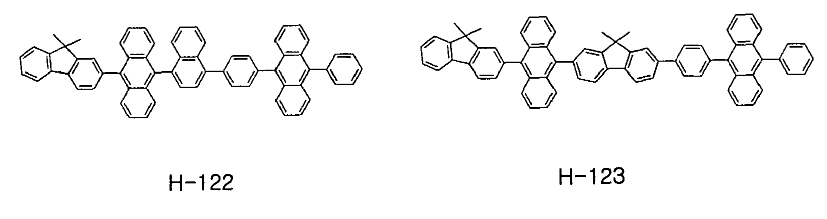

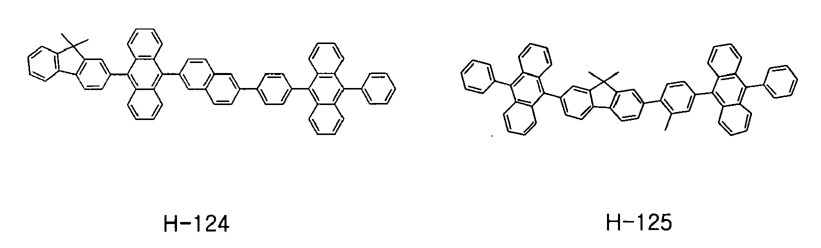

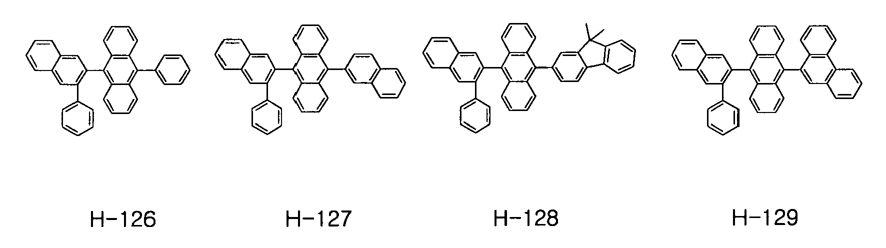

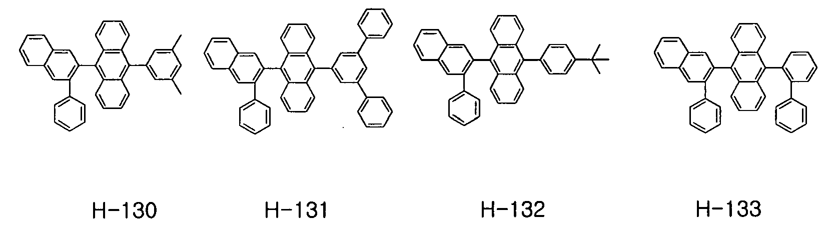

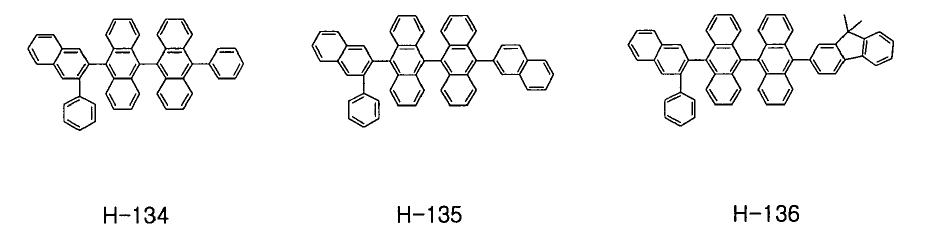

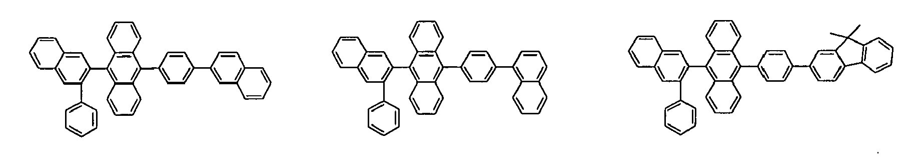

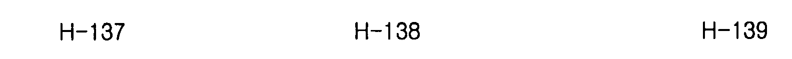

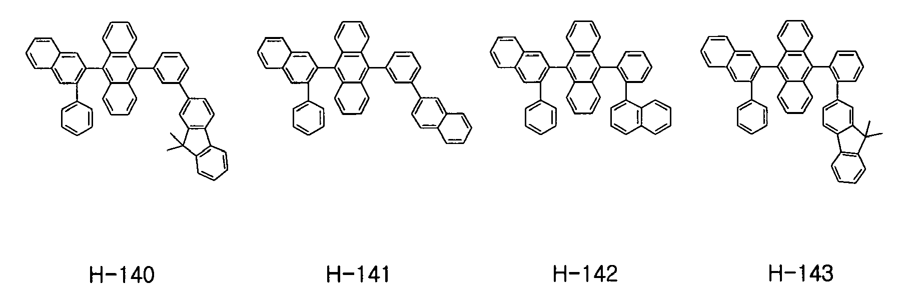

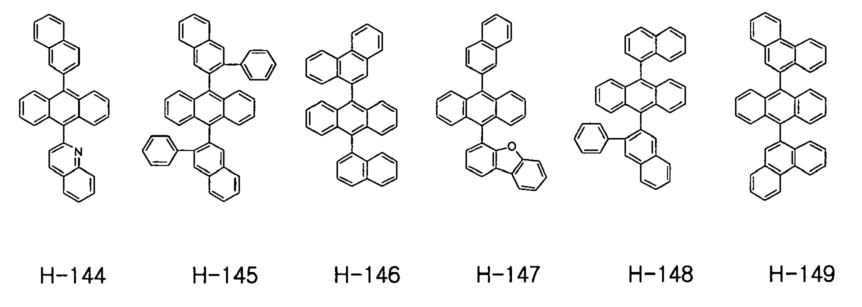

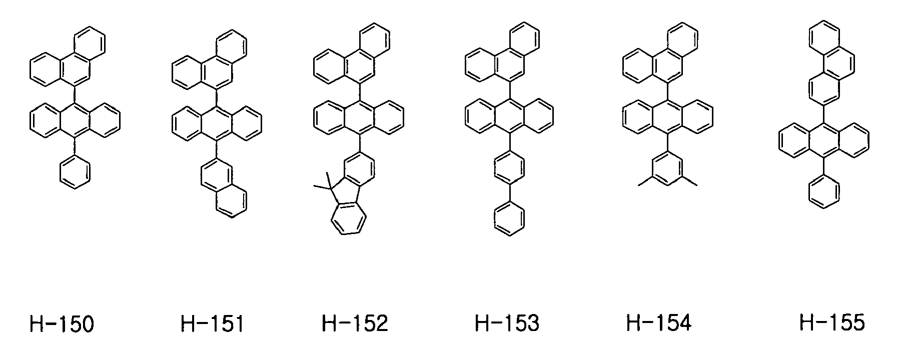

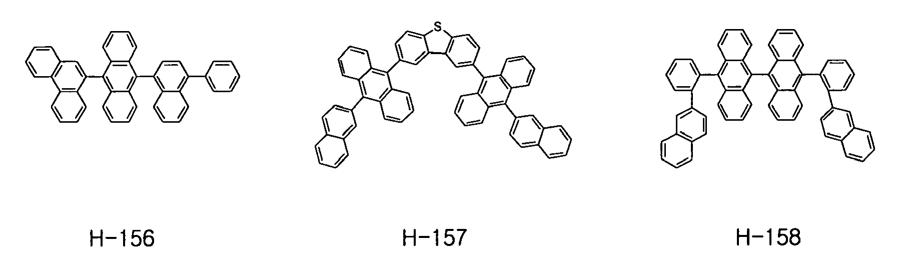

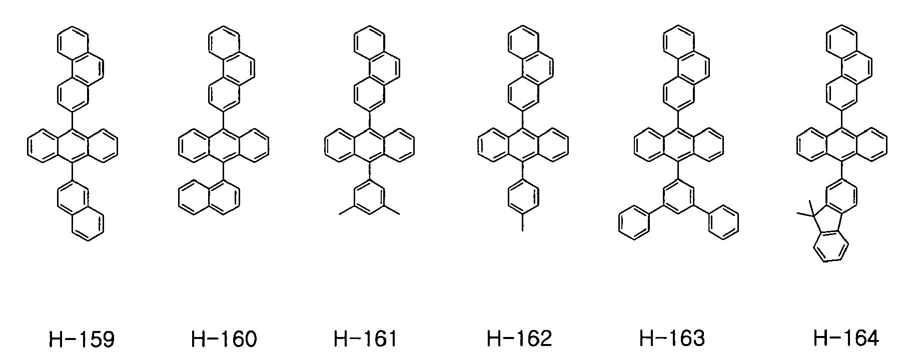

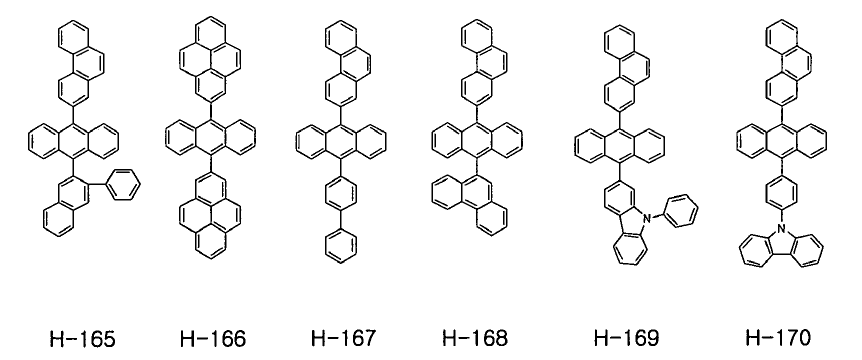

The organic electroluminescent device according to the invention may further comprise one or more compound(s) selected from arylamine compounds and styrylarylamine compounds, as well as the organic electroluminescent compound represented by Chemical Formula (1). Examples of arylamine or styrylarylamine compounds include the compounds represented by Chemical Formula (9), but they are not restricted thereto: 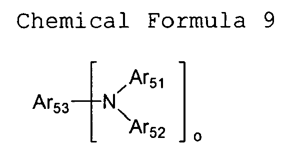 wherein, Ar₅₁ and Ar₅₂ independently represent (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₅₁ and Ar₅₂ may be linked via (C3-C60)alkylene or (C3-C60)alkenylene with or without a fused ring to form an alicyclic ring, or a monocyclic or polycyclic aromatic ring;
when o is 1, Ar₅₃ represents (C6-C60)aryl, (C4-C60)heteroaryl, or a substituent represented by one of the following structural formulas: 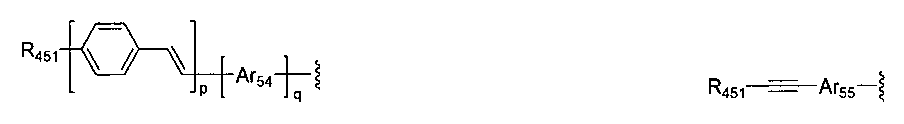 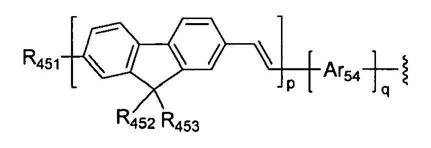 when o is 2, Ar₅₃ represents (C6-C60)arylene, (C4-C60)heteroarylene, or an arylene represented by one of the following structural formulas: 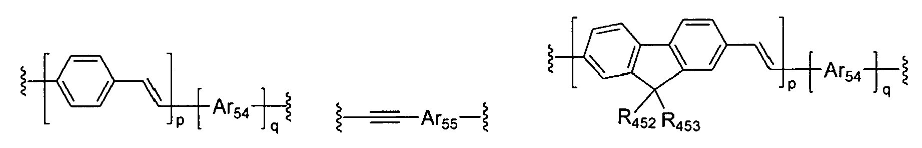 wherein Ar₅₄ and Ar₅₅ independently represent (C6-C60)arylene or (C4-C60)heteroarylene;
R₄₅₁, R₄₅₂ and R₄₅₃ independently represent hydrogen, (C1-C60)alkyl or (C6-C60)aryl;
p is an integer from 1 to 4, q is an integer of 0 or 1; and
the alkyl, aryl, heteroaryl, arylamino, alkylamino, cycloalkyl or heterocycloalkyl of Ar₅₁ and Ar₅₂, or the aryl, heteroaryl, arylene or heteroarylene of Ar₅₃, or the arylene or heteroarylene of Ar₅₄ and Ar₅₅, or the alkyl or aryl of R₄₅₁ through R₄₅₃ may be further substituted by one or more substituent(s) selected from a group consisting of deuterium, halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds and styrylarylamine compounds may be more specifically exemplified by the following compounds, but are not restricted thereto.

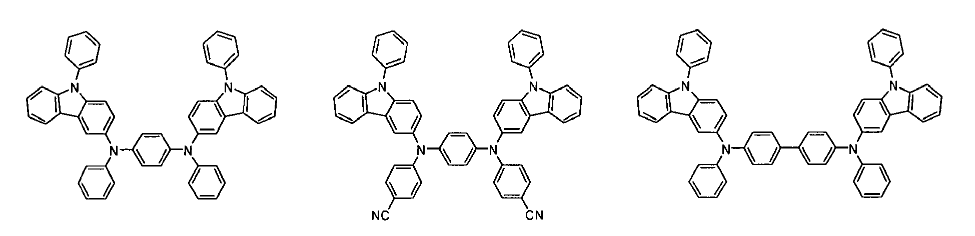

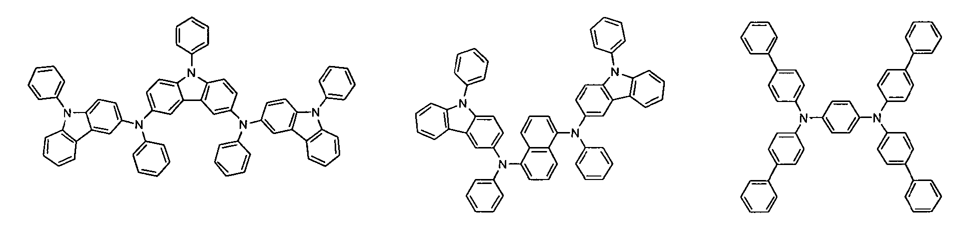

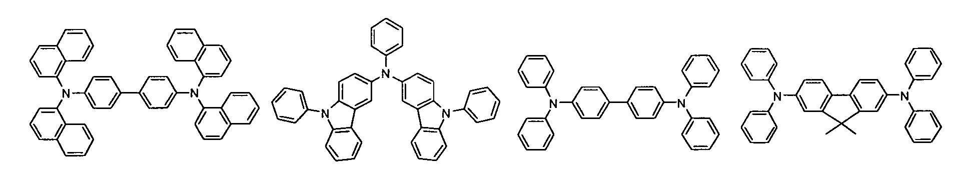

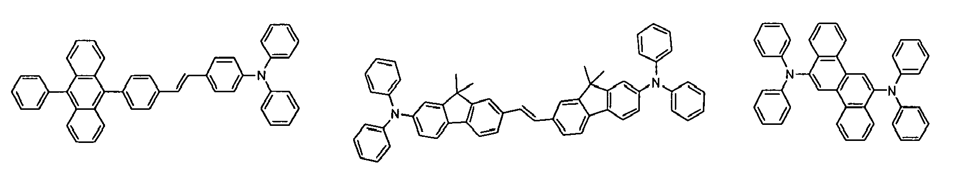

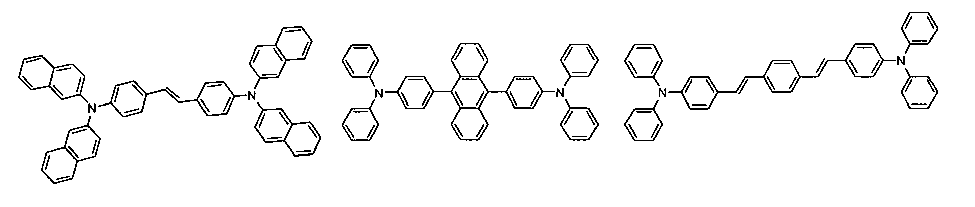

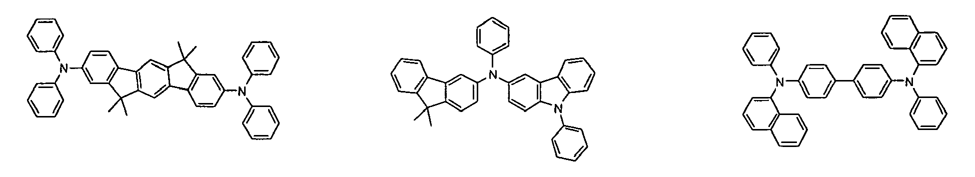

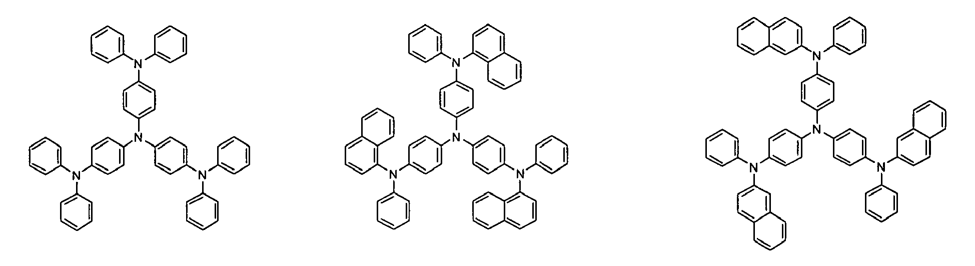

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements, as well as the organic electroluminescent compound represented by Chemical Formula (1). The organic layer may comprise a charge generating layer in addition to the electroluminescent layer.

The present invention can realize an electroluminescent device having a pixel structure of independent light-emitting mode, which comprises an organic electroluminescent device containing the compound of Chemical Formula (1) as a sub-pixel, and one or more sub-pixel(s) comprising one or more metal compound(s) selected from a group consisting of Ir, Pt, Pd, Rh, Re, Os, T1, Pb, Bi, In, Sn, Sb, Te, Au and Ag, patterned in parallel at the same time.

Further, the organic electroluminescent device is an organic display wherein the organic layer comprises, in addition to the organic electroluminescent compound according to the invention, one or more compound(s) selected from compounds having electroluminescent peak of wavelength of not more than 500 nm or those having electroluminescent peak of wavelength of not less than 560 nm, at the same time. The compounds having electroluminescent peak of wavelength of not more than 500 nm or those having electroluminescent peak of wavelength of not less than 560 nm may be exemplified by the compounds represented by one of Chemical Formulas (10) to (16), but they are not restricted thereto.

Chemical Formula 10 M¹L¹⁰¹L¹⁰²L¹⁰³

In Chemical Formula (10), M¹ is selected from Group 7, 8, 9, 10, 11, 13, 14, 15 and 16 metals in the Periodic Table, and ligands L¹⁰¹, L¹⁰² and L¹⁰³ are independently selected from the following structures: 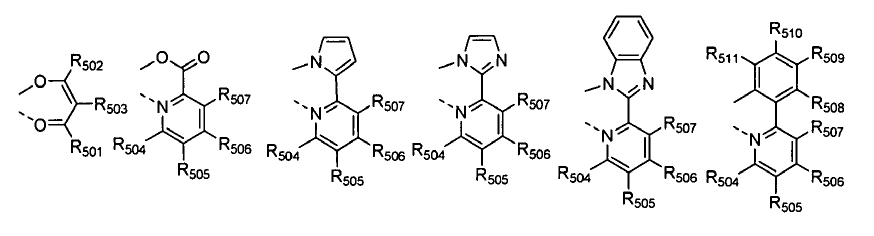 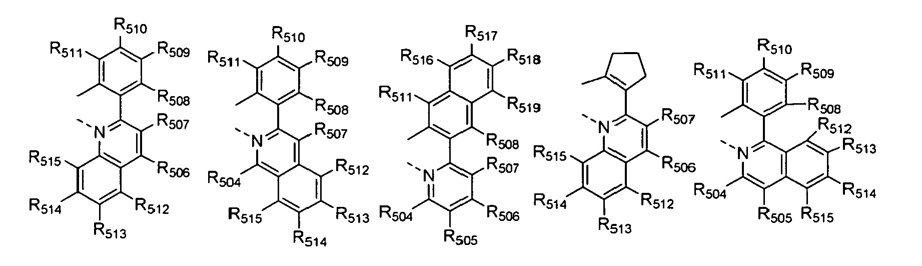 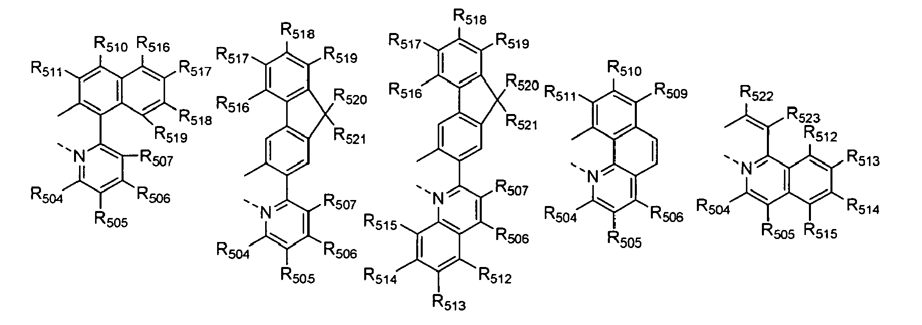 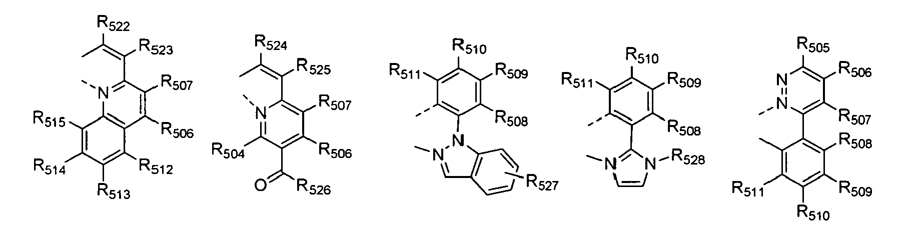 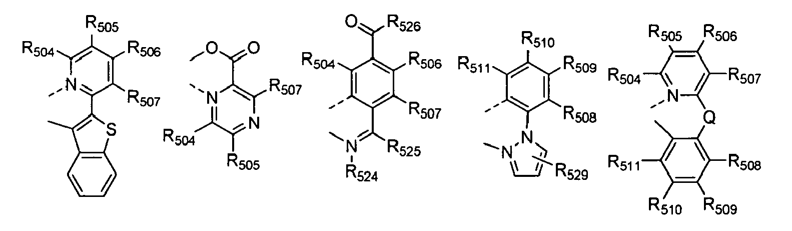 wherein, R₅₀₁ through R₅₀₃ independently represent hydrogen, (C1-C60)alkyl with or without halogen substituent(s), (C6-C60)aryl with or without (C1-C60)alkyl substituent(s), or halogen;
R₅₀₄ through R₅₁₉ independently represent hydrogen, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₅₀₄ through R₅₁₉ may be further substituted by one or more substituent(s) selected from (C1-C60)alkyl, (C6-C60)aryl and halogen;
R₅₂₀ through R₅₂₃ independently represent hydrogen, (C1-C60)alkyl with or without halogen substituent(s), (C6-C60)aryl with or without (C1-C60)alkyl substituent(s);
R₅₂₄ and R₅₂₅ independently represent hydrogen, (C1-C60)alkyl, (C6-C60) aryl or halogen, or R₅₂₄ and R₅₂₅ may be linked via (C3-C12)alkylene or (C3-C12)alkenylene with or without a fused ring to form an alicyclic ring, or a monocyclic or polycyclic aromatic ring; and the alkyl or aryl of R₅₂₄ and R₅₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from (C1-C60)alkyl with or without halogen substituent(s), (C1-C30)alkoxy, halogen, tri(C1-C30)alkylsilyl, tri(C6-C30)arylsilyl and (C6-C60)aryl;
R₅₂₆ represents (C1-C60)alkyl, (C6-C60)aryl, or (C5-C60)heteroaryl containing one or more heteroatom(s) selected from N, O and S, or halogen;
R₅₂₇ through R₅₂₉ independently represent hydrogen, (C1-C60)alkyl, (C6-C60)aryl or halogen, and the alkyl or aryl of R₅₂₆ through R₅₂₉ may be further substituted by halogen or (C1-C60)alkyl; Q represents 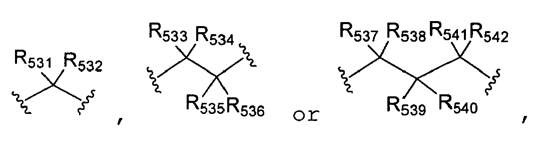 and R₅₃₁ through R₅₄₂ independently represent hydrogen, (C1-C60)alkyl with or without halogen substituent(s), (C1-C30)alkoxy, halogen, (C6-C60)aryl, cyano, (C5-C60)cycloalkyl, or each of R₅₃₁ through R₅₄₂ may be linked to an adjacent substituent via alkylene or alkenylene to form a (C5-C7) spiro-ring or (C5-C9) fused ring, or each of them may be linked to R₅₀₇ or R₅₀₈ to form a (C5-C7) fused ring.

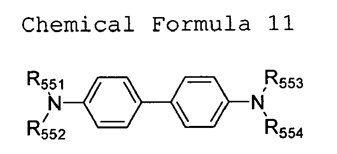

In Chemical Formula (11), R₅₅₁ through R₅₅₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl, aryl of R₅₅₁ through R₅₅₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

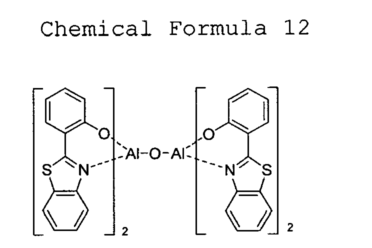

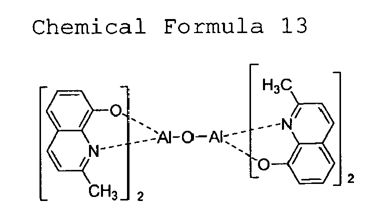

Chemical Formula 14 L²⁰¹L²⁰²M²(T)ᵣ

In Chemical Formula (14), the ligands, L²⁰¹ and L²⁰² are independently selected from the following structures: 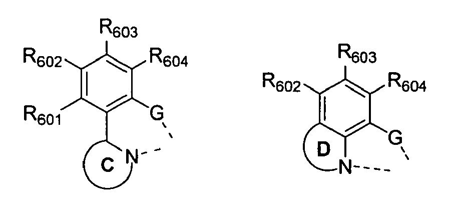 M² is a bivalent or trivalent metal;
r is 0 when M² is a bivalent metal, while r is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alkyl, or phenyl or naphthyl with or without (C1-C60)alkyl substituent(s);
R₆₀₁ through R₆₀₄ independently represent hydrogen, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₆₀₁ to form a fused ring;
the ring C and aryl of R₆₀₁ through R₆₀₄ may be further substituted by one or more substituent(s) selected from (C1-C60)alkyl, halogen, (C1-C60)alkyl with halogen substituent(s), phenyl, naphthyl, tri(C1-C60)alkylsilyl, tri(C6-C60)arylsilyl or amino group.

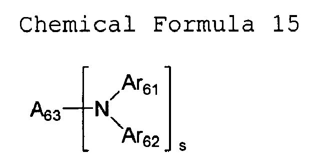

In Chemical Formula (15), Ar₆₁ and Ar₆₂ independently represent hydrogen, deuterium, halogen, (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₆₁ and Ar₆₂ may be linked via (C3-C60)alkylene or (C3-C60)alkenylene with or without a fused ring to form an alicyclic ring, or a monocyclic or polycyclic aromatic ring; when s is 1, Ar₆₃ represents (C6-C60)aryl, (C4-C60)heteroaryl, or a substituent represented by one of the following structural formulas: 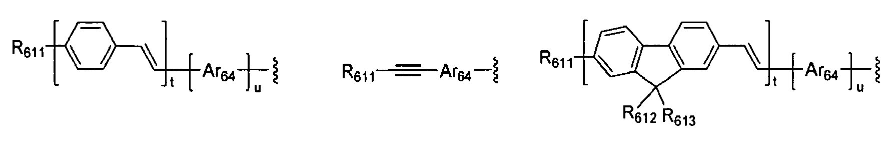 when s is 2, Ar₆₃ represents (C6-C60)arylene, (C4-C60)heteroarylene, or a substituent represented by one of the following structural formulas: 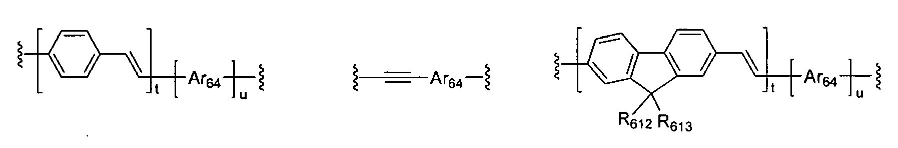 wherein Ar₆₁ and Ar₆₂ independently represent (C6-C60)arylene or (C4-C60)heteroarylene;
R₆₁₁ through R₆₁₃ independently represent hydrogen, deuterium, (C1-C60)alkyl or (C6-C60)aryl;
t is an integer from 1 to 4, u is an integer of 0 or 1; and
the alkyl, aryl, heteroaryl, arylamino, alkylamino, cycloalkyl or heterocycloalkyl of Ar₆₁ and Ar₆₂, or the aryl, heteroaryl, arylene or heteroarylene of Ar₆₃, or the arylene or heteroarylene of Ar₆₄ and Ar₆₅, or the alkyl or aryl of R₆₁₁ through R₆₁₃ may be further substituted by one or more substituent(s) selected from a group consisting of halogen, deuterium, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

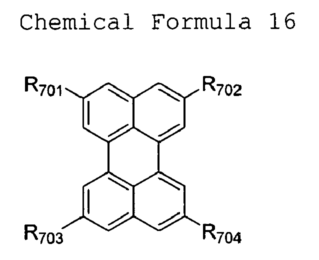

In Chemical Formula (16), R₇₀₁ through R₇₀₄ independently represent hydrogen, deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro or hydroxyl, or each of R₇₀₁ through R₇₀₄ may be linked to an adjacent substituent via (C3-C60)alkylene or (C3-C60)alkenylene with or without a fused ring to form an alicyclic ring, or a monocyclic or polycyclic aromatic ring;
the alkyl, alkenyl, alkynyl, cycloalkyl, heterocycloalkyl, aryl, heteroaryl, arylsilyl, alkylsilyl, alkylamino or arylamino of R₇₀₁ through R₇₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from deuterium, halogen, (C1-C60)alkyl, (C6-C60)aryl, (C4-C60)heteroaryl containing one or more heteroatom(s) selected from N, O and S, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds for an electroluminescent layer, having electroluminescent peak of wavelength of not more than 500 nm or those having electroluminescent peak of wavelength of not less than 560 nm, may be exemplified by the following compounds, but they are not restricted thereto.

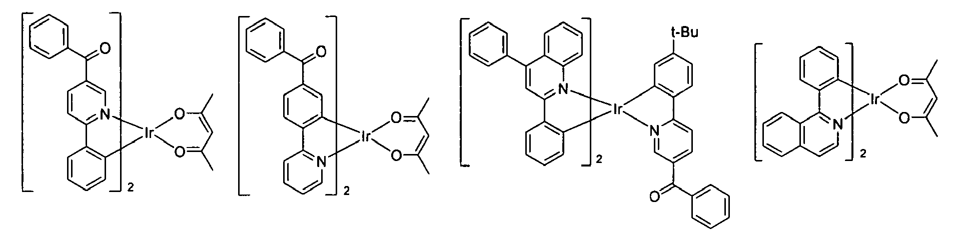

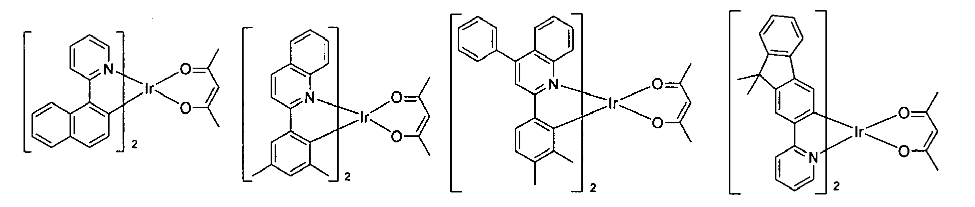

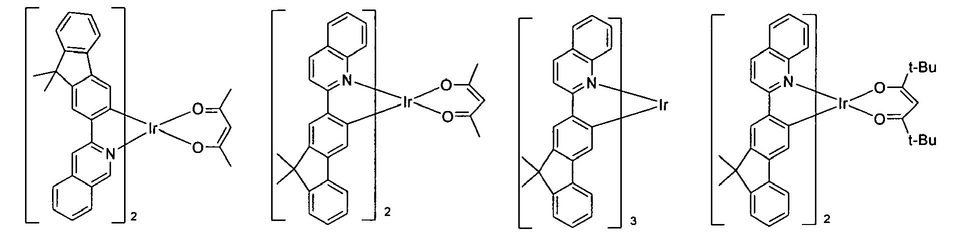

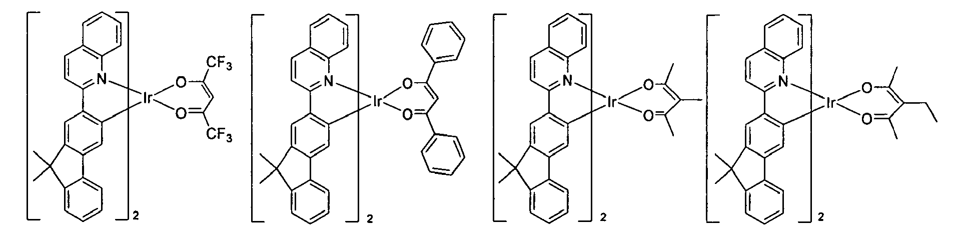

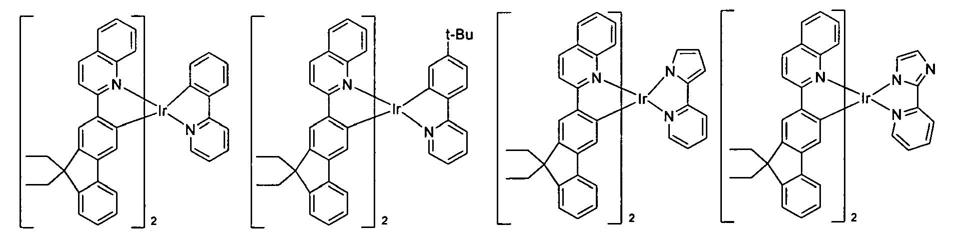

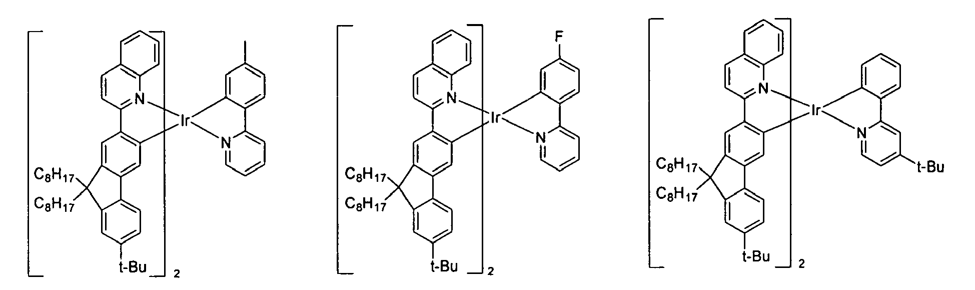

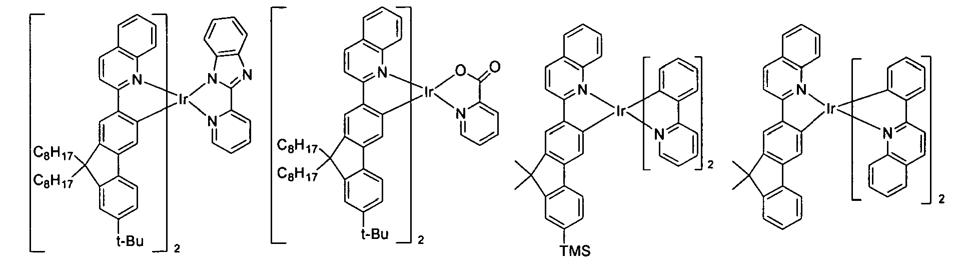

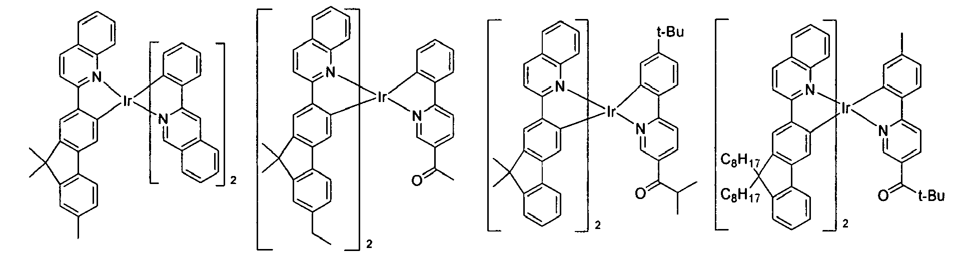

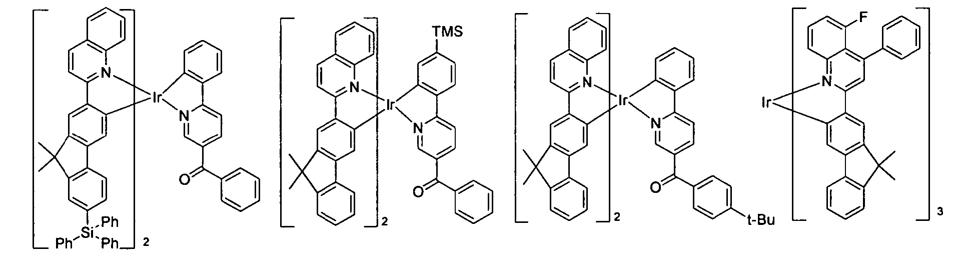

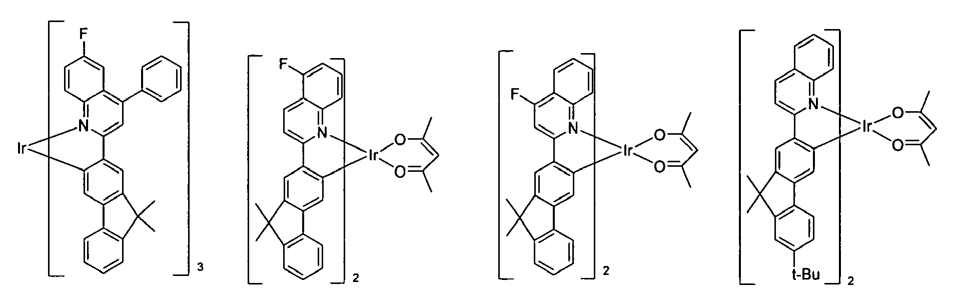

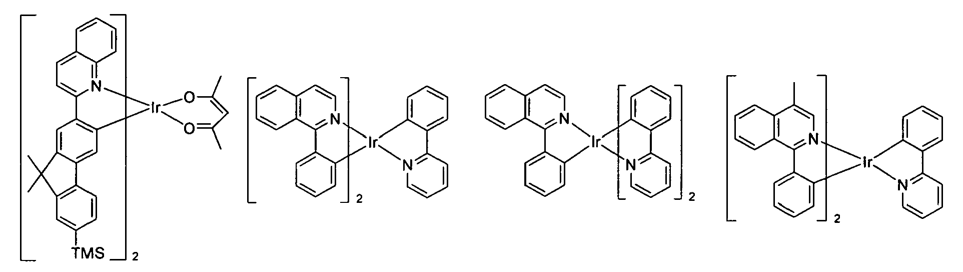

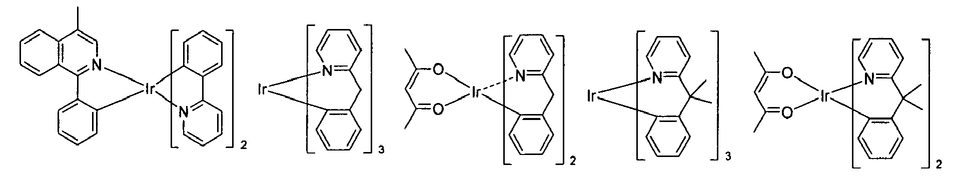

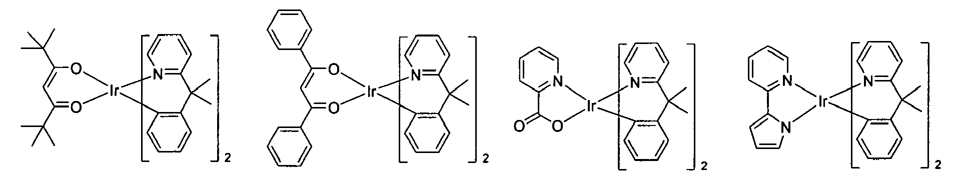

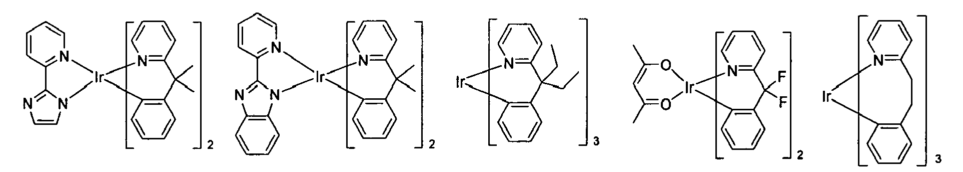

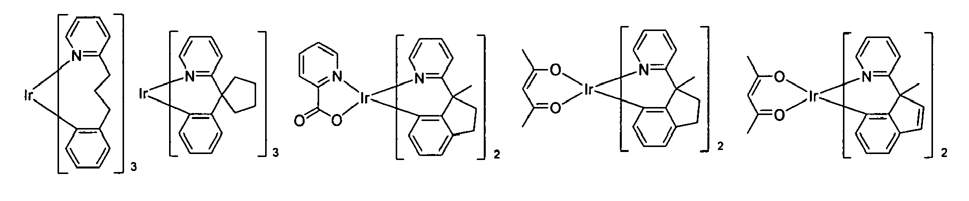

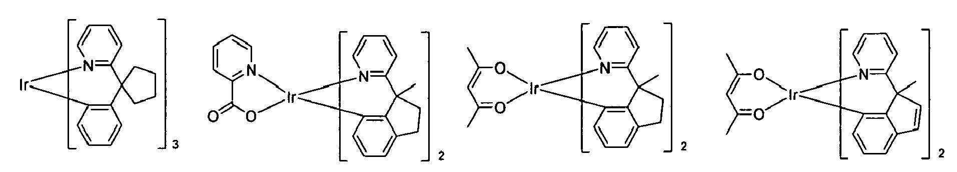

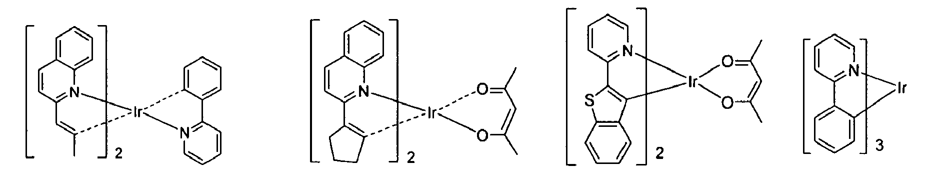

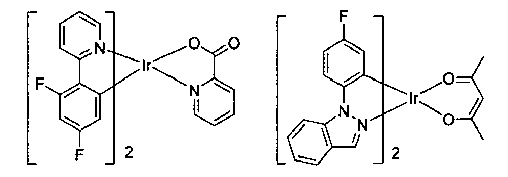

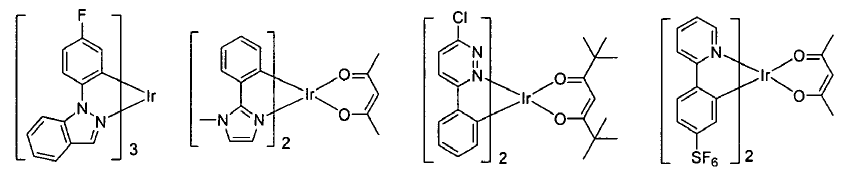

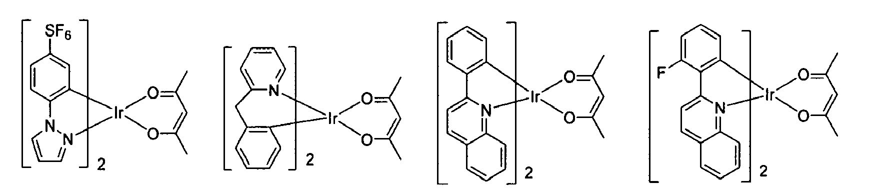

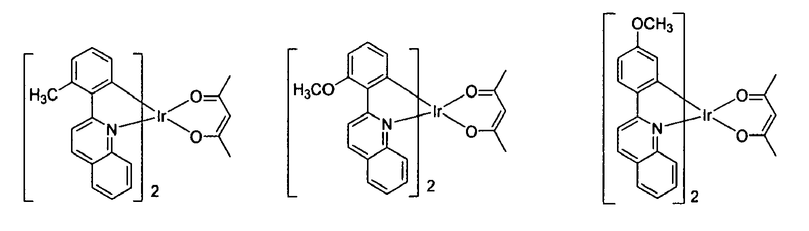

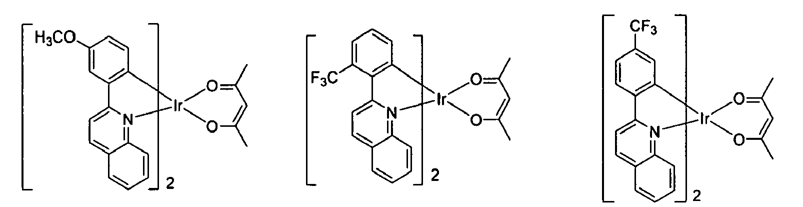

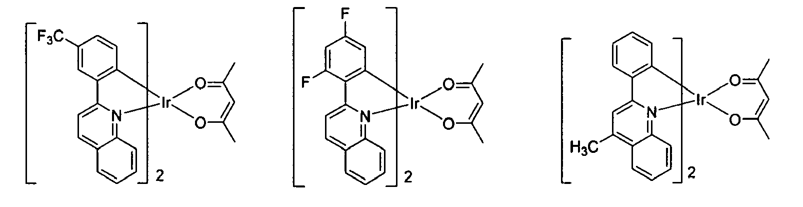

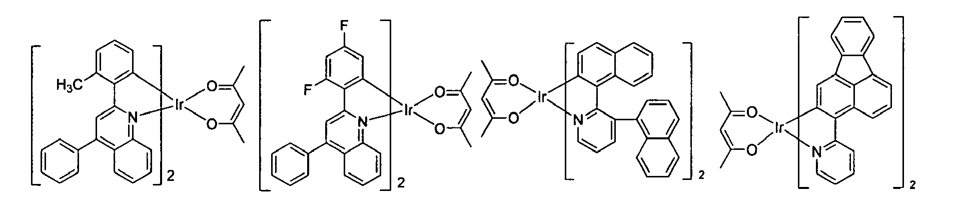

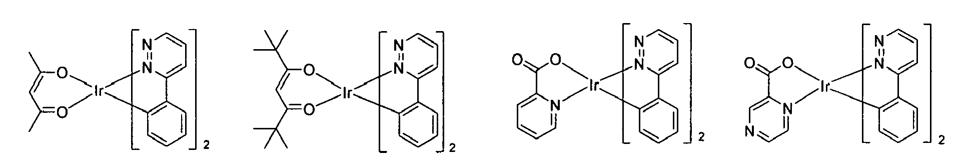

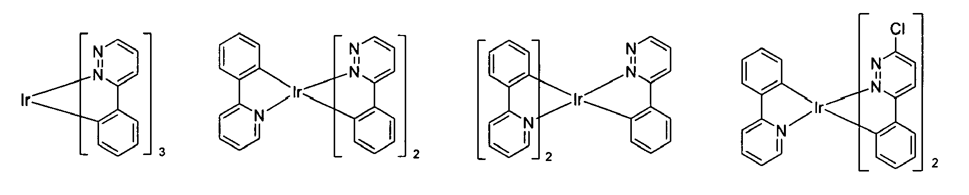

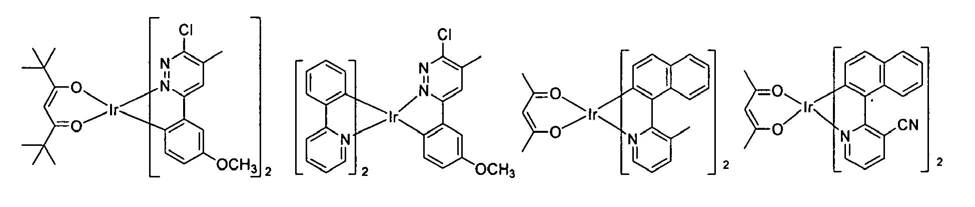

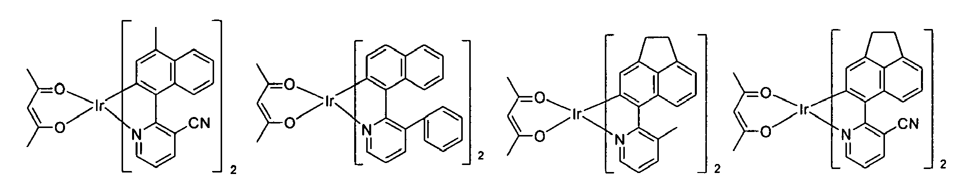

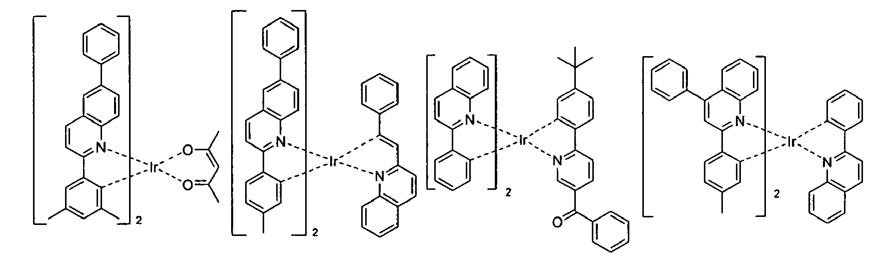

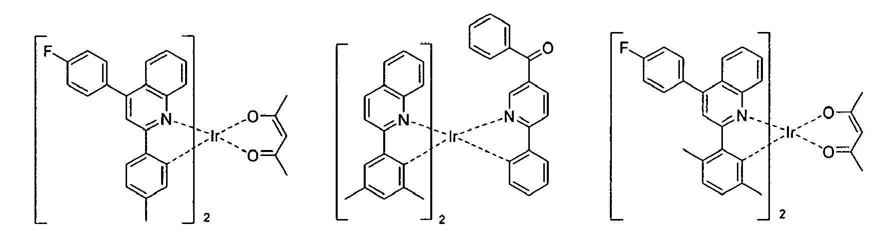

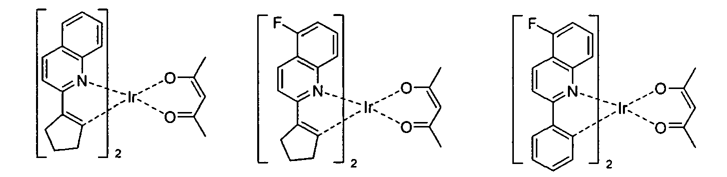

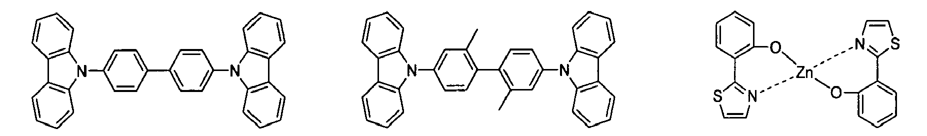

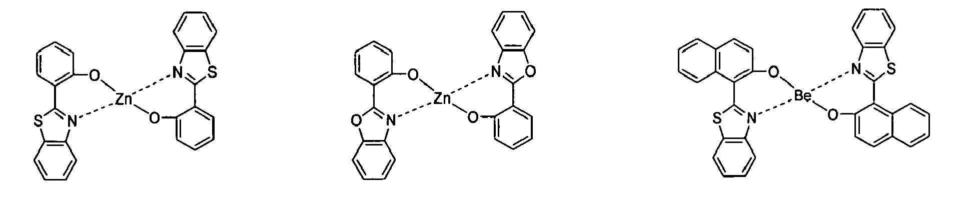

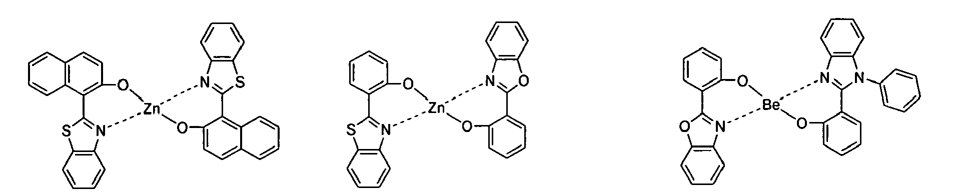

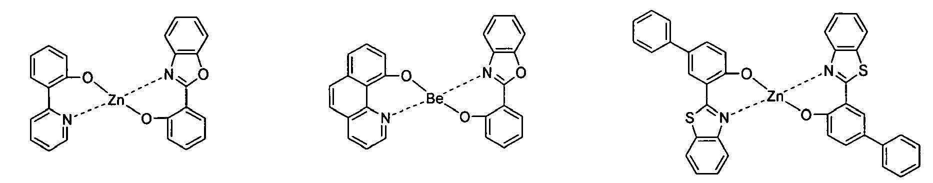

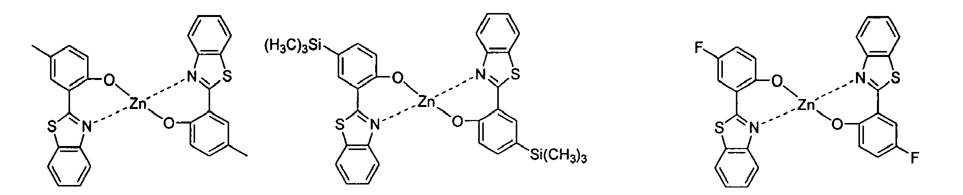

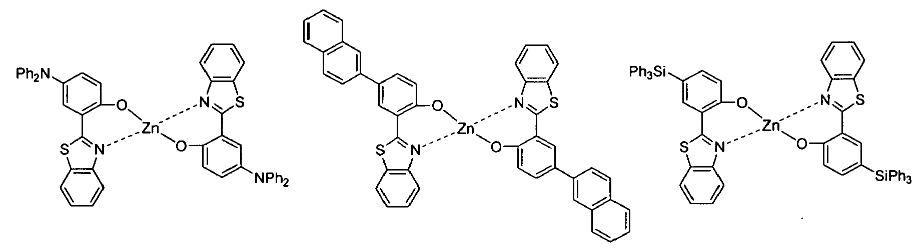

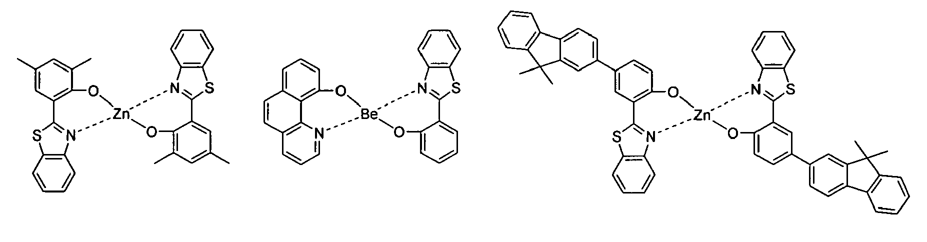

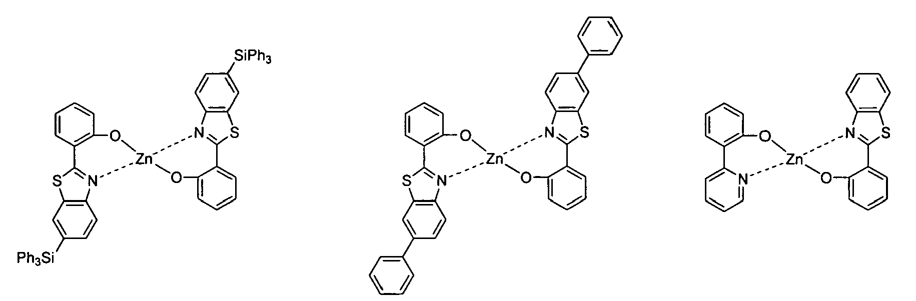

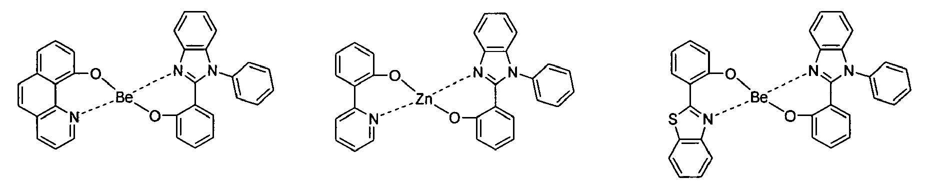

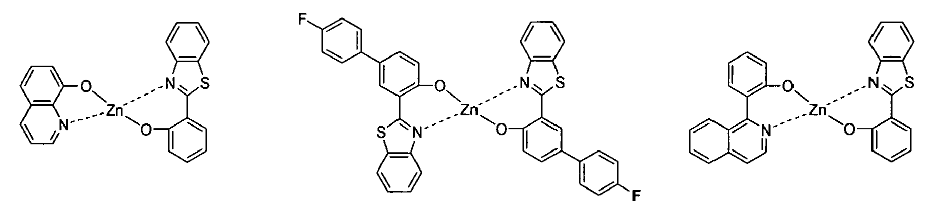

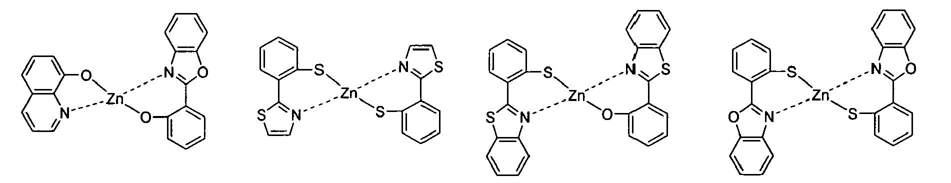

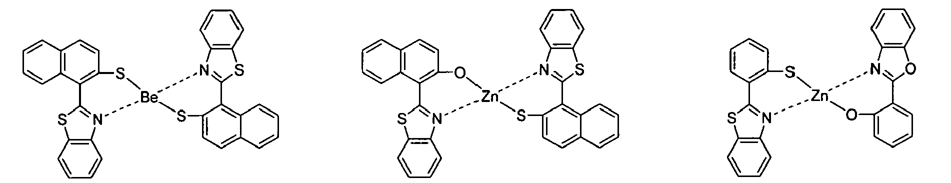

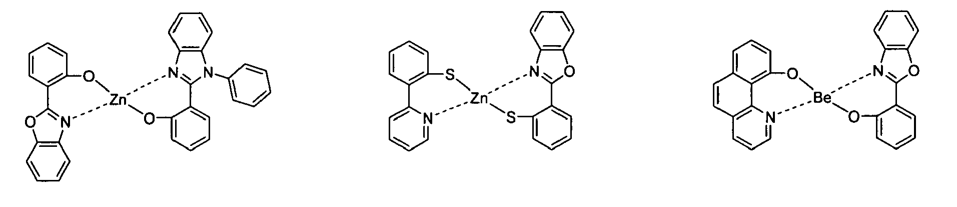

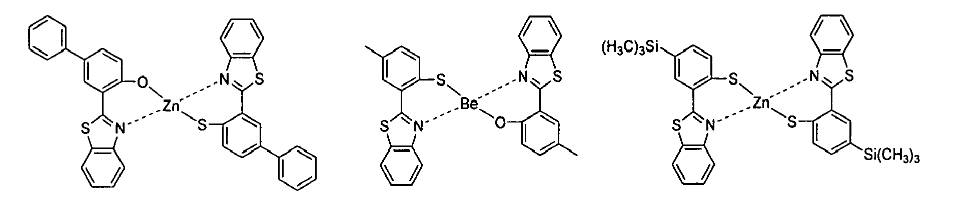

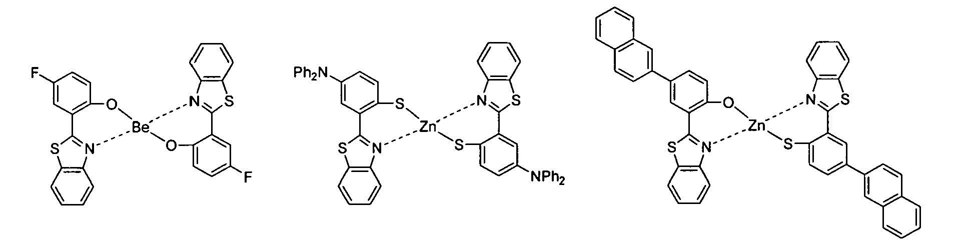

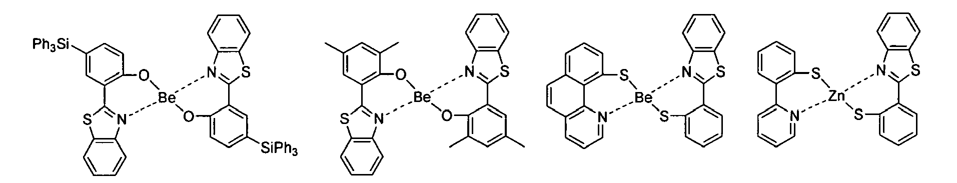

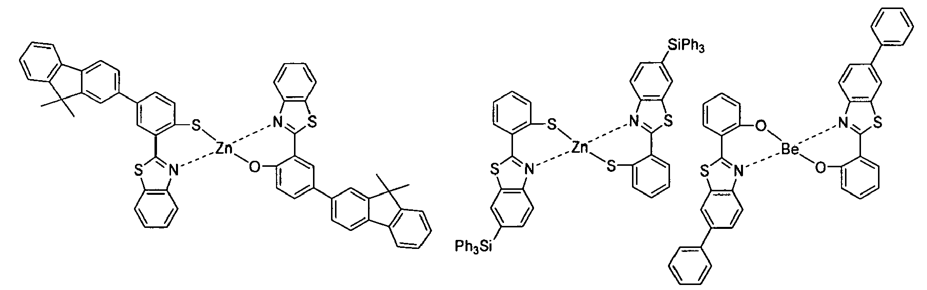

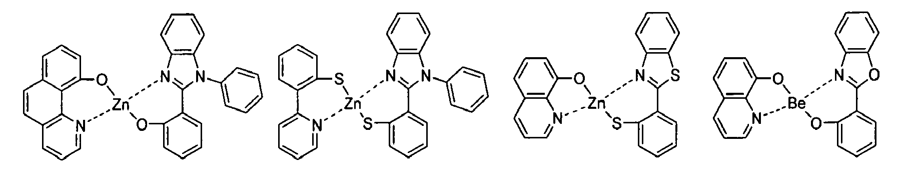

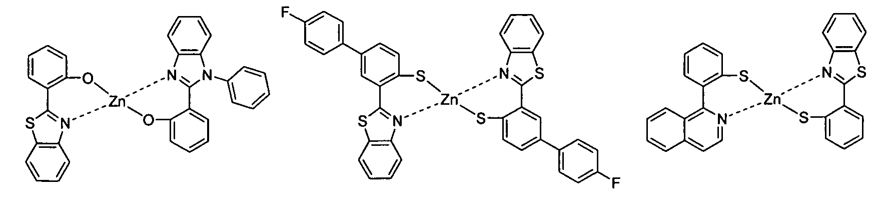

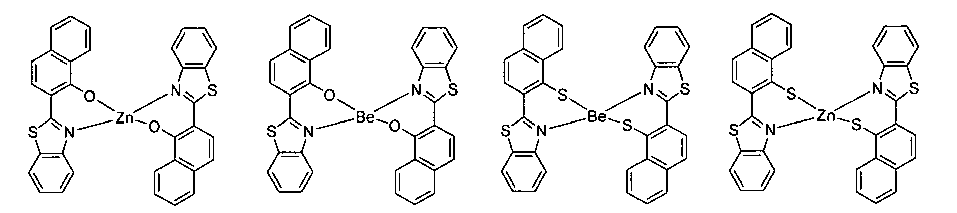

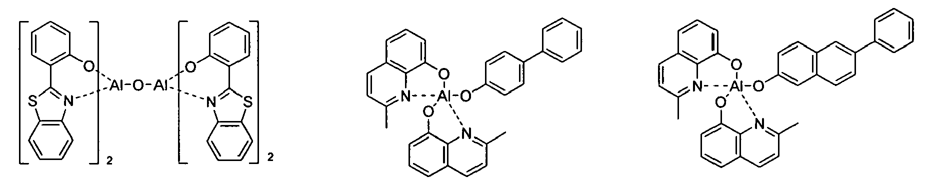

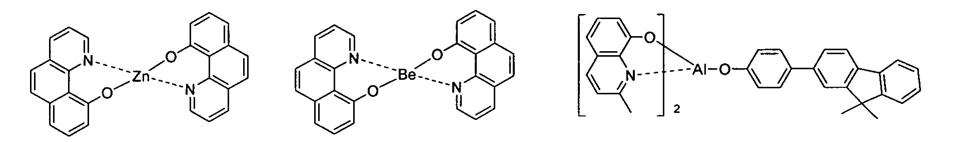

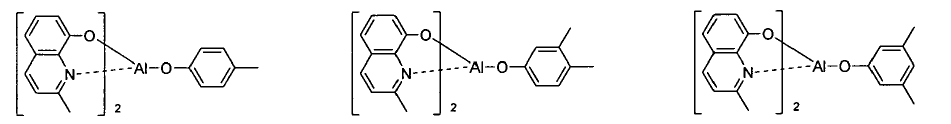

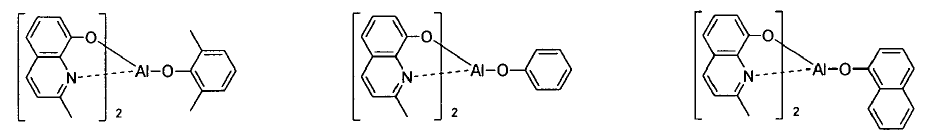

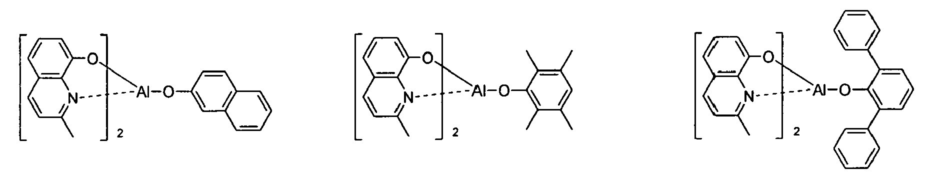

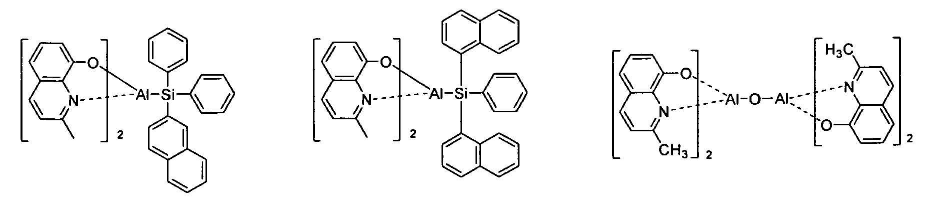

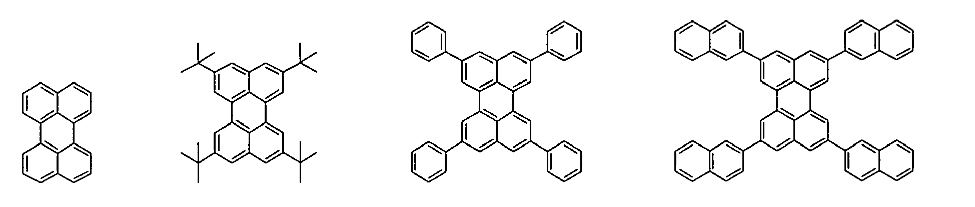

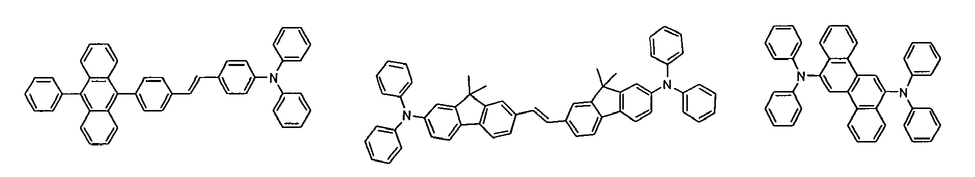

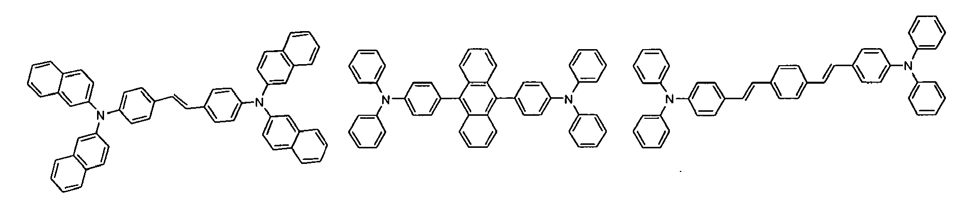

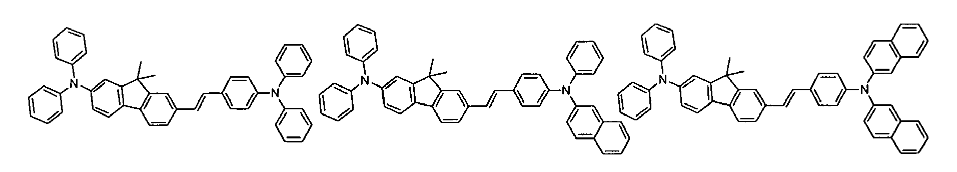

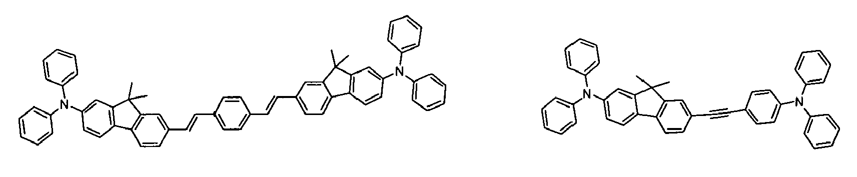

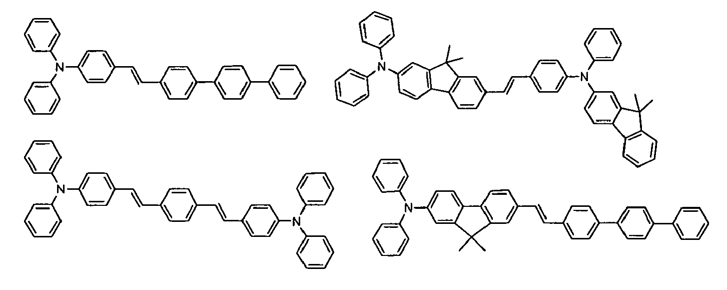

In an organic electroluminescent device according to the present invention, it is preferable to displace one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer side, and a metal halide layer or a metal oxide layer on the cathode surface of the electroluminescent (EL) medium layer side. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include Cs₂O, Li₂O, MgO, SrO, BaO, CaO, or the like.

In an organic electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

Since the organic electroluminescent compounds according to the invention show high luminous efficiency and provide excellent life property of devices manufactured therefrom, OLED's with very good operation lifetime can be manufactured.

### [Best Mode]

The present invention is further described with respect to the compounds according to the invention, the processes for preparing the same, and electroluminescent properties of devices manufactured therefrom by referring to the representative compounds of the invention, which are provided for illustration of the embodiments only but are not intended to limit the scope of the invention by any means.

### [Preparation Examples]

### [Preparation Example 1] Preparation of Compound (1023)

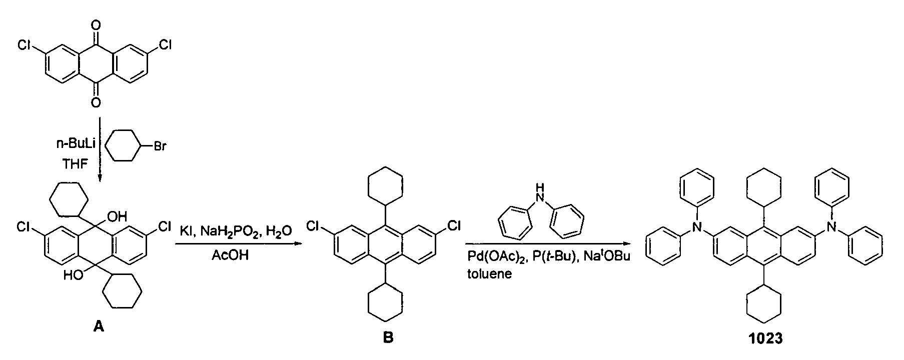

### Preparation of Compound (A)

Bromocyclohexane (44.1 g, 270.7 mmol) was dissolved in dry tetrahydrofuran solvent (500 ml), and 2.5 M n-butyllithium (in n-hexane) (130 mL, 324.9 mmol) was added thereto at -78°C. After stirring for 1 hour, 2,7-dichloroanthracene-9,10-dione (30.0 g, 108.3 mmol) was added thereto, and the resultant mixture was stirred while slowly raising the temperature to room temperature. After 17 hours, water was added, and the mixture was stirred for 30 minutes and extracted with 500 mL of ethyl acetate. The extract was washed with 500 mL of water, and the organic layer was dried over magnesium sulfate, and evaporated under reduced pressure. The residue was dried to obtain Compound (A) (21.3 g, 47.8 mmol).

### Preparation of Compound (B)

A reaction vessel was charged with Compound (A) (21.3 g, 47.8 mmol), potassium iodide (31.7 g, 191.2 mmol), sodium hydrophosphite (40.5 g, 382.4 mmol) and acetic acid (300 mL), and the mixture was stirred under reflux at 120°C. After 15 hours, water (500 mL) was added thereto, and the resultant mixture was stirred for 1 hour. The precipitate obtained from filtration under reduced pressure was washed three times with water (300 mL) and once with acetone (300 mL), and dried to obtain Compound (B) (12.5 g, 30.4 mmol).

### Preparation of Compound (1023)

A reaction vessel was charged with Compound (B) (12.5 g, 30.4 mmol), diphenylamine (15.4 g, 91.2 mmol), palladium acetate (0.34 g, 1.52 mmol), tributylphosphine (0.6 g, 3.0 mmol), sodium t-butoxide (9.3 g, 97.3 mmol) and toluene (250 mL), and the mixture was stirred under reflux in the presence of nitrogen atmosphere. After 8 hours, the mixture was cooled to room temperature, and extracted with ethyl acetate (400 mL), and the extract was dried under reduced pressure. Purification via column chromatography (dichloromethane:n-hexane = 5:1) gave the target Compound (1023) (9.2 g, 44%).

According to the same procedure as Preparation Example 1, organic electroluminescent compounds (Compound 1 through Compound 5137) in Table 1 were prepared, of which the ¹H NMR and MS/FAB data are listed in Table 2.

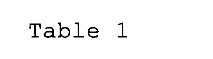

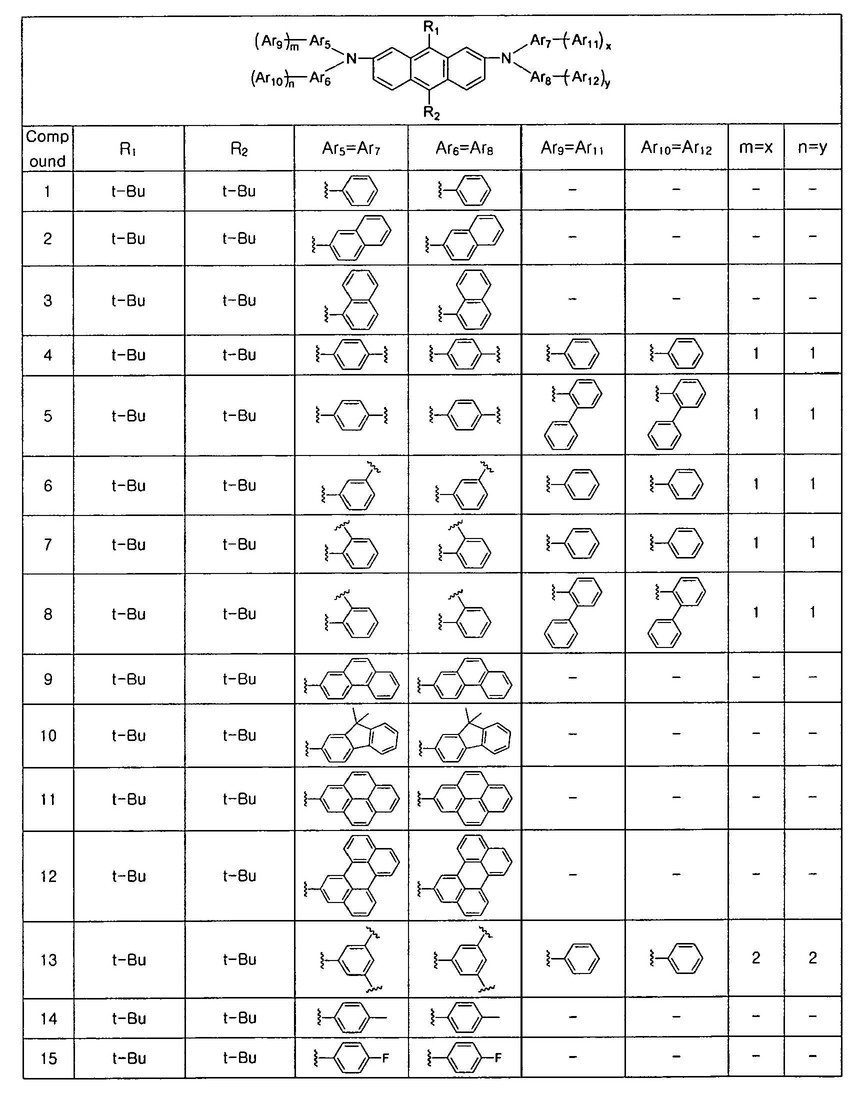

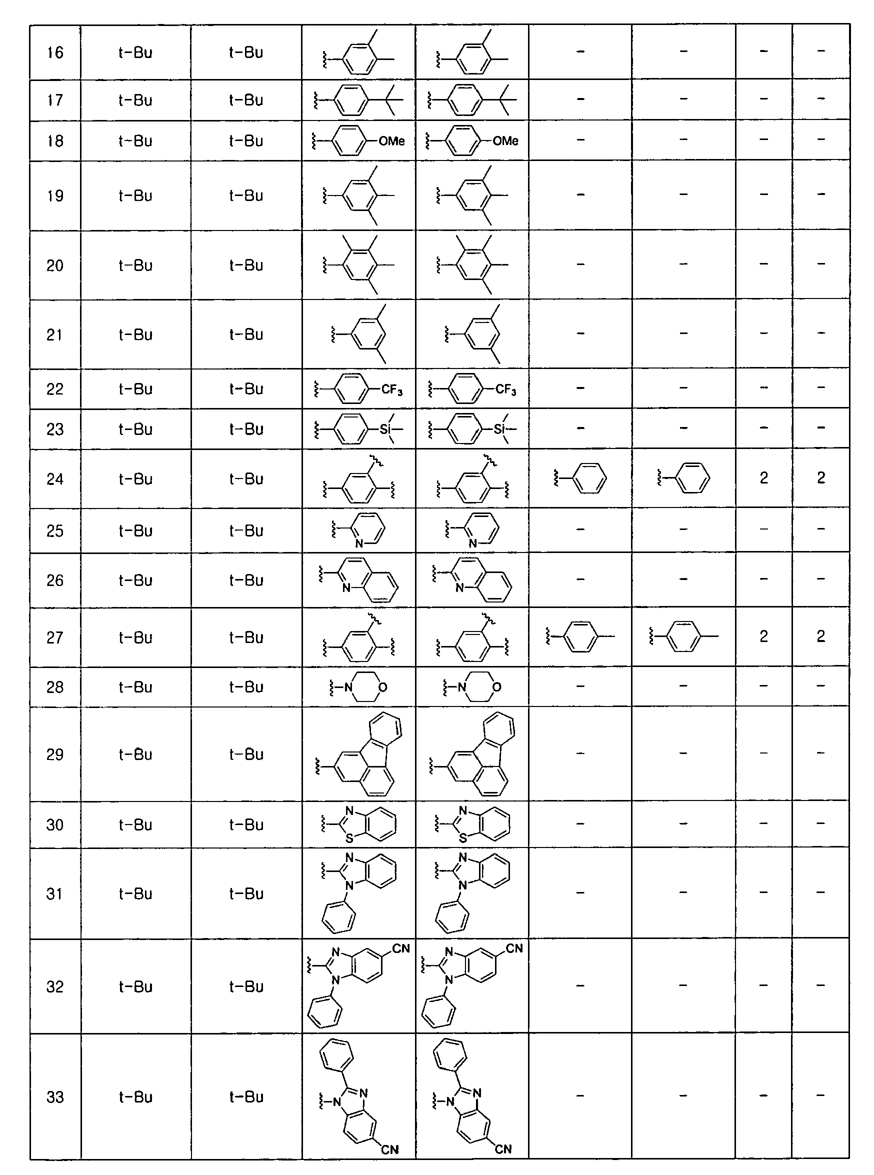

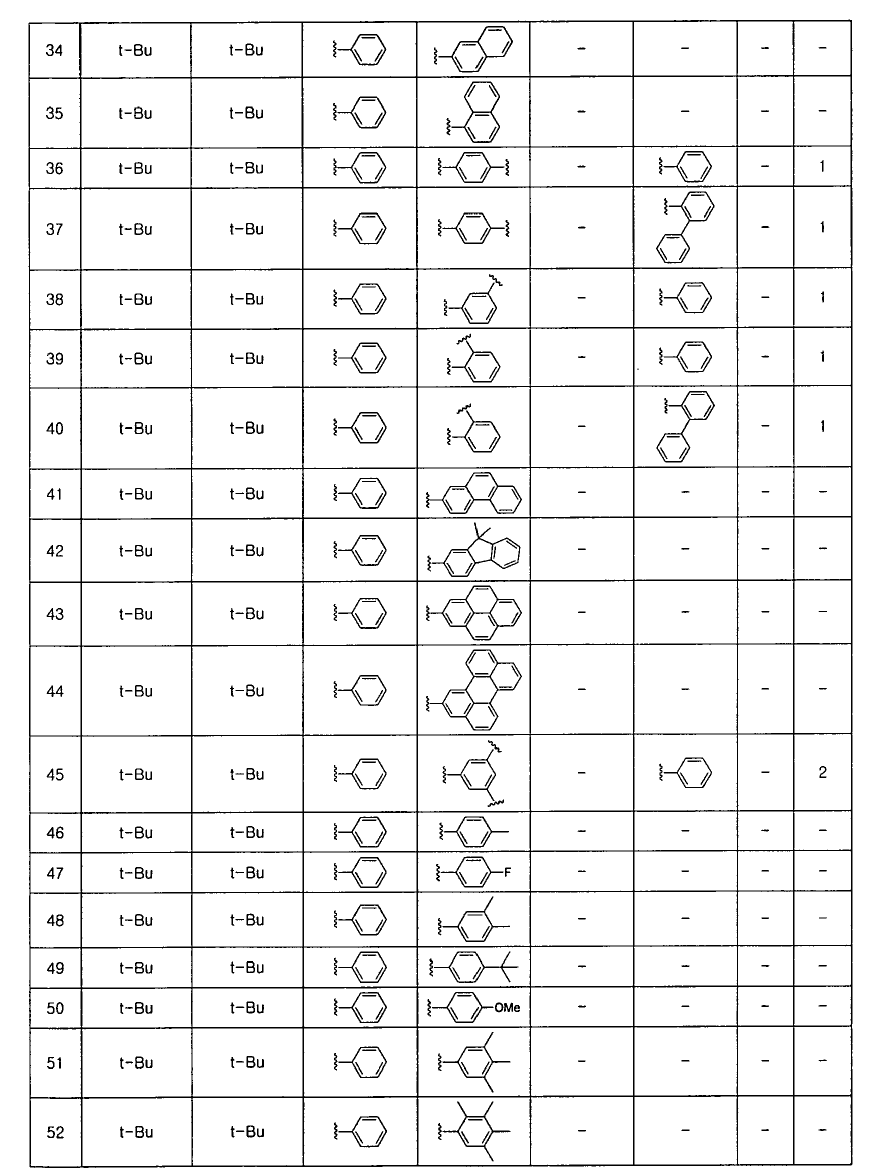

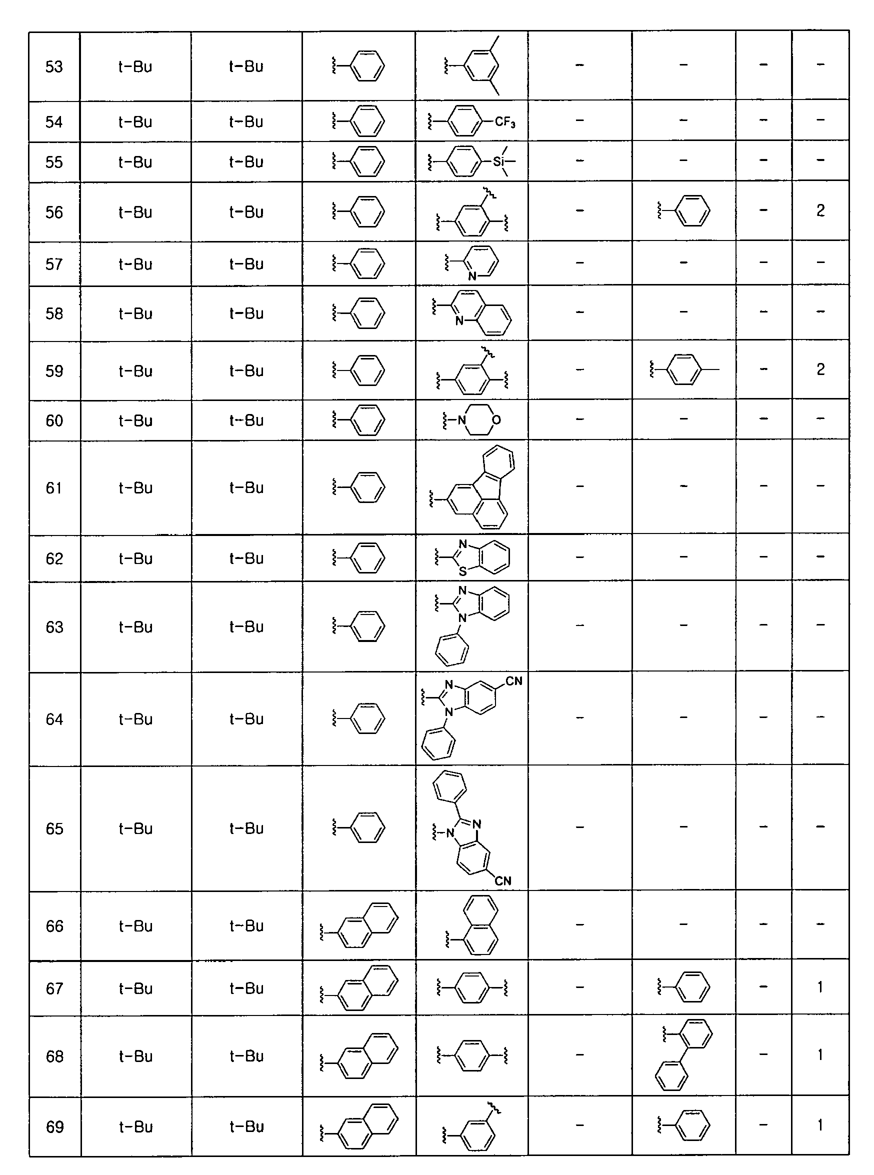

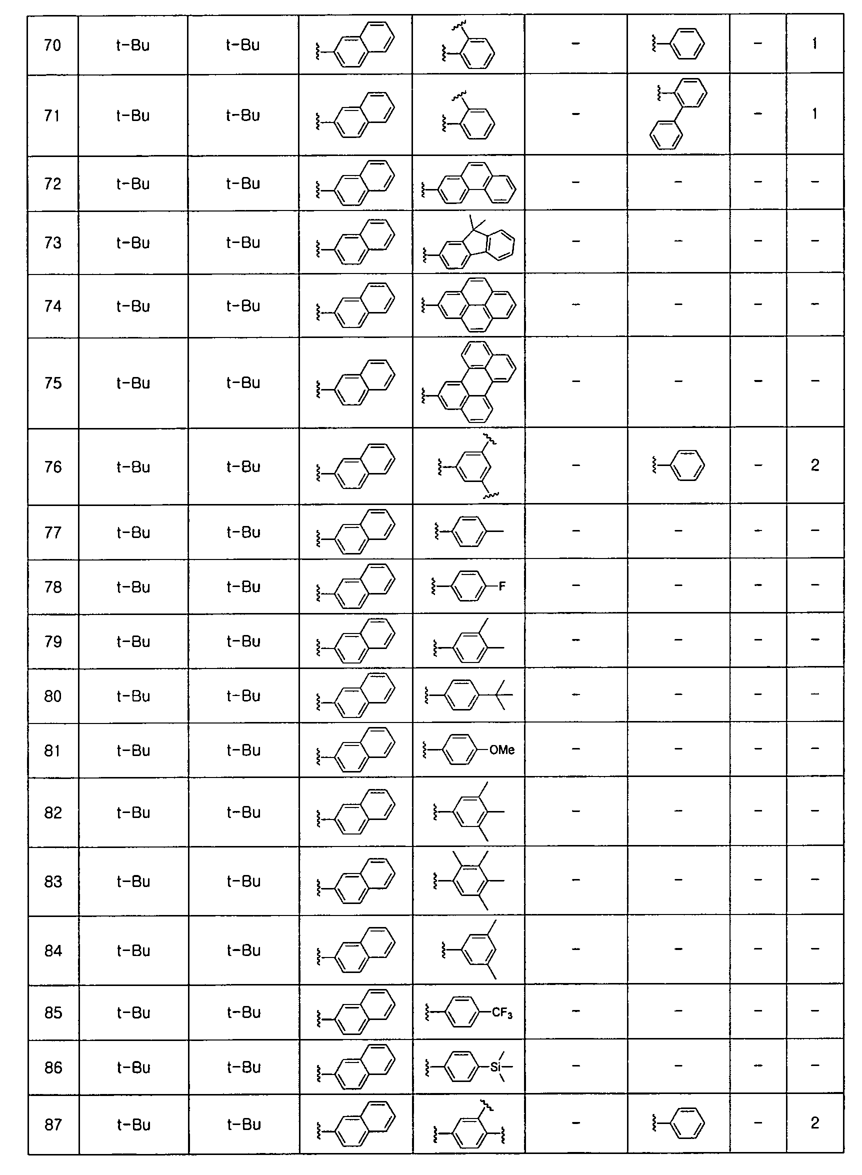

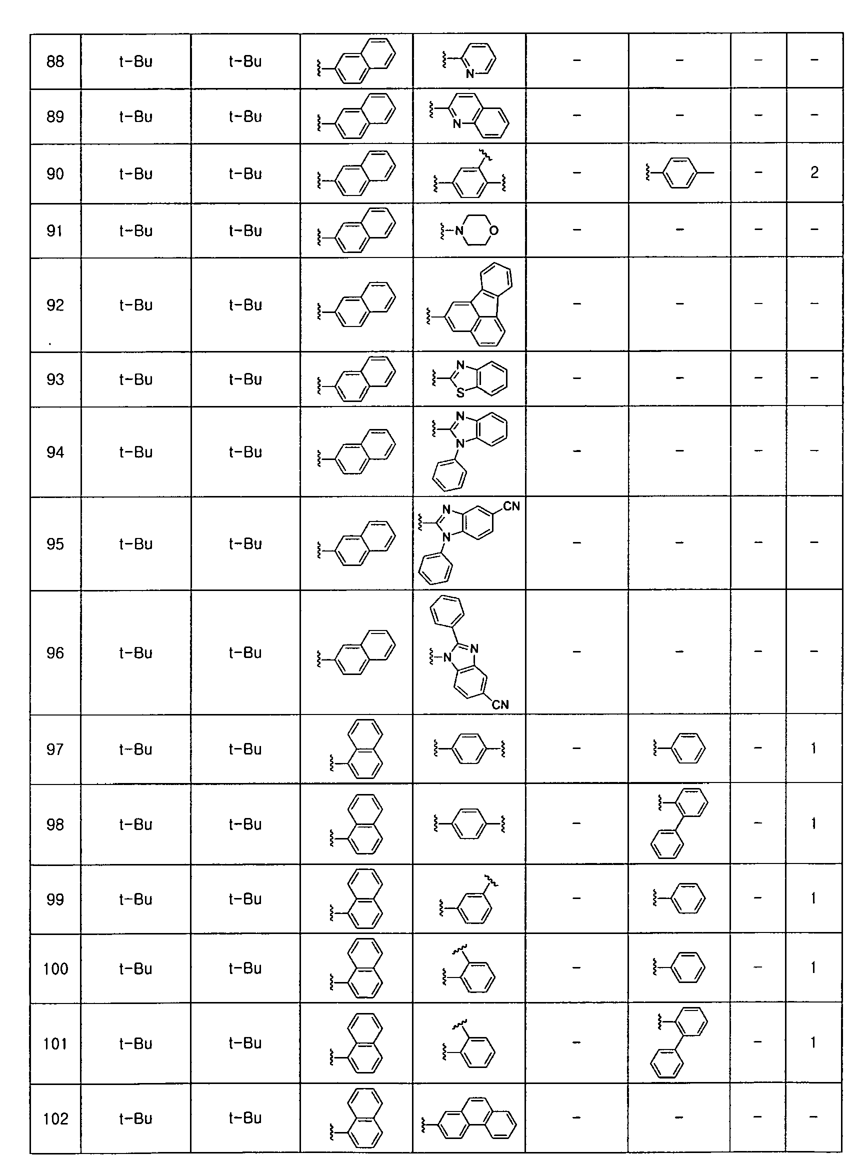

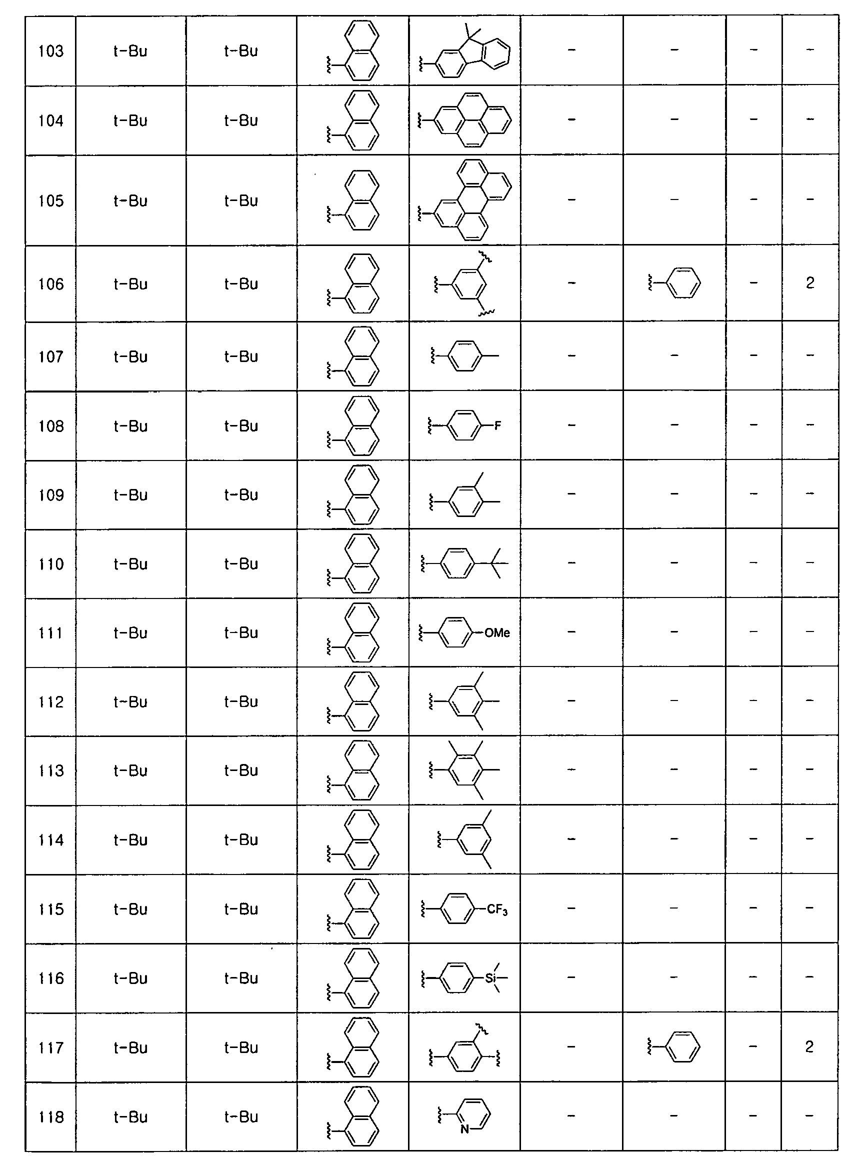

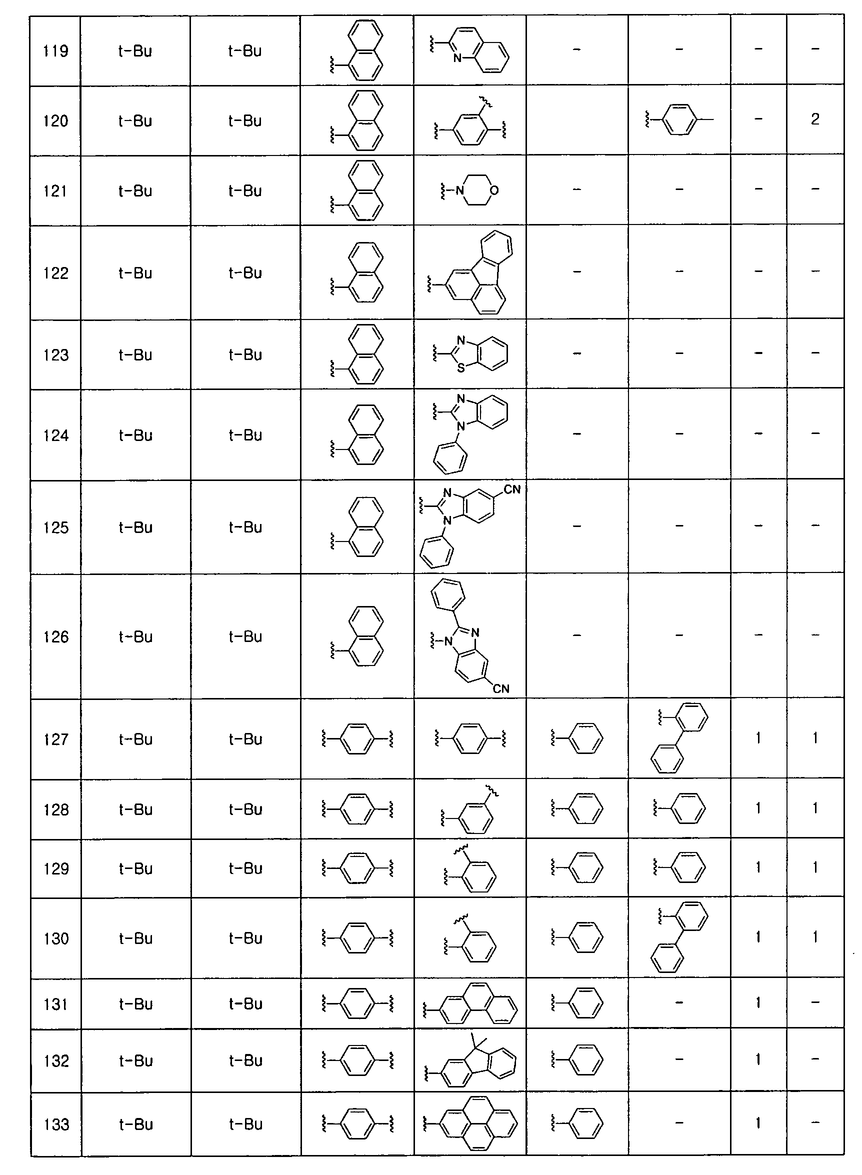

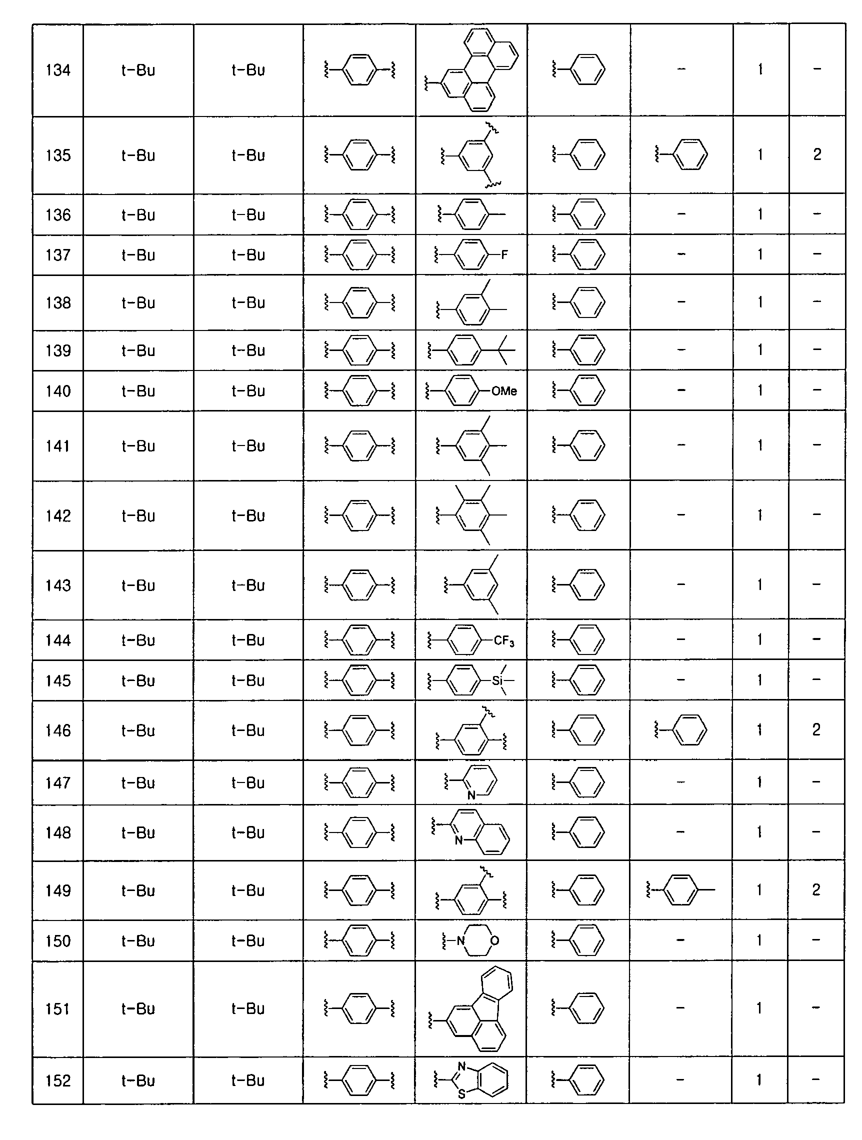

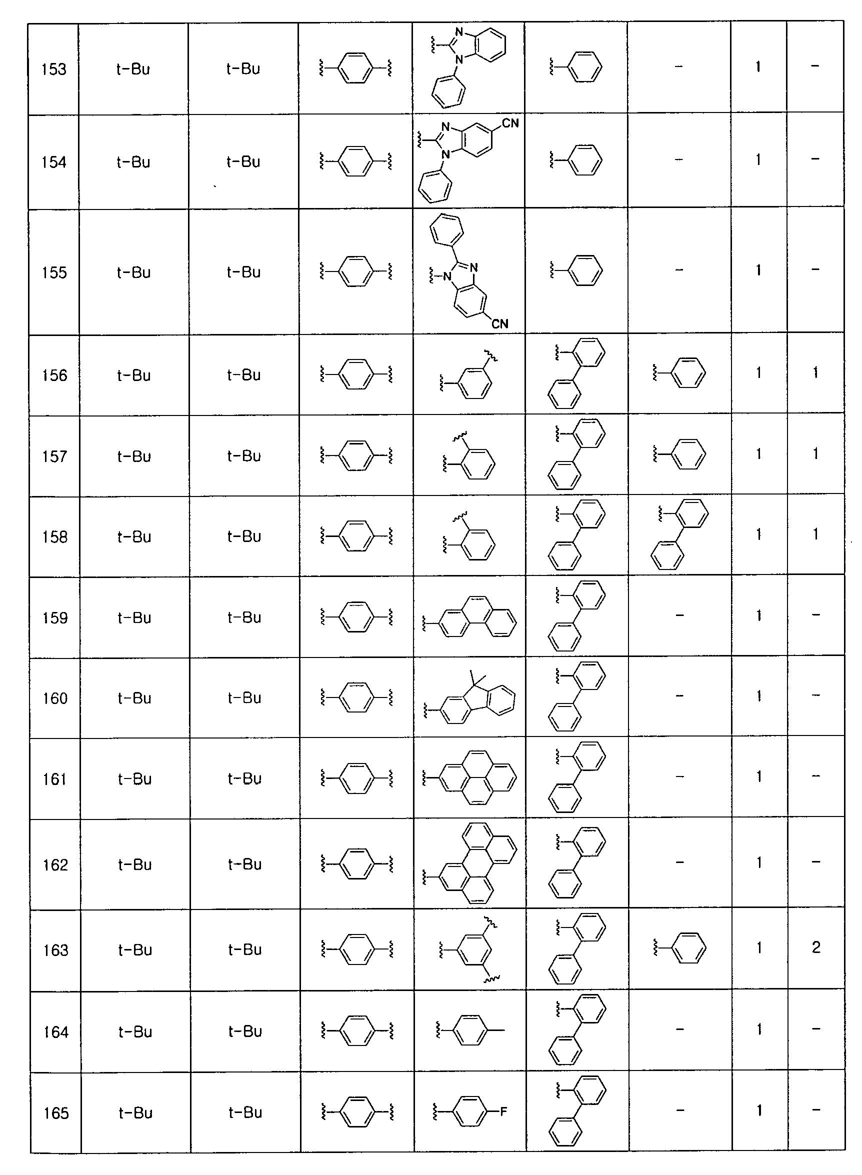

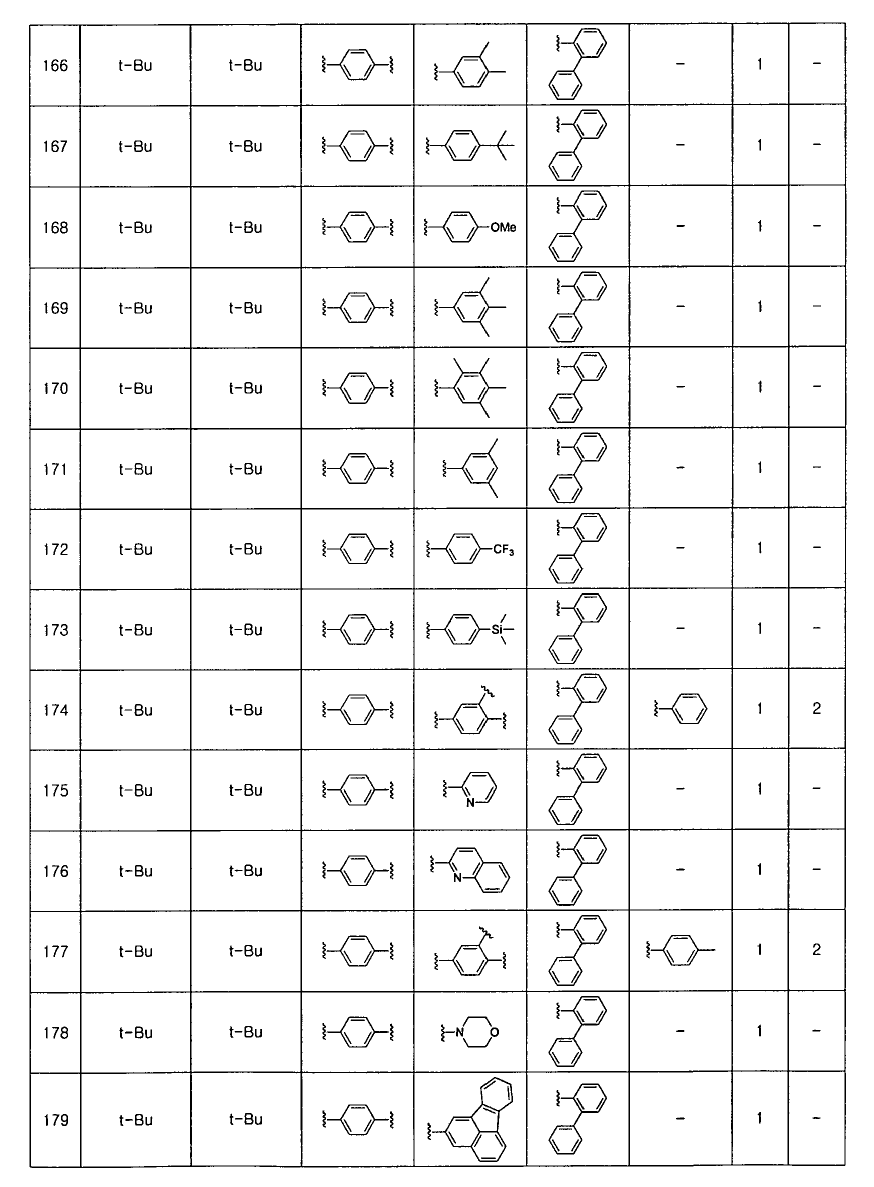

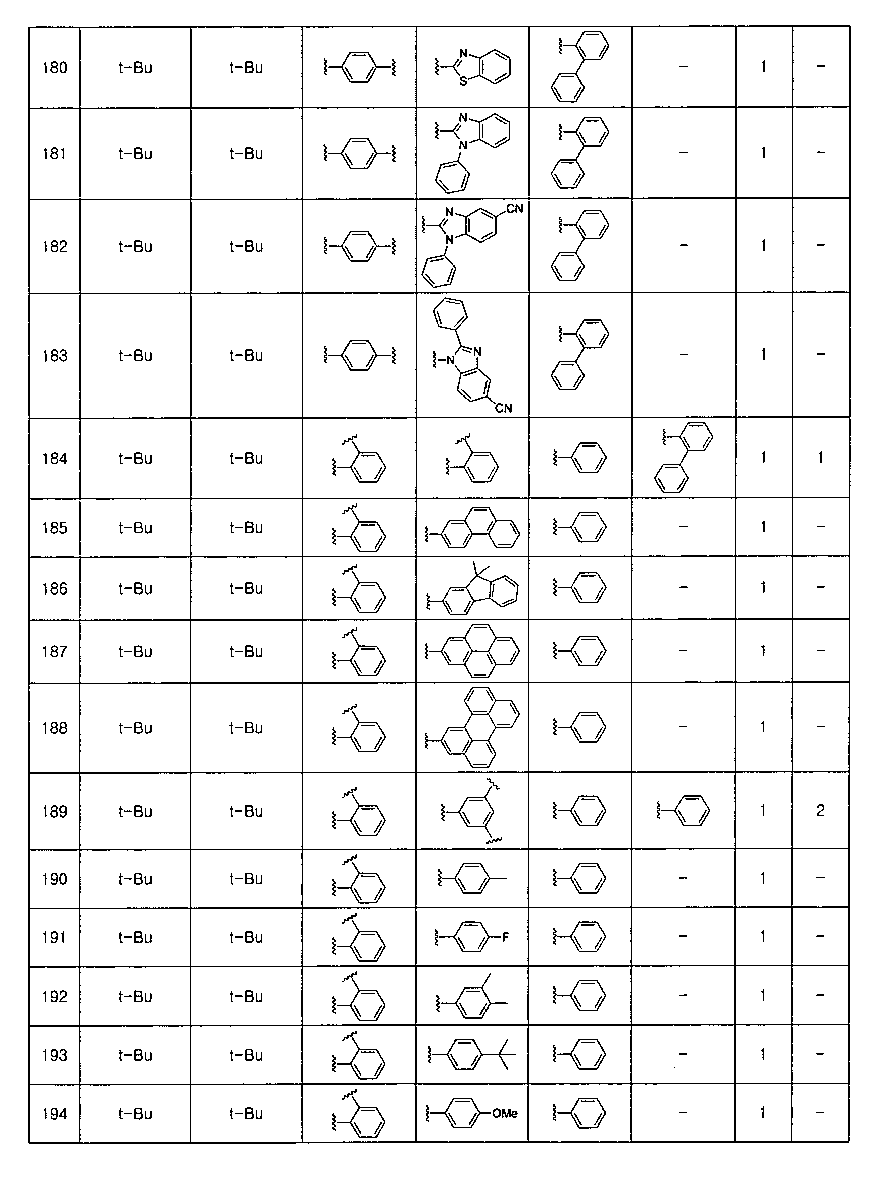

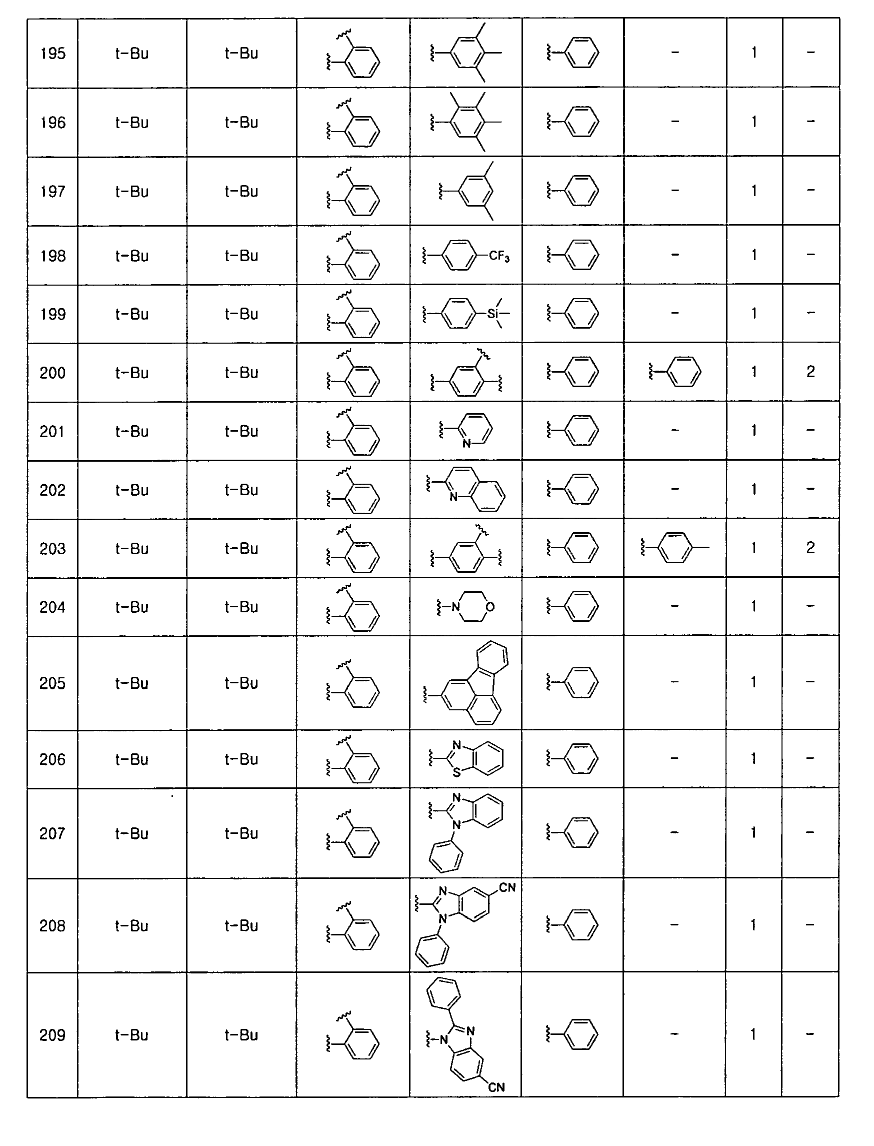

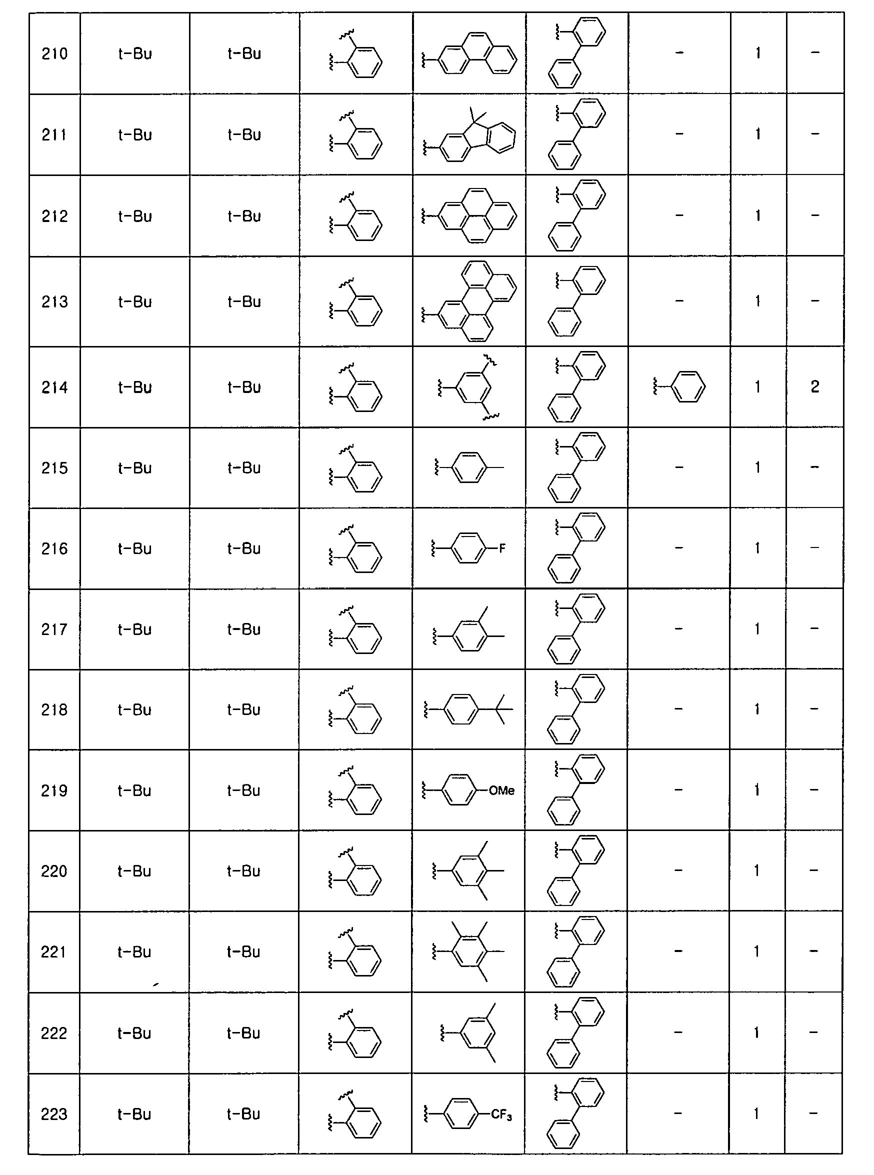

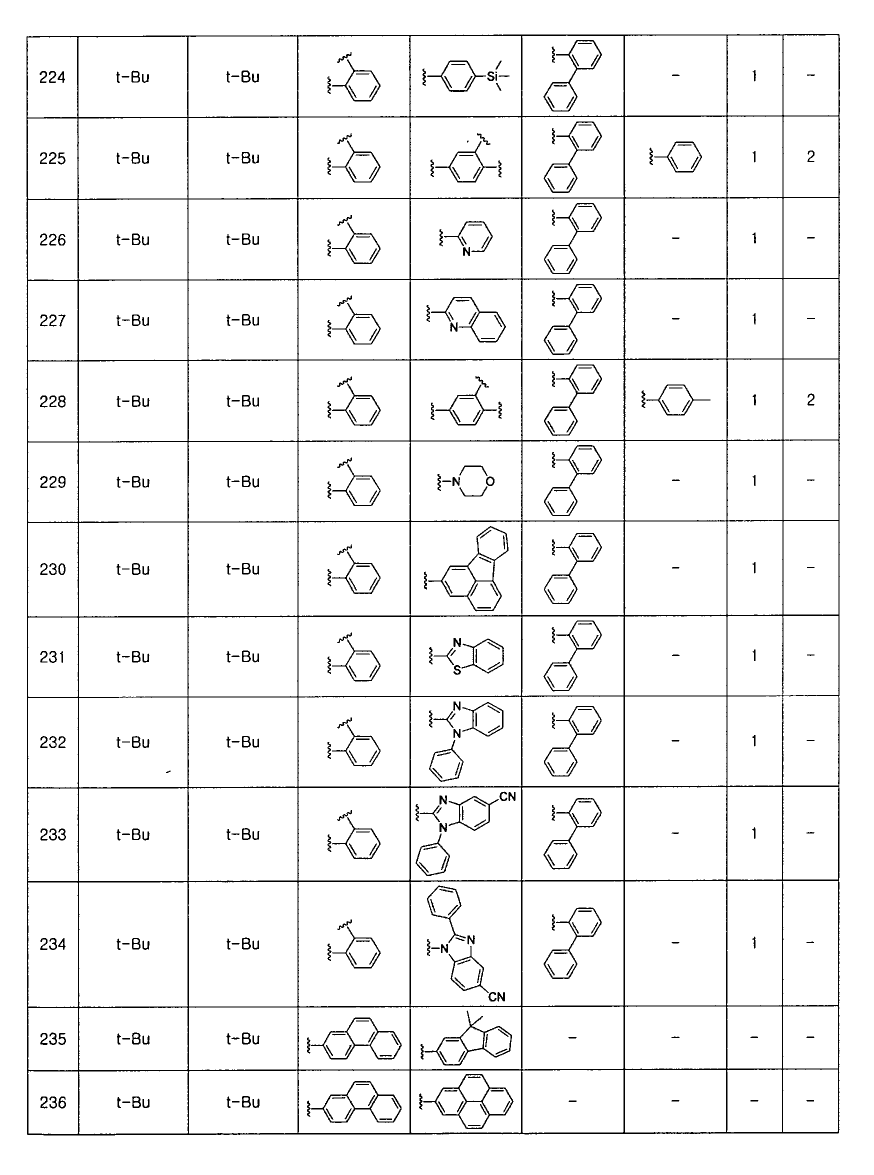

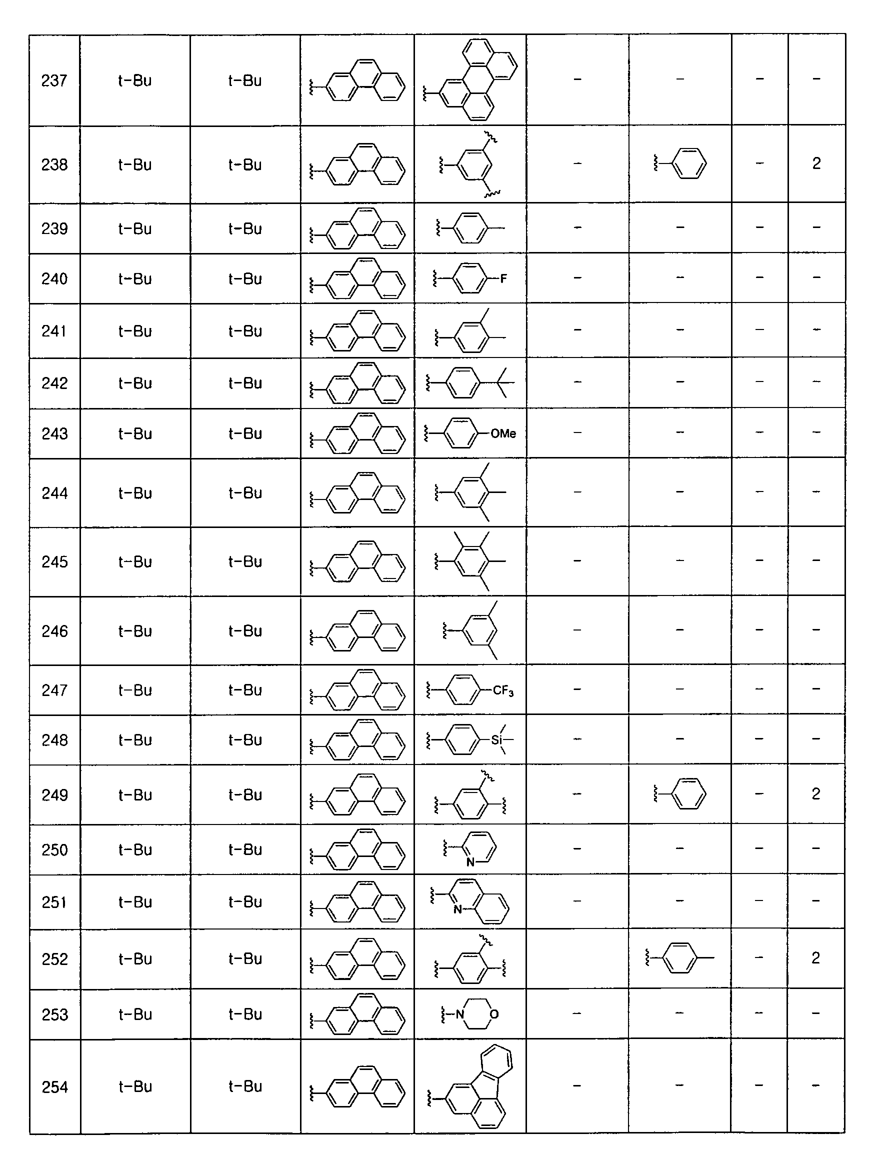

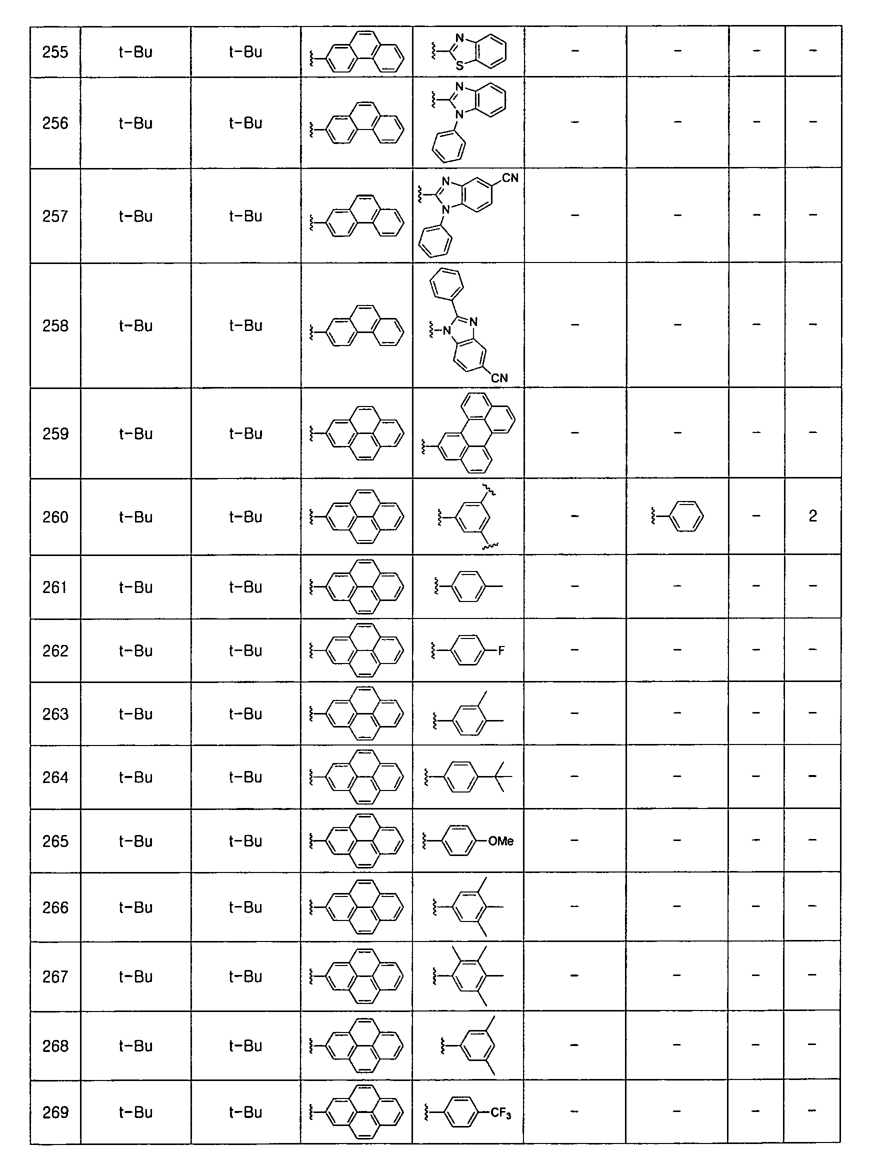

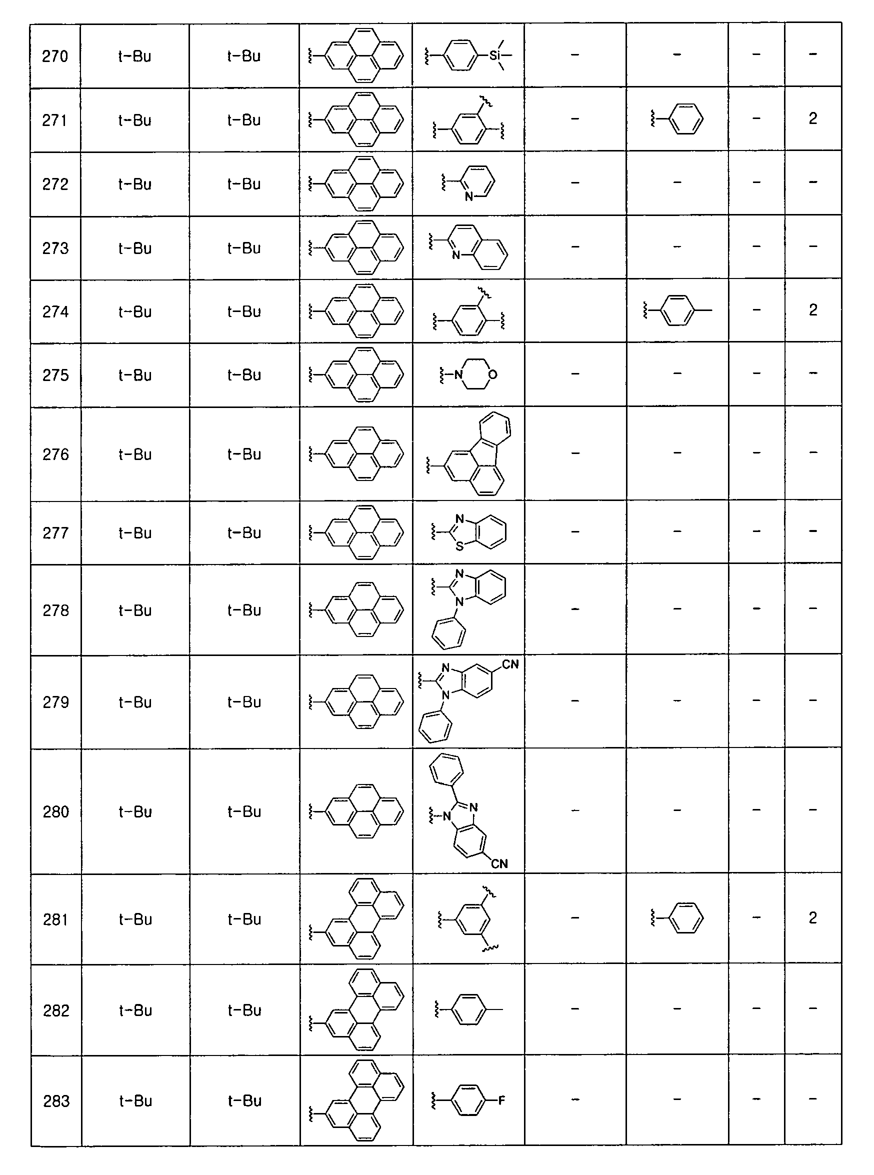

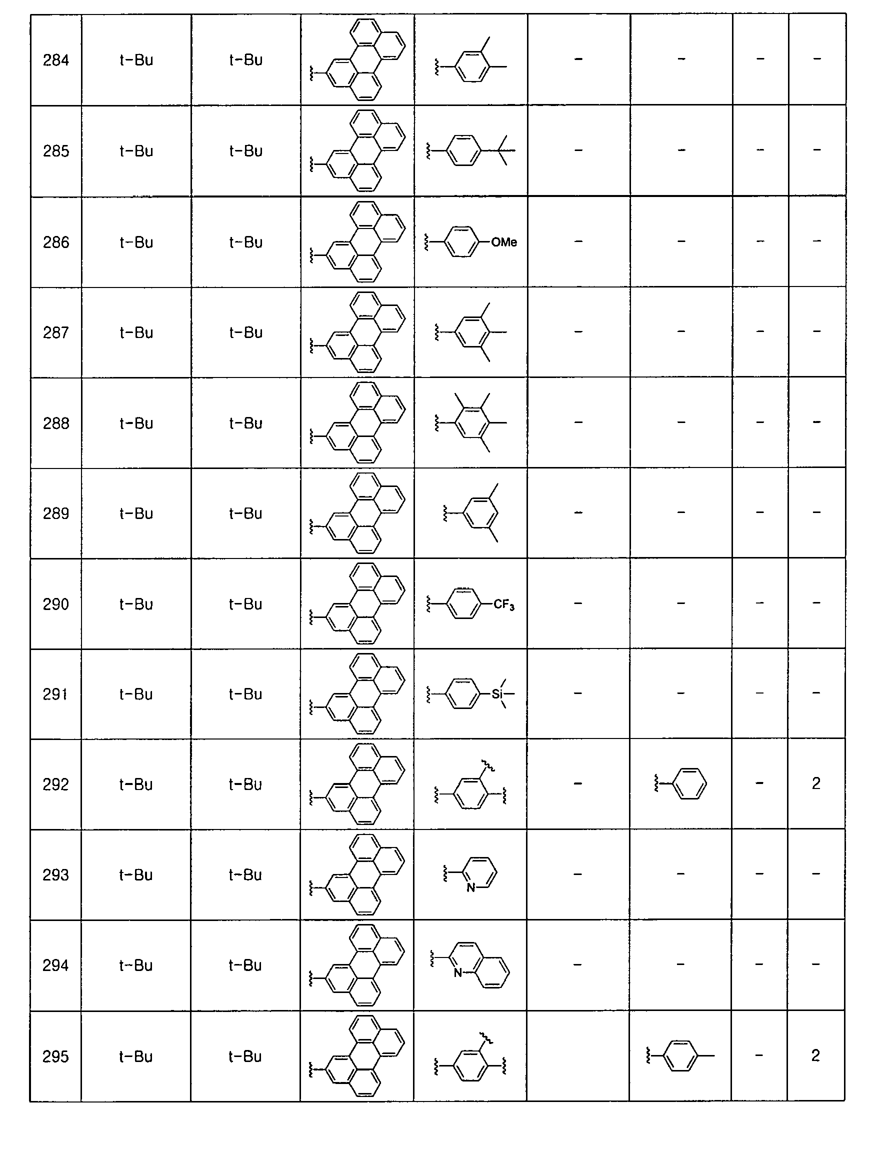

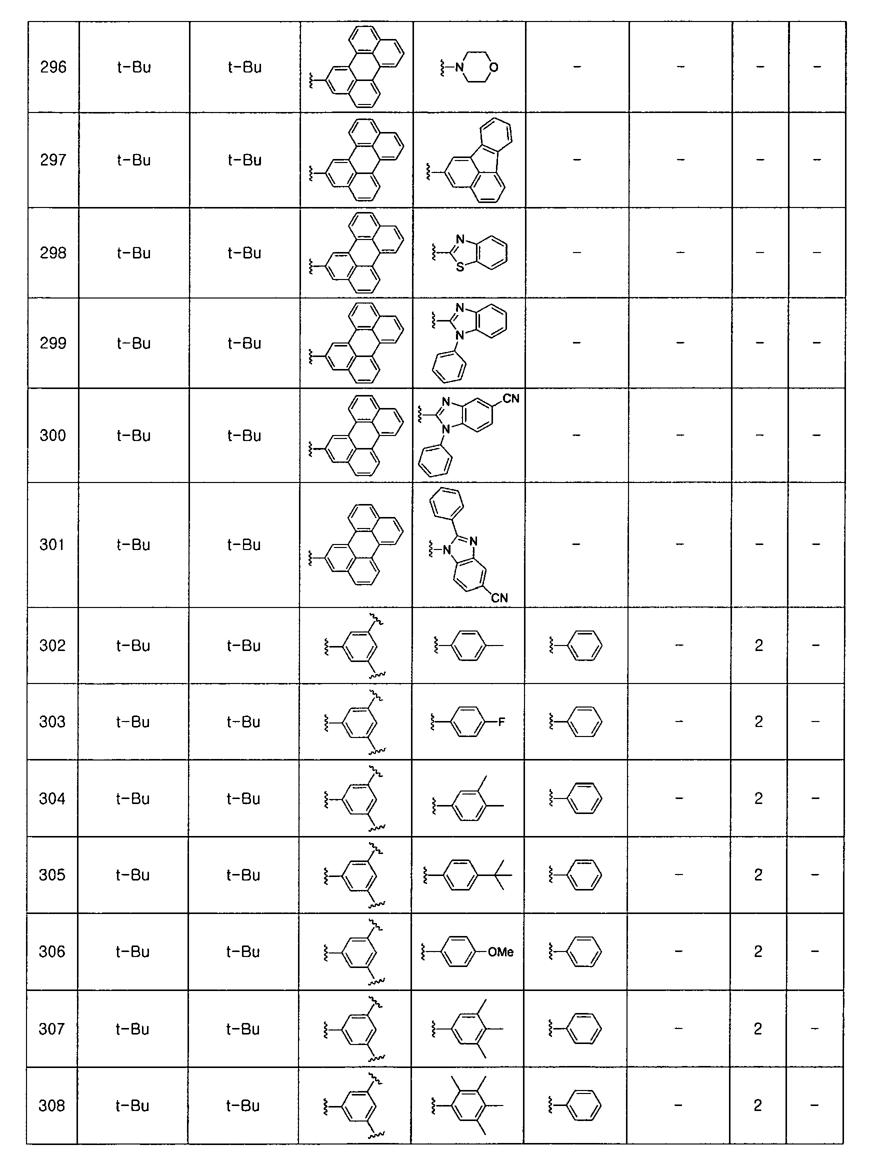

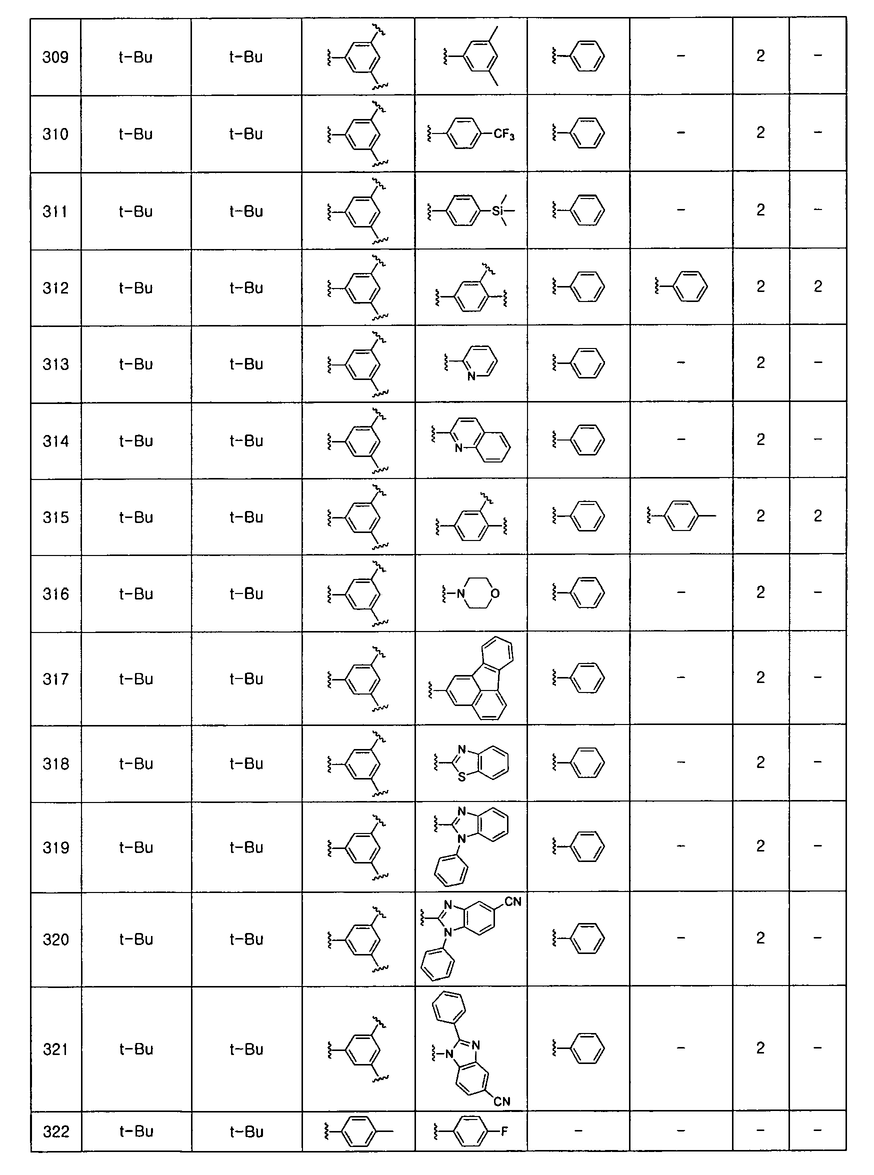

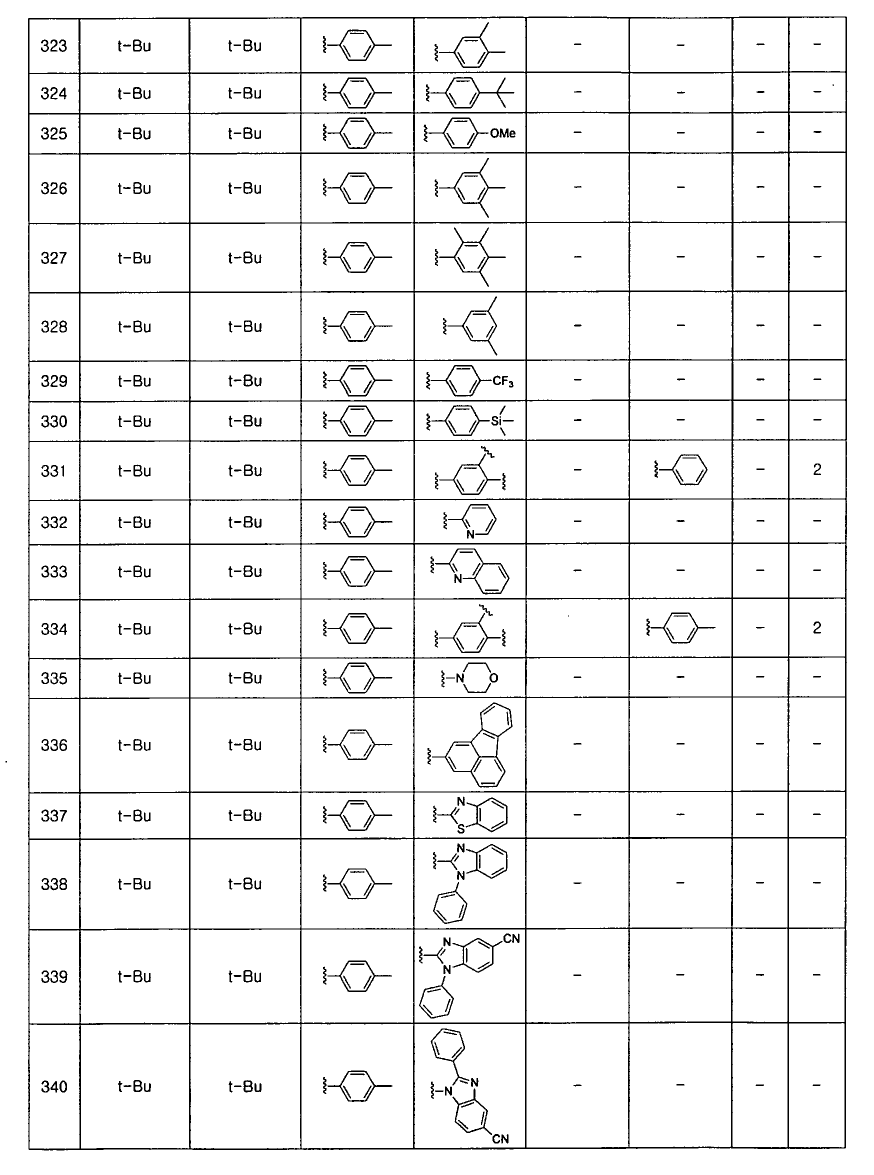

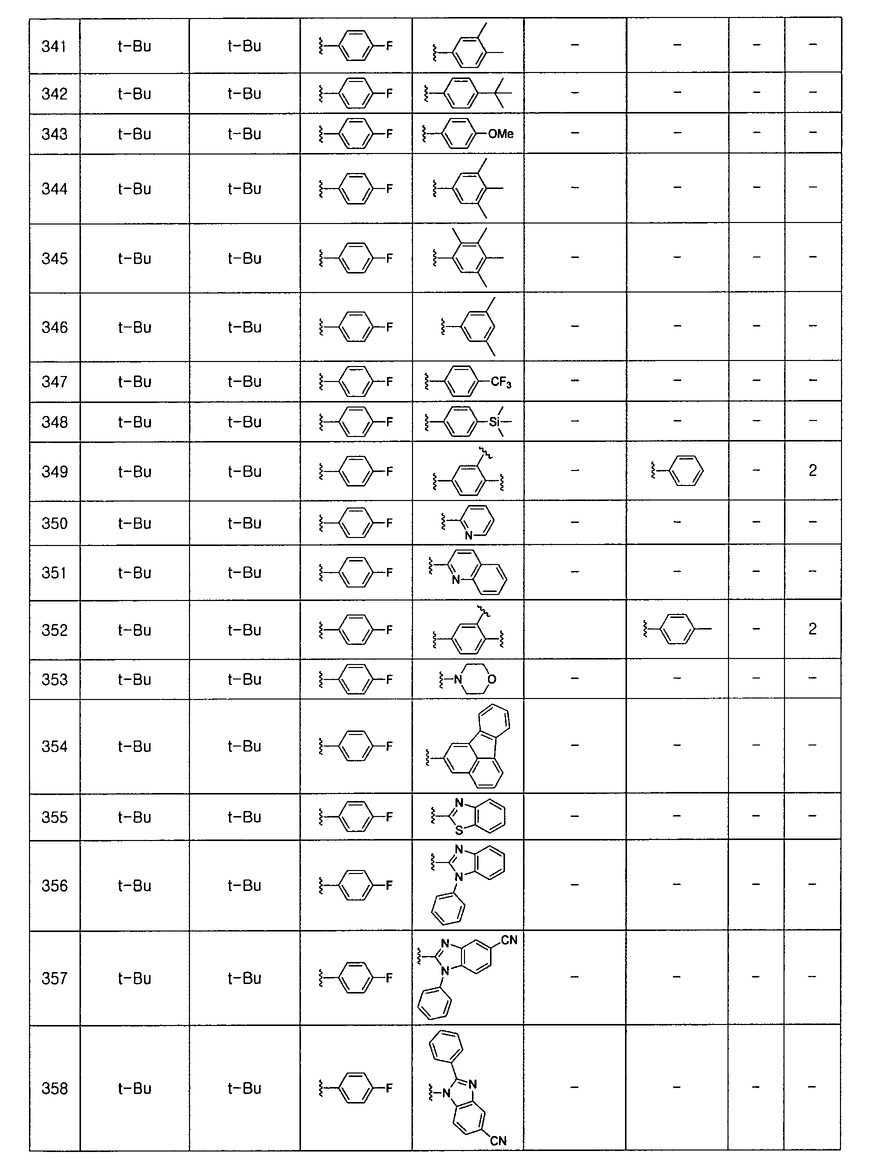

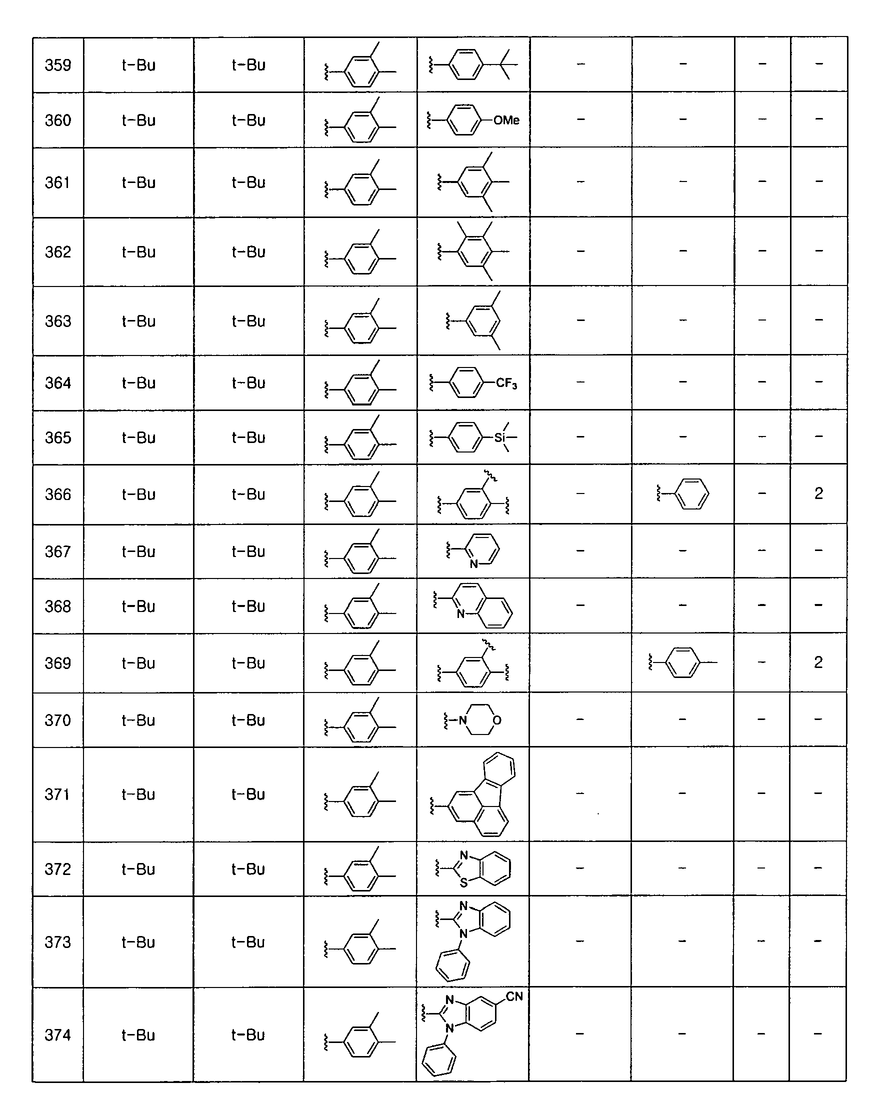

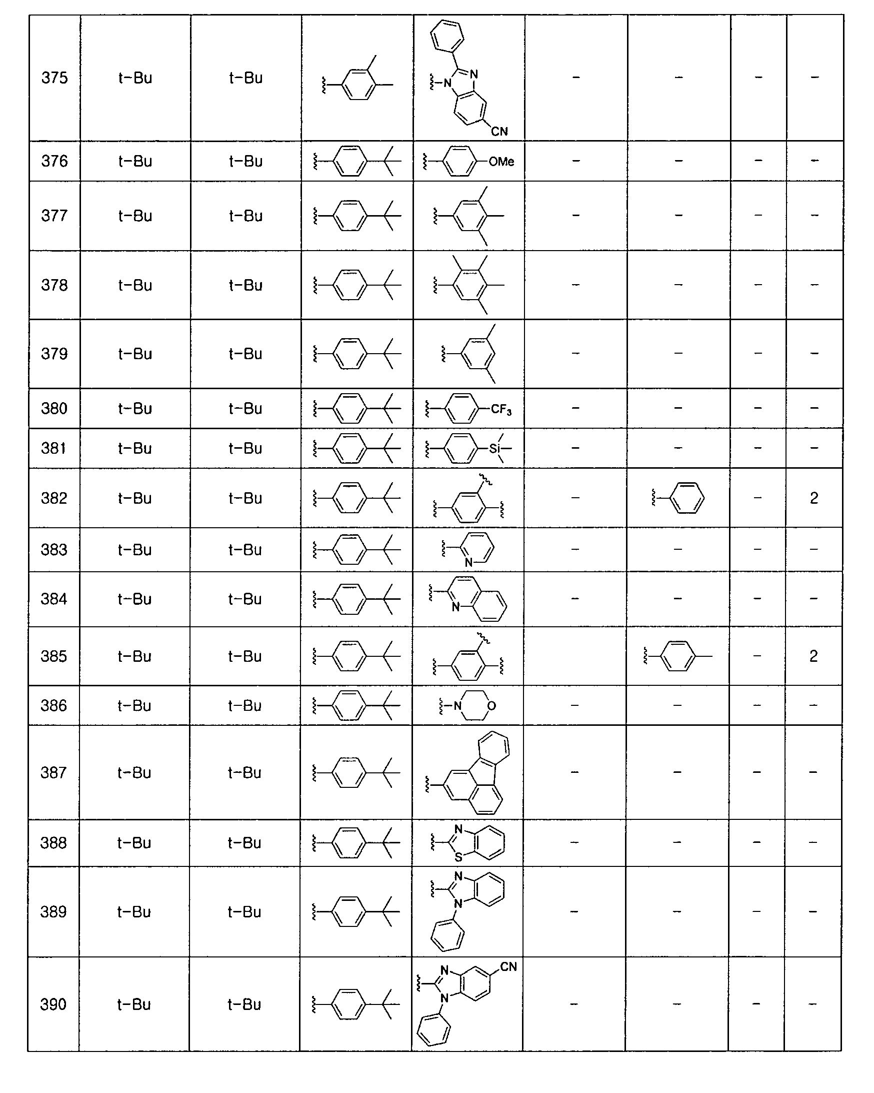

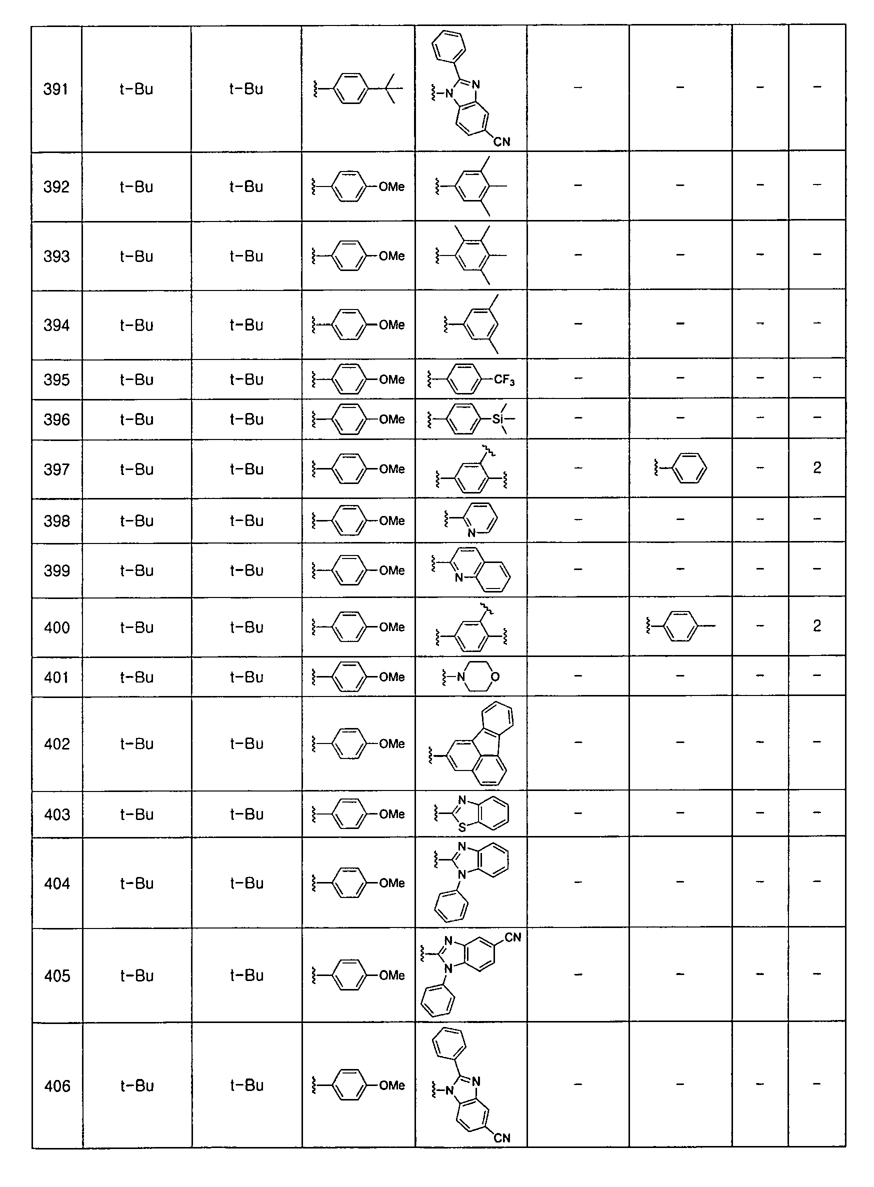

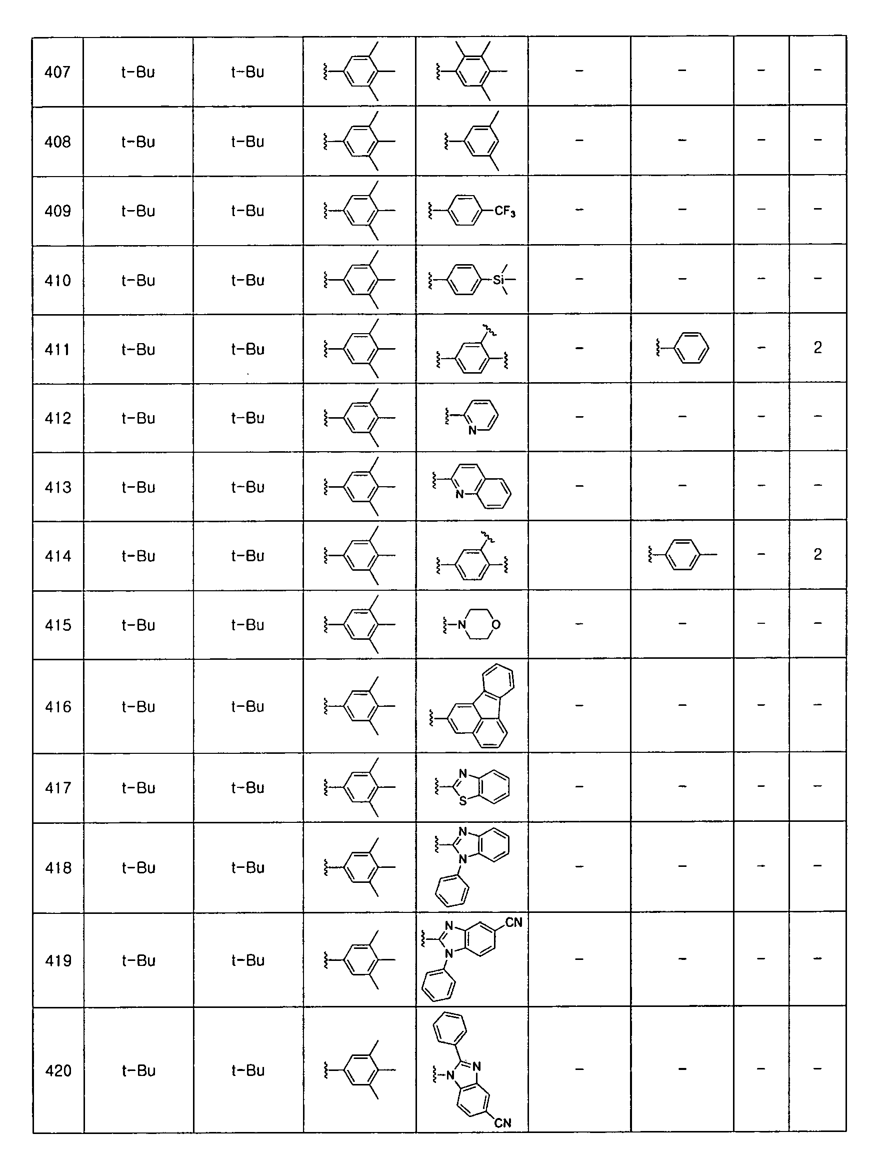

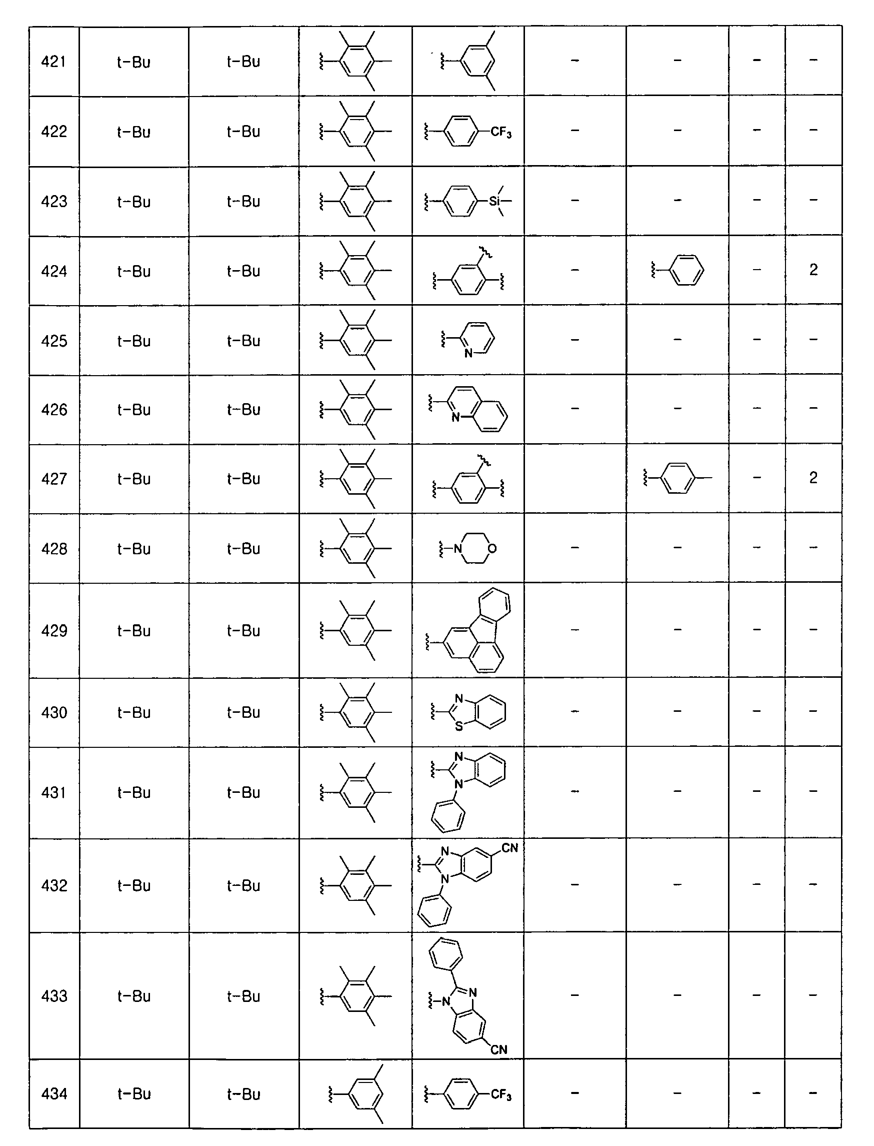

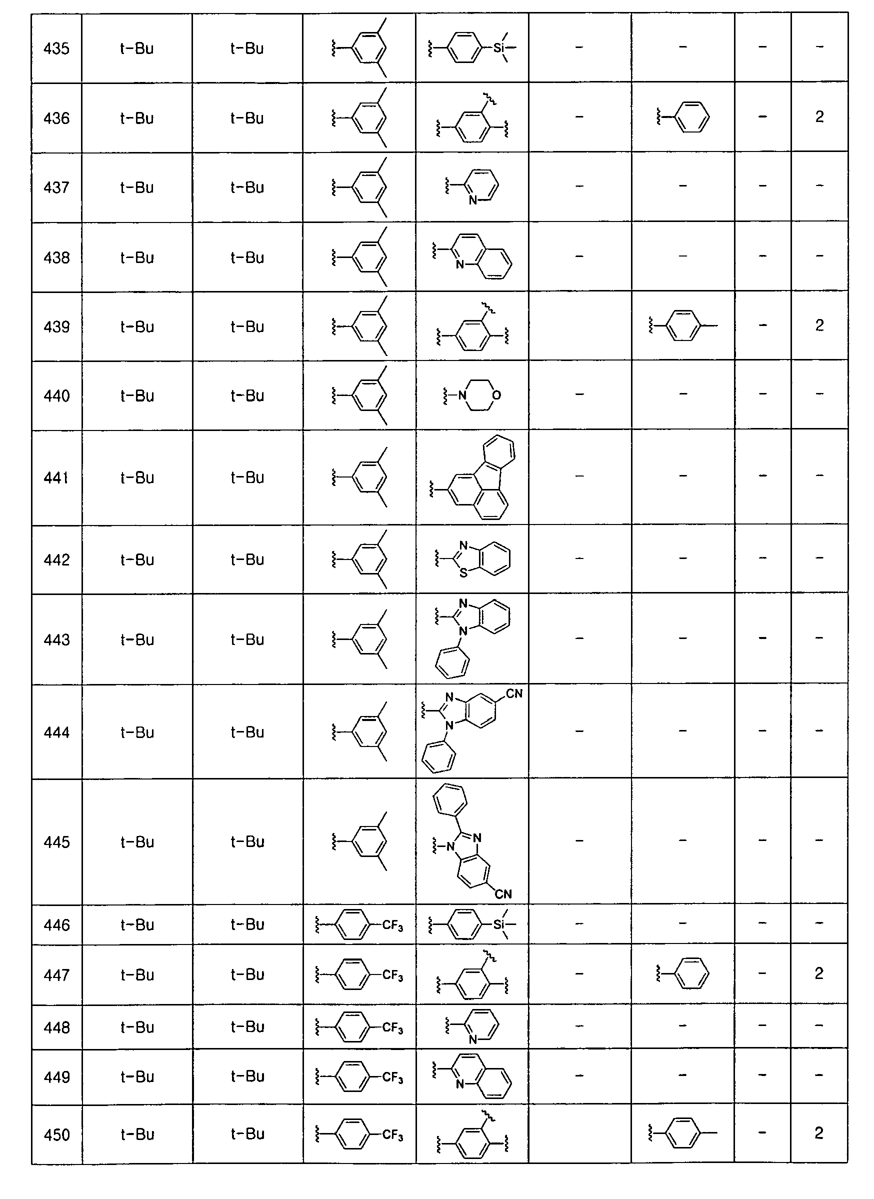

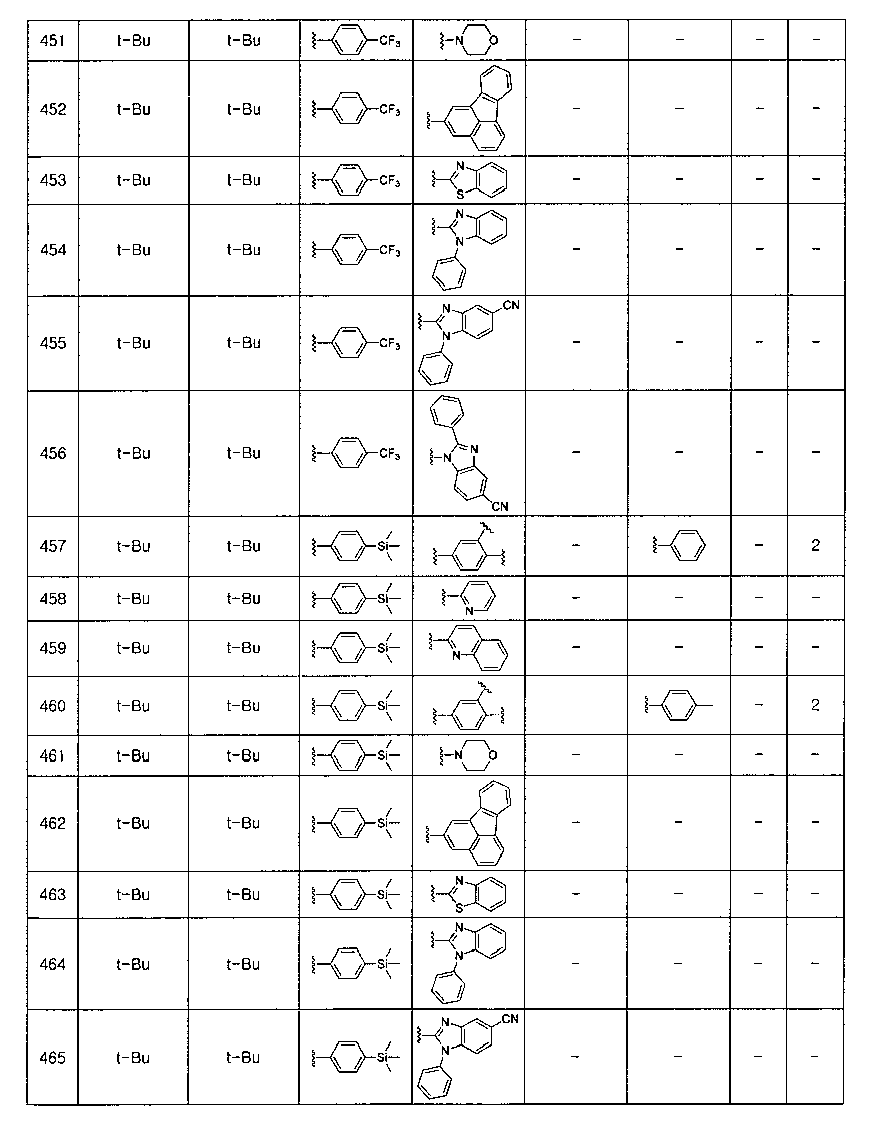

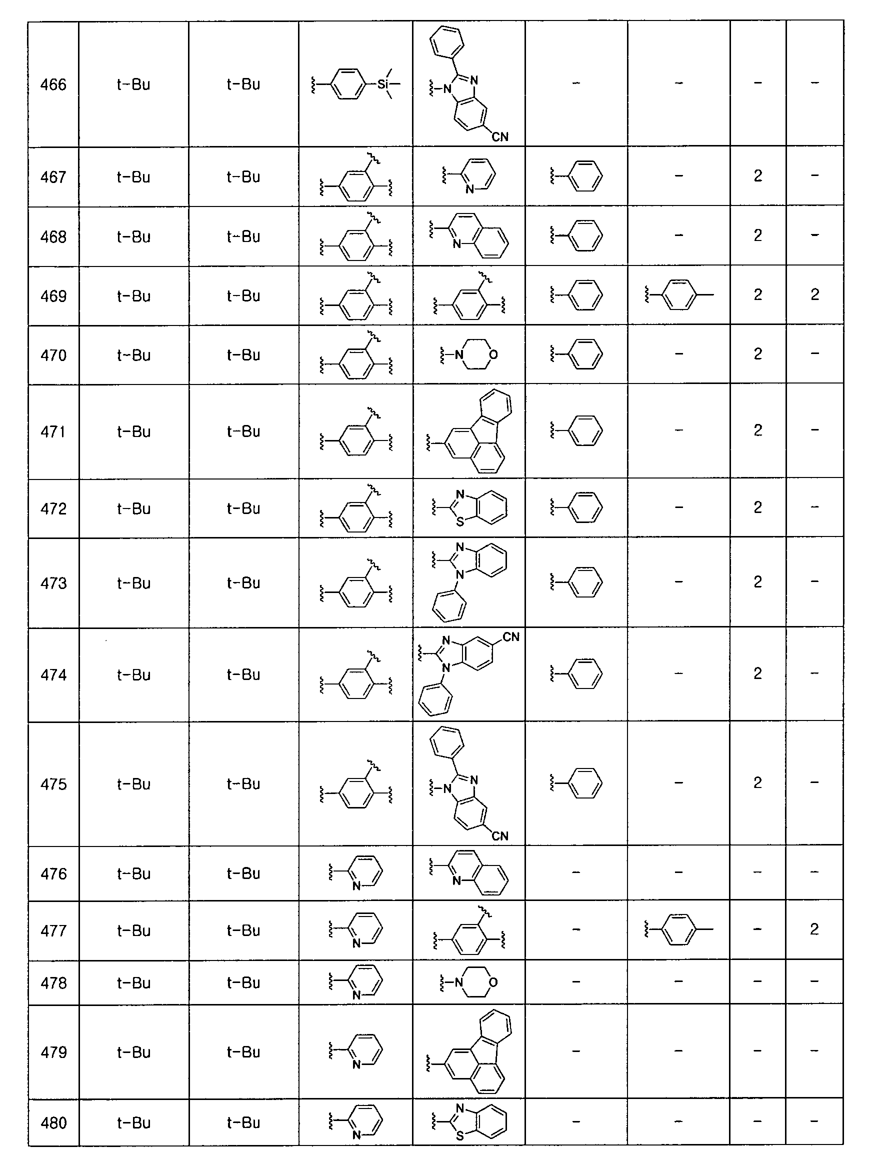

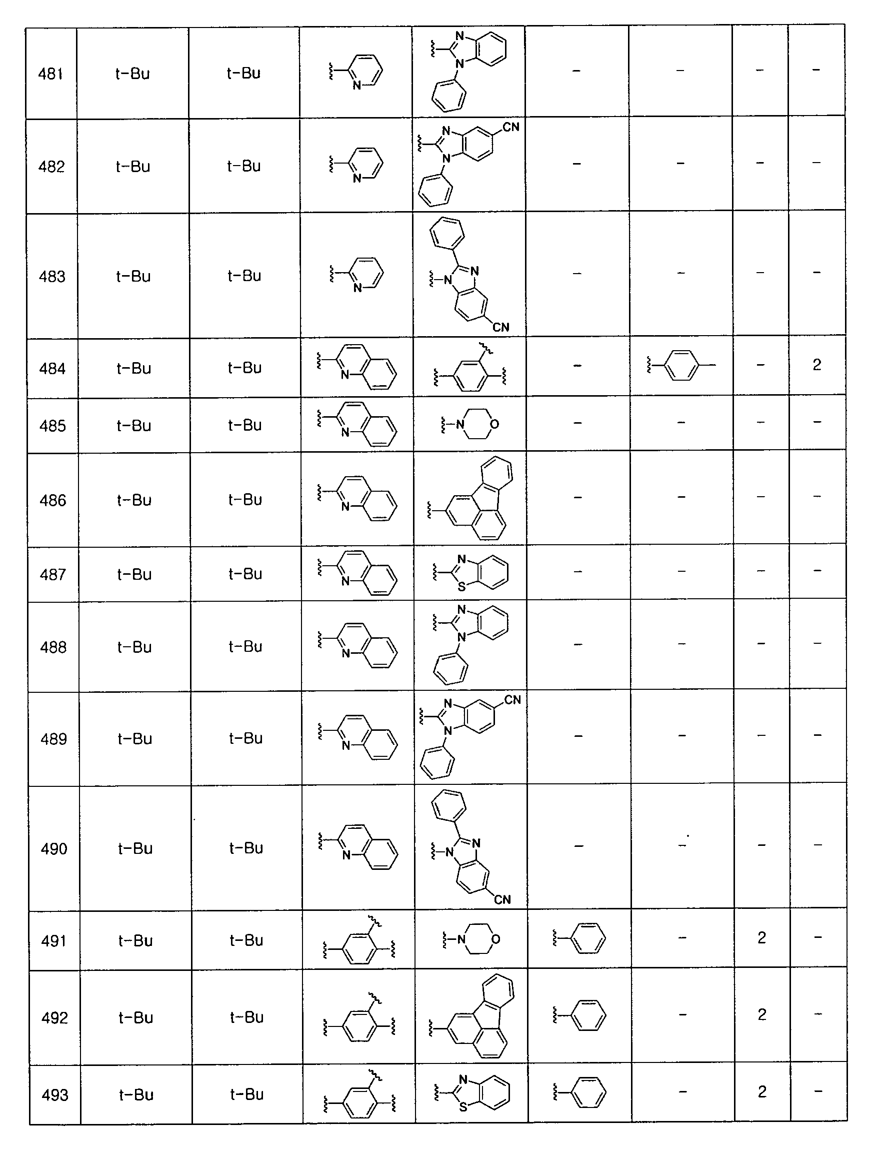

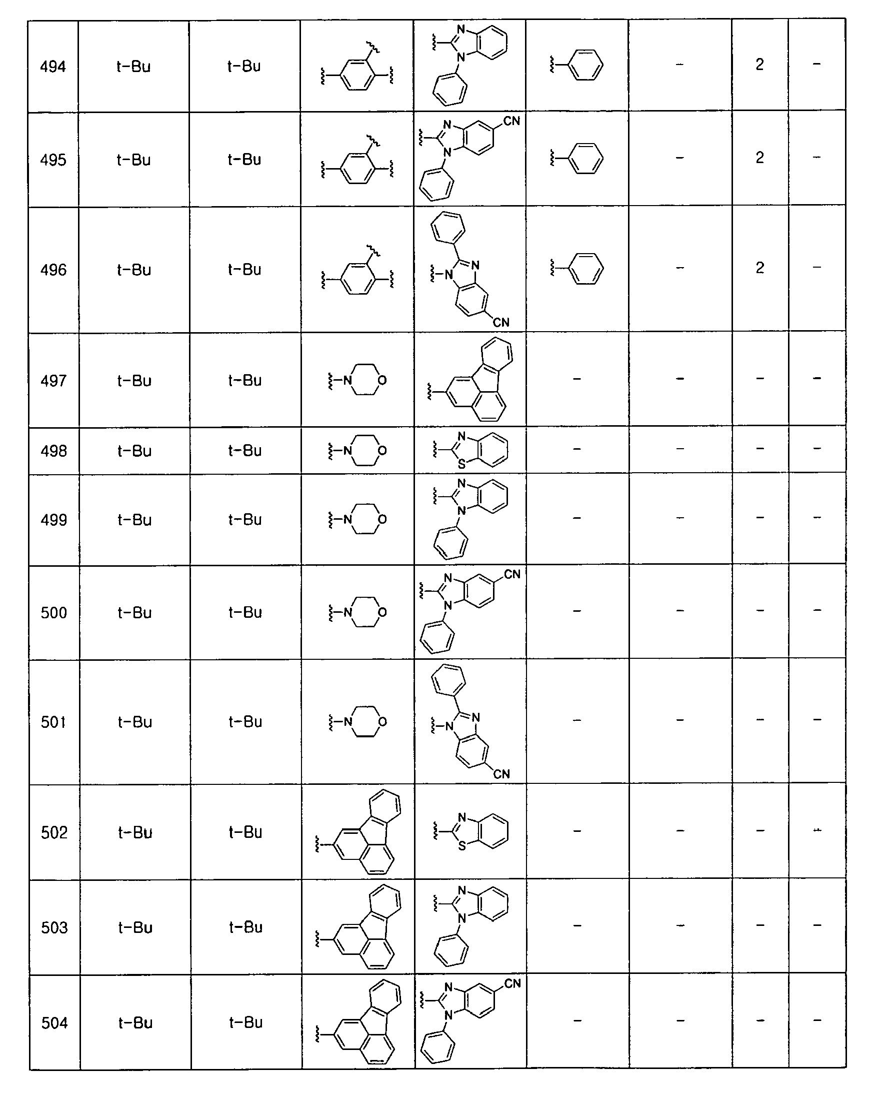

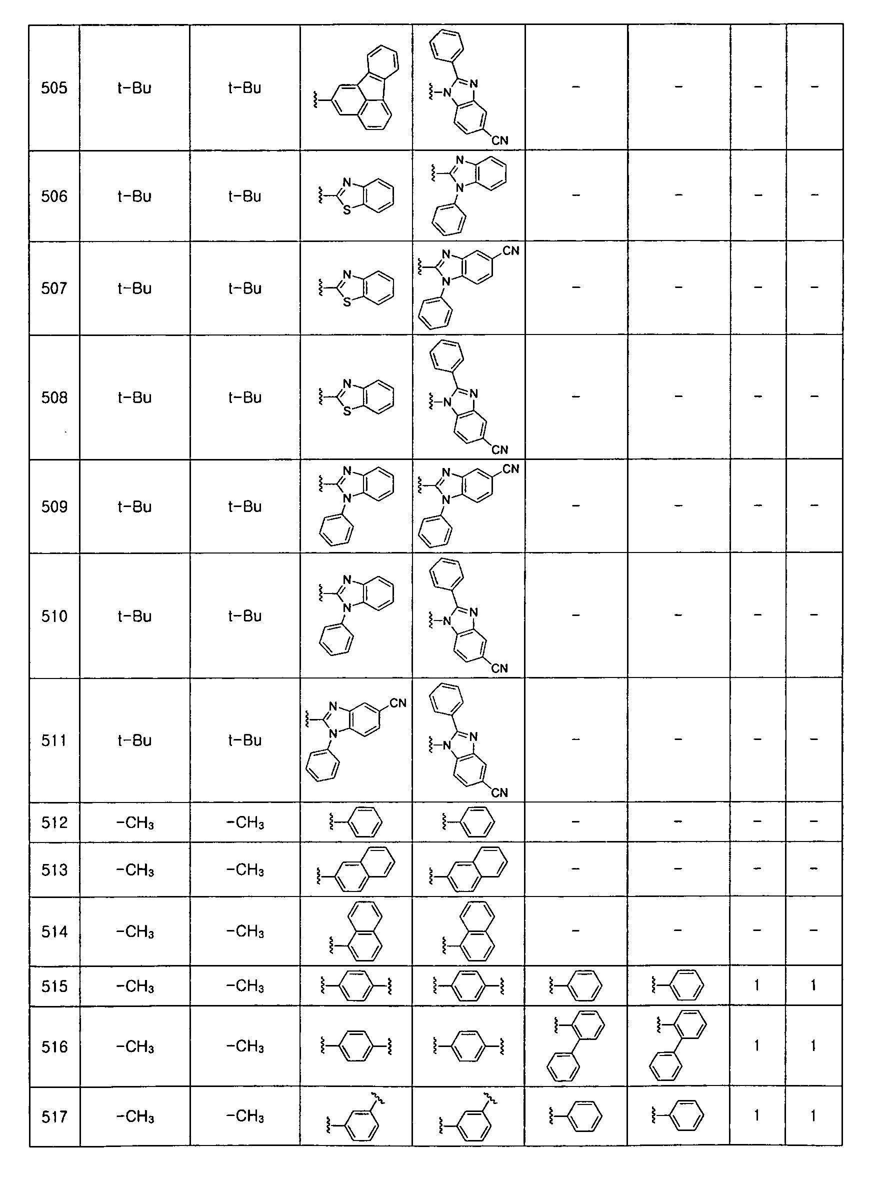

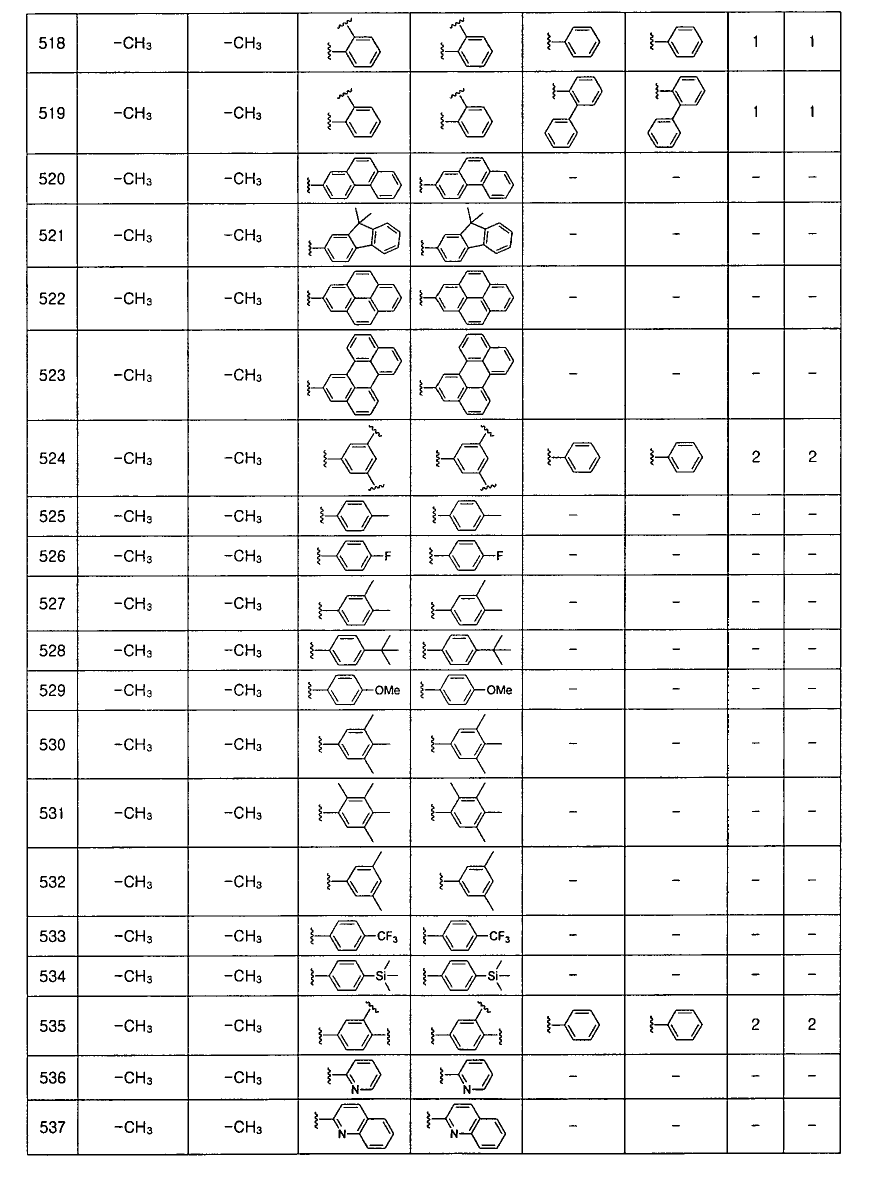

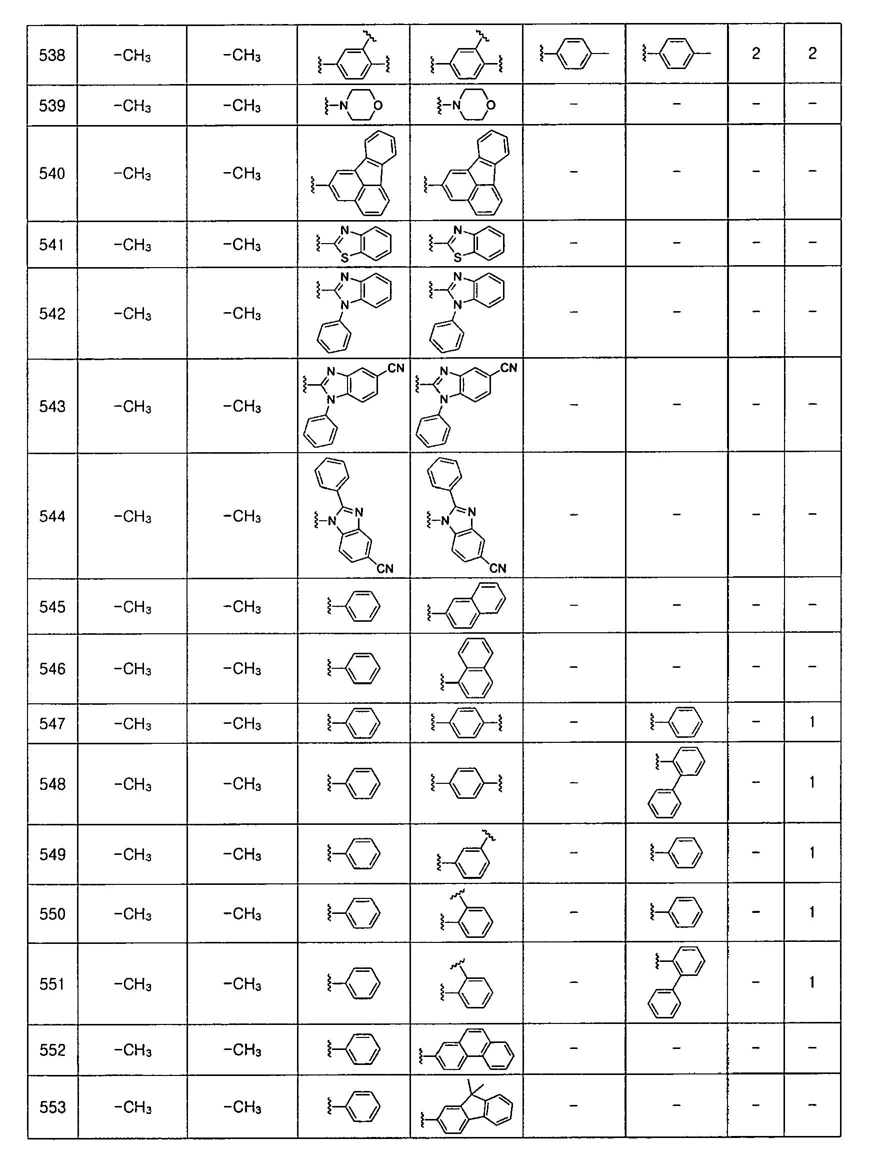

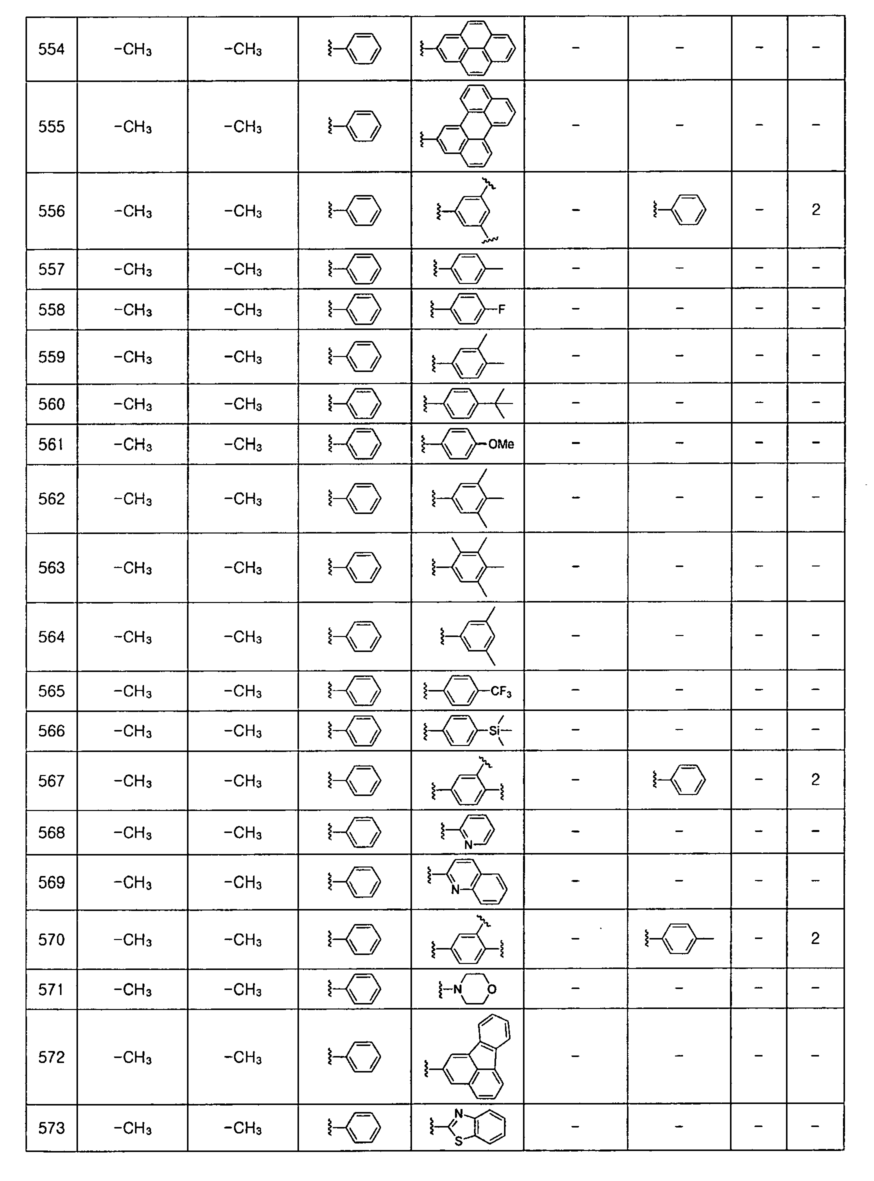

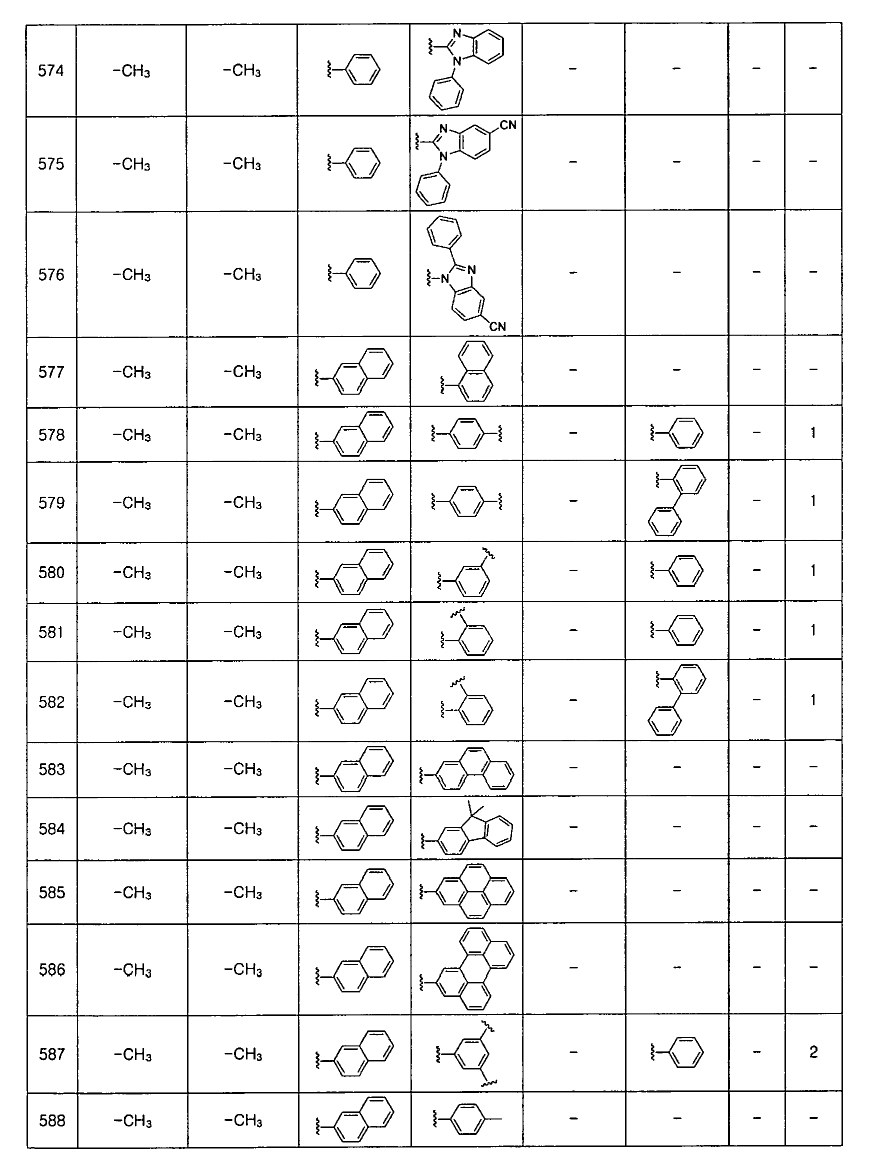

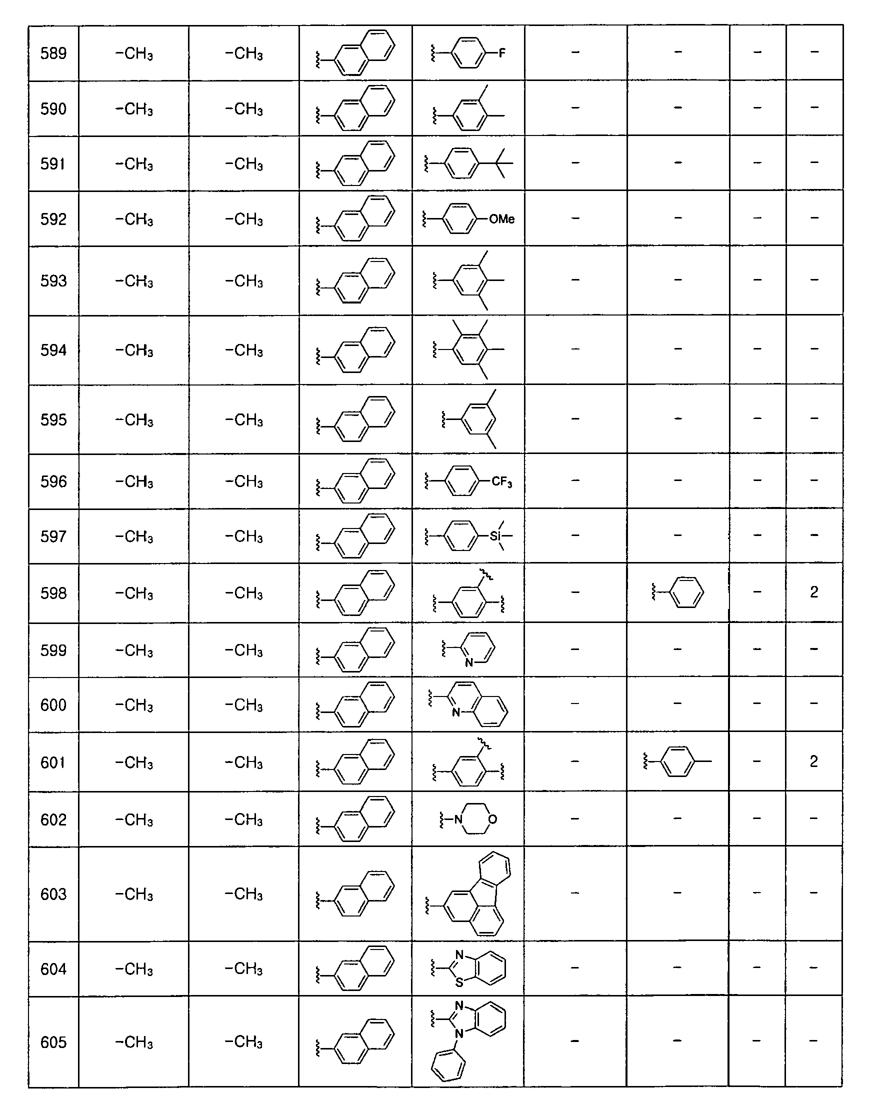

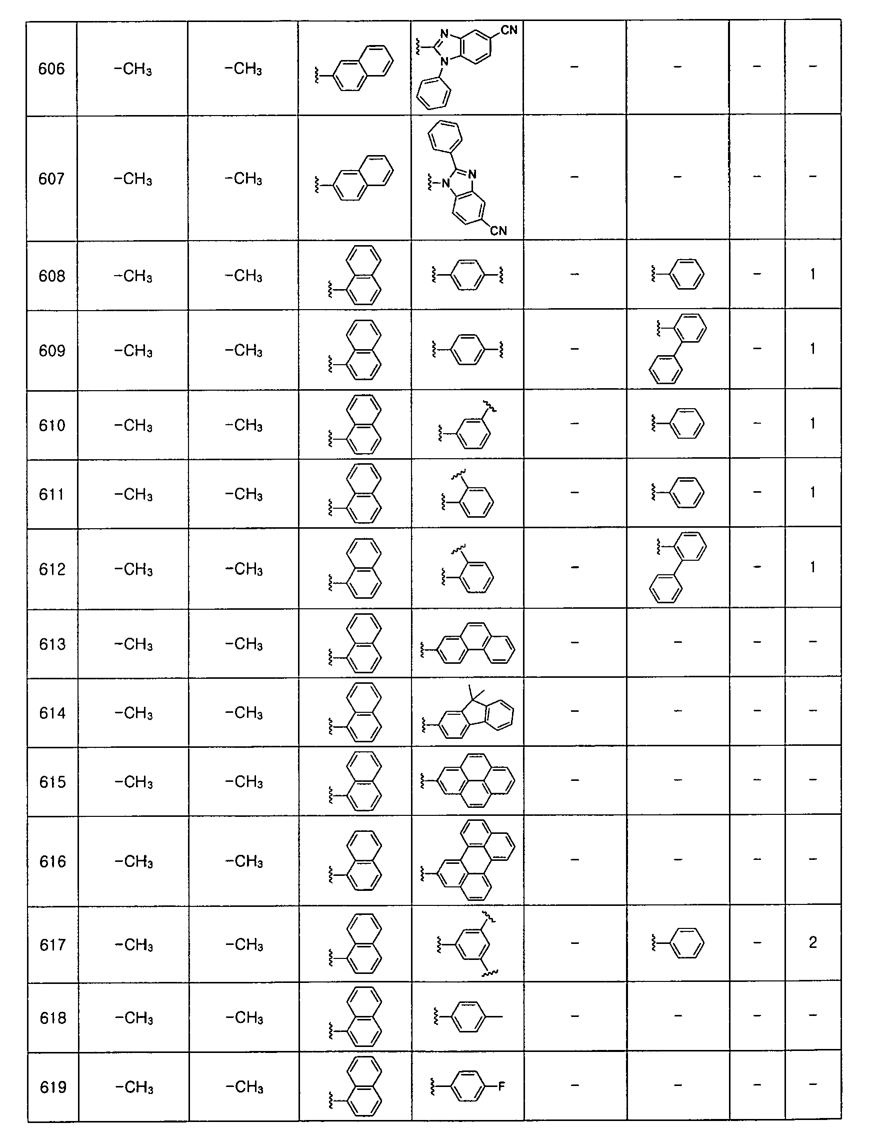

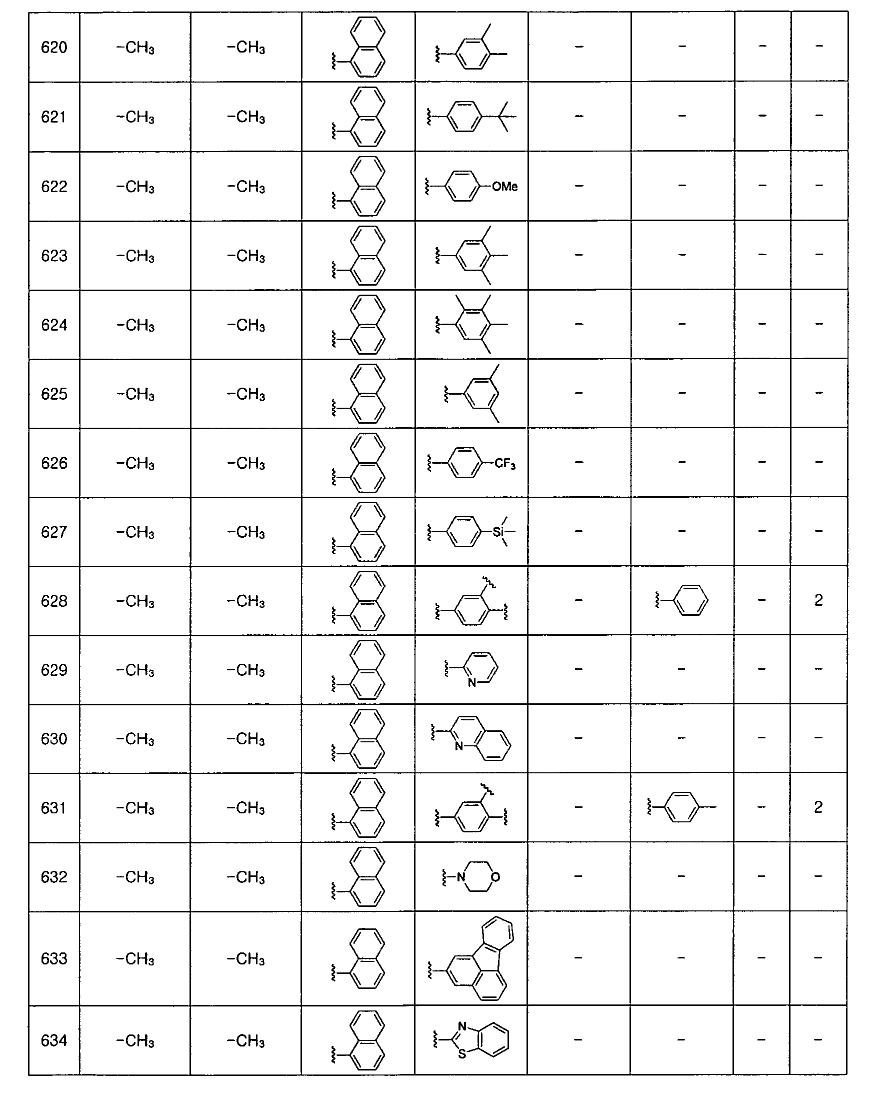

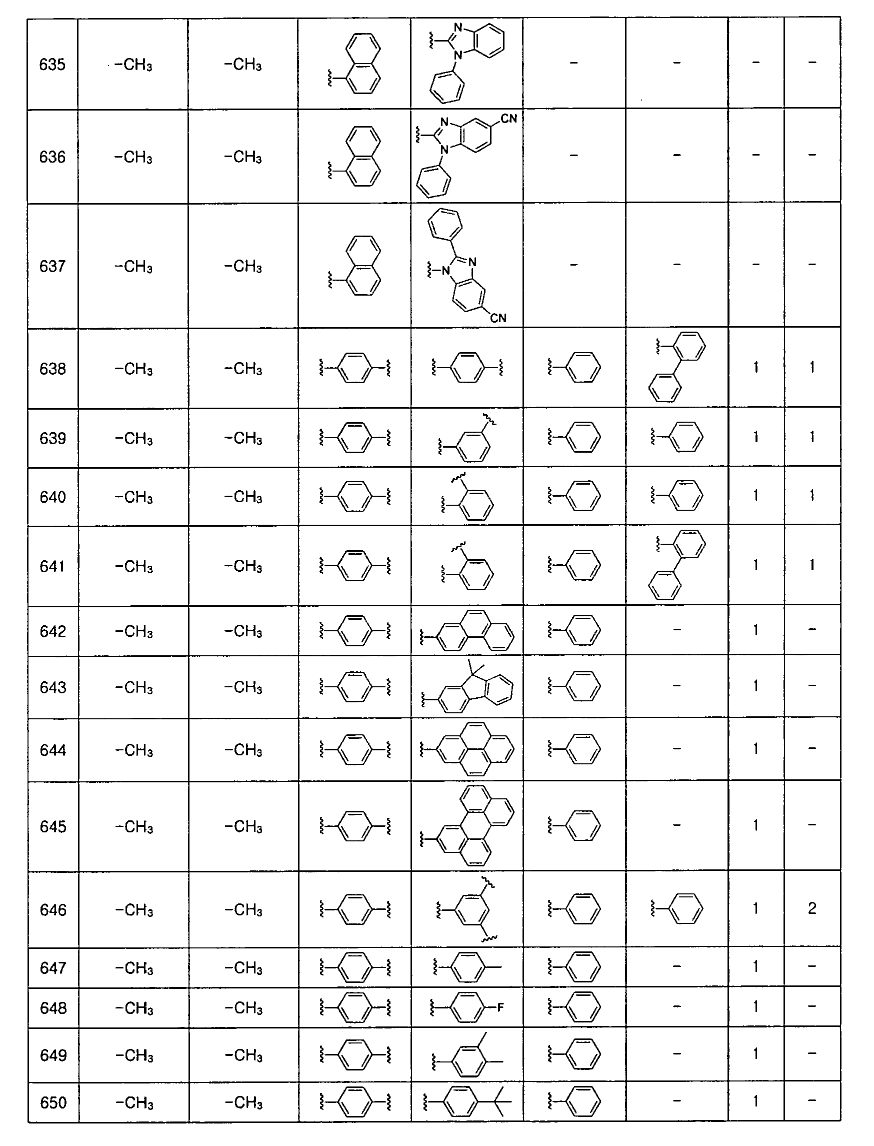

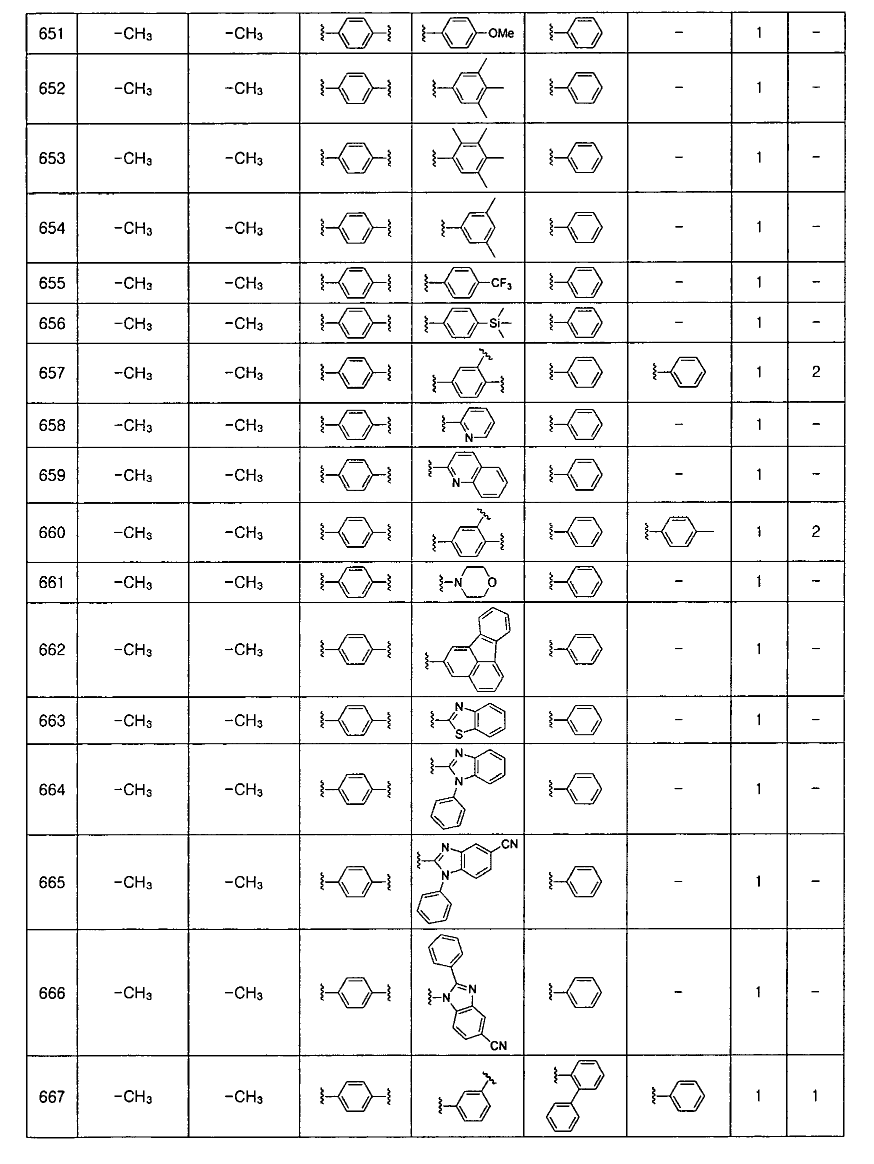

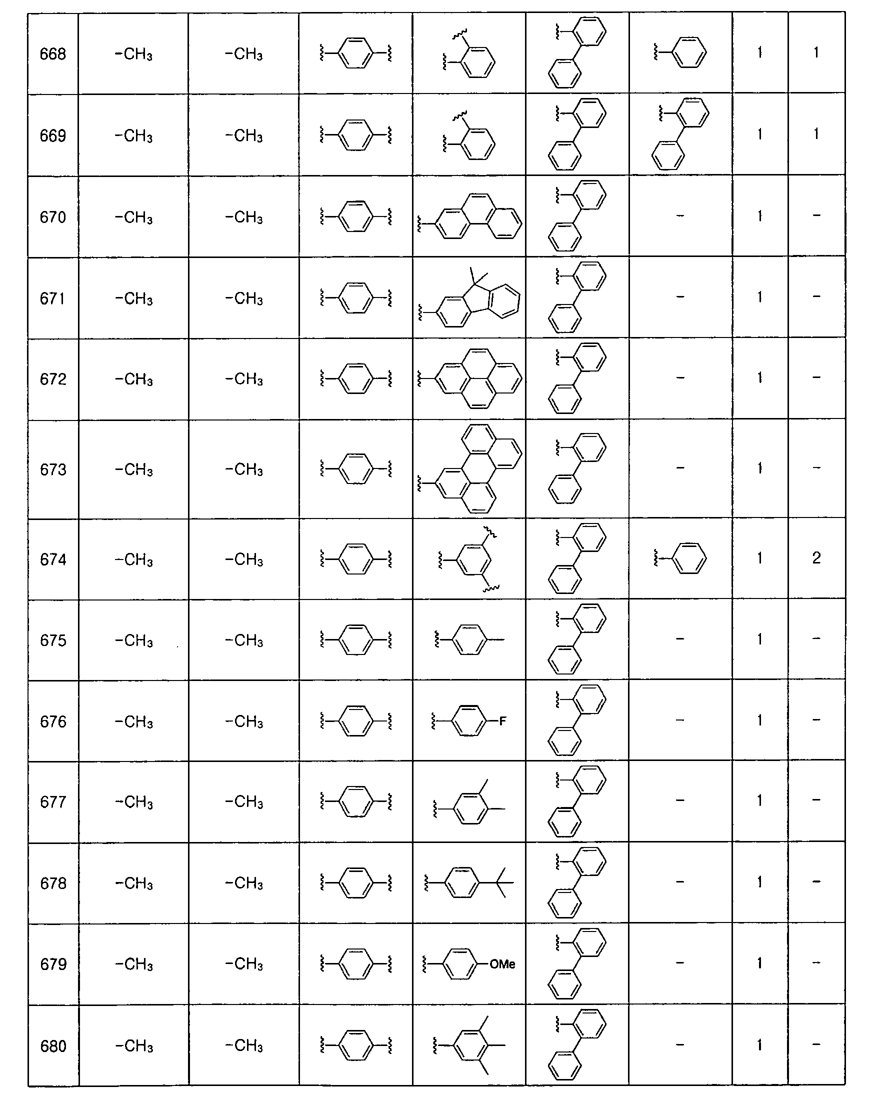

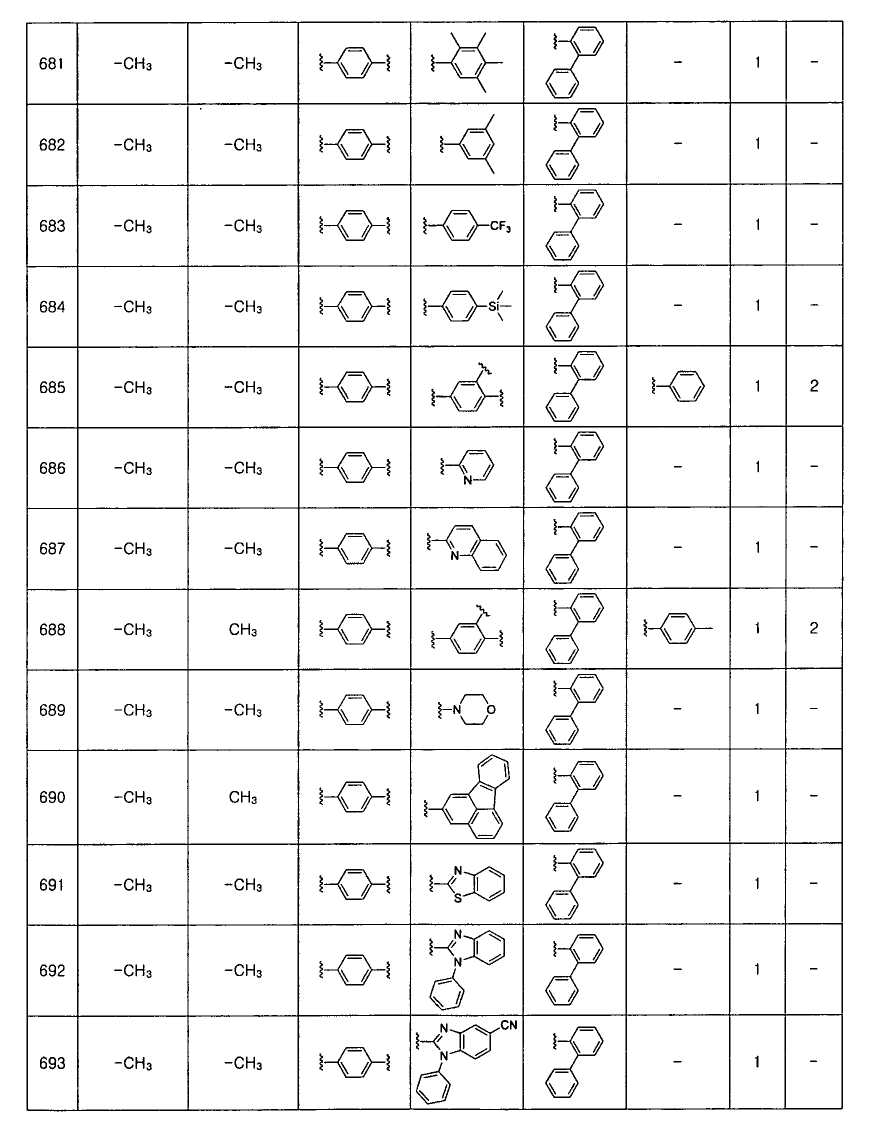

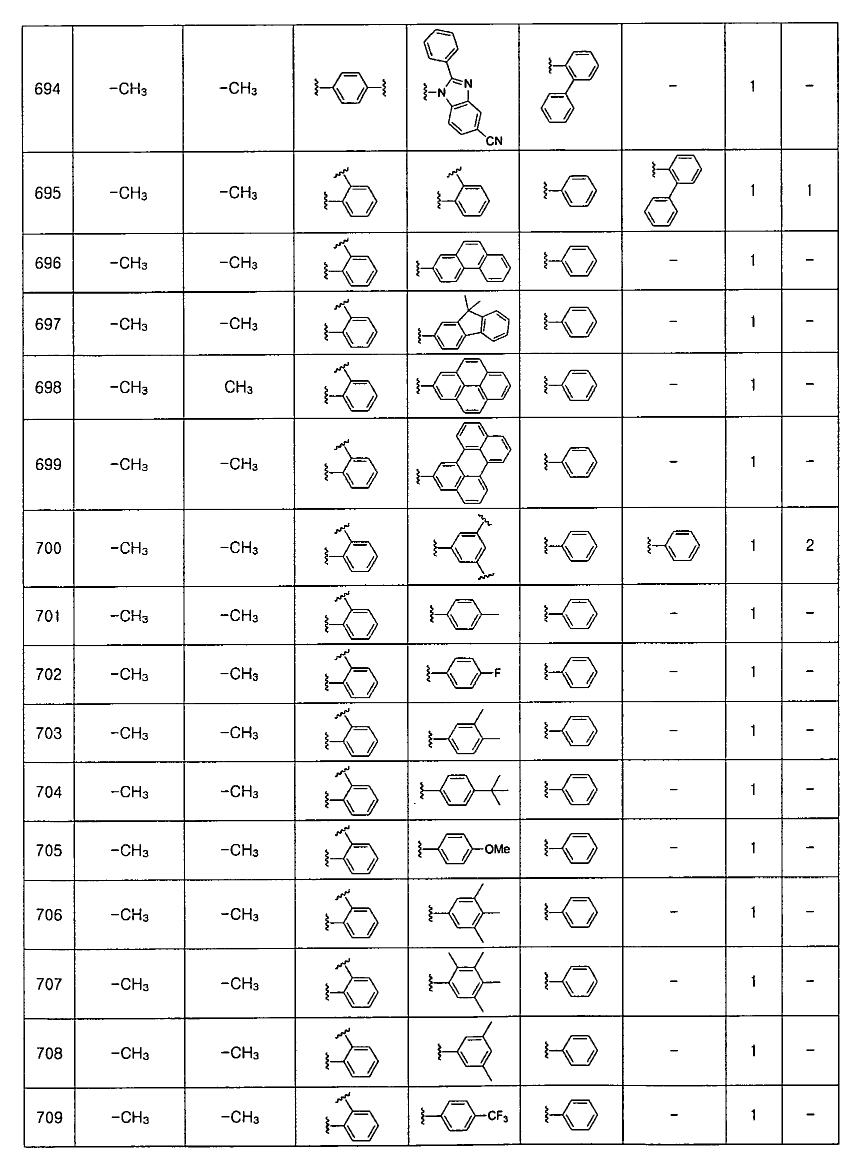

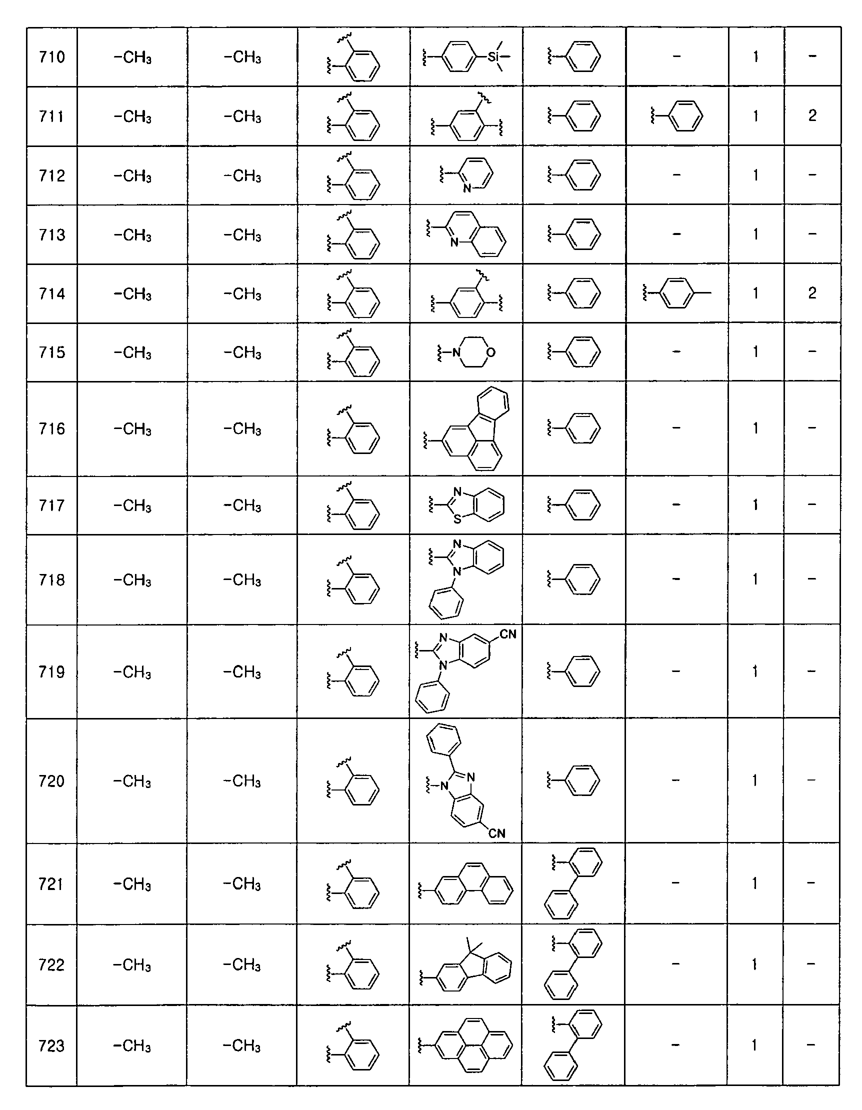

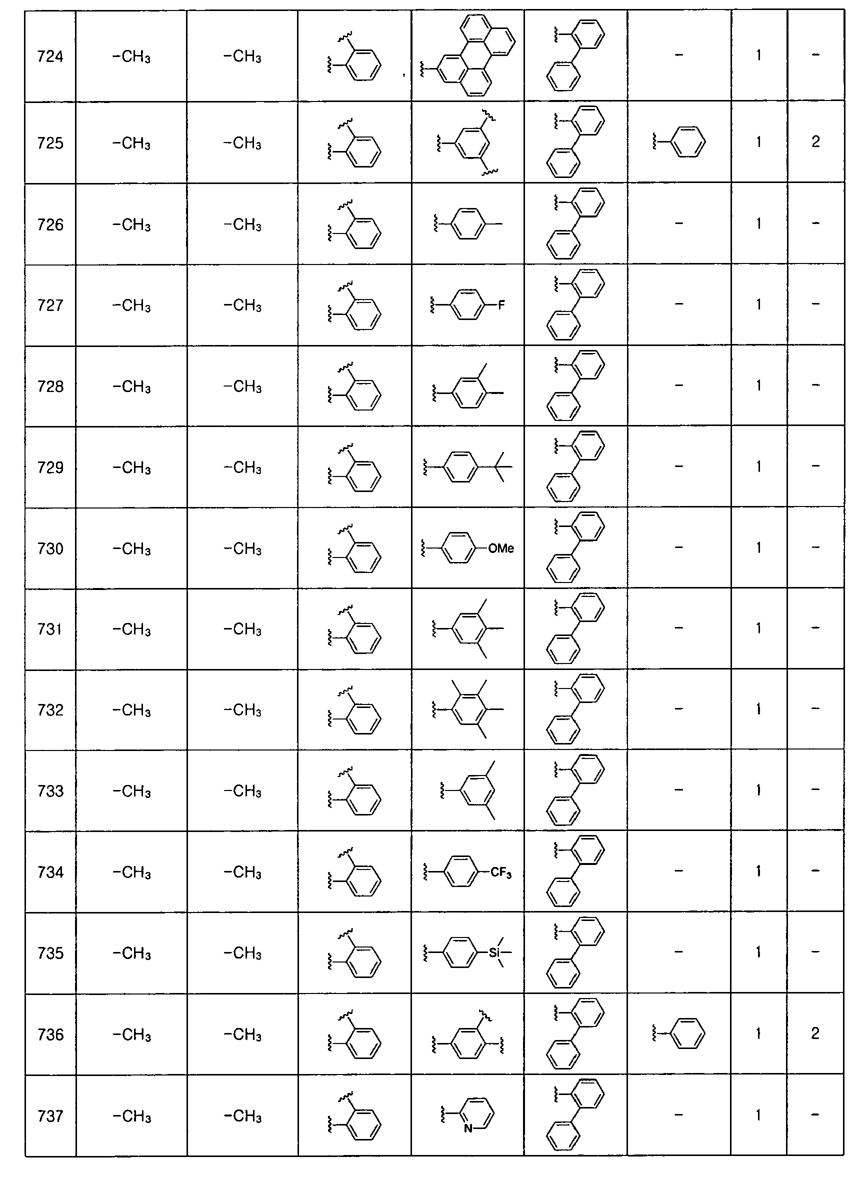

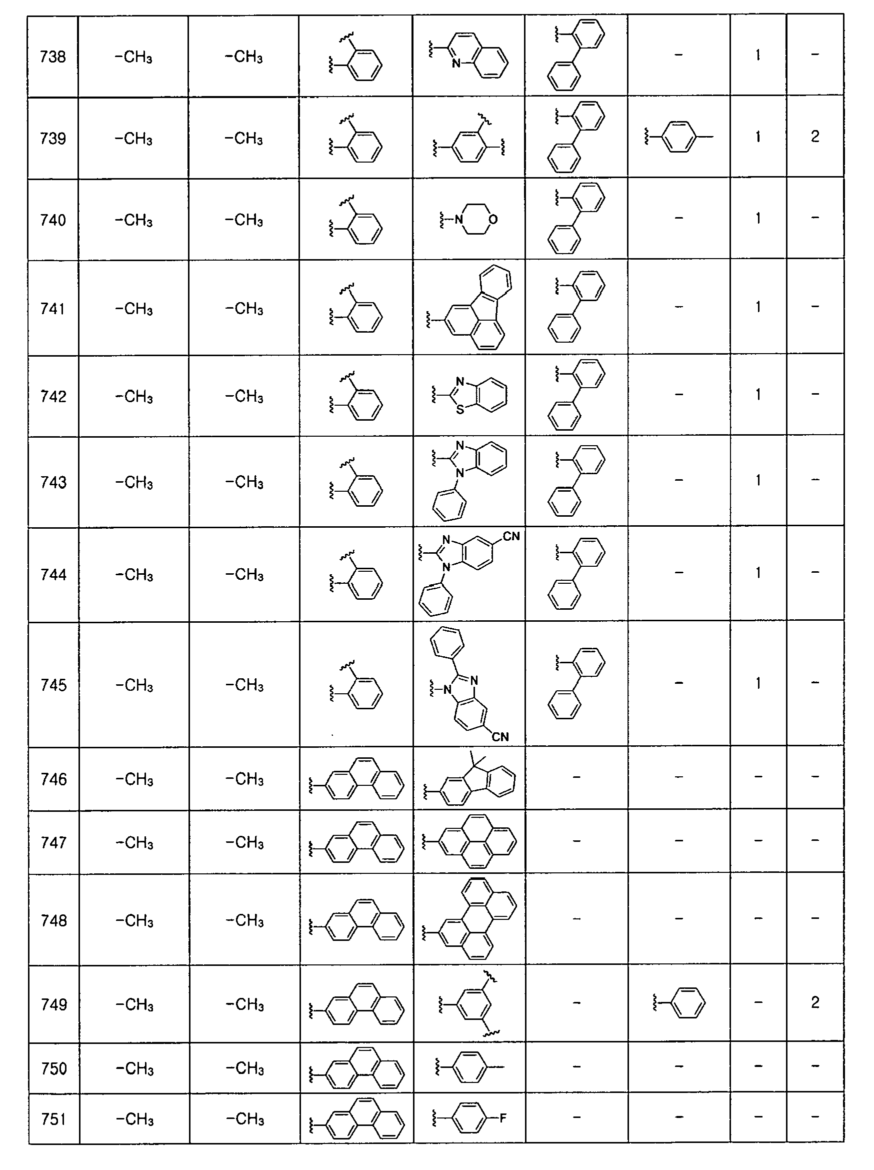

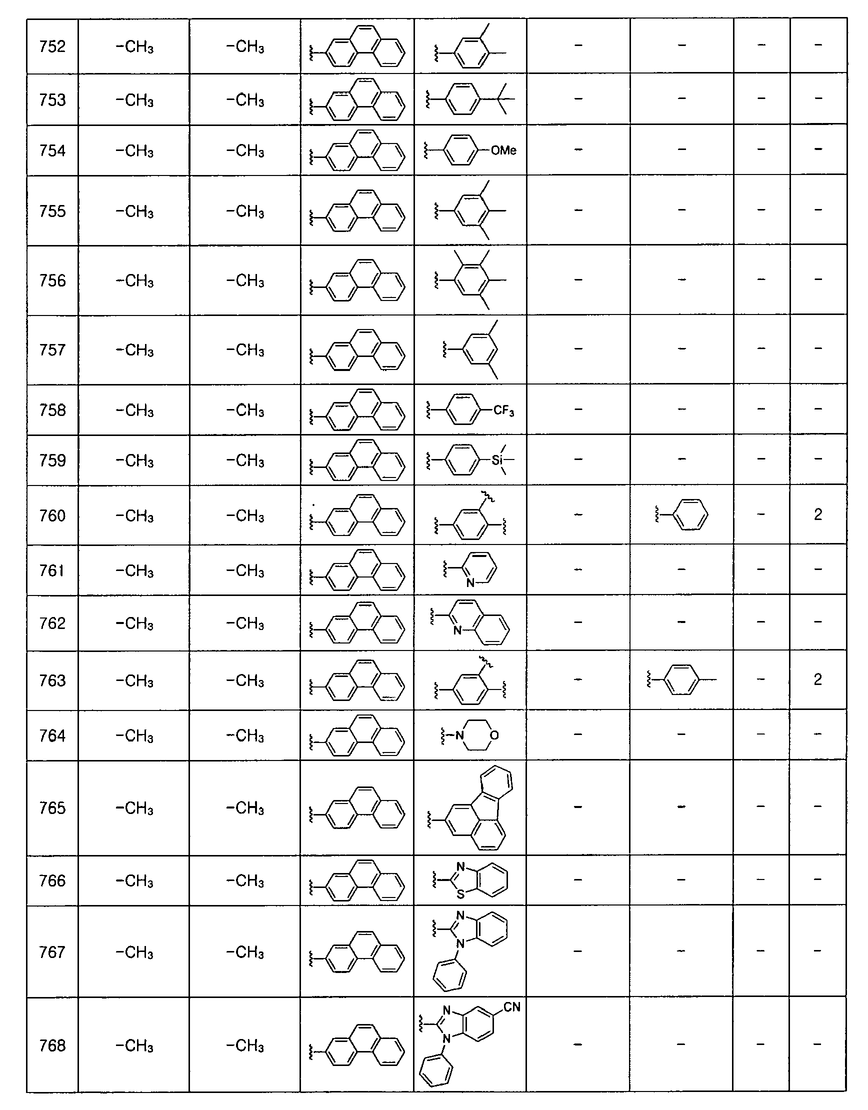

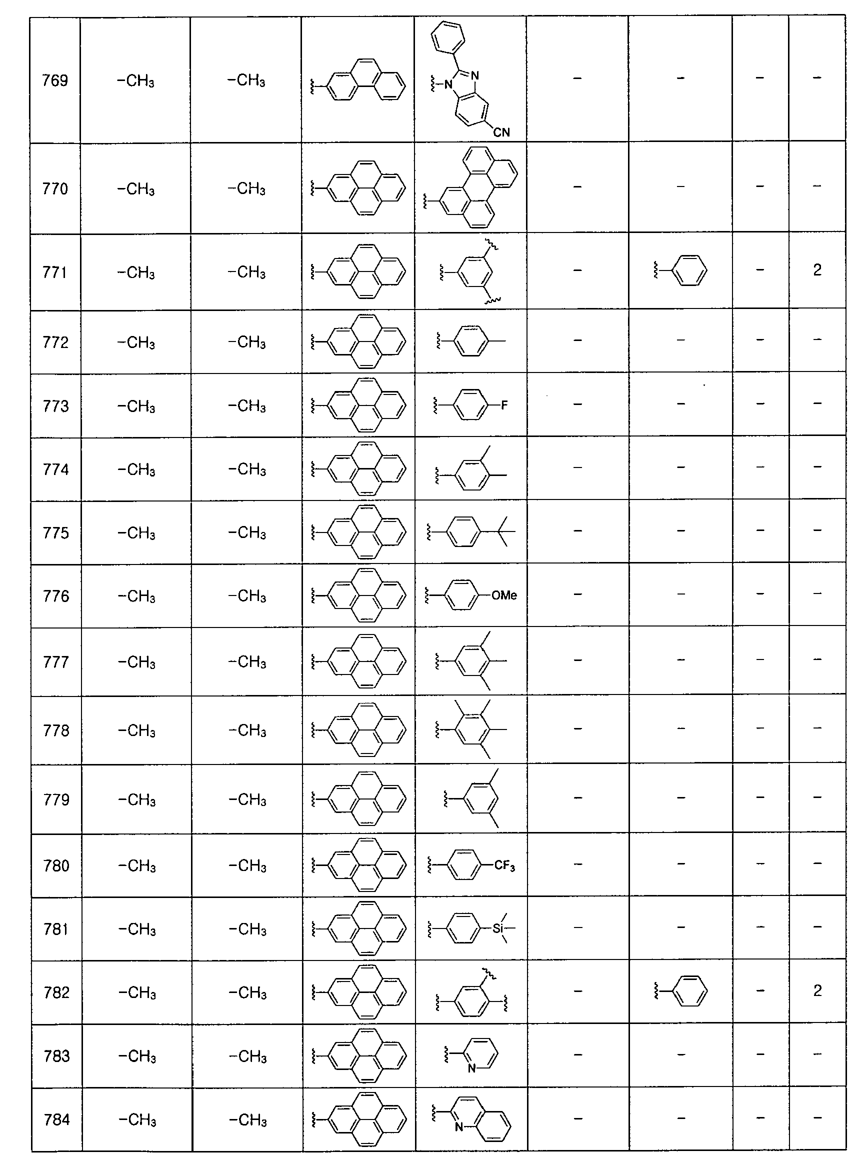

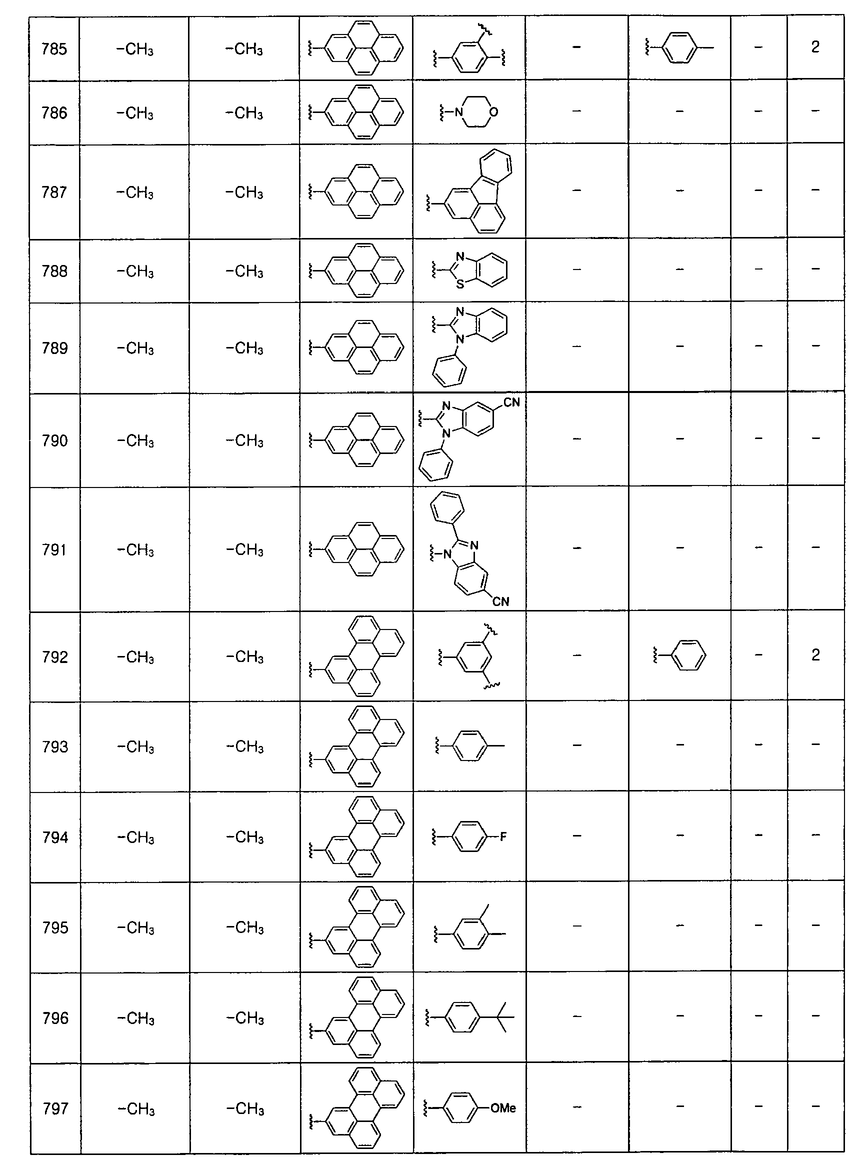

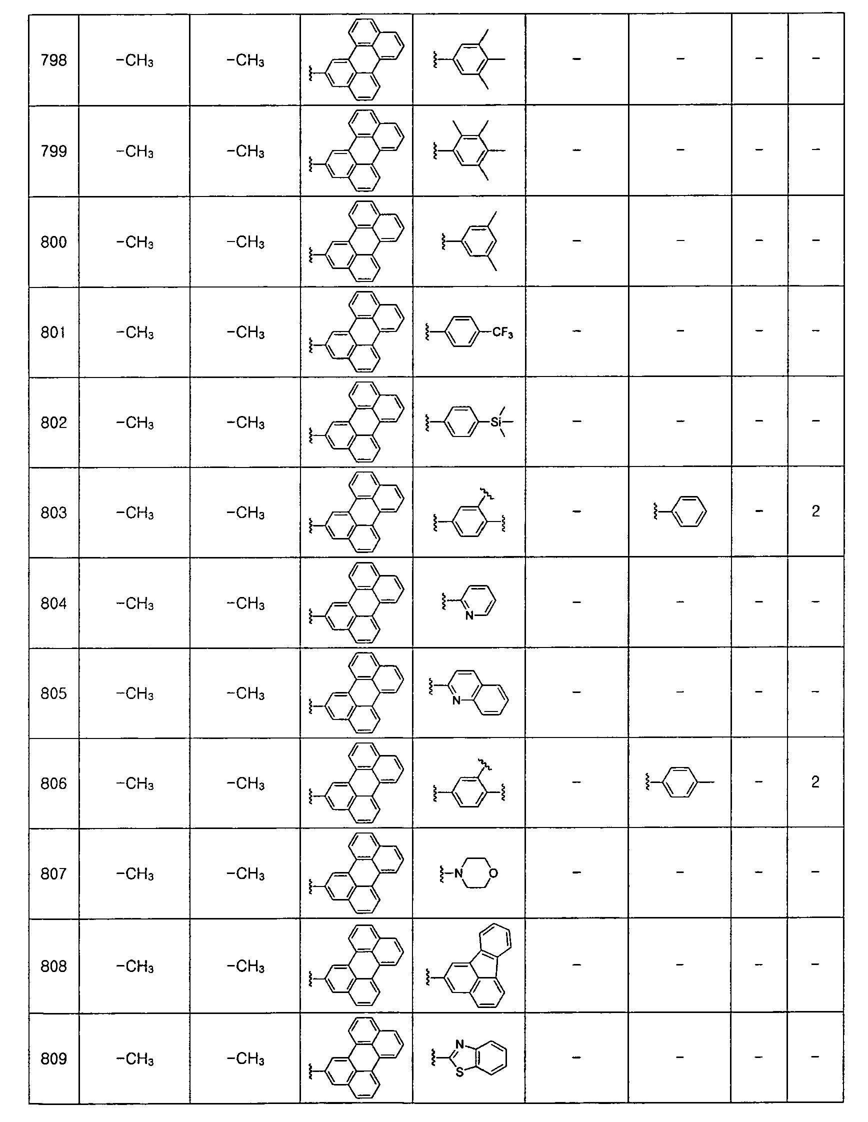

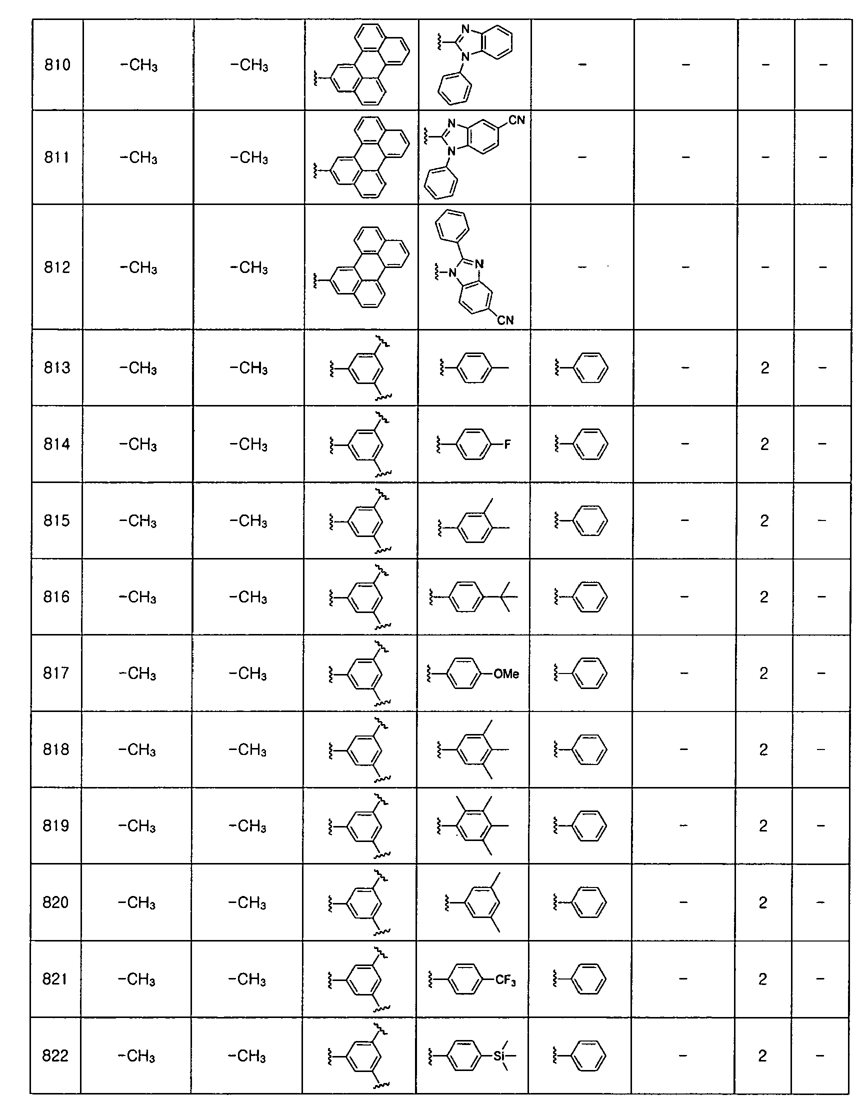

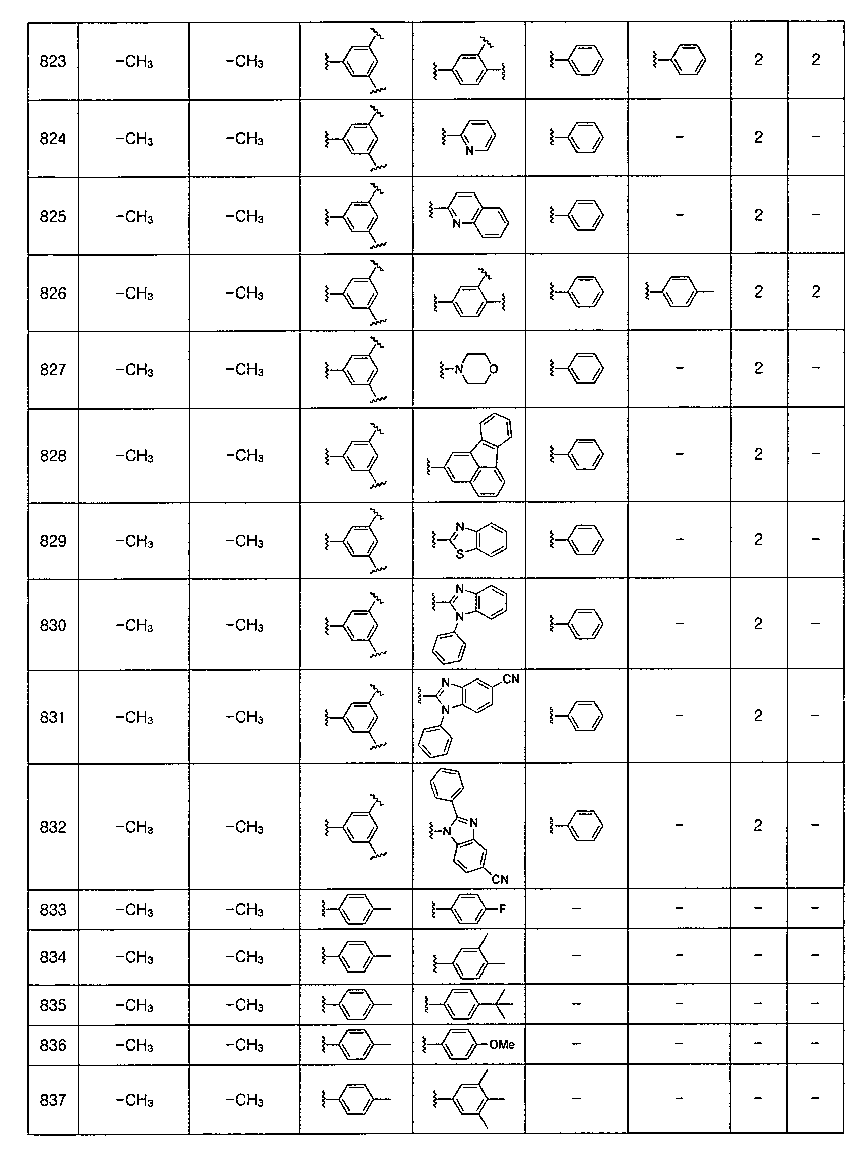

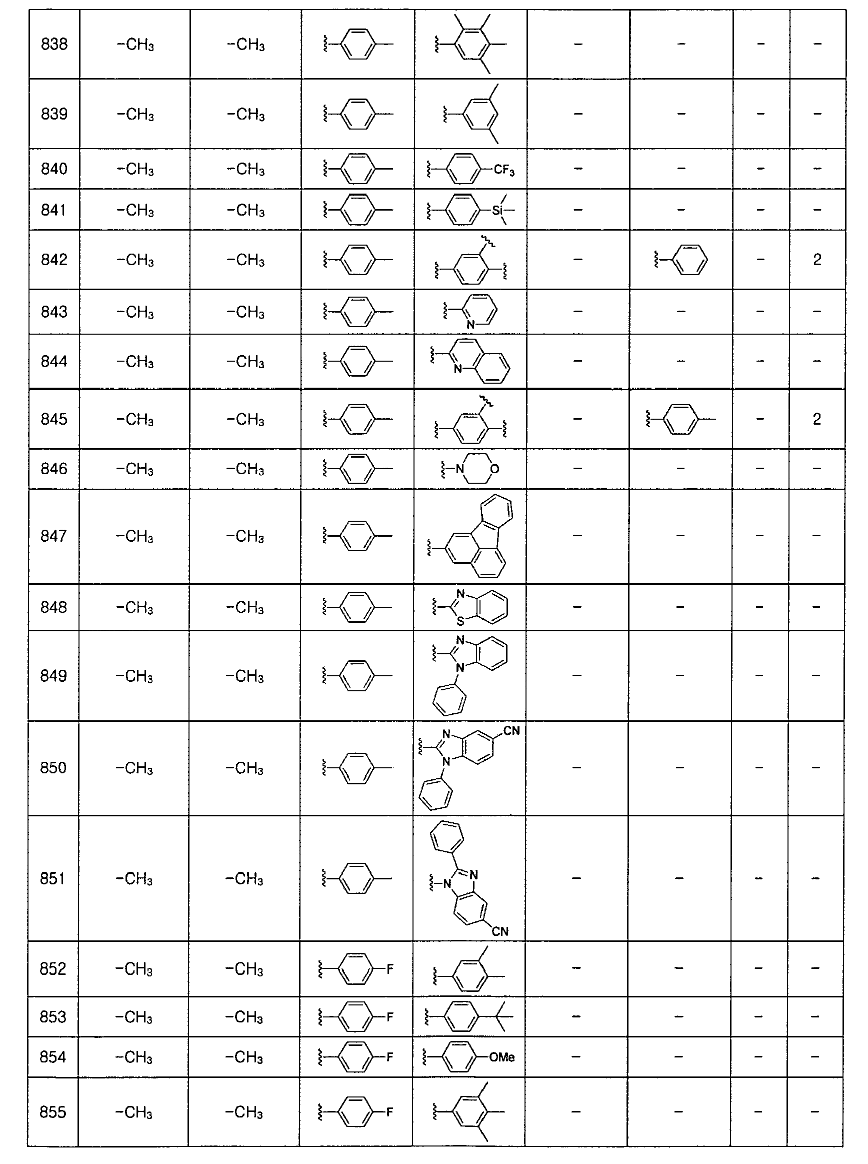

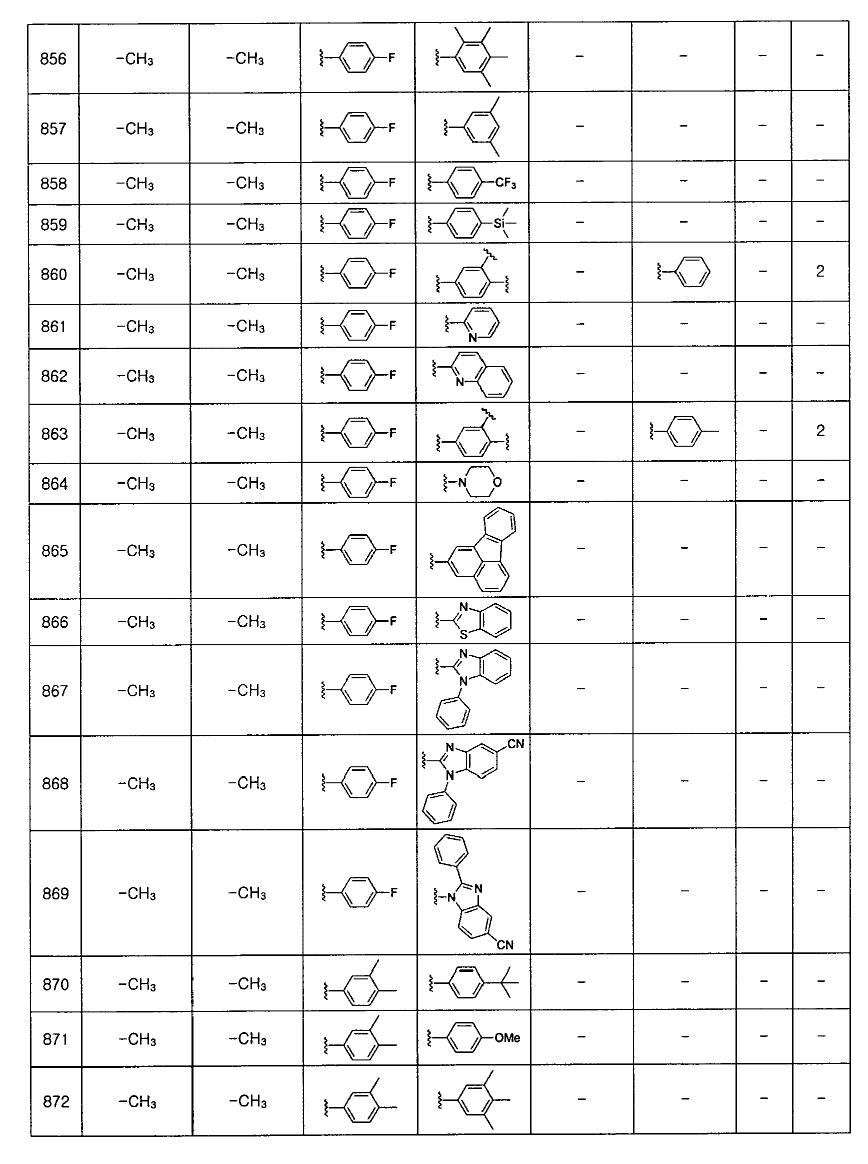

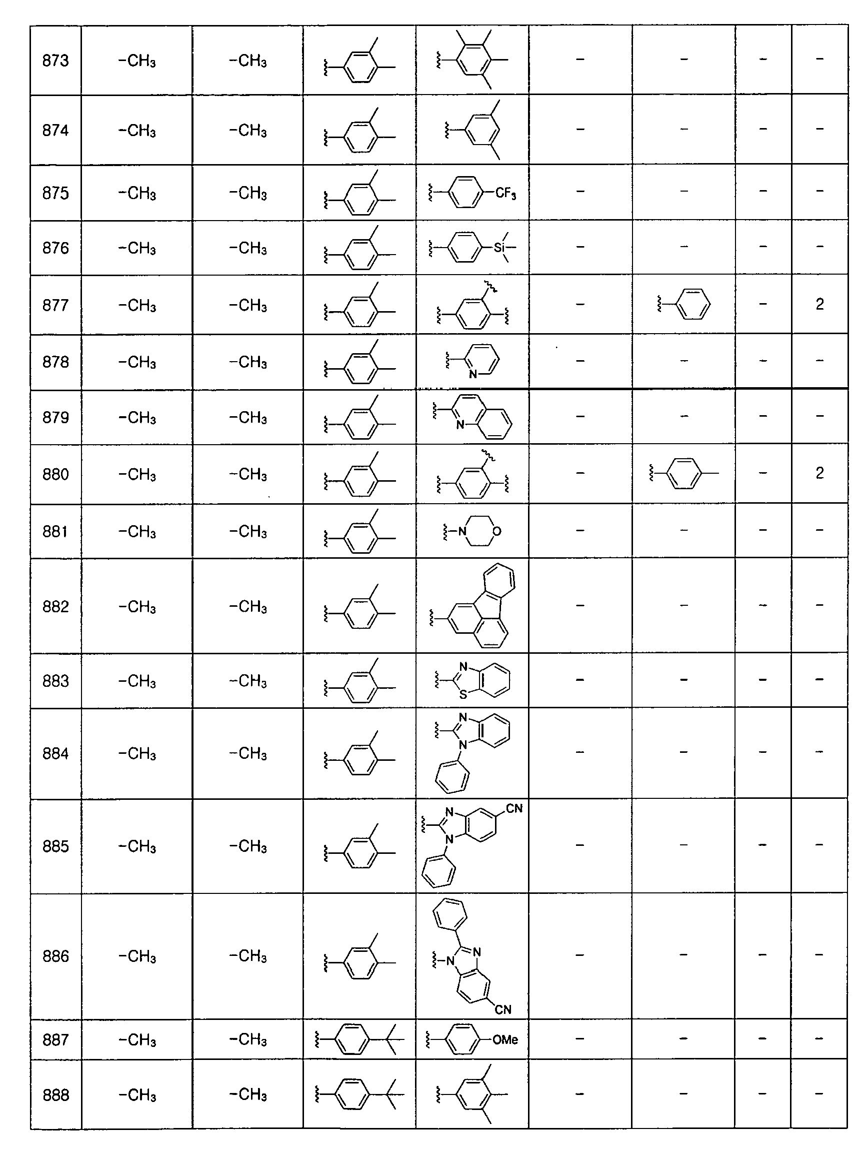

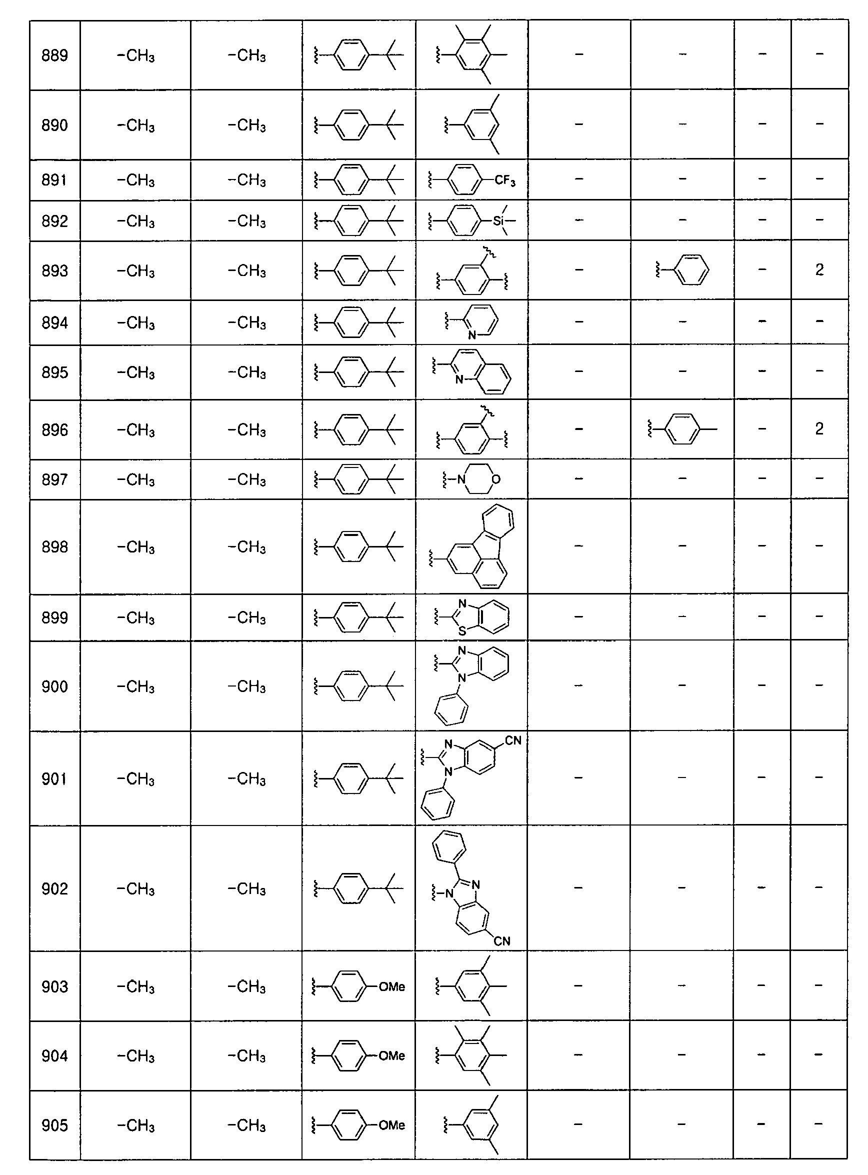

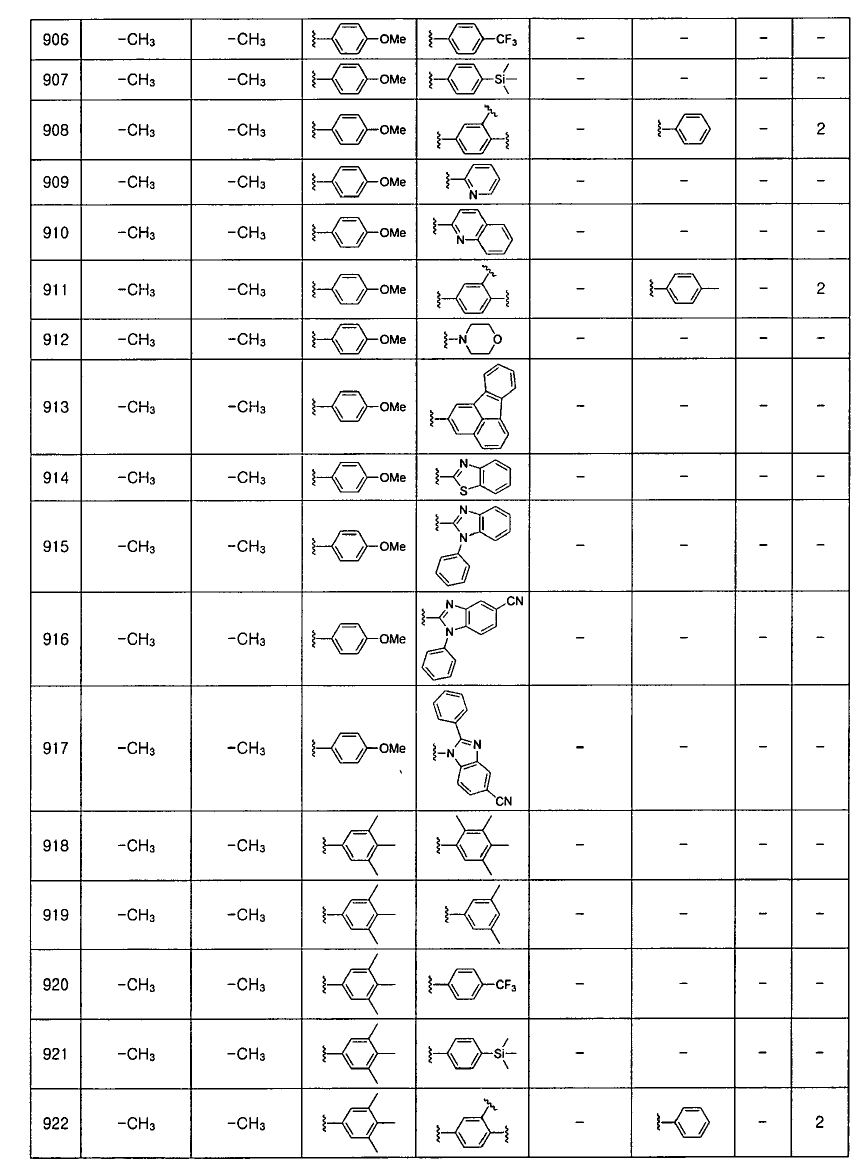

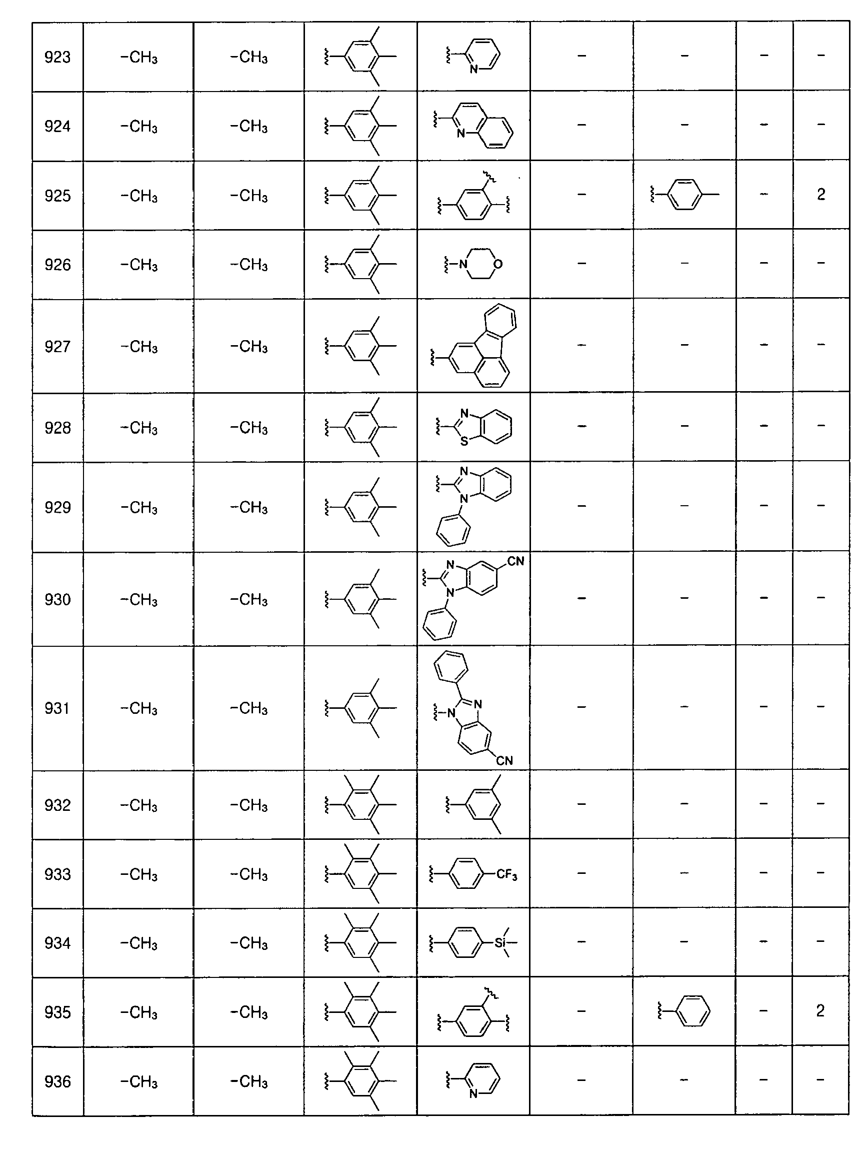

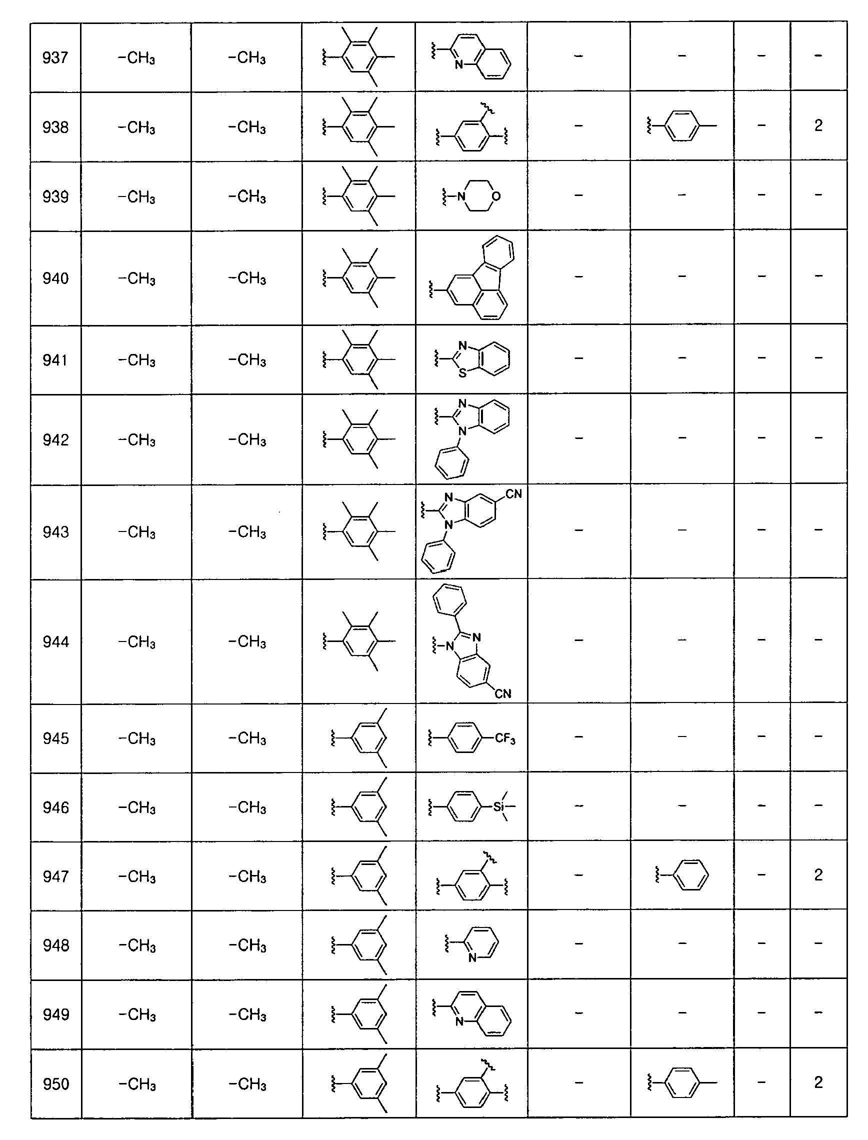

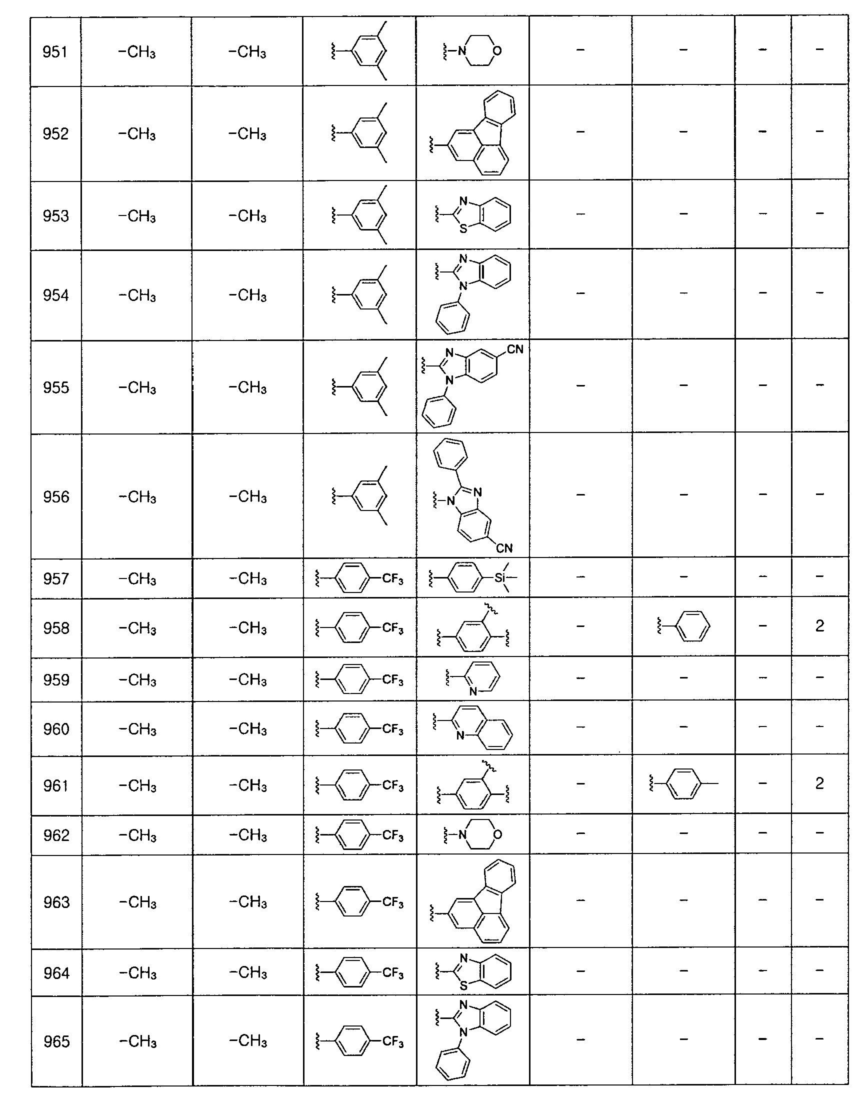

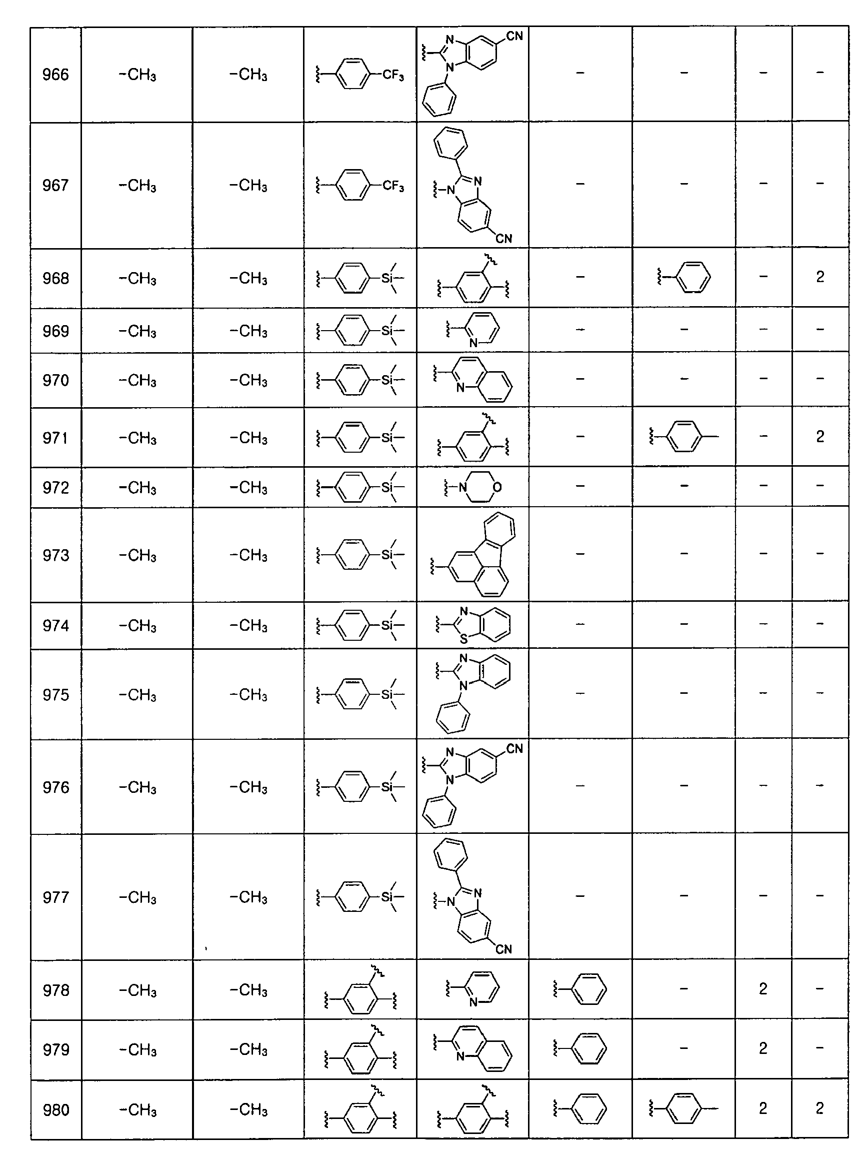

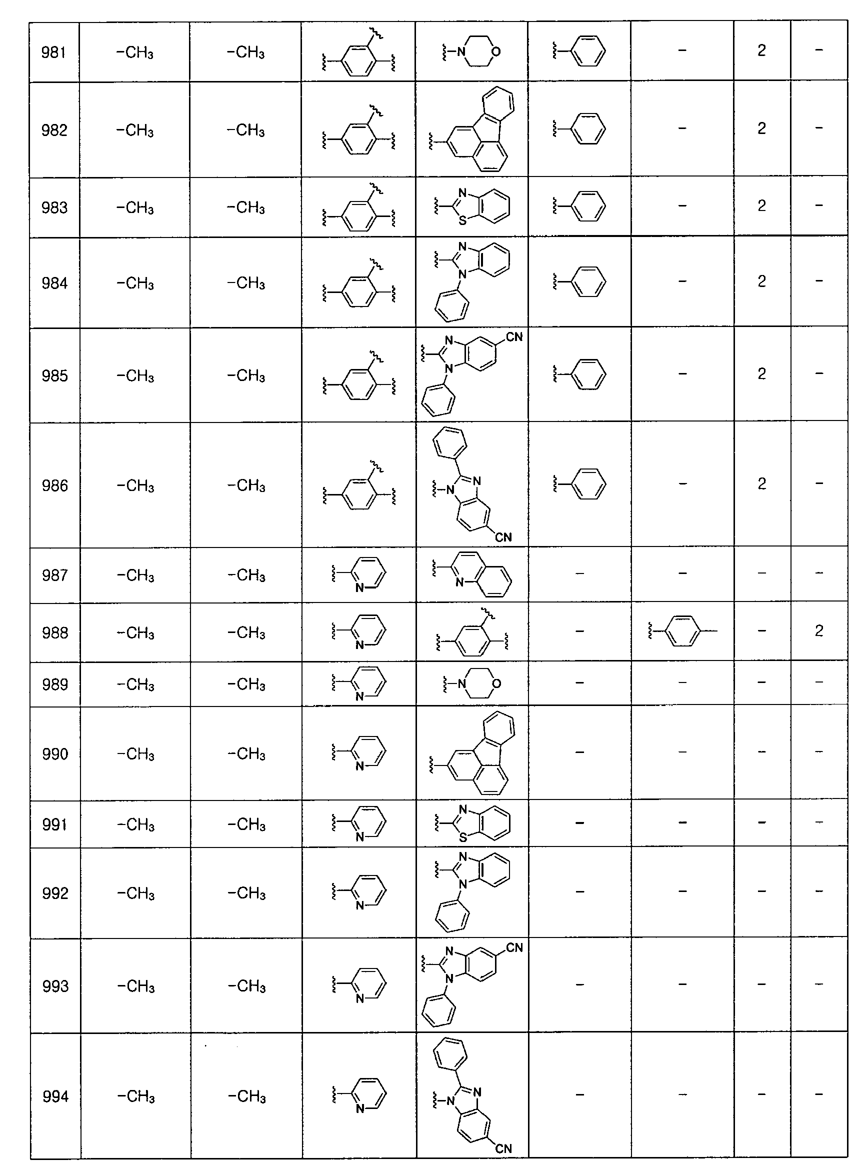

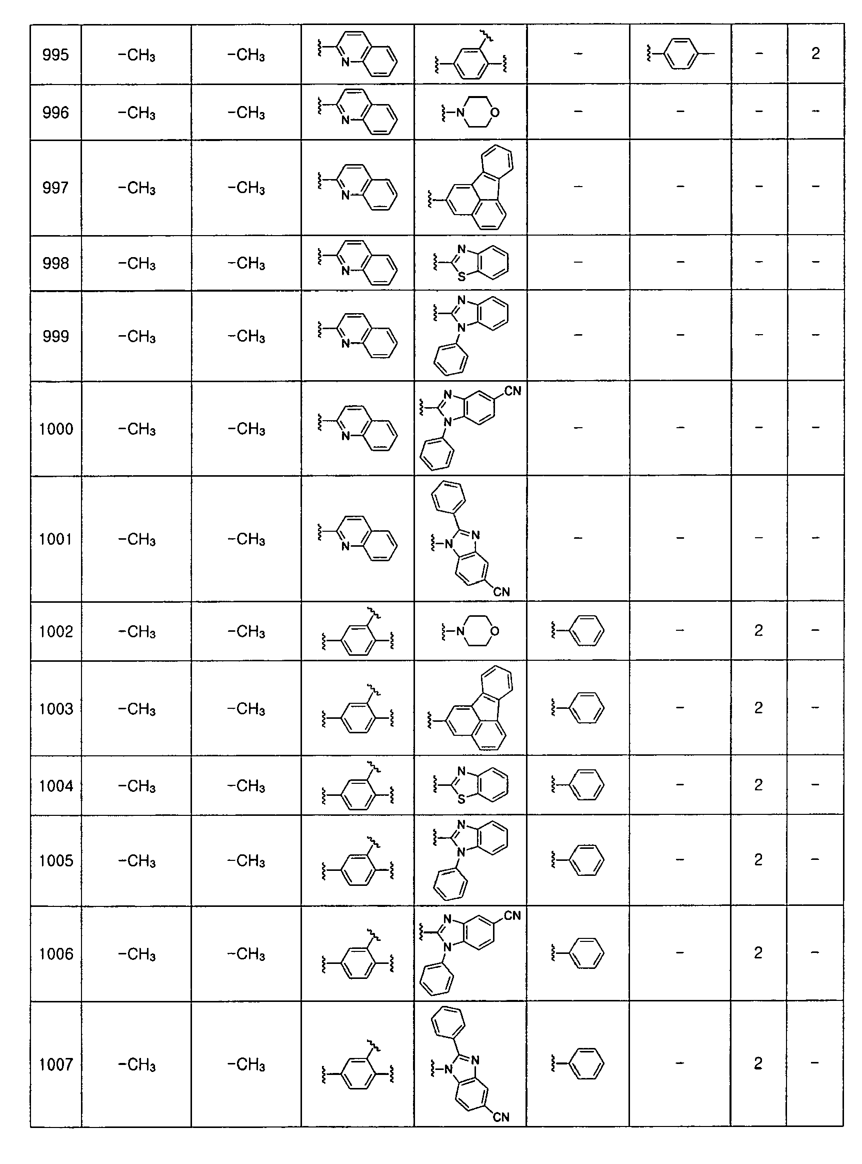

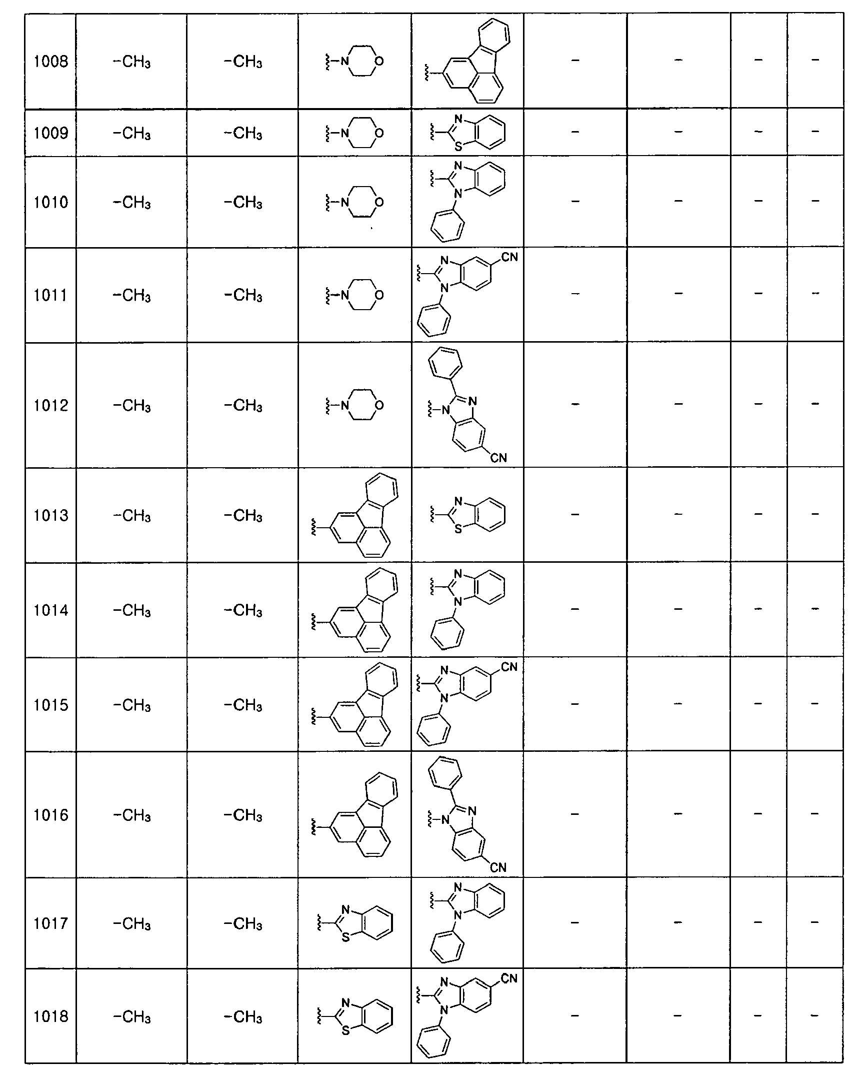

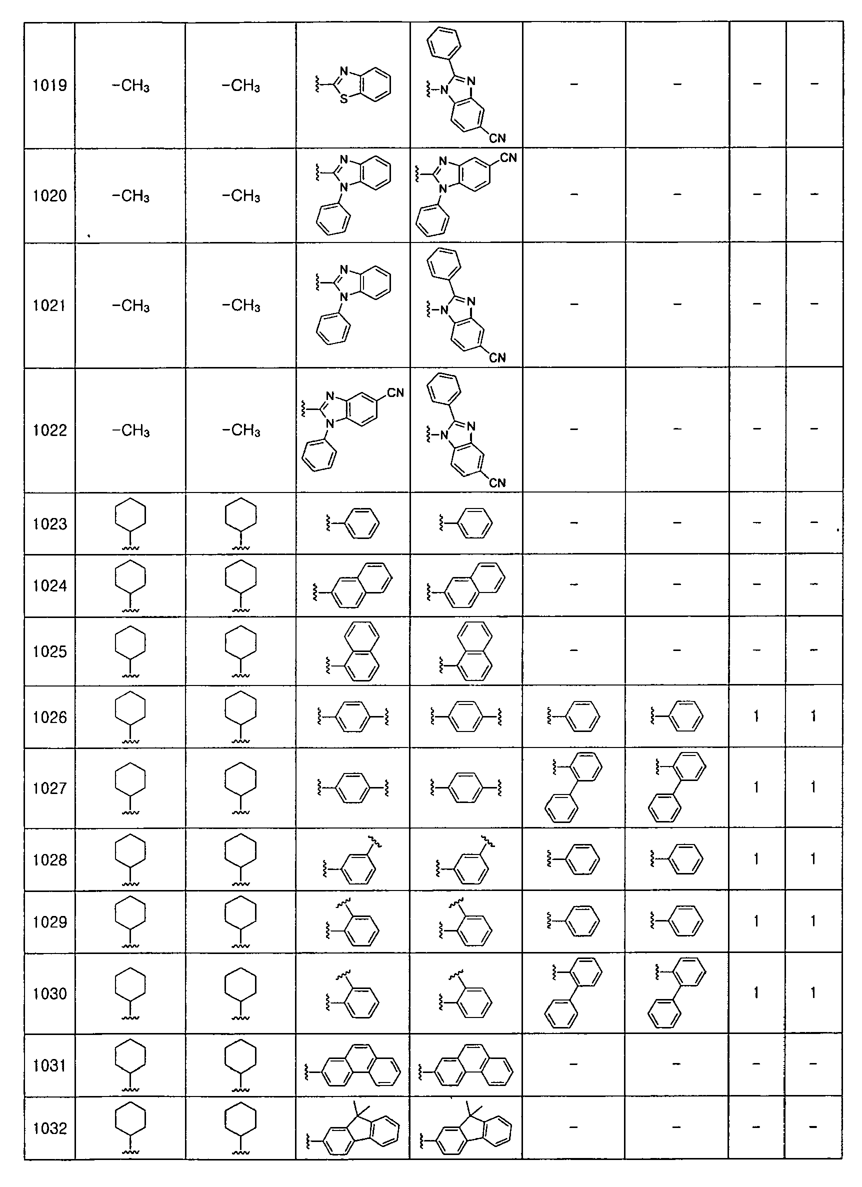

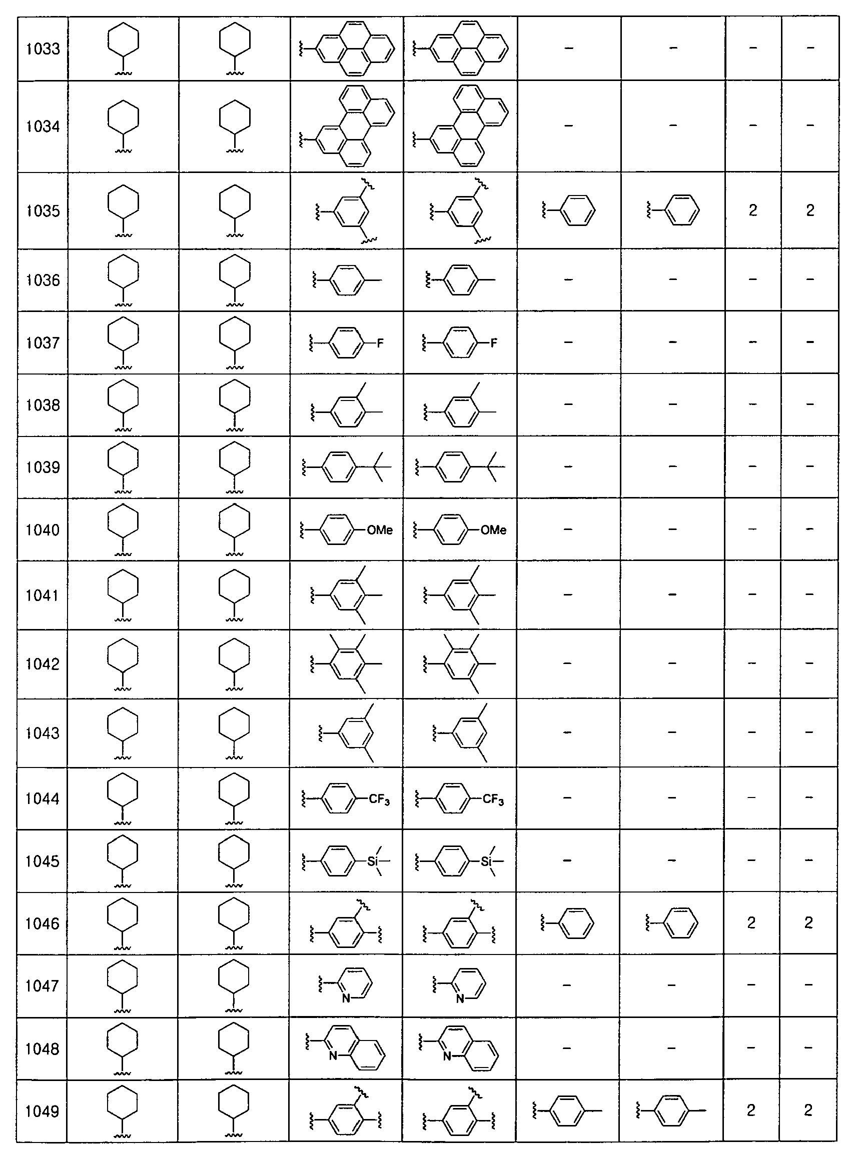

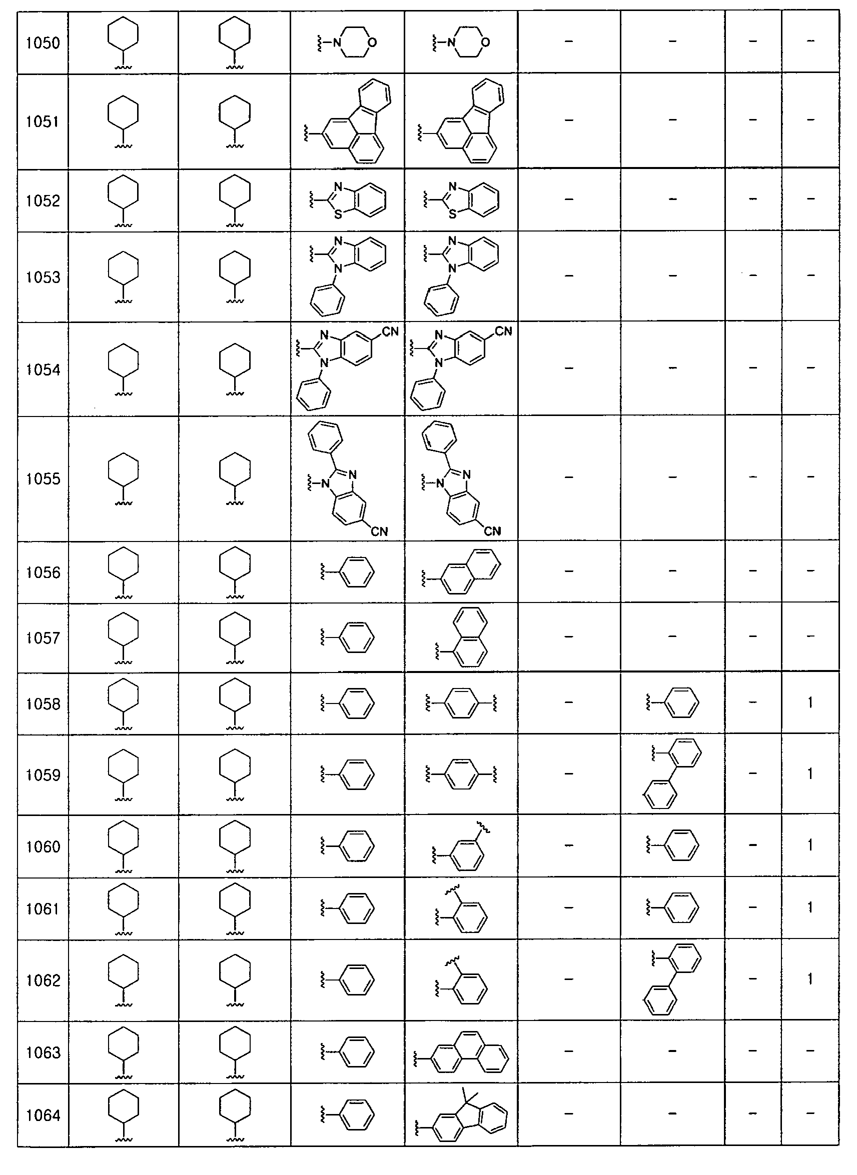

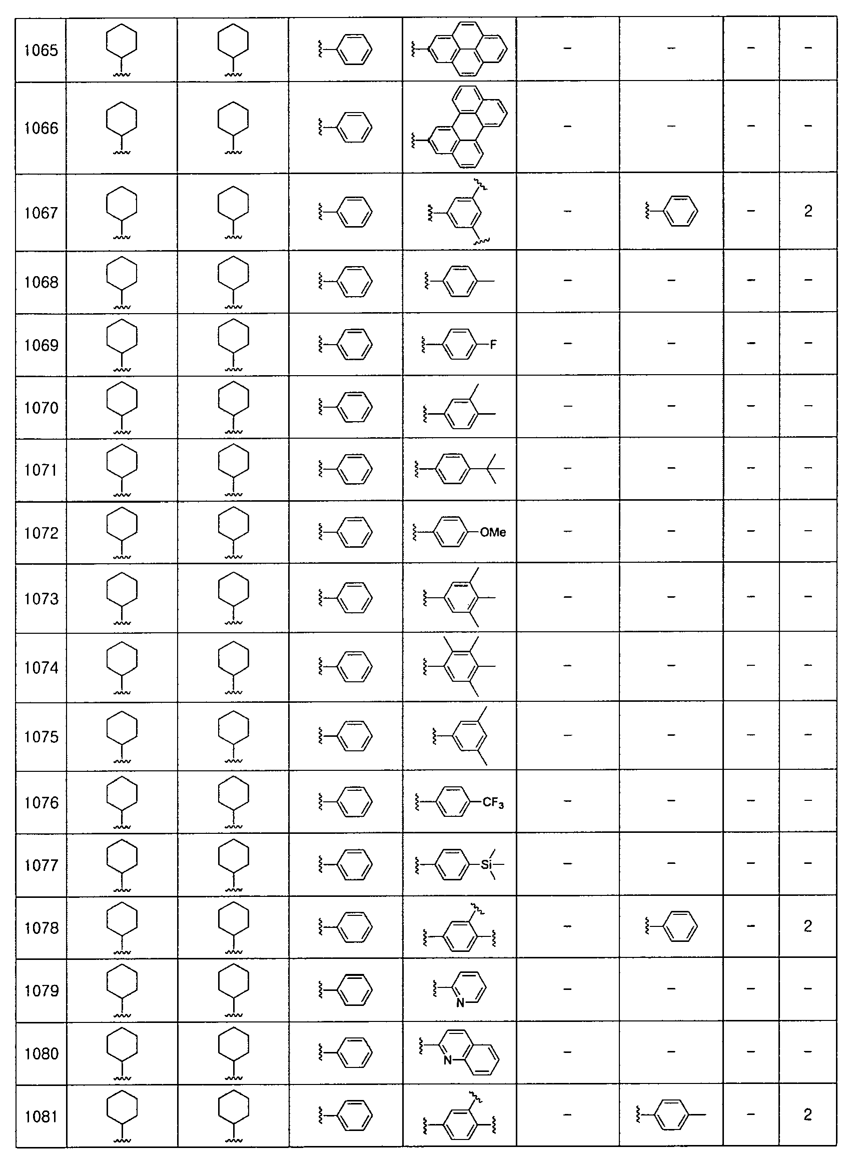

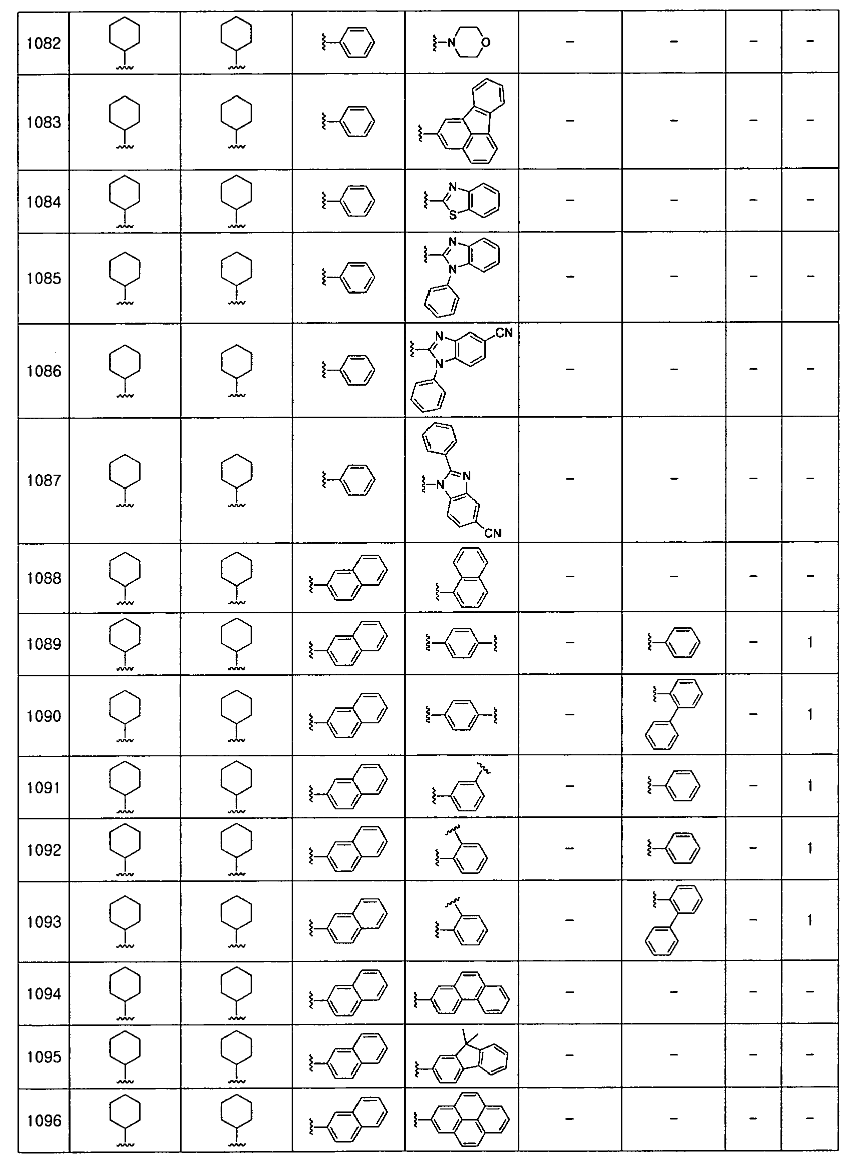

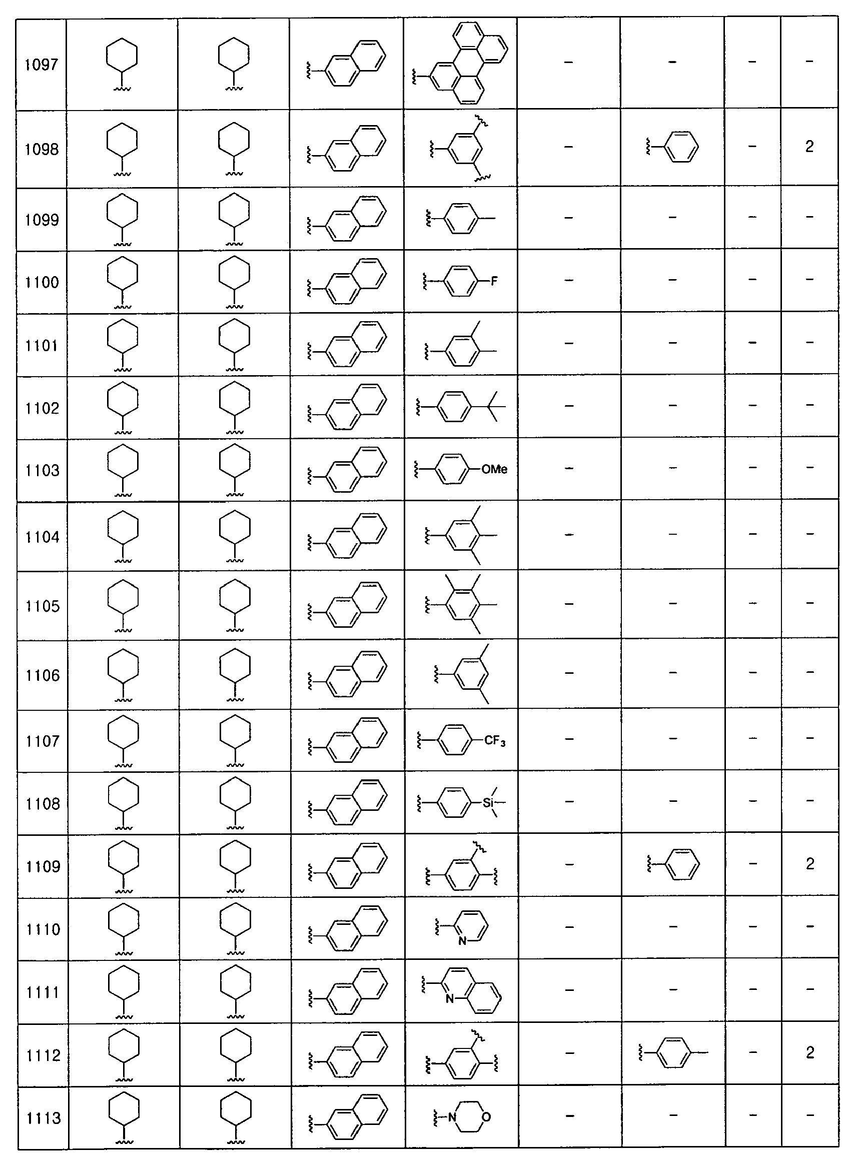

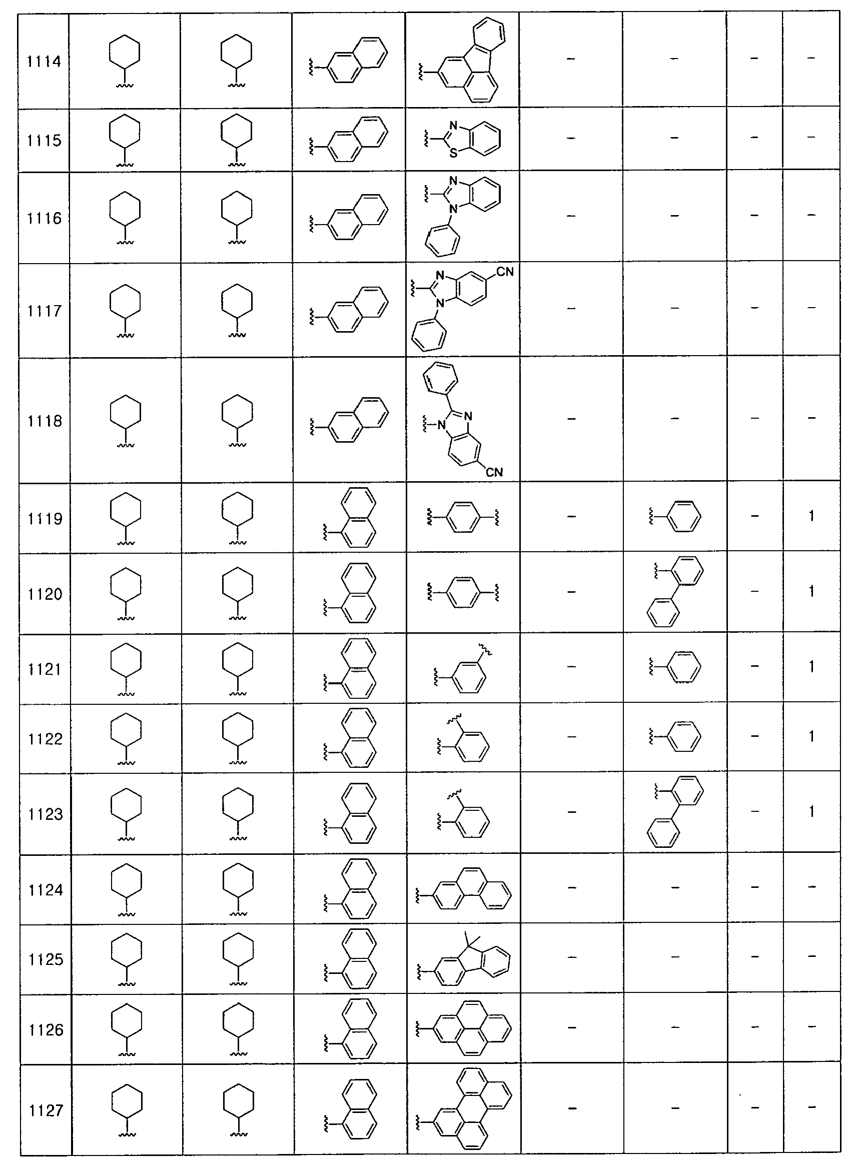

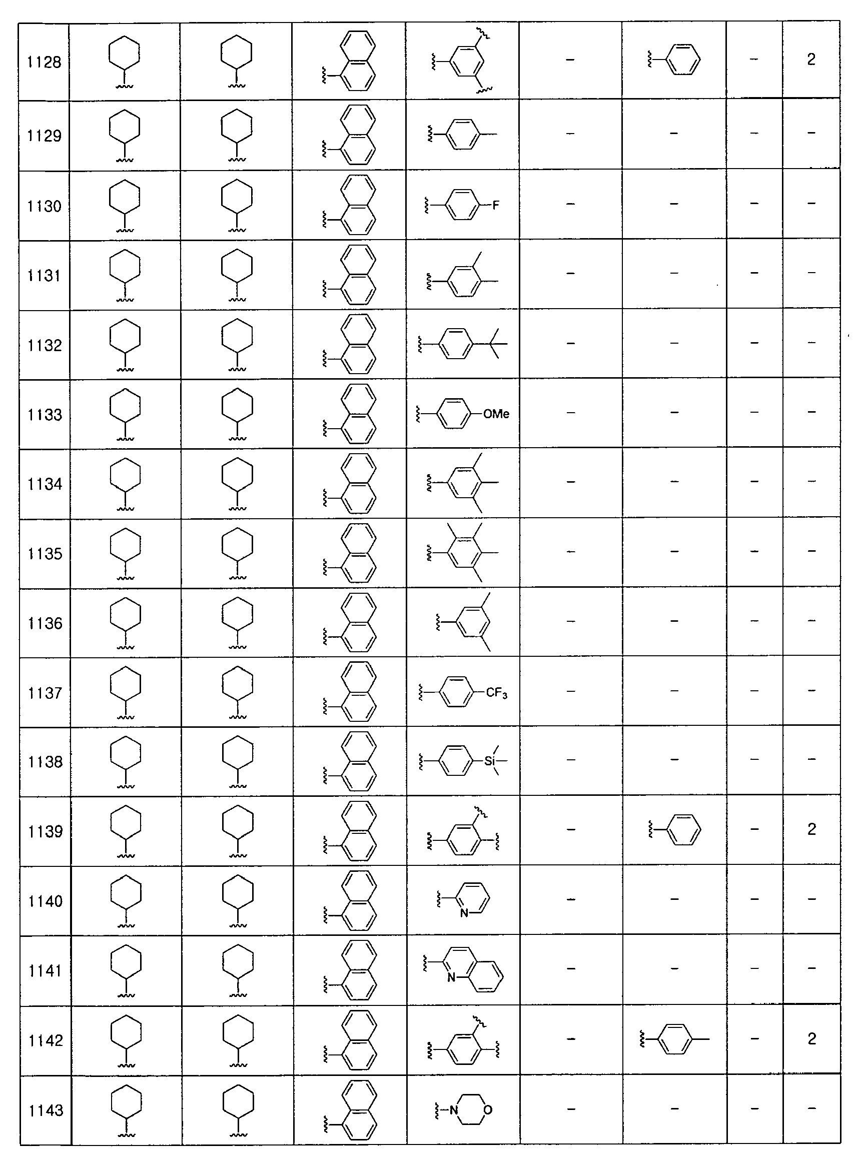

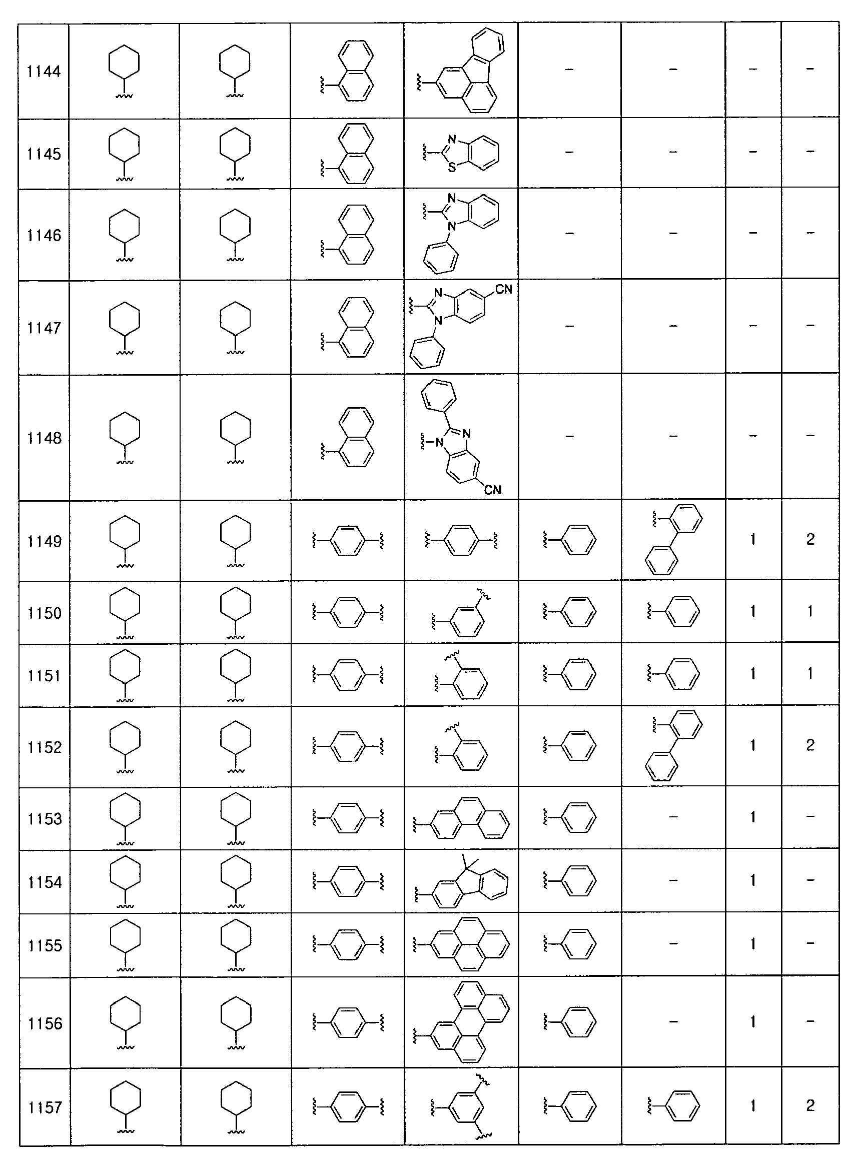

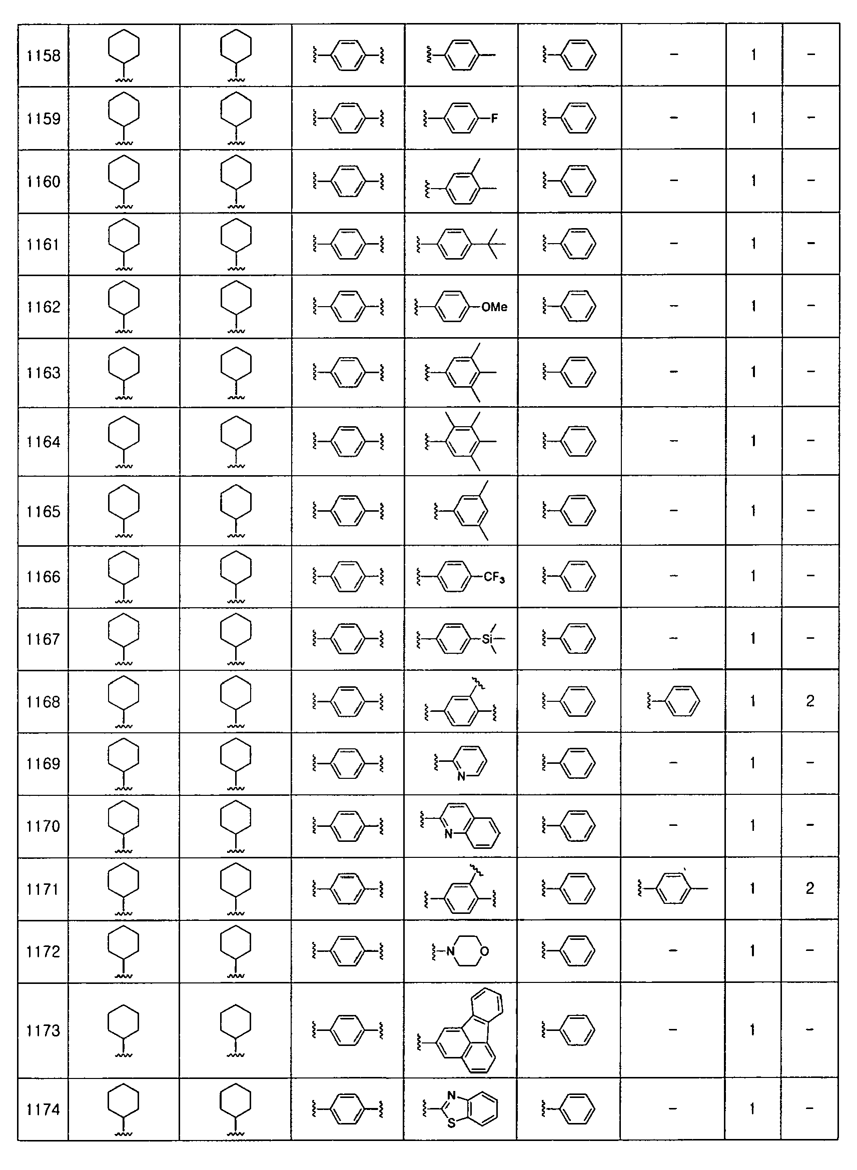

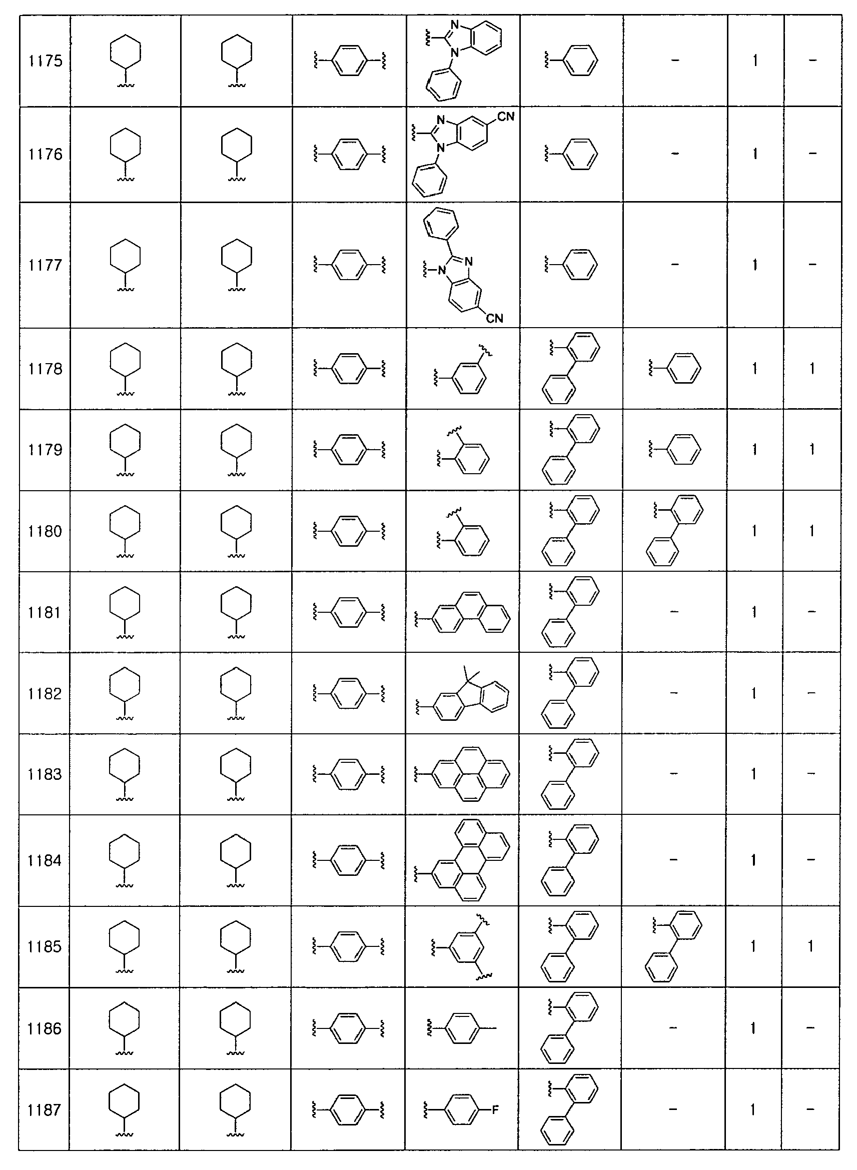

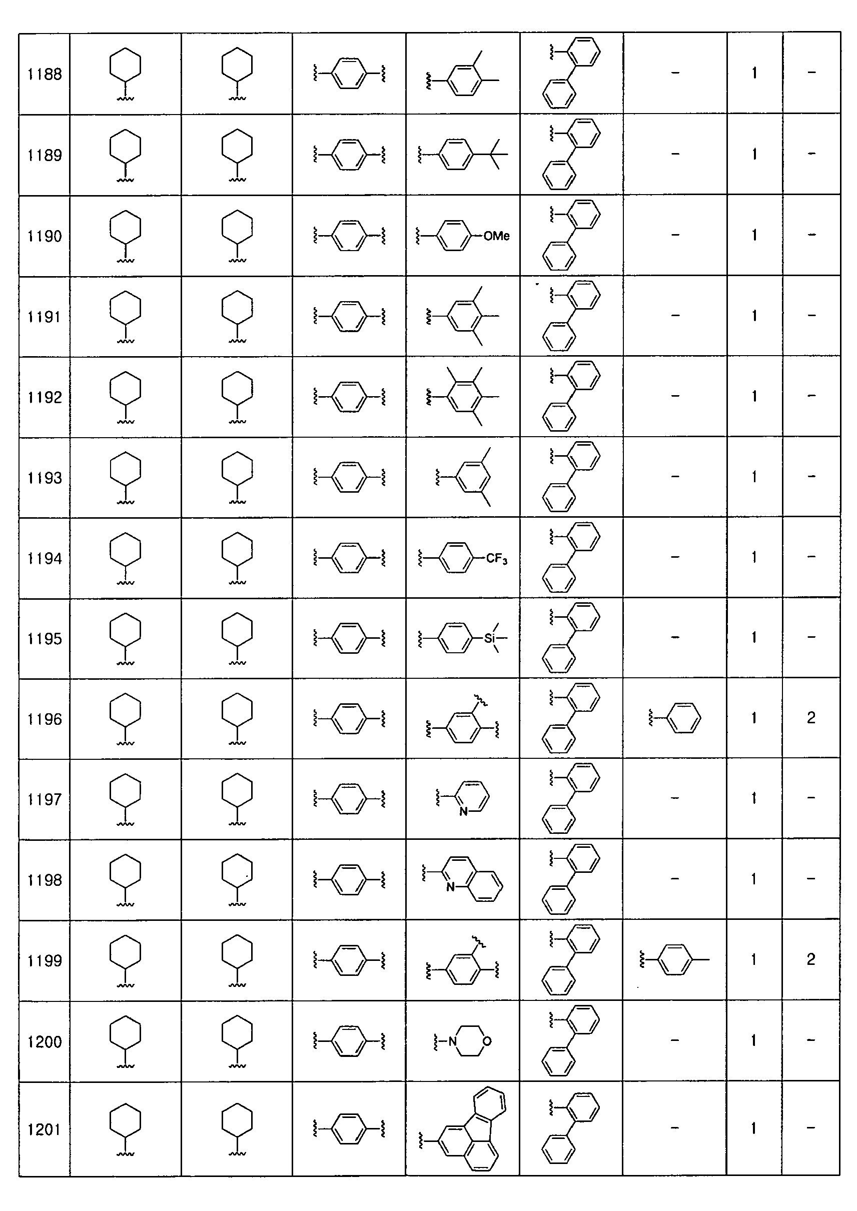

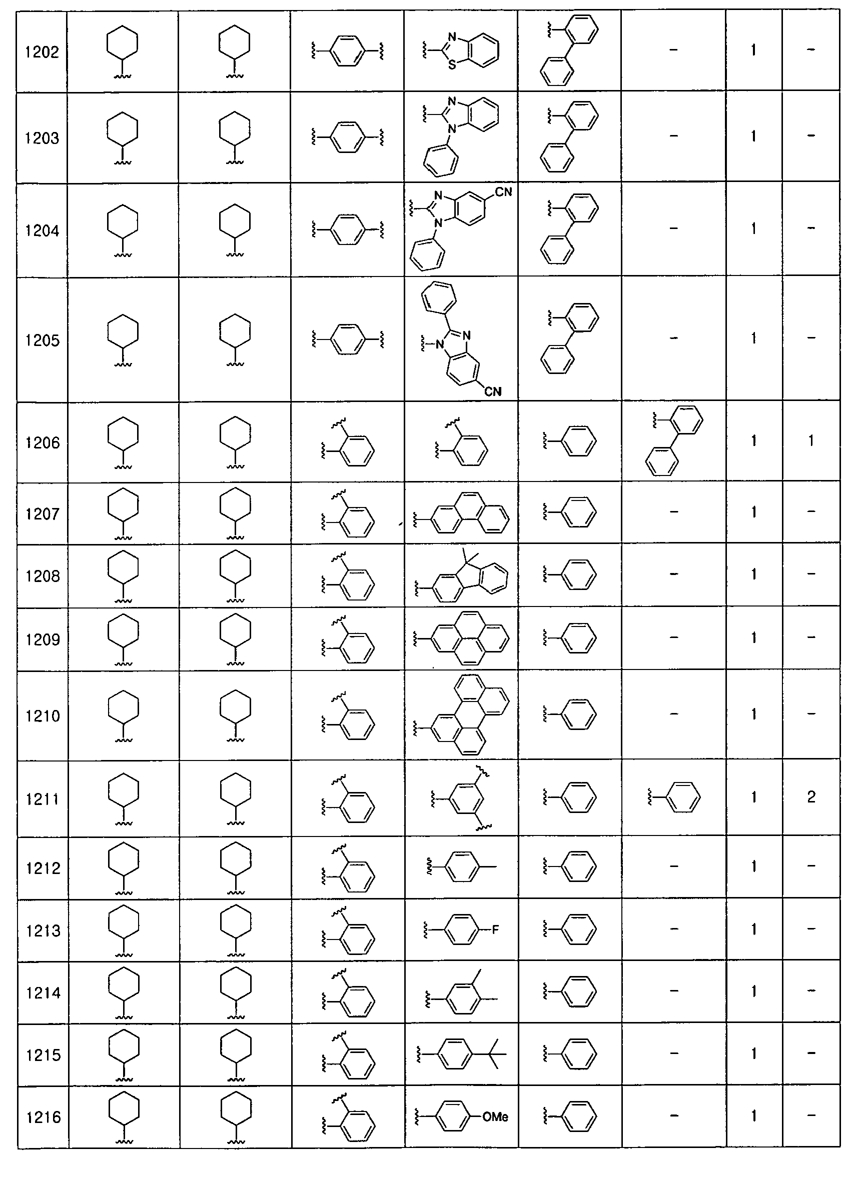

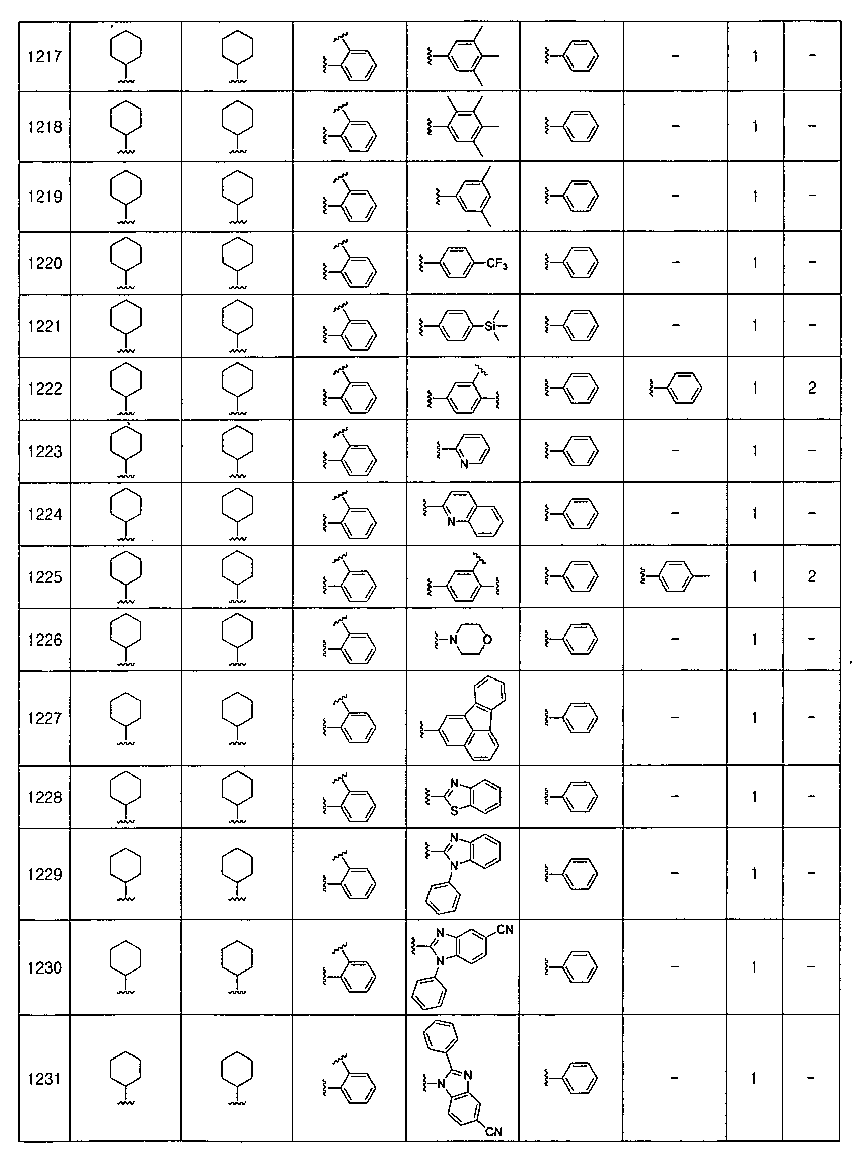

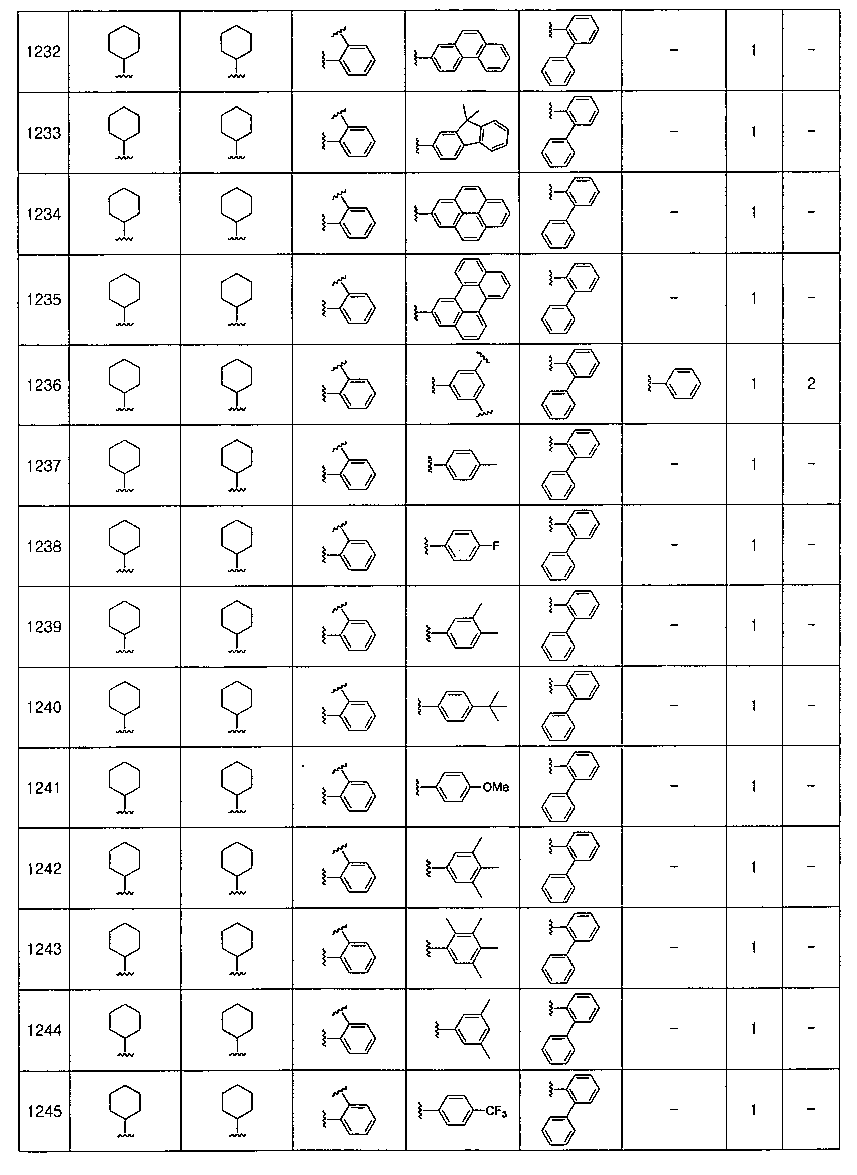

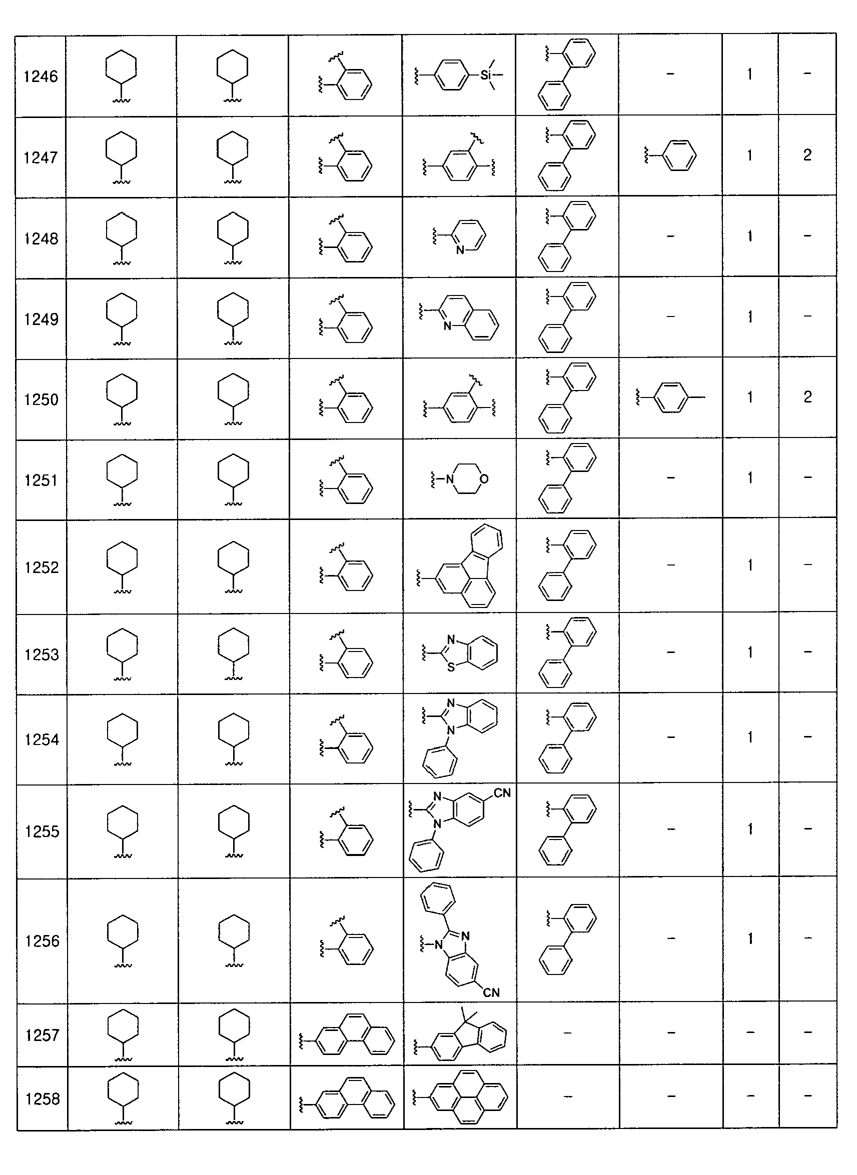

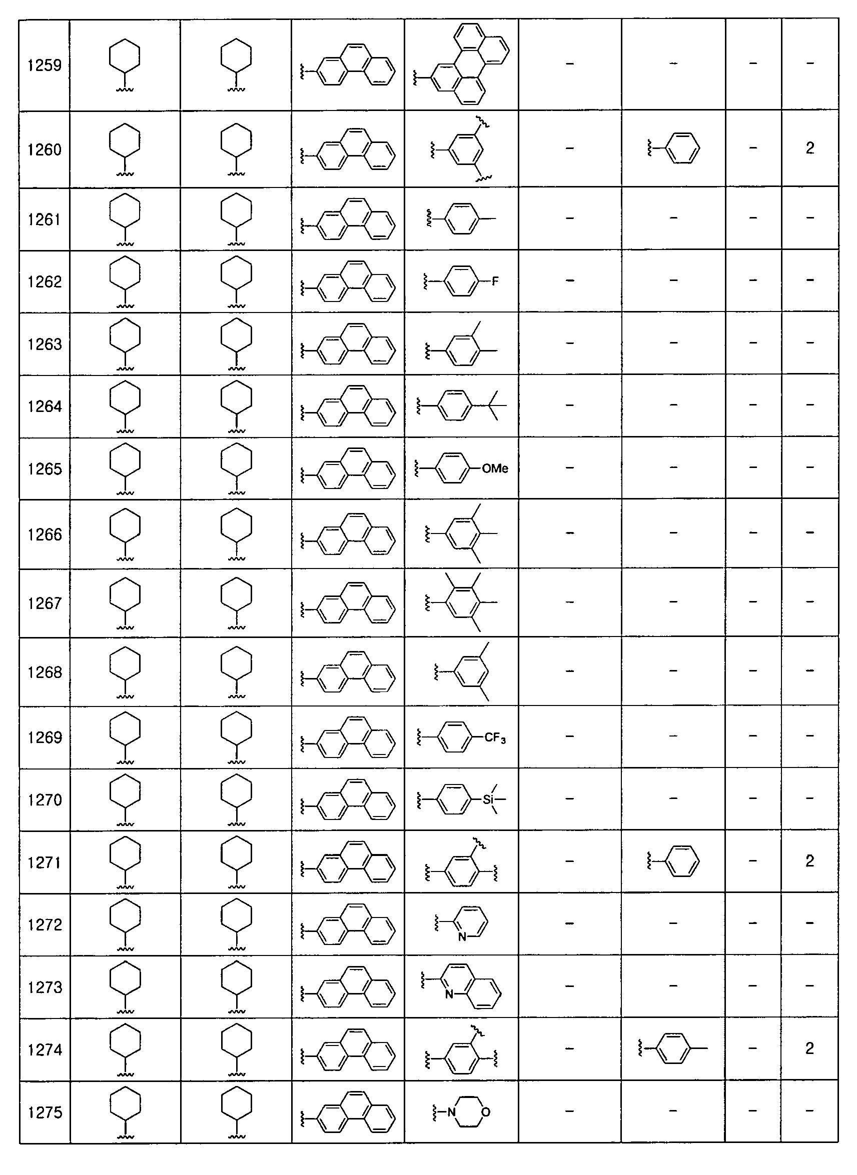

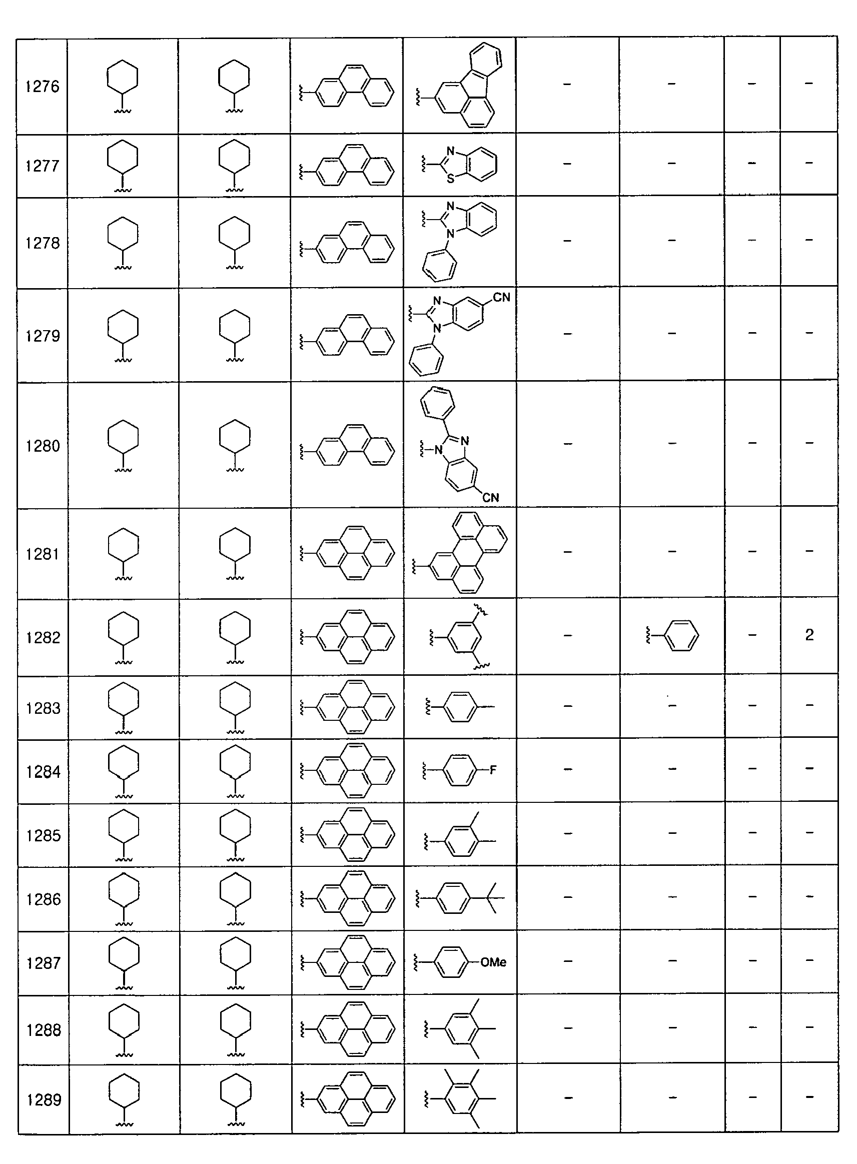

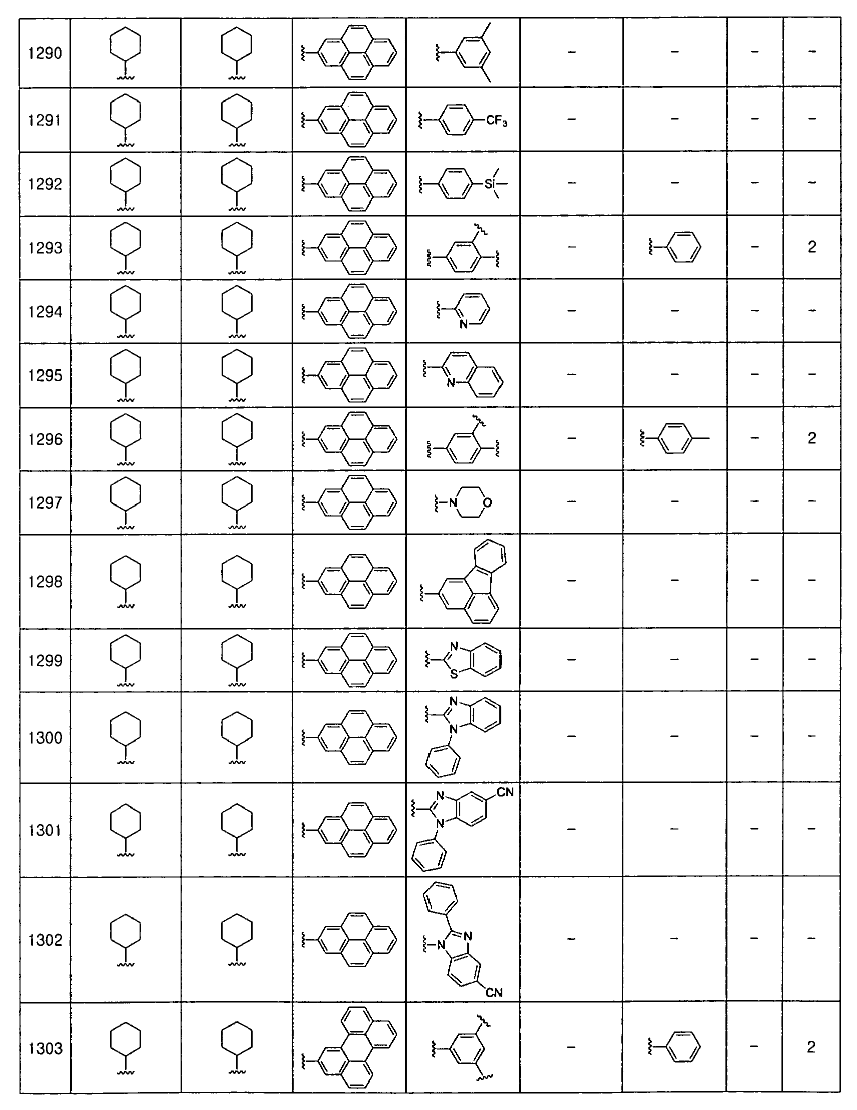

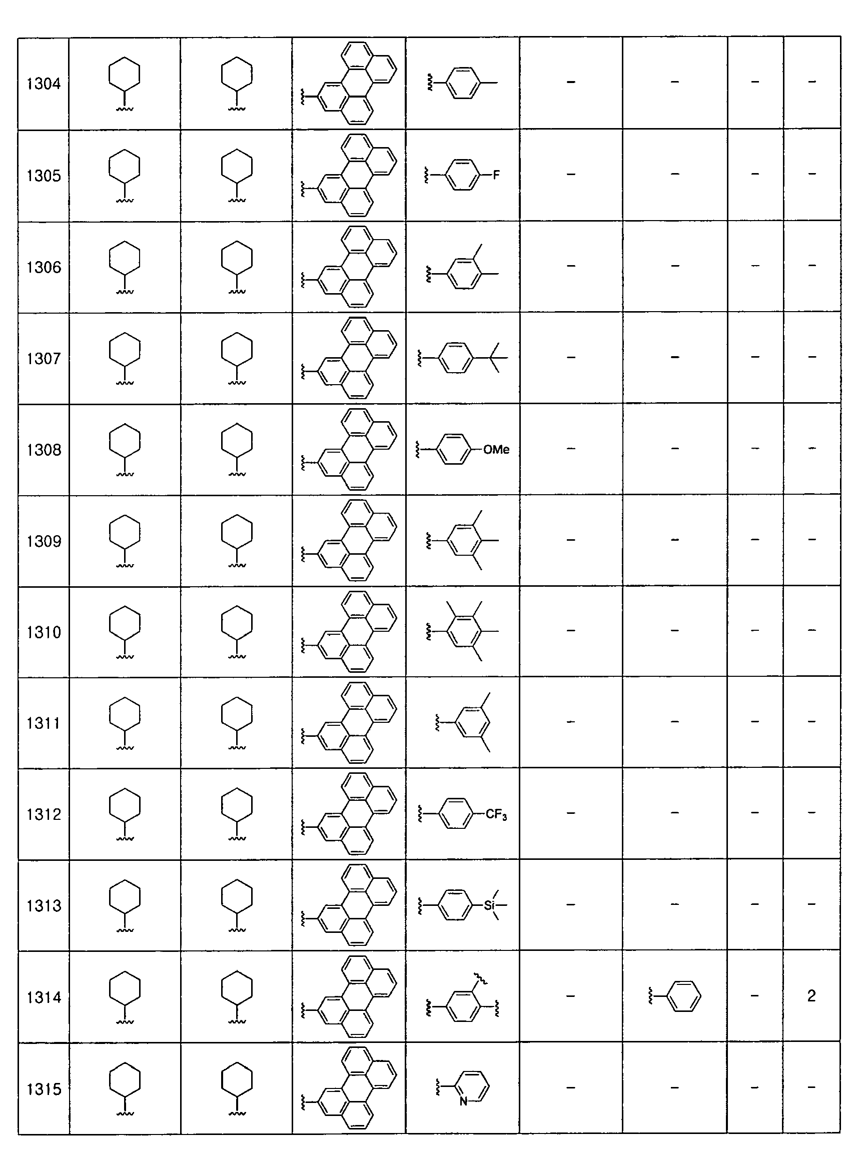

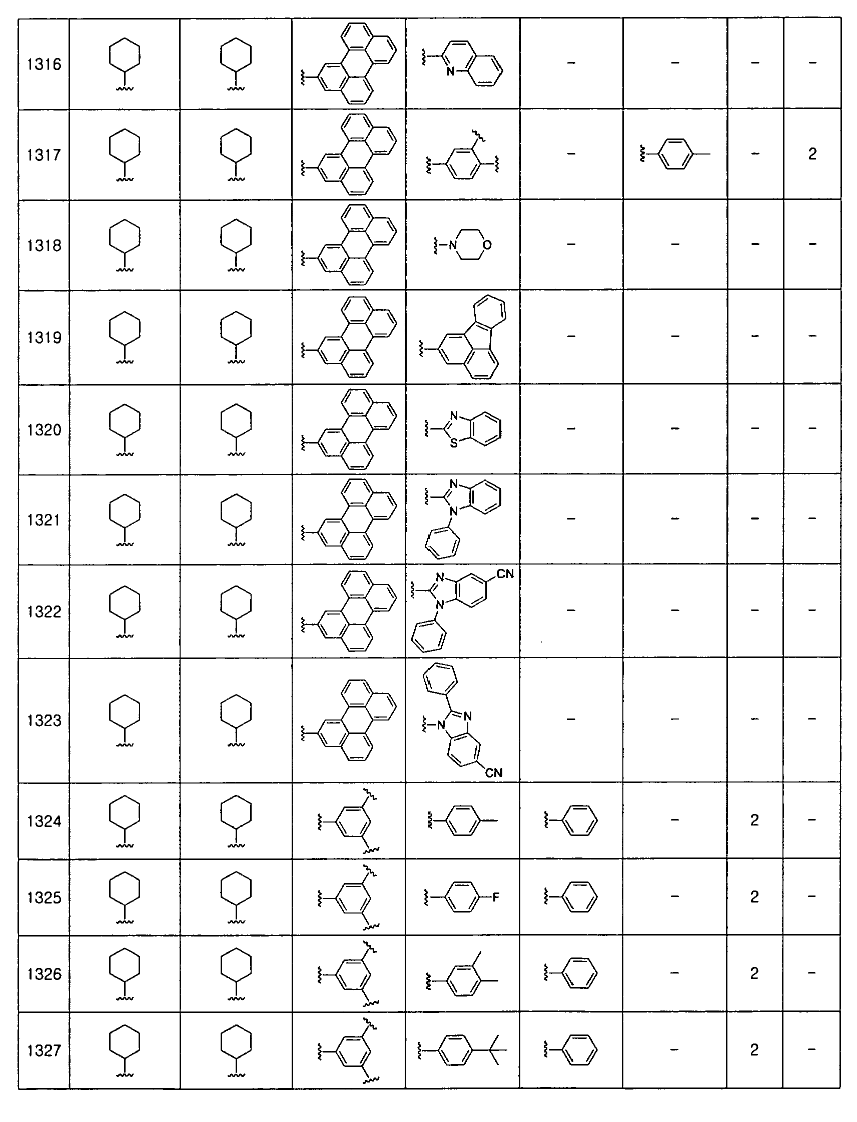

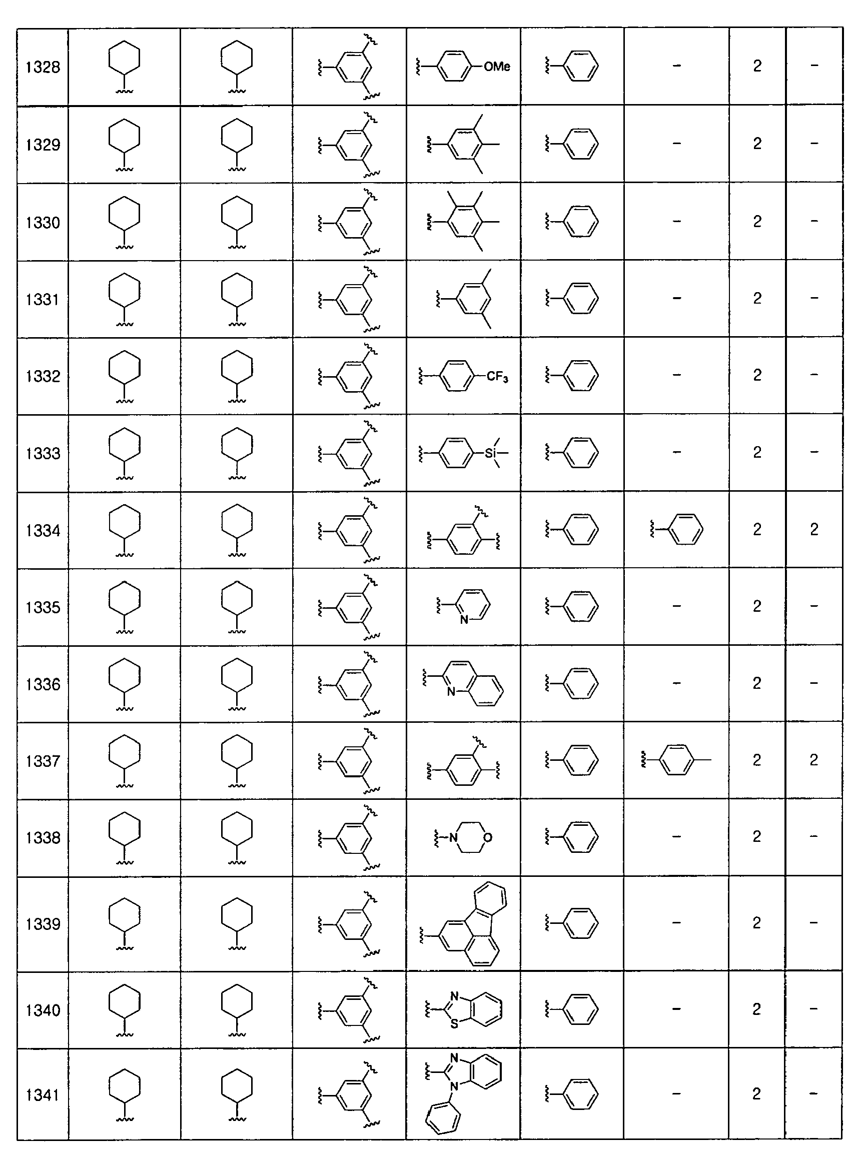

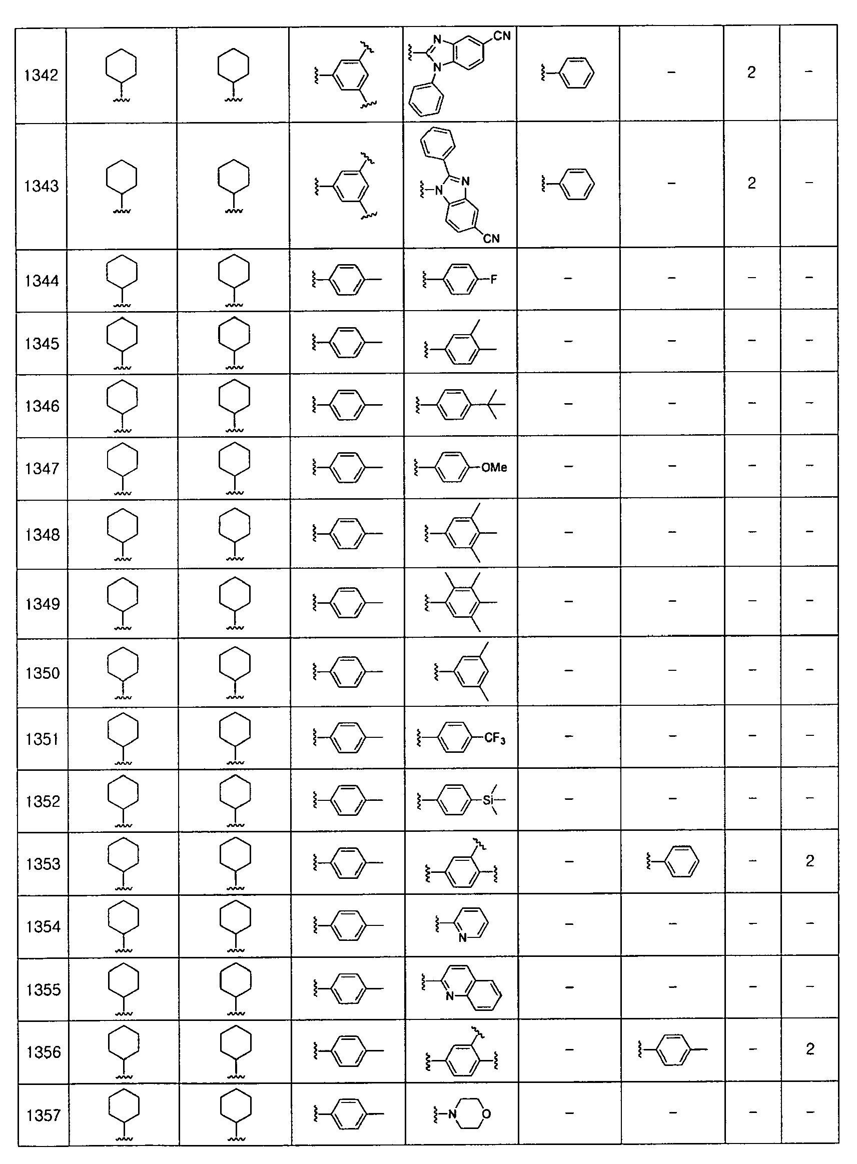

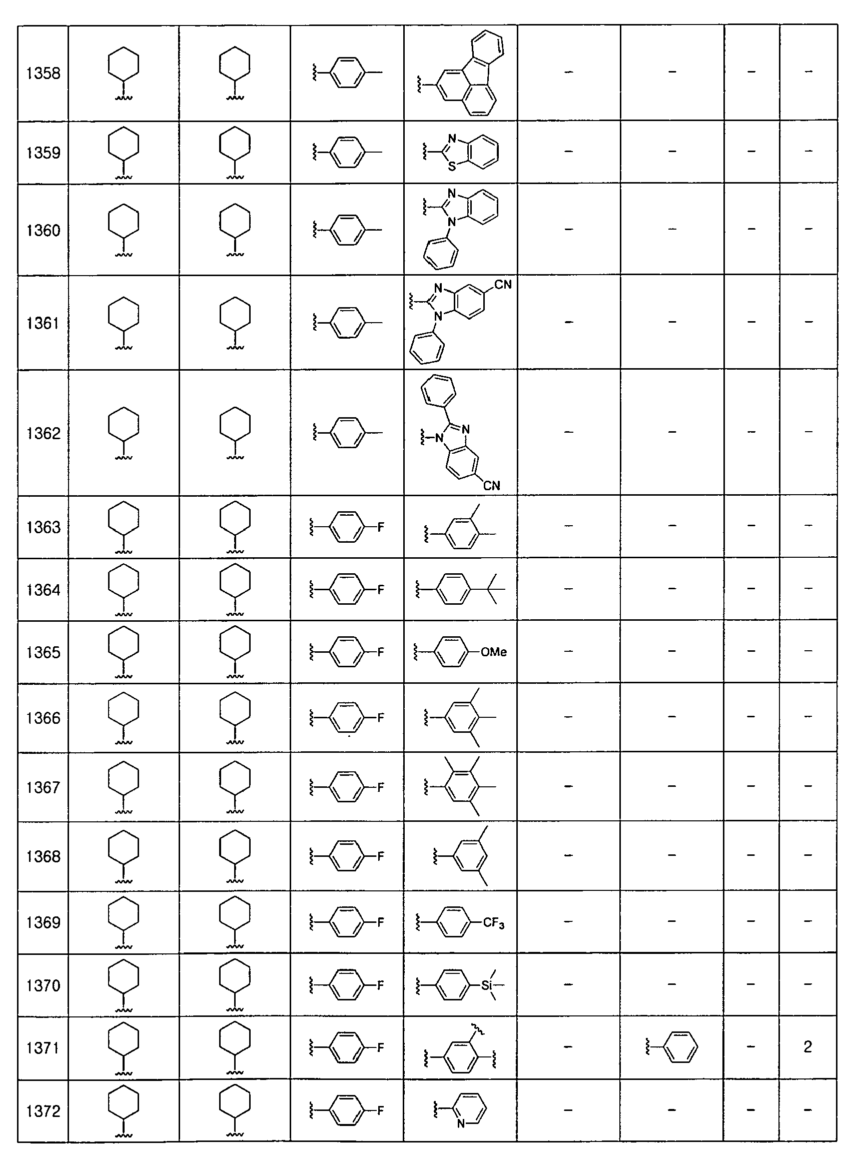

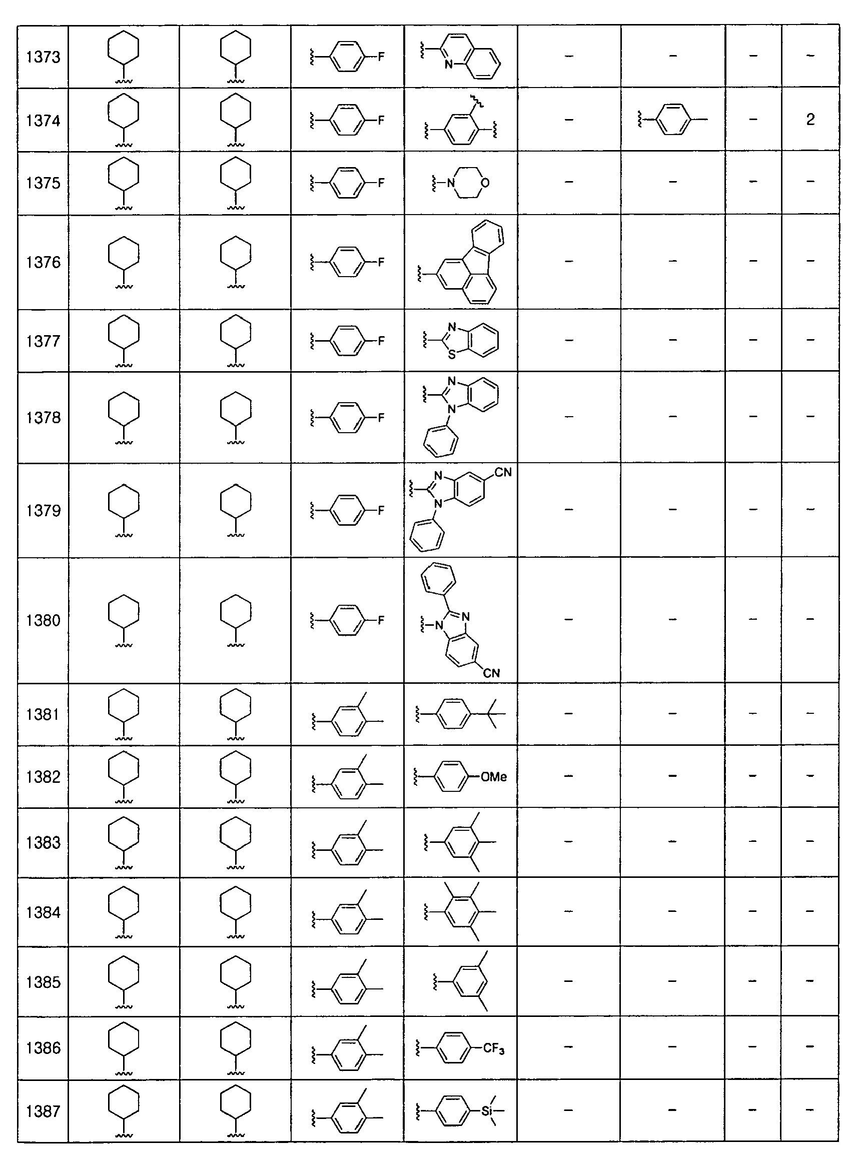

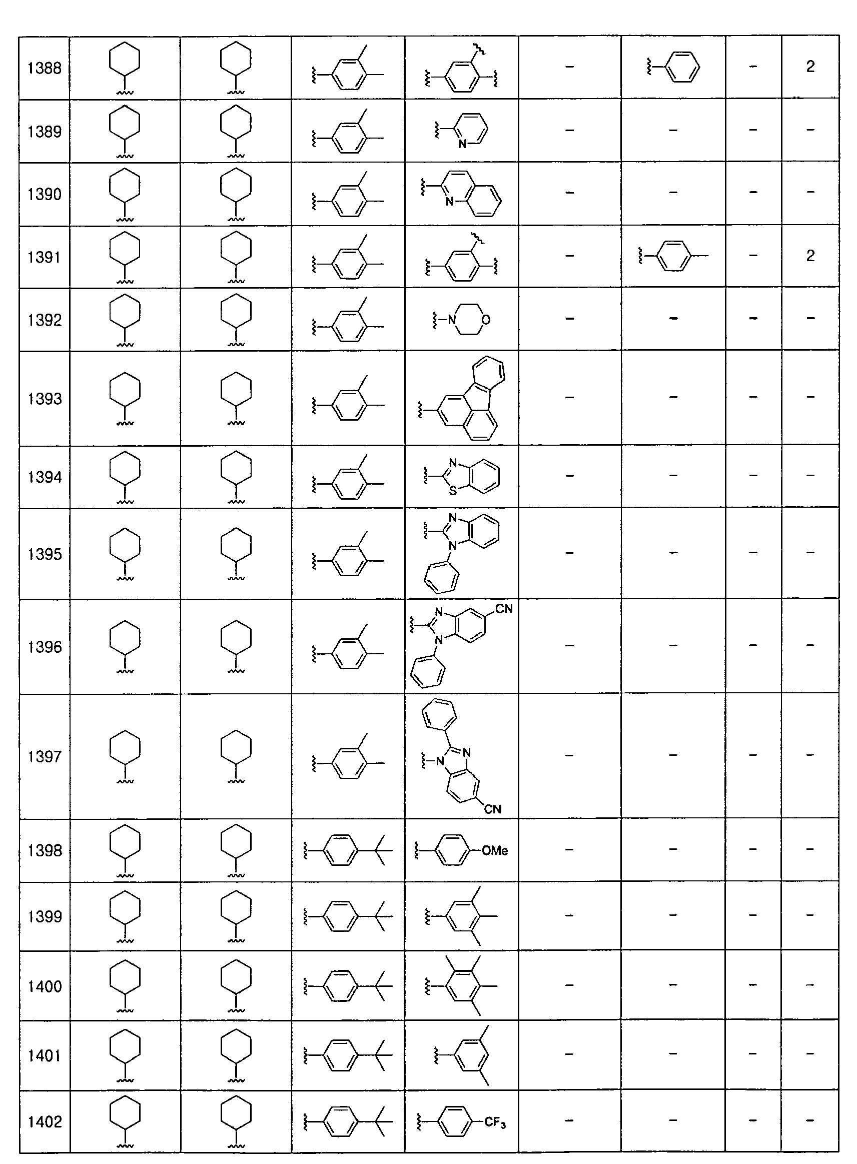

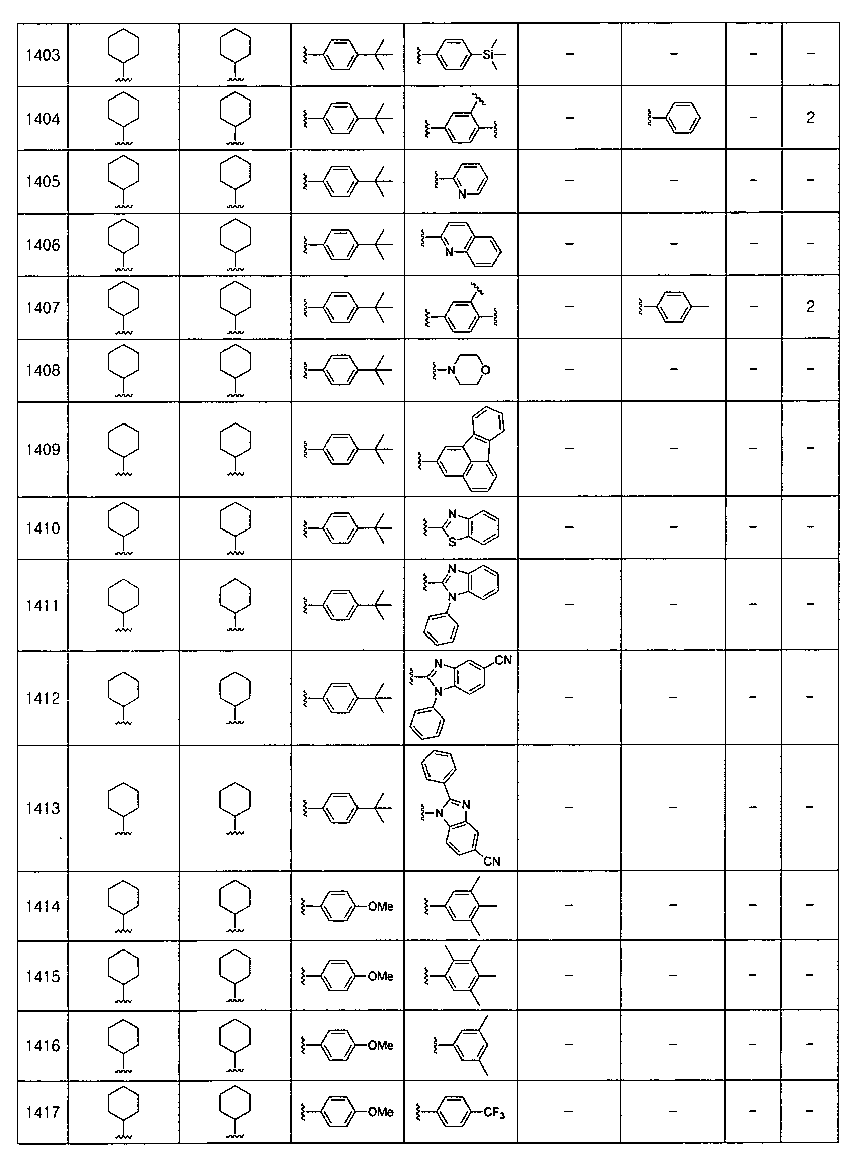

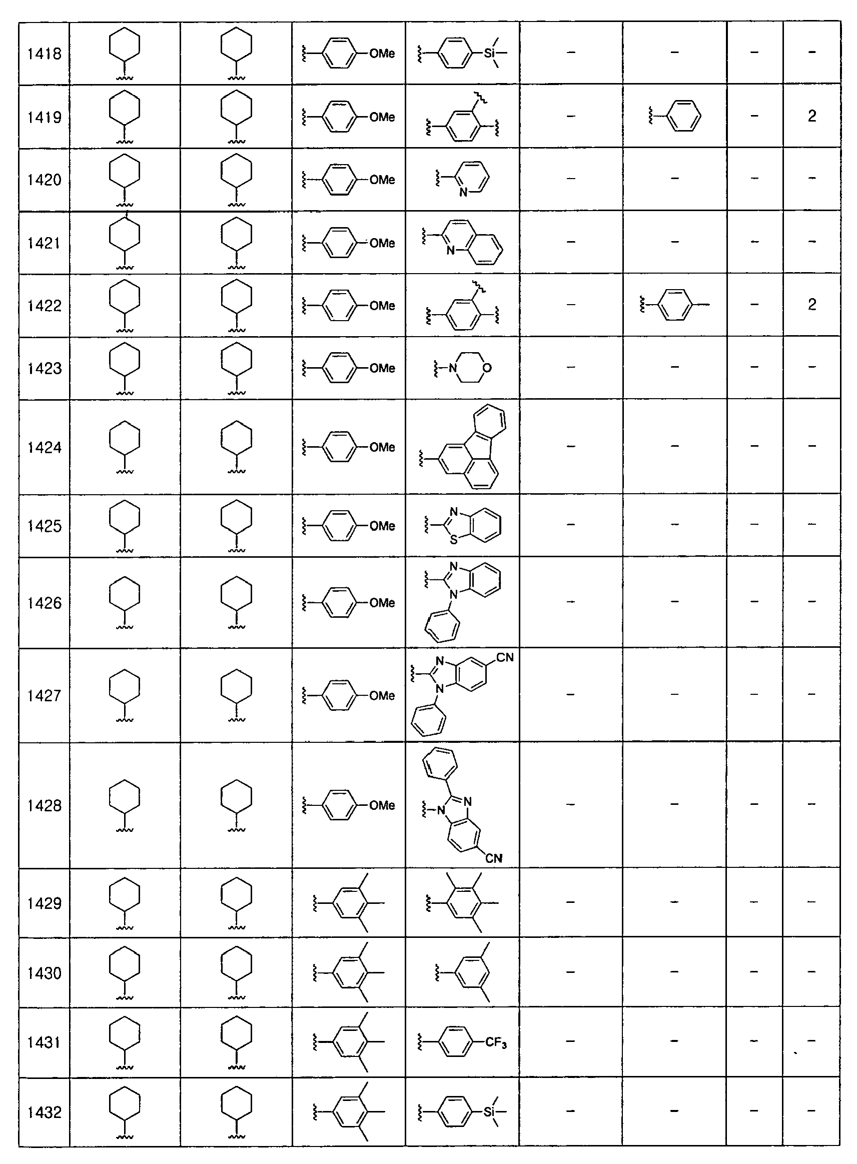

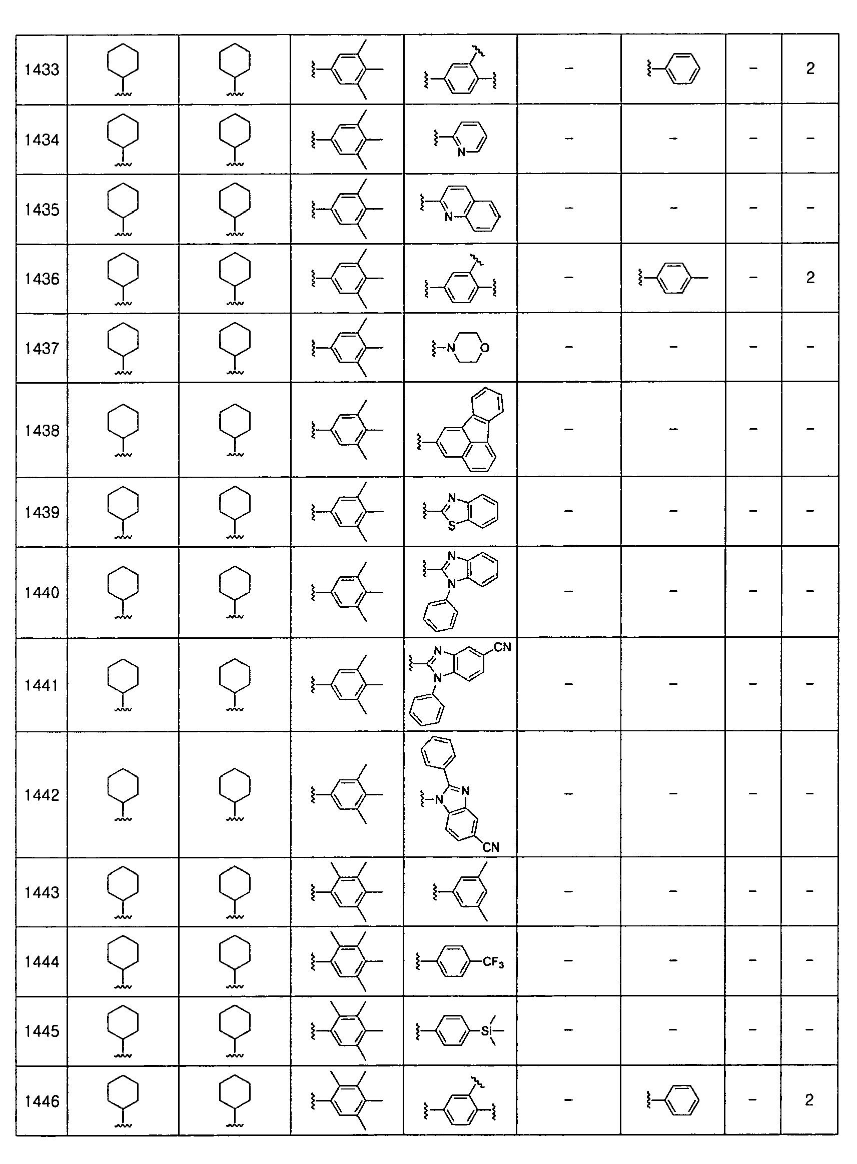

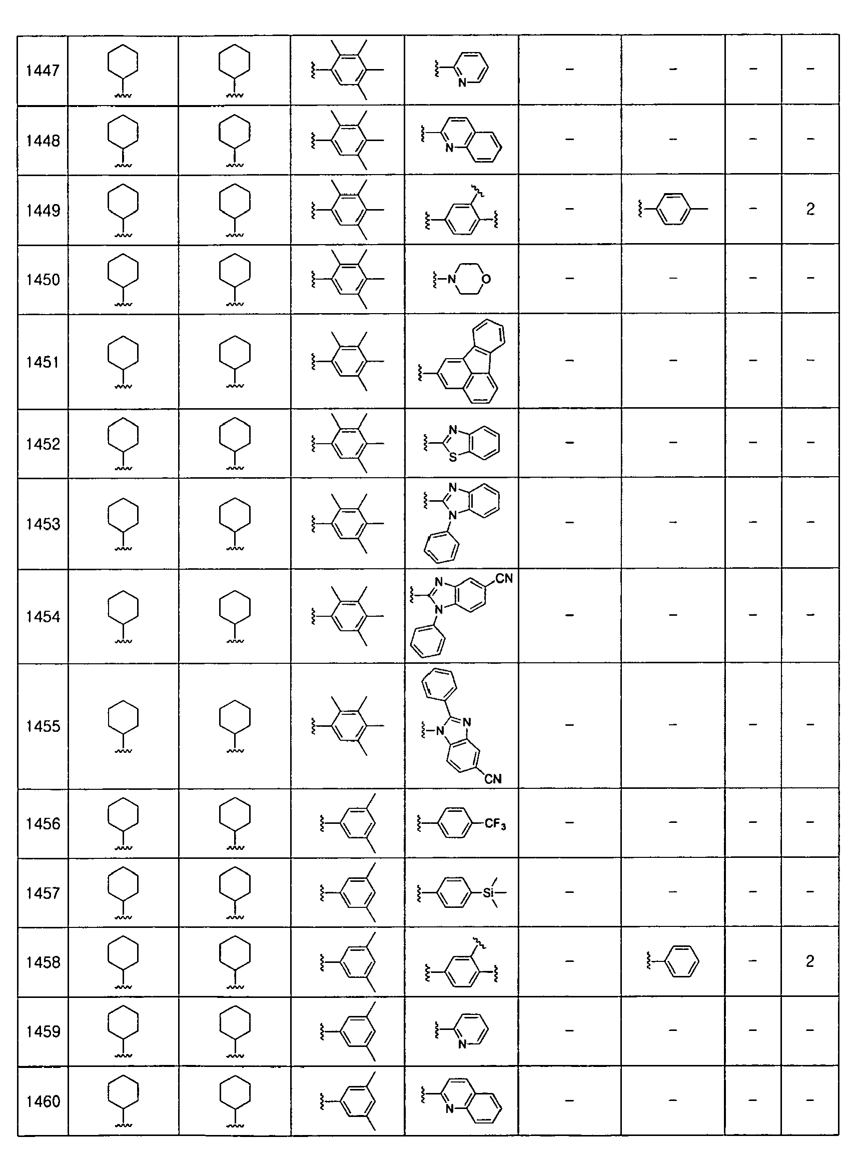

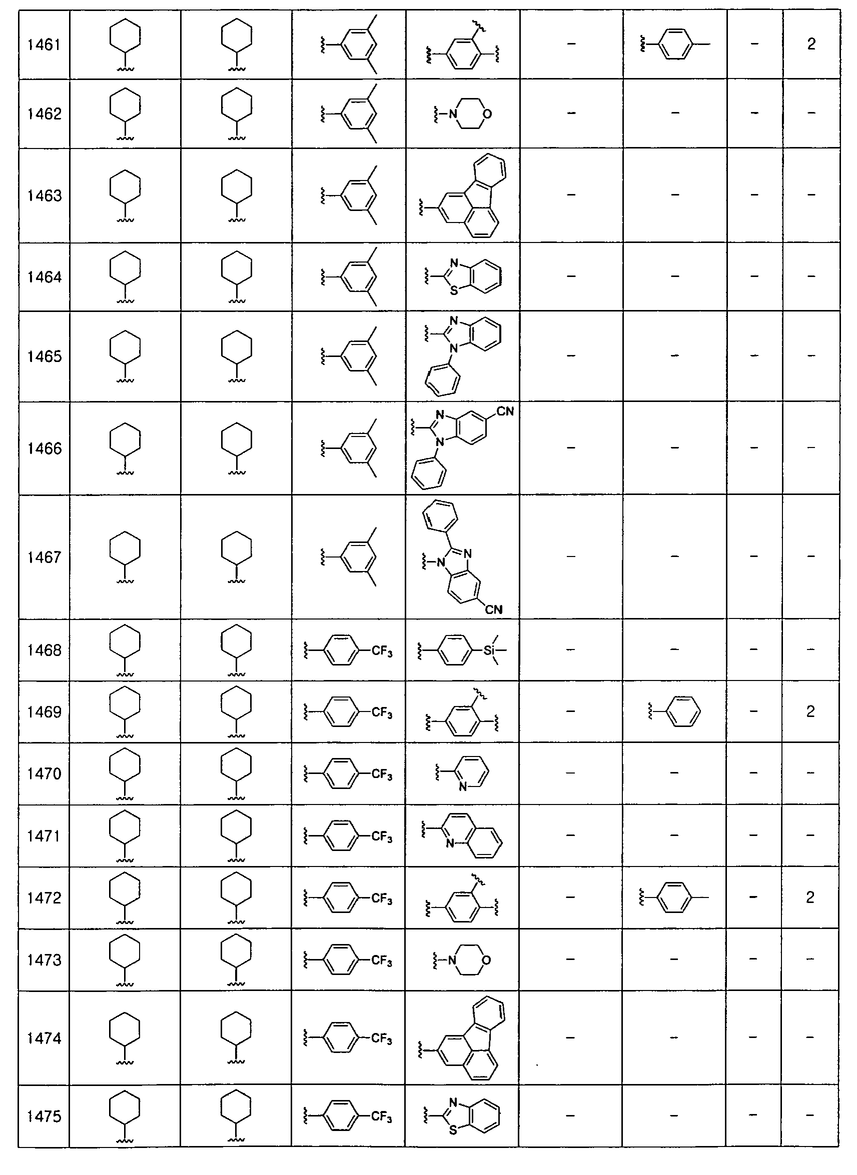

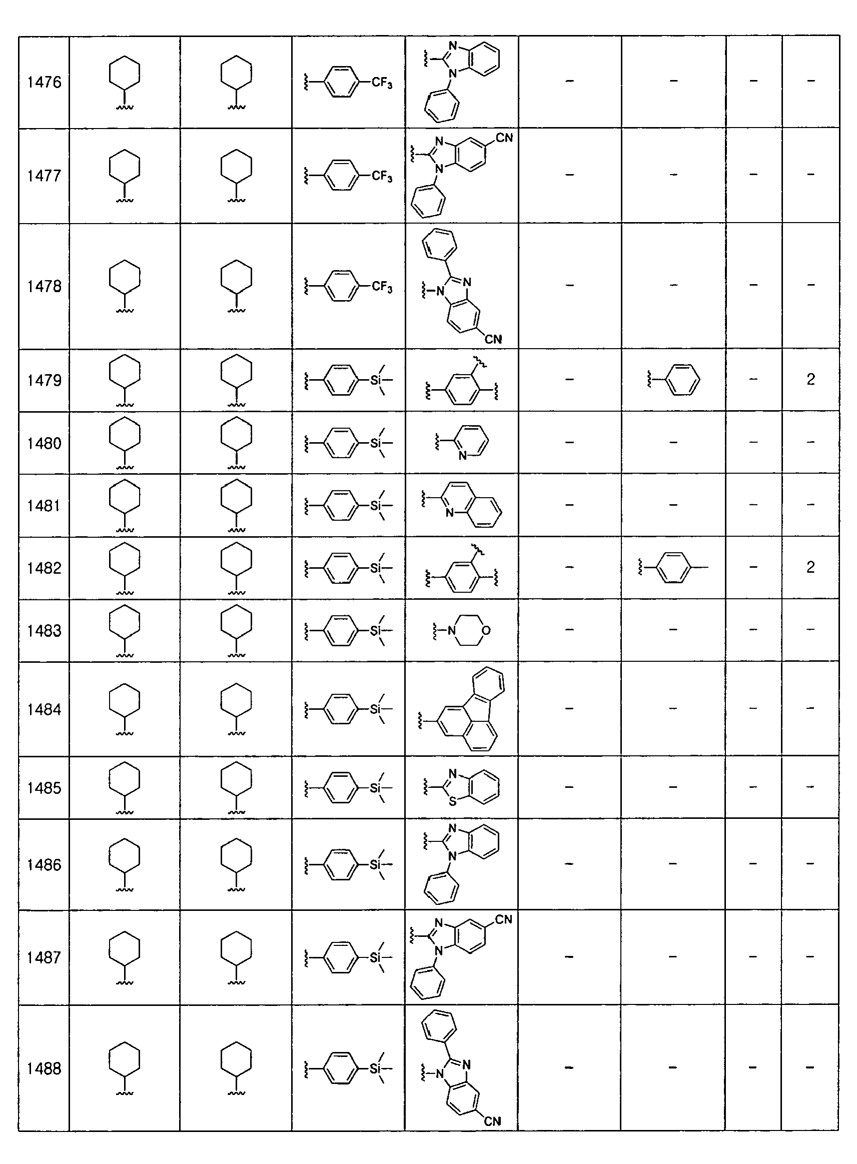

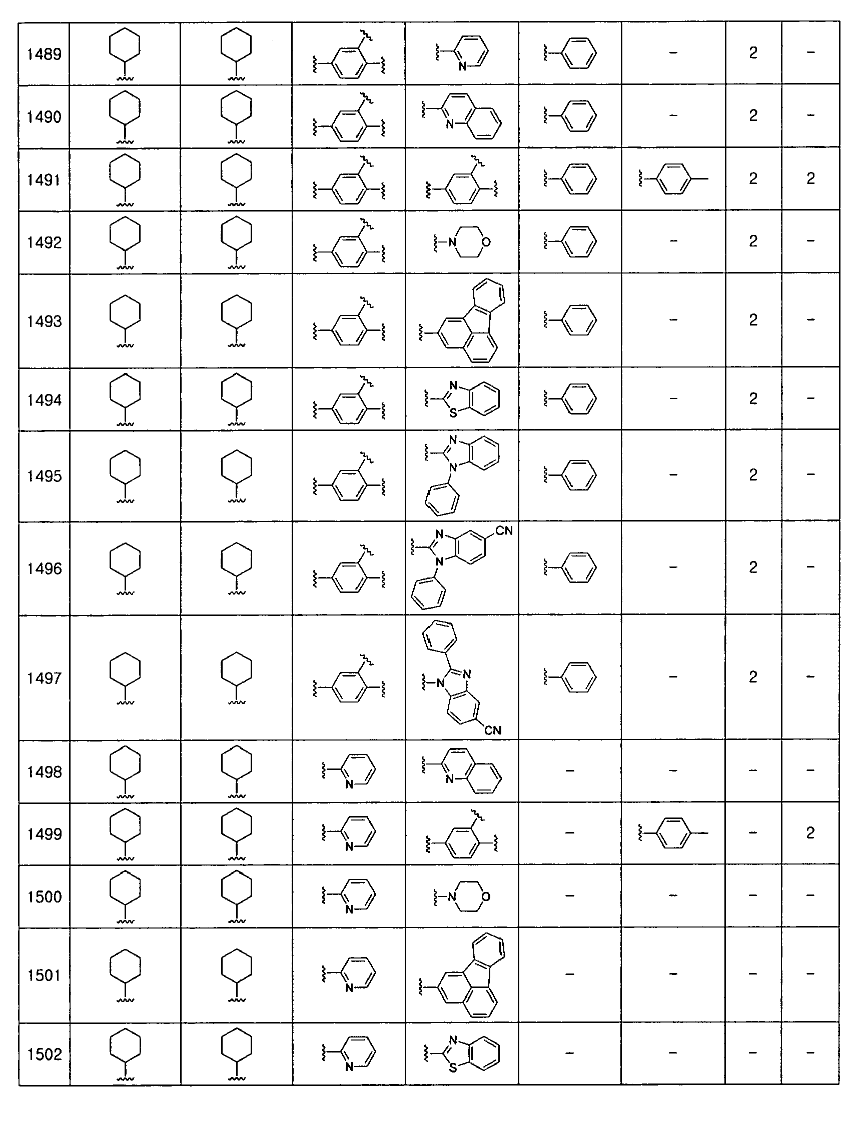

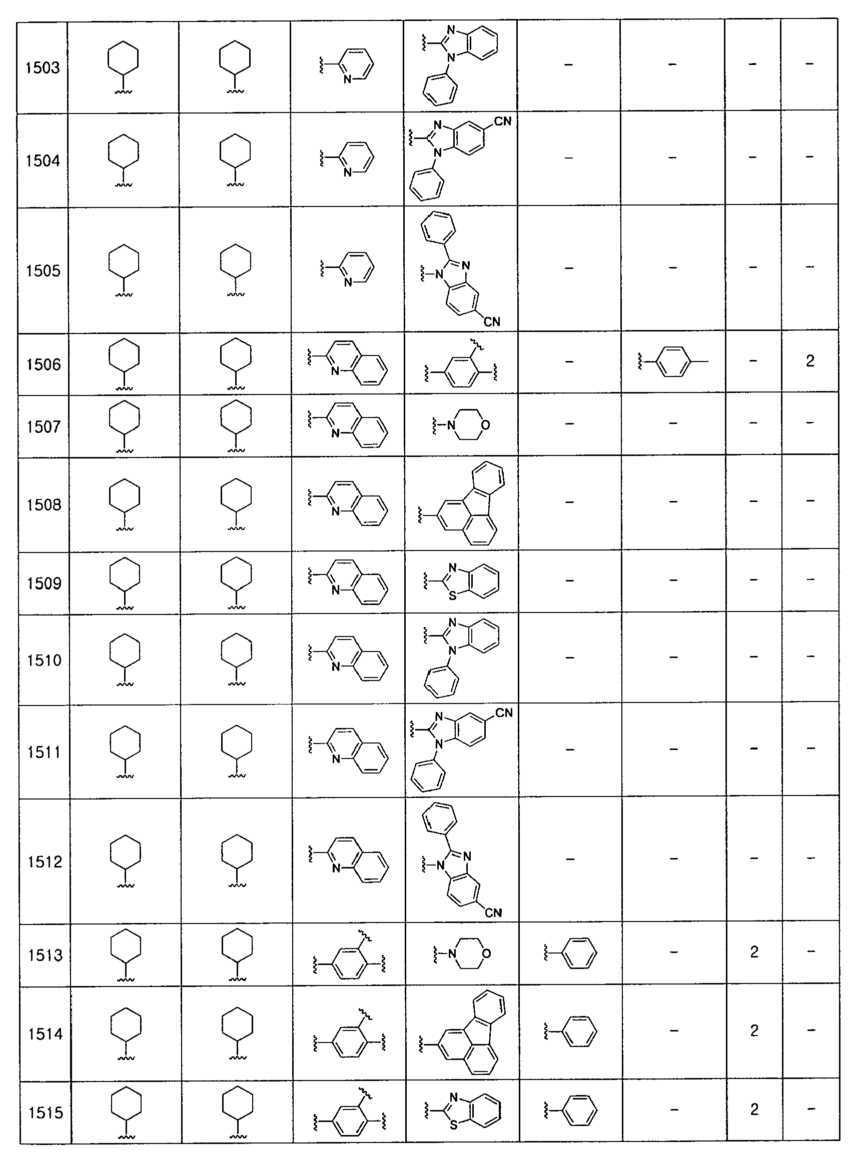

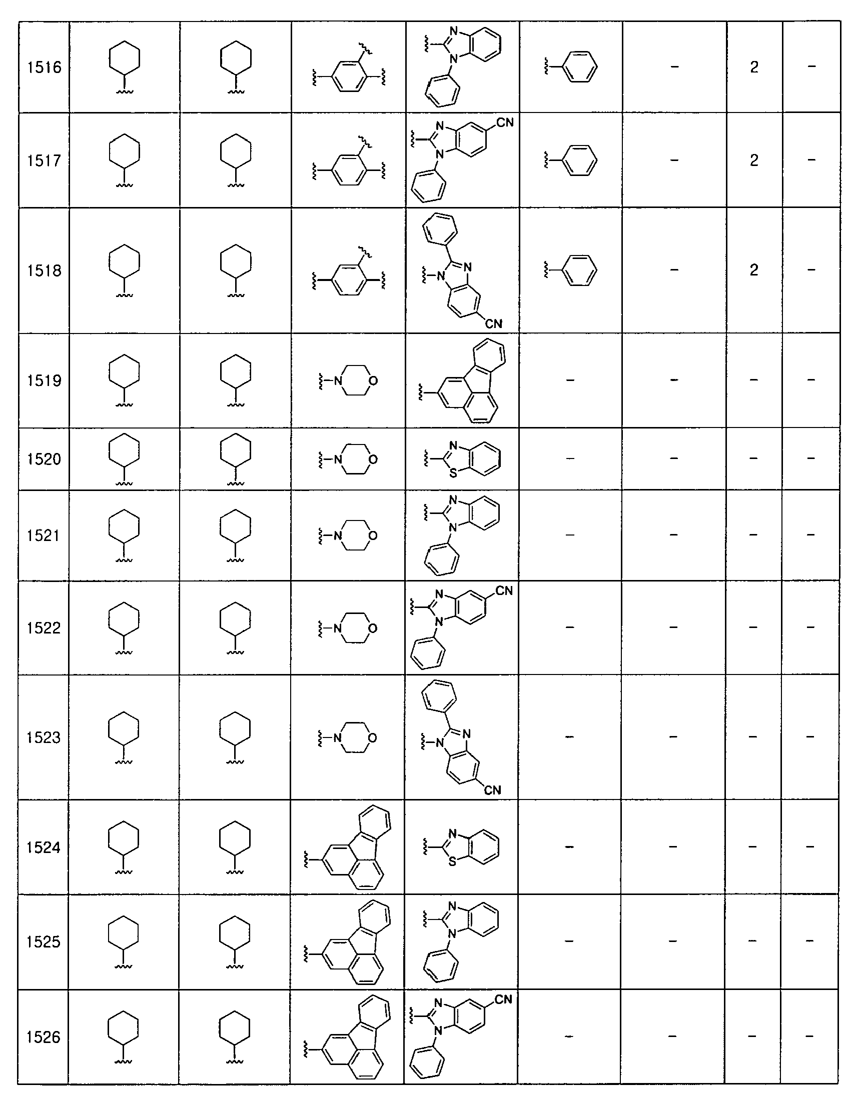

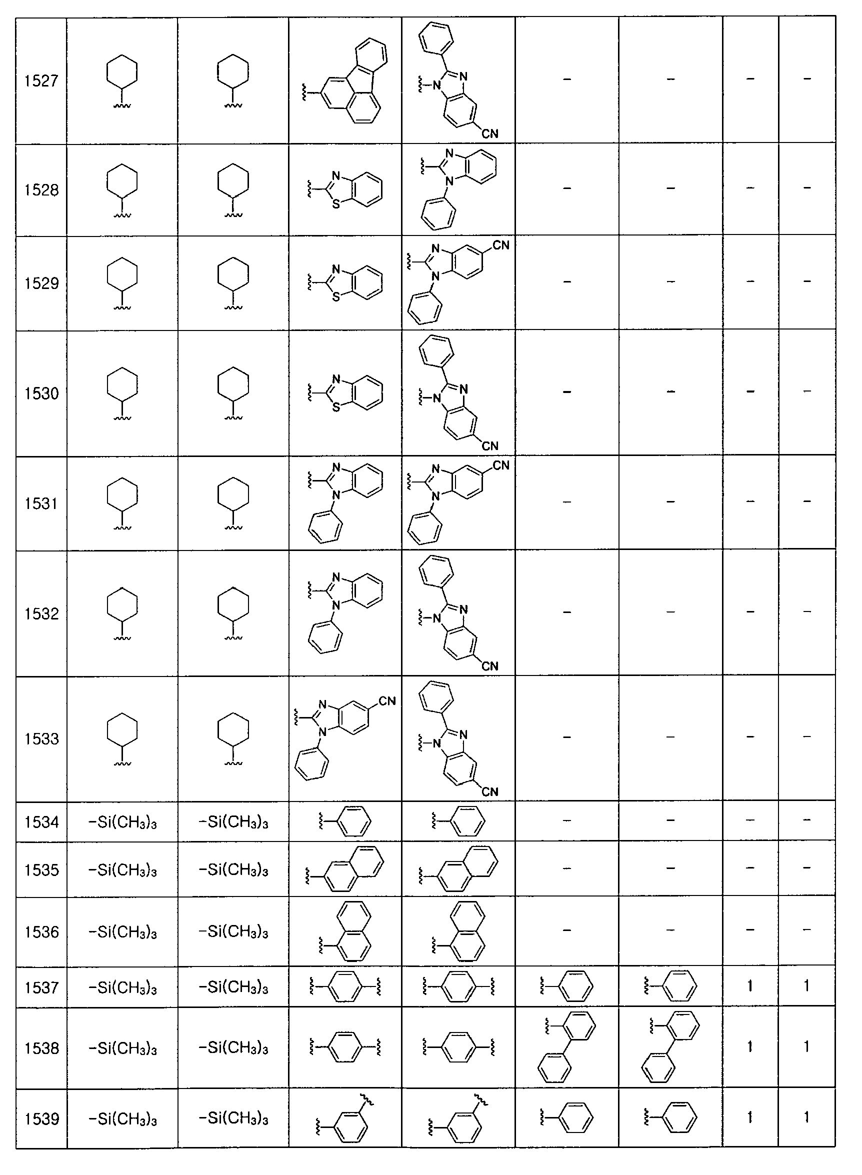

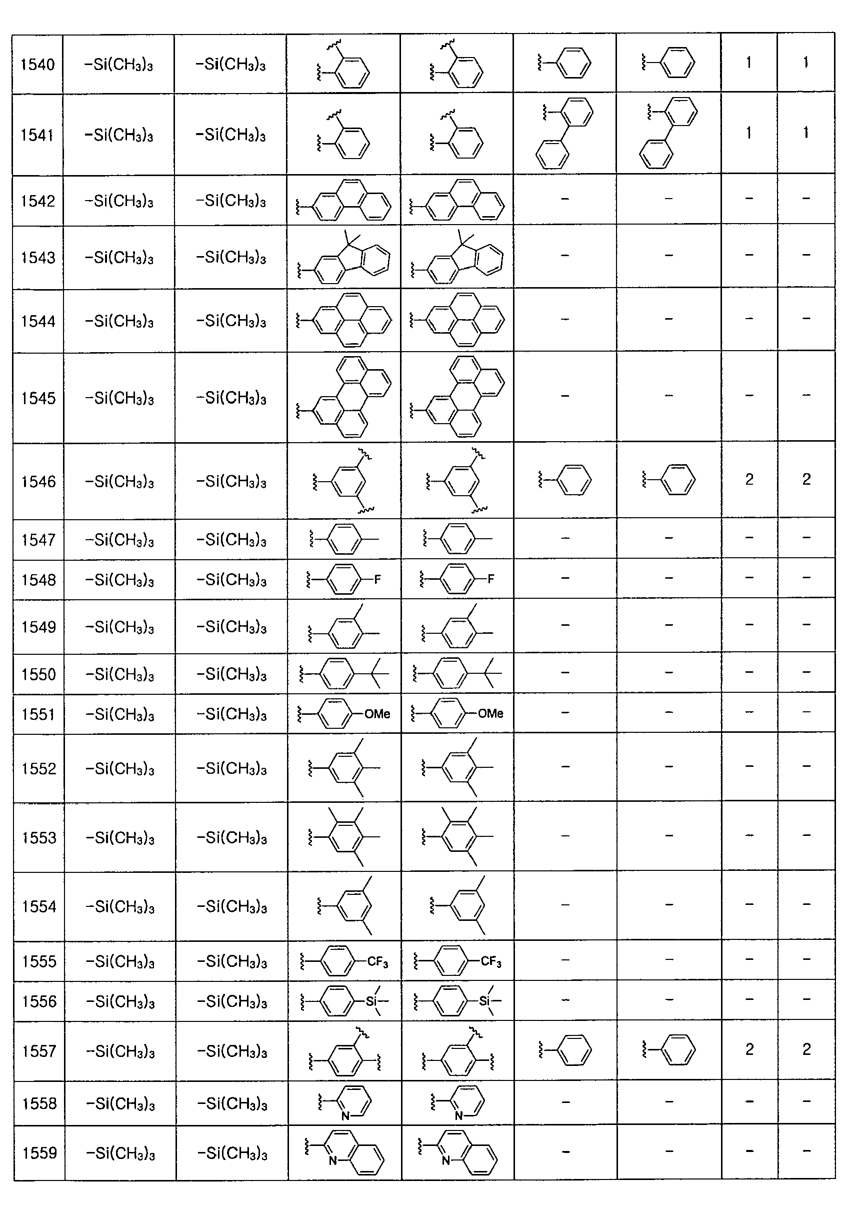

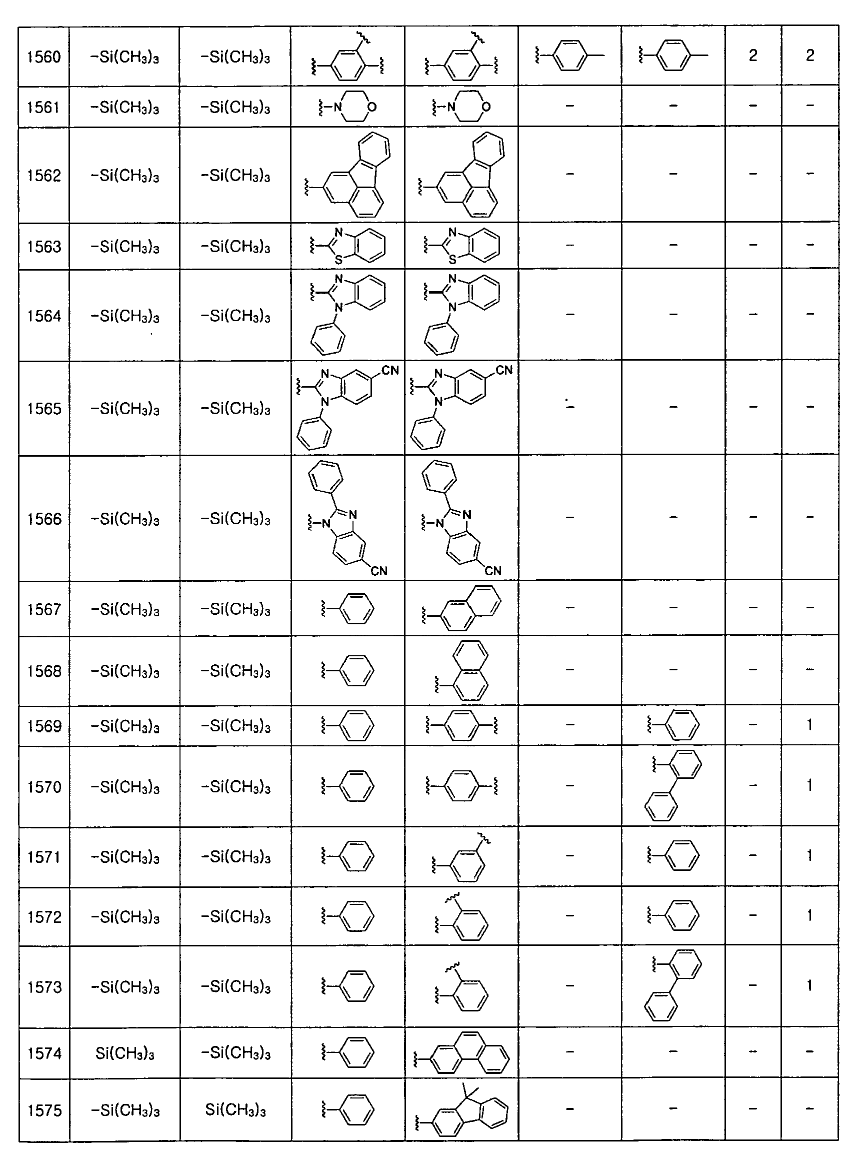

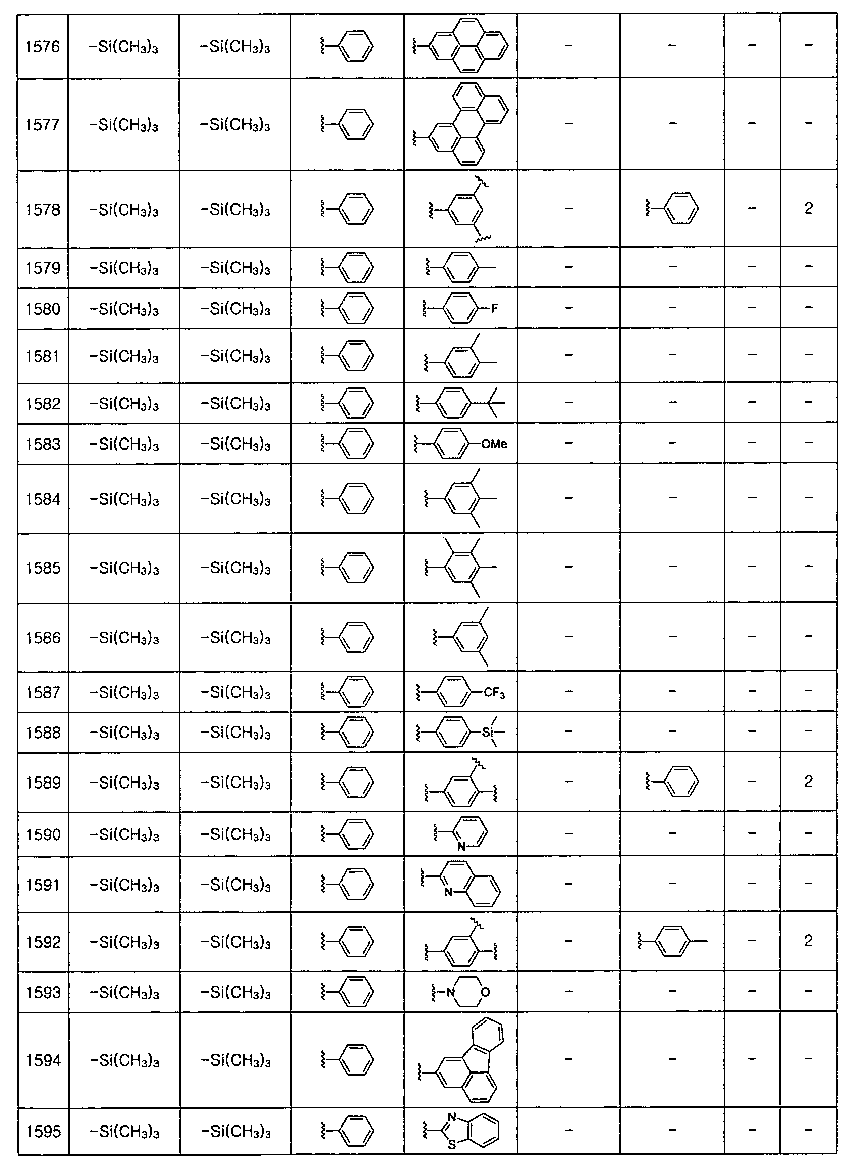

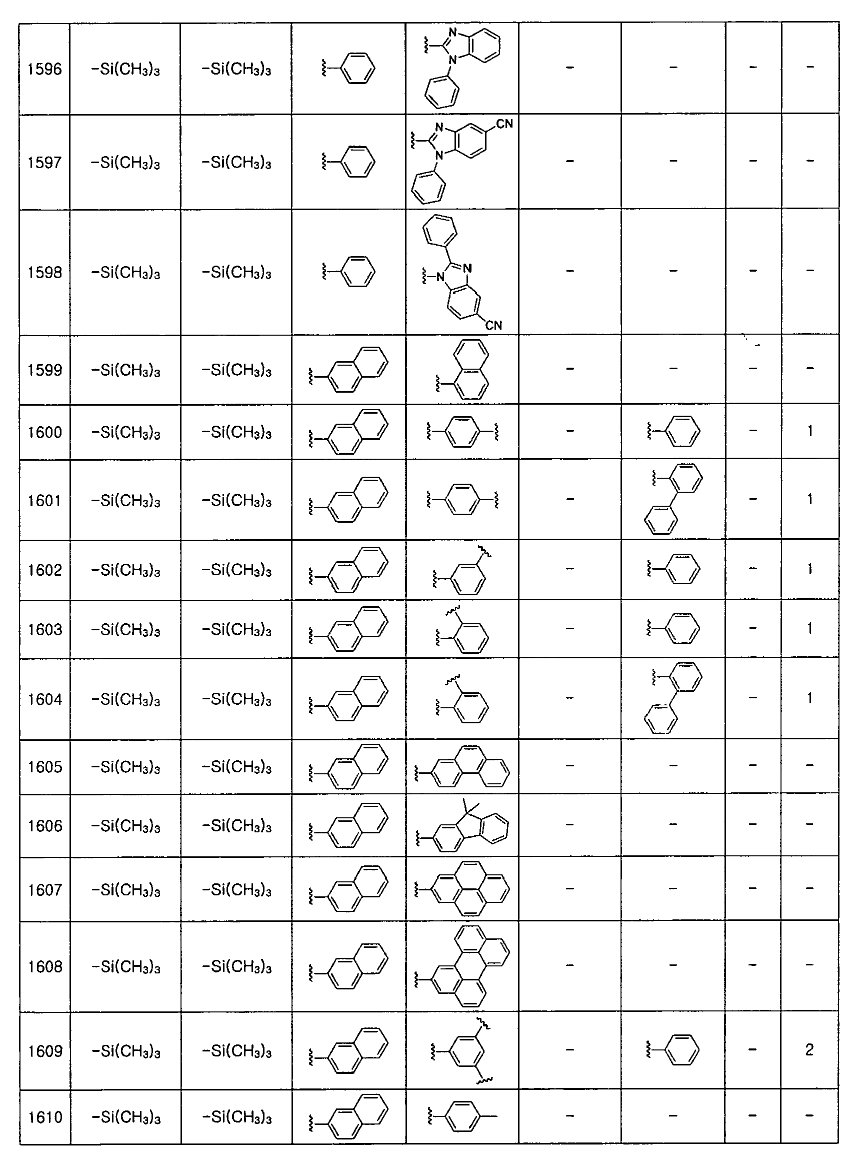

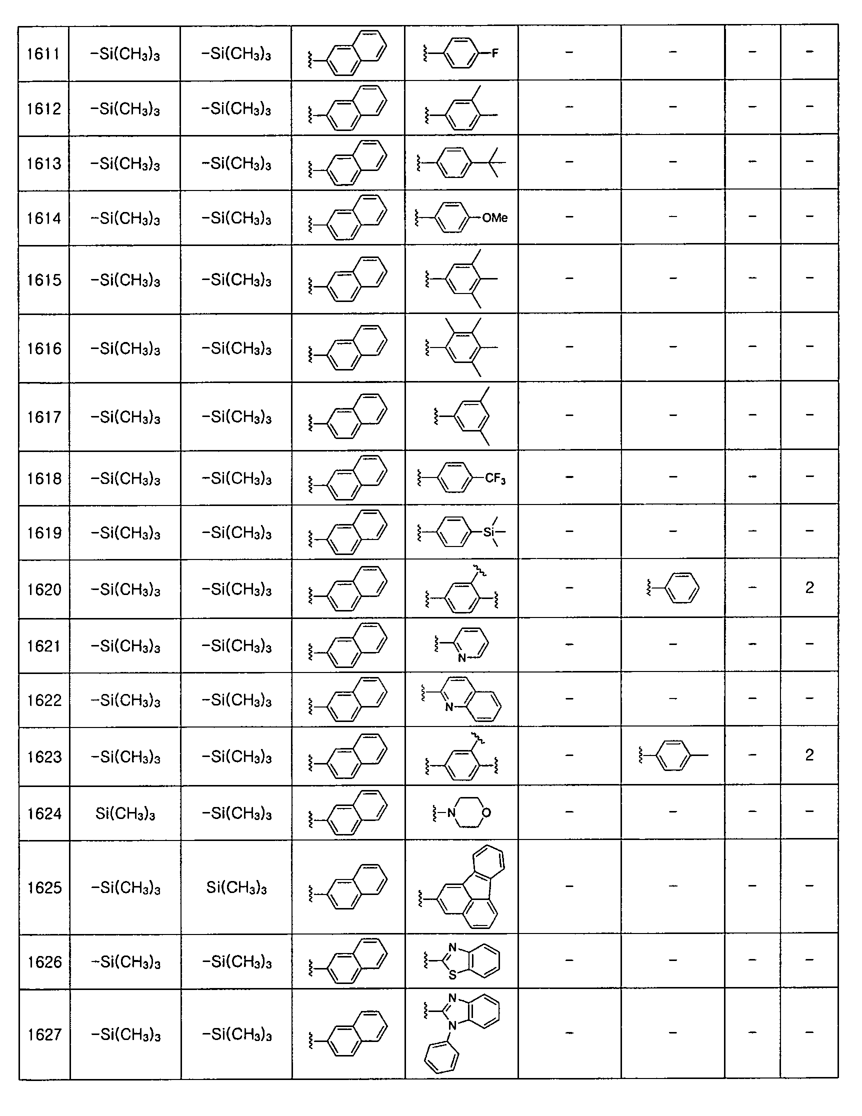

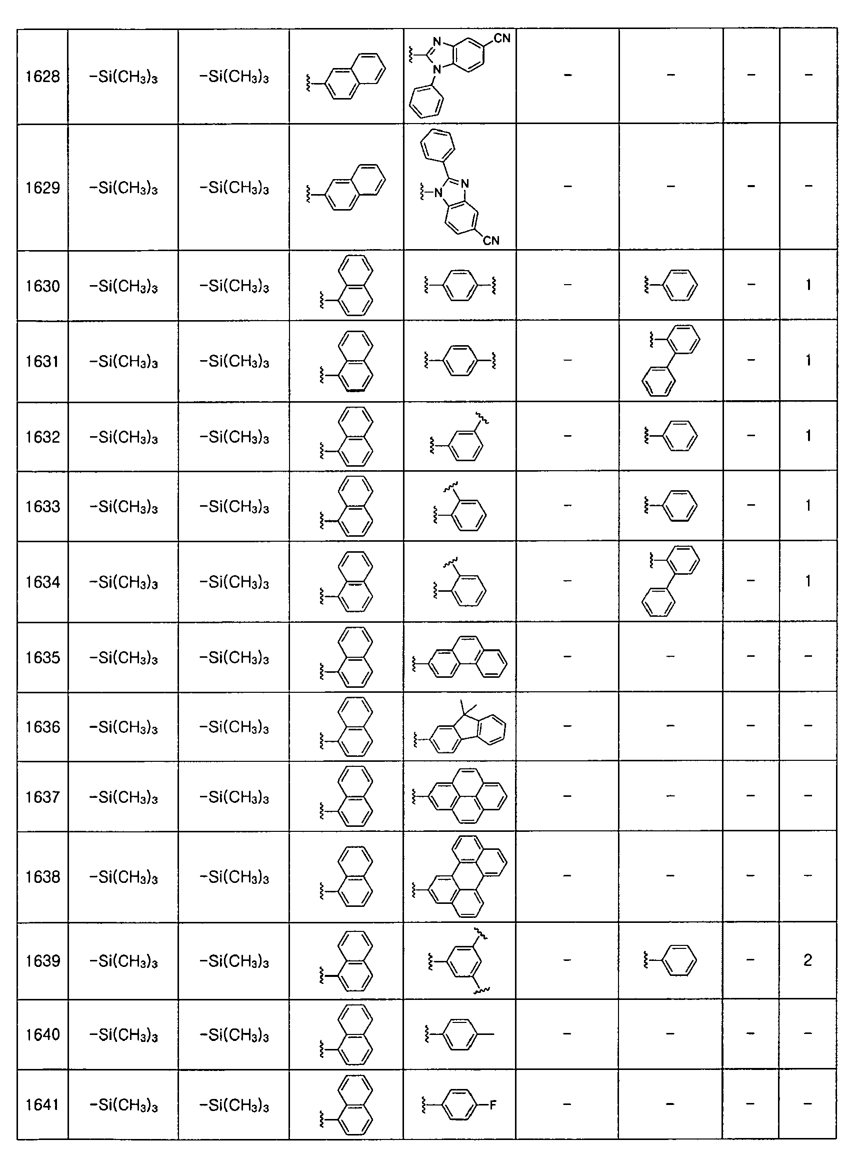

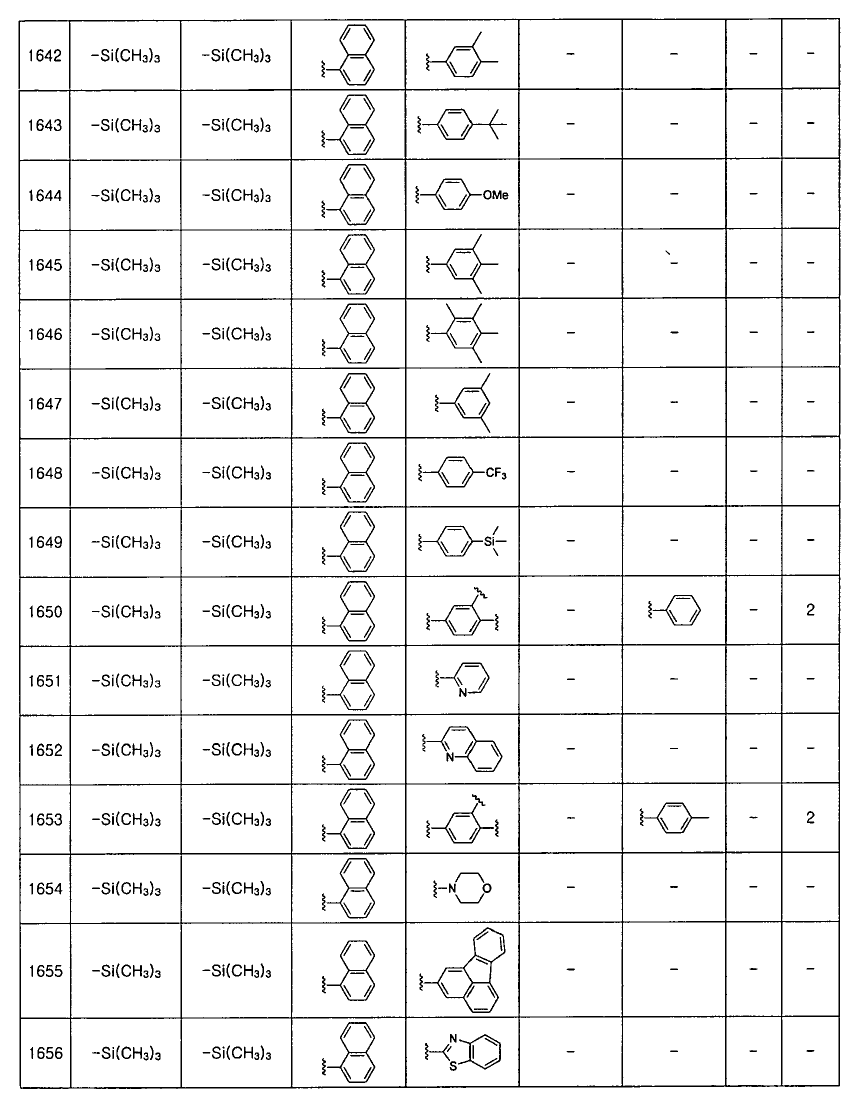

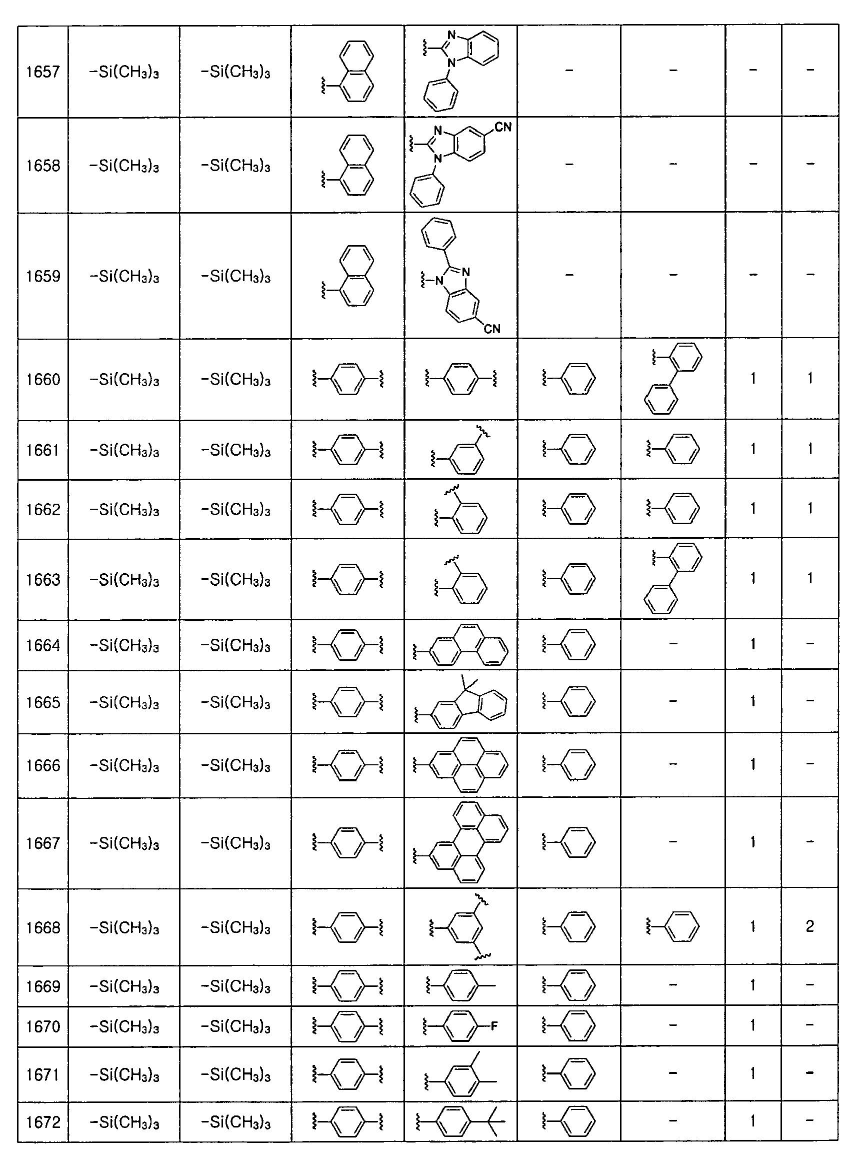

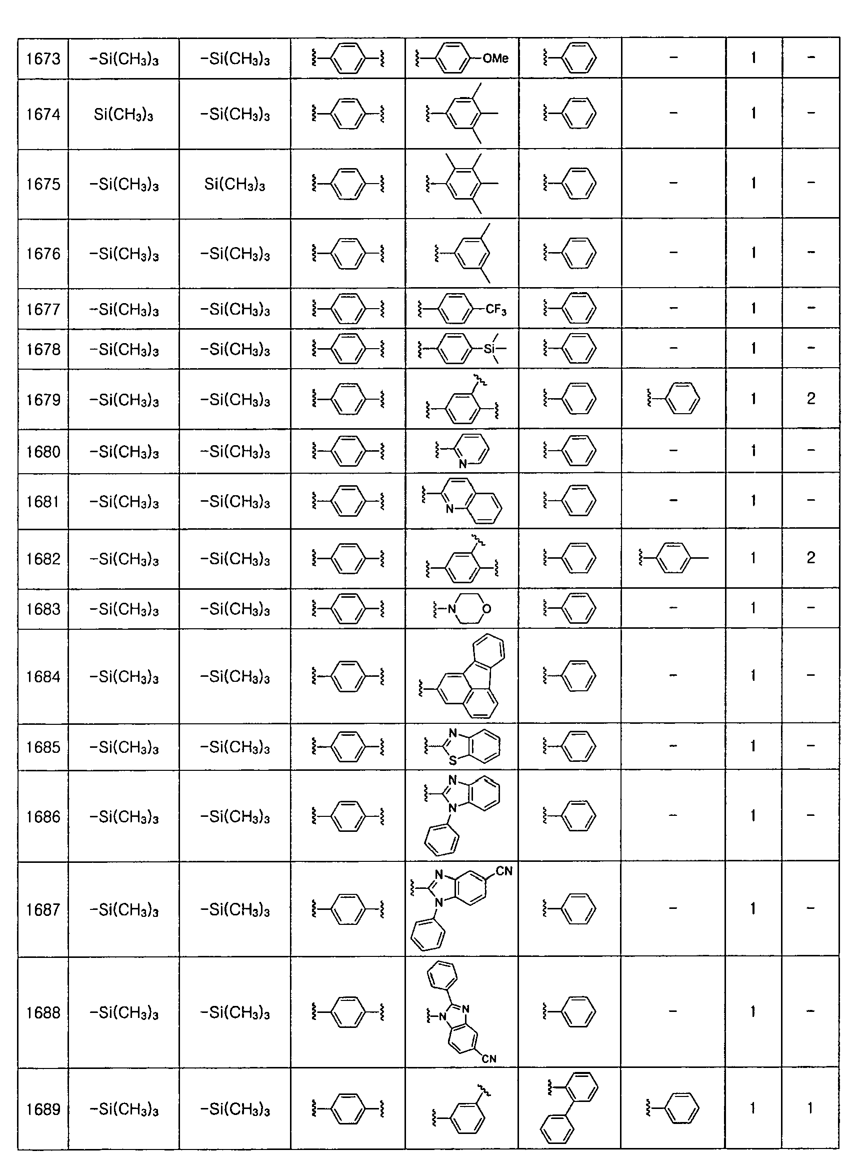

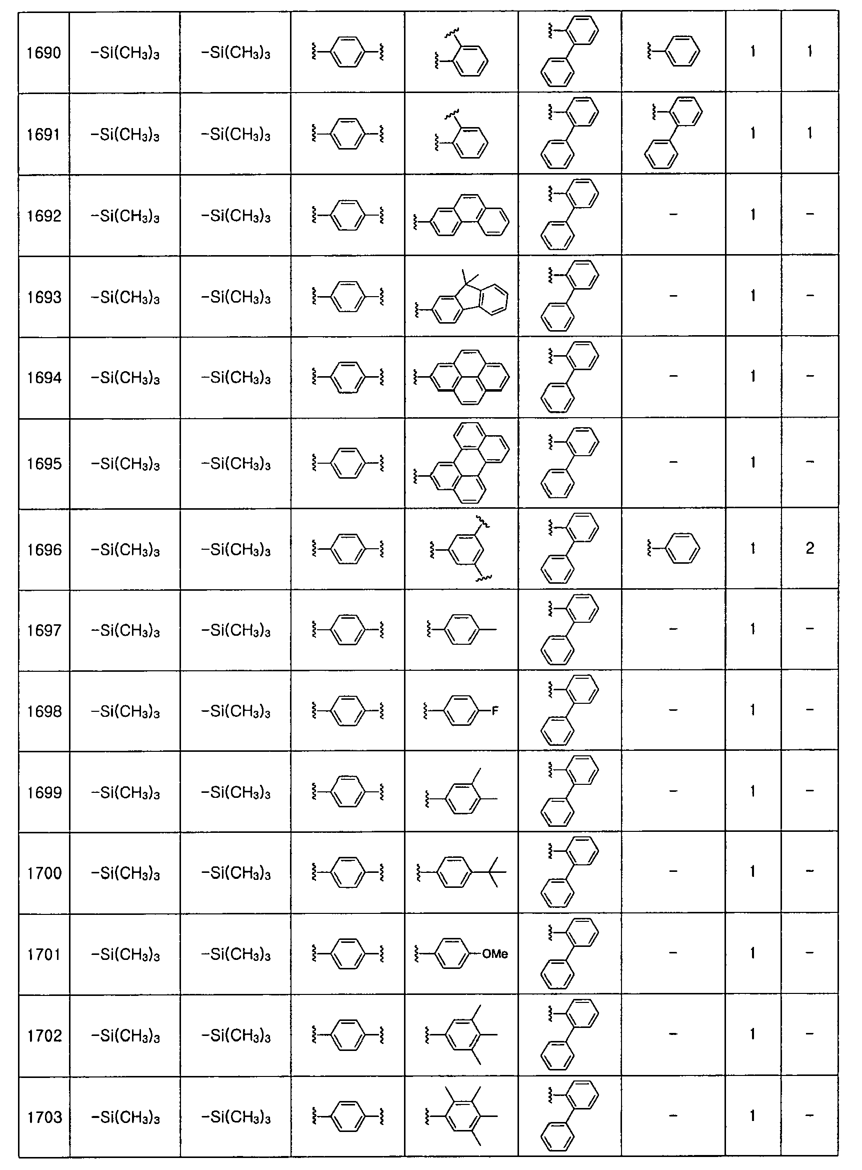

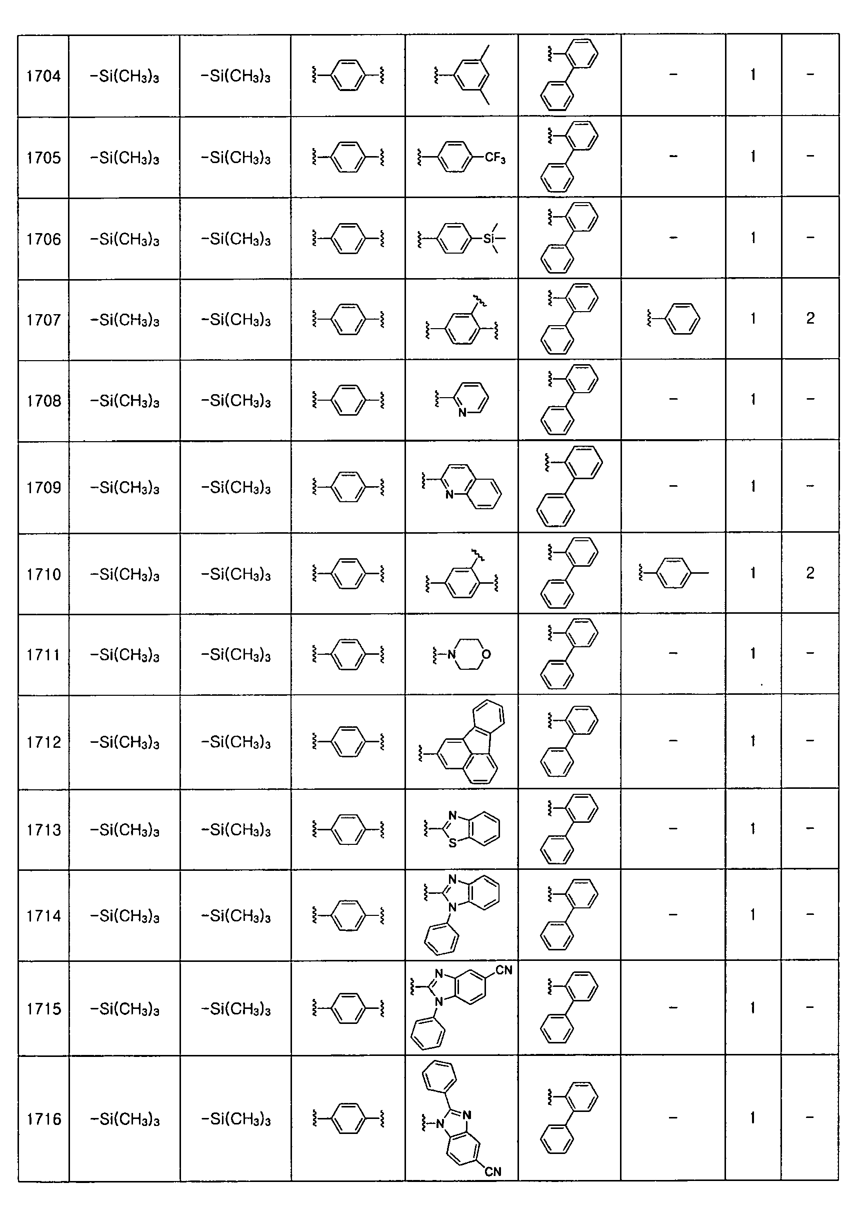

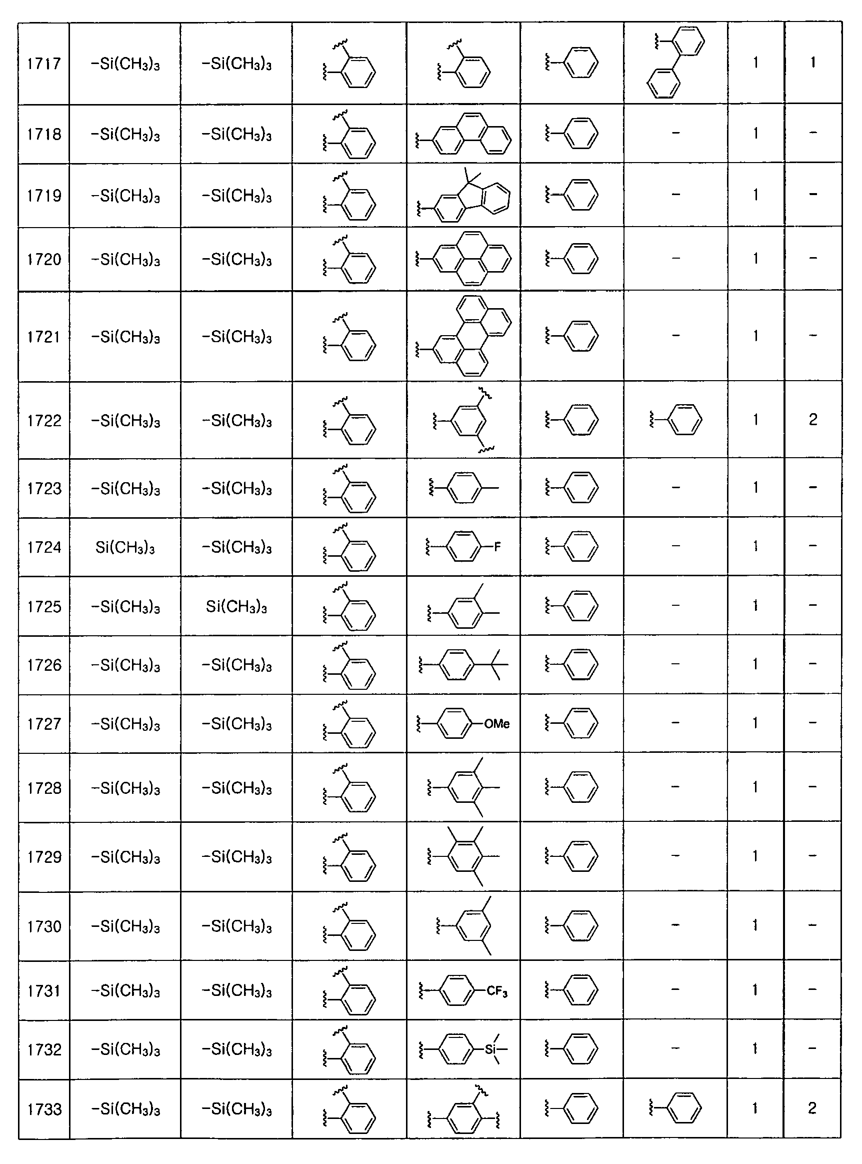

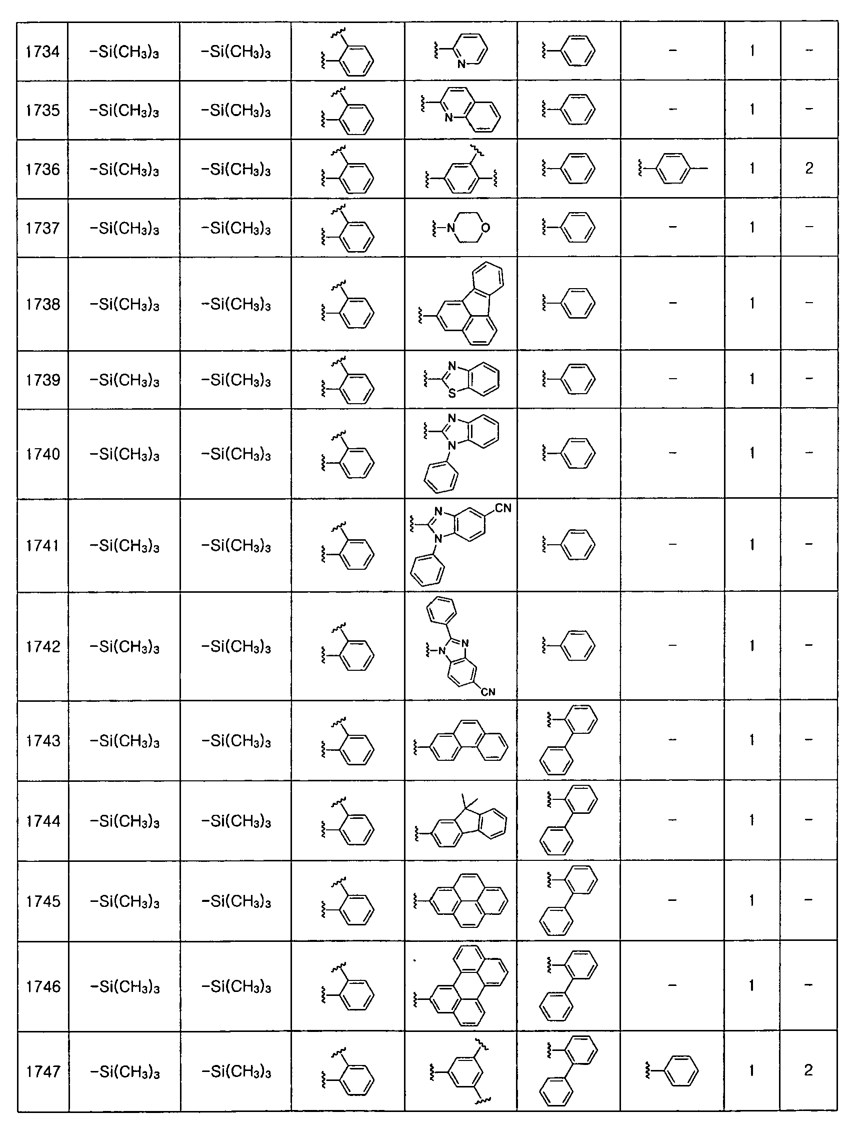

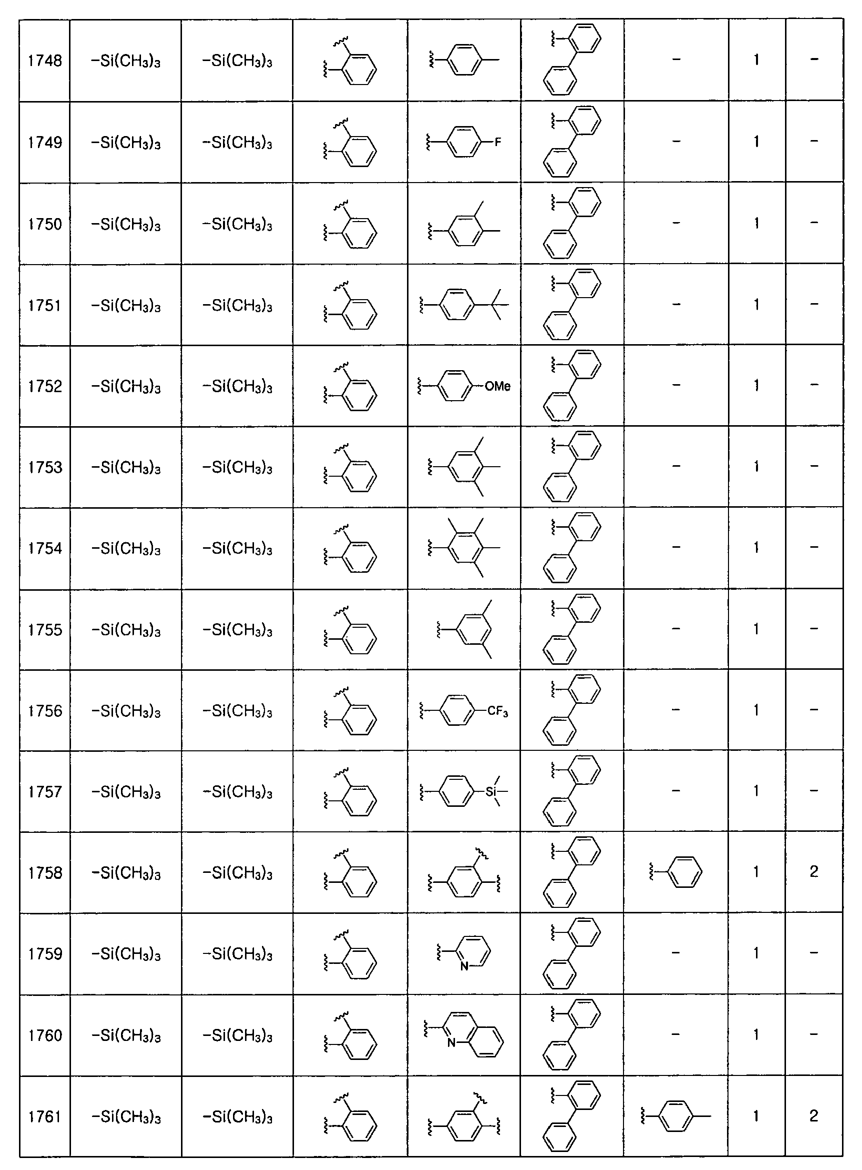

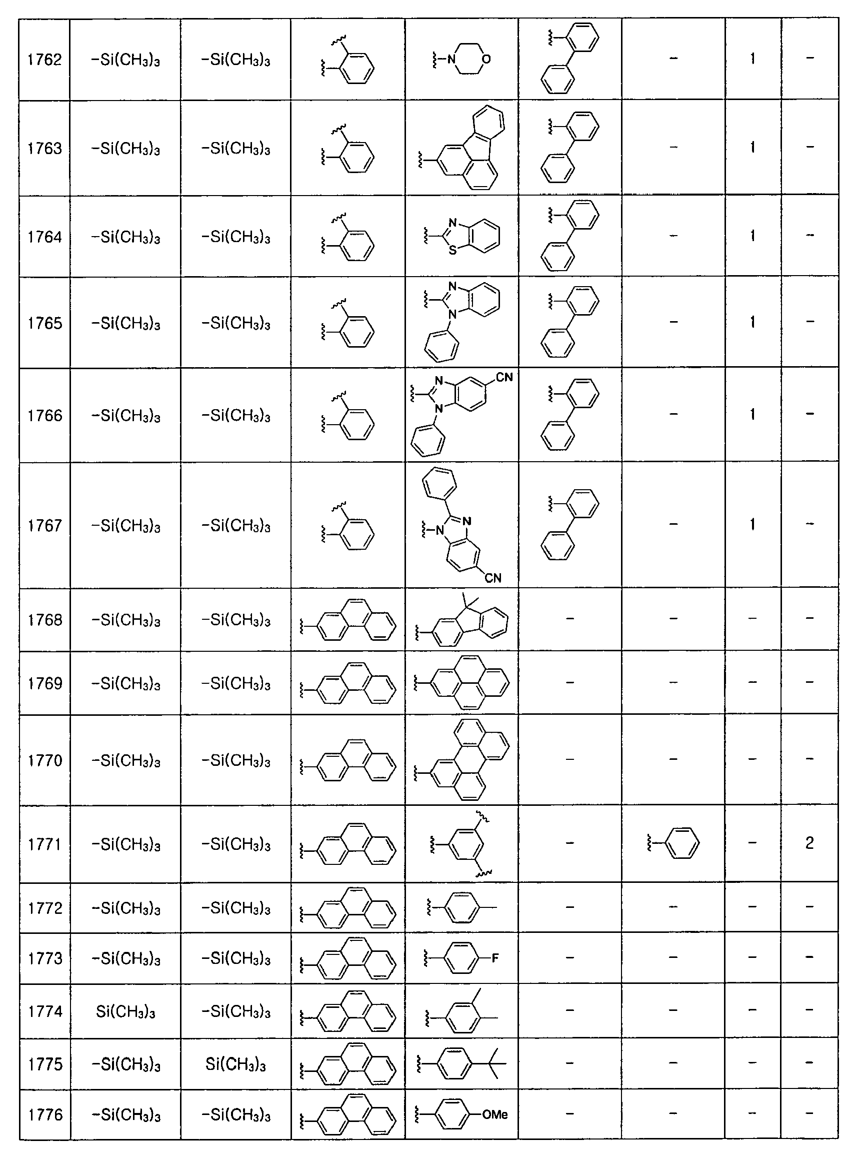

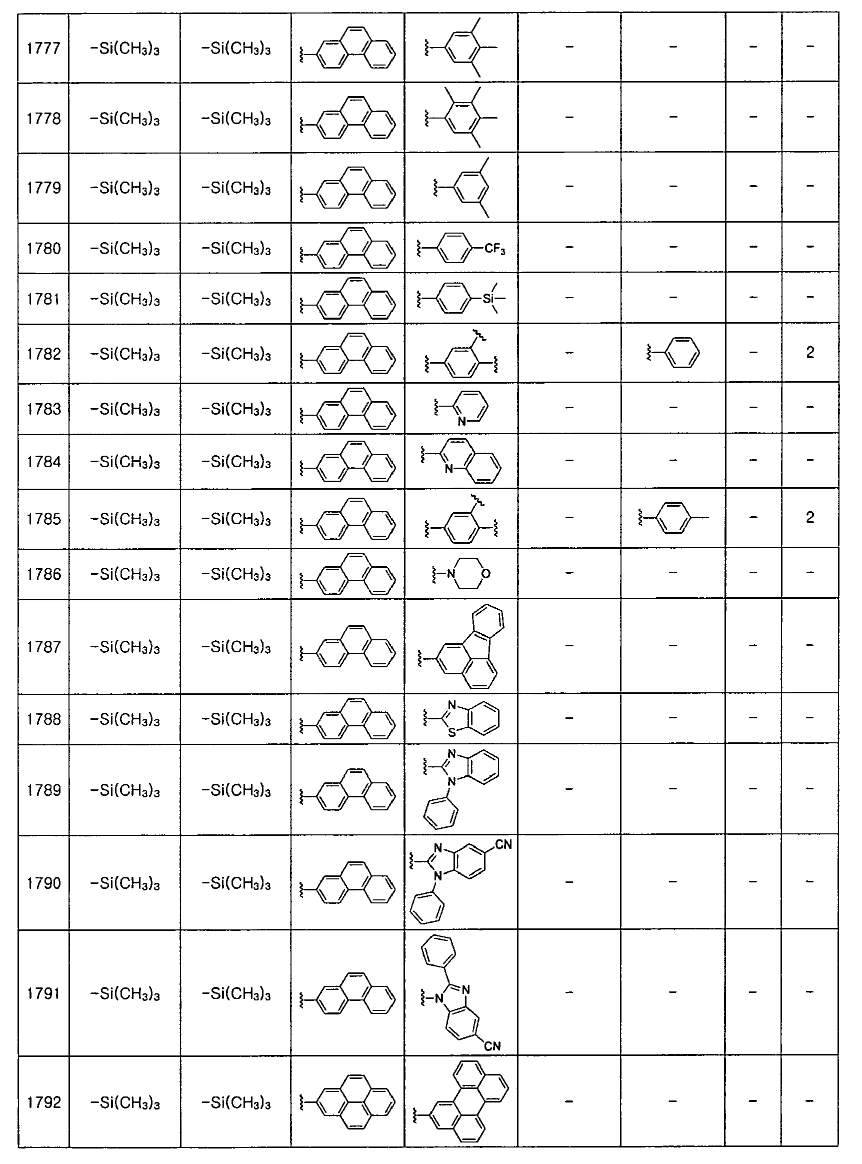

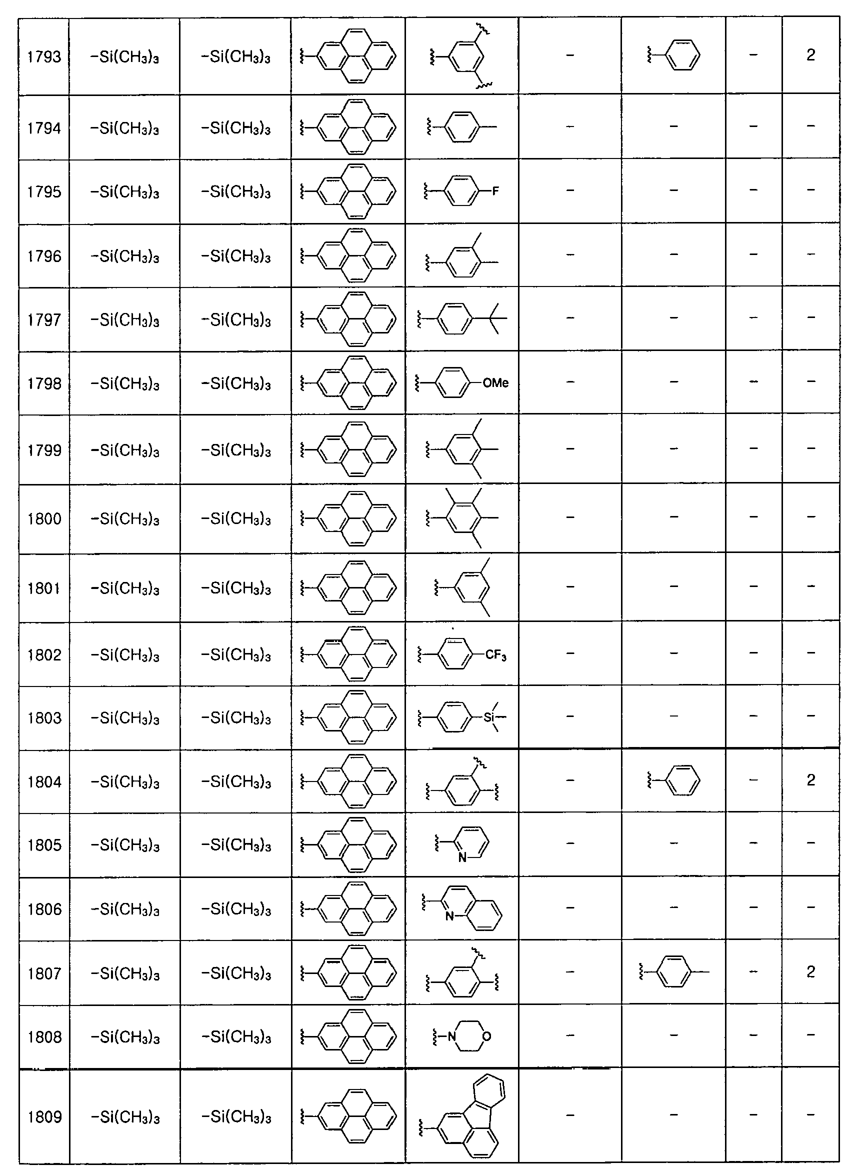

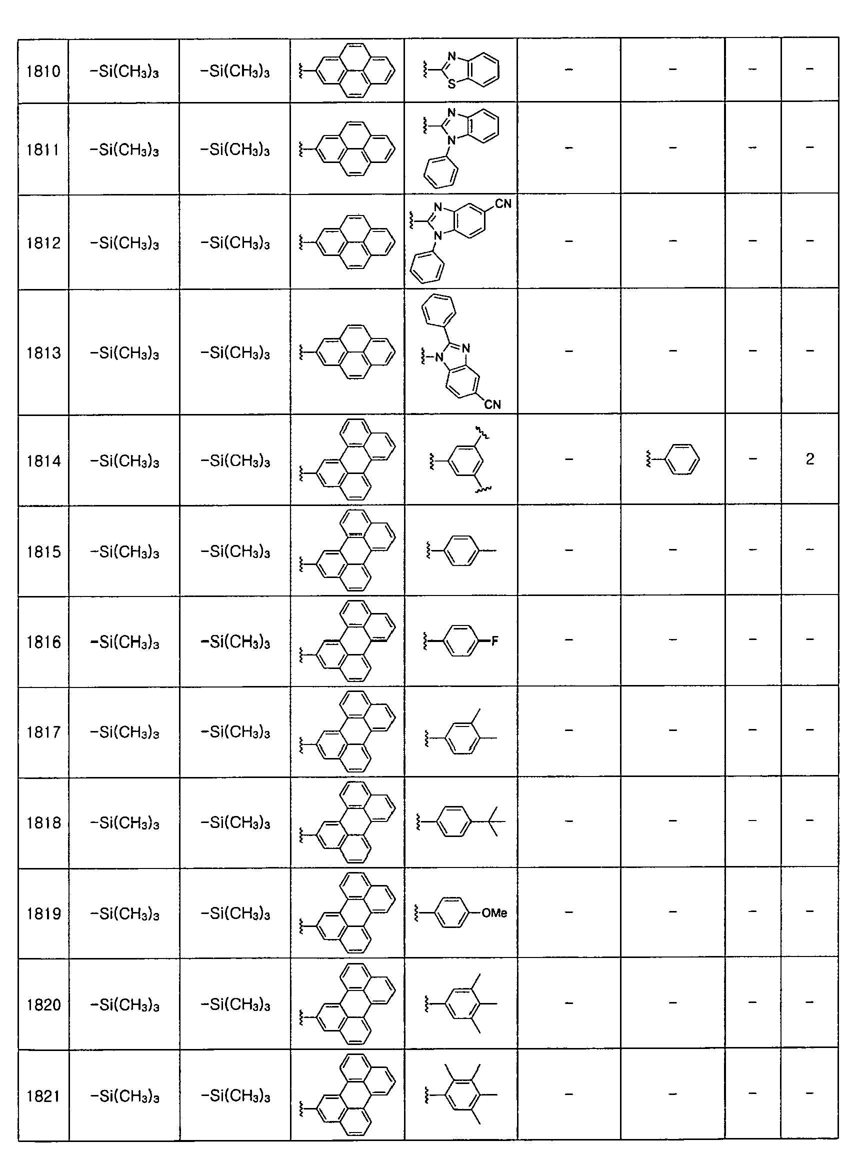

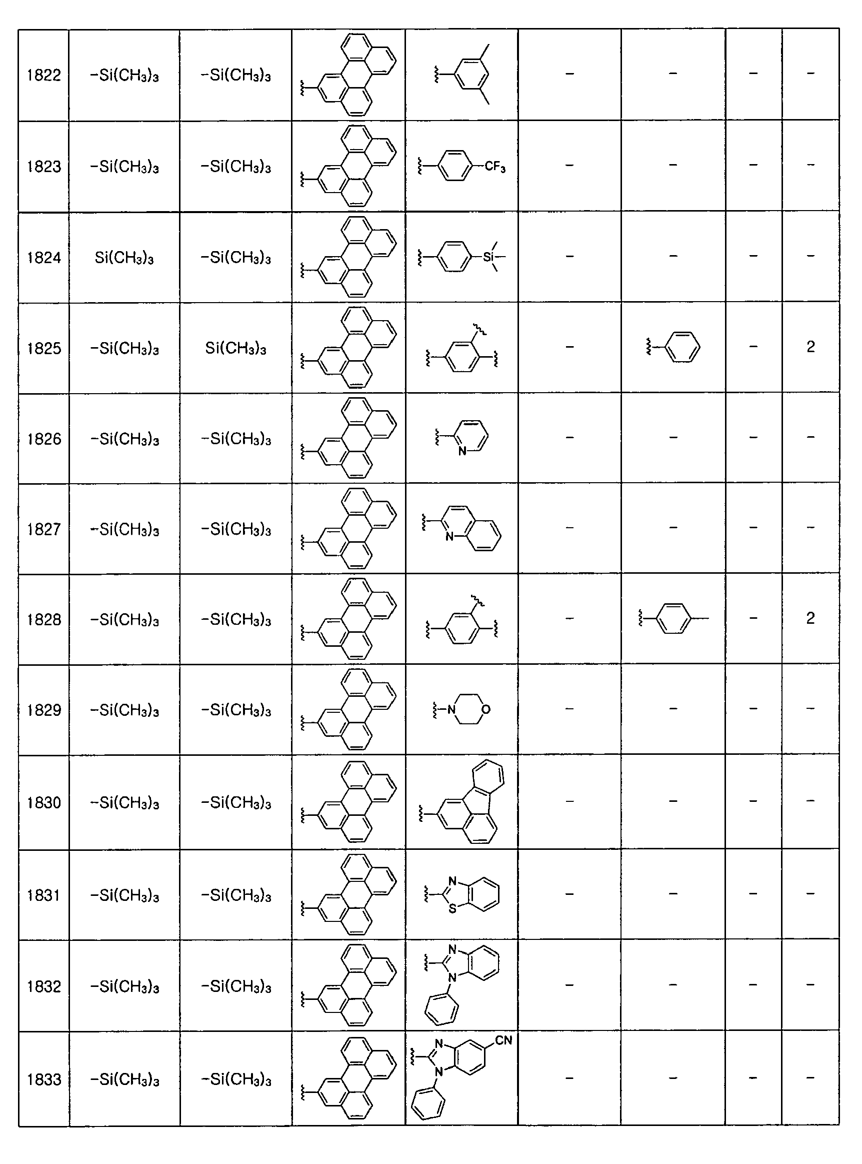

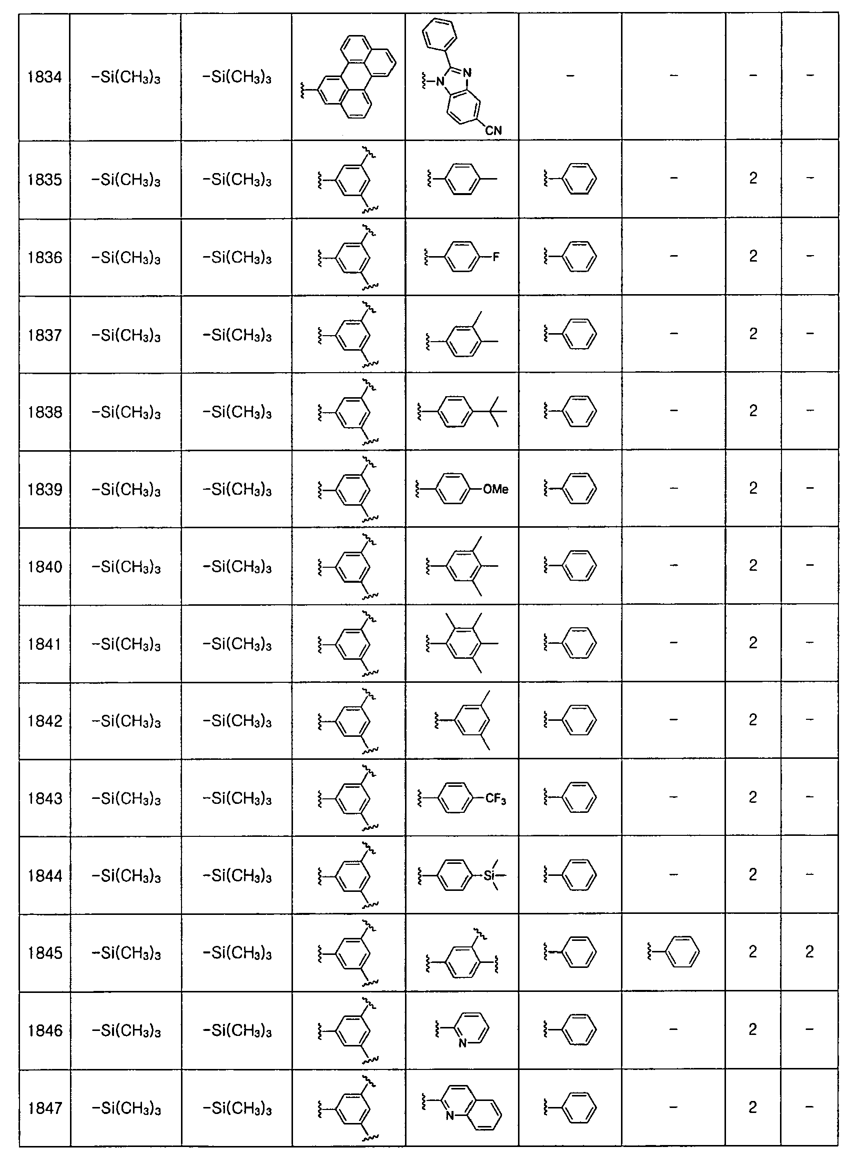

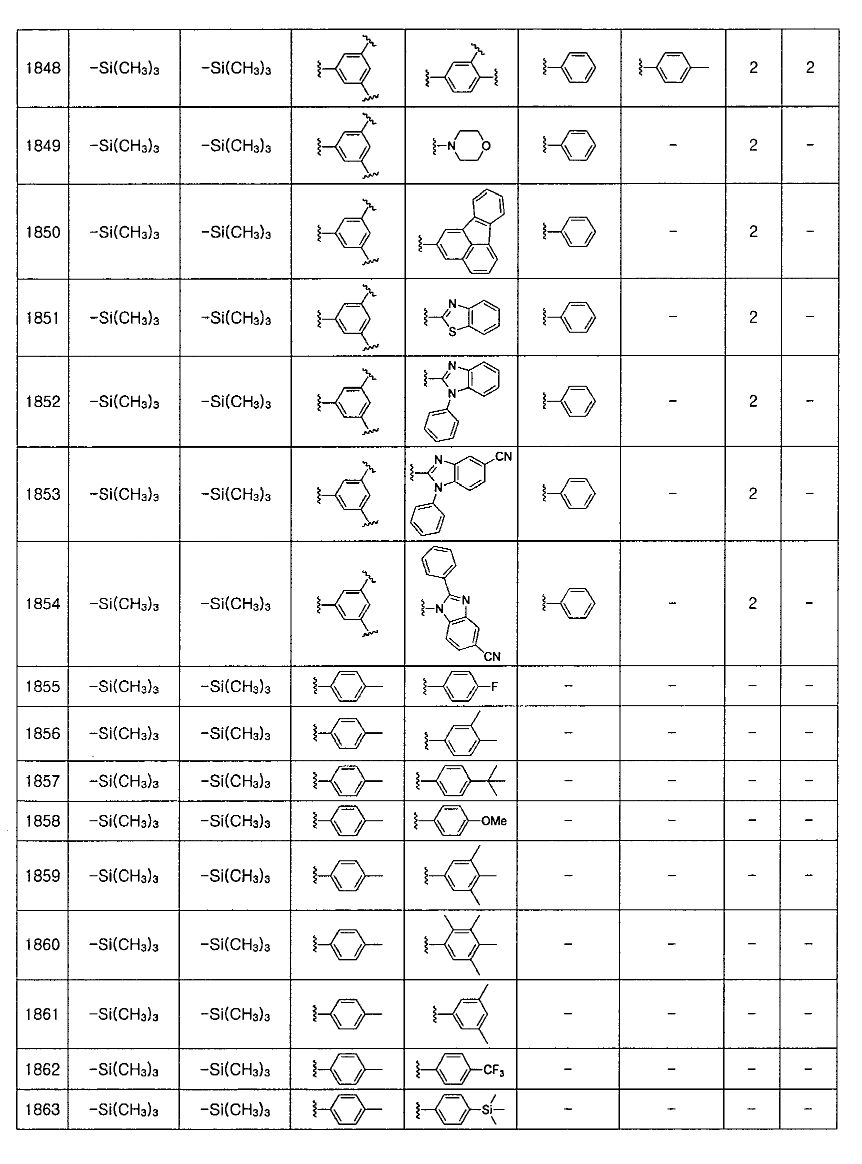

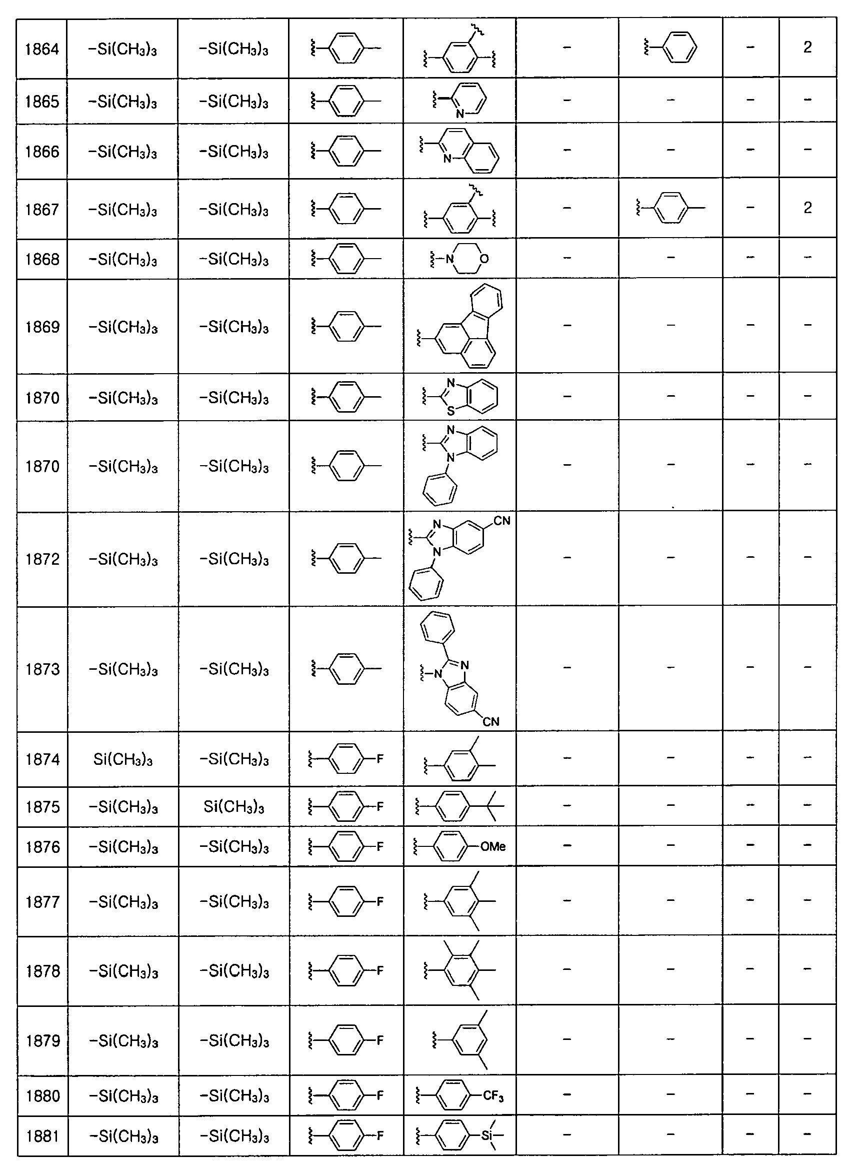

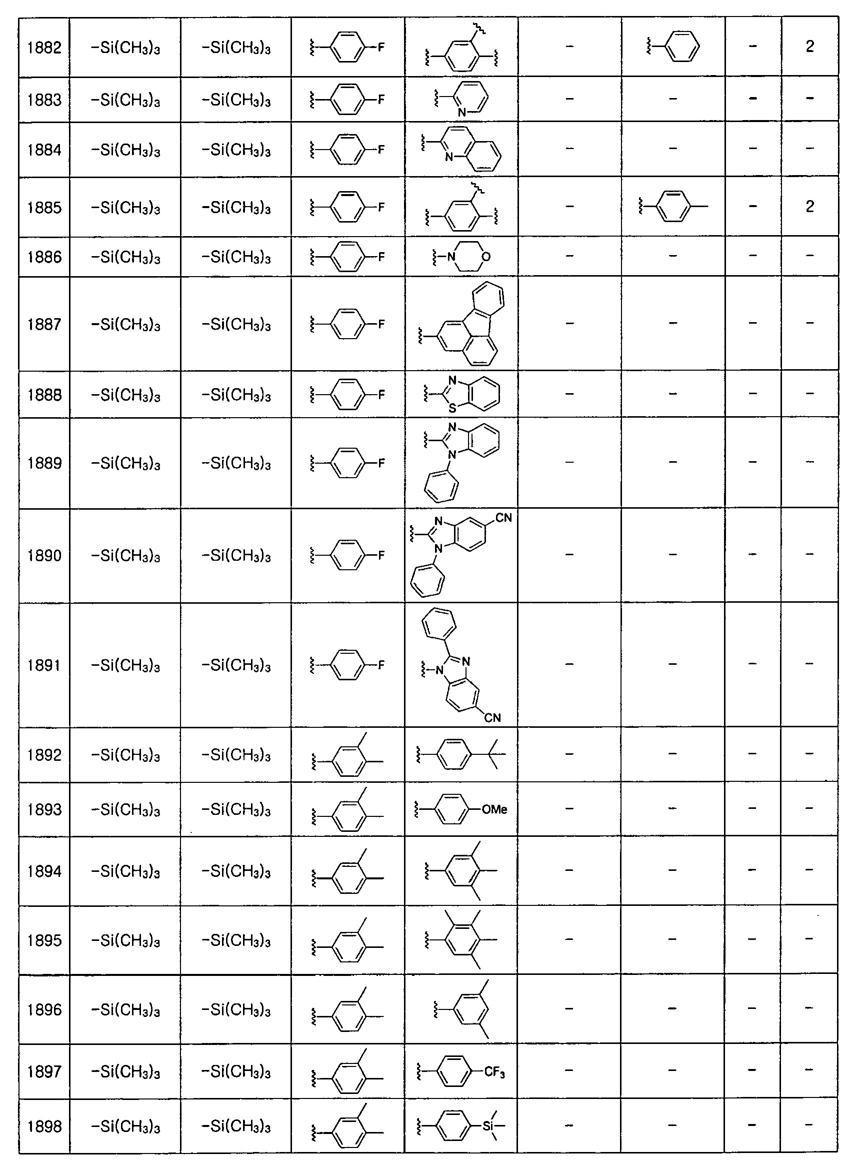

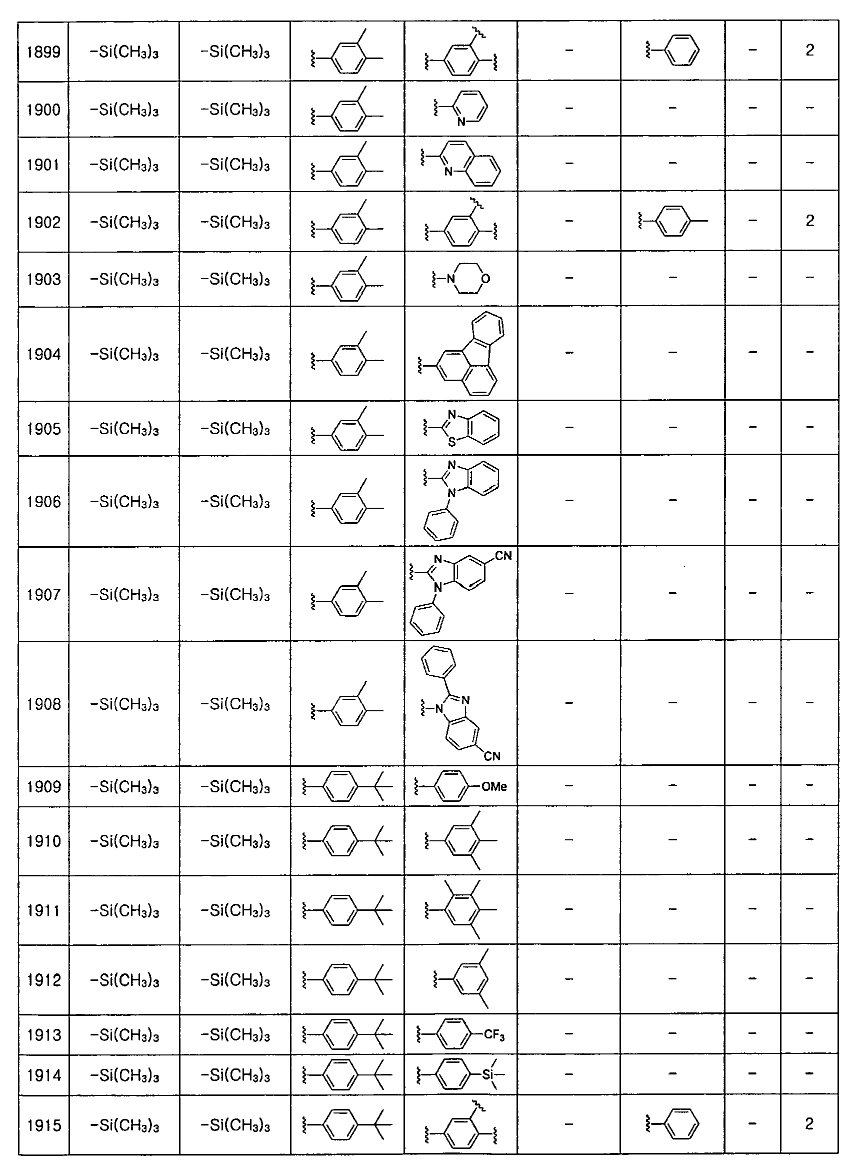

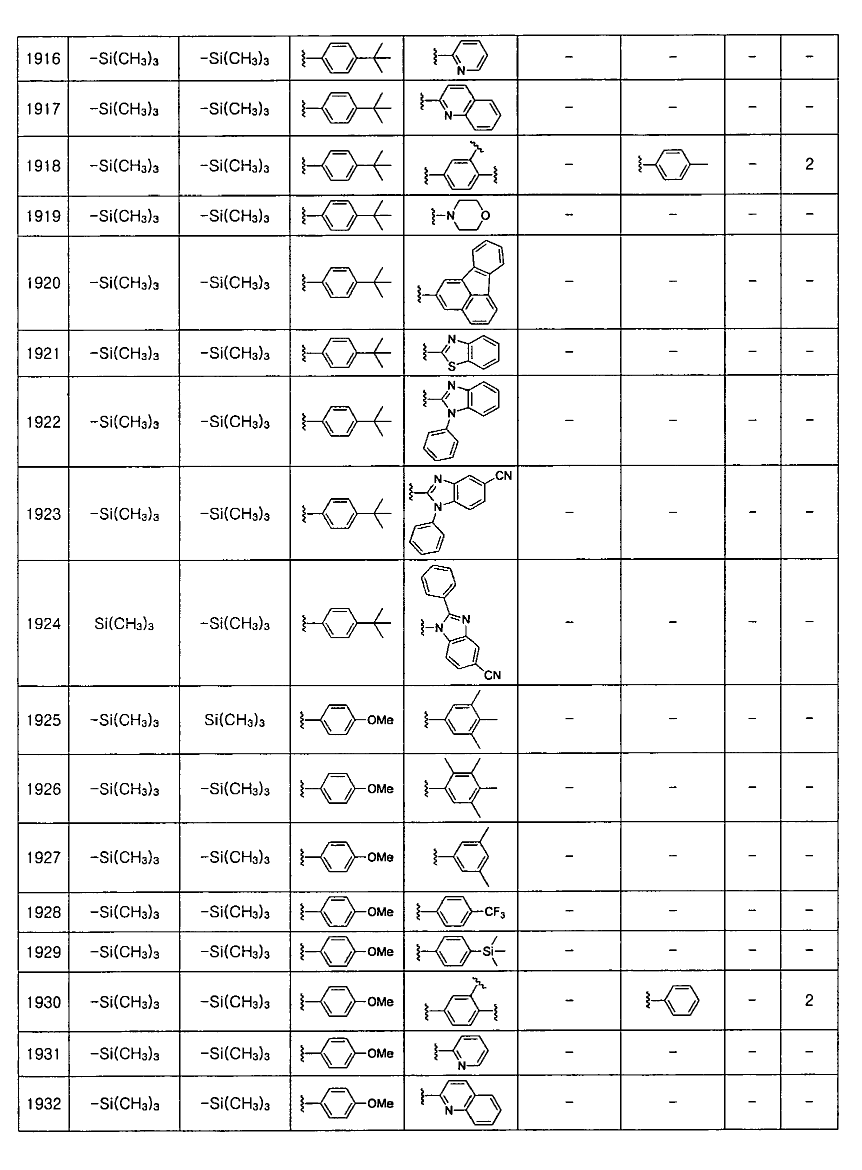

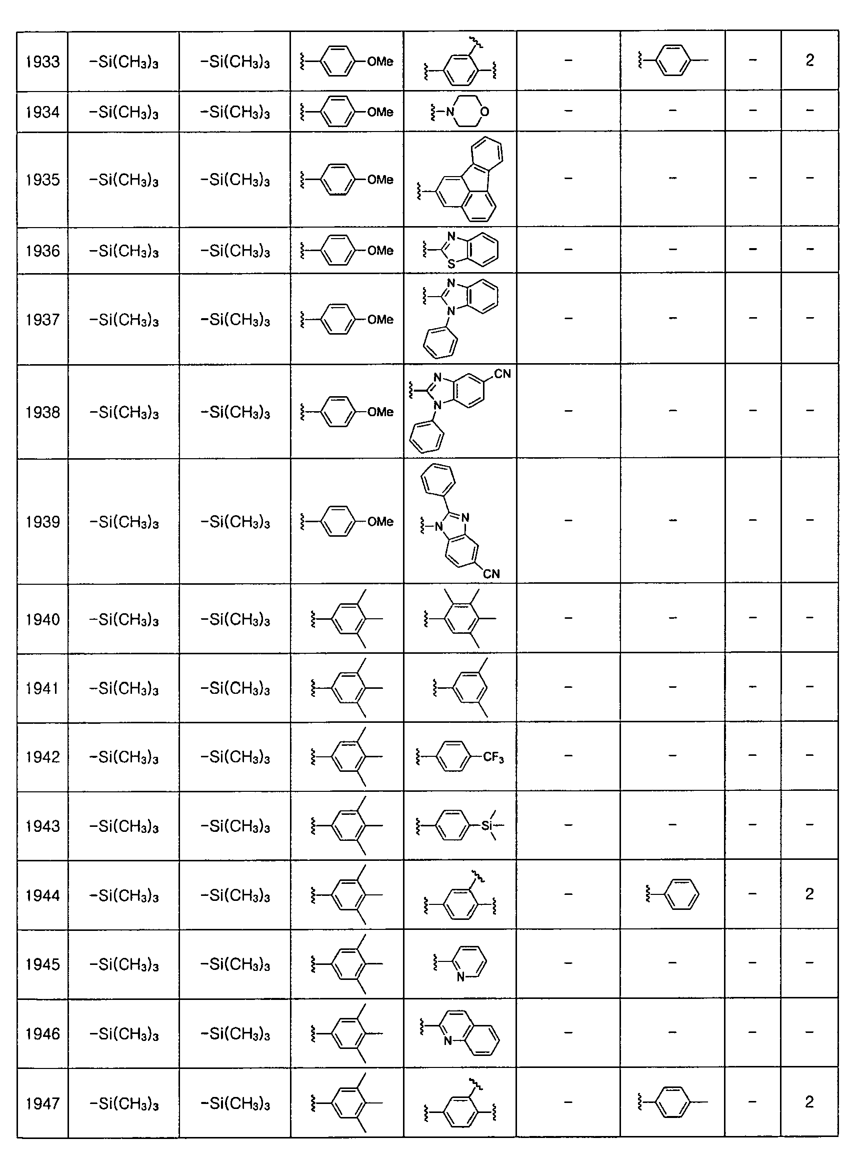

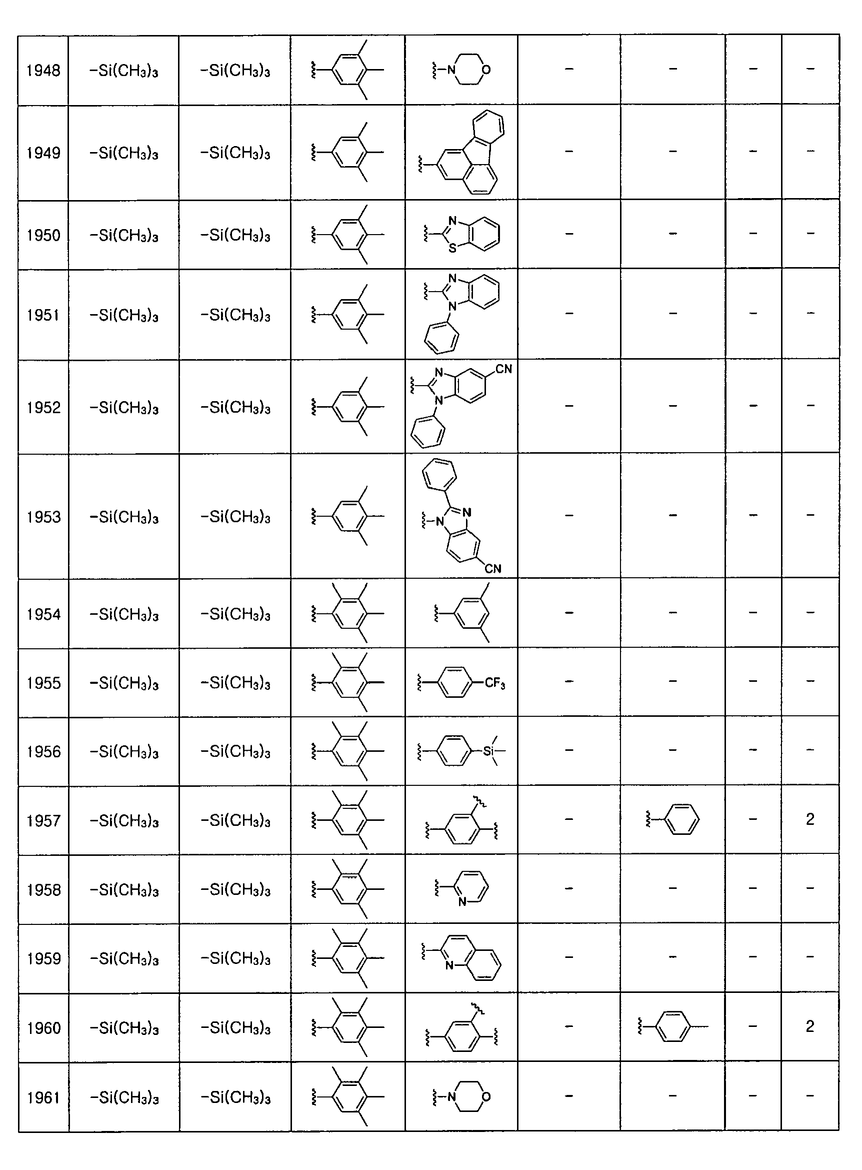

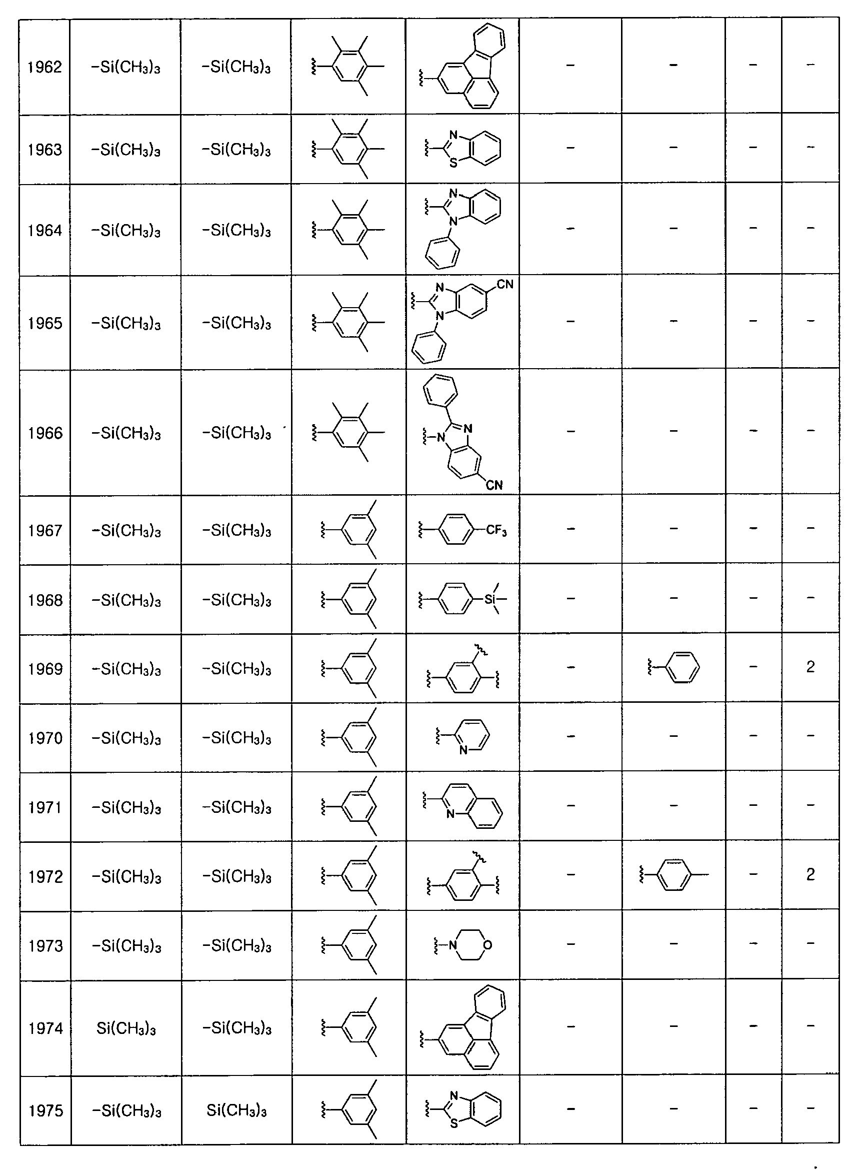

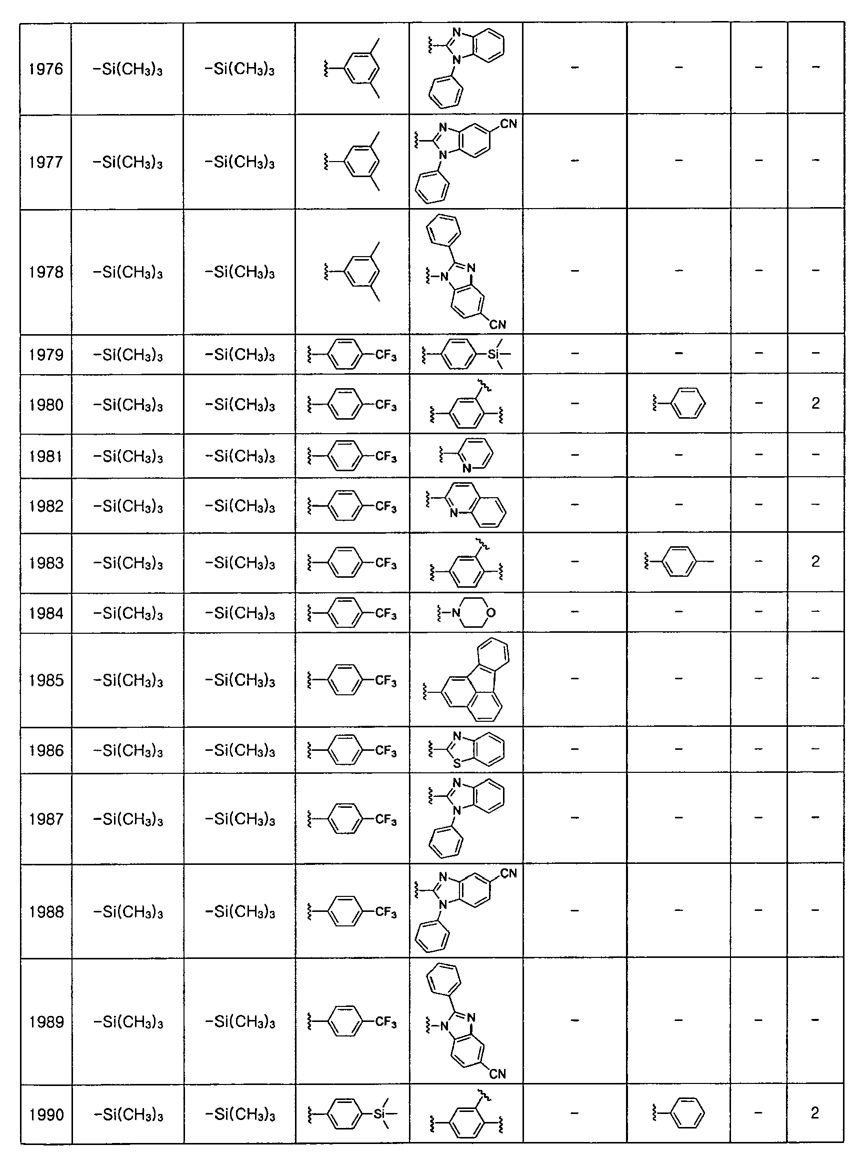

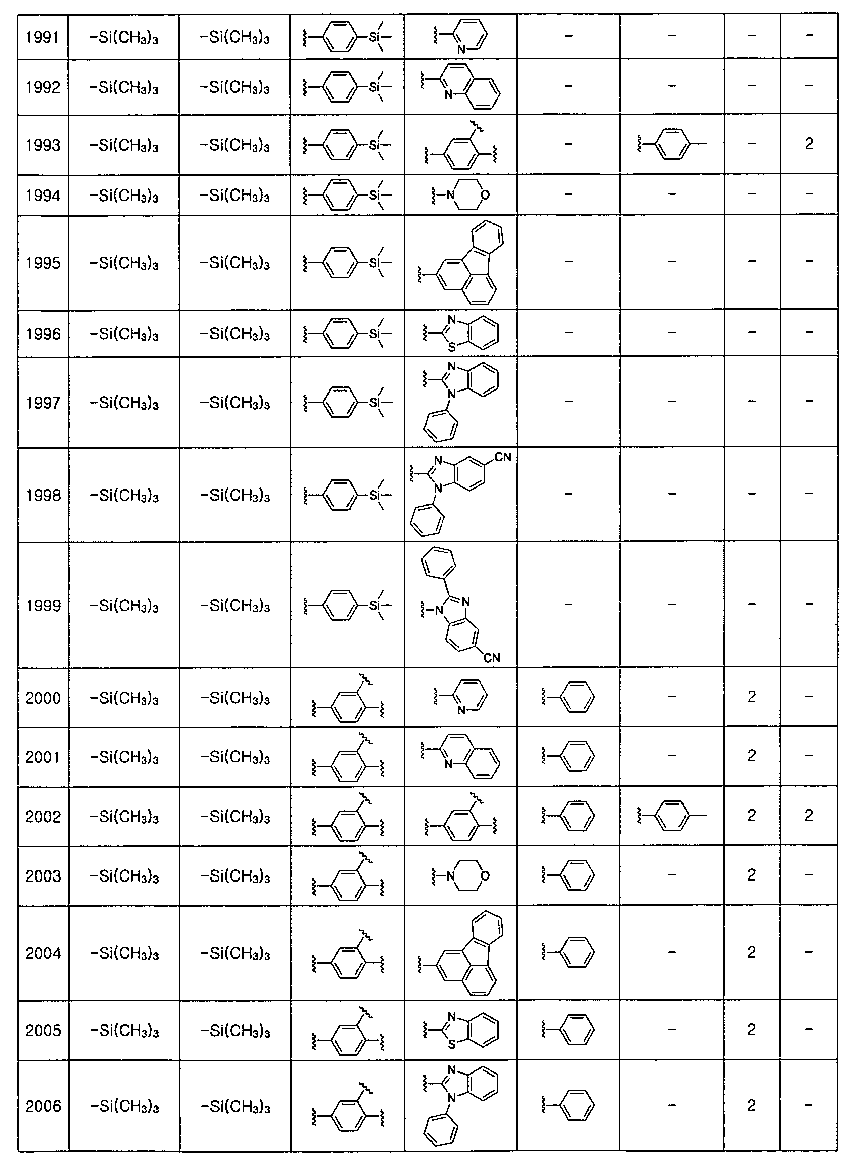

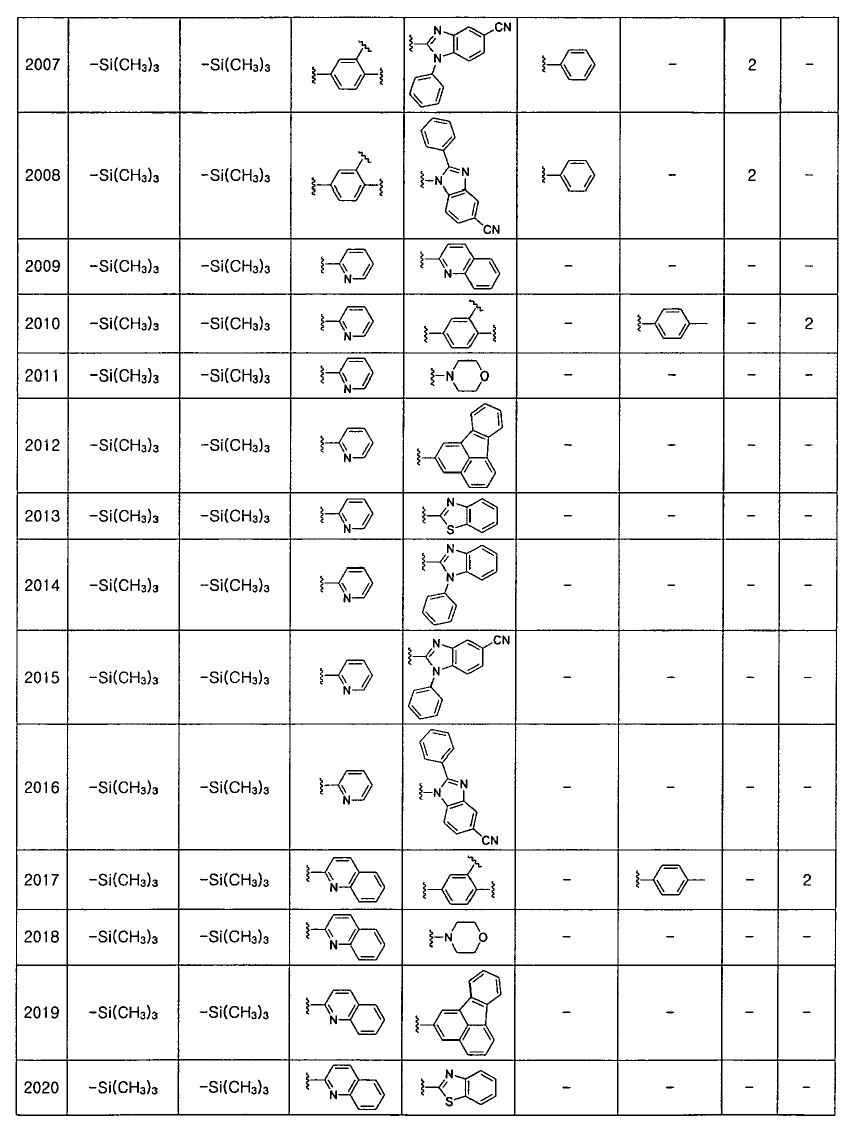

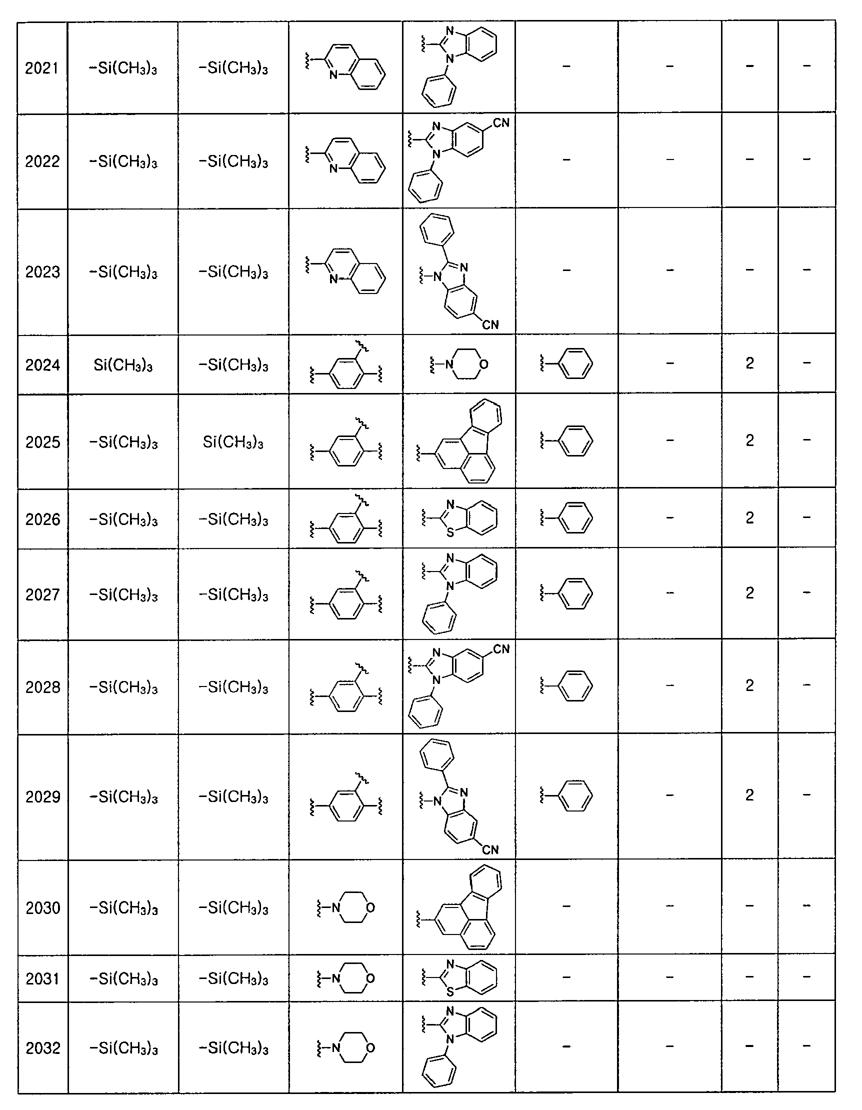

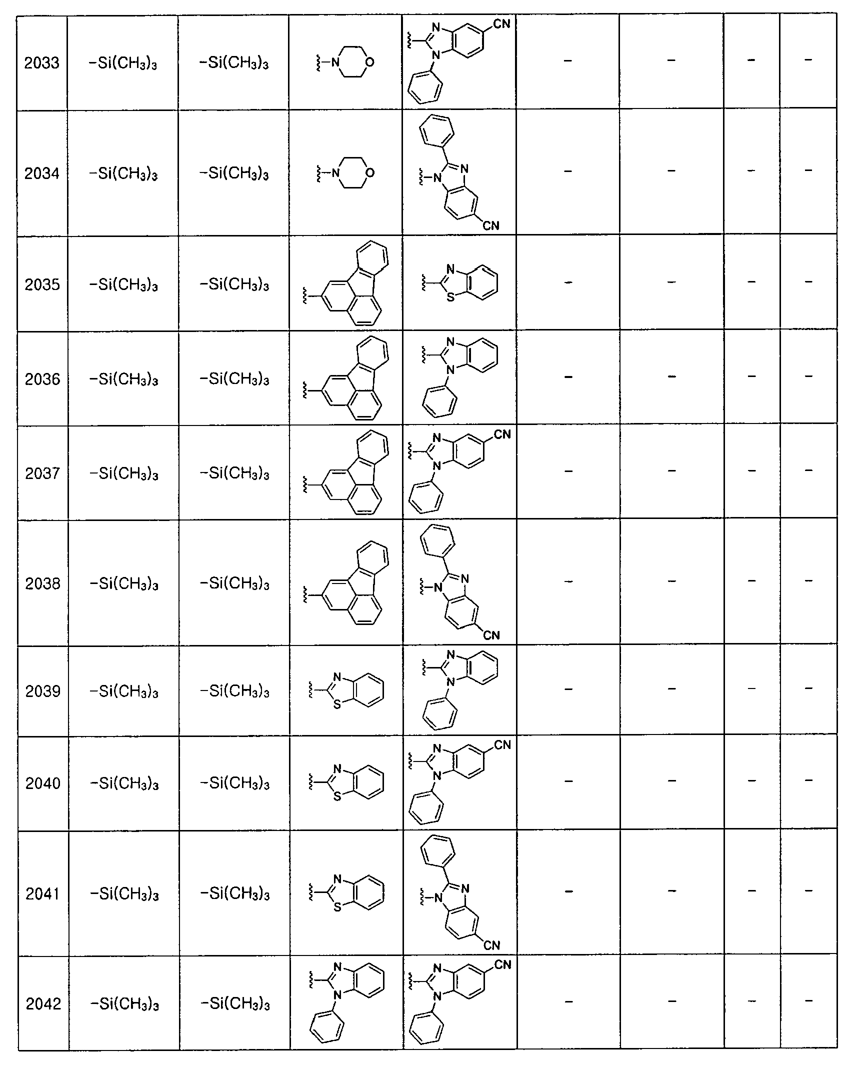

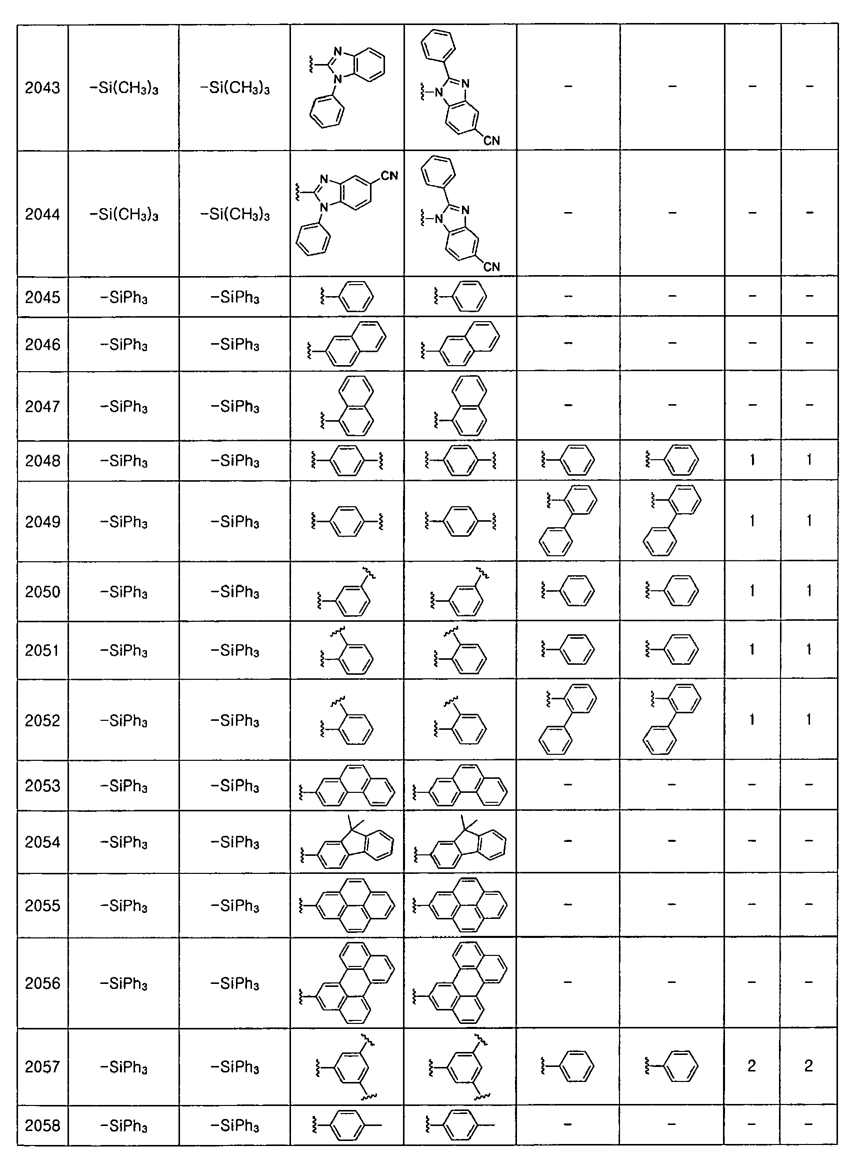

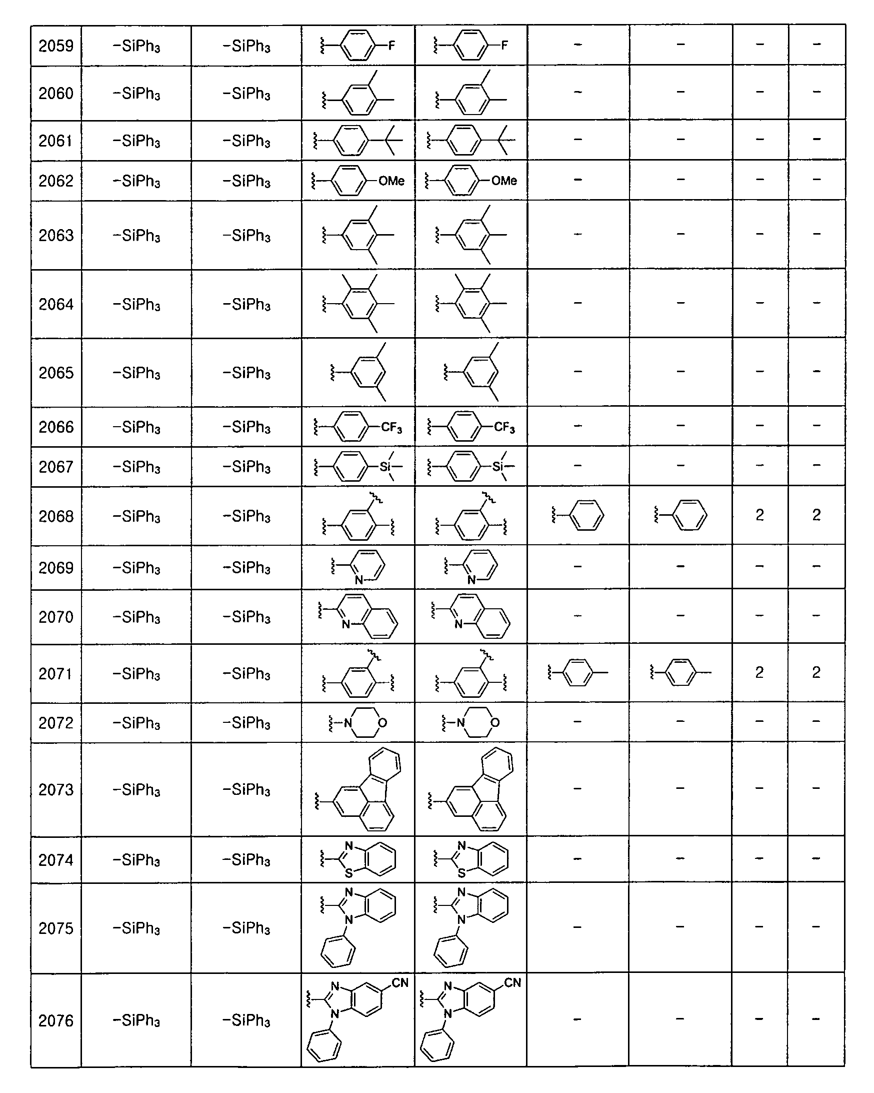

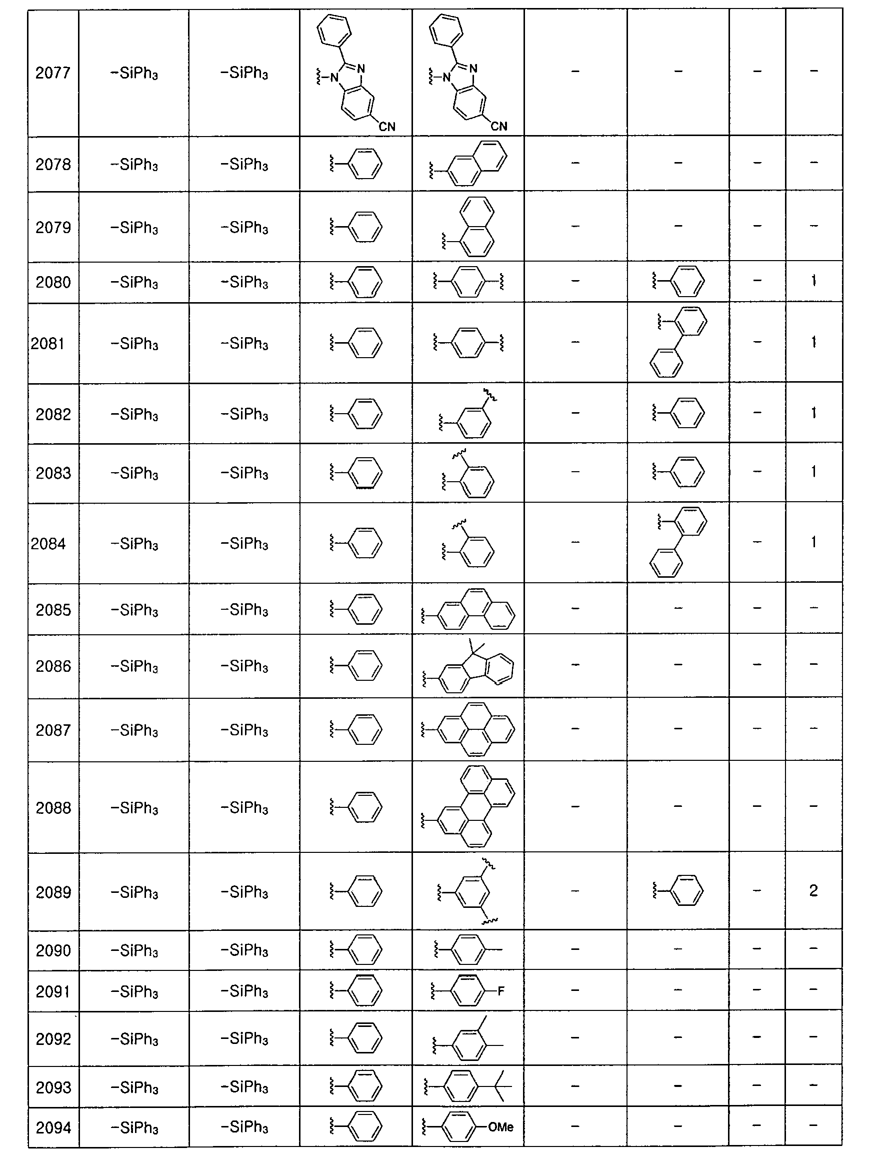

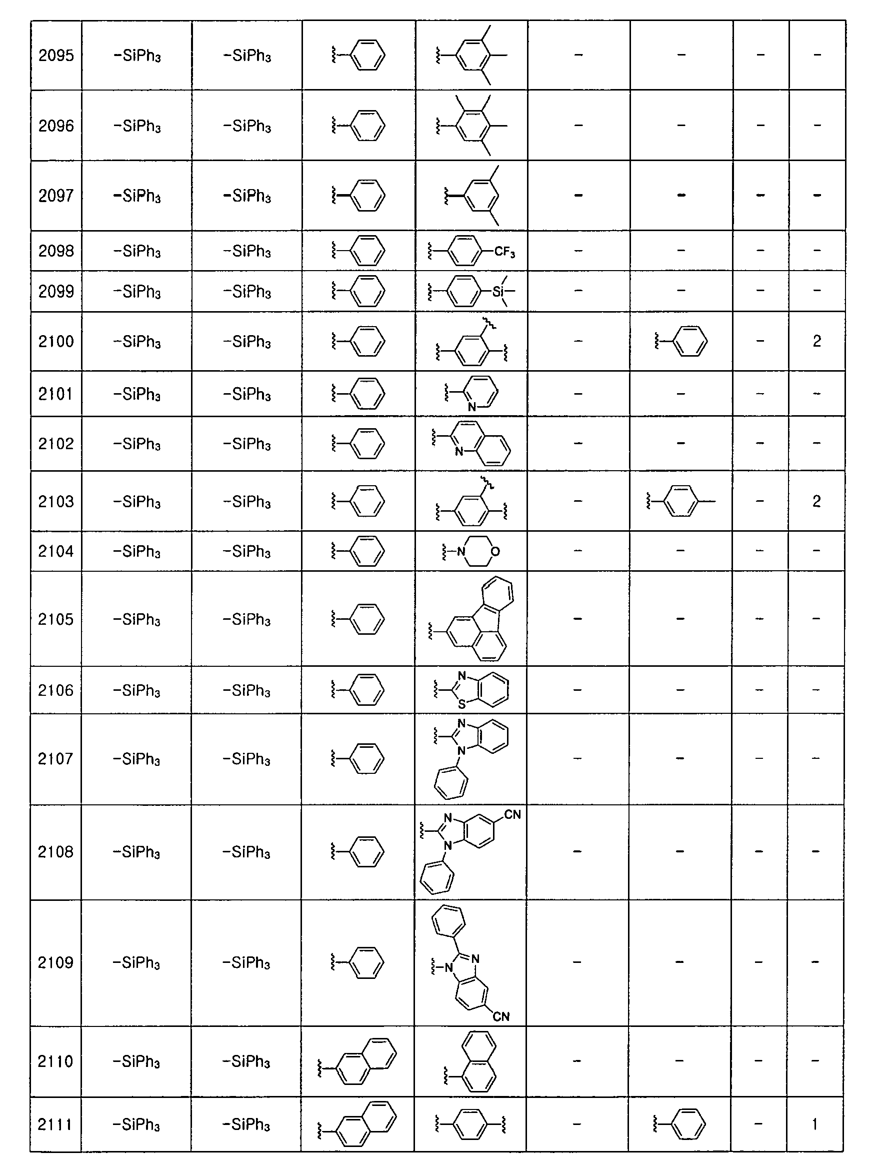

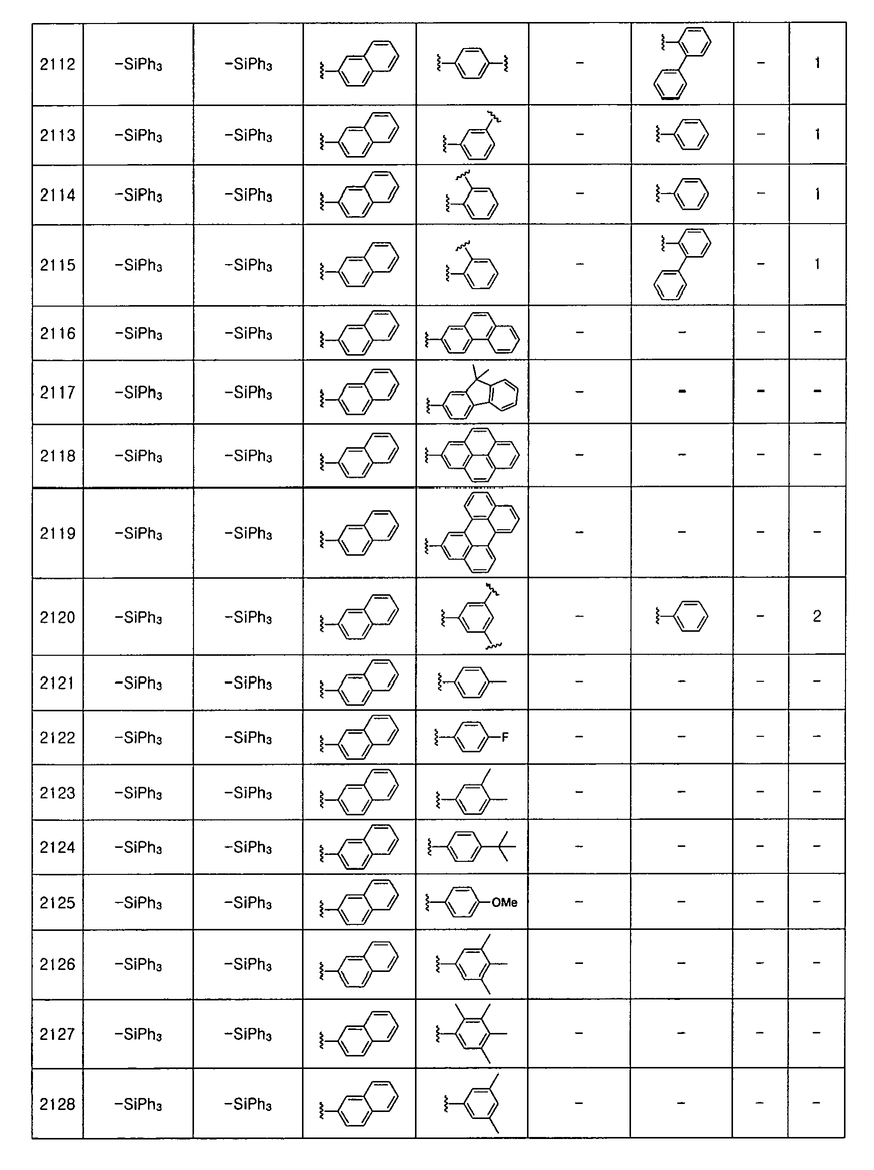

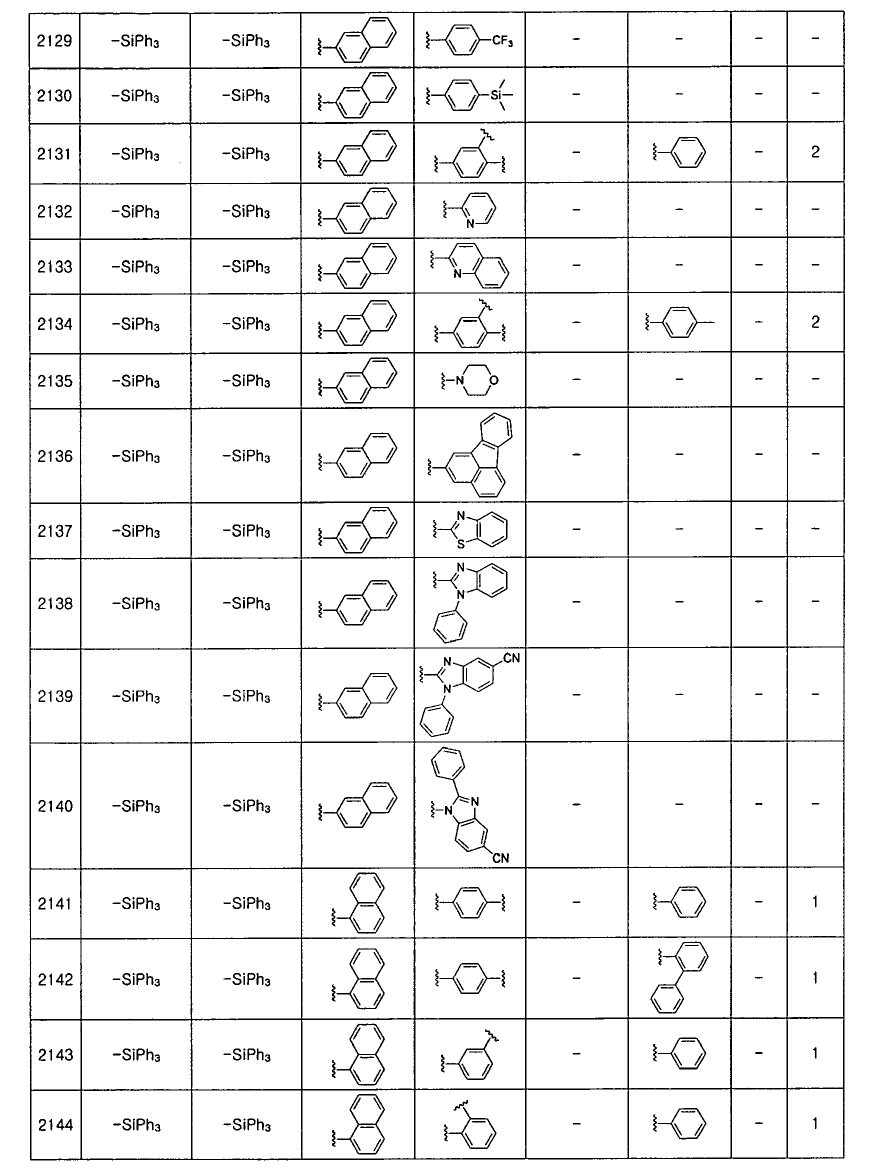

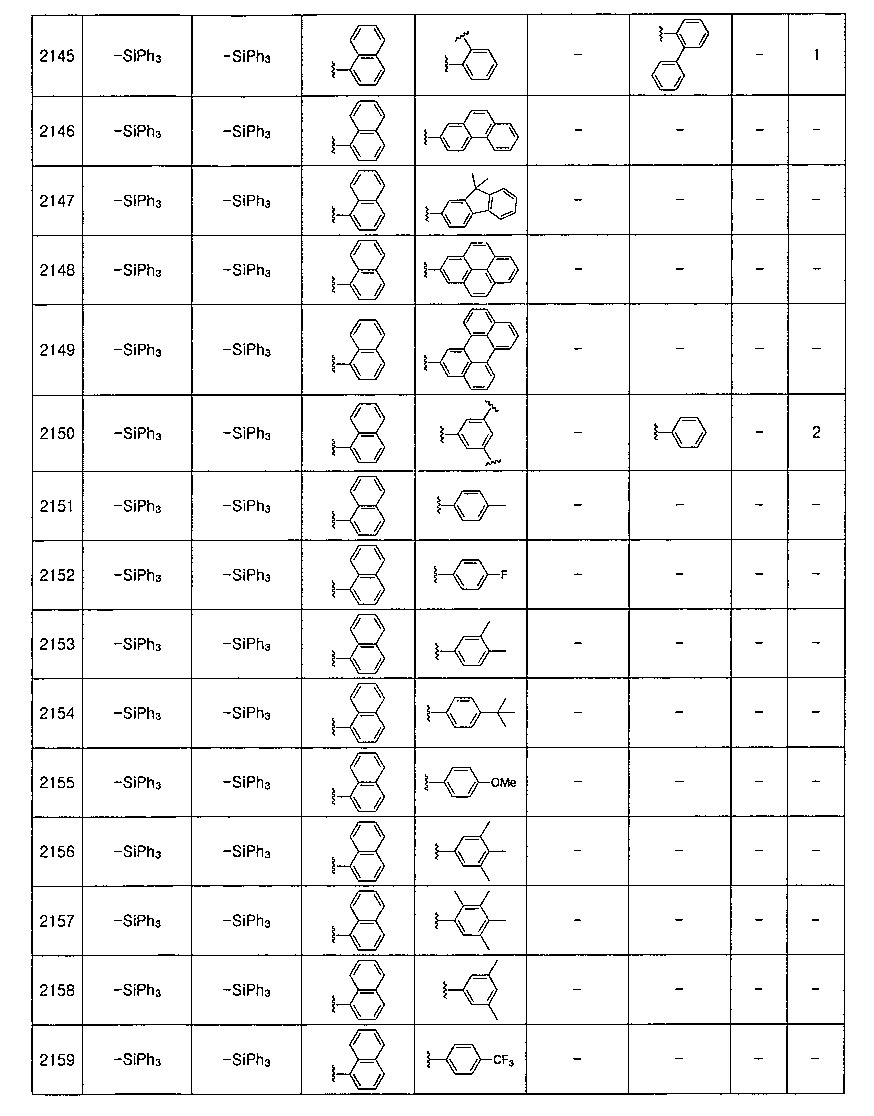

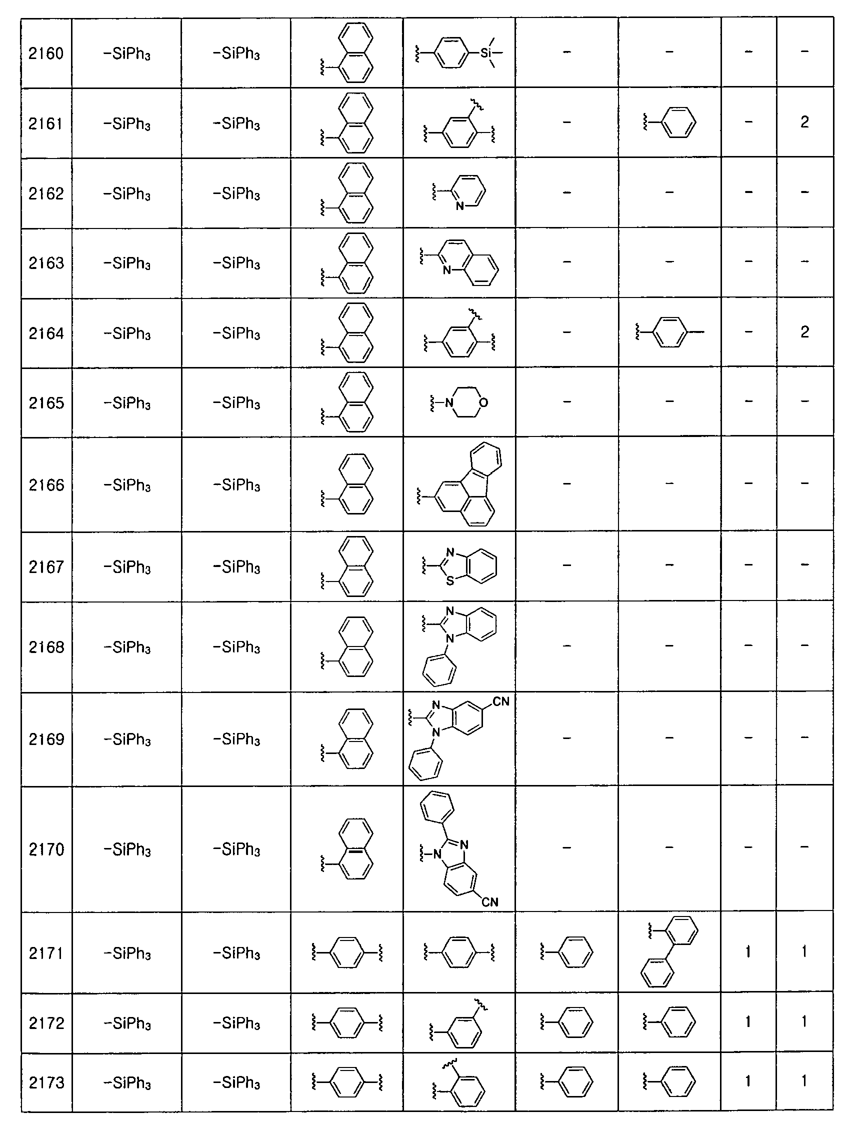

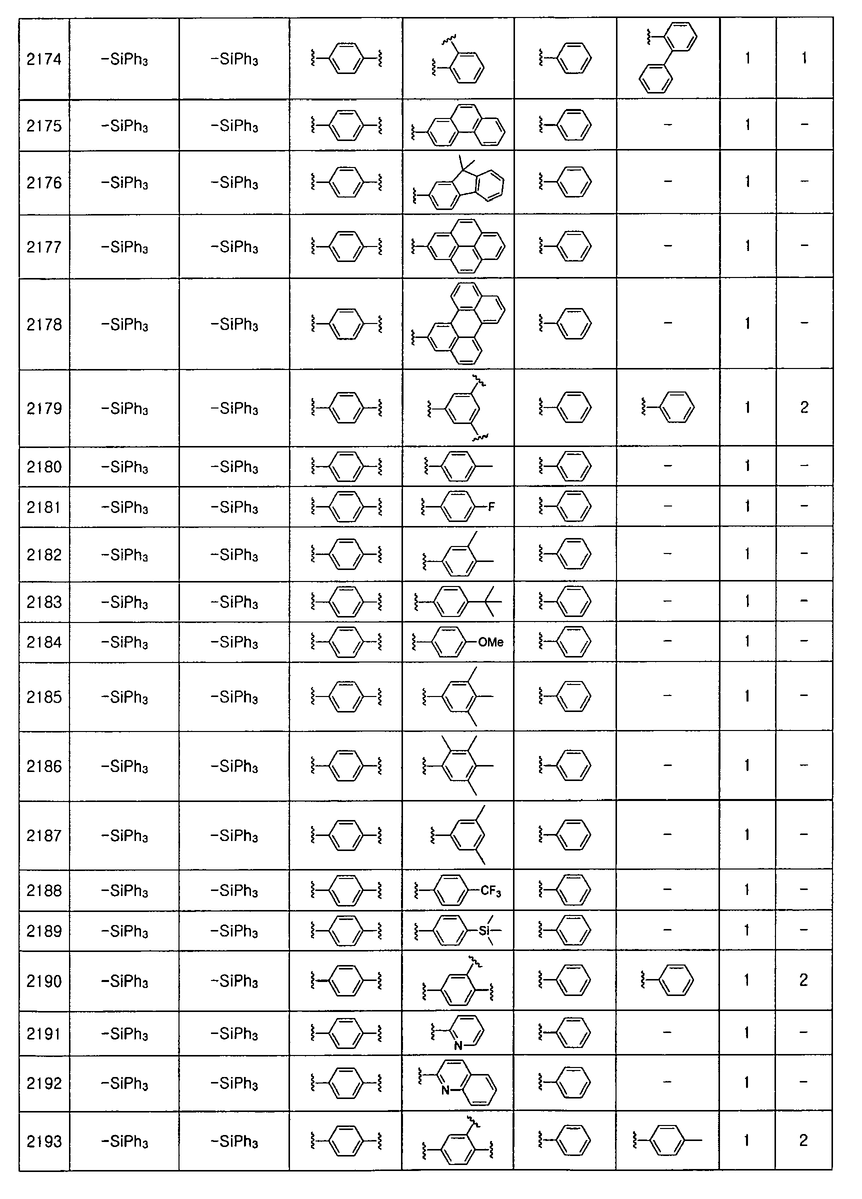

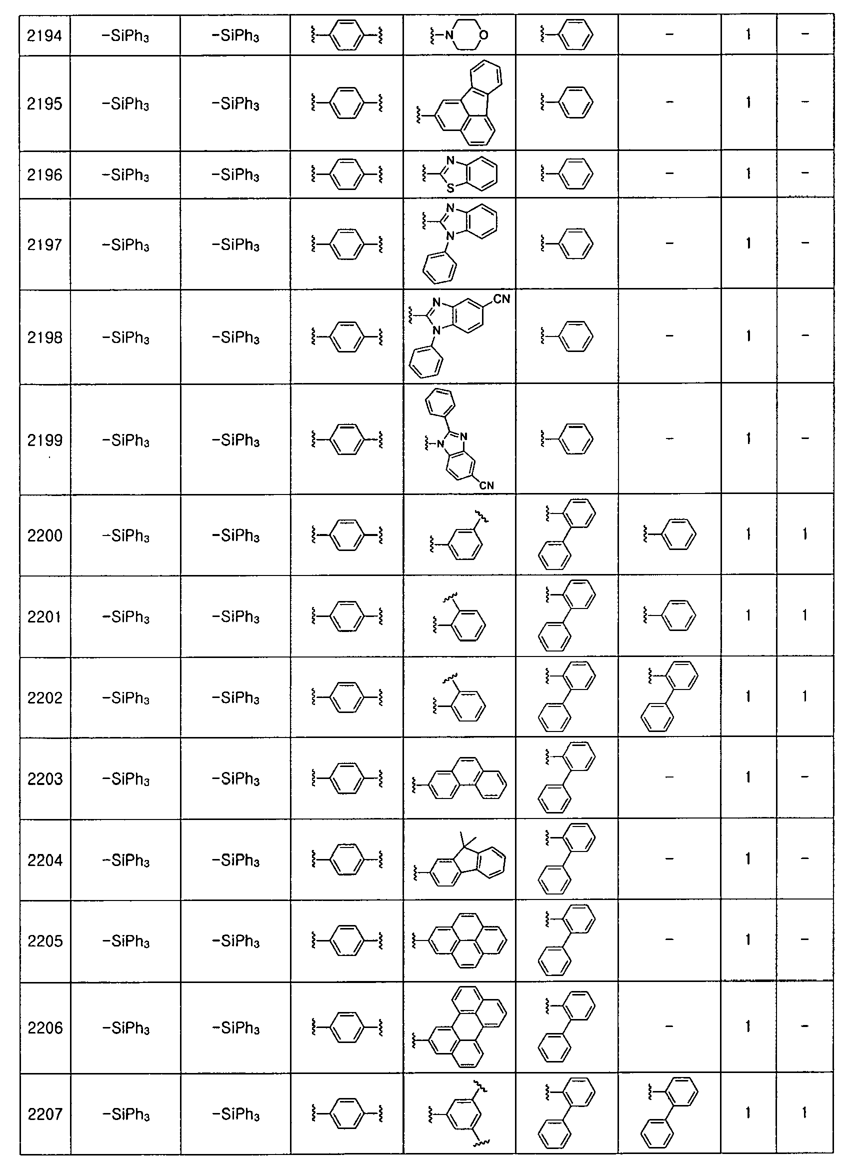

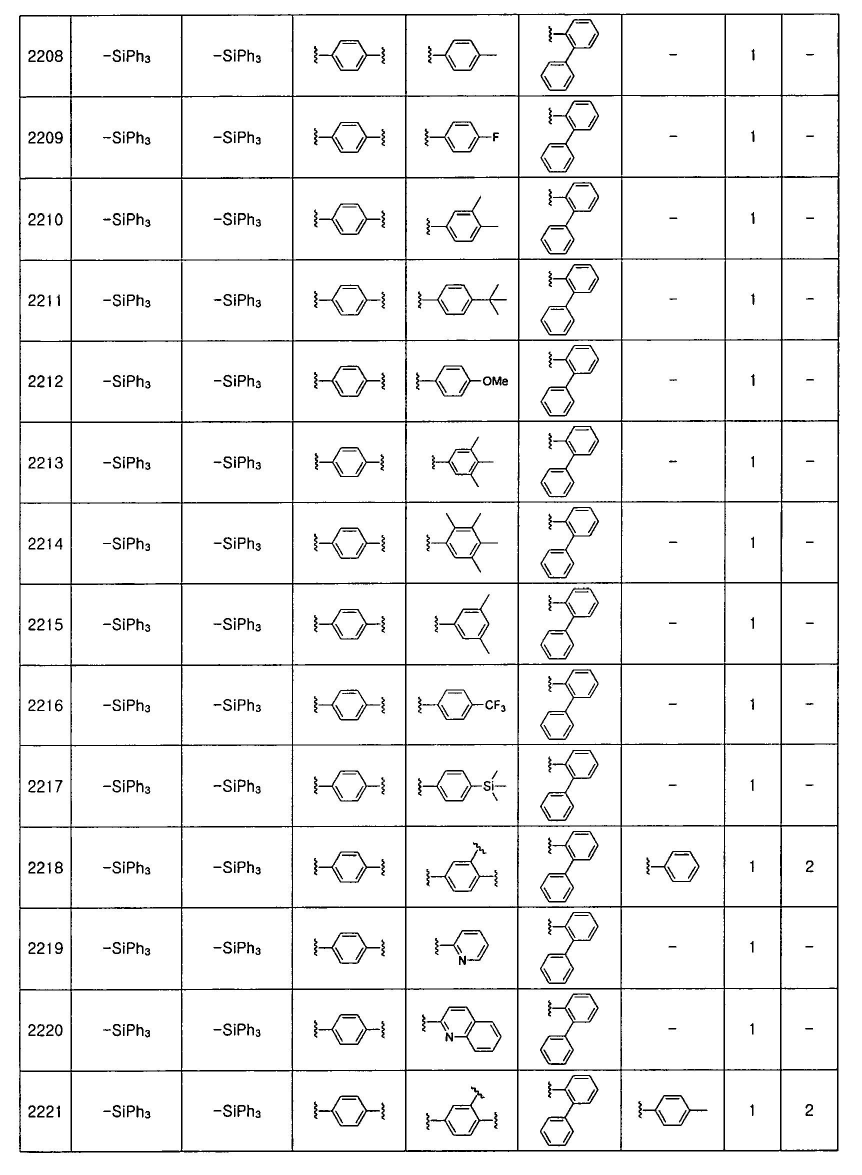

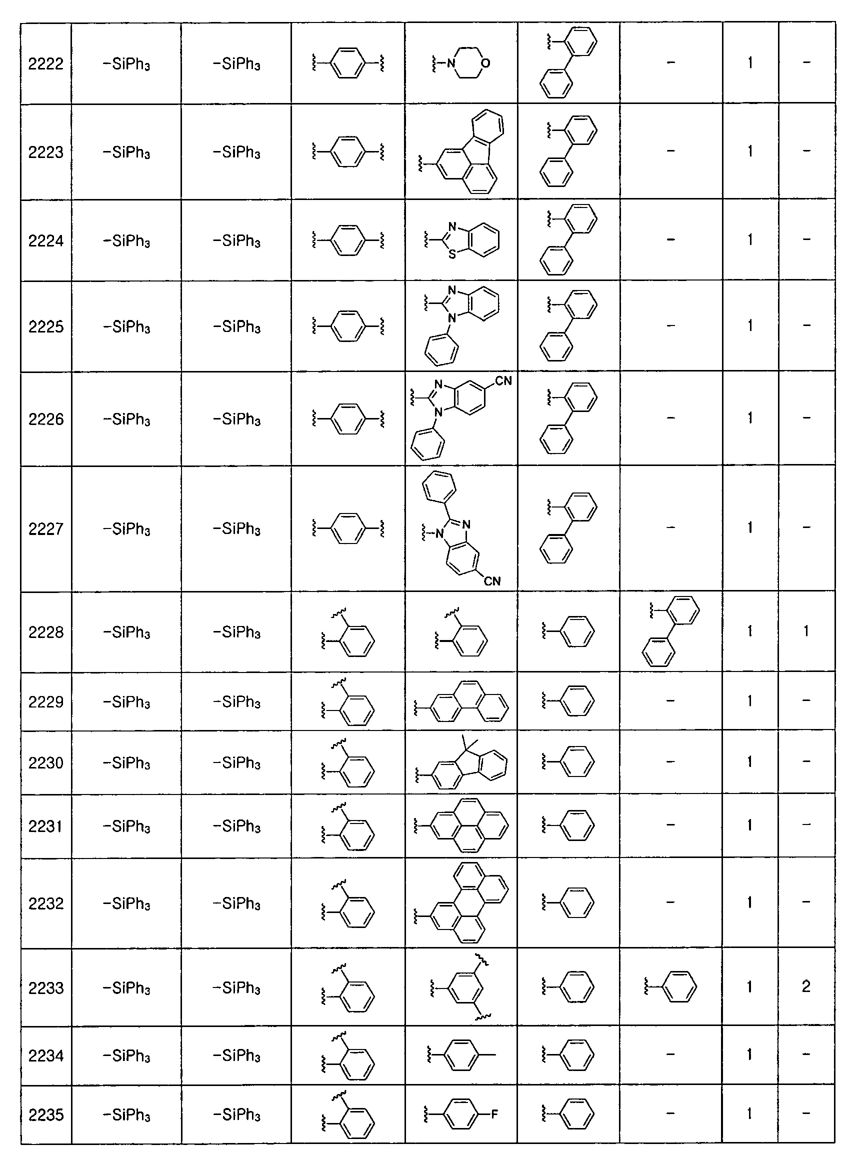

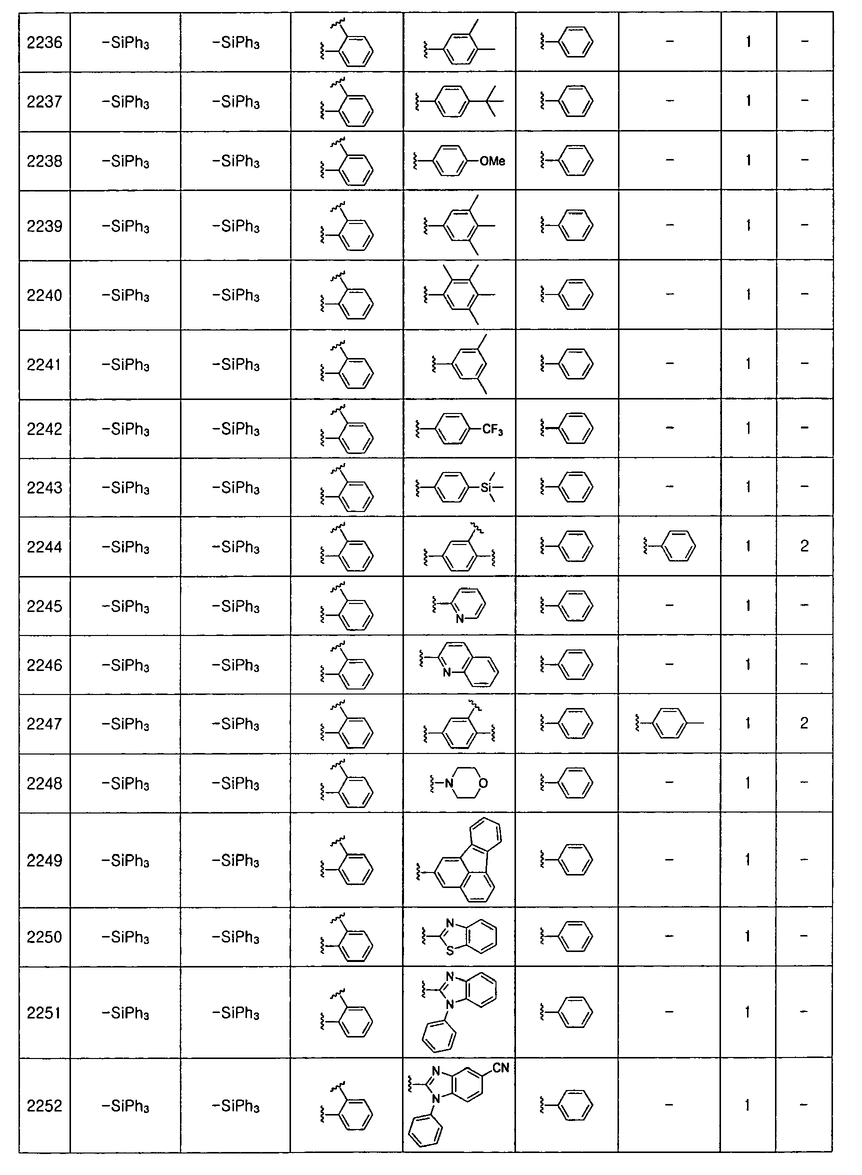

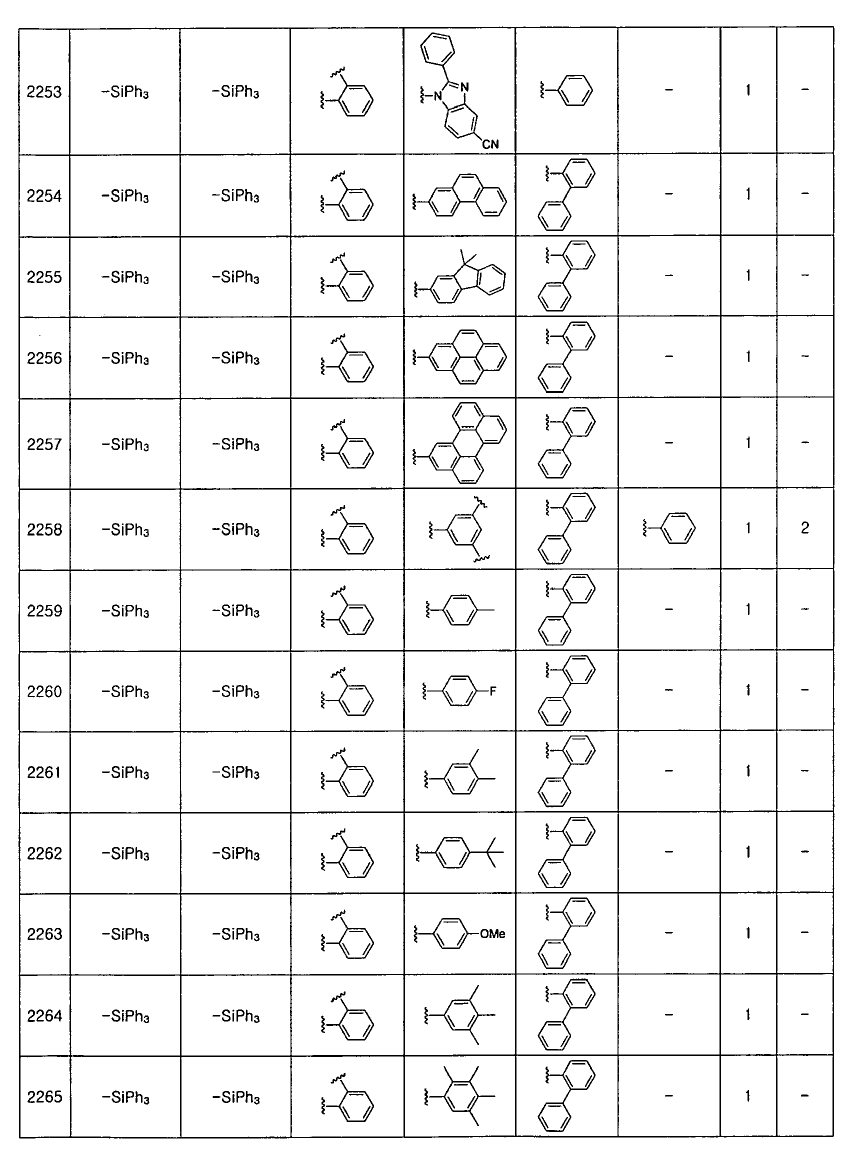

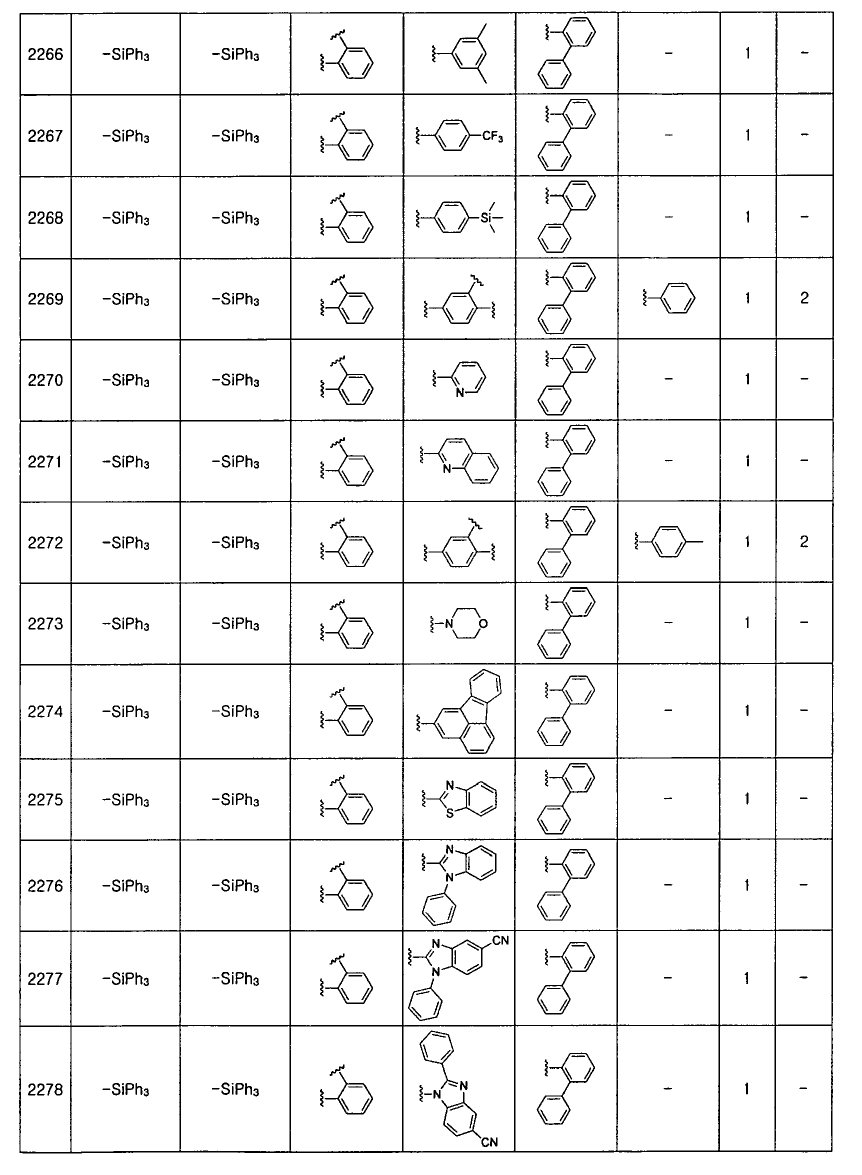

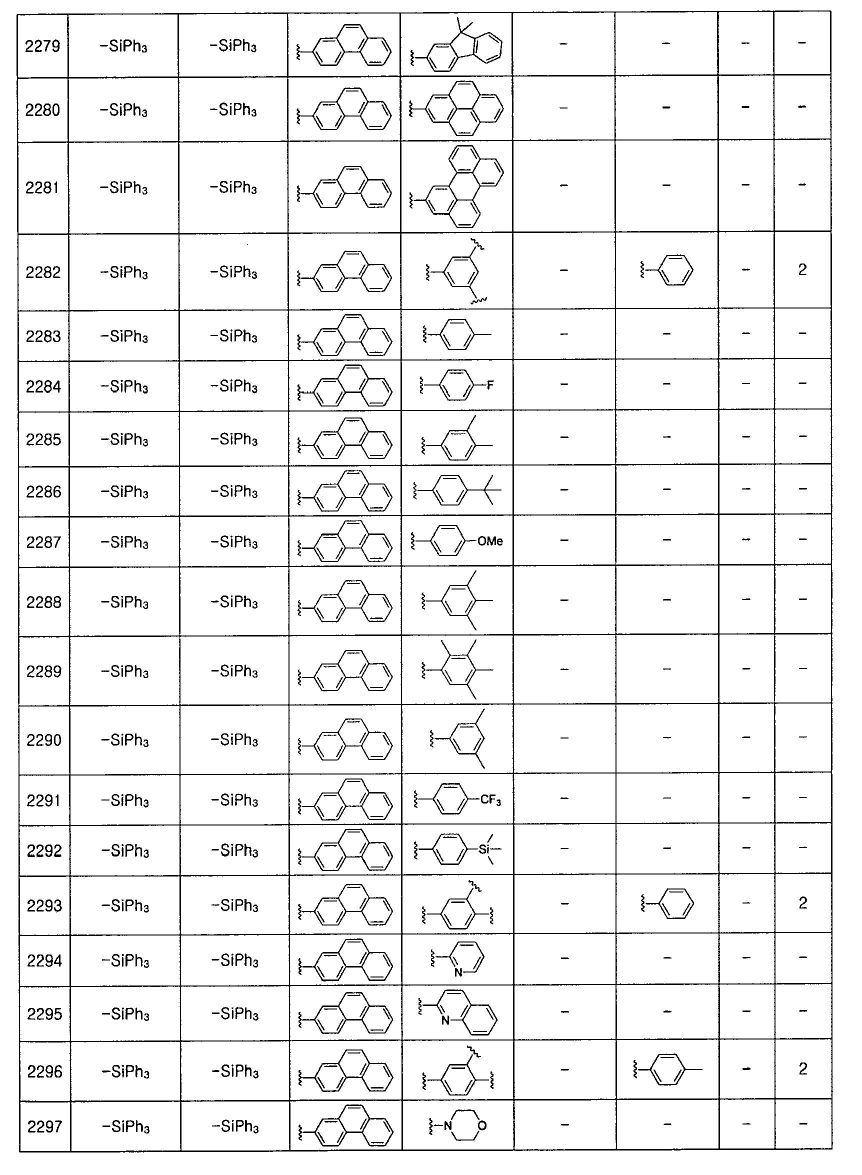

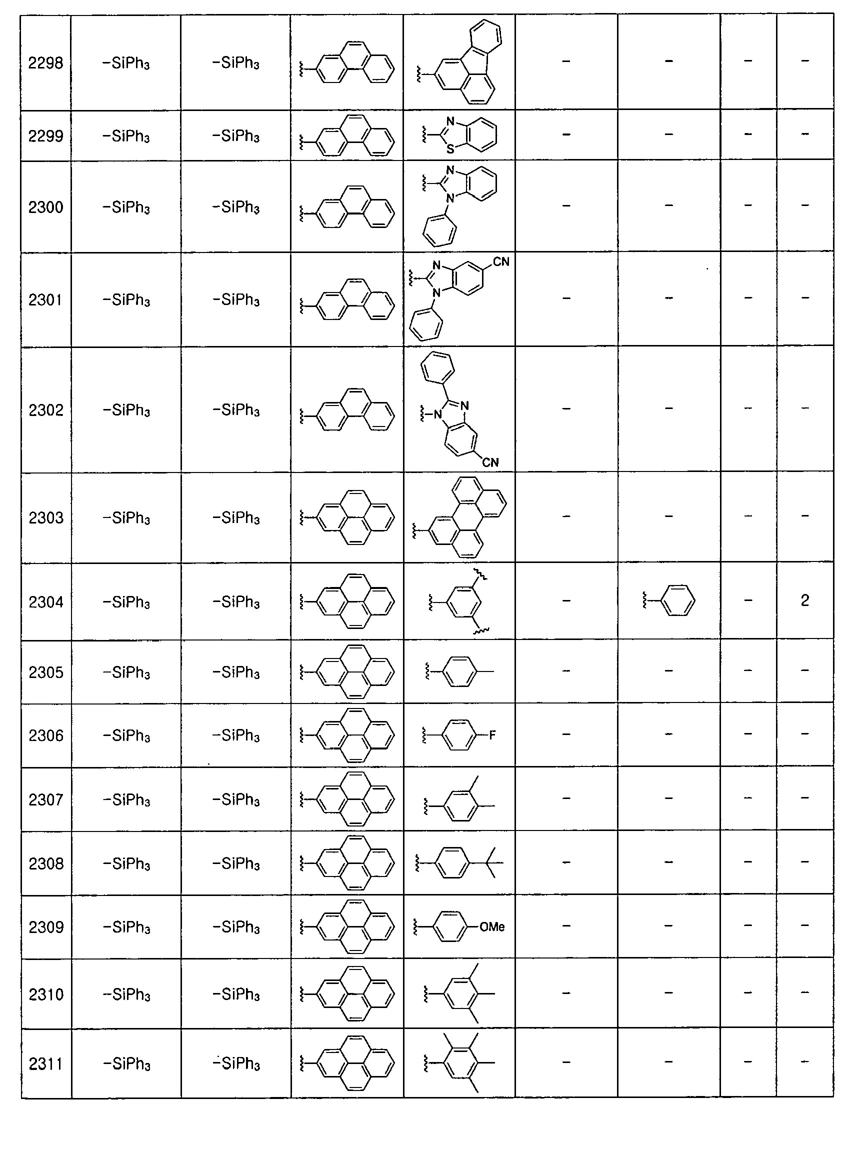

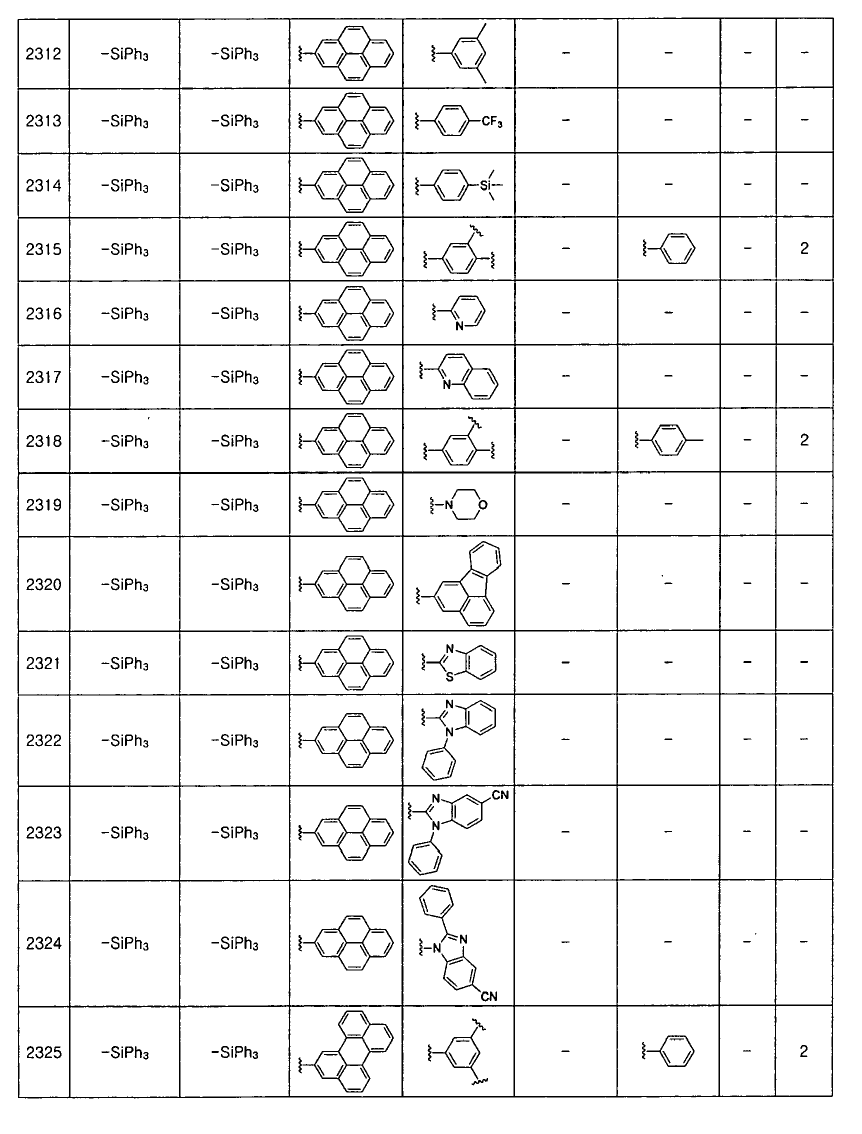

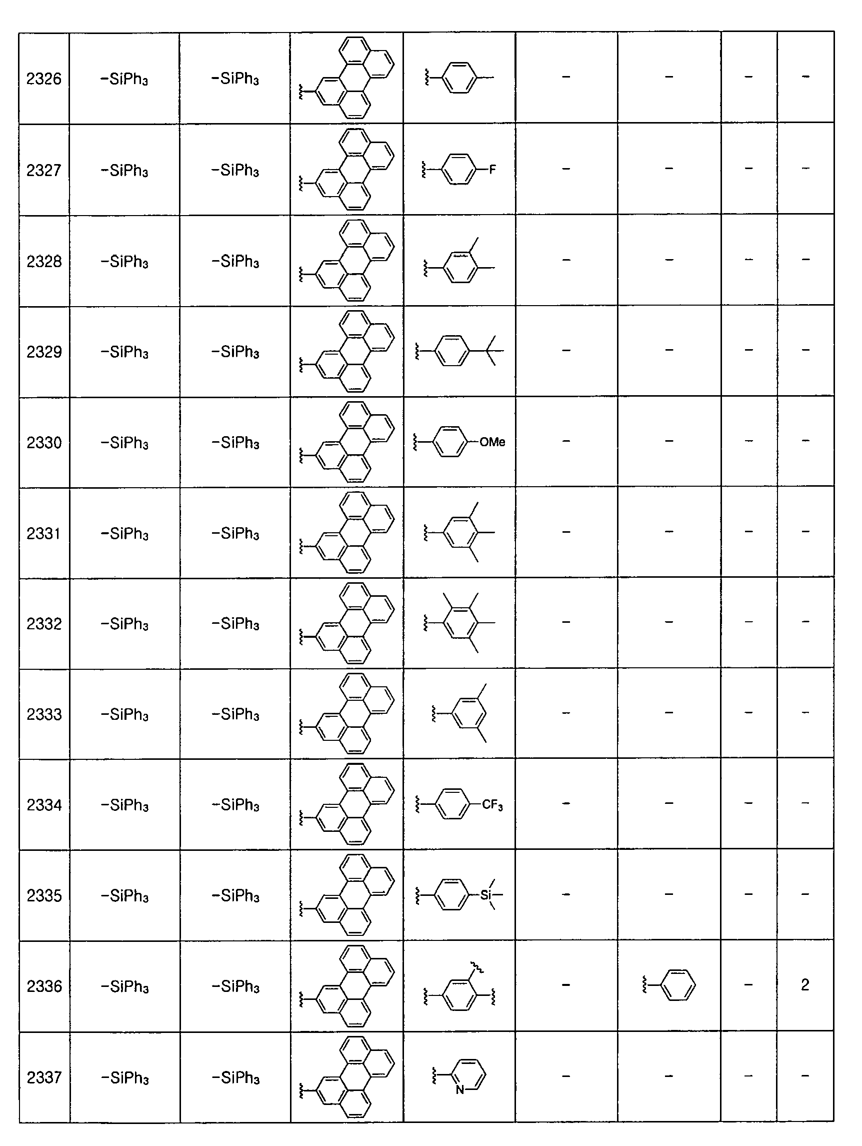

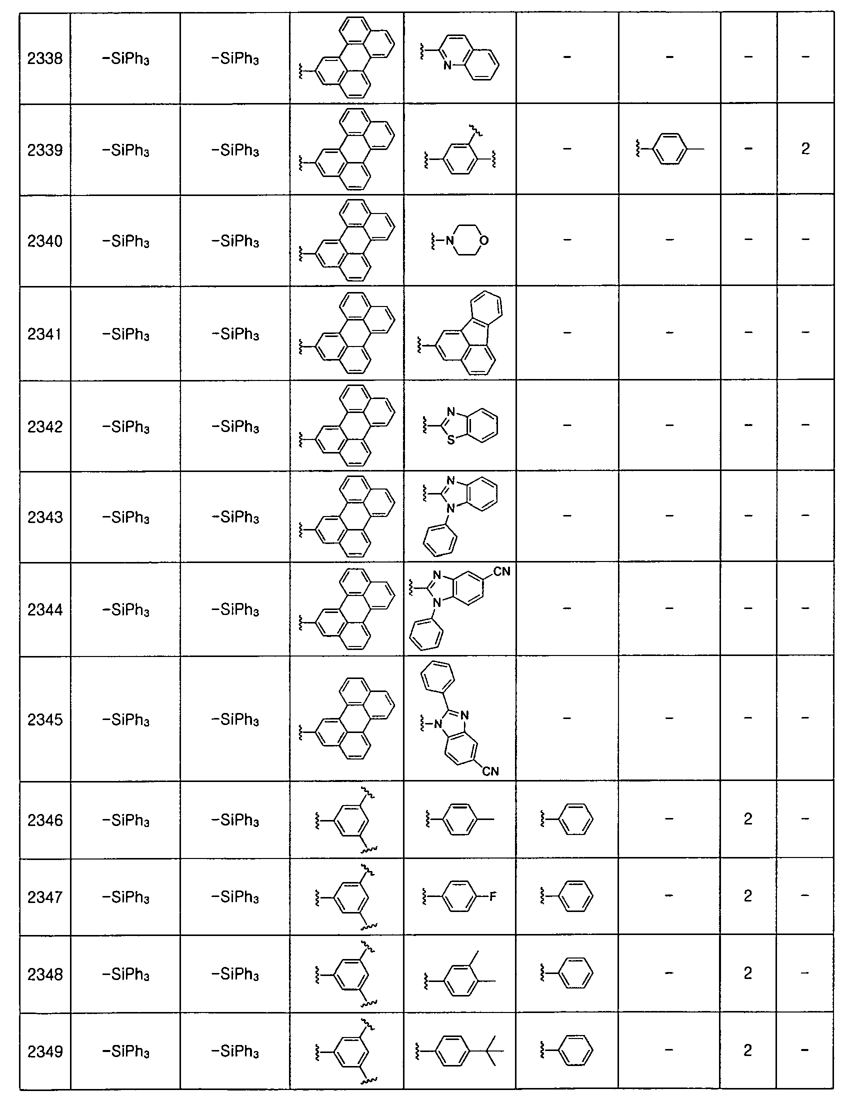

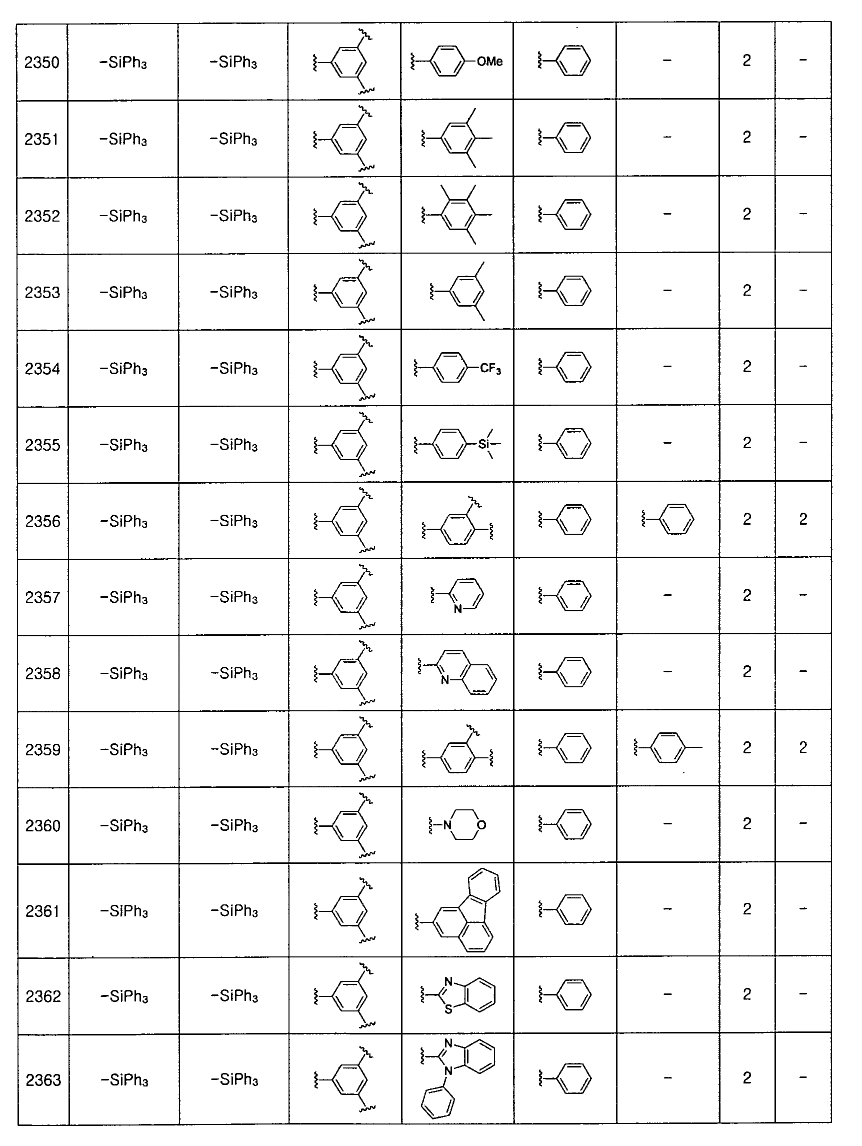

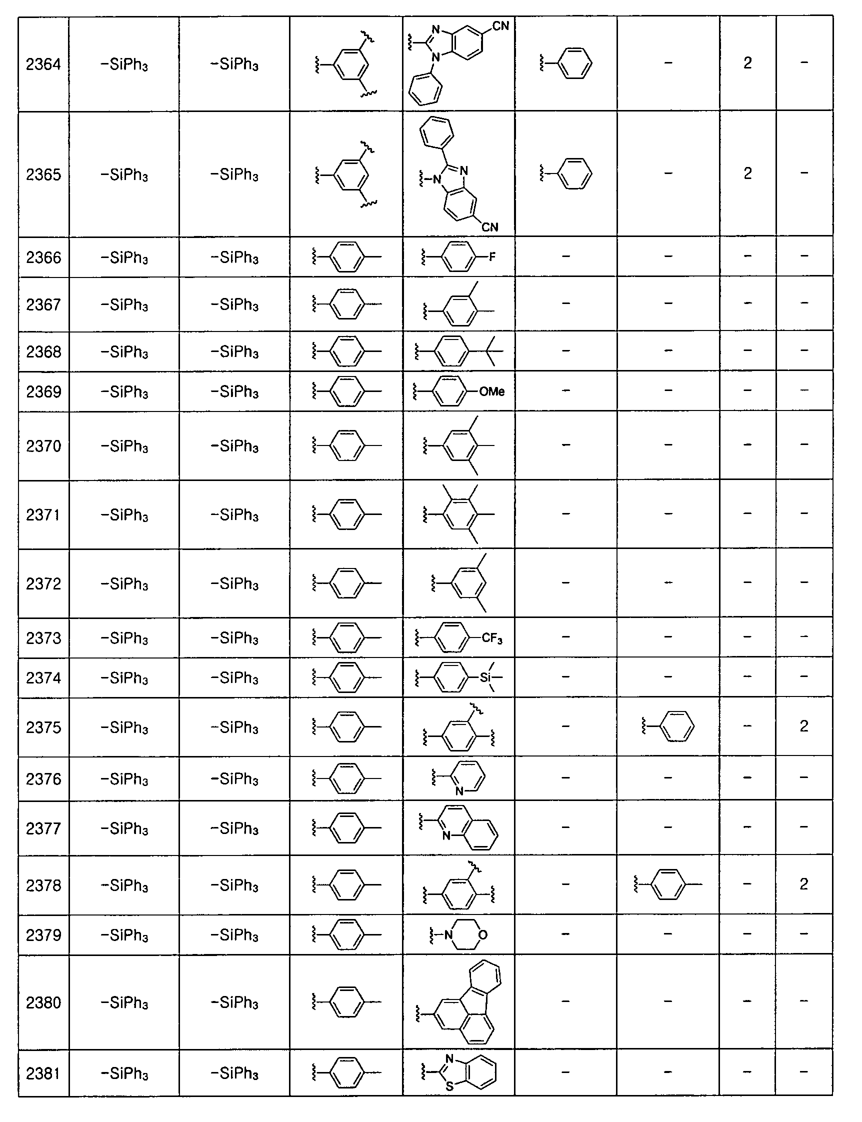

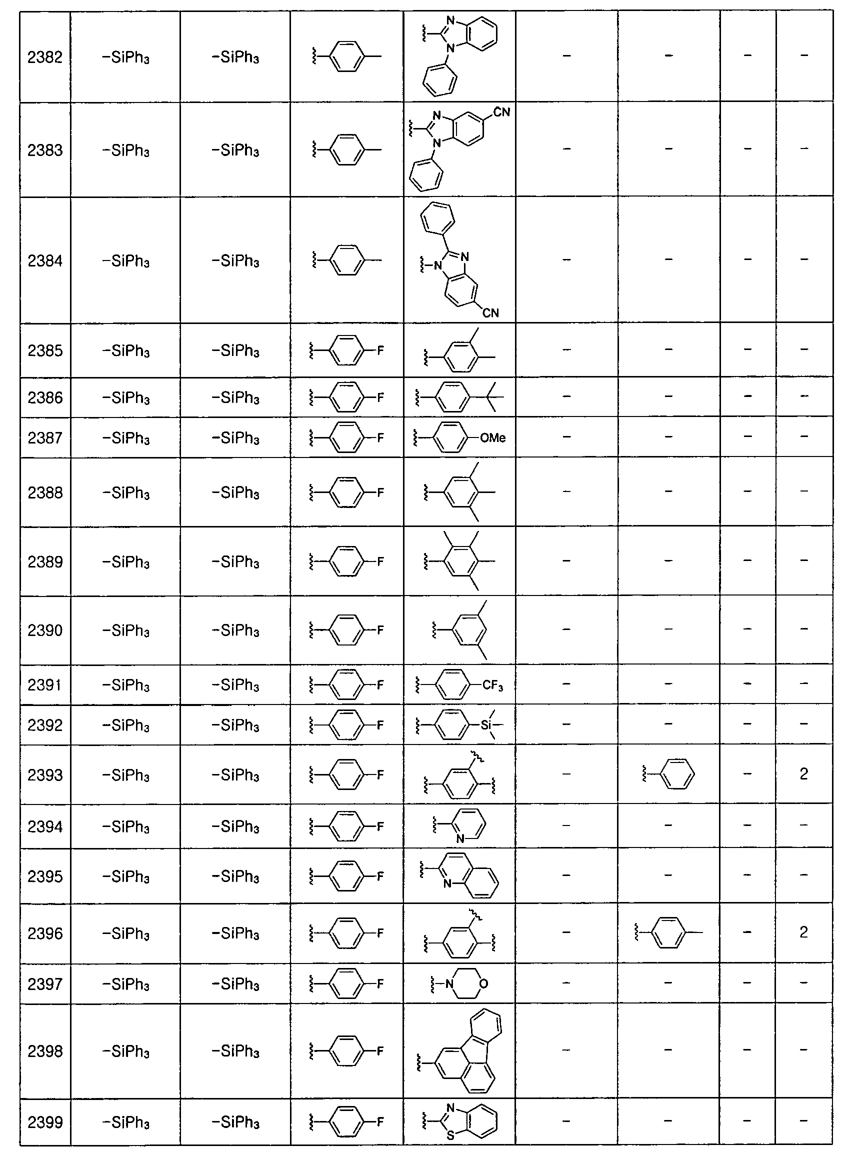

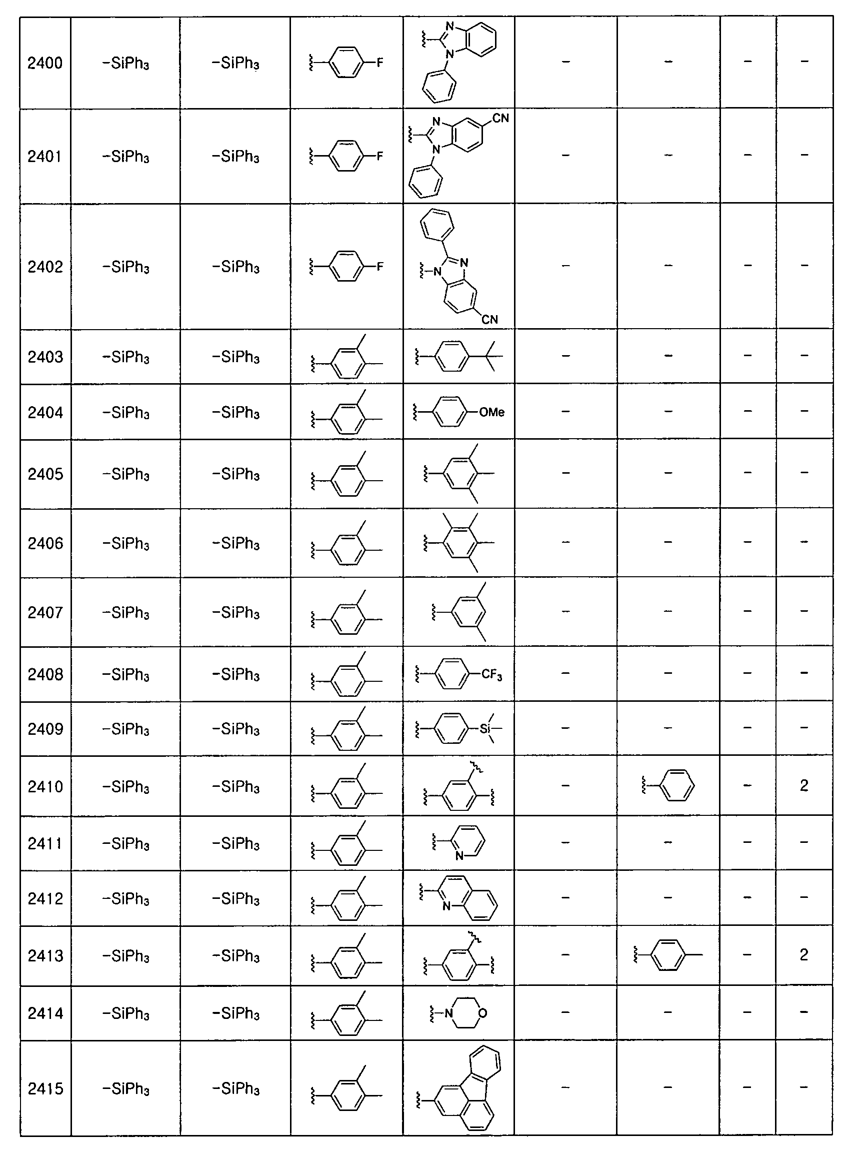

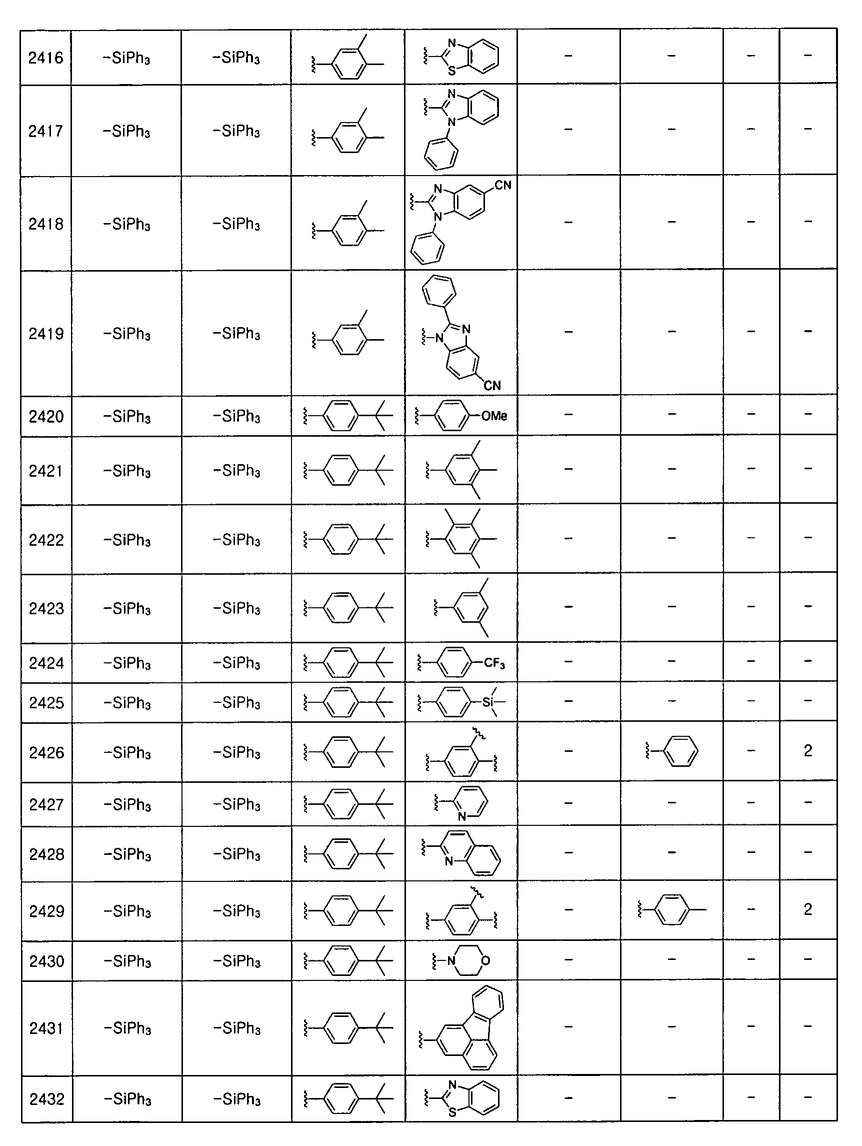

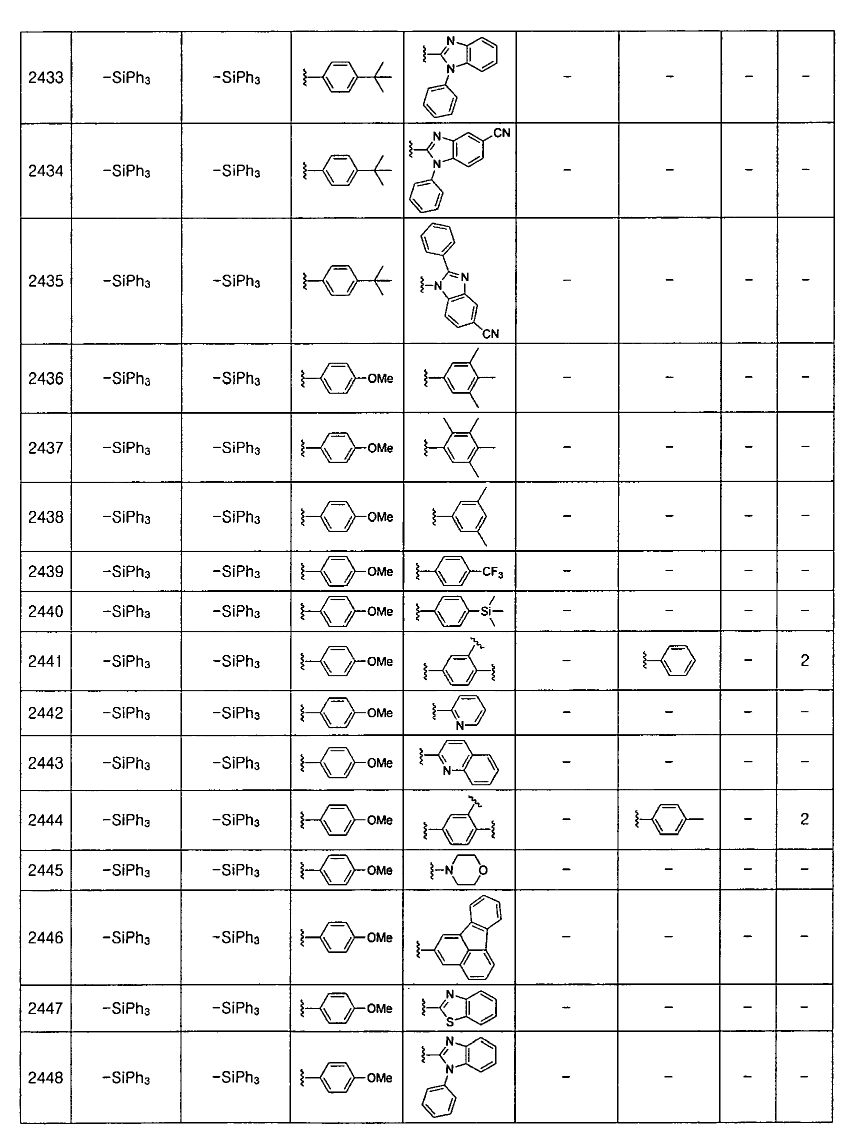

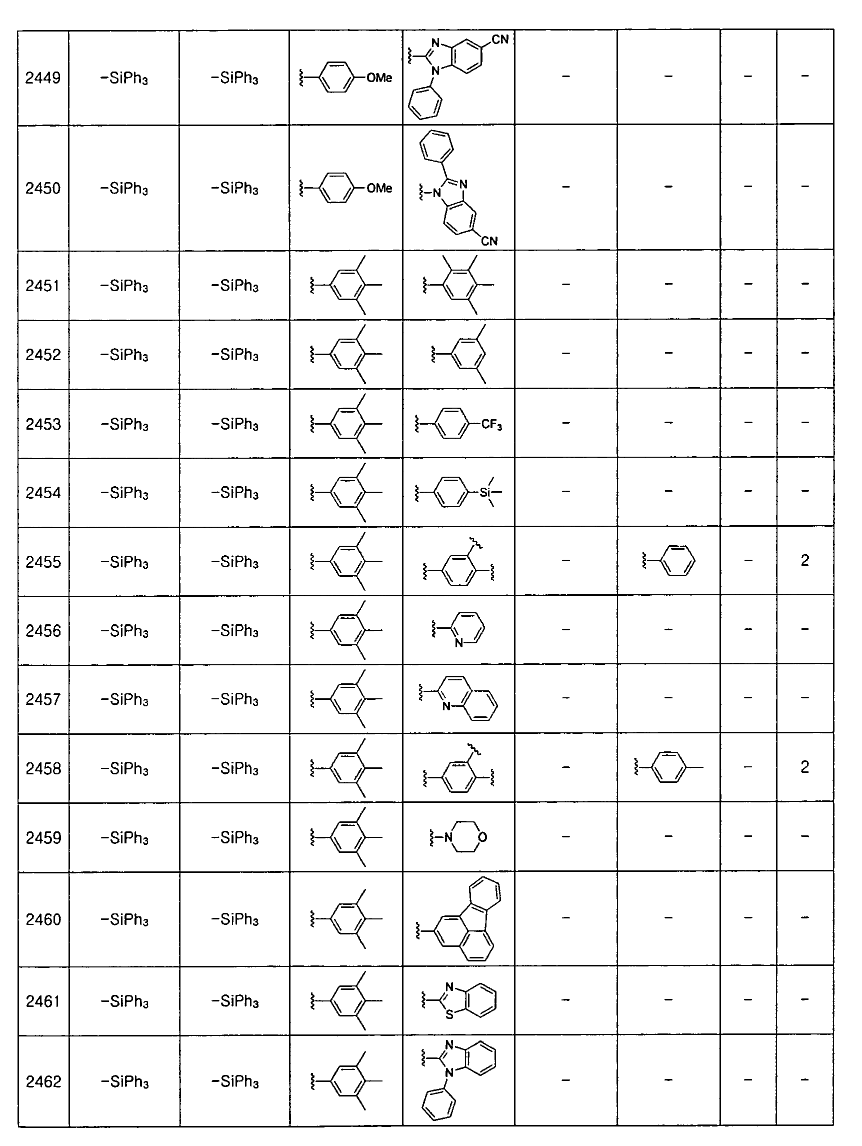

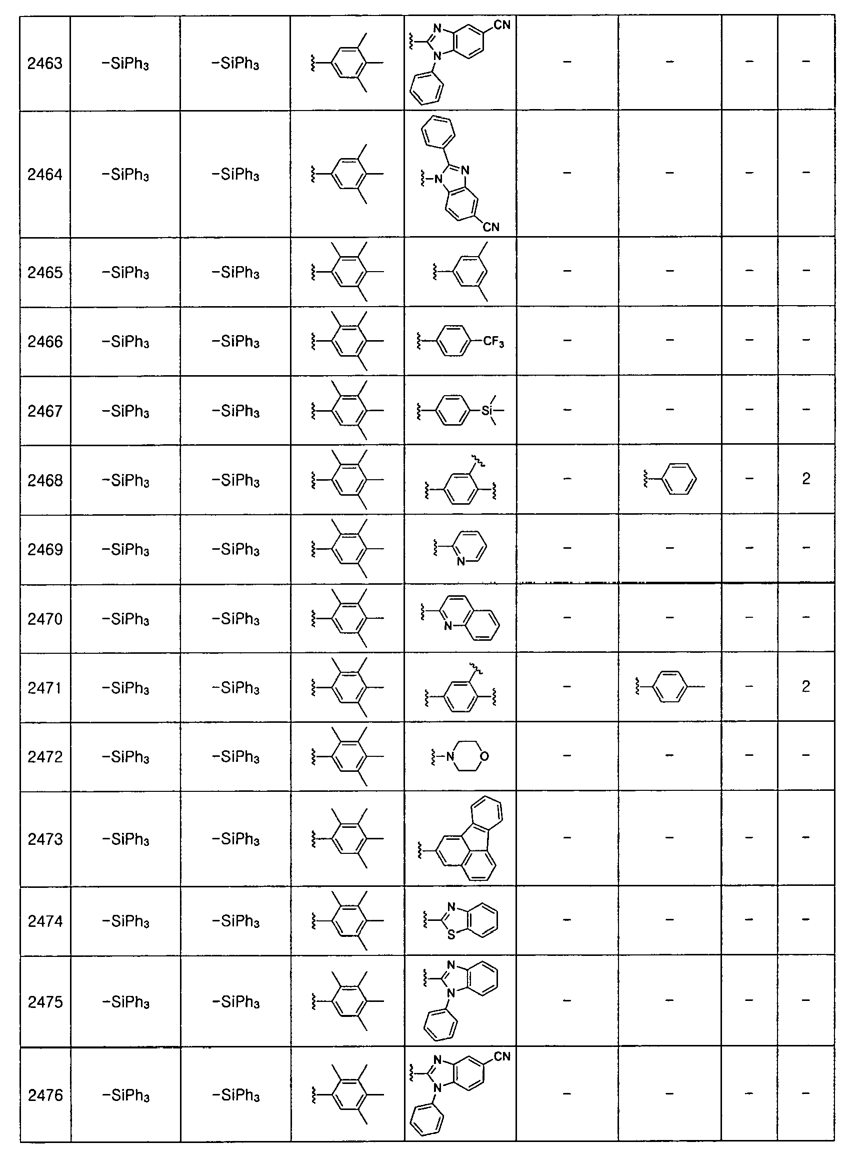

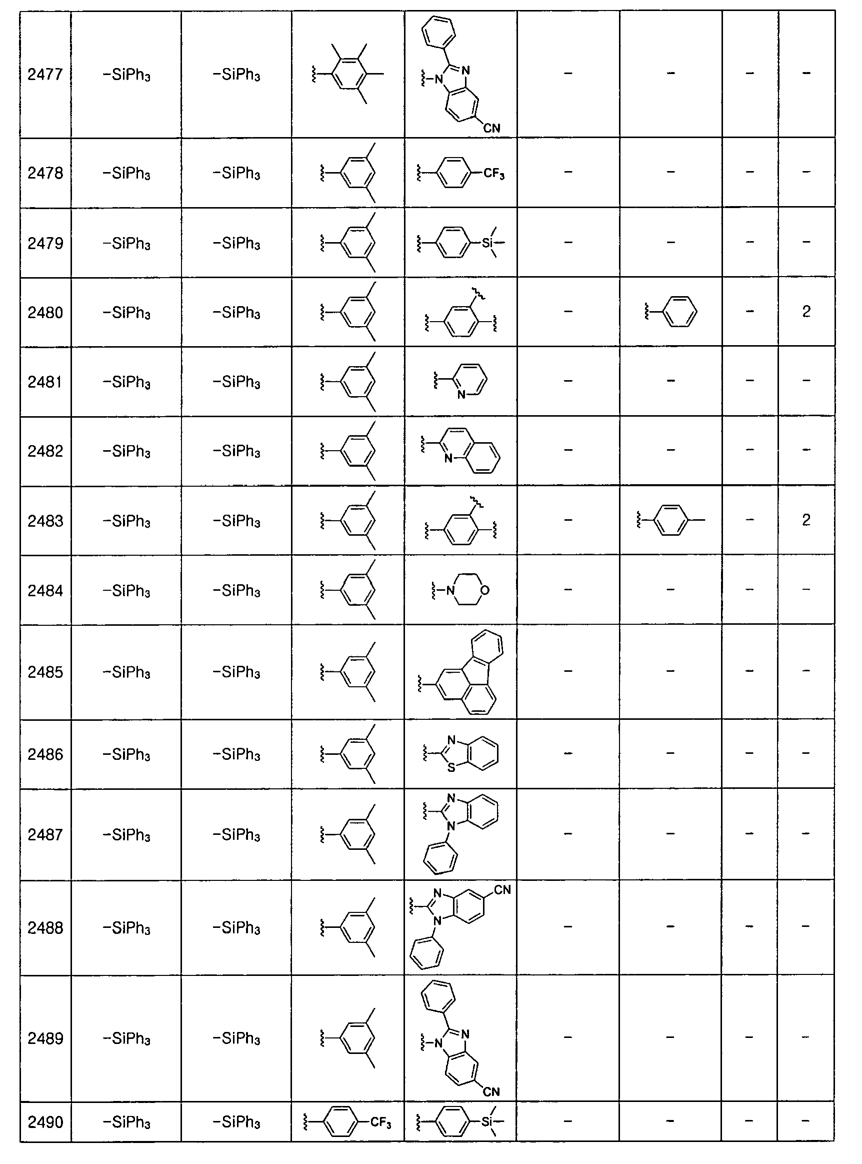

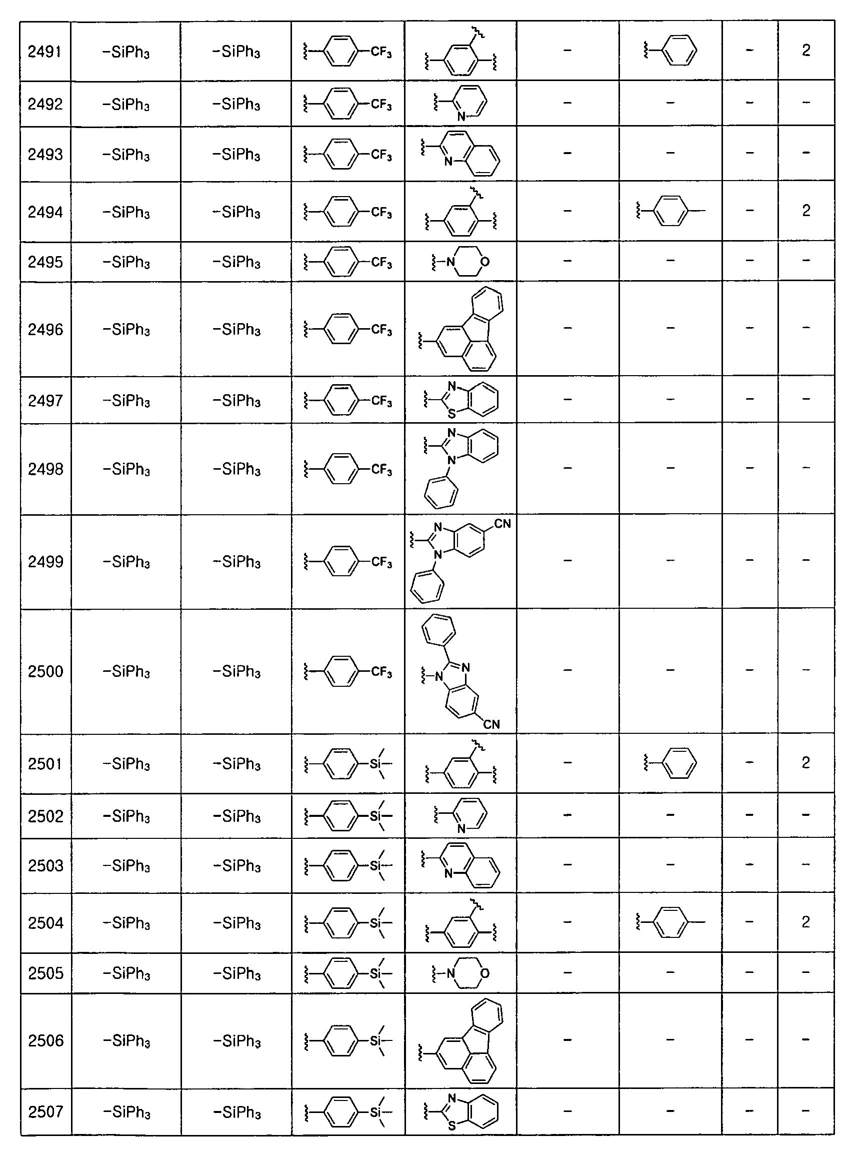

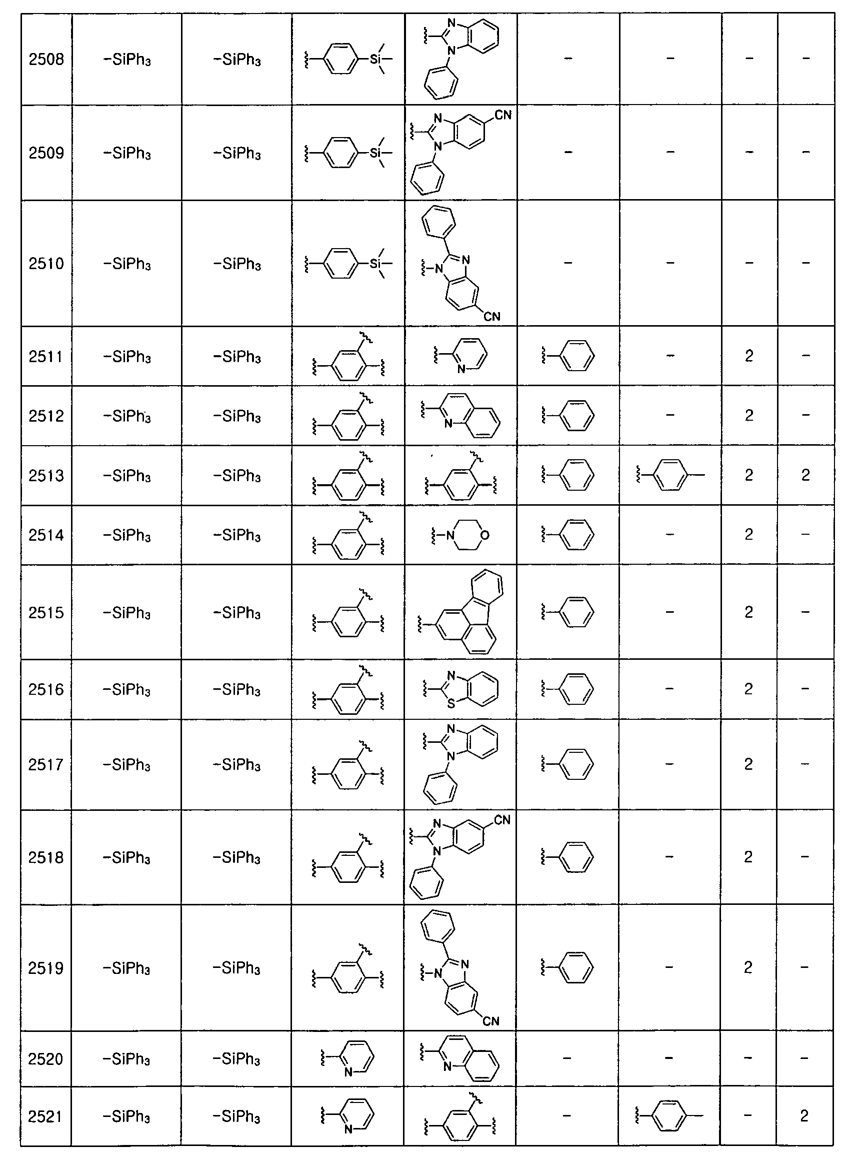

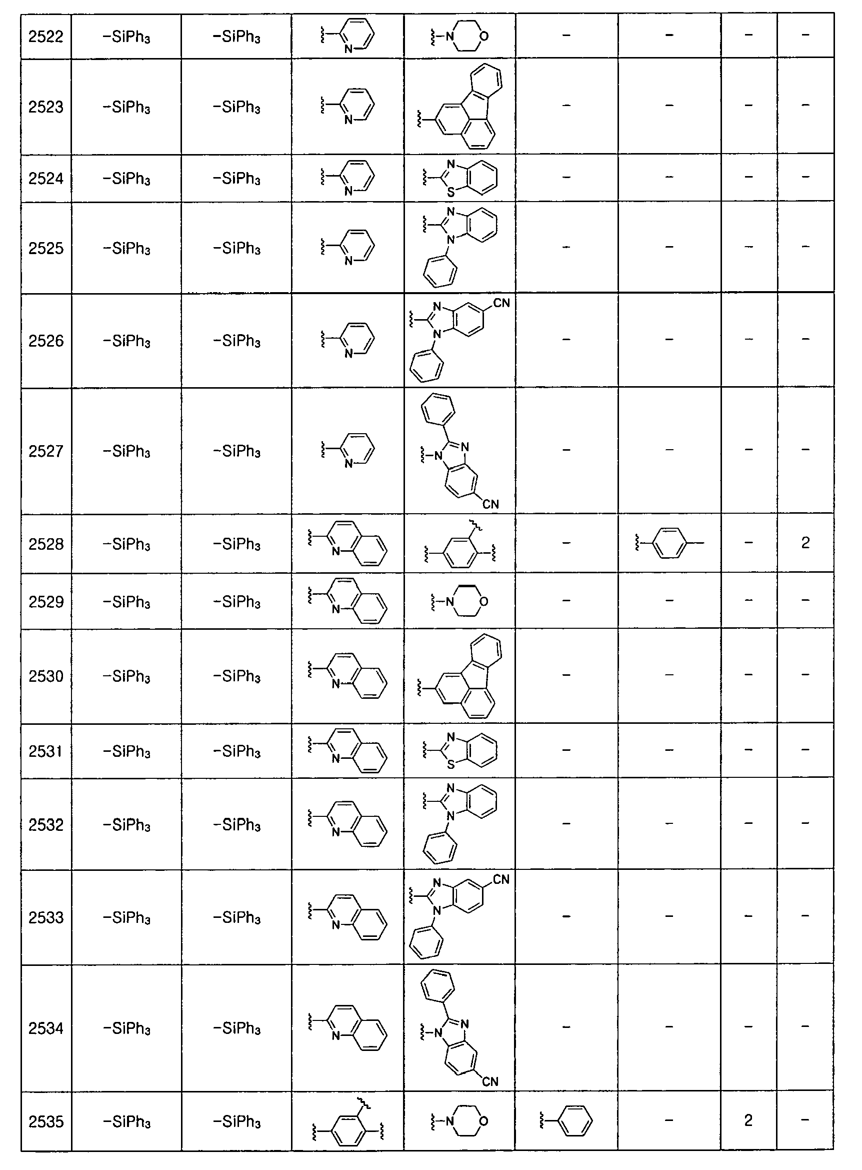

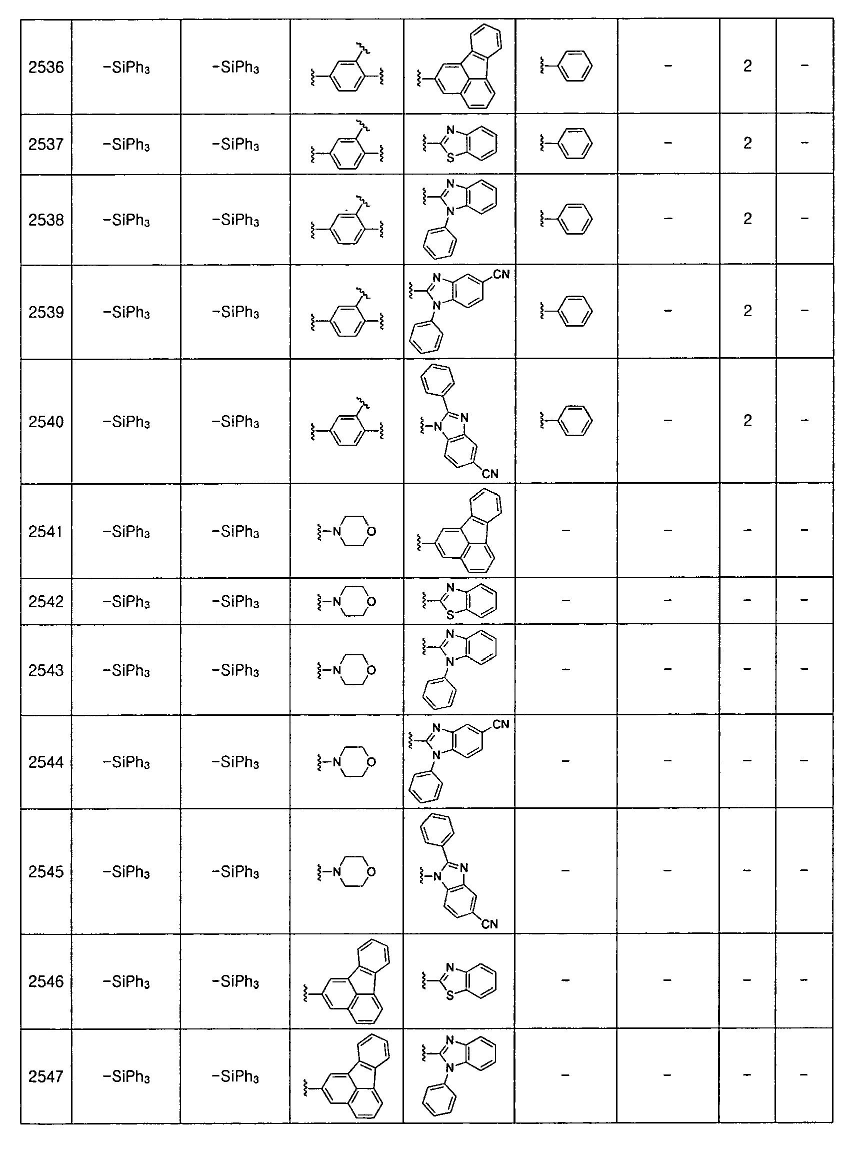

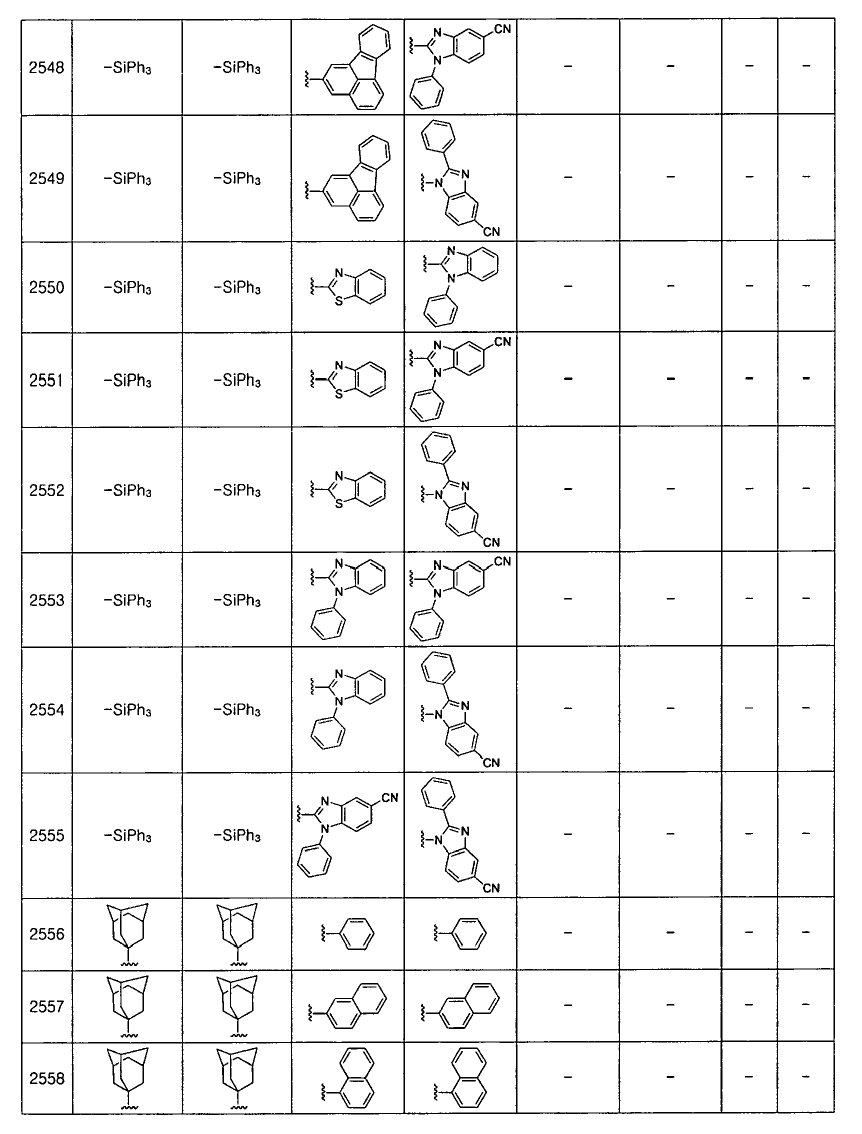

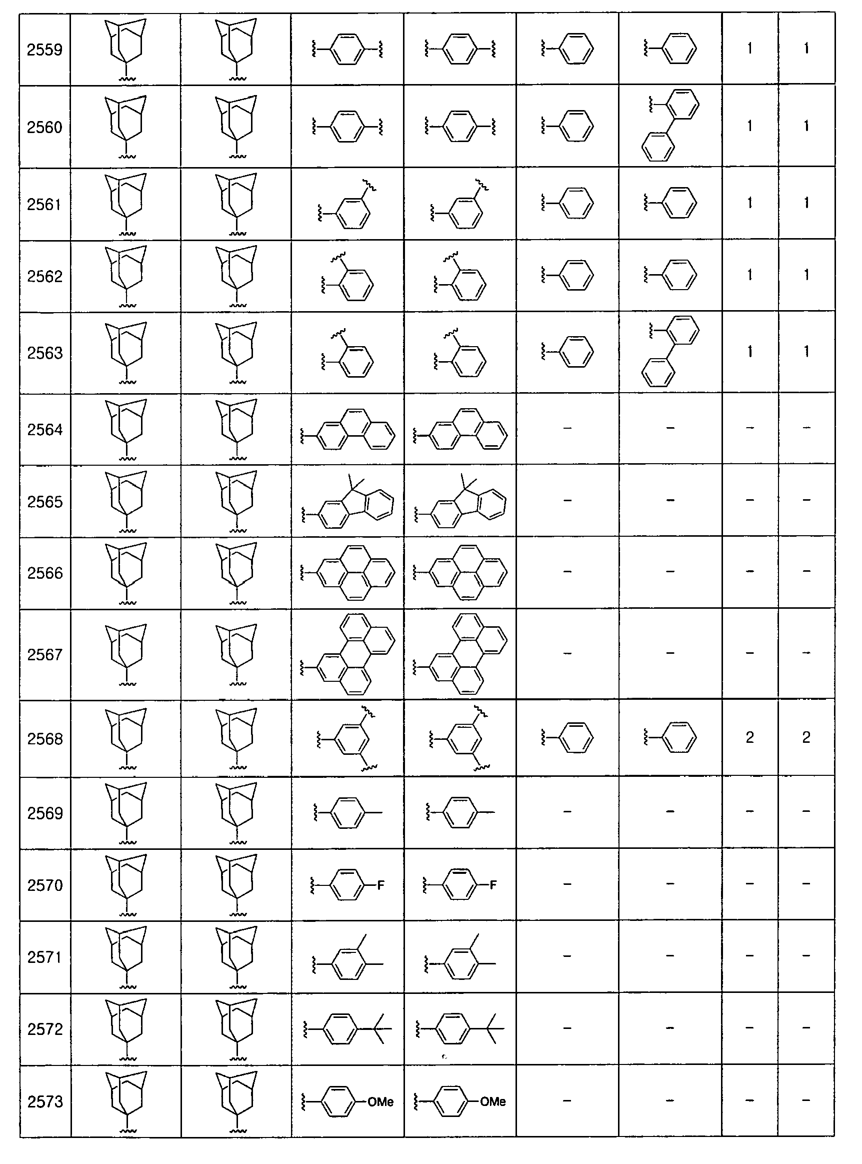

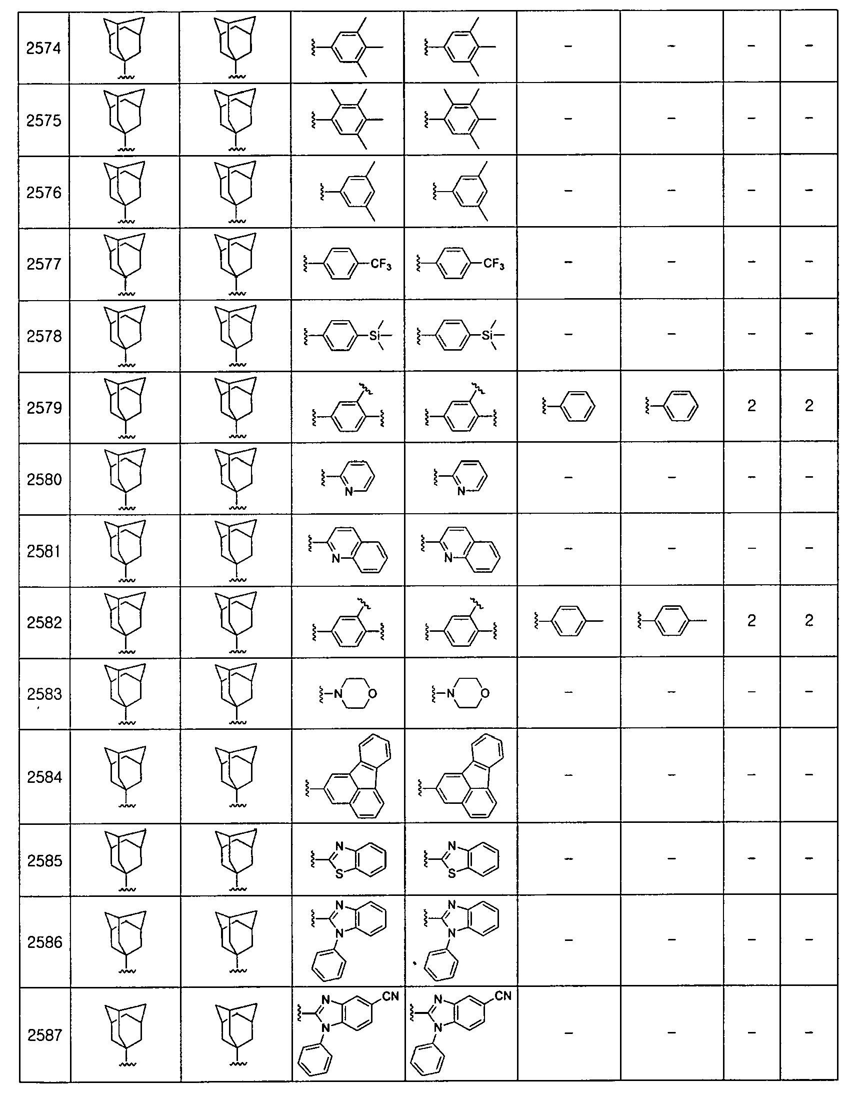

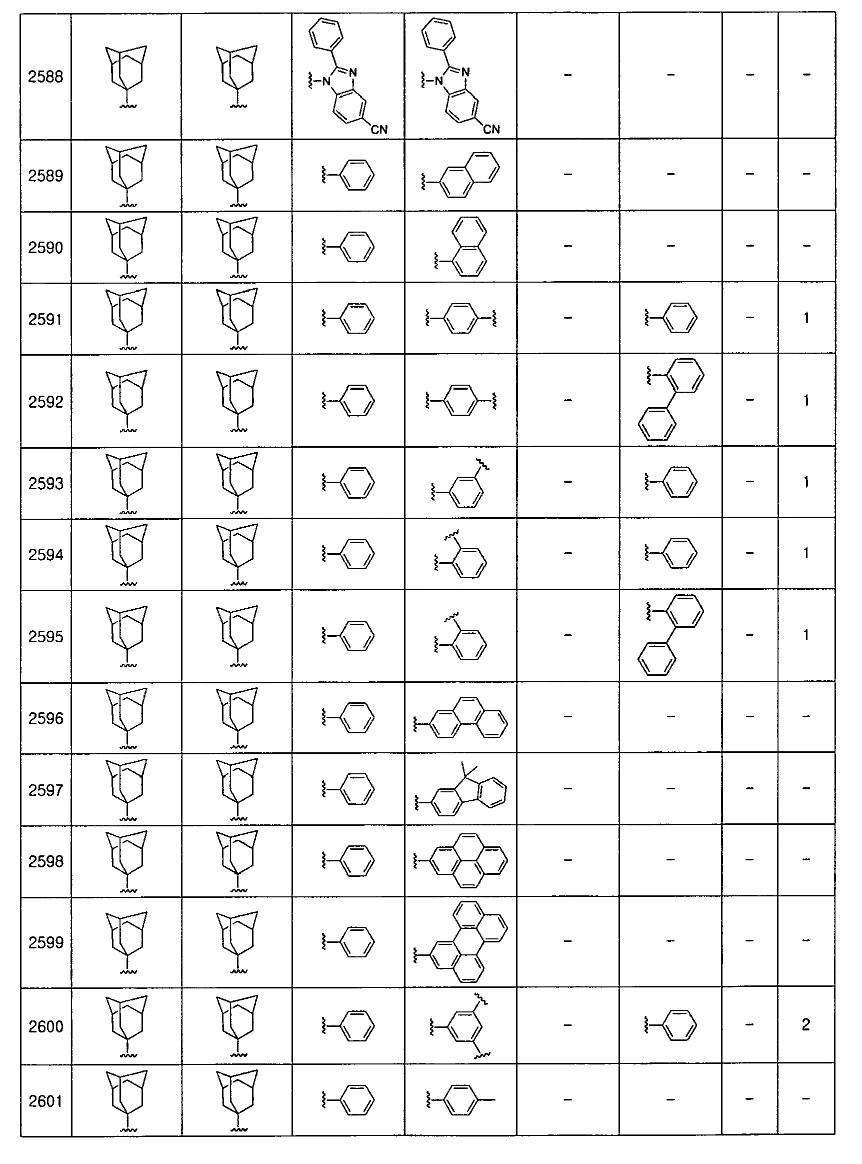

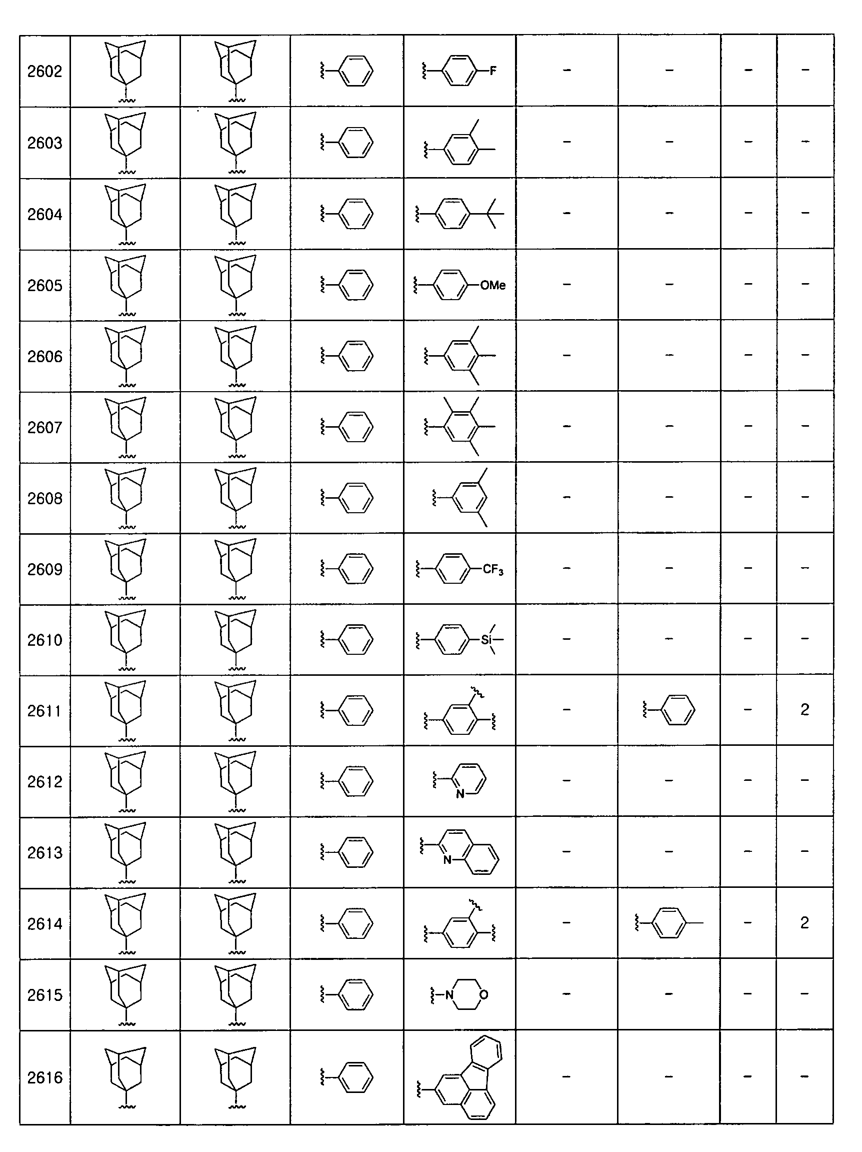

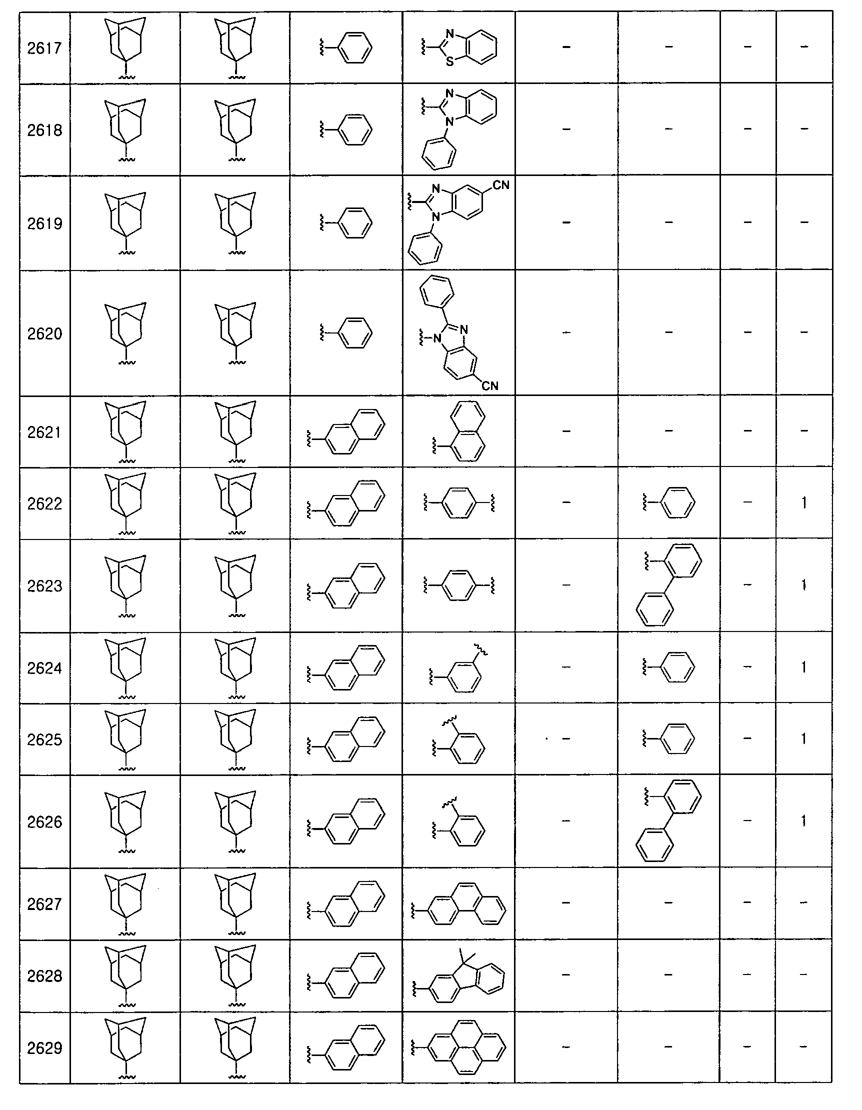

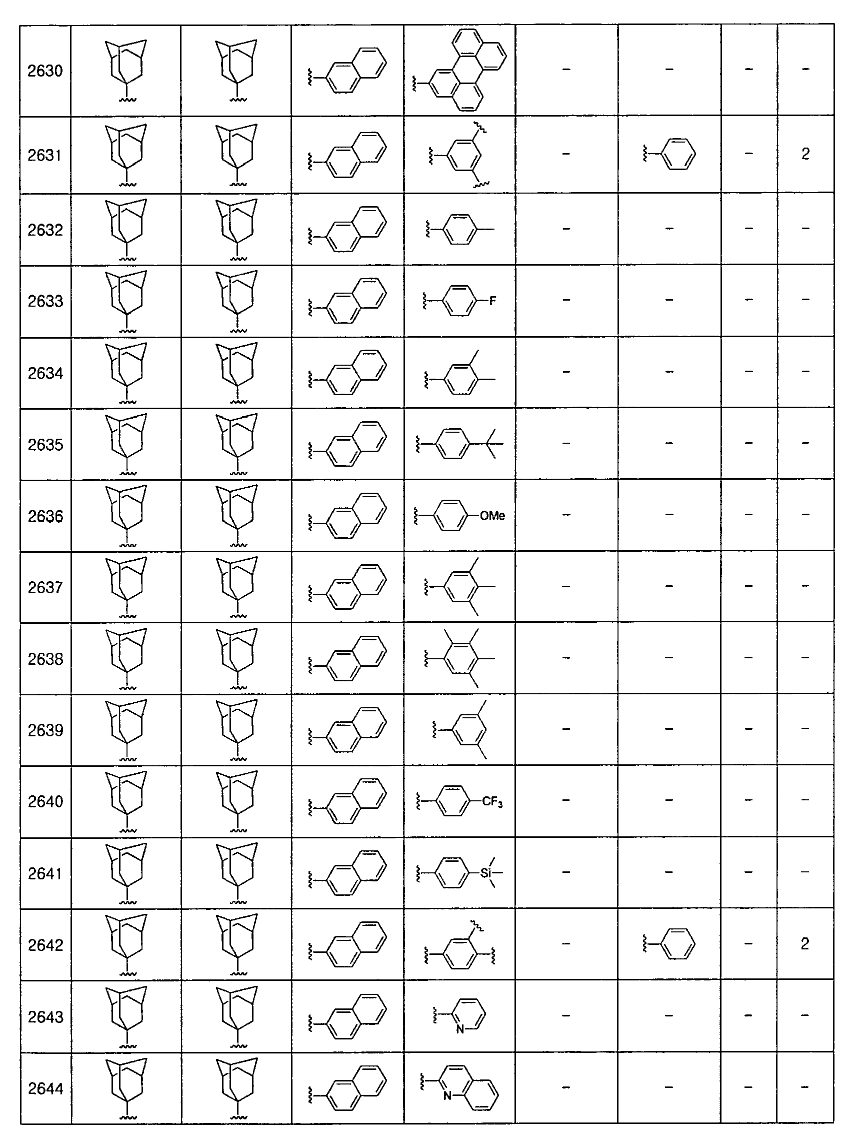

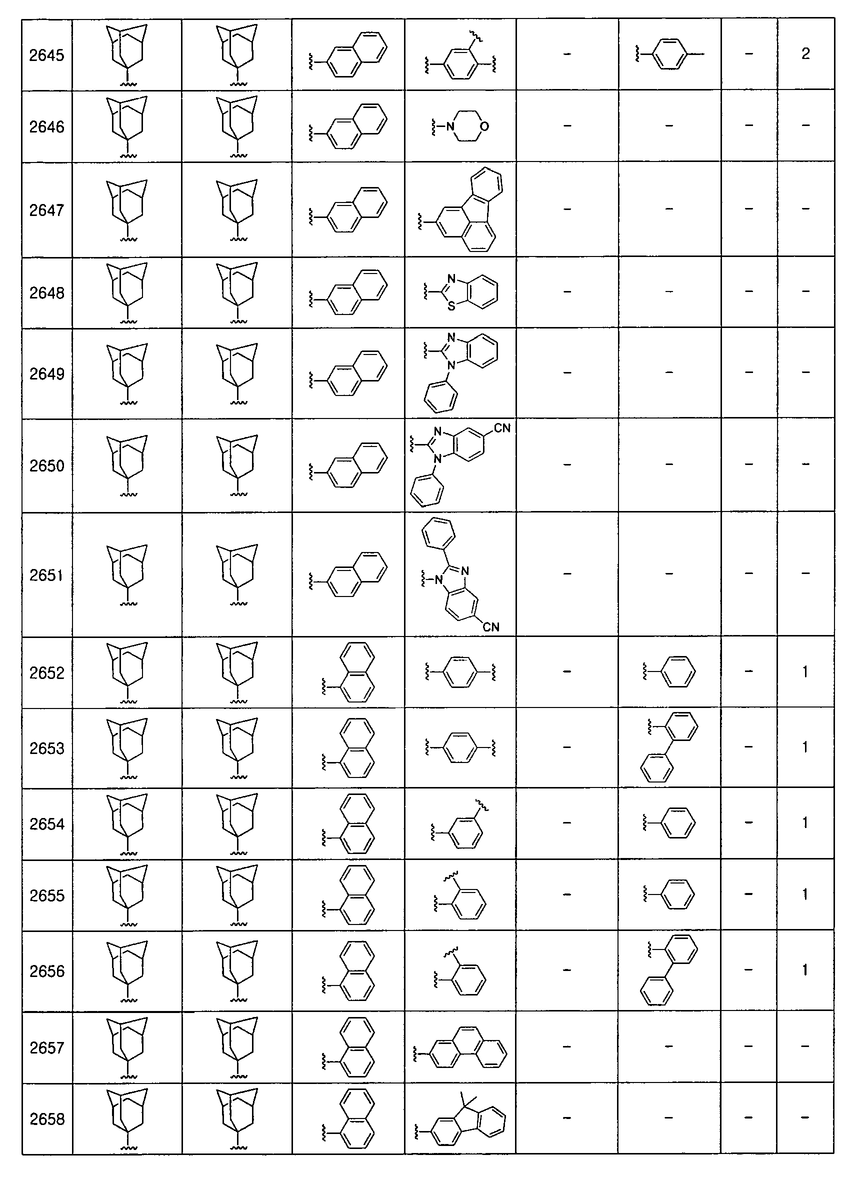

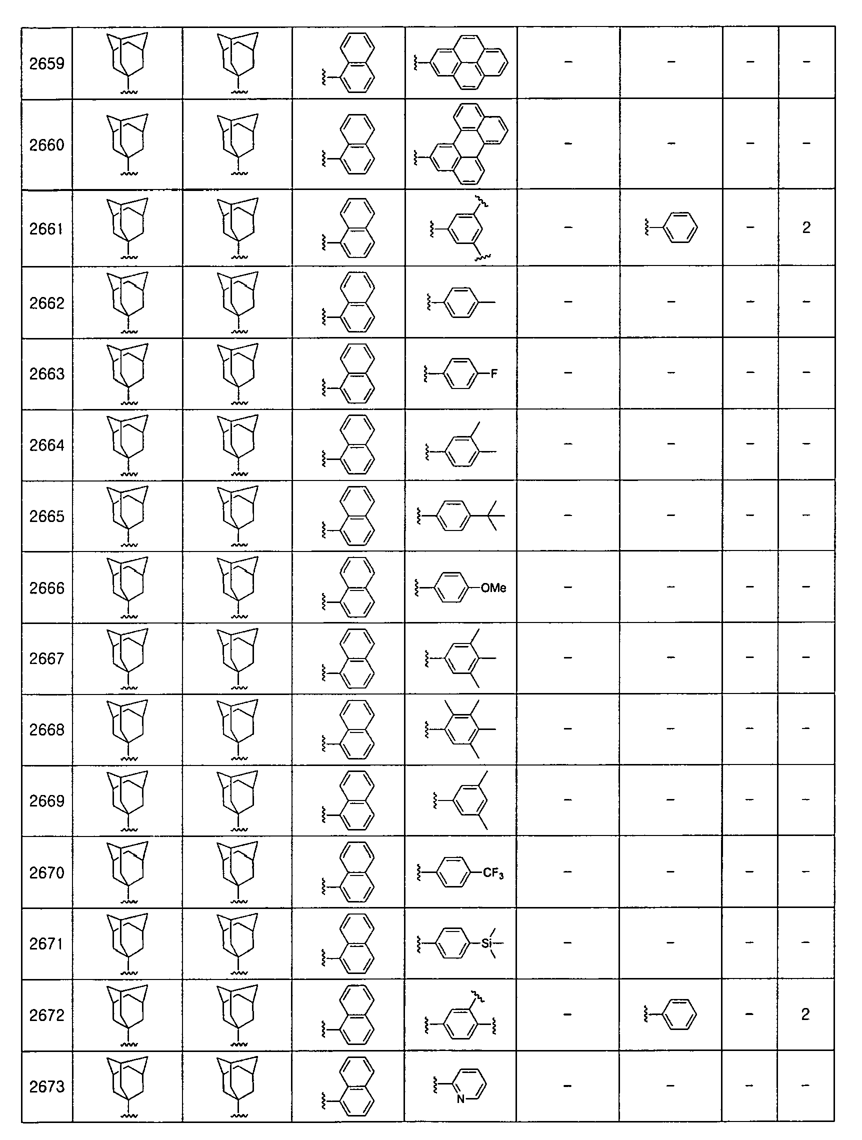

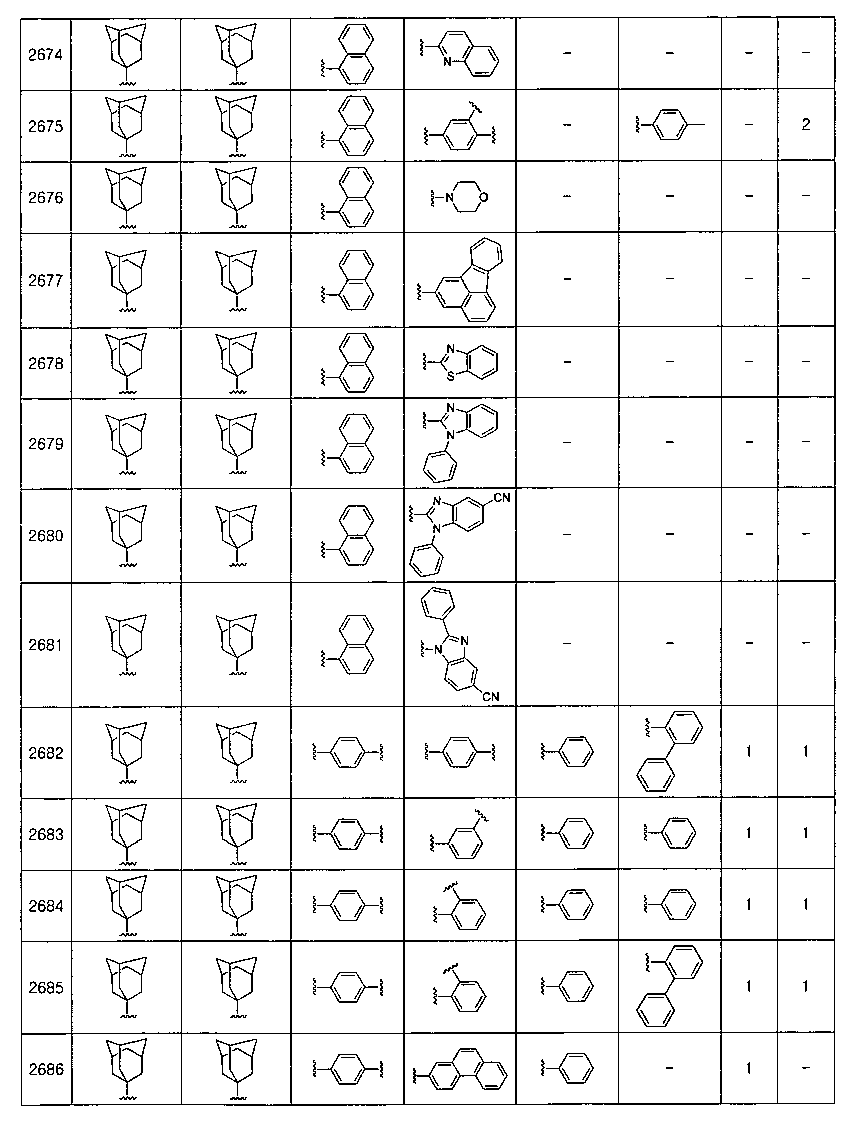

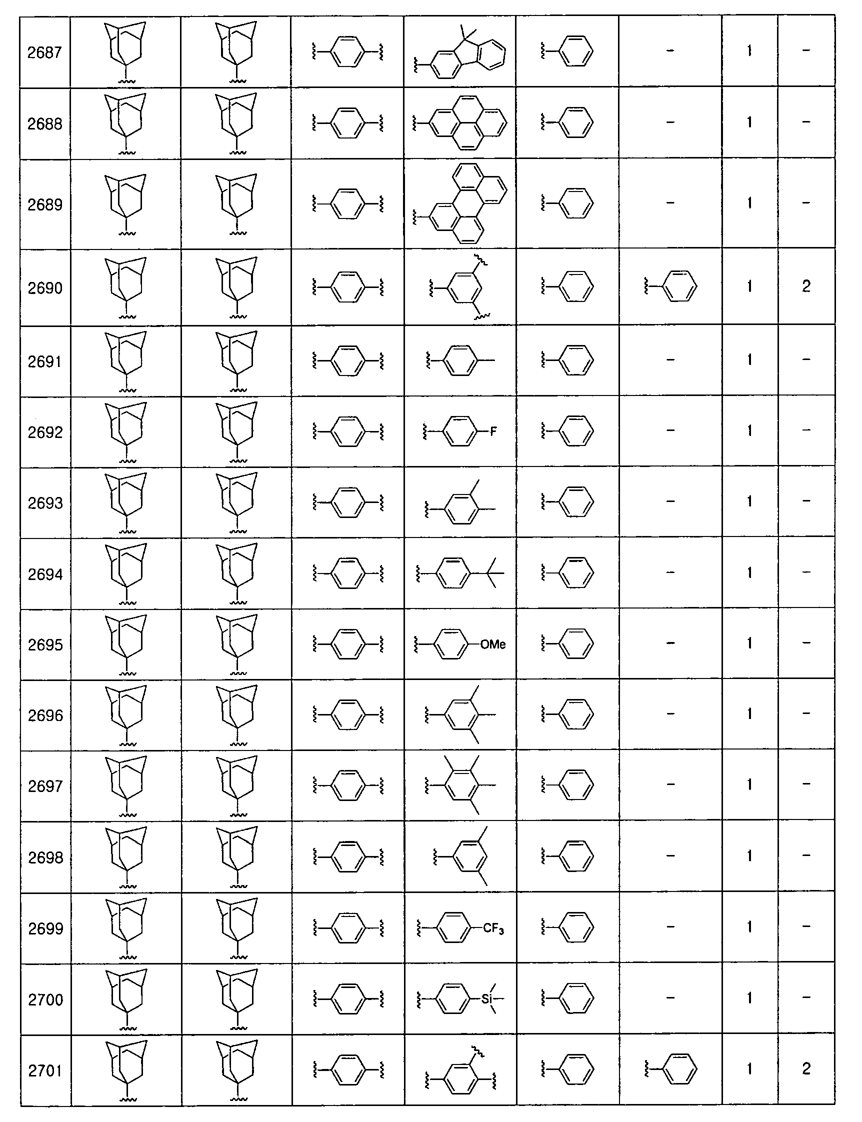

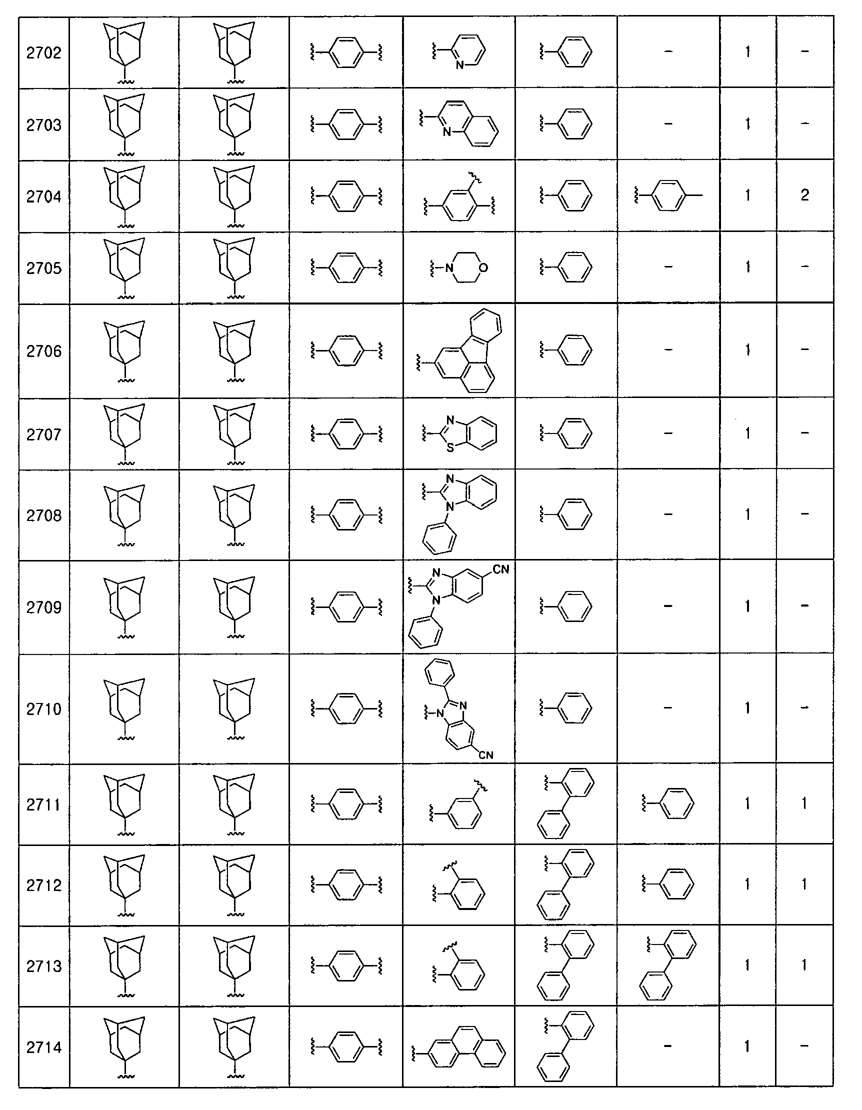

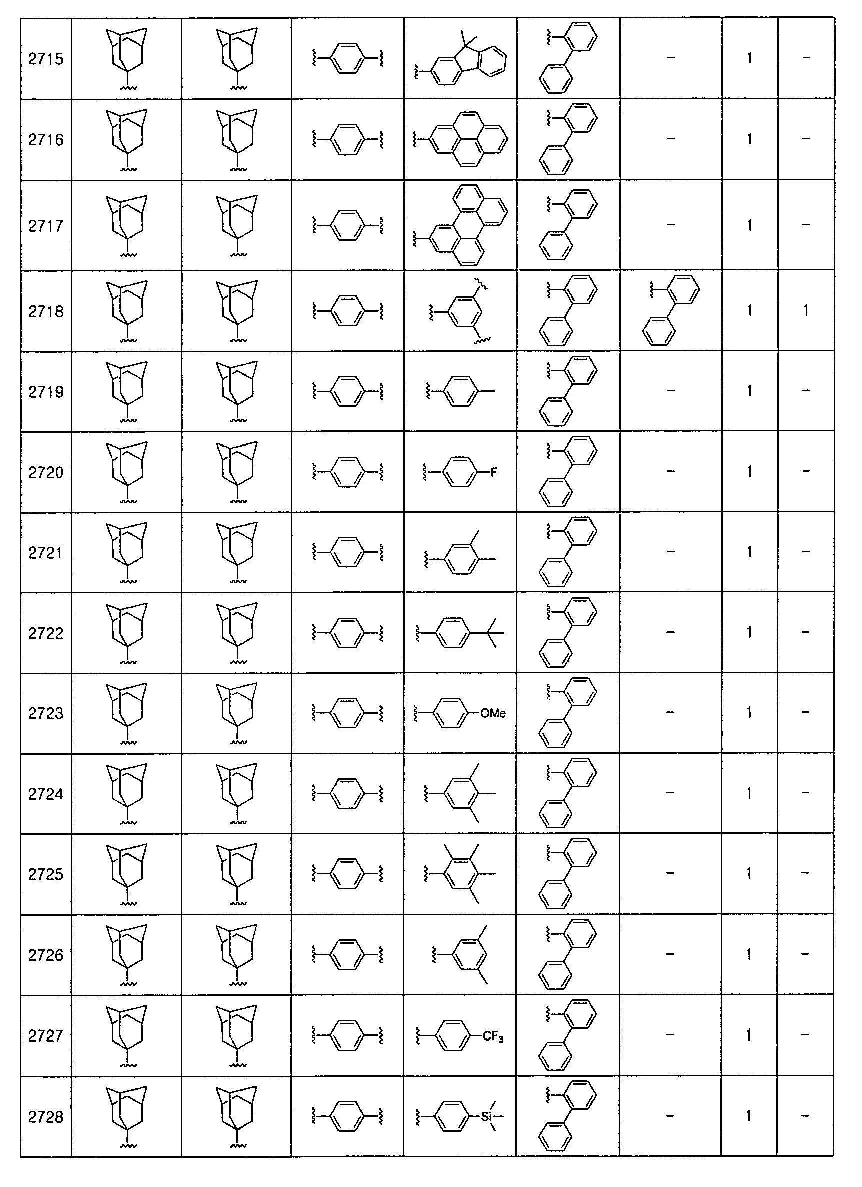

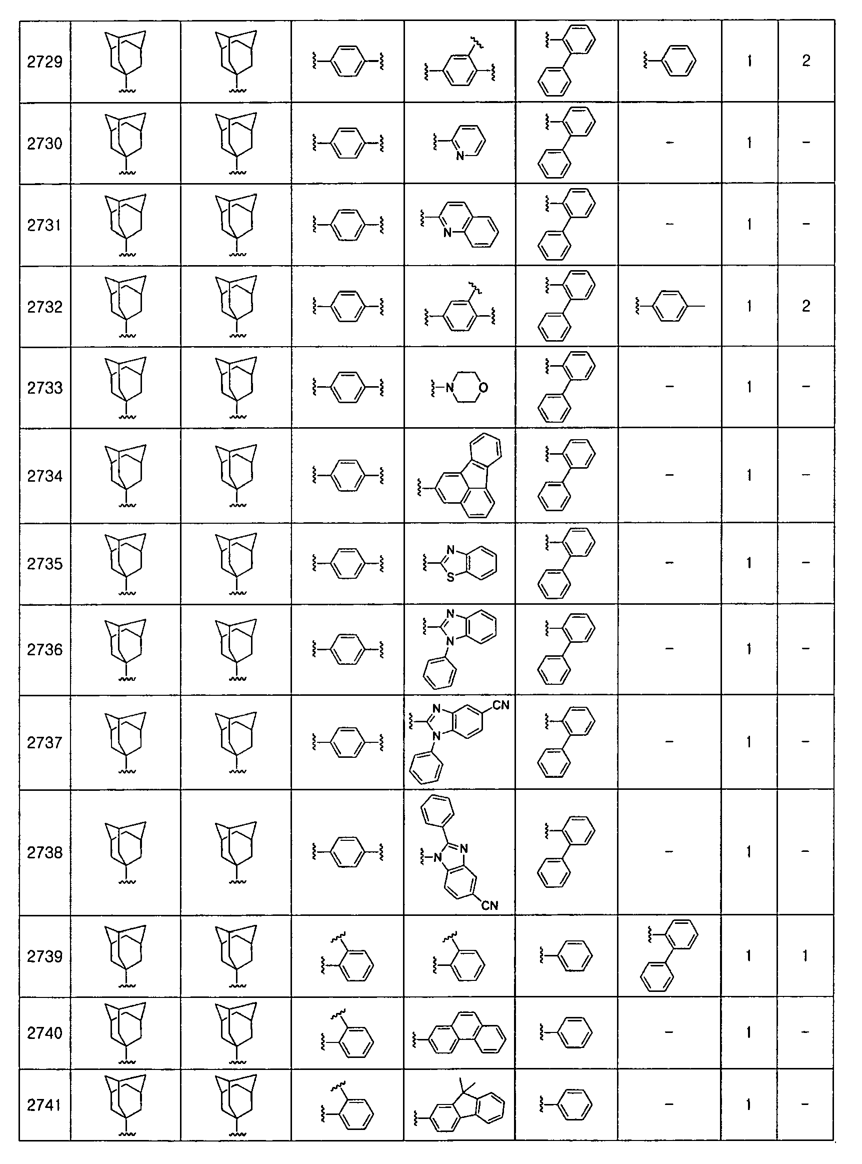

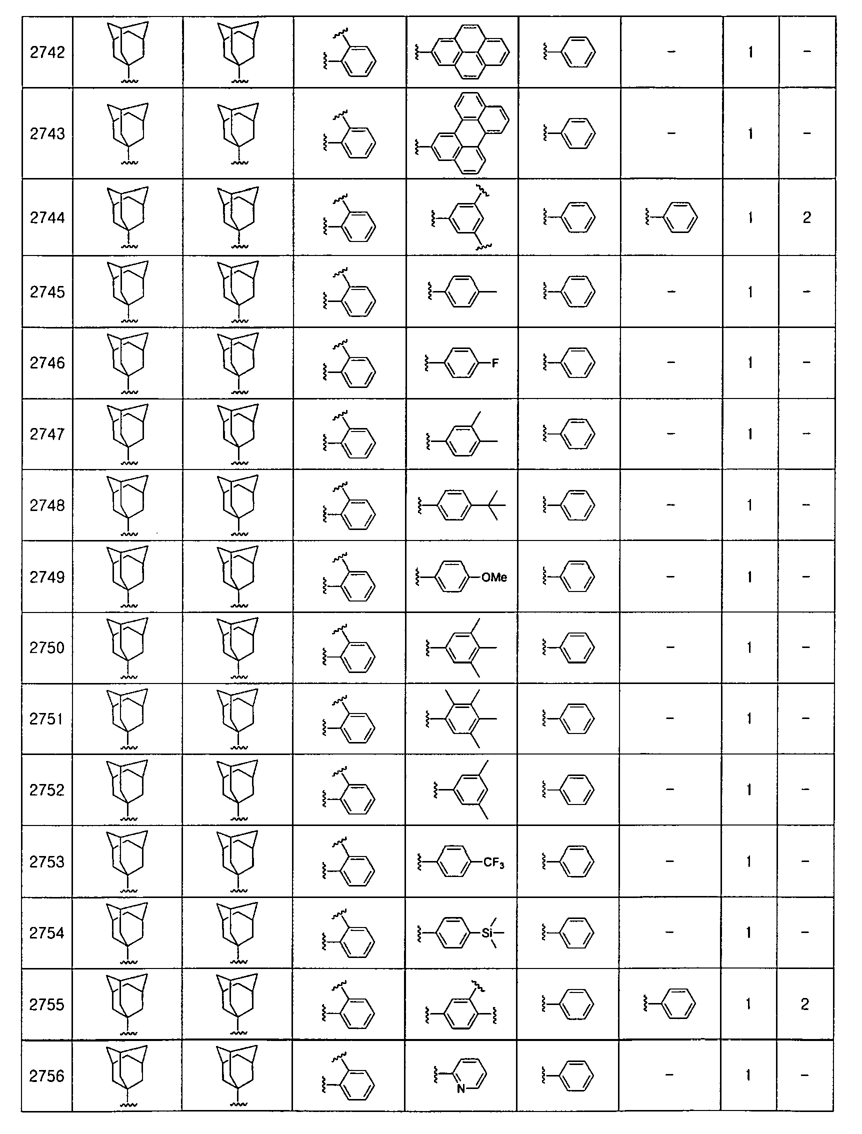

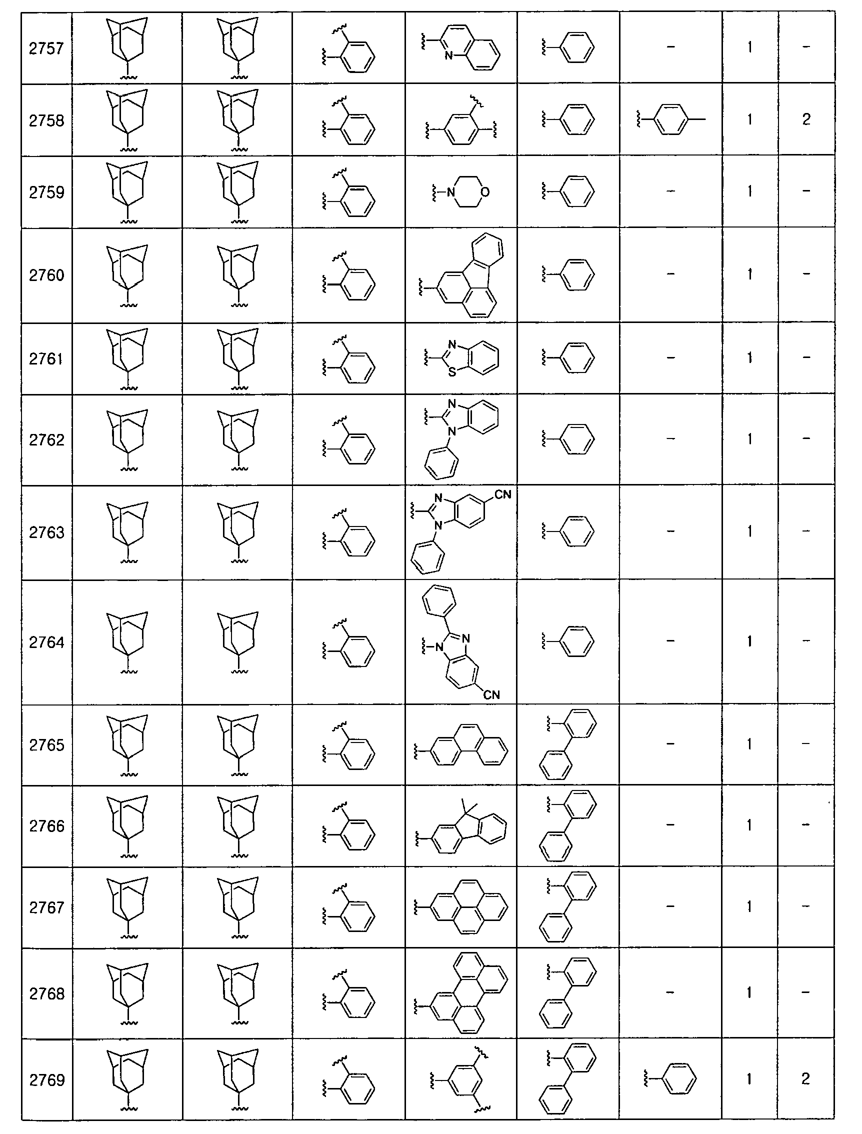

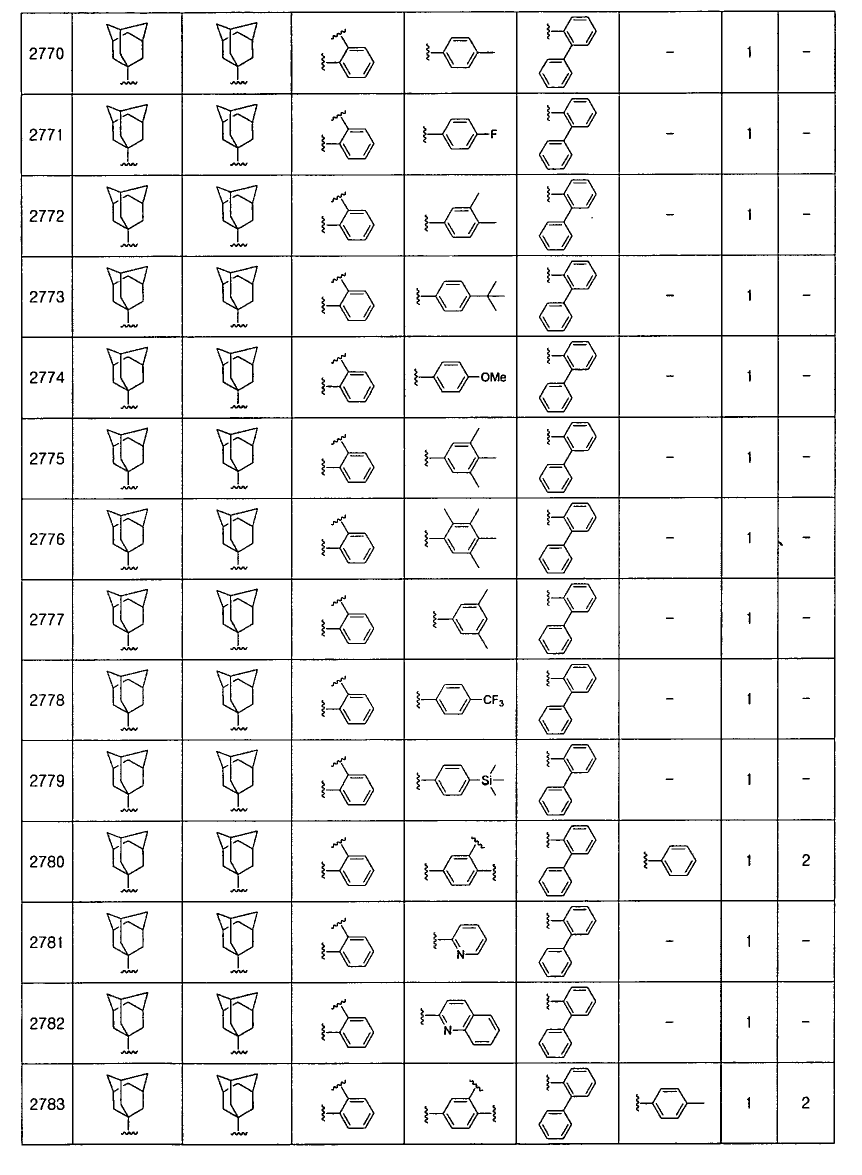

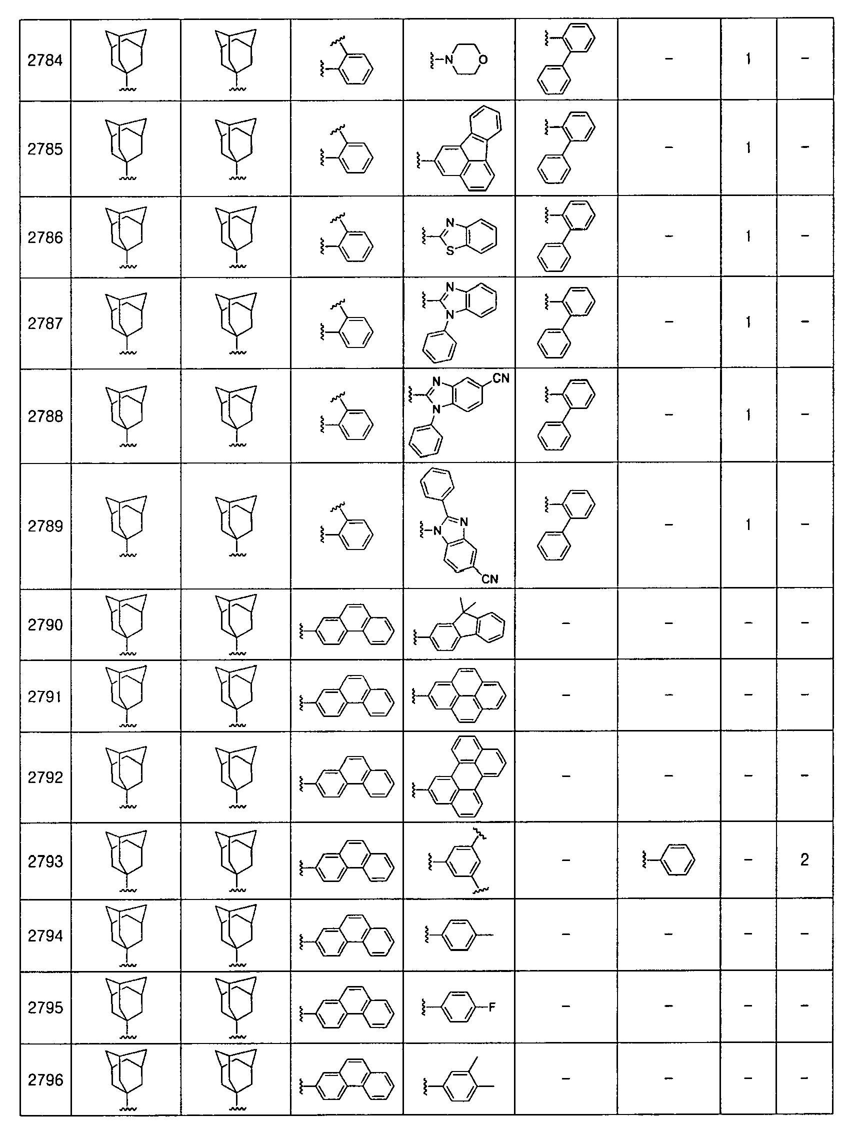

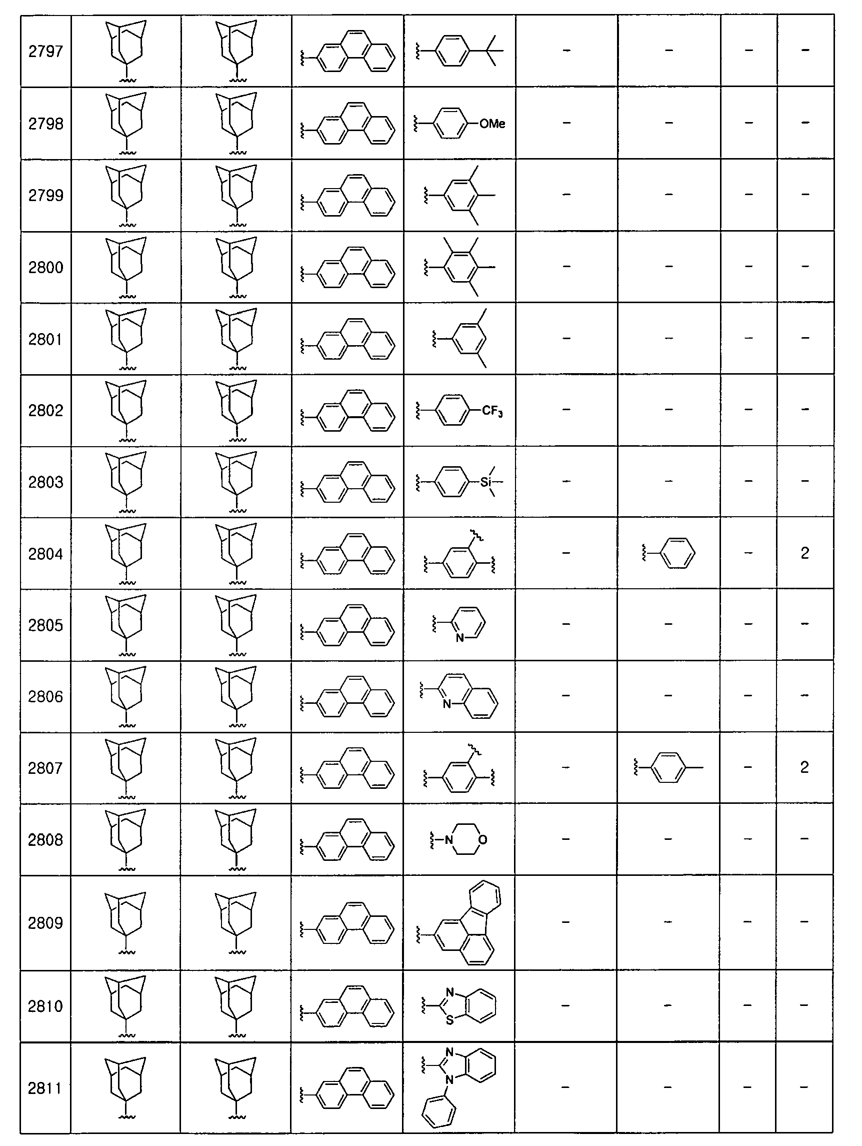

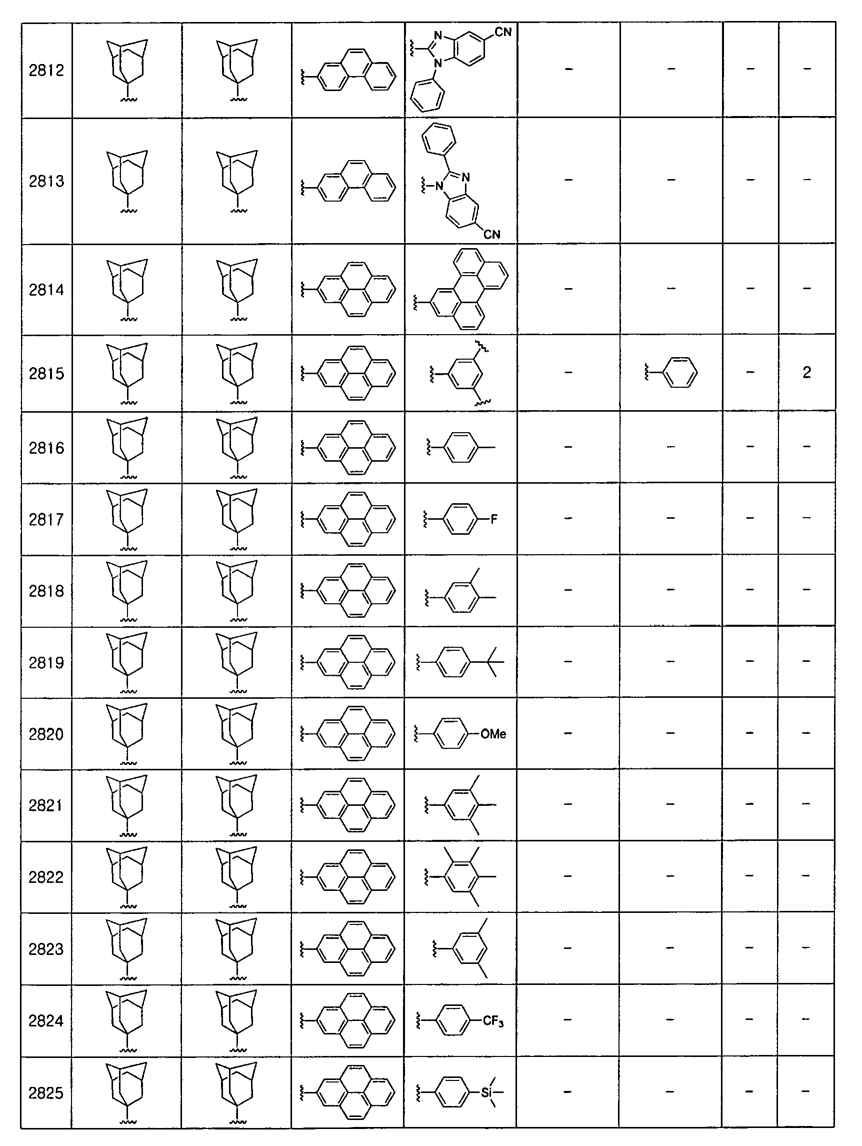

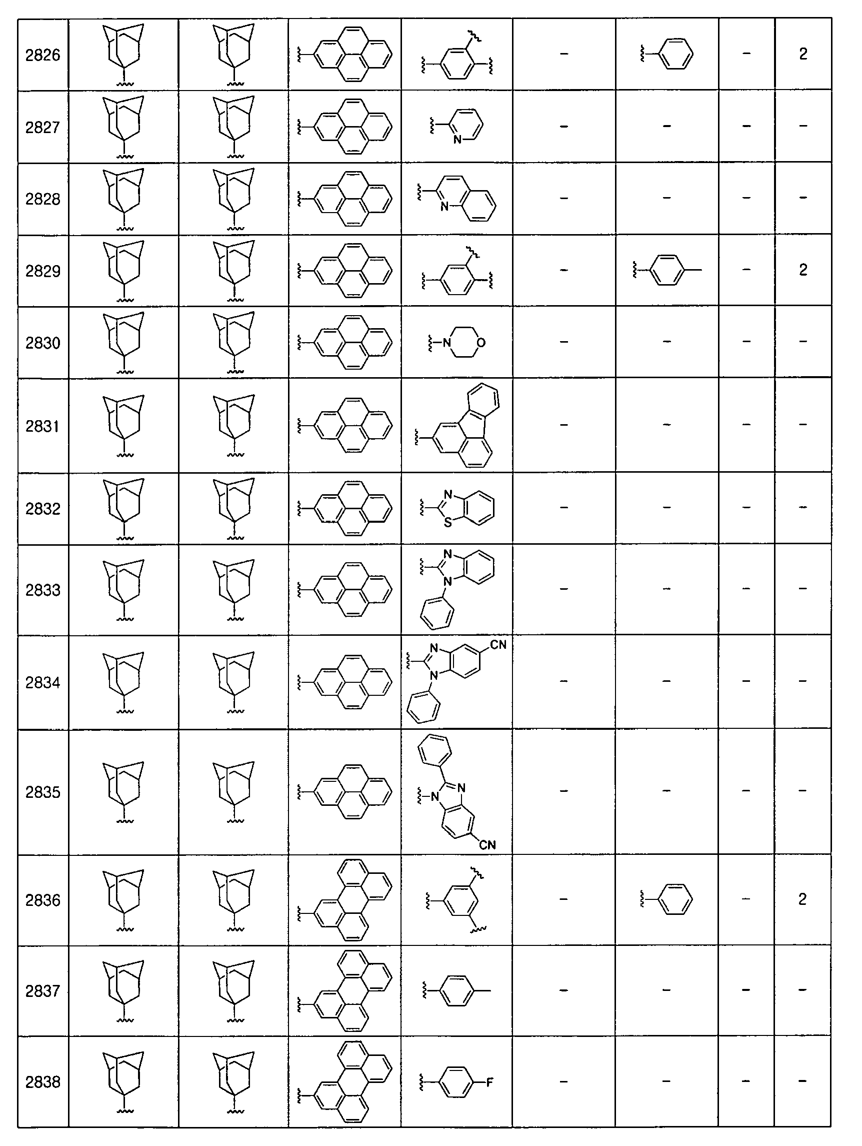

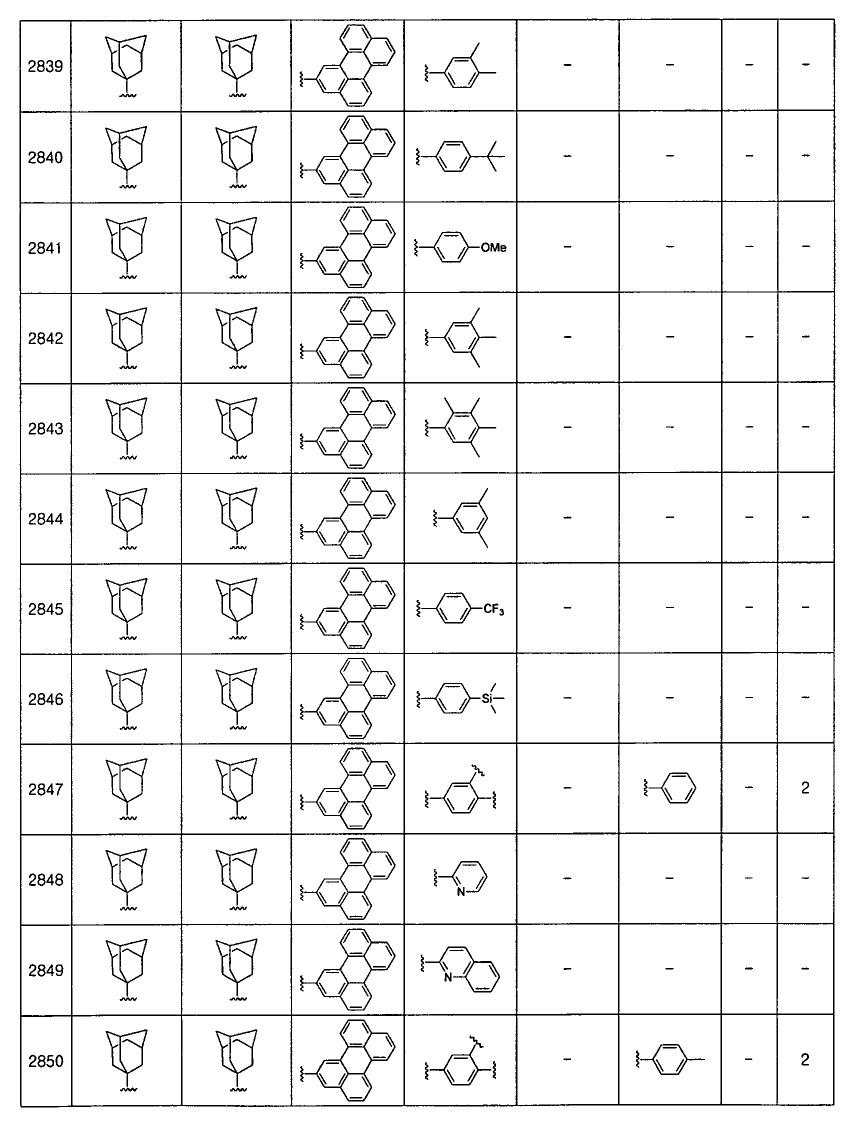

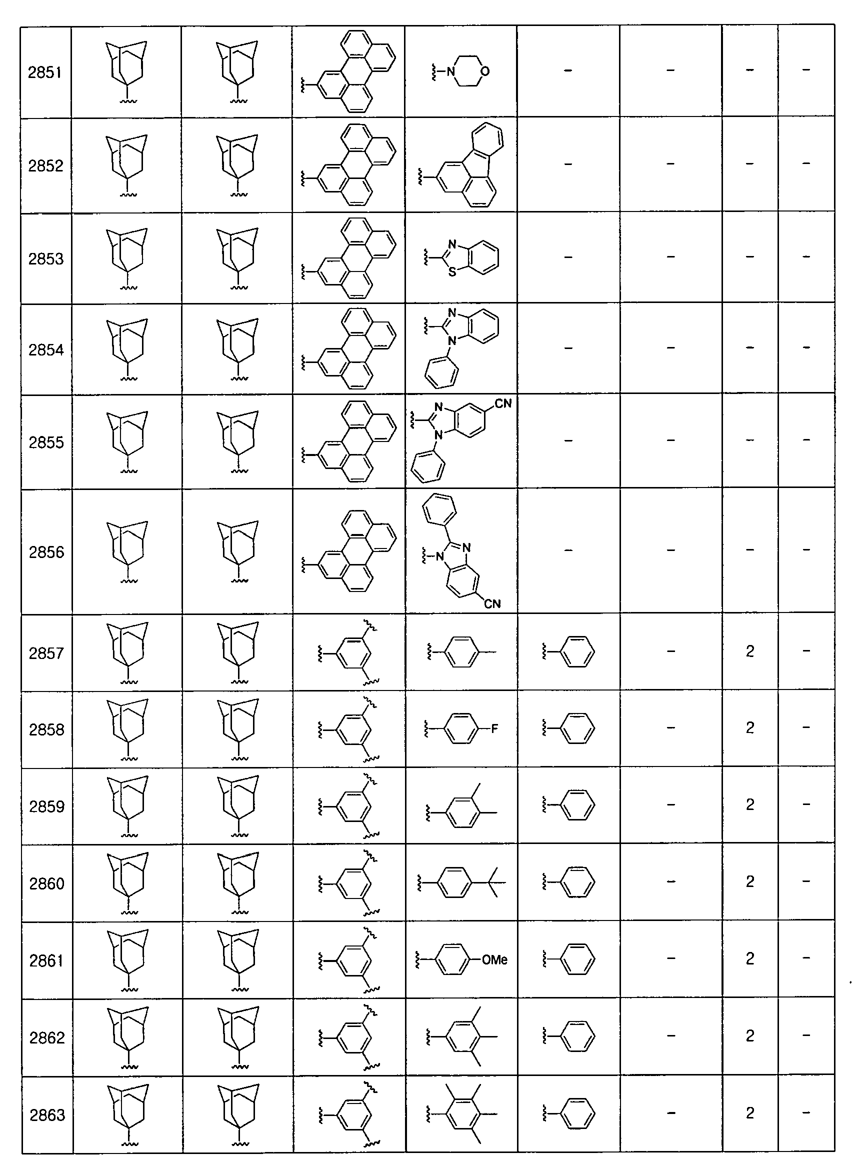

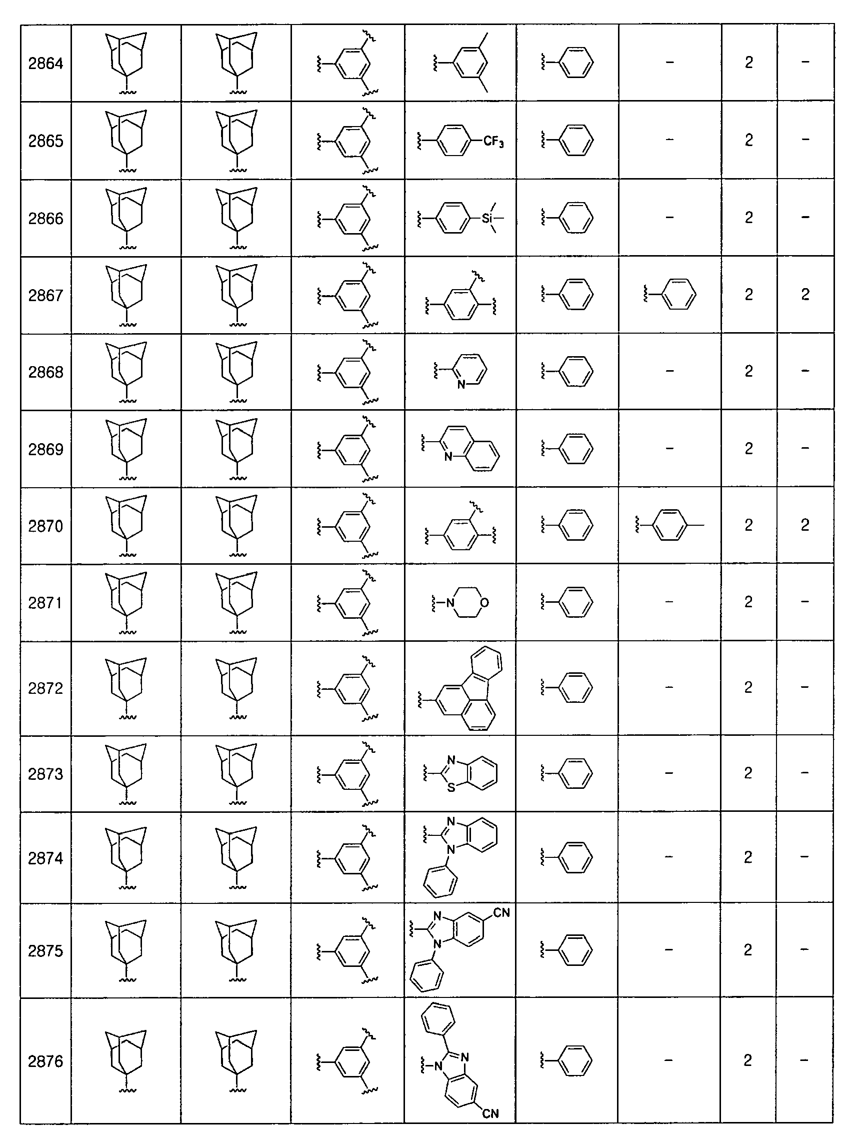

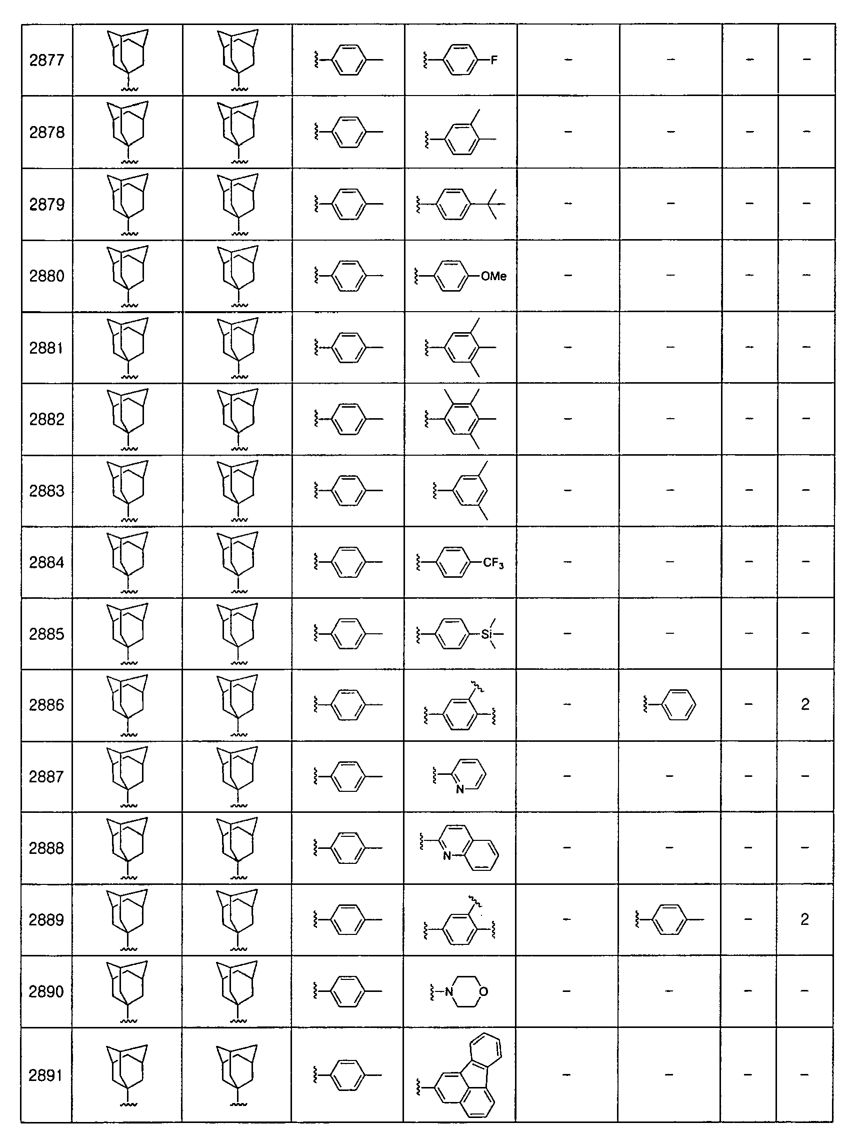

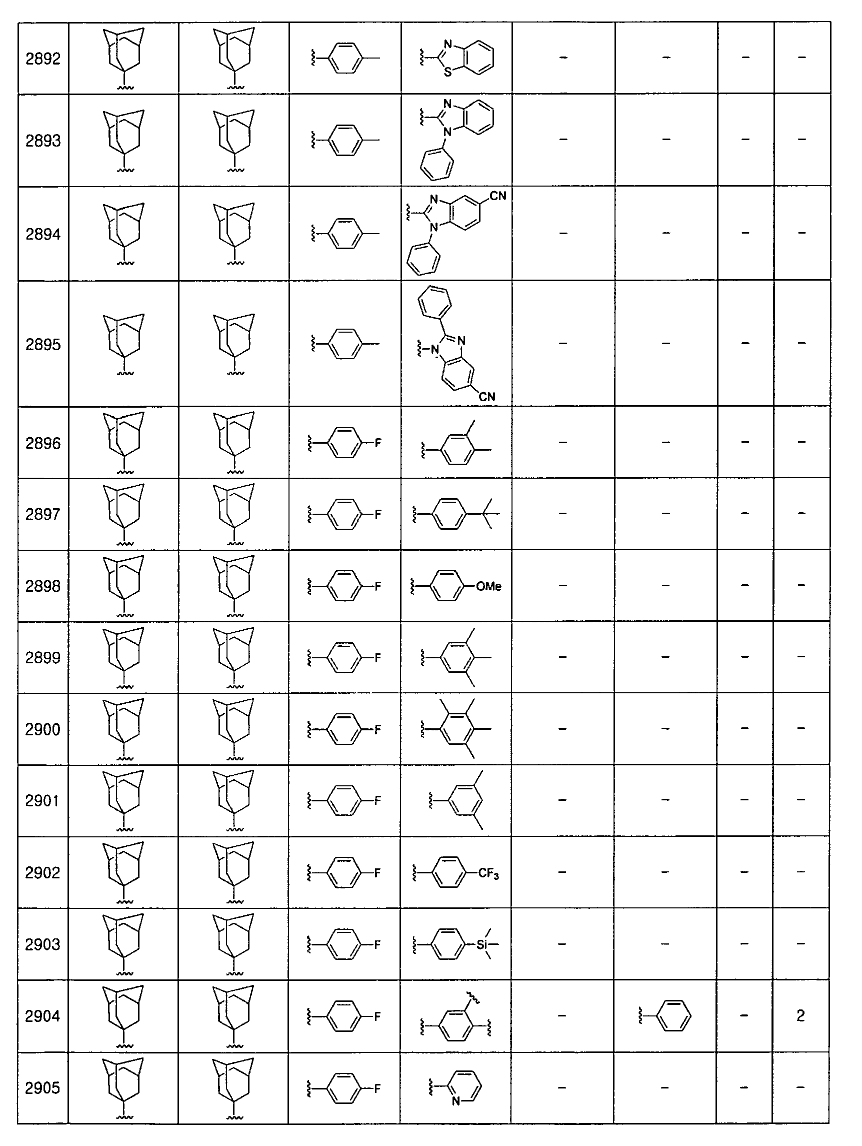

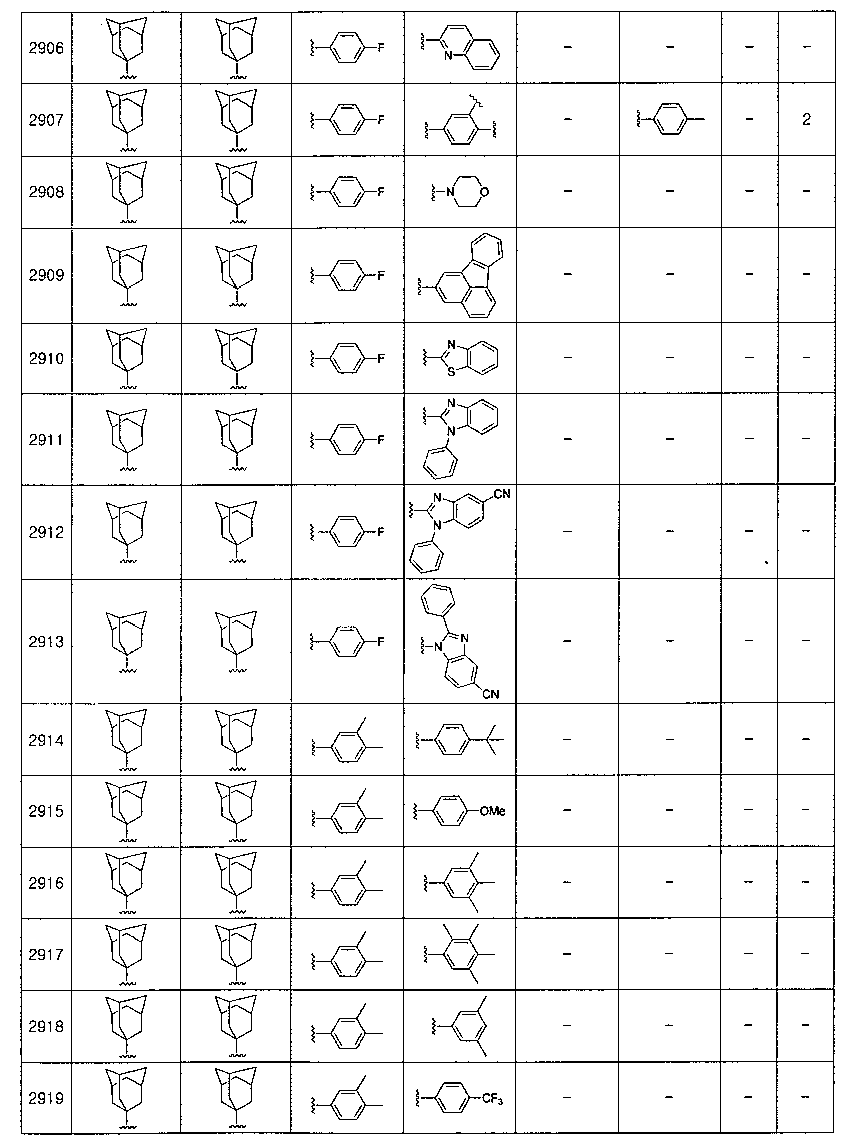

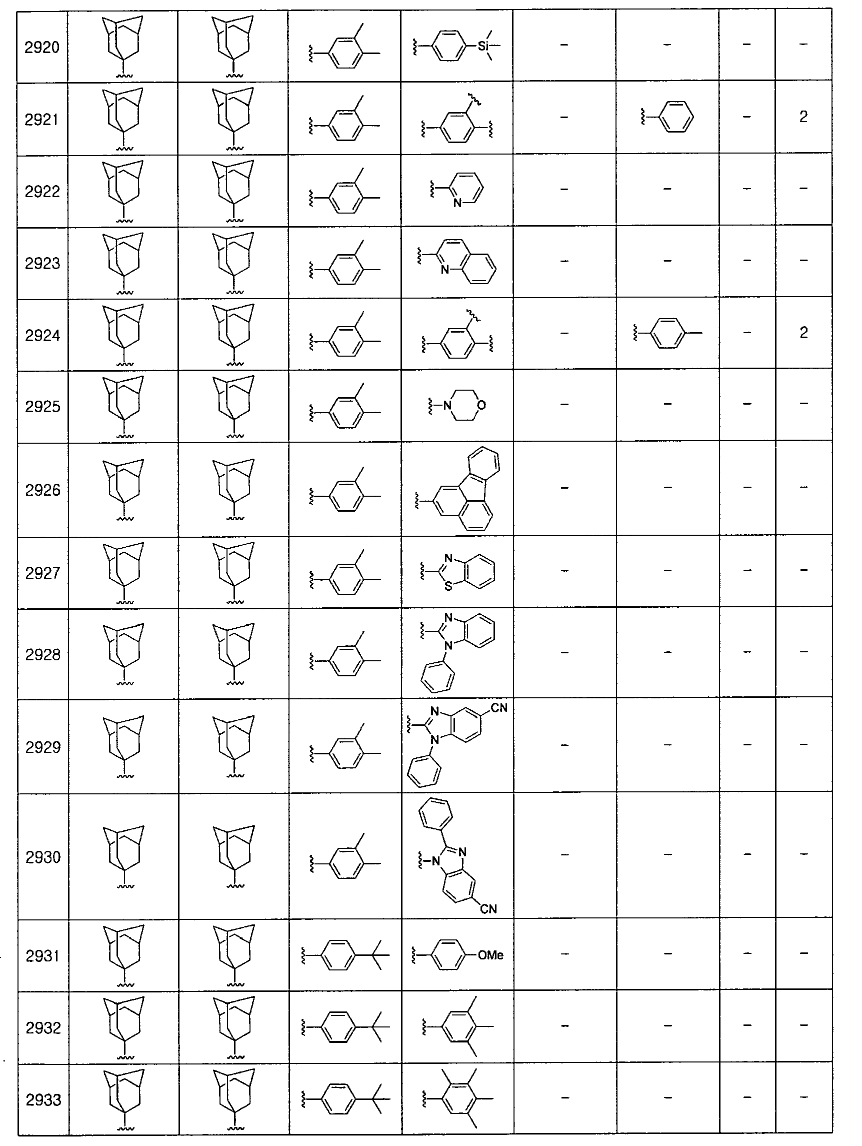

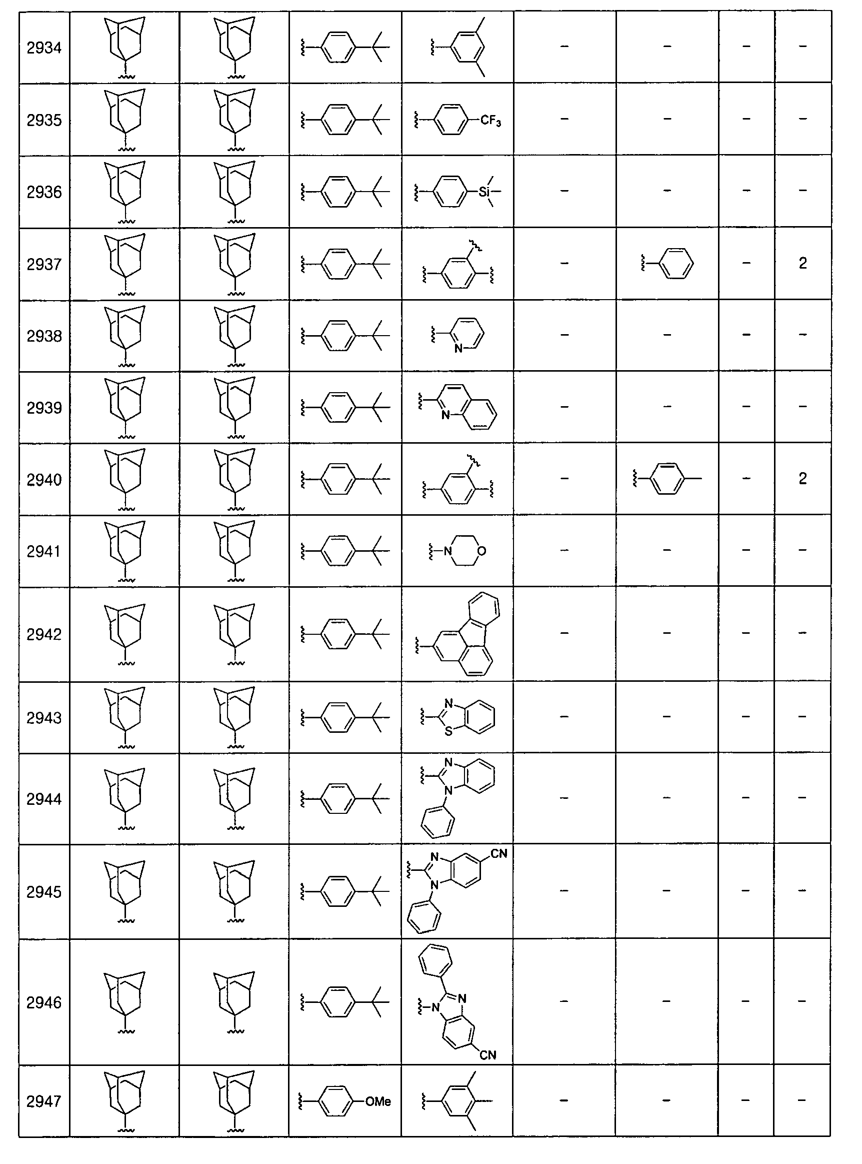

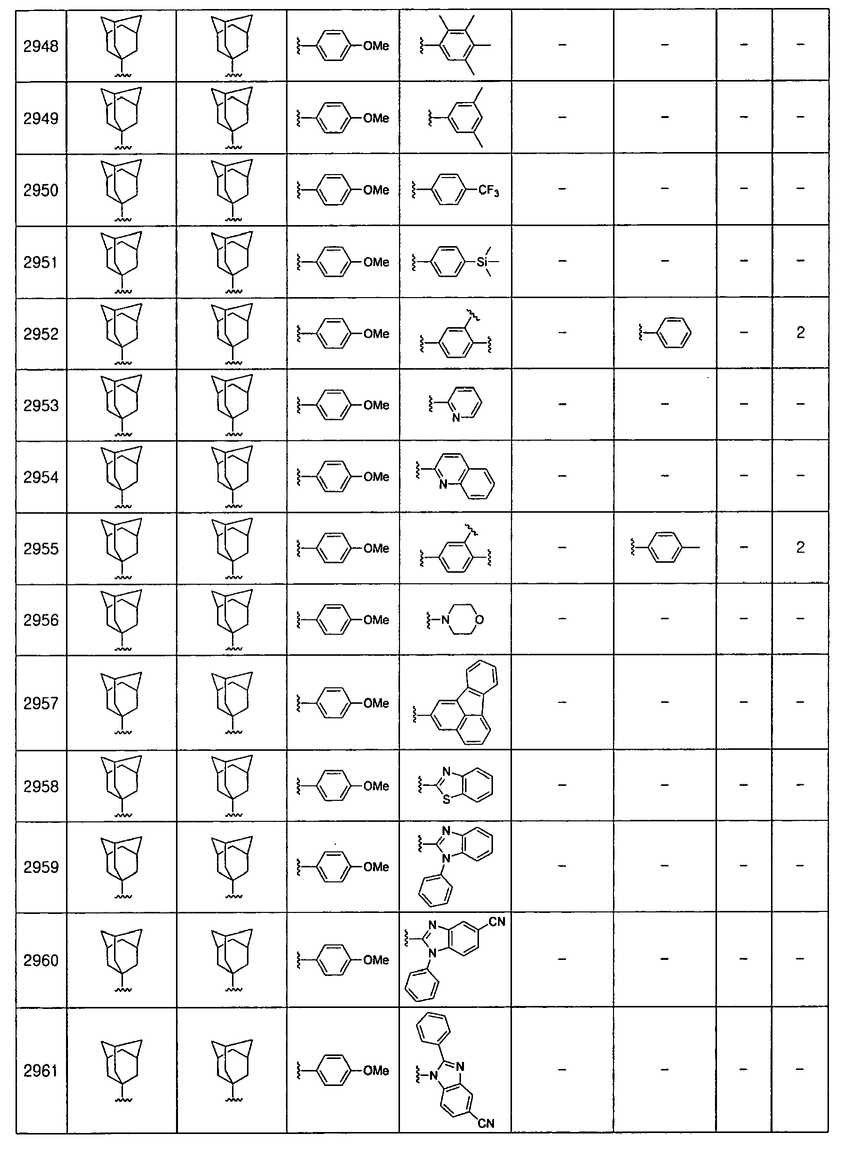

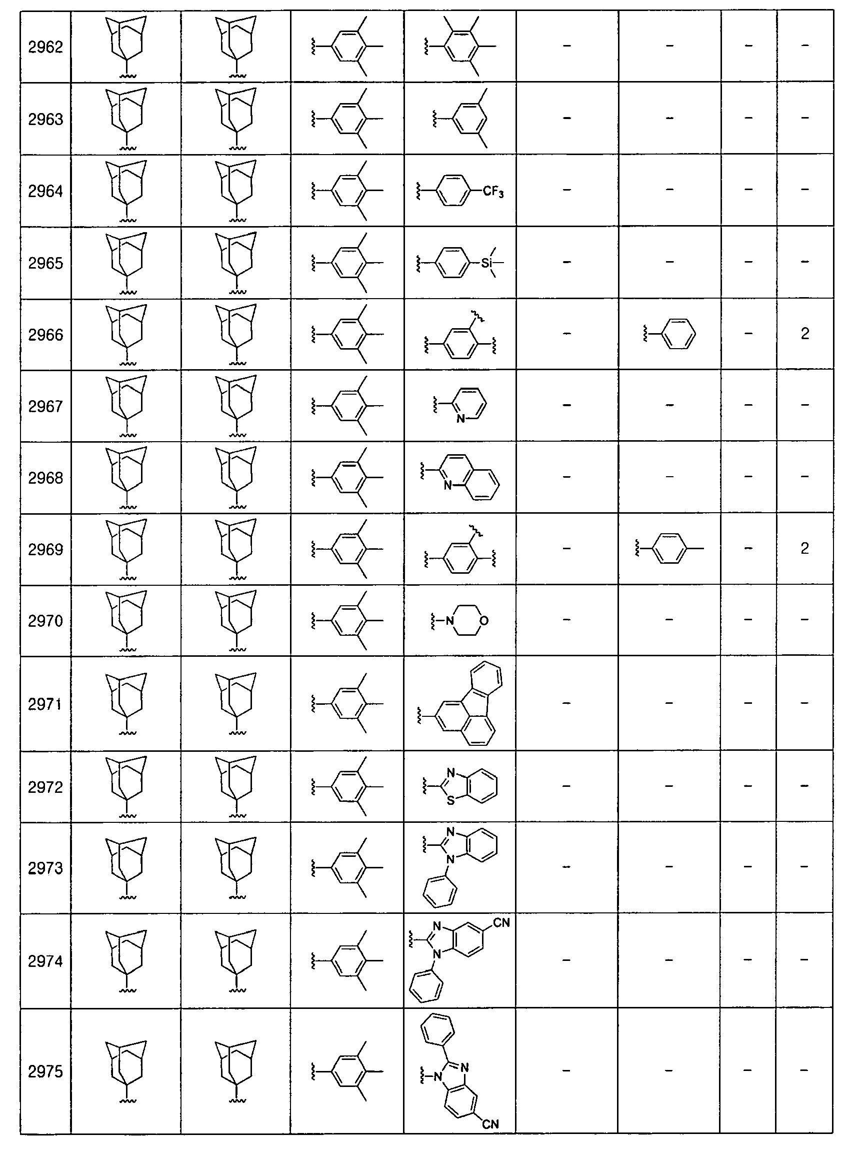

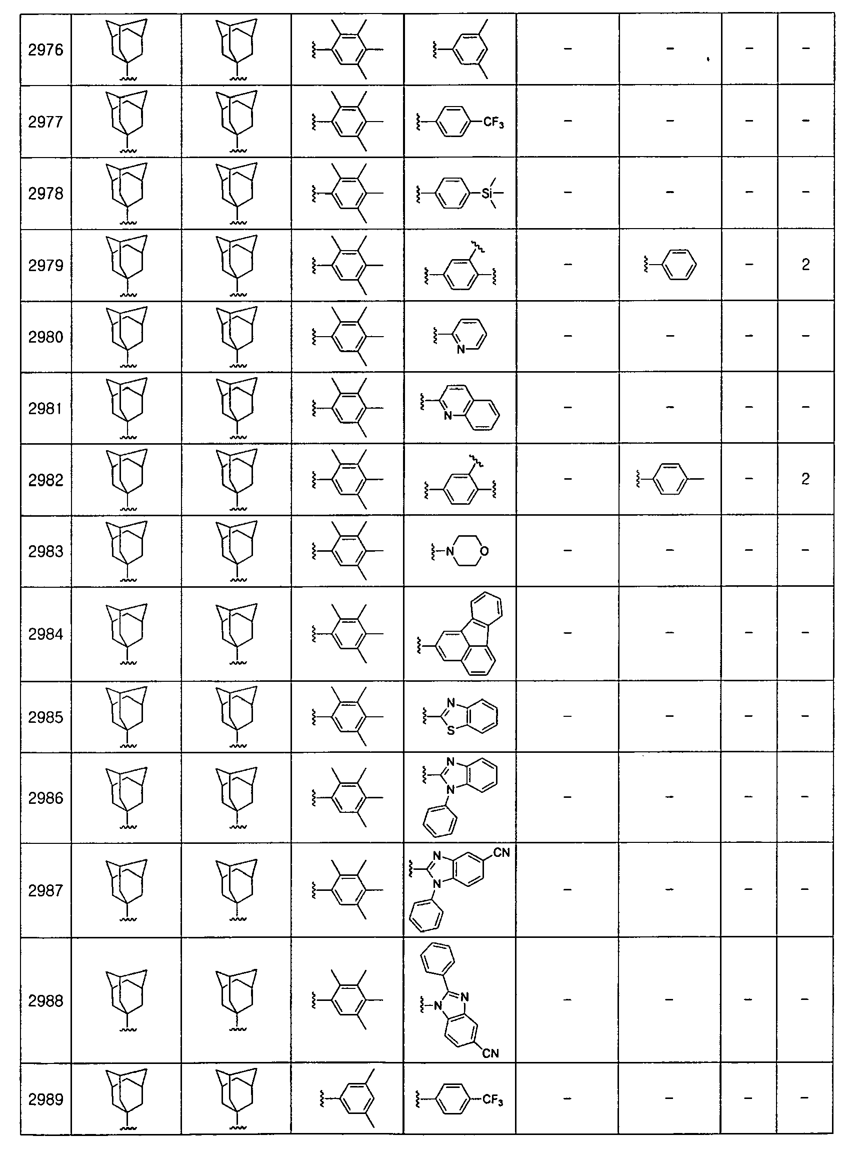

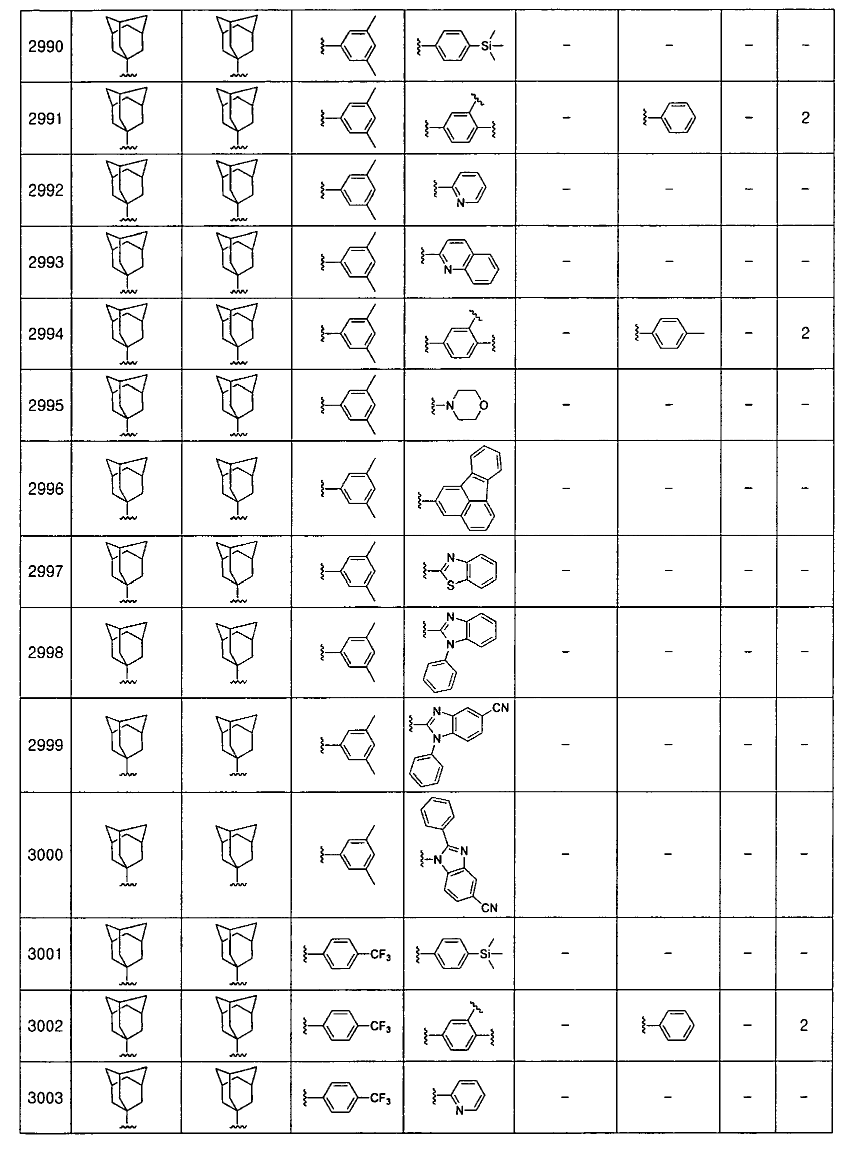

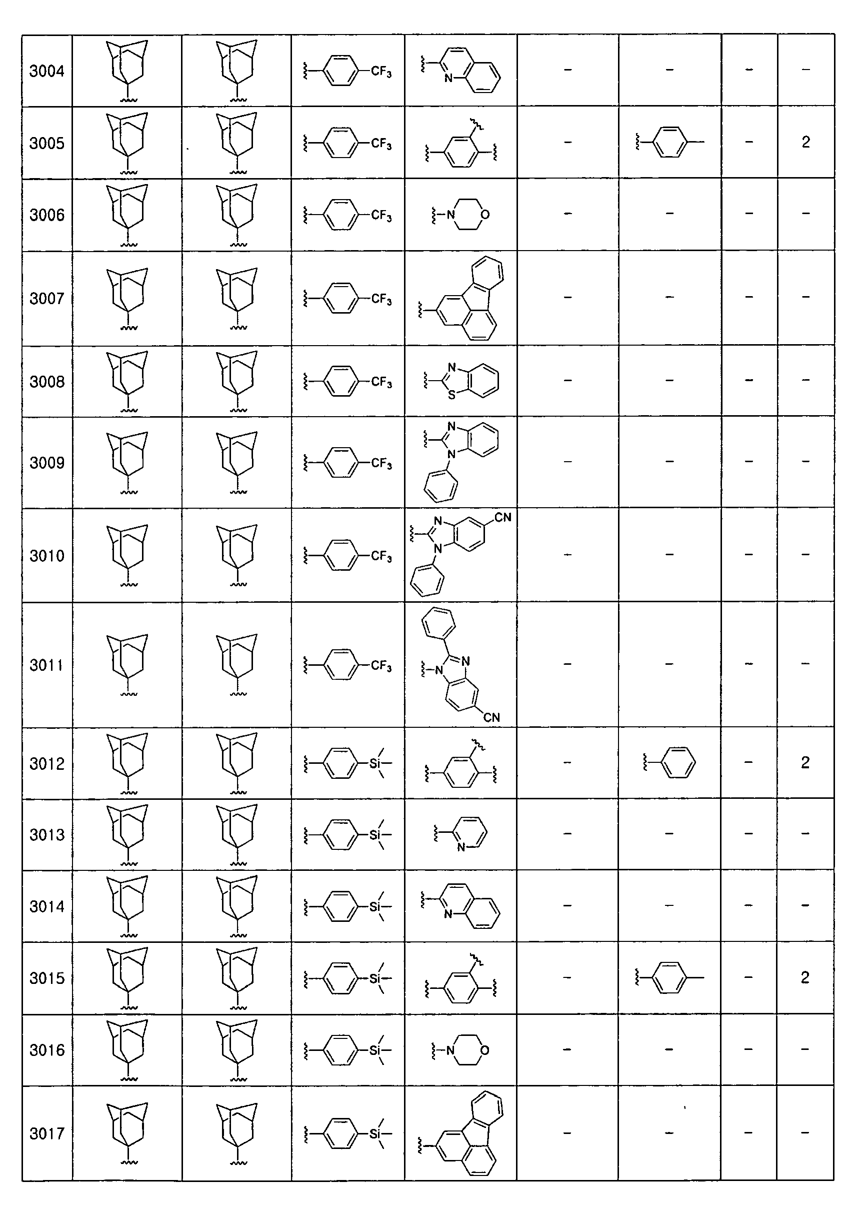

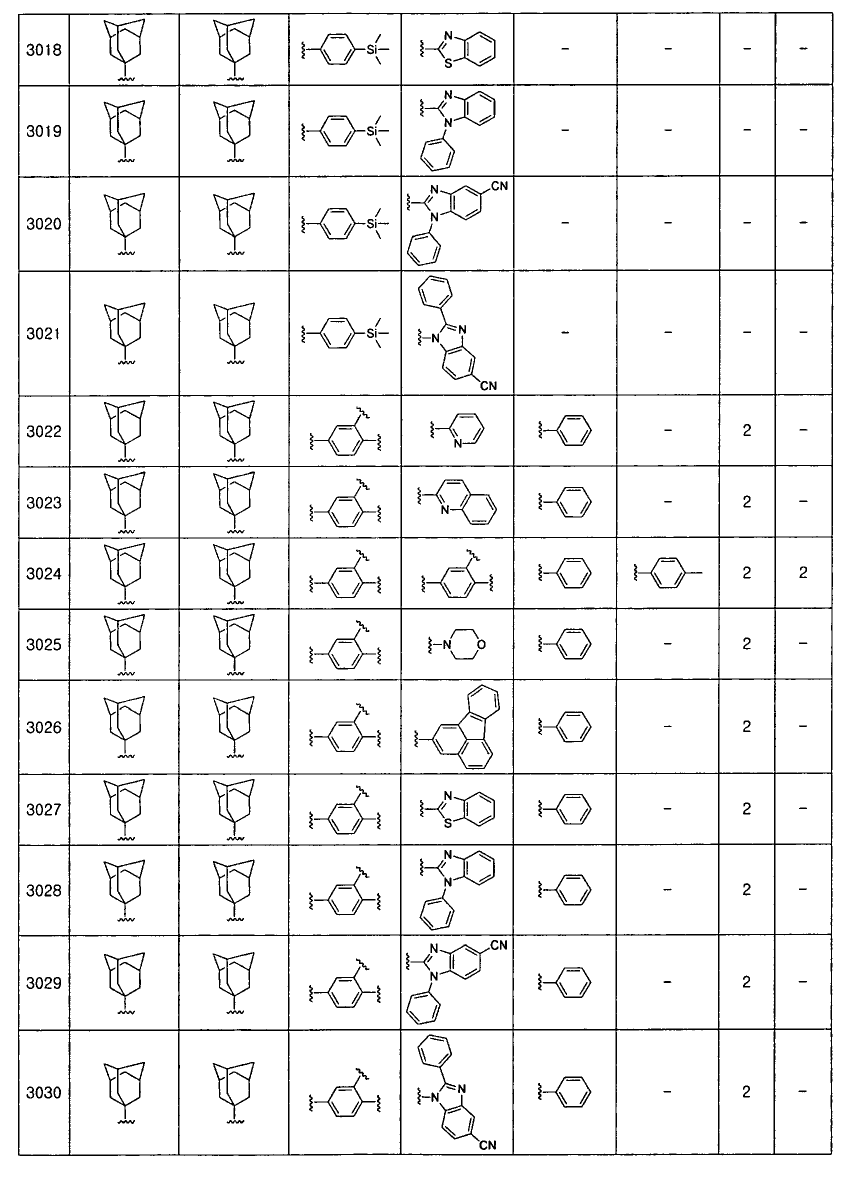

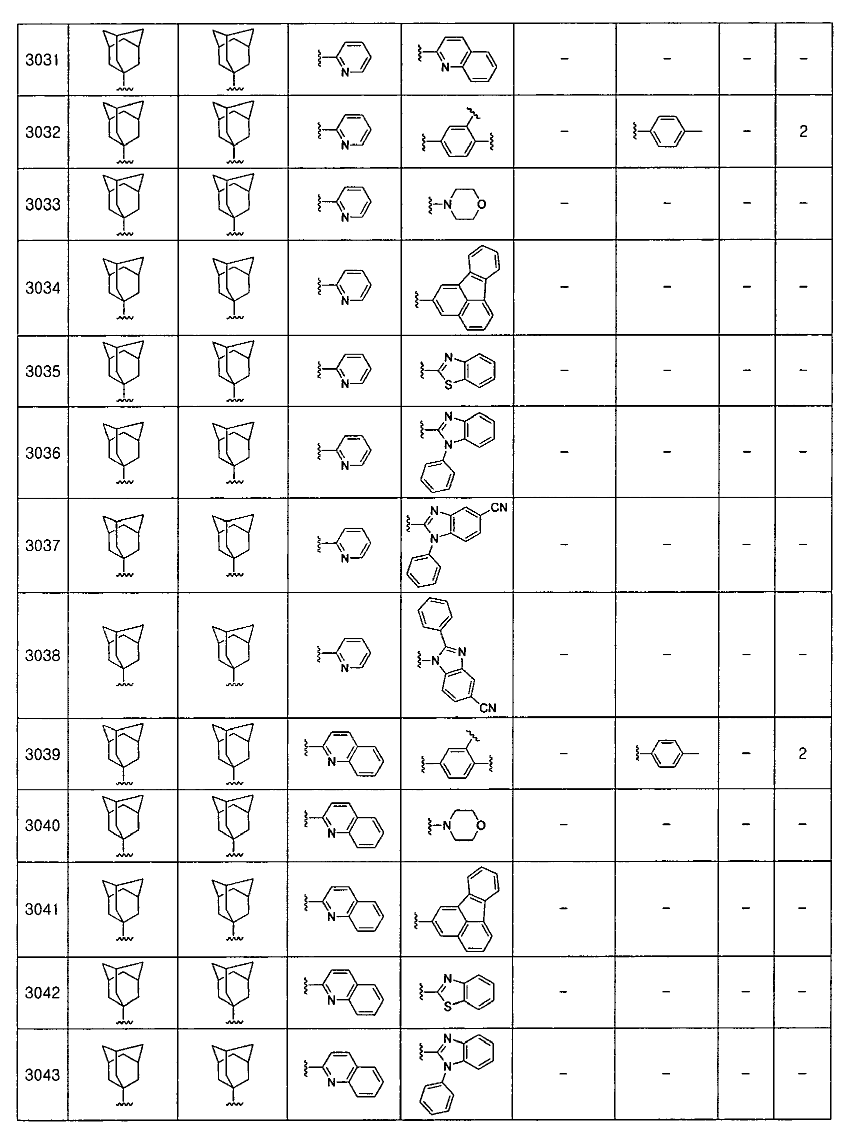

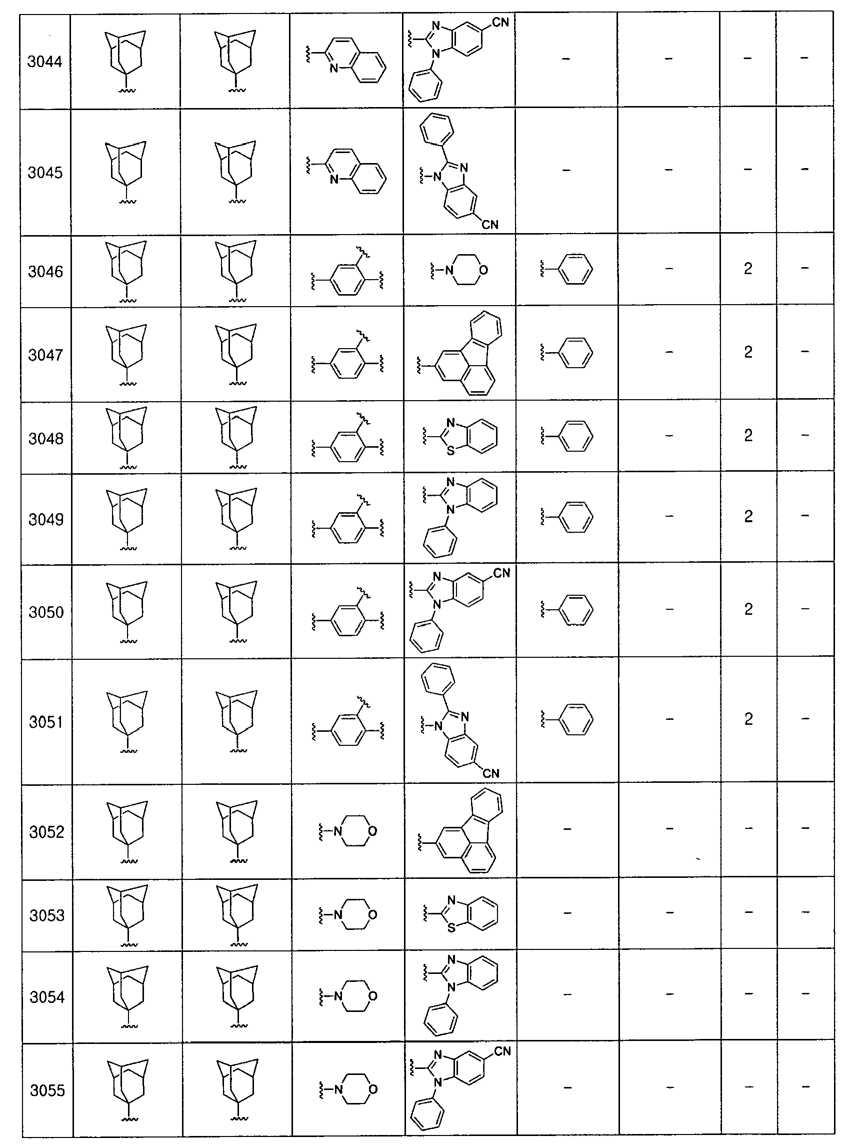

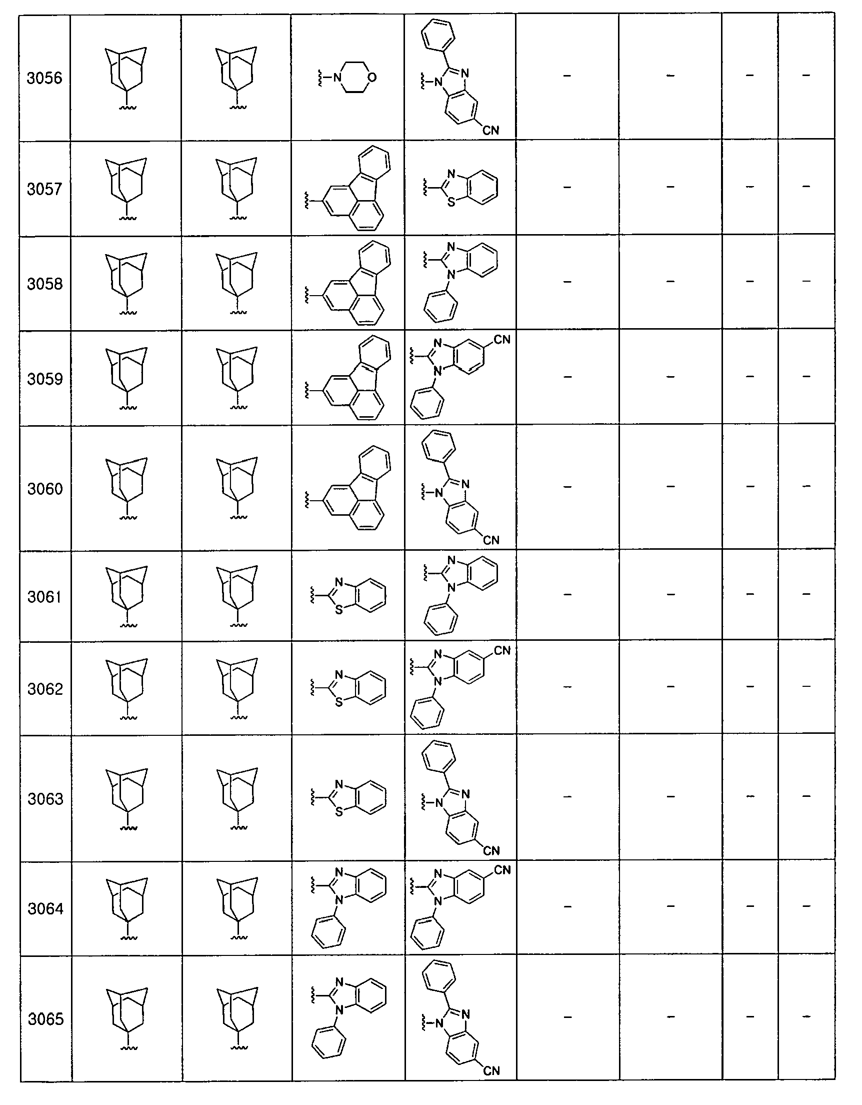

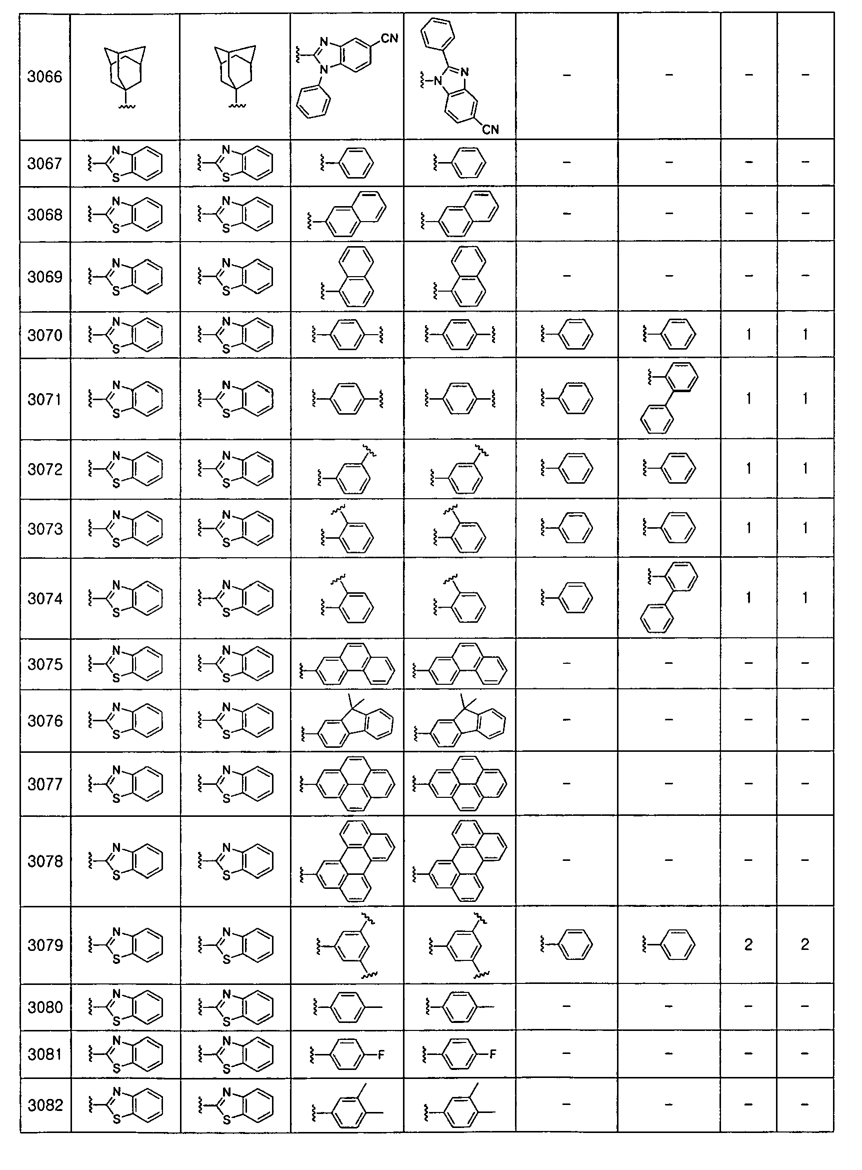

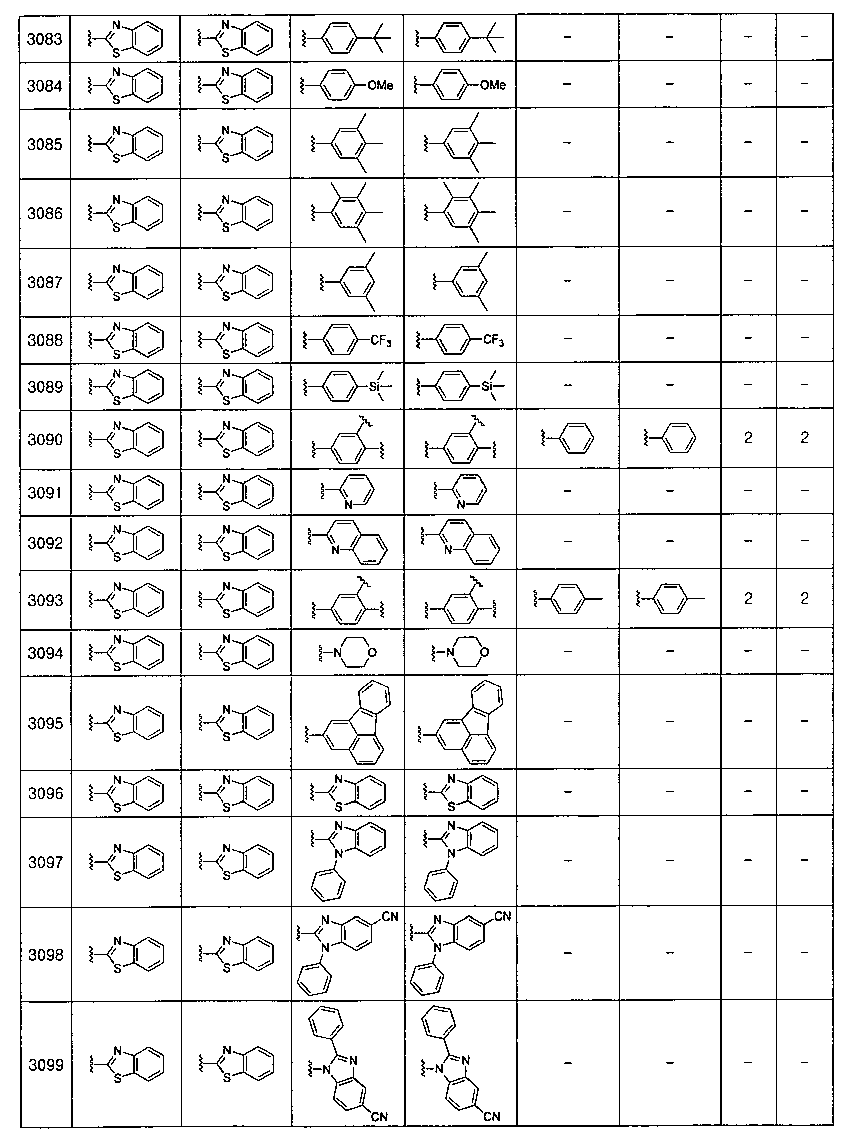

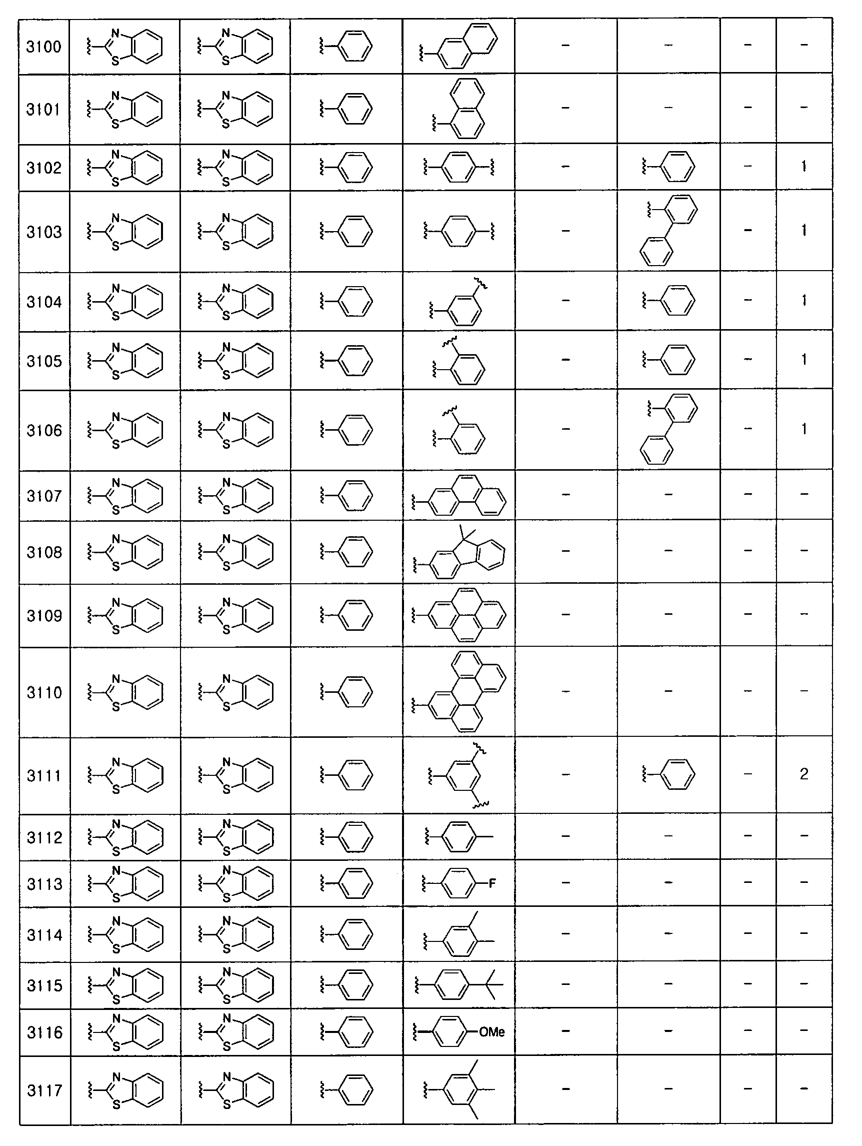

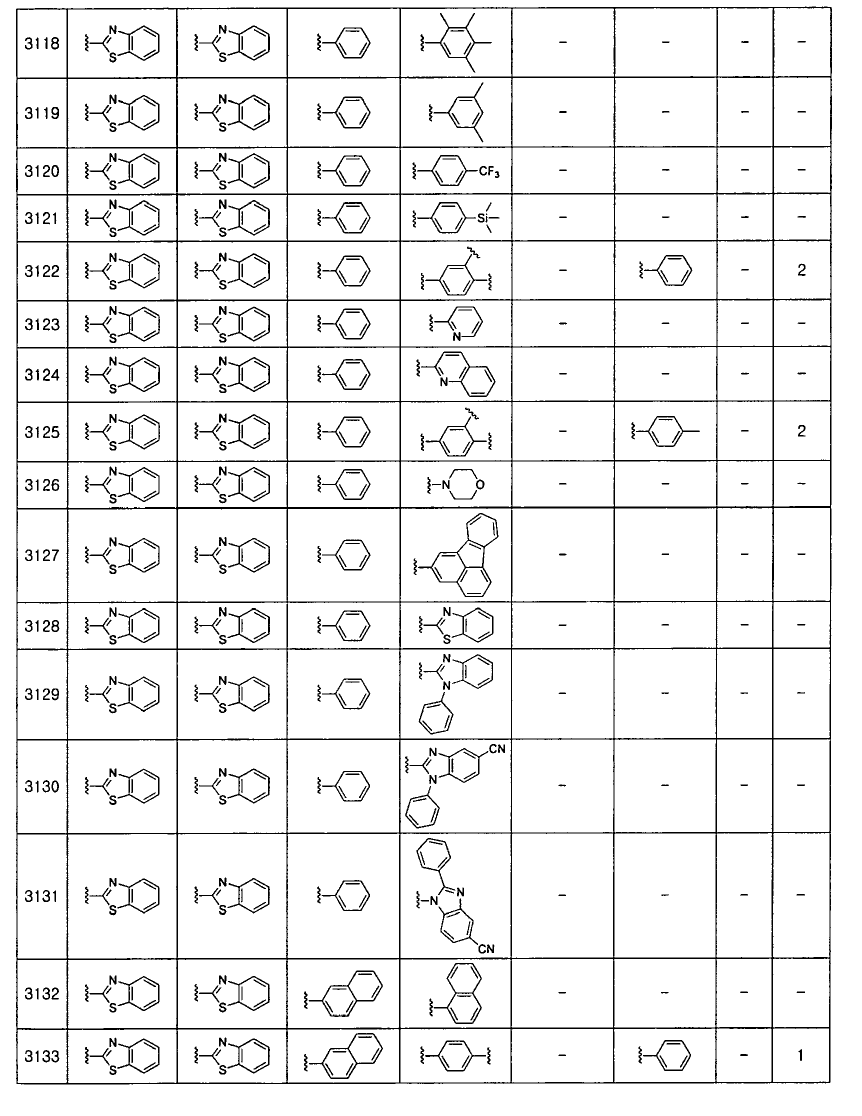

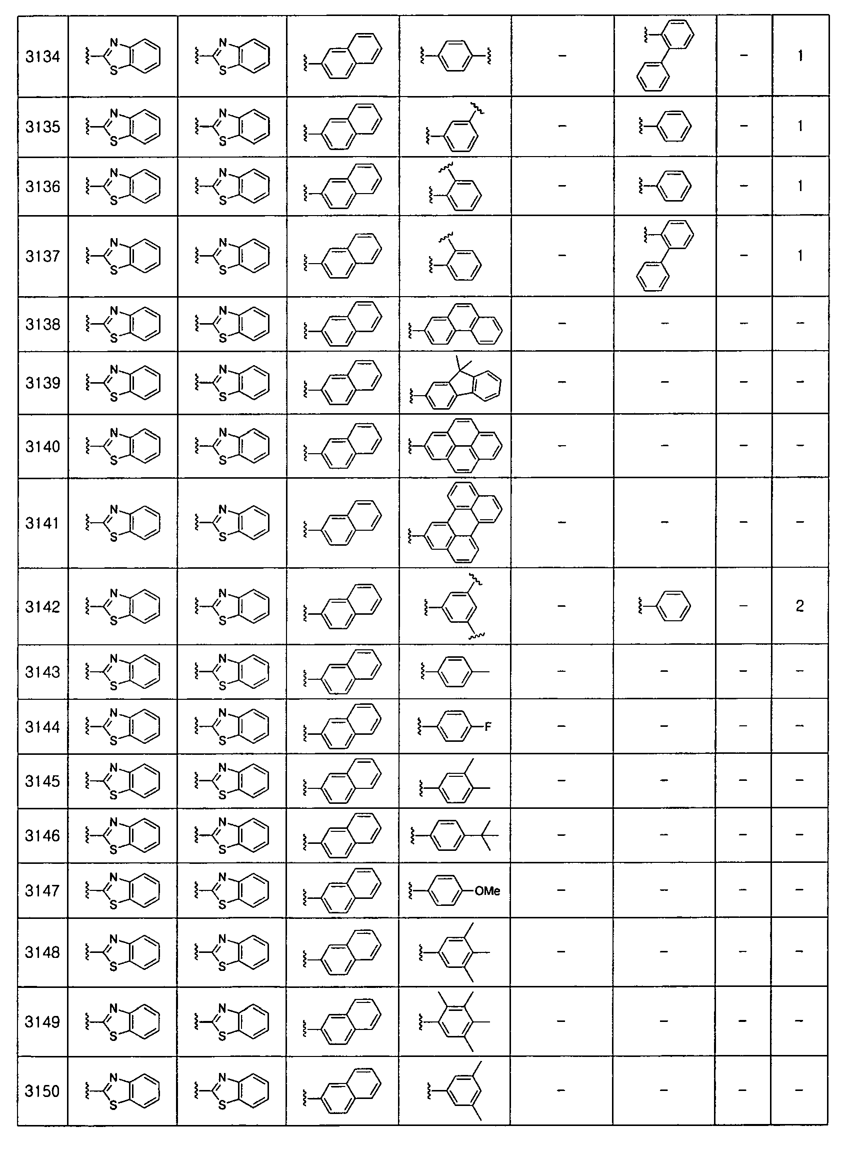

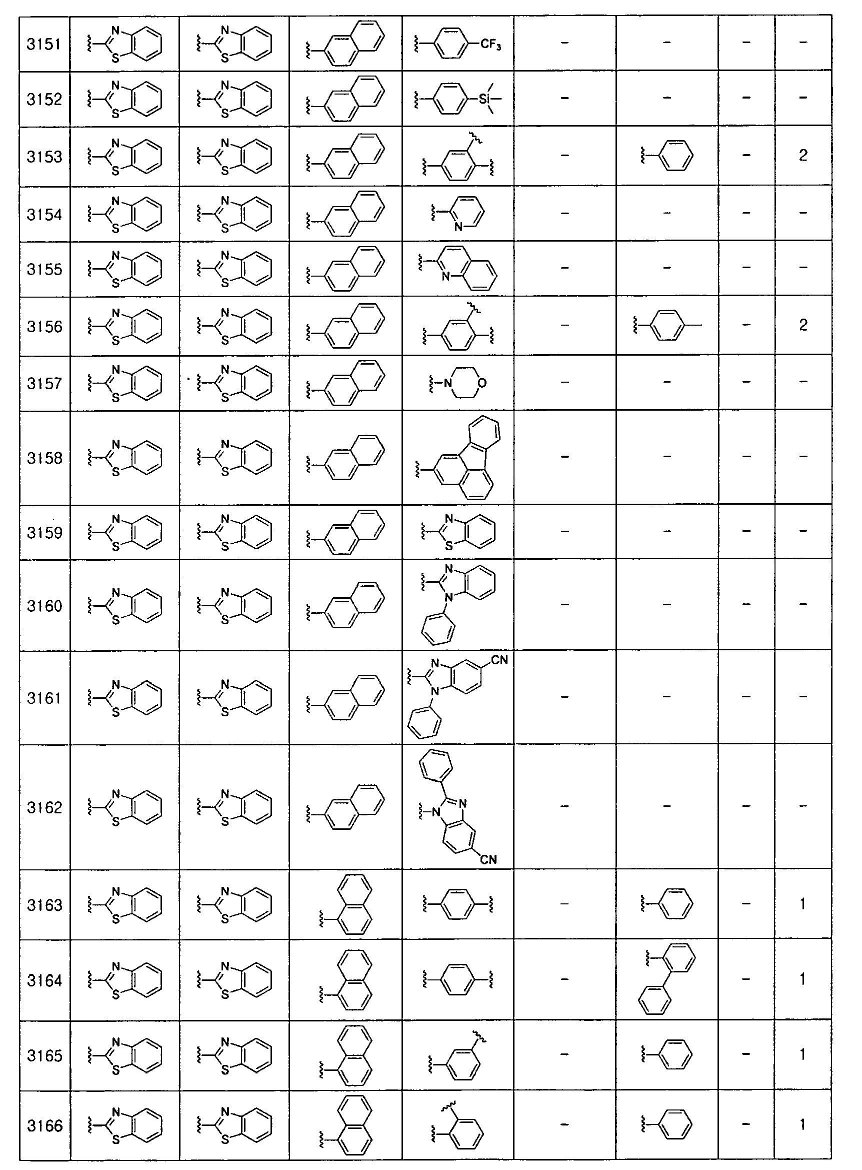

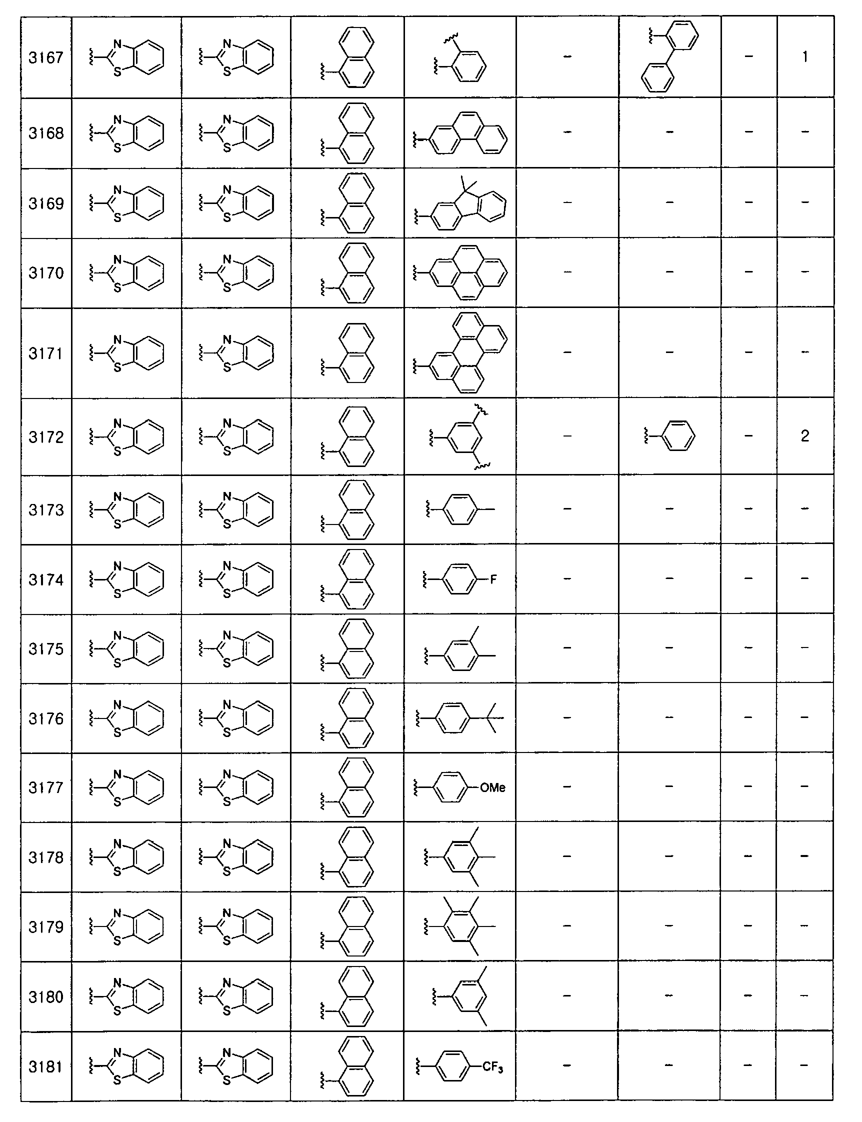

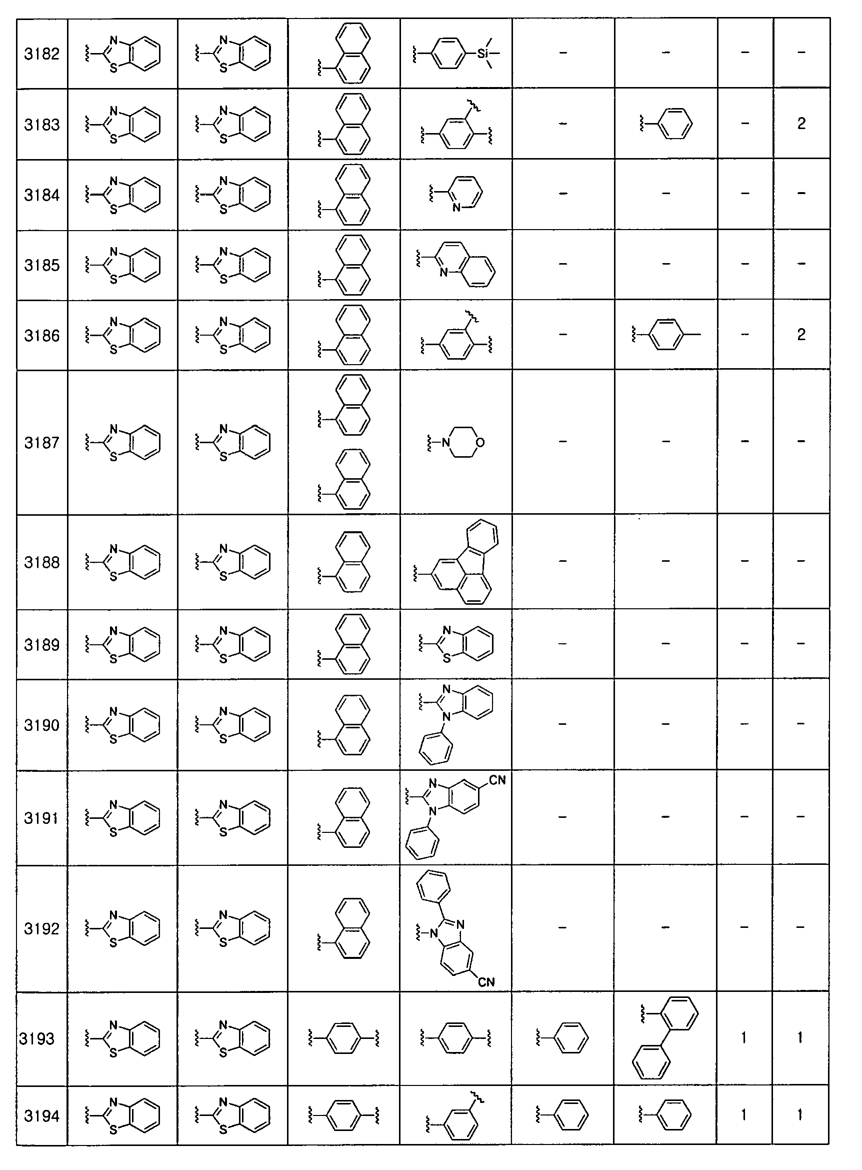

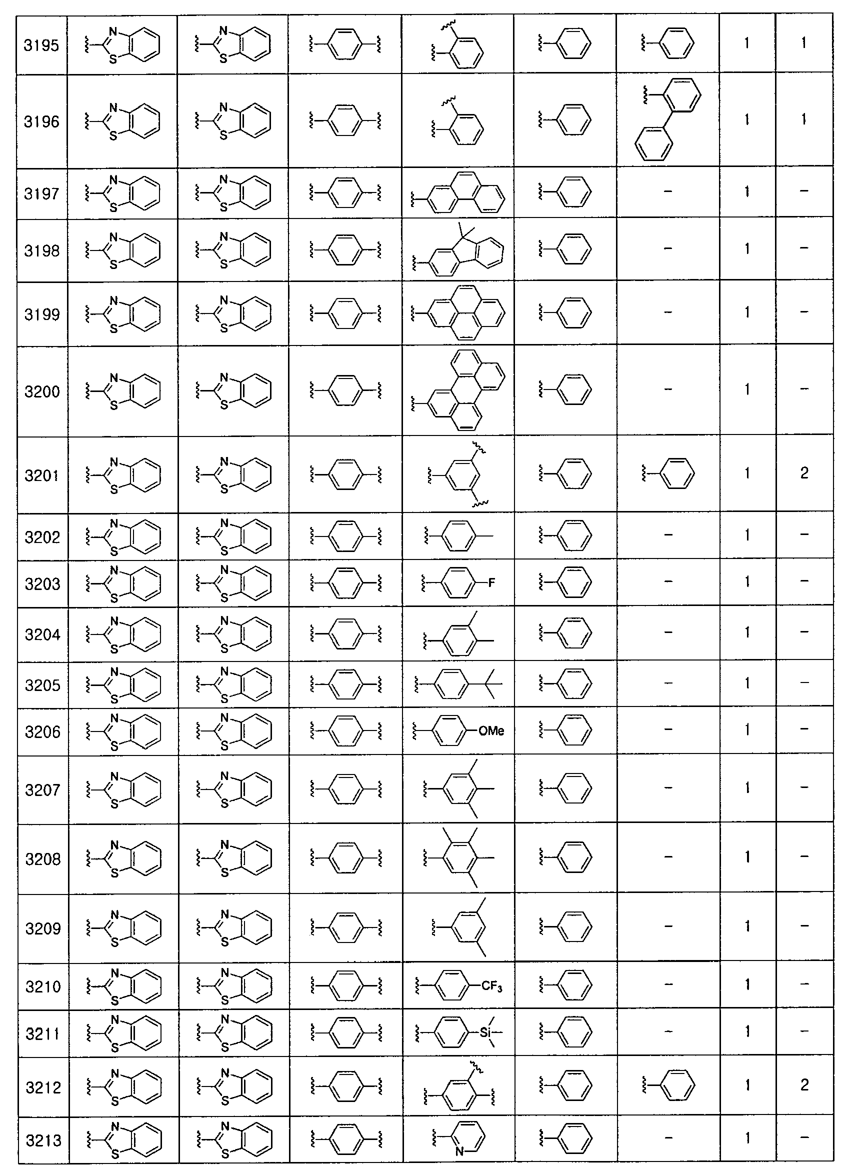

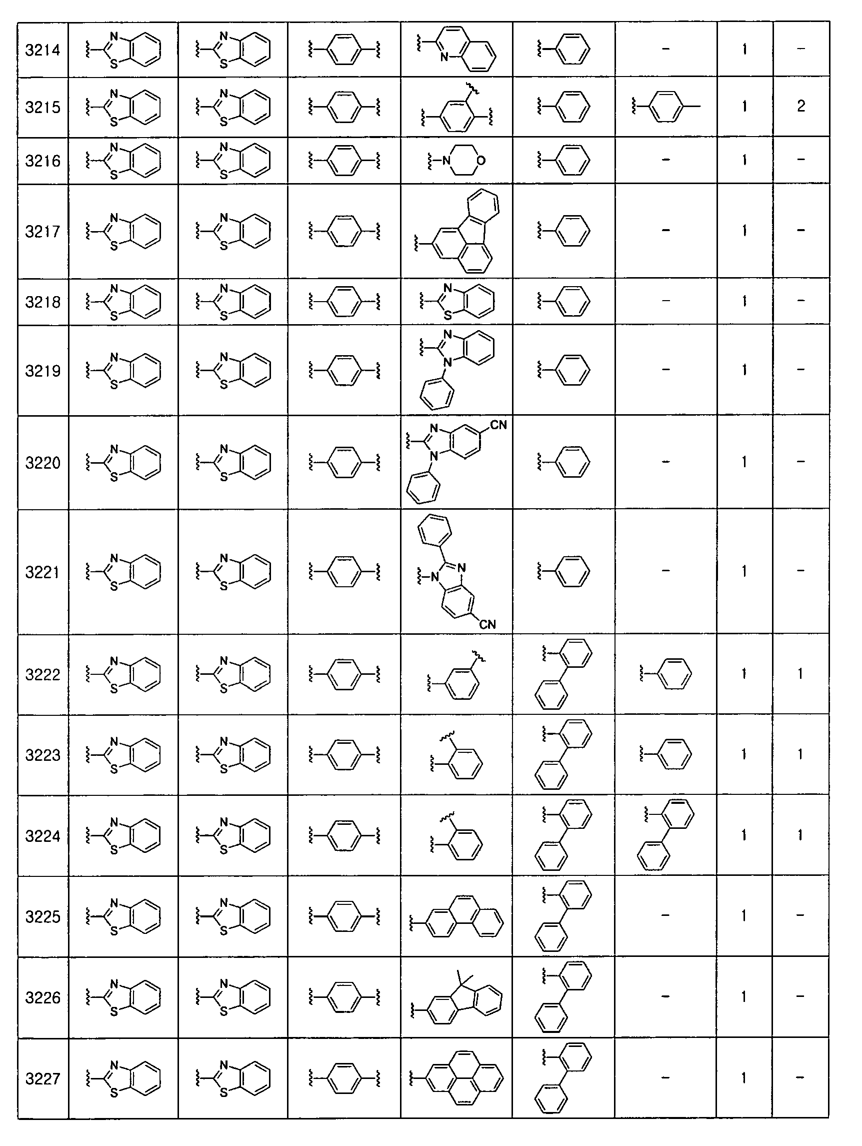

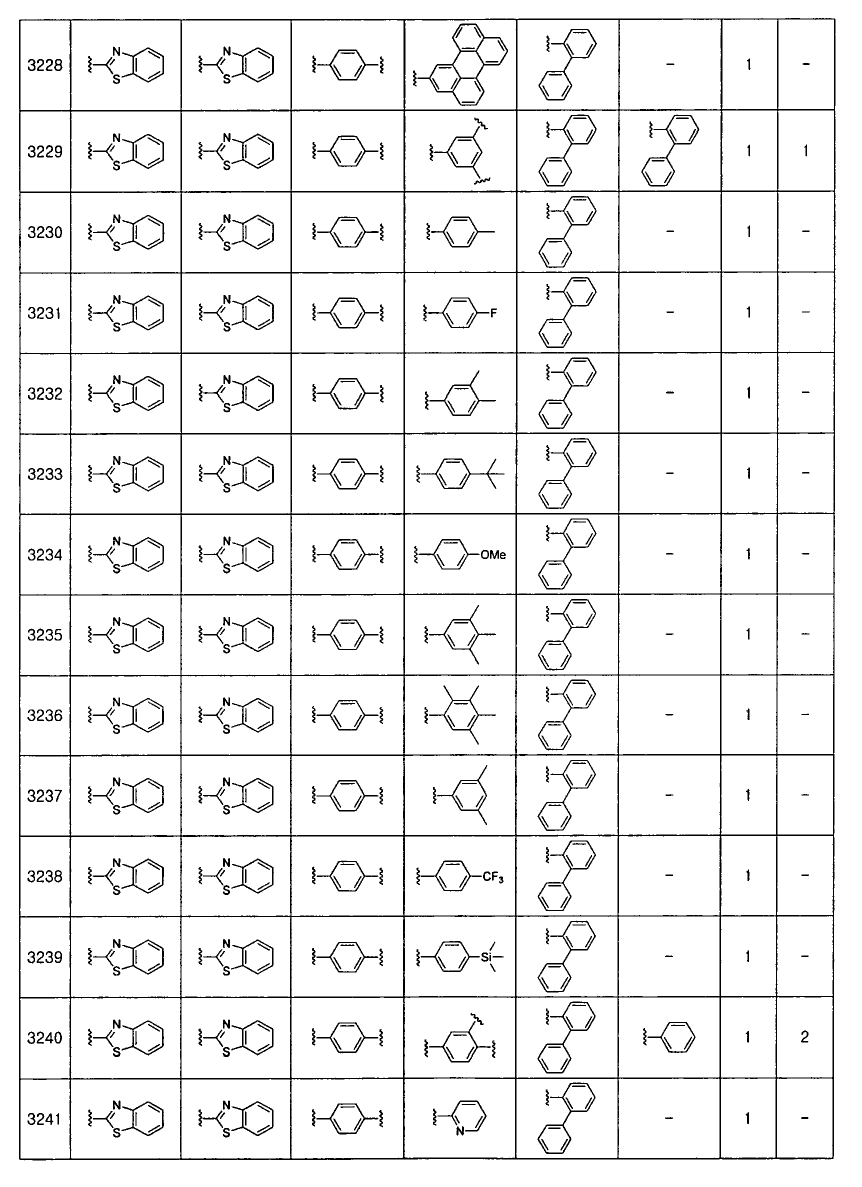

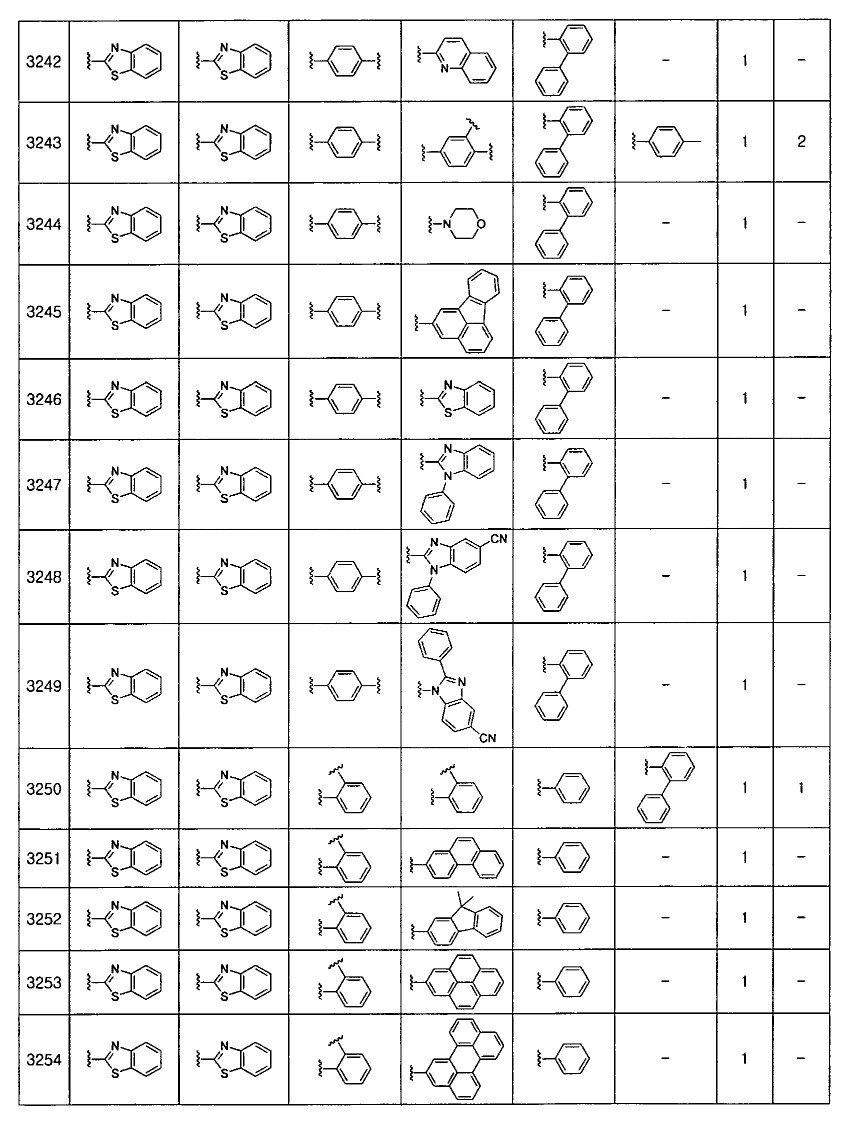

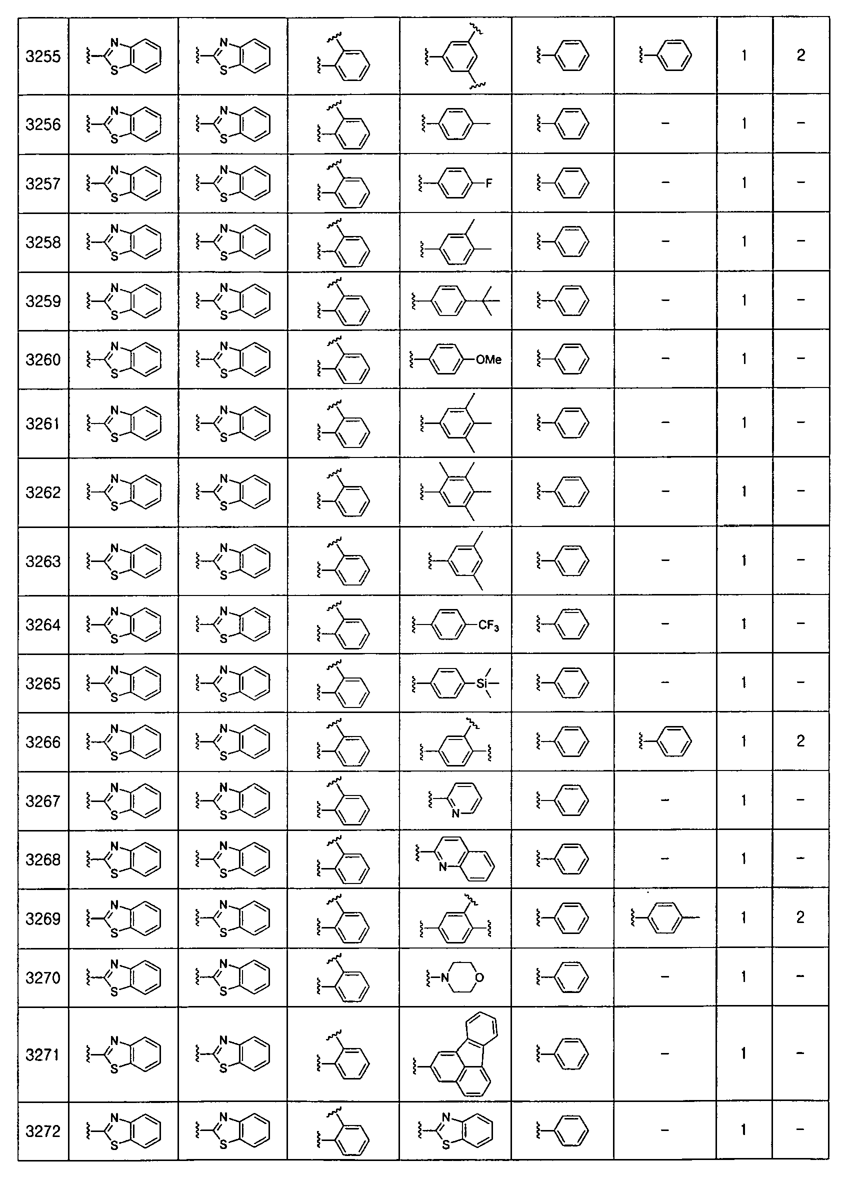

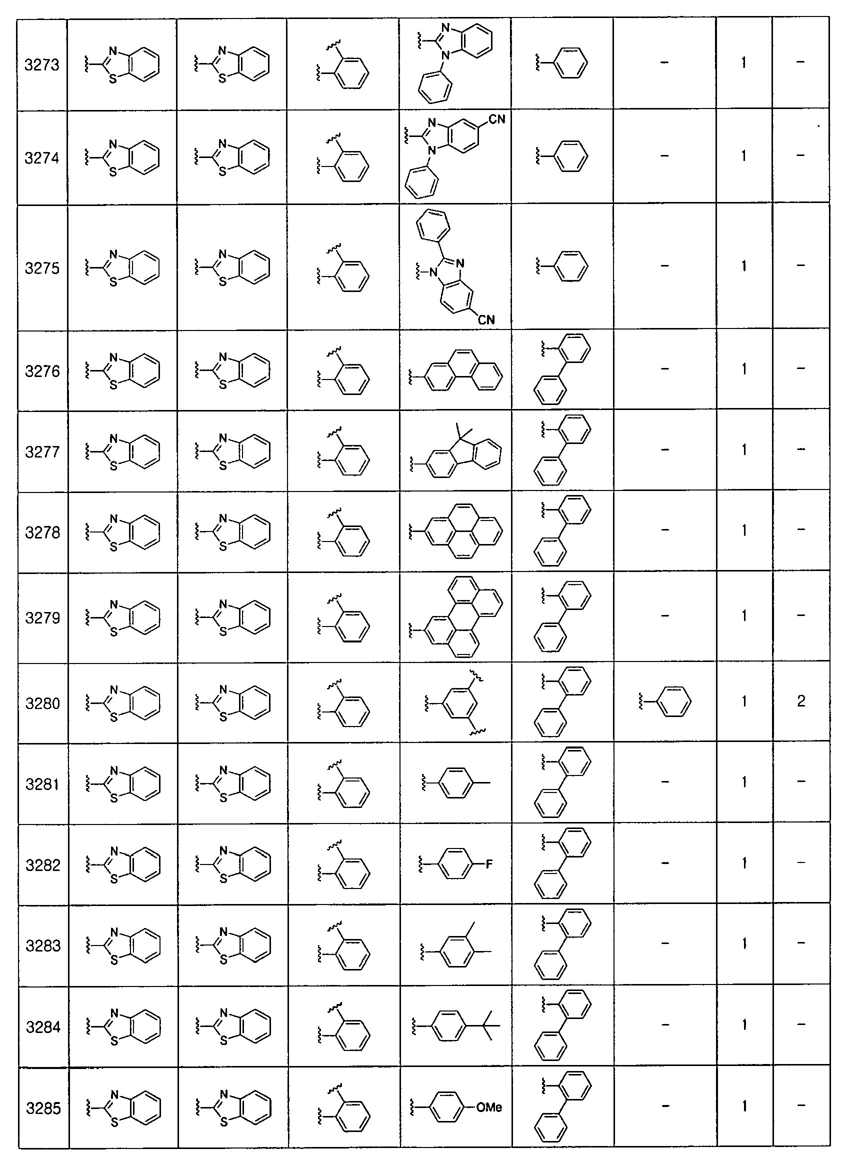

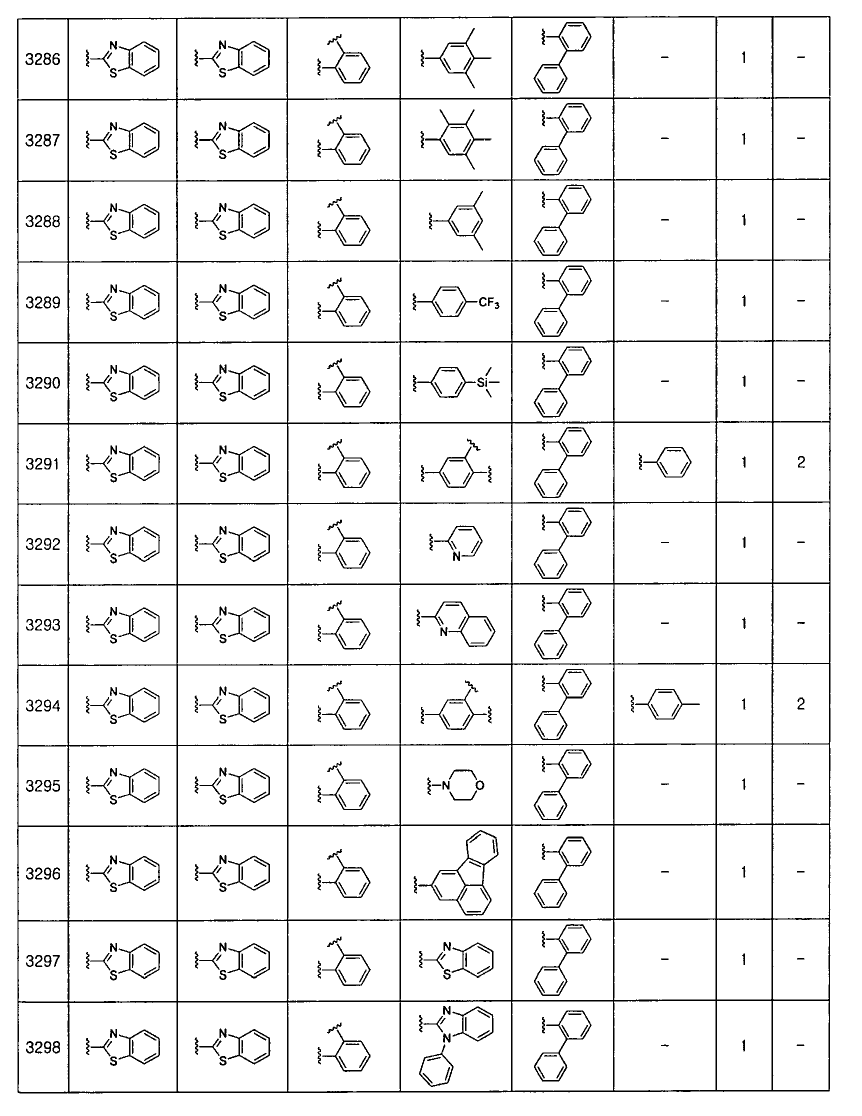

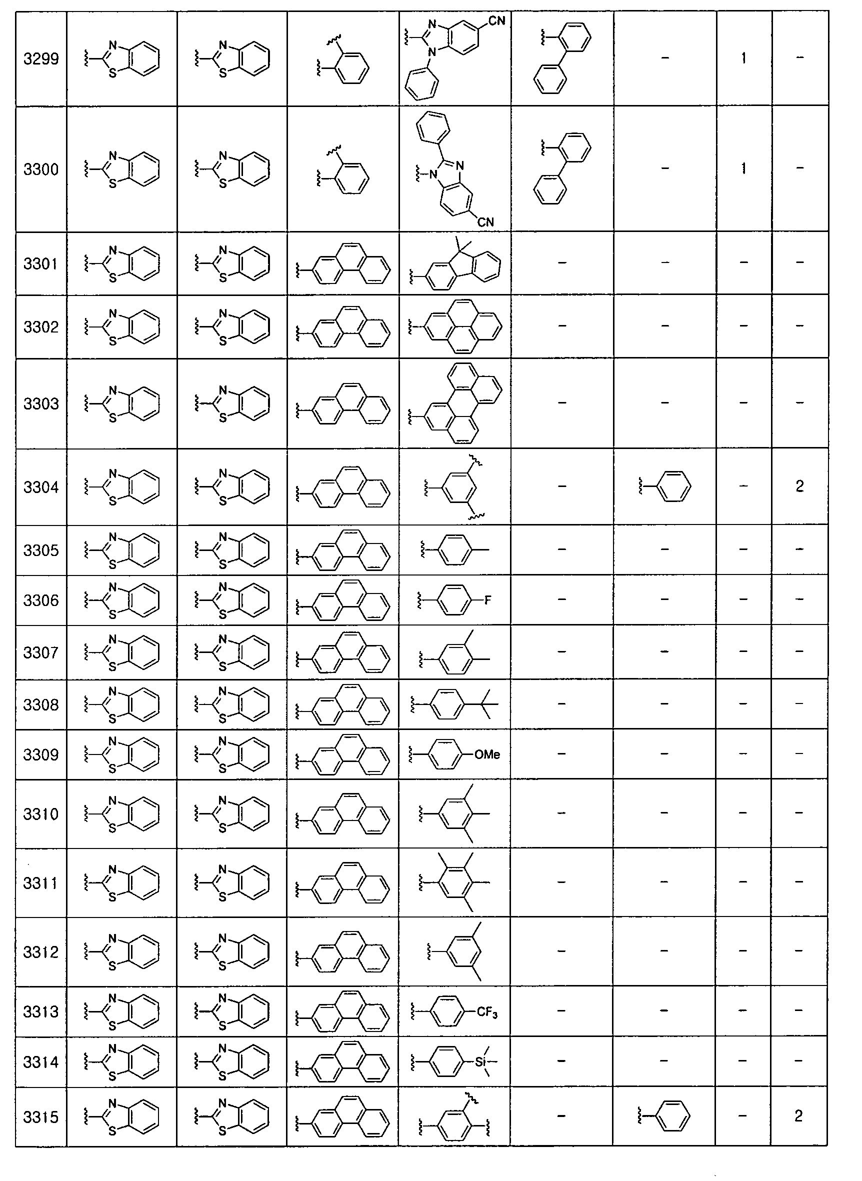

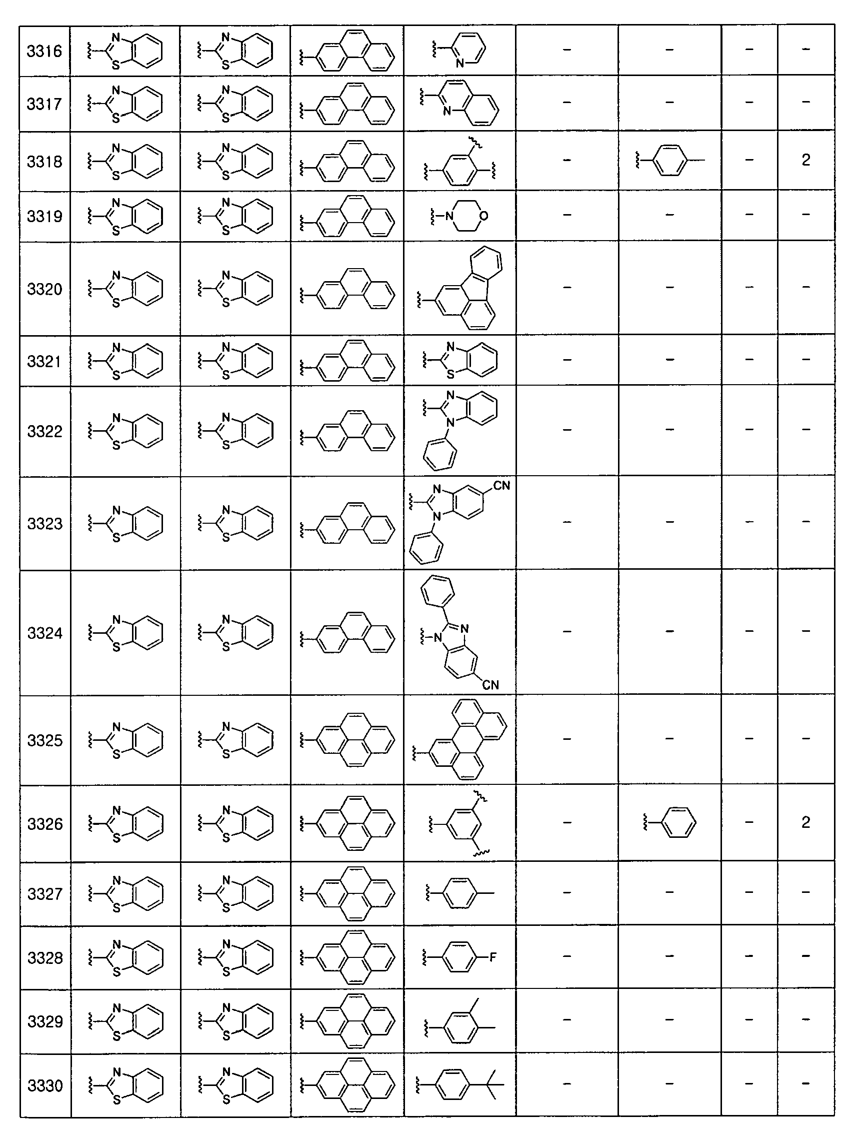

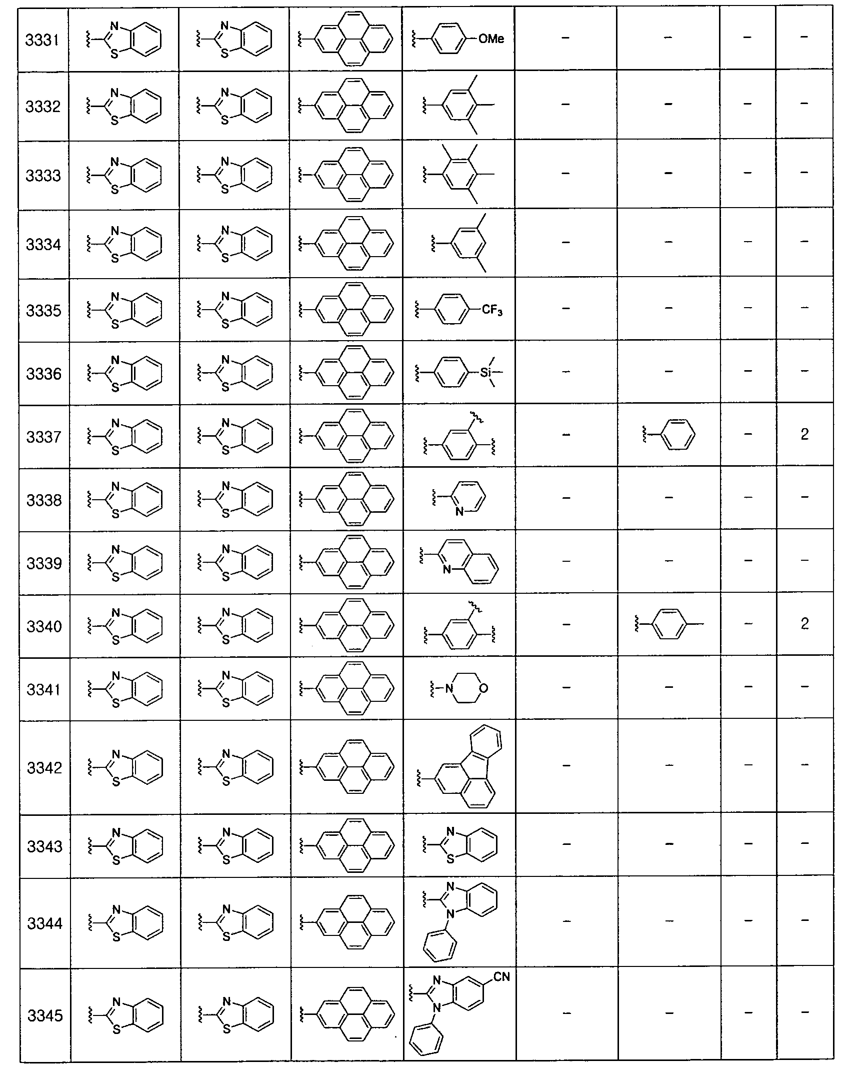

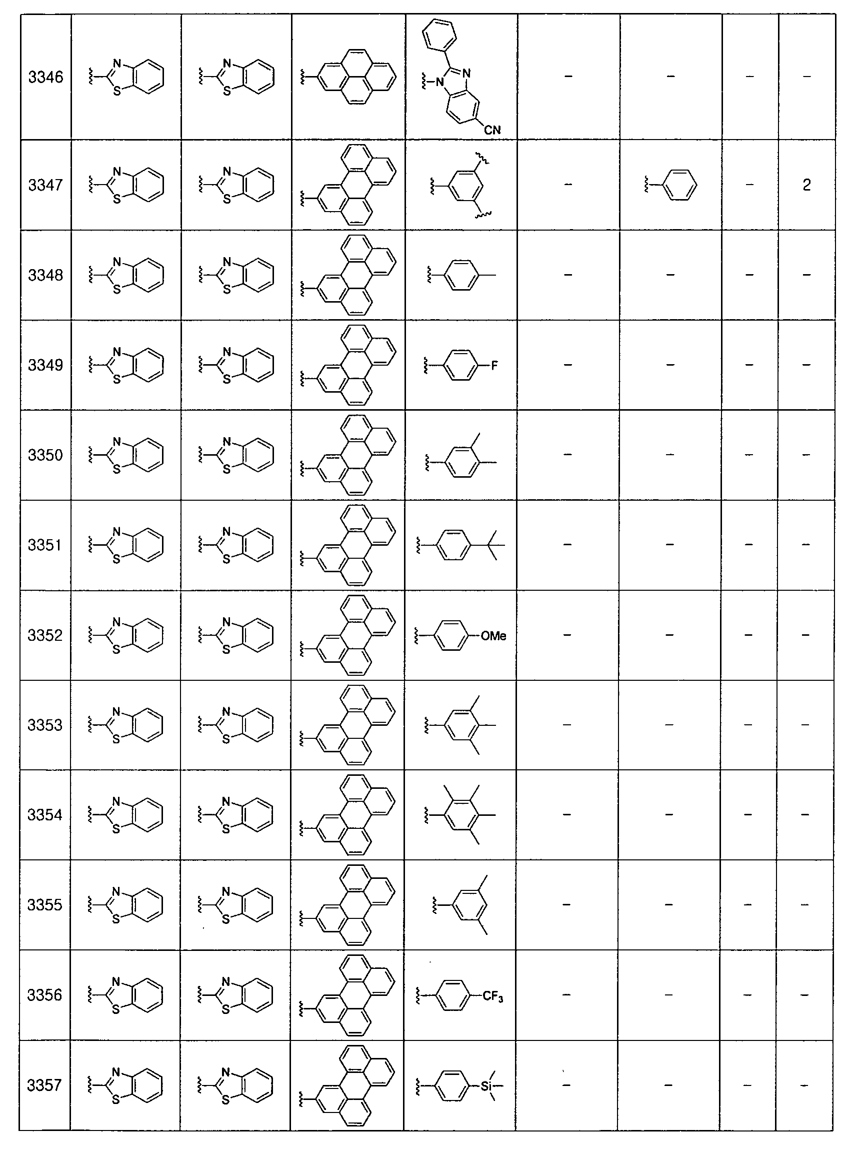

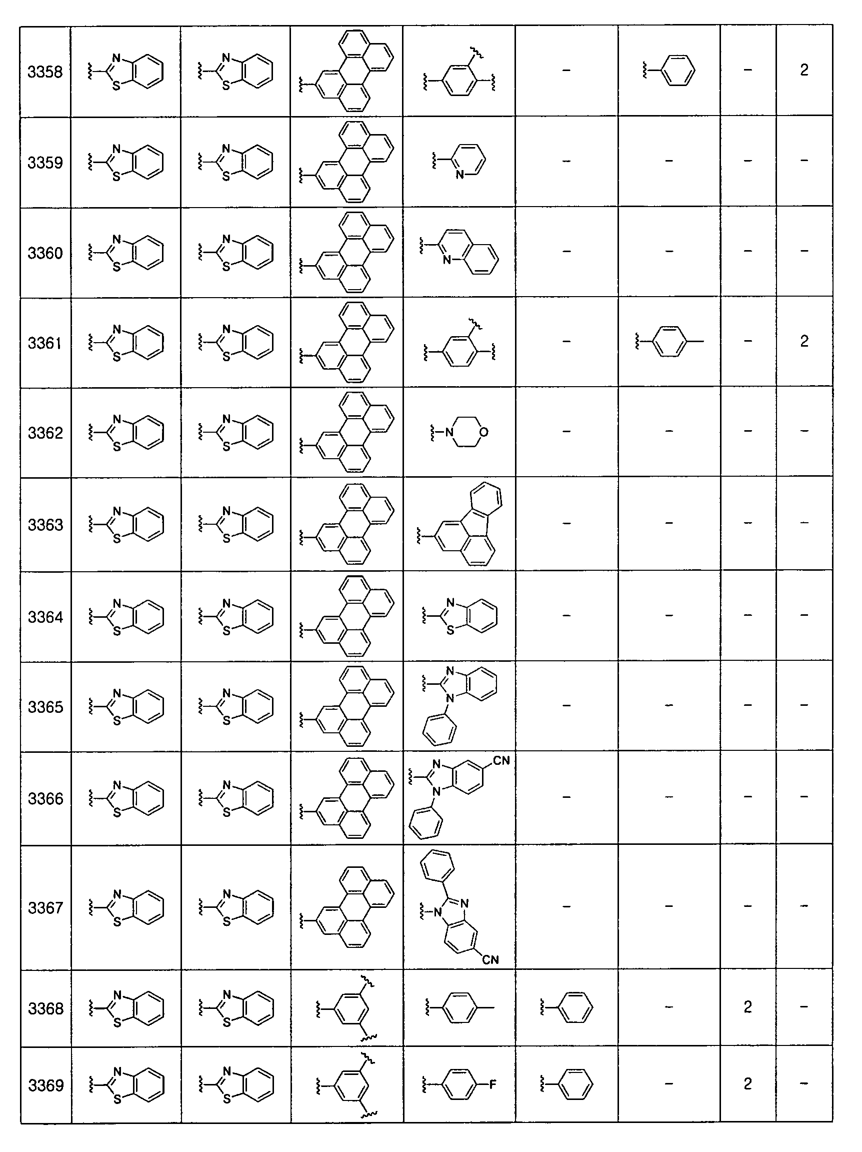

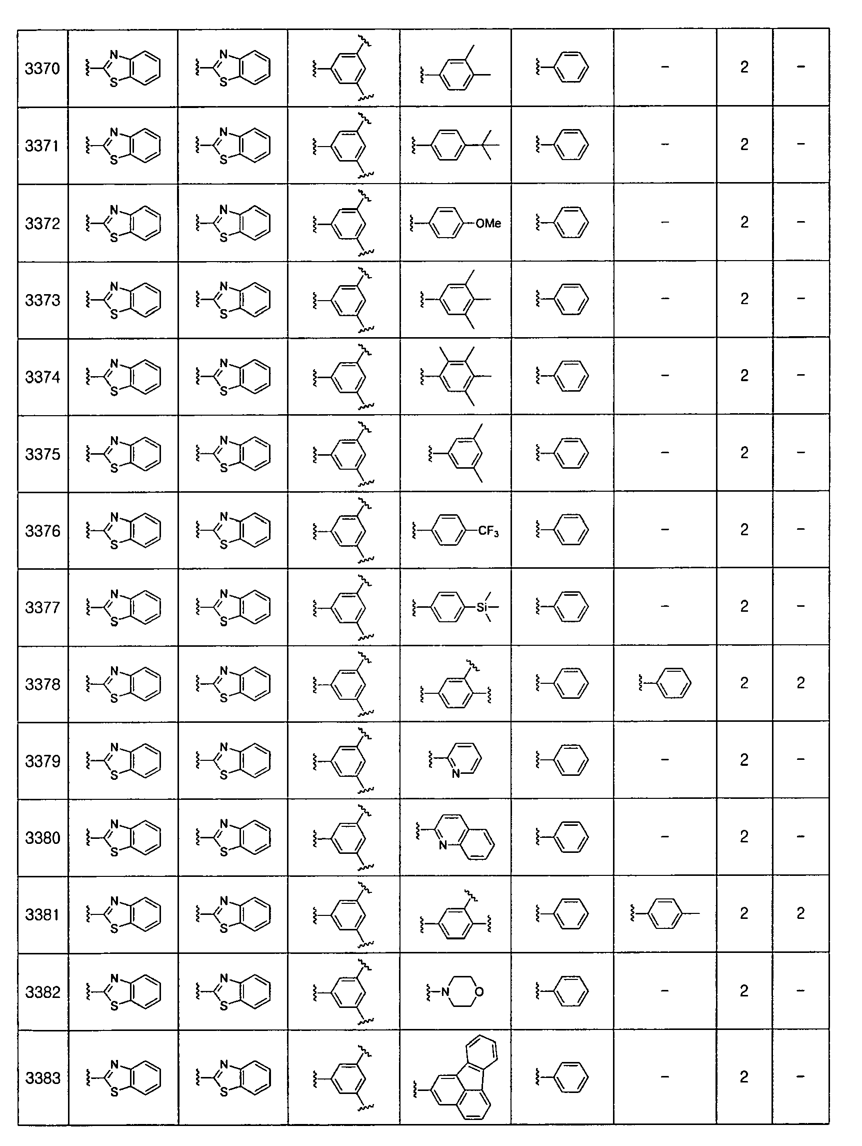

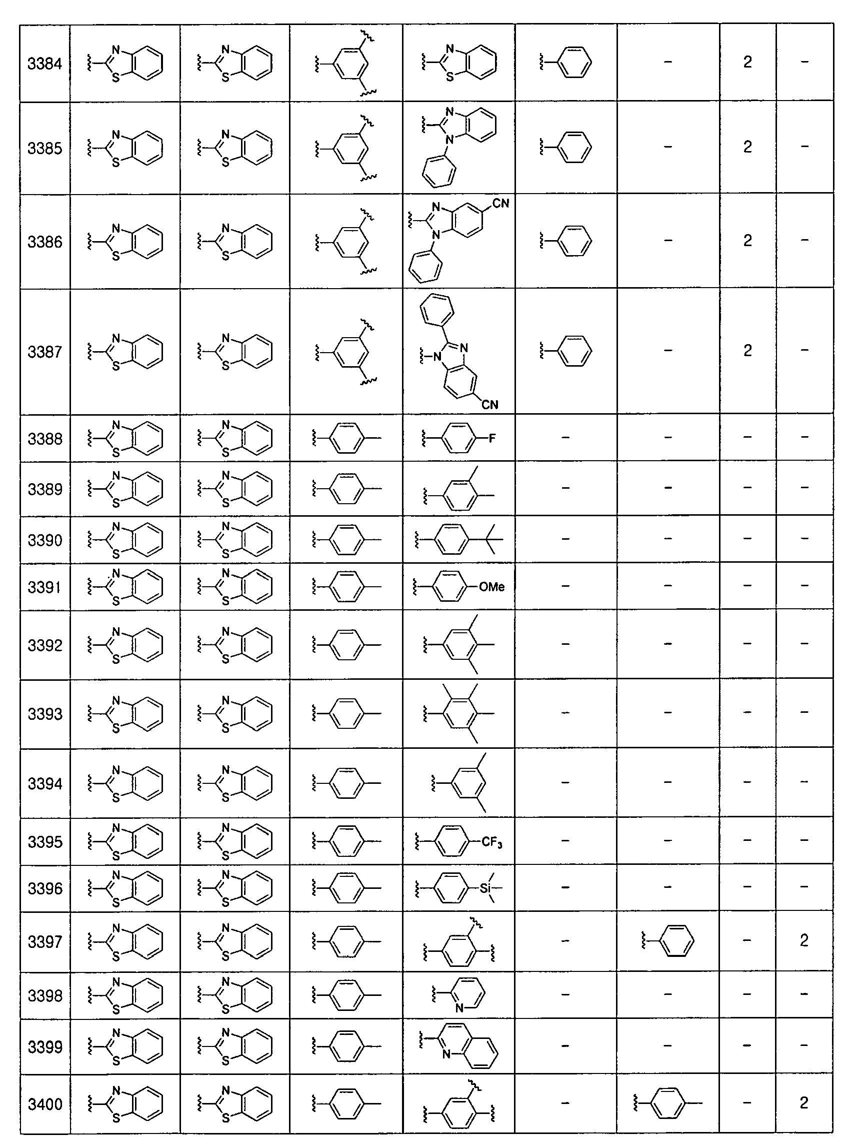

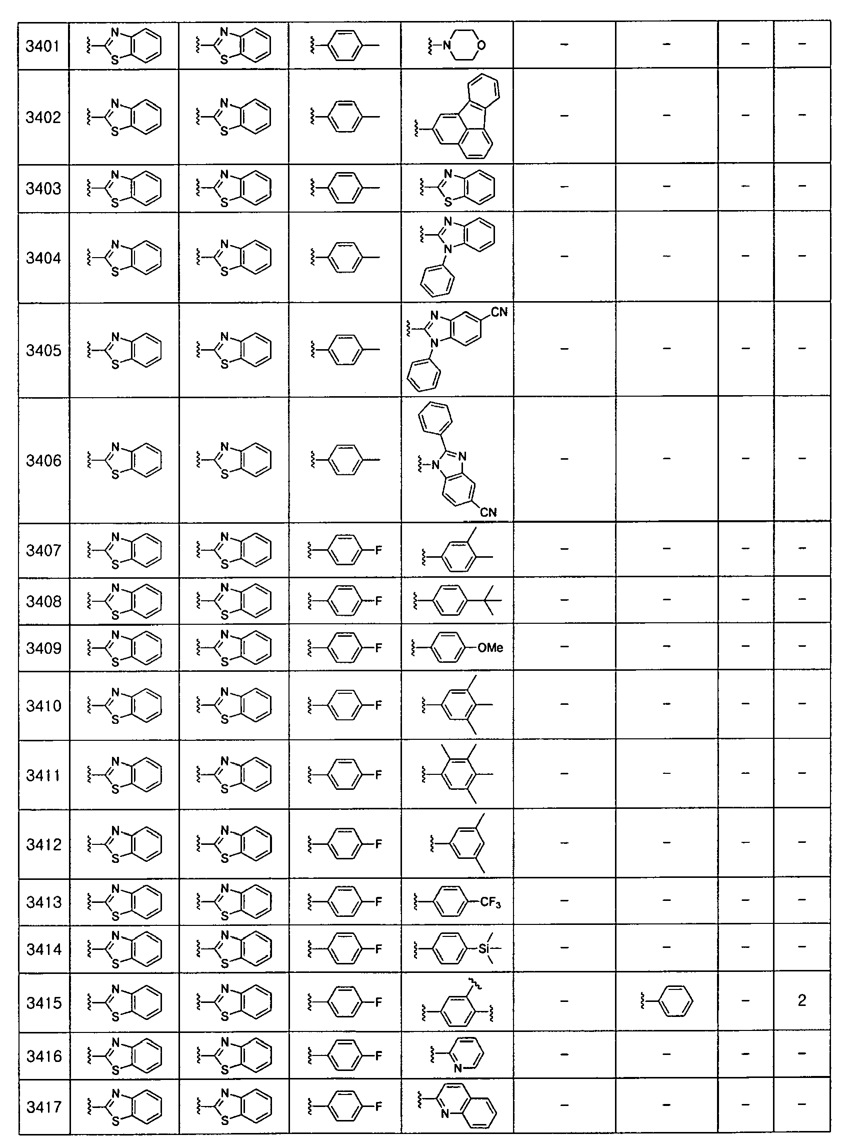

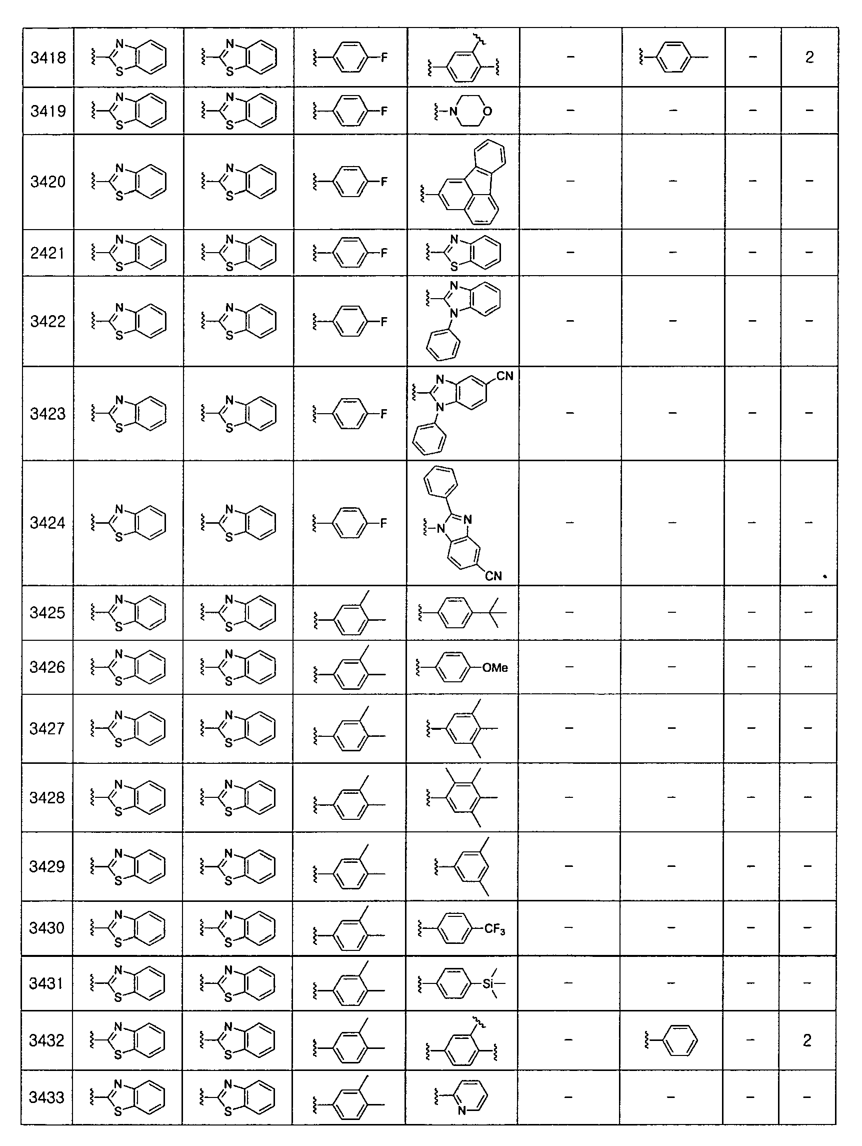

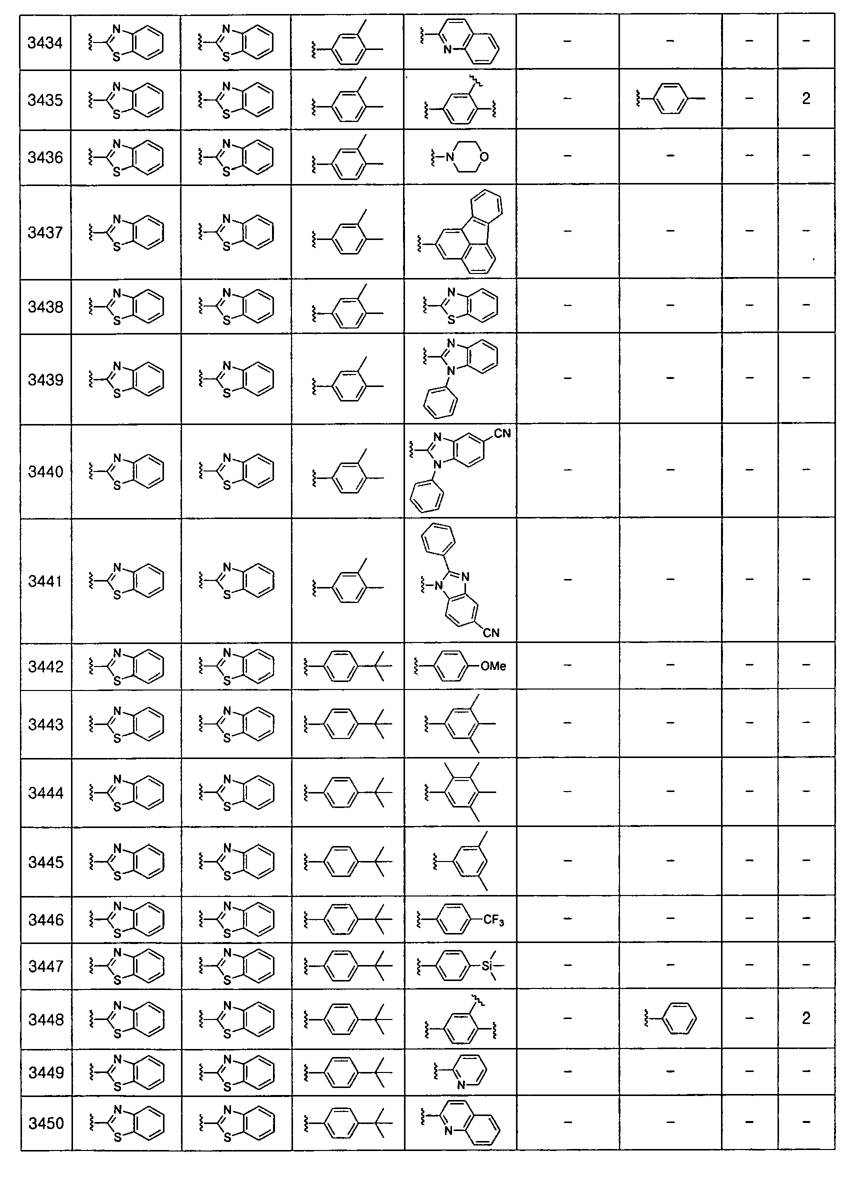

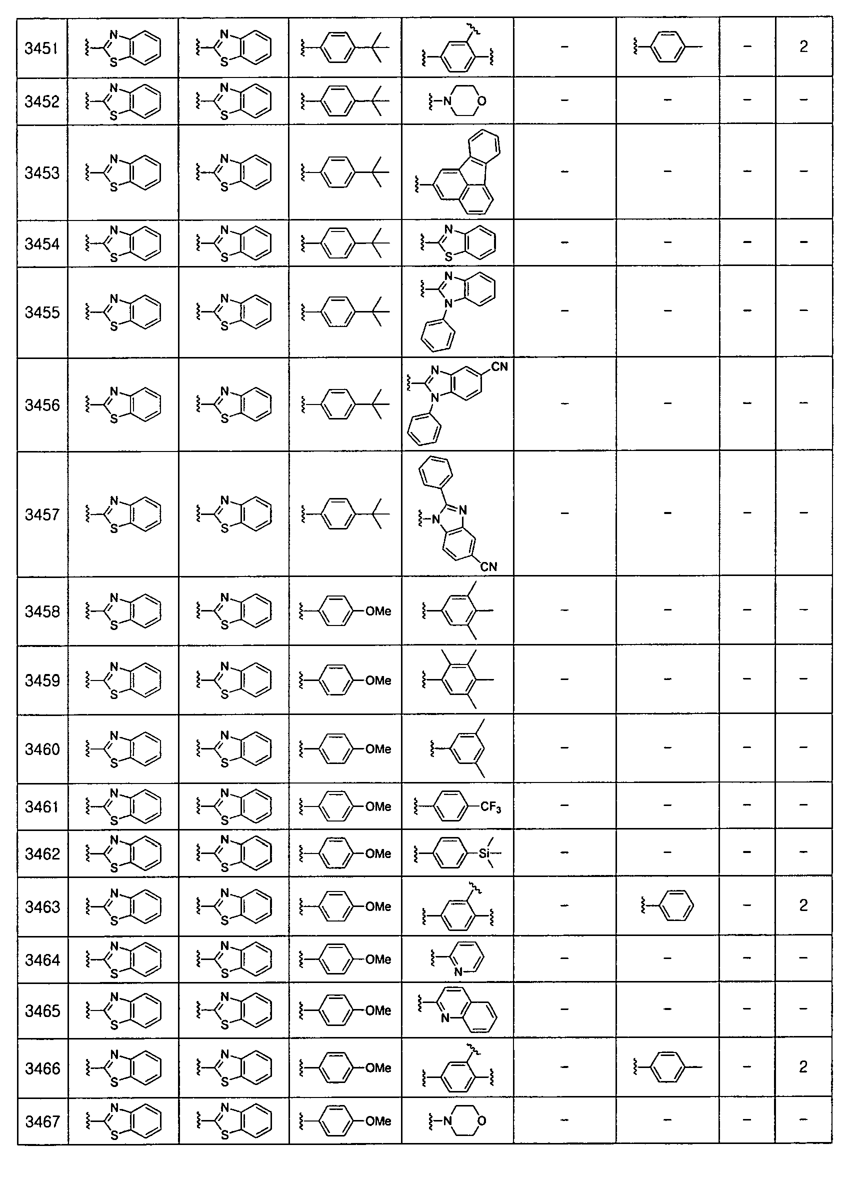

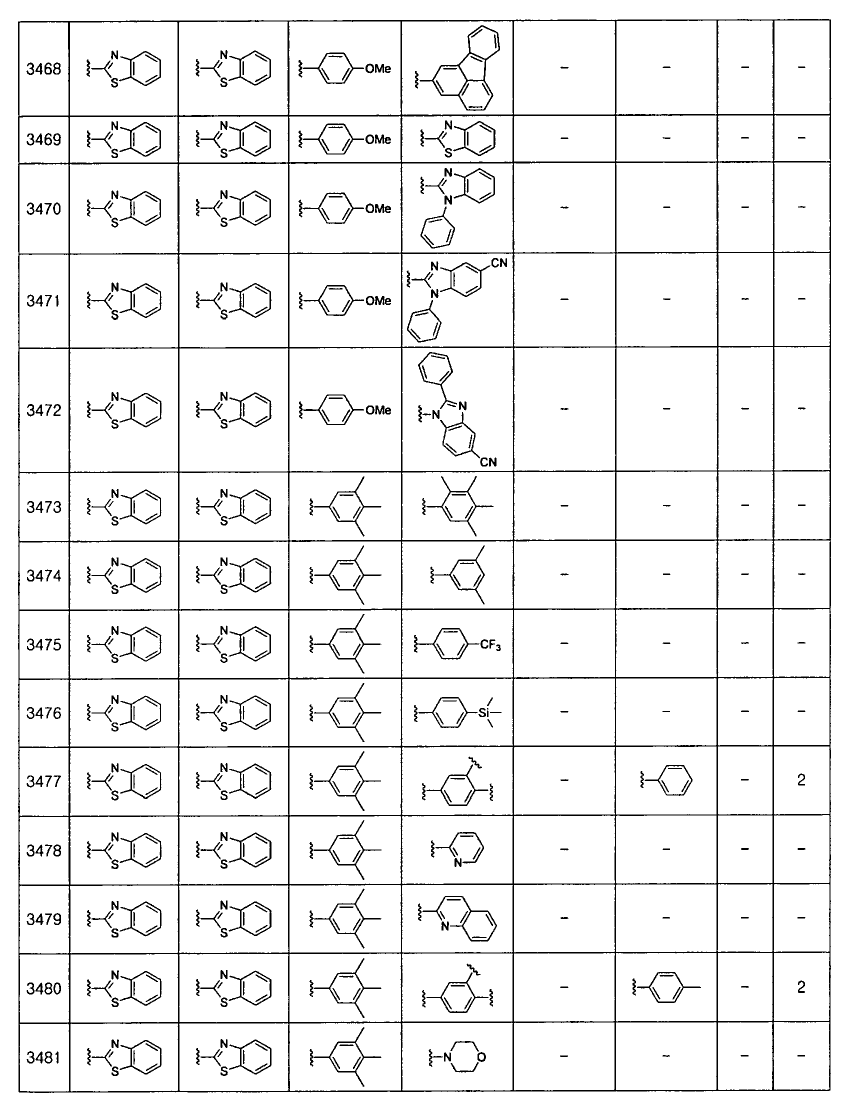

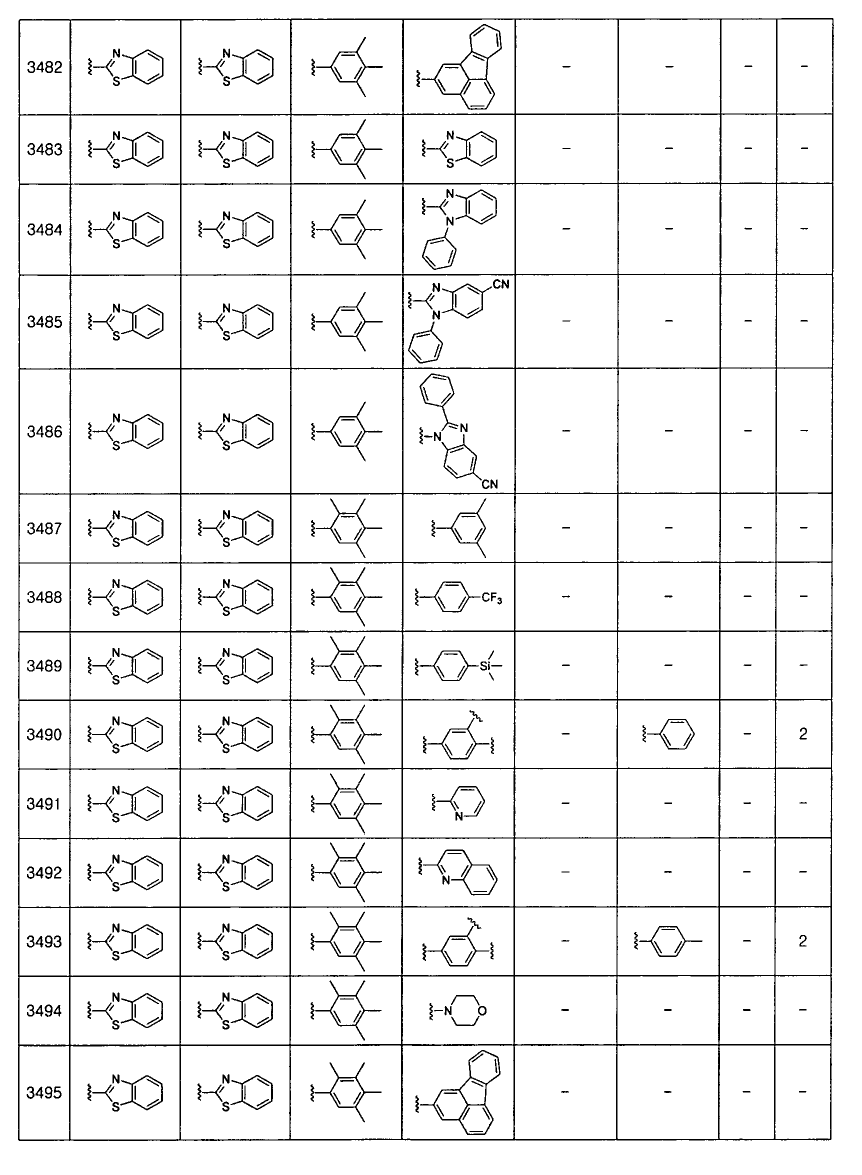

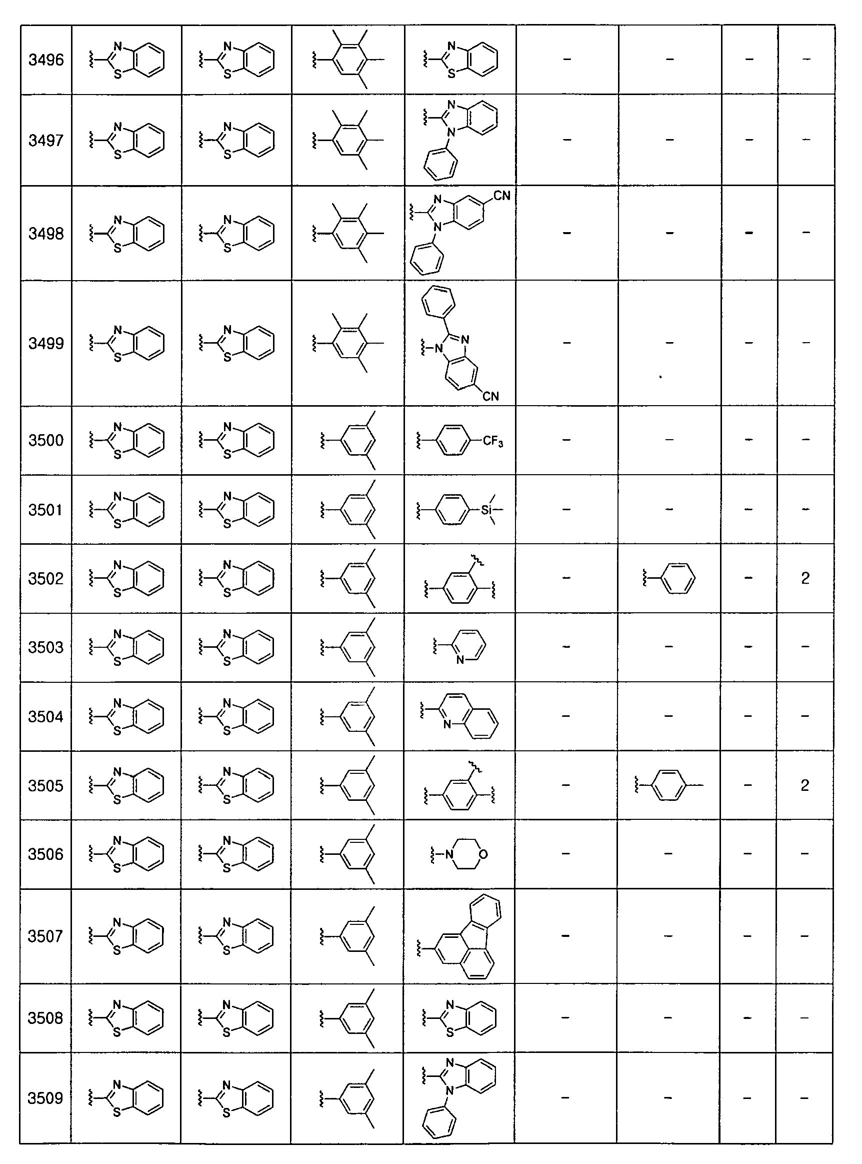

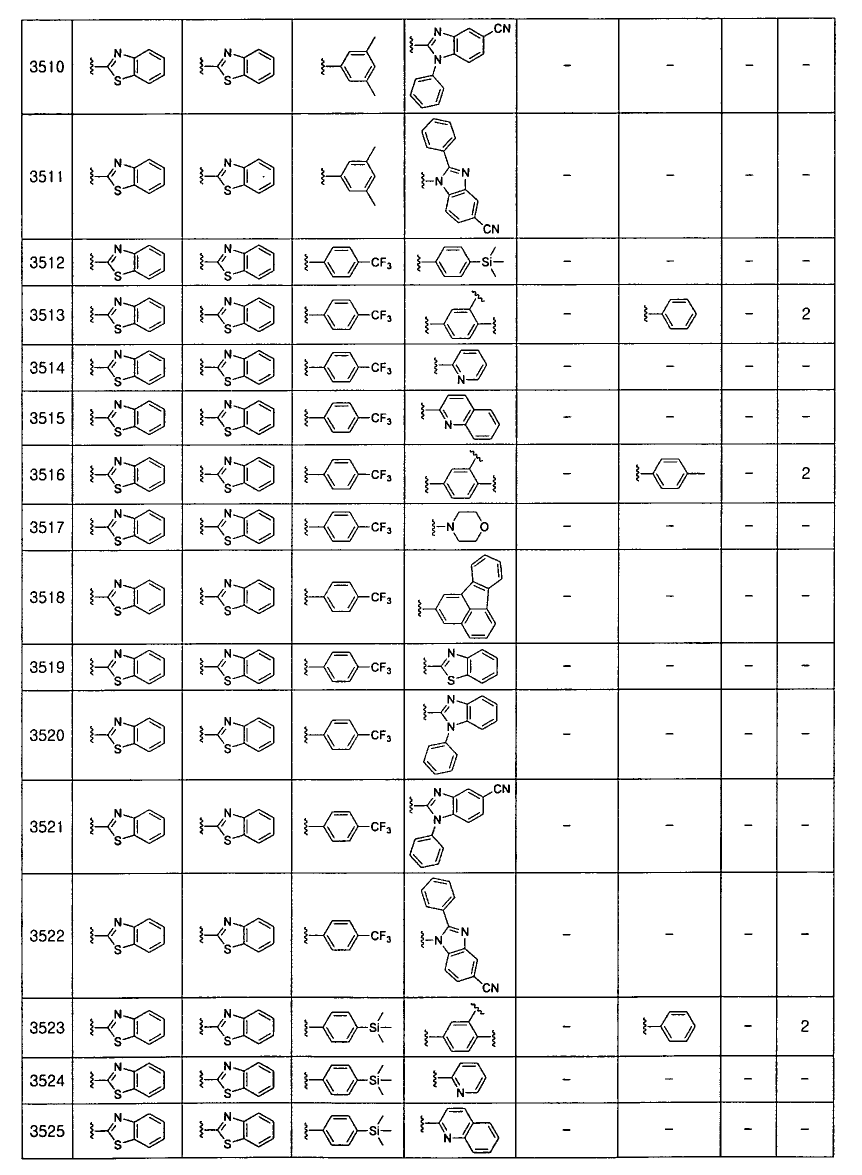

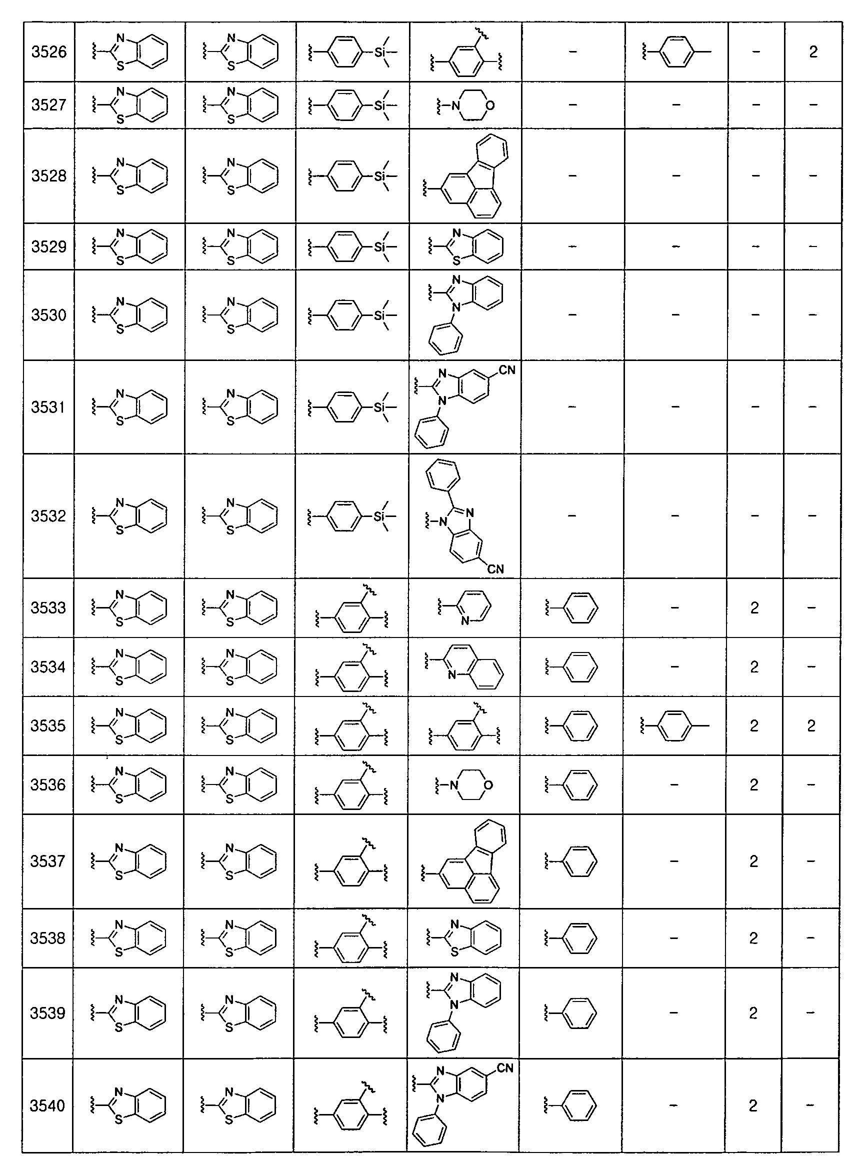

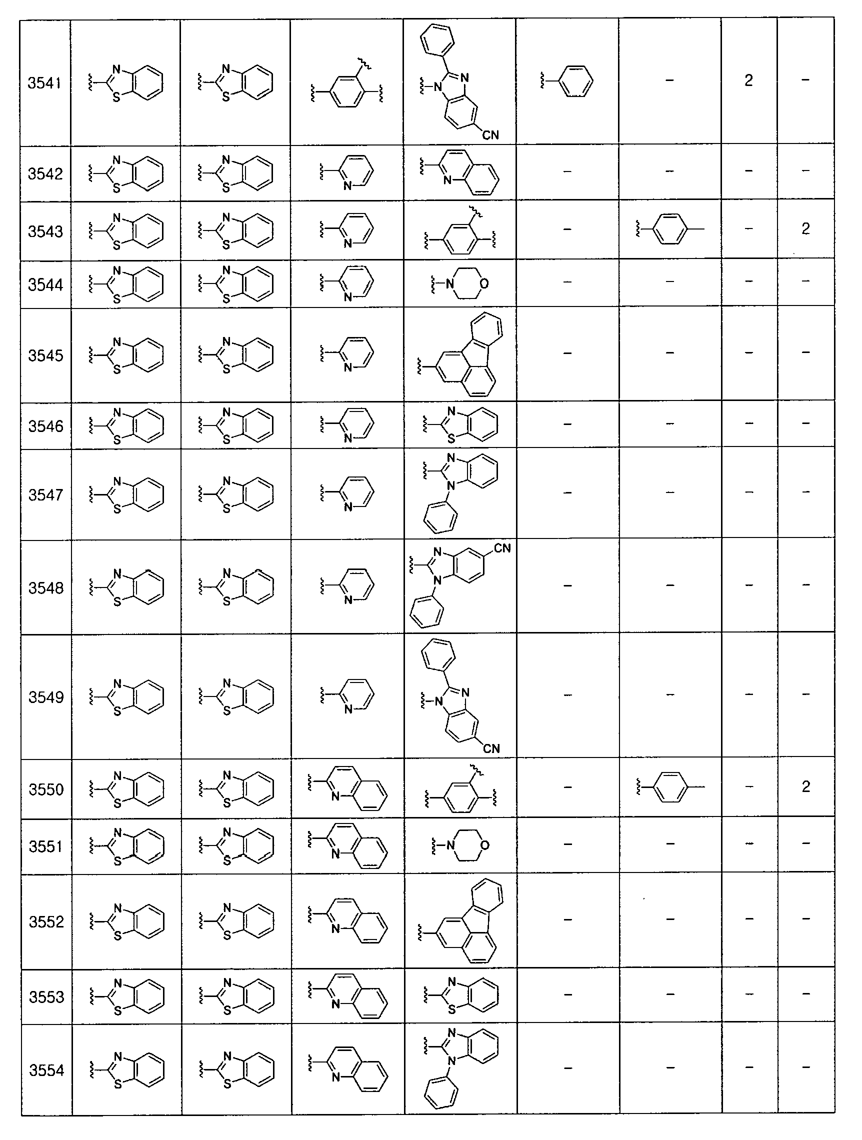

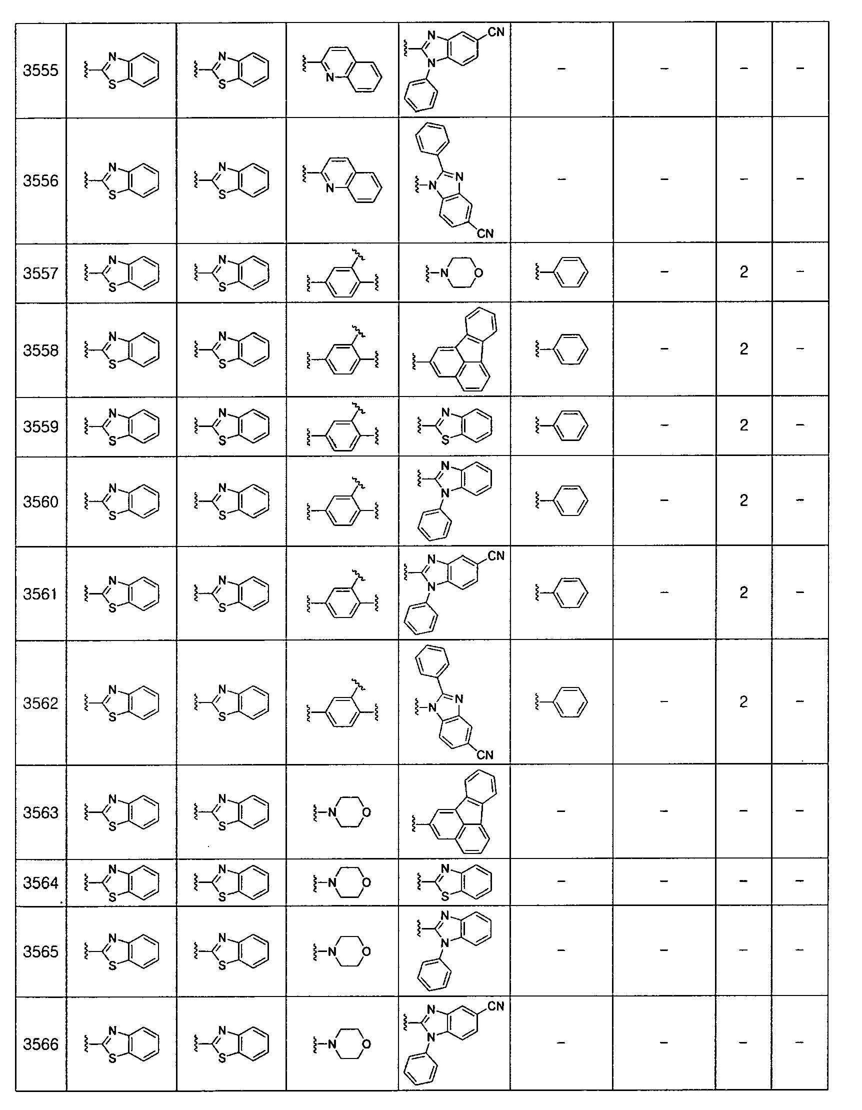

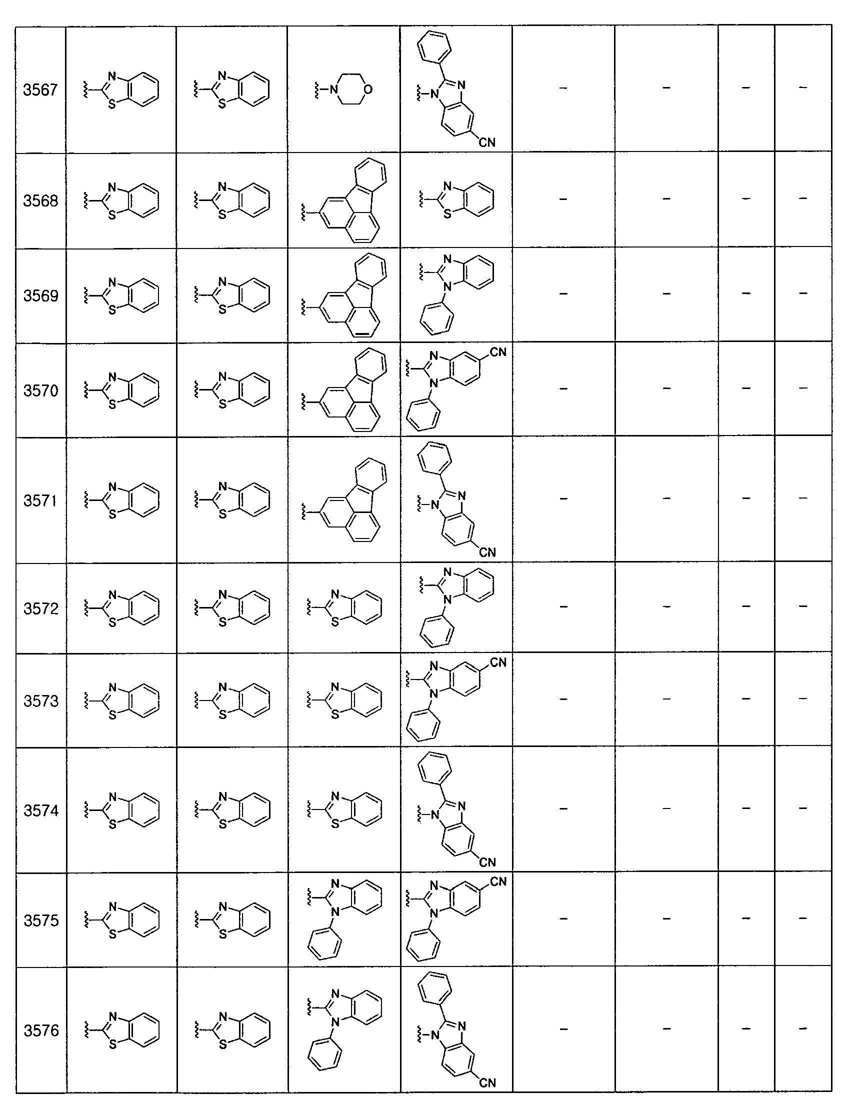

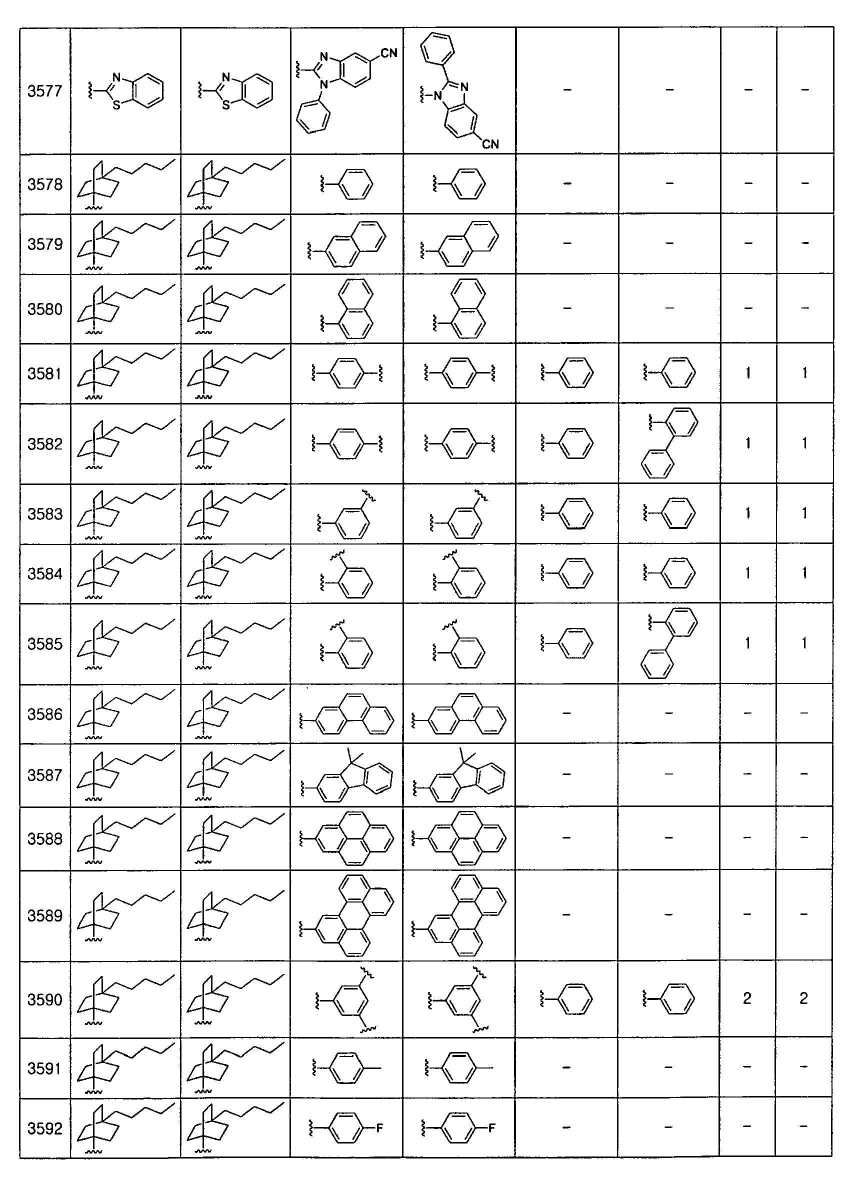

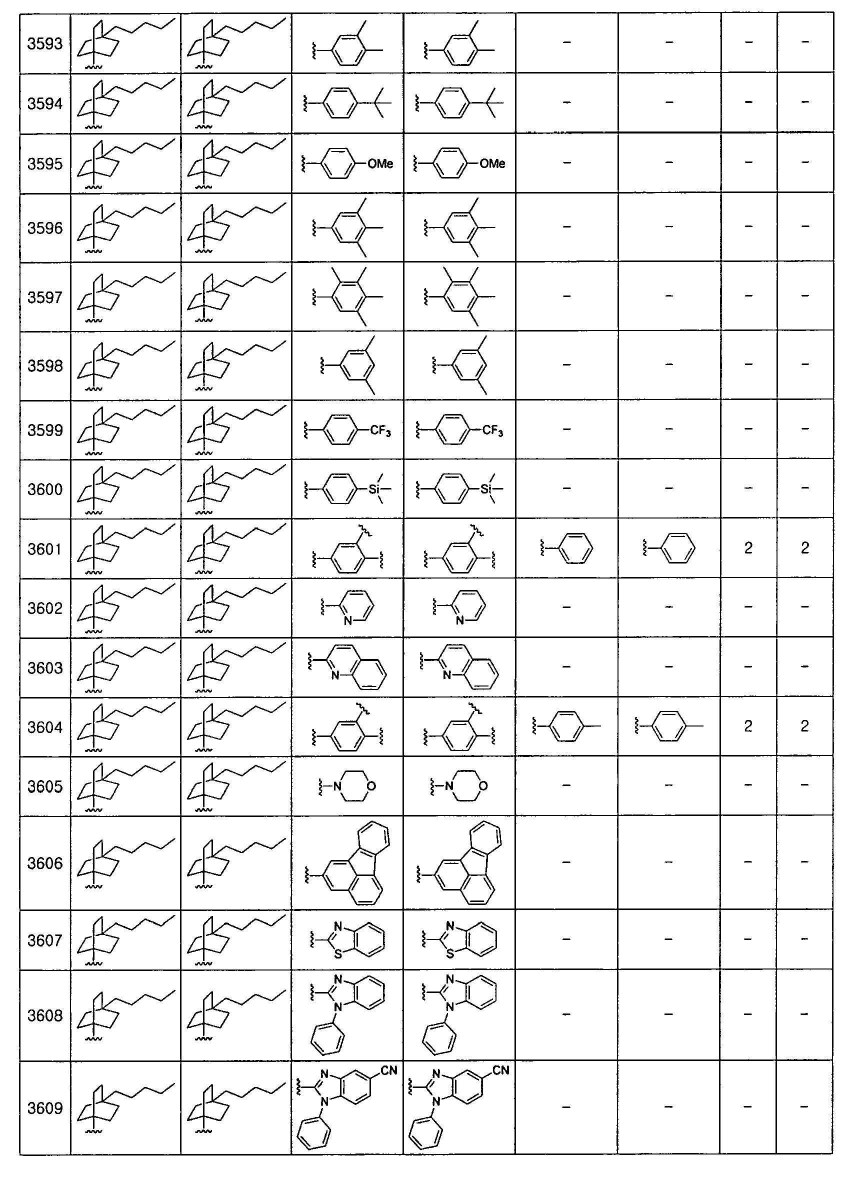

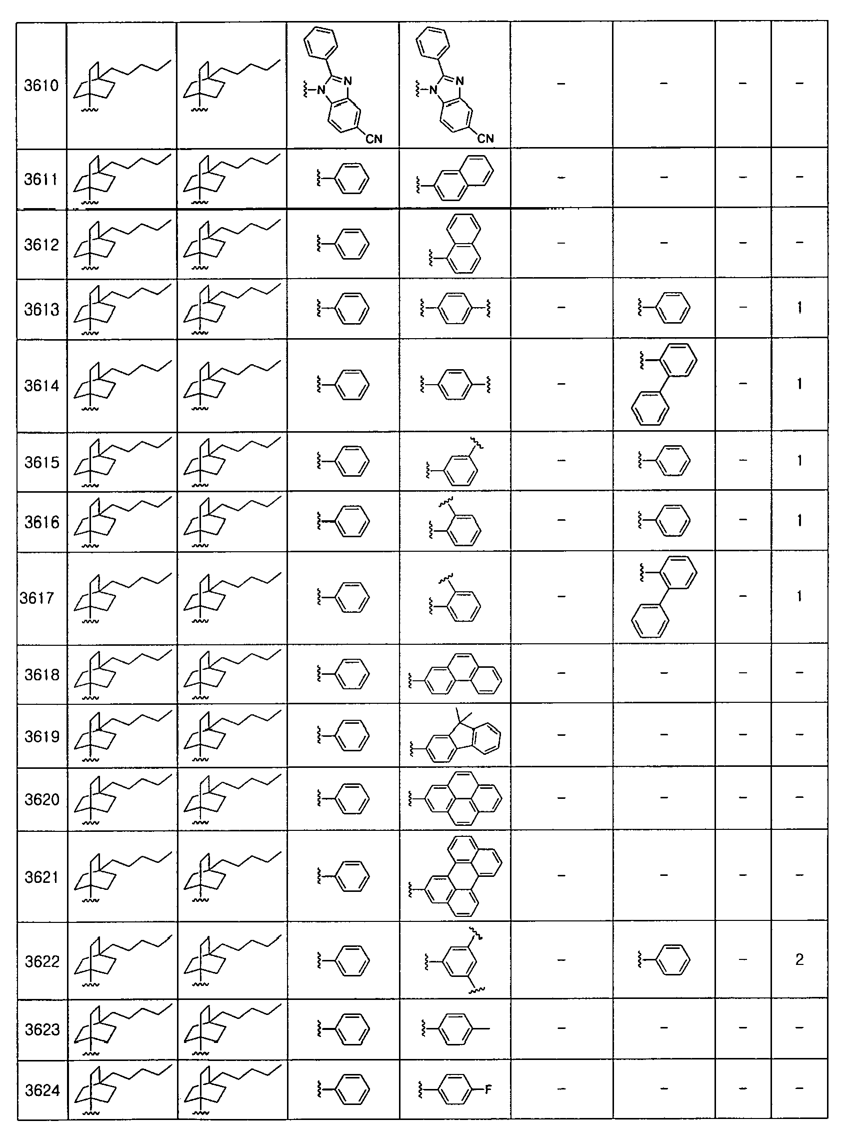

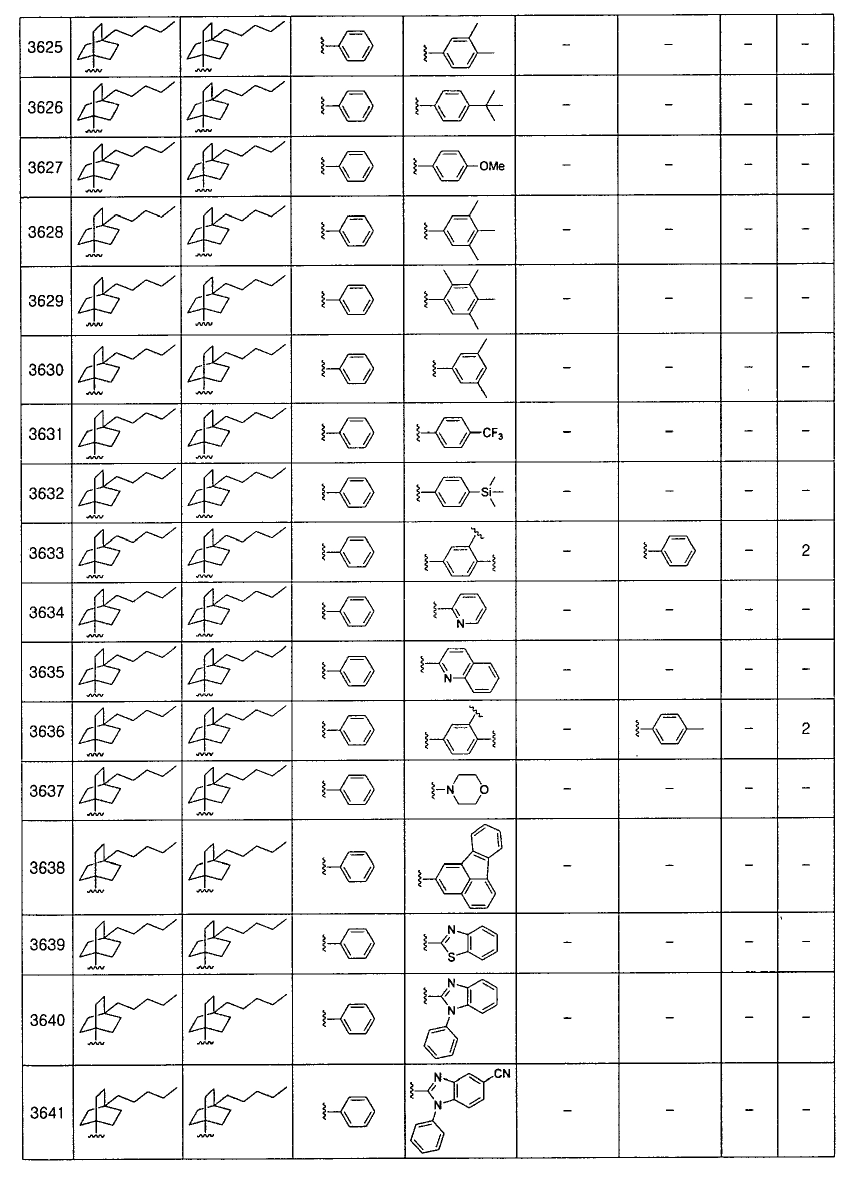

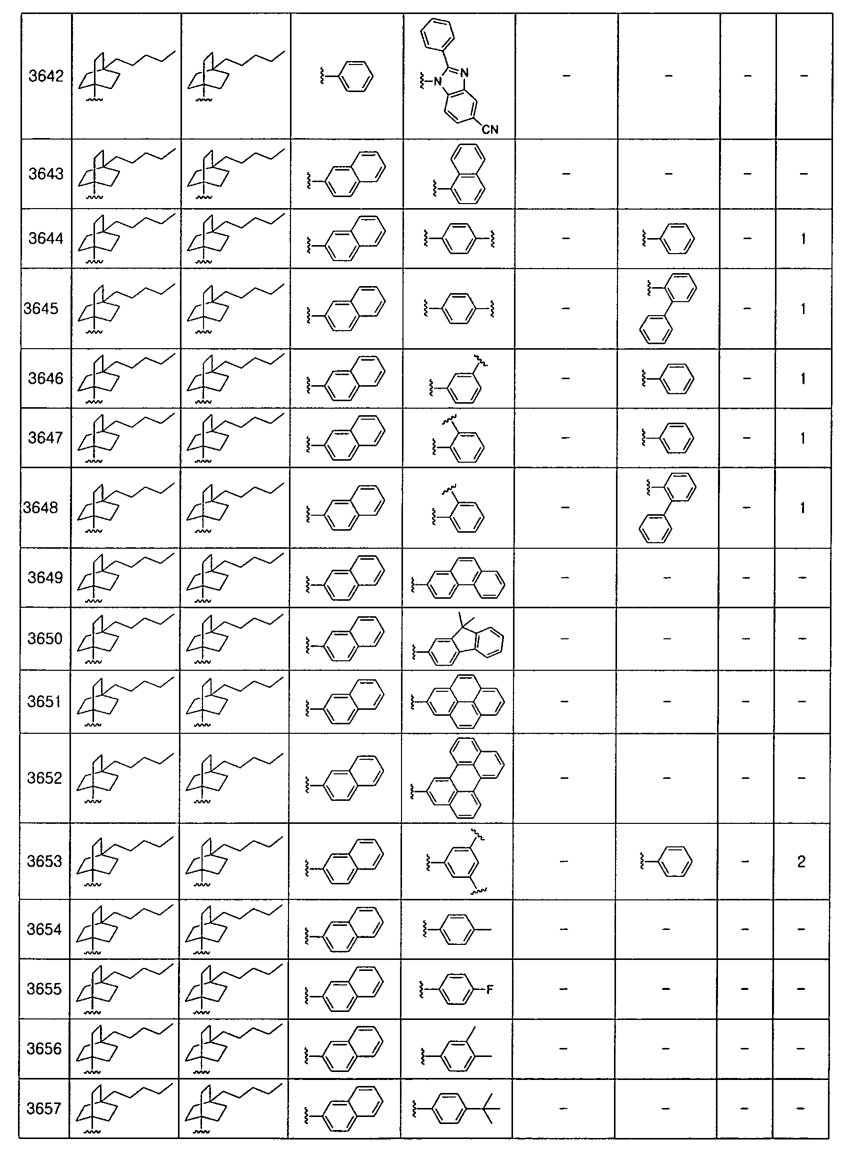

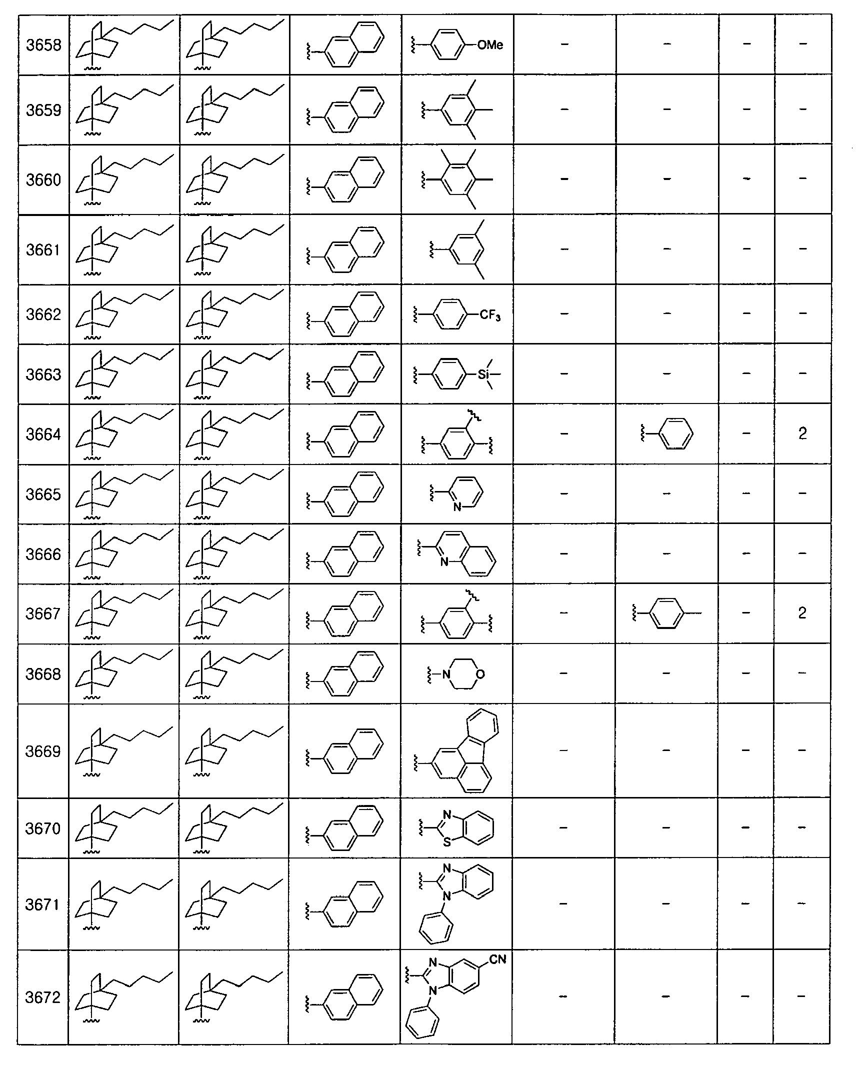

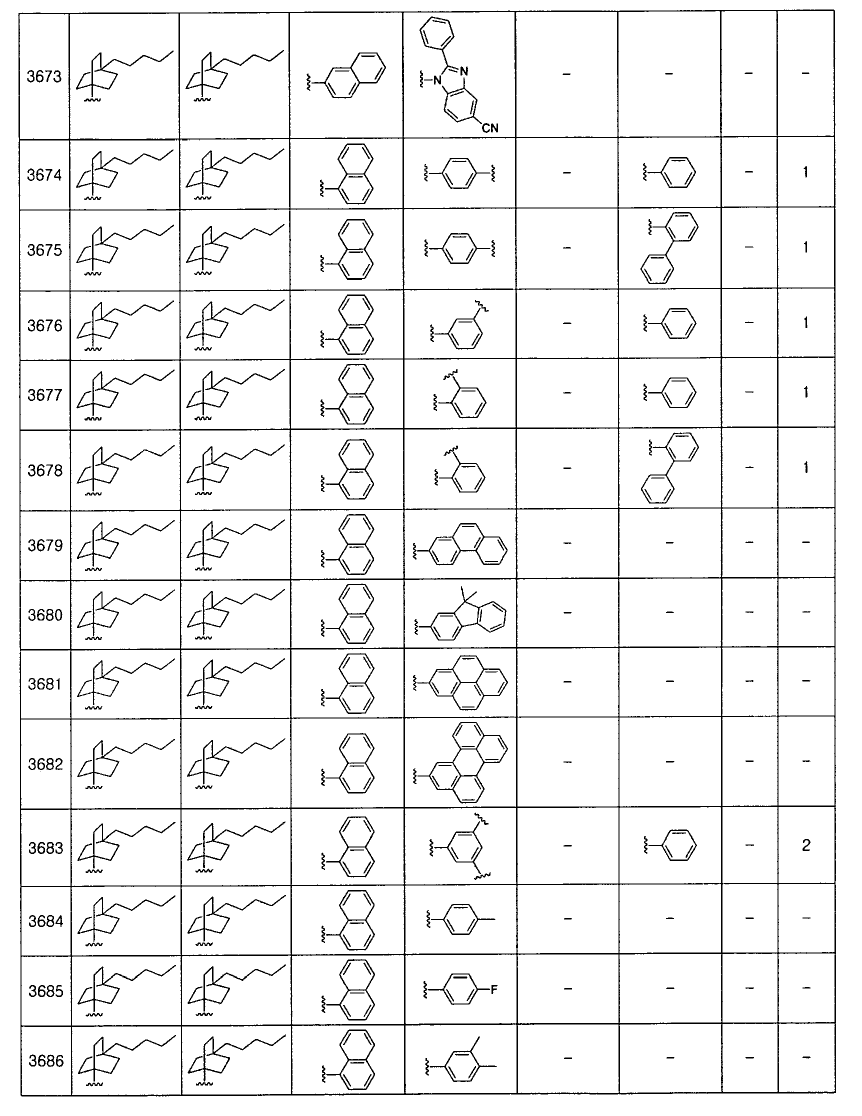

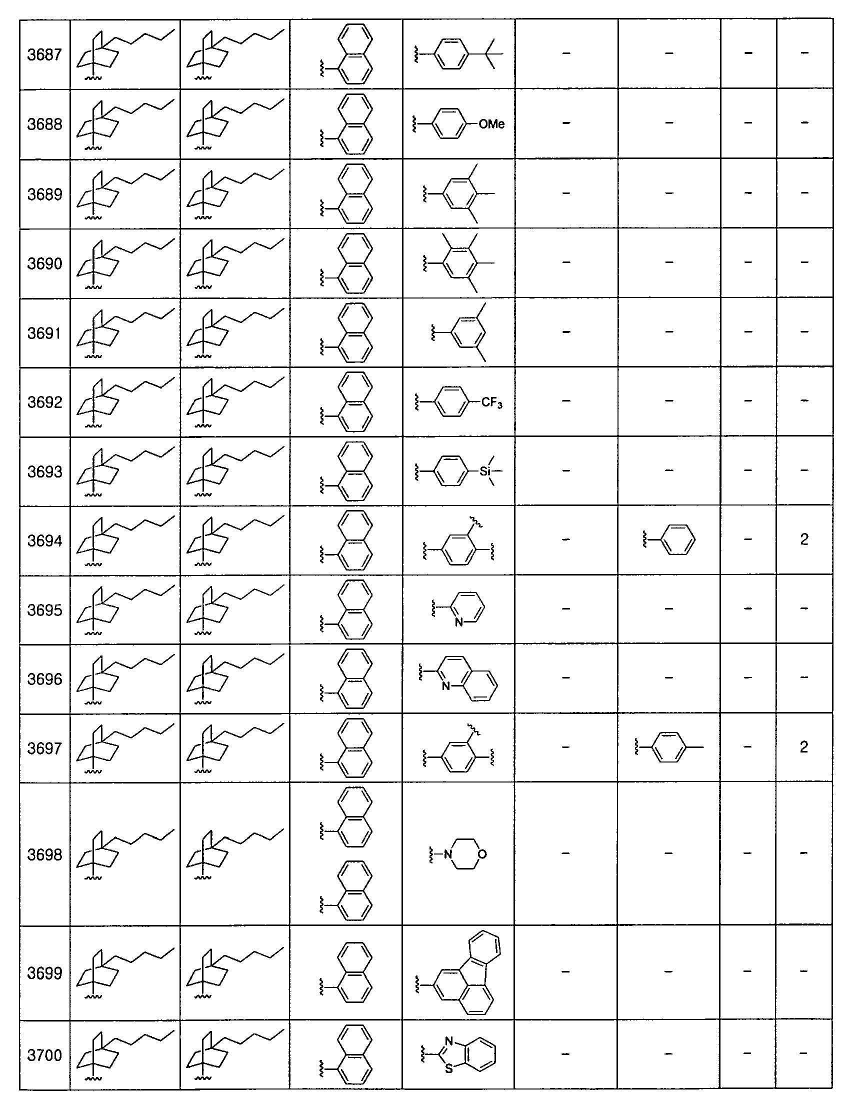

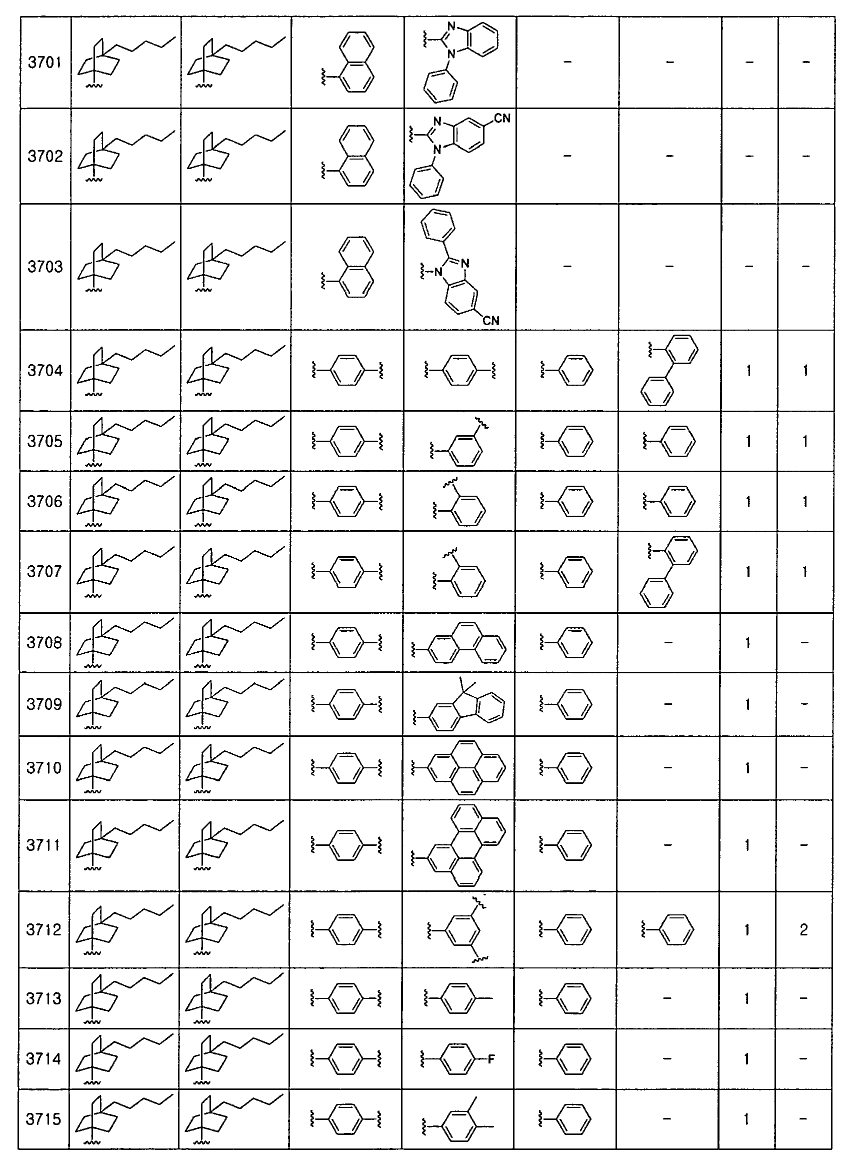

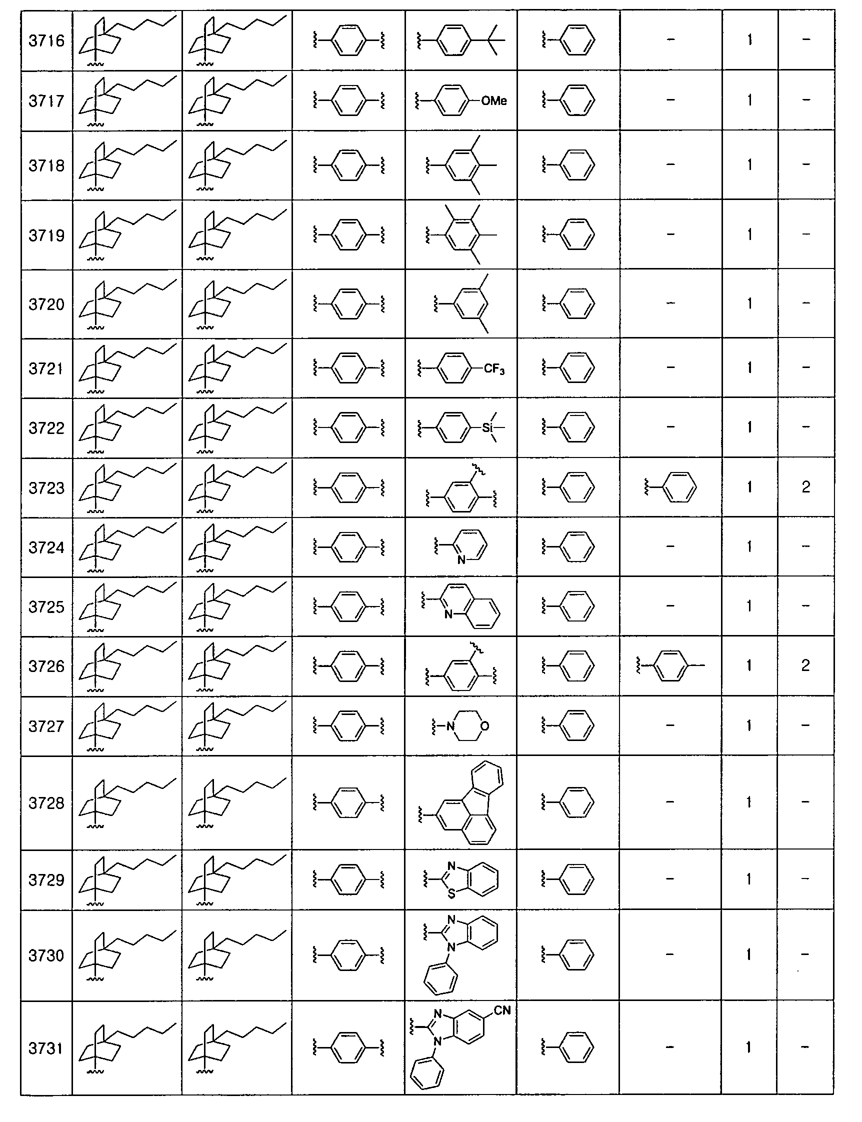

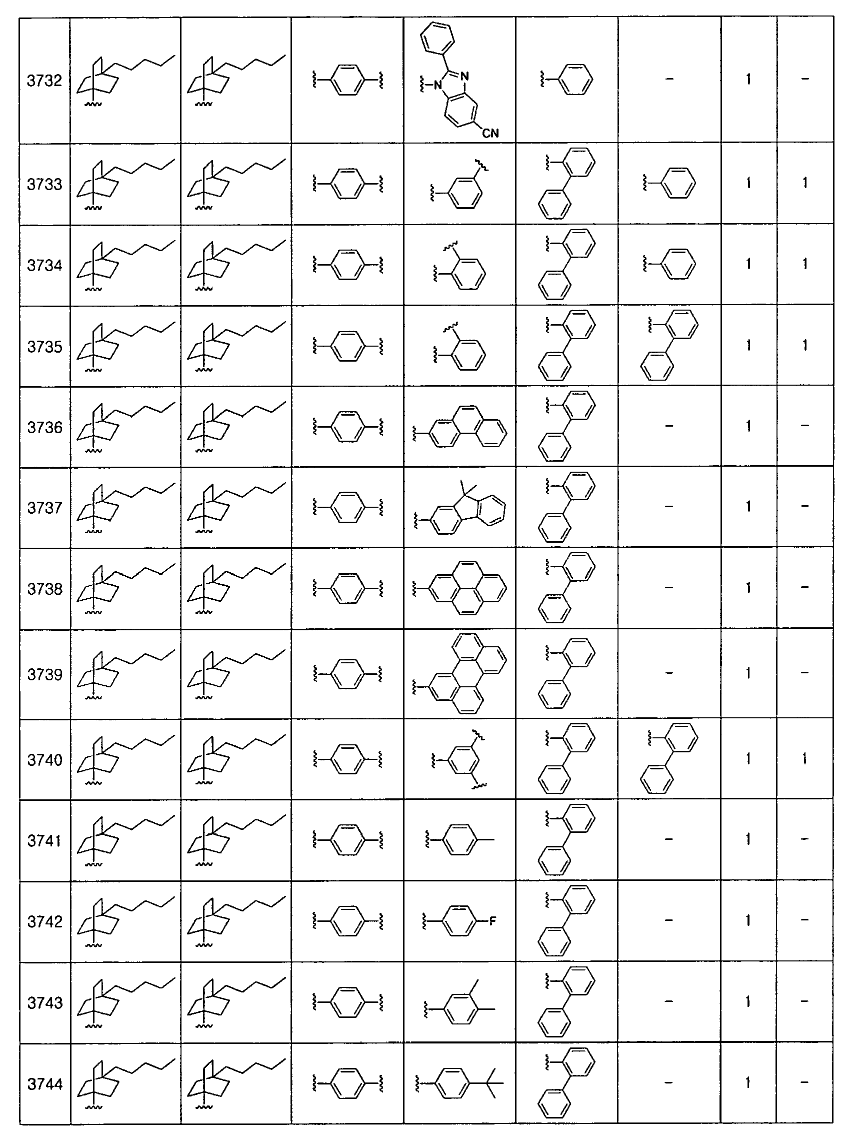

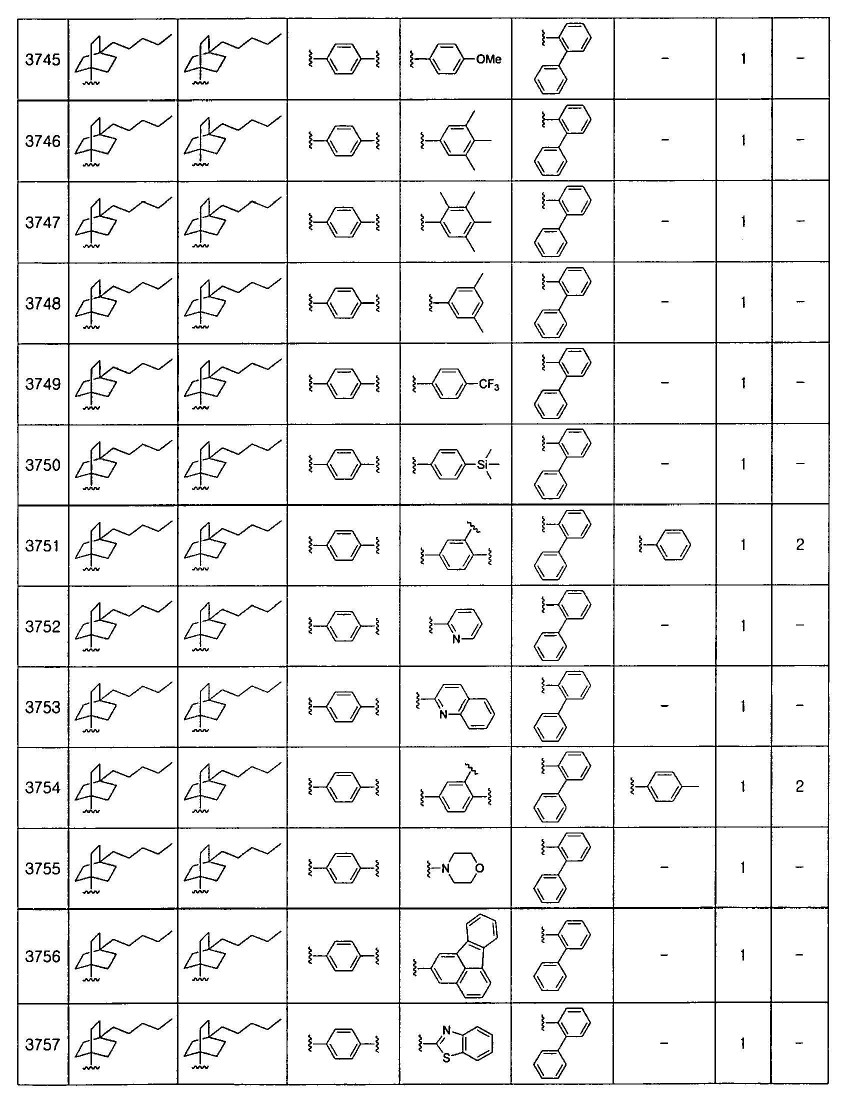

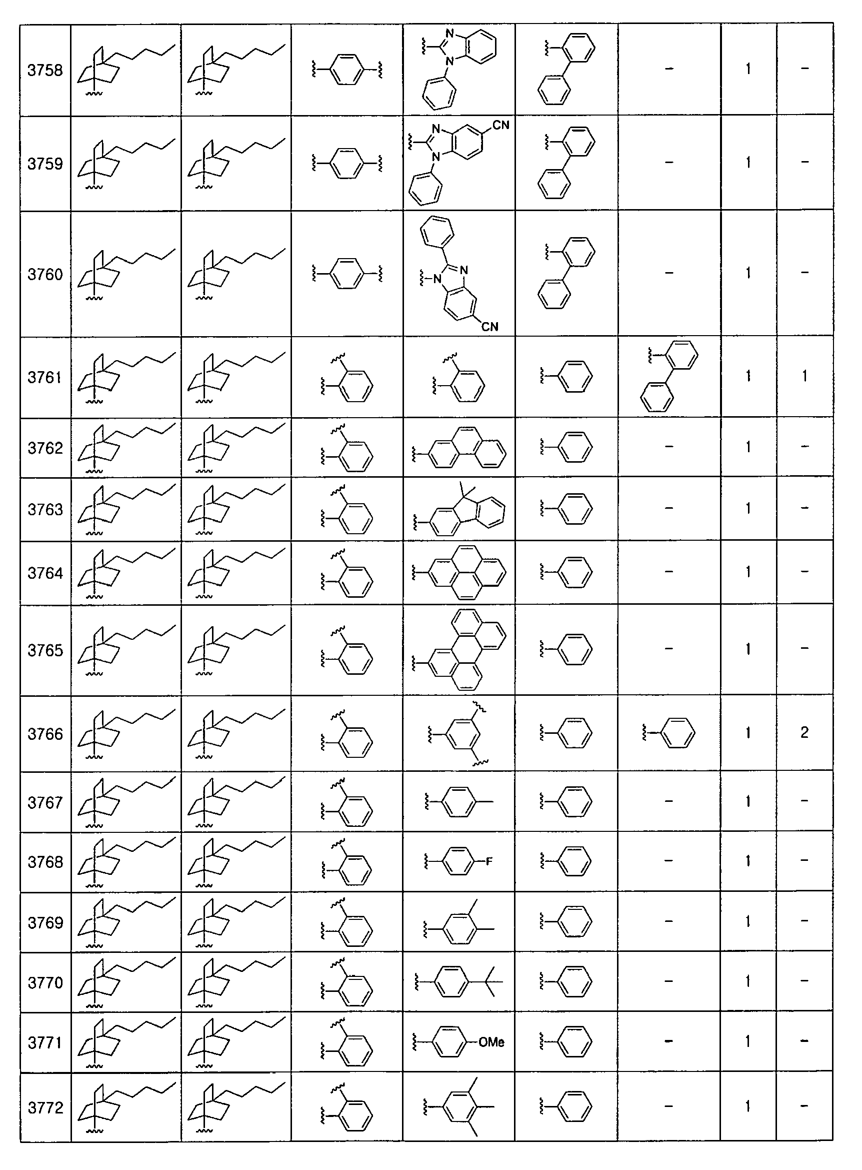

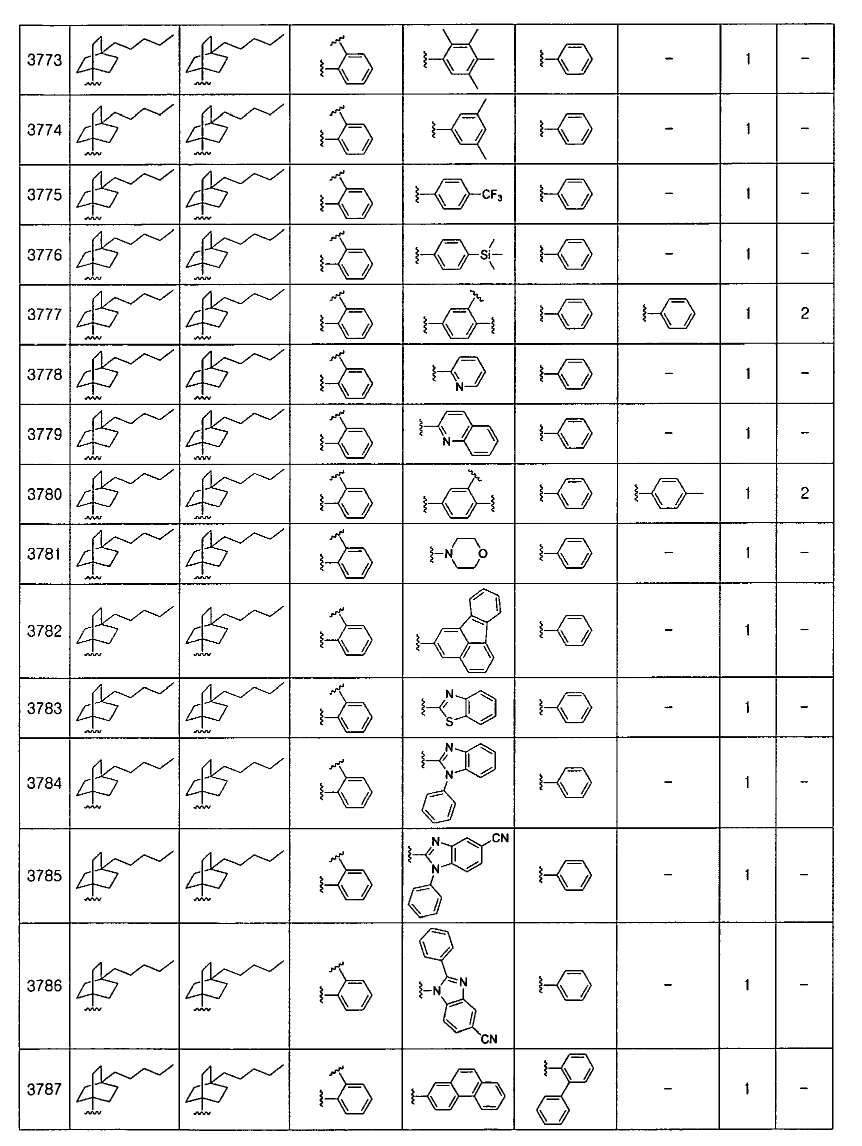

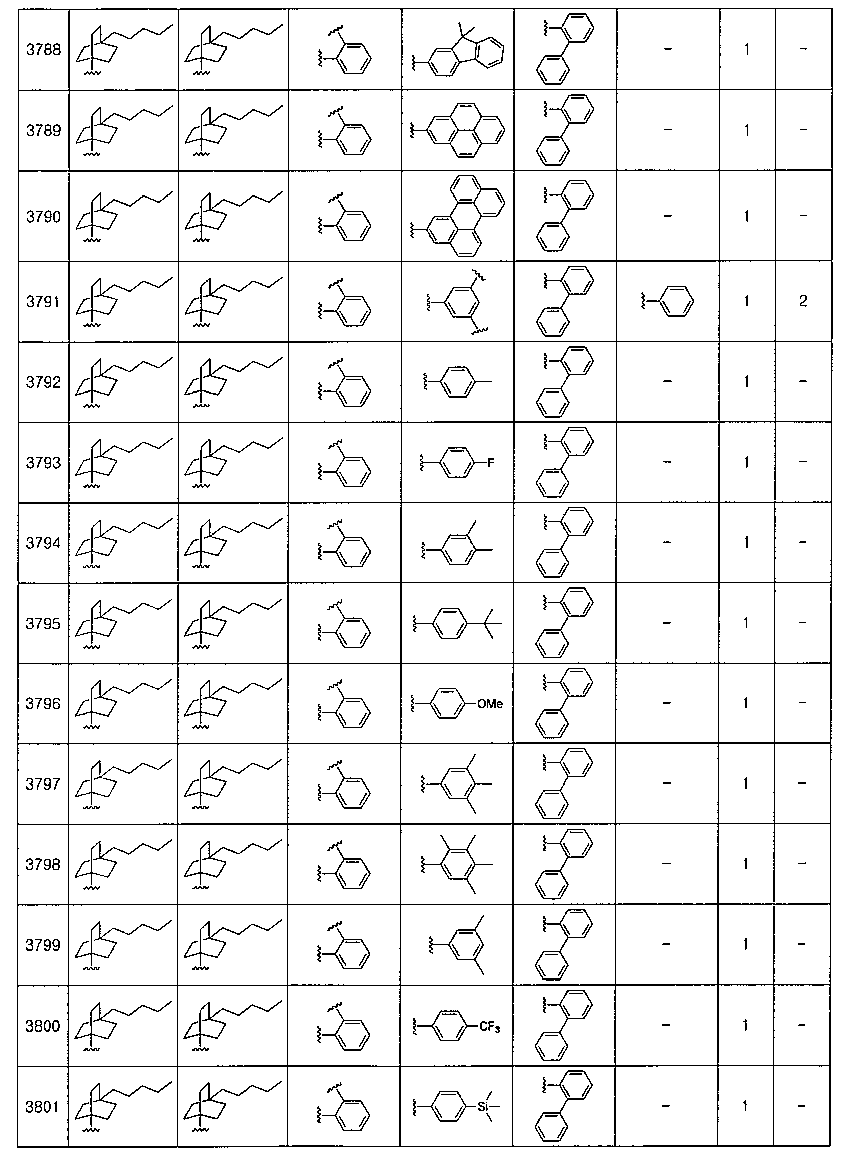

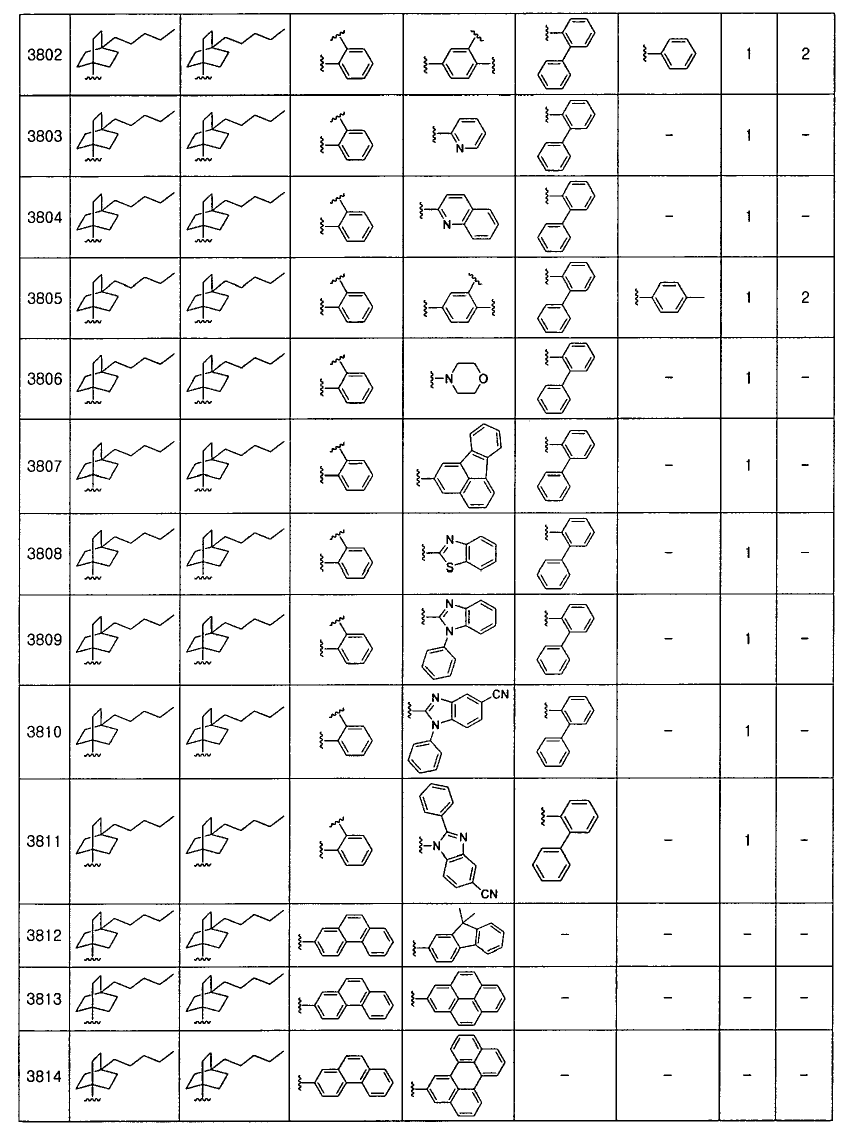

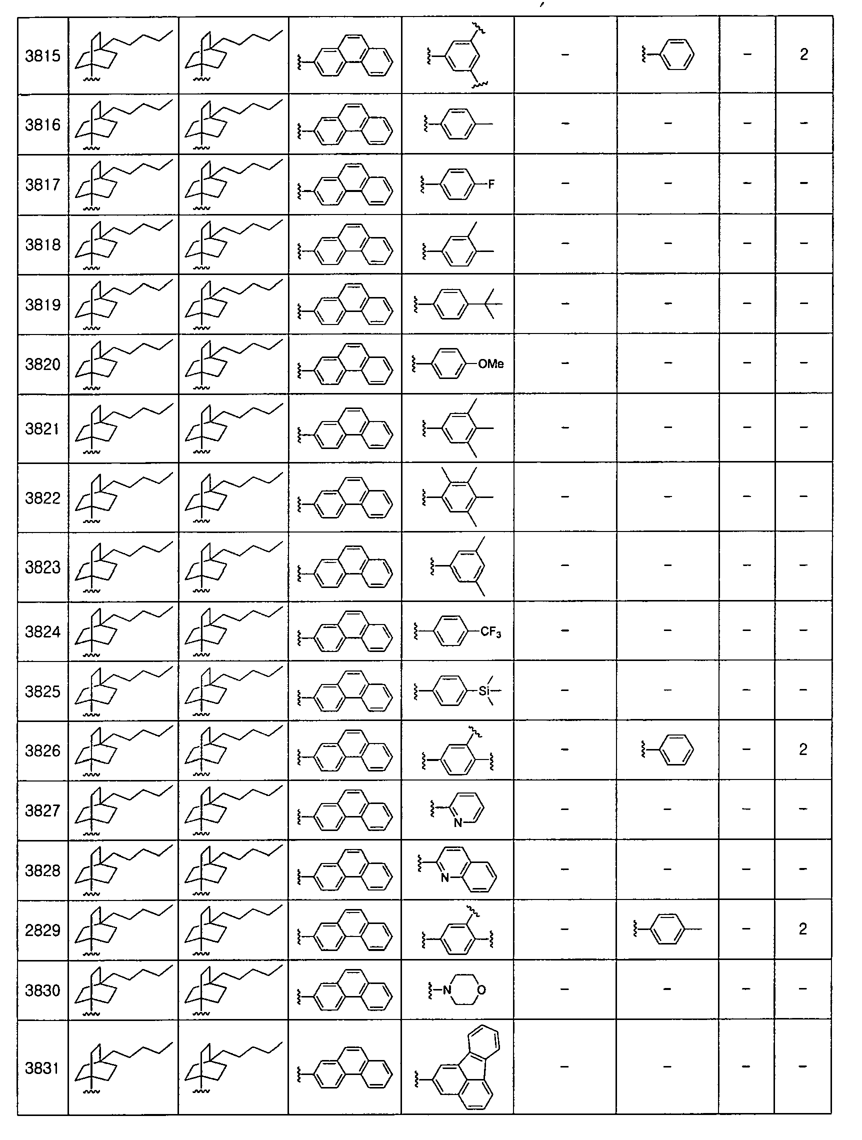

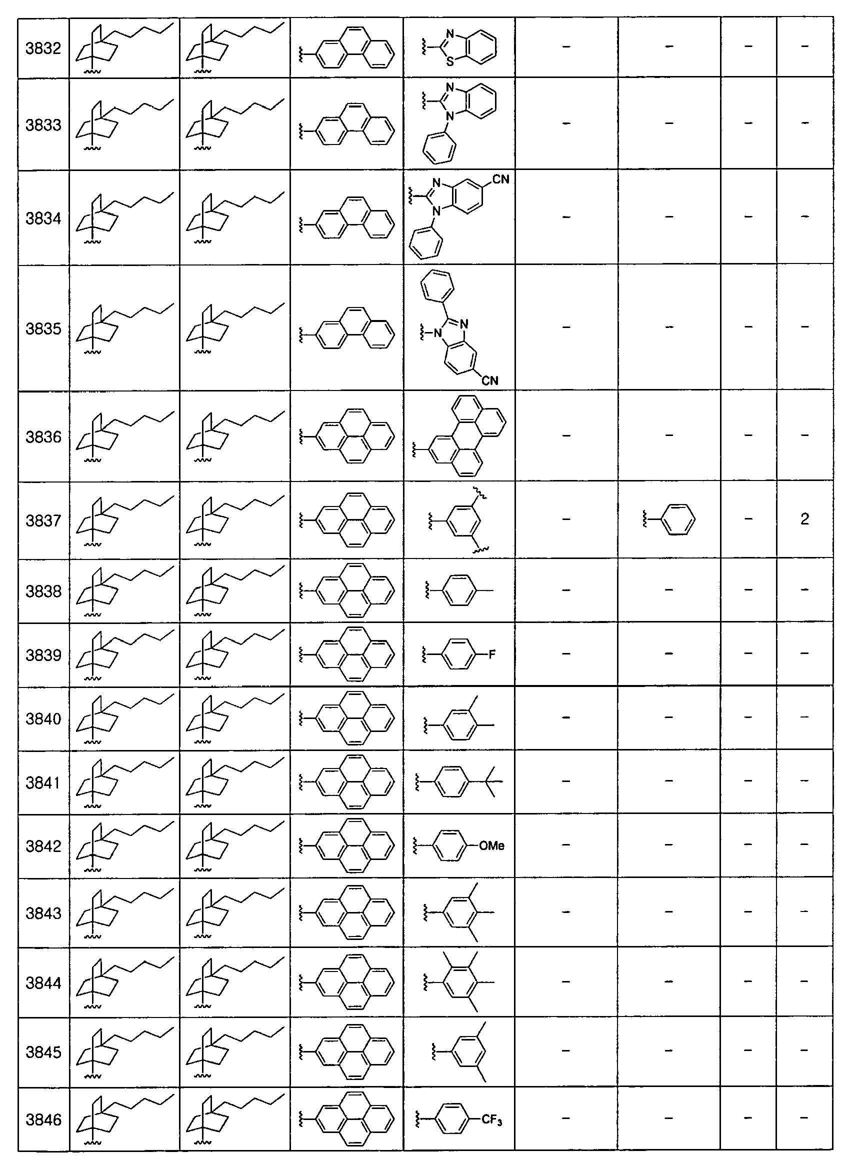

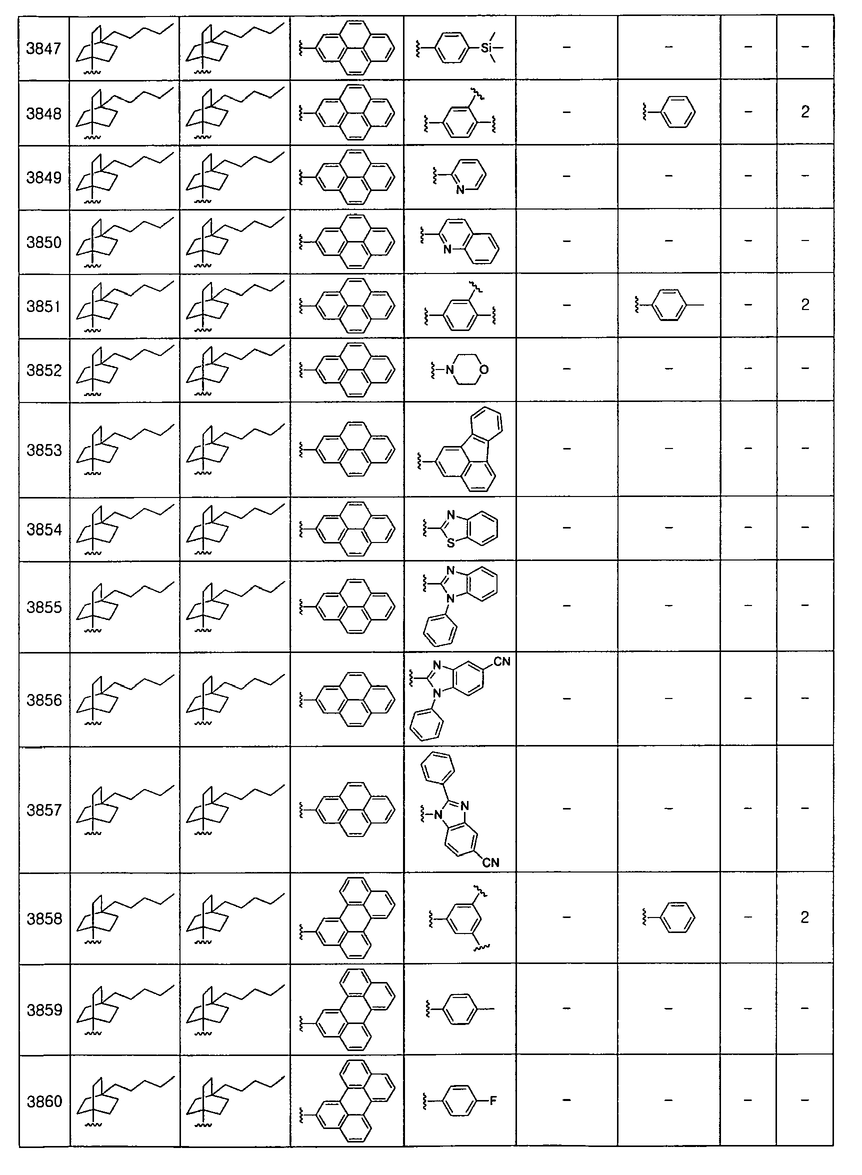

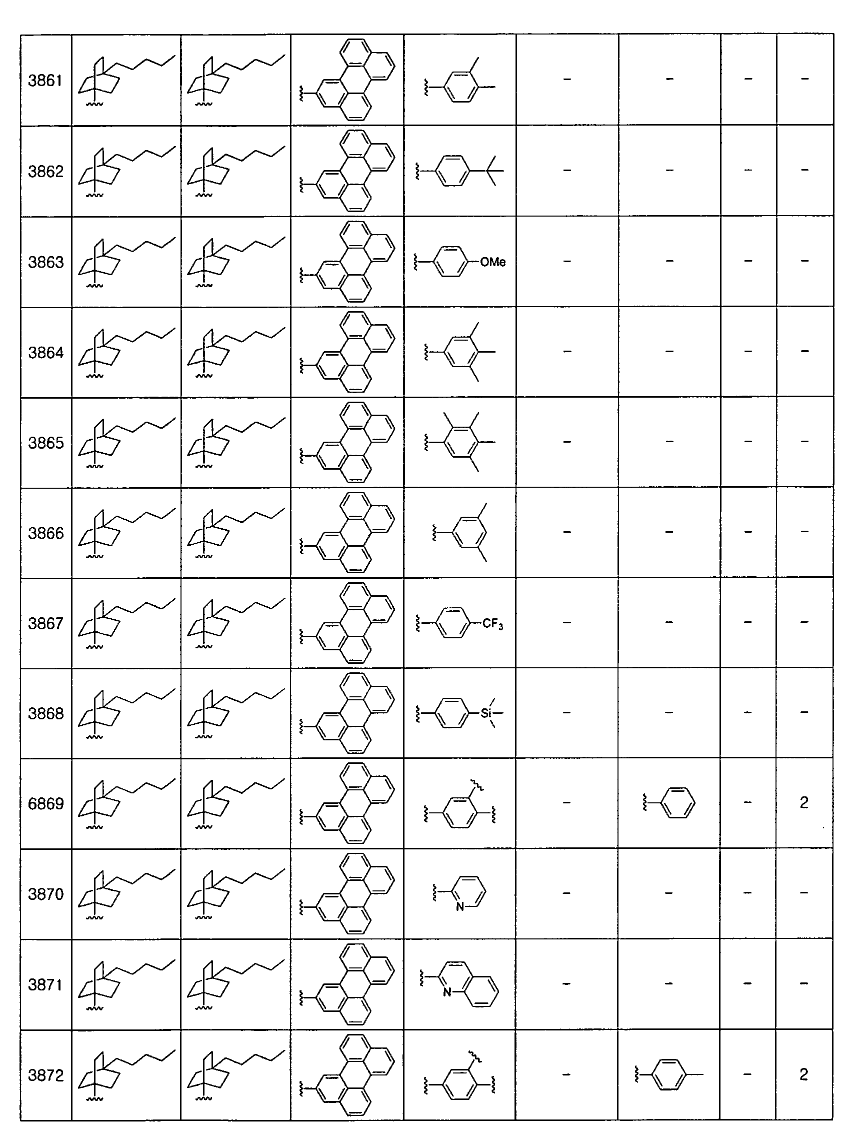

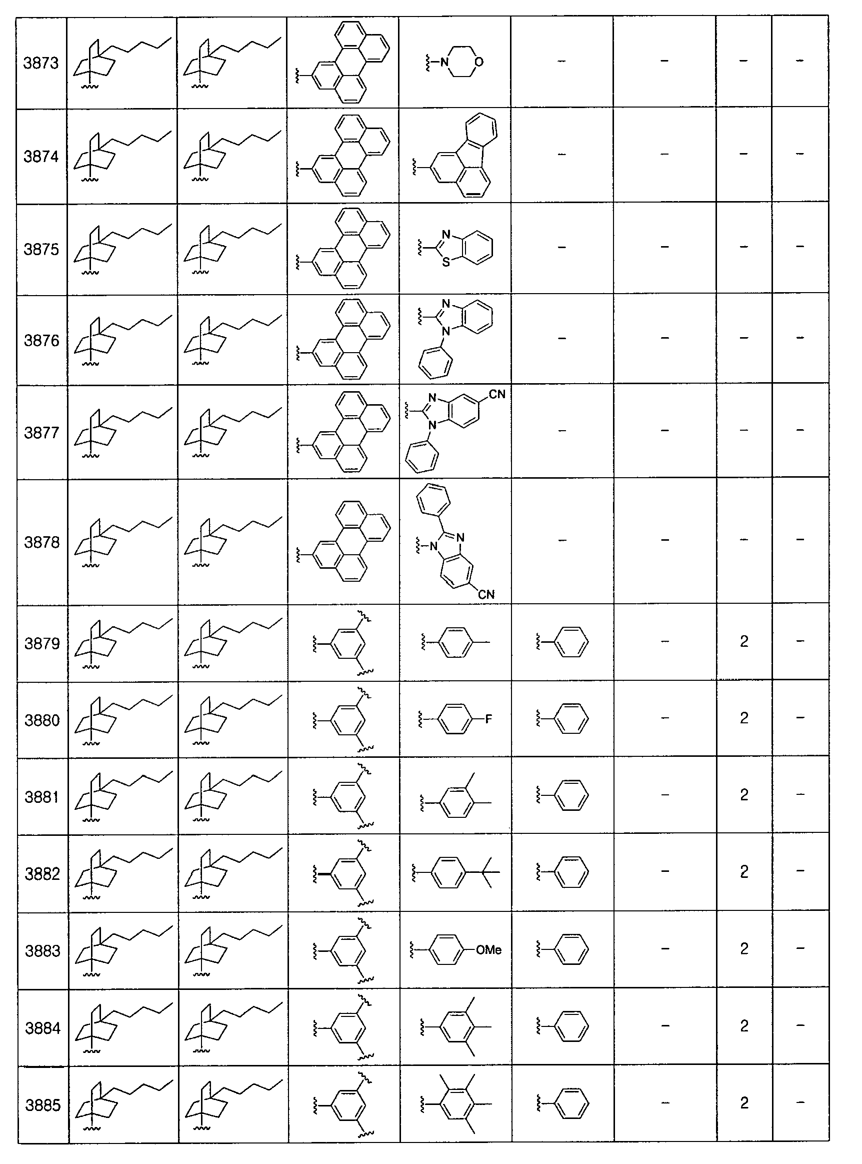

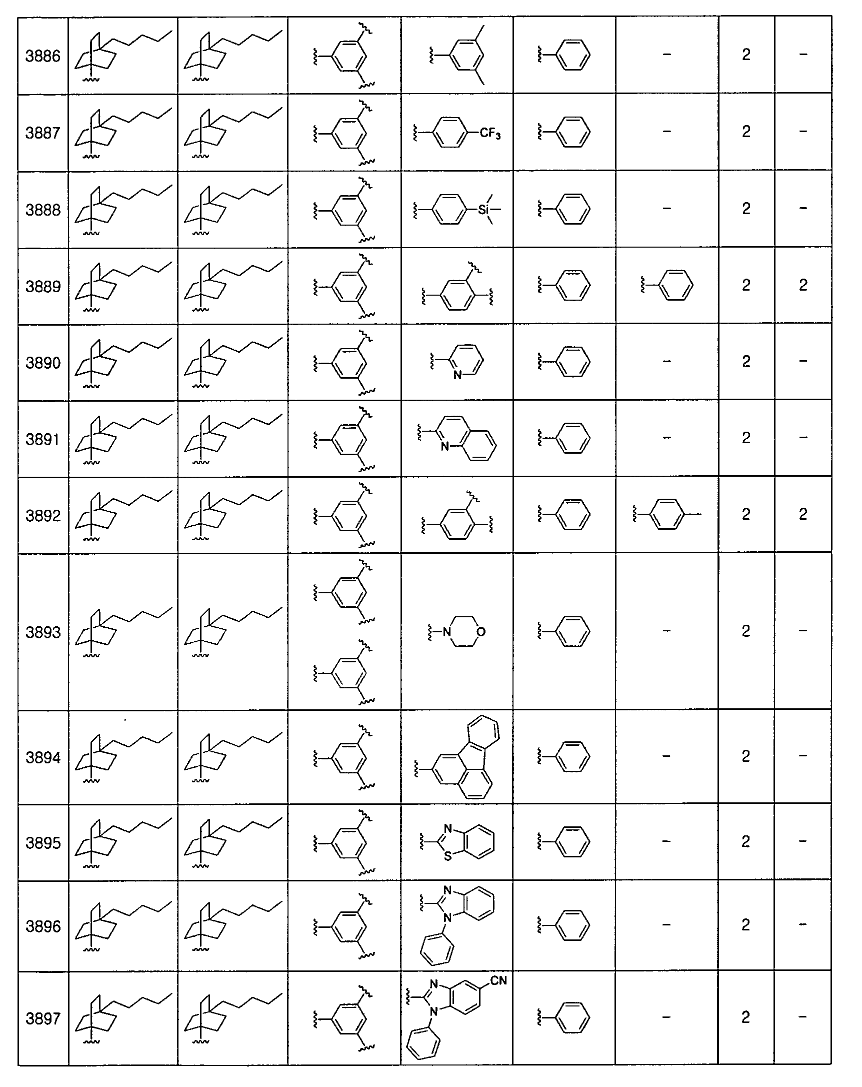

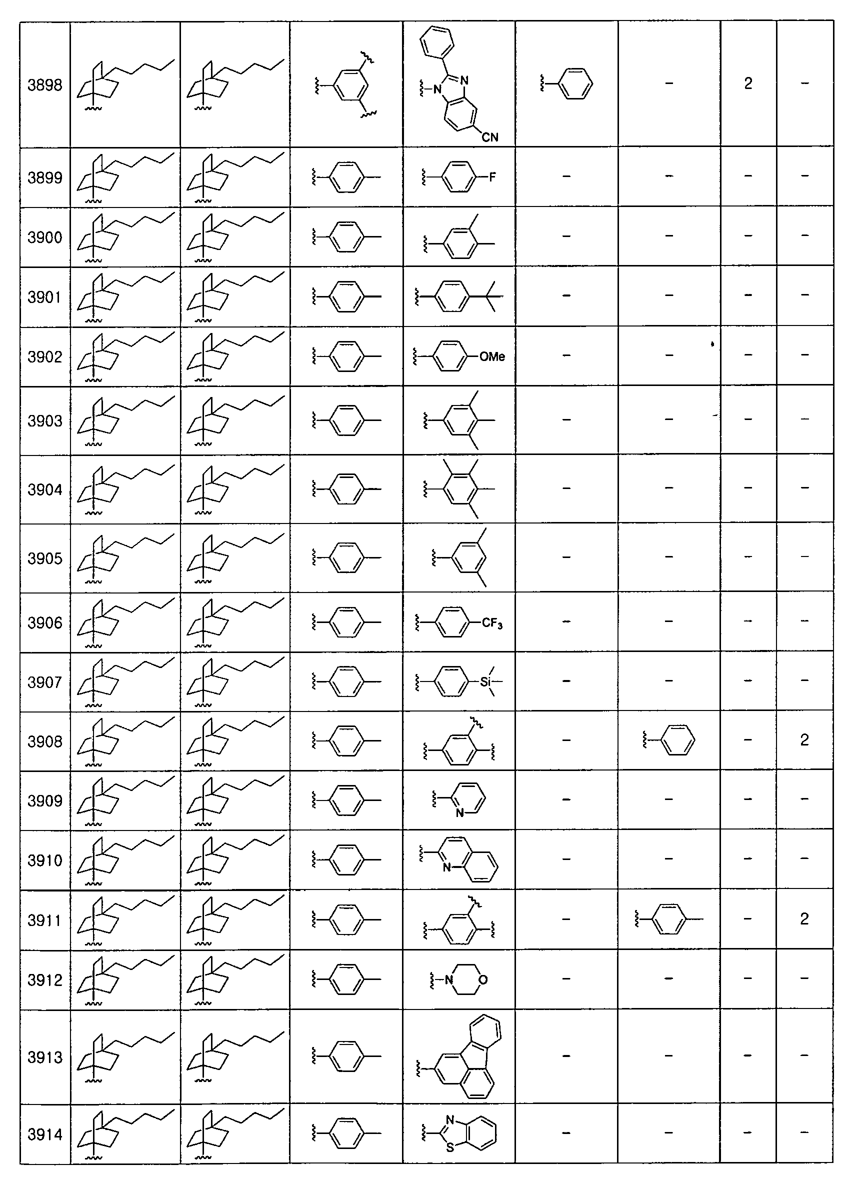

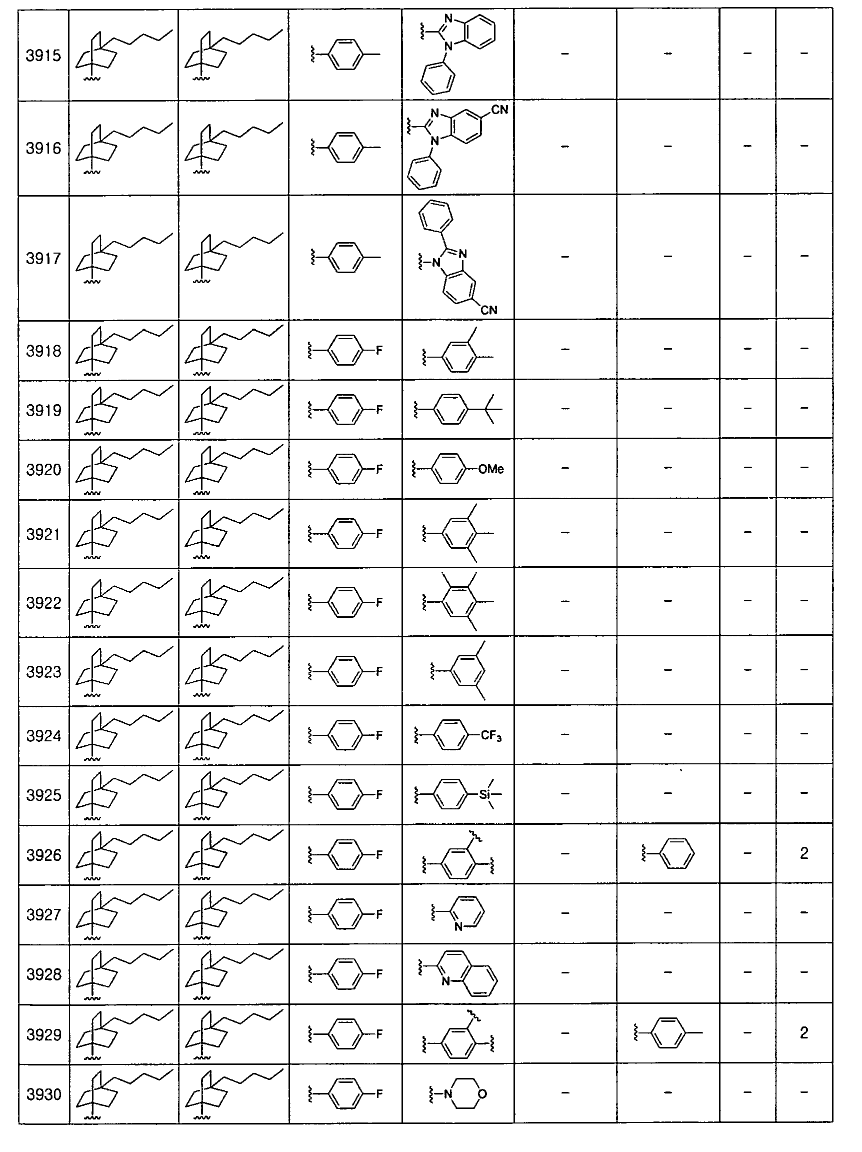

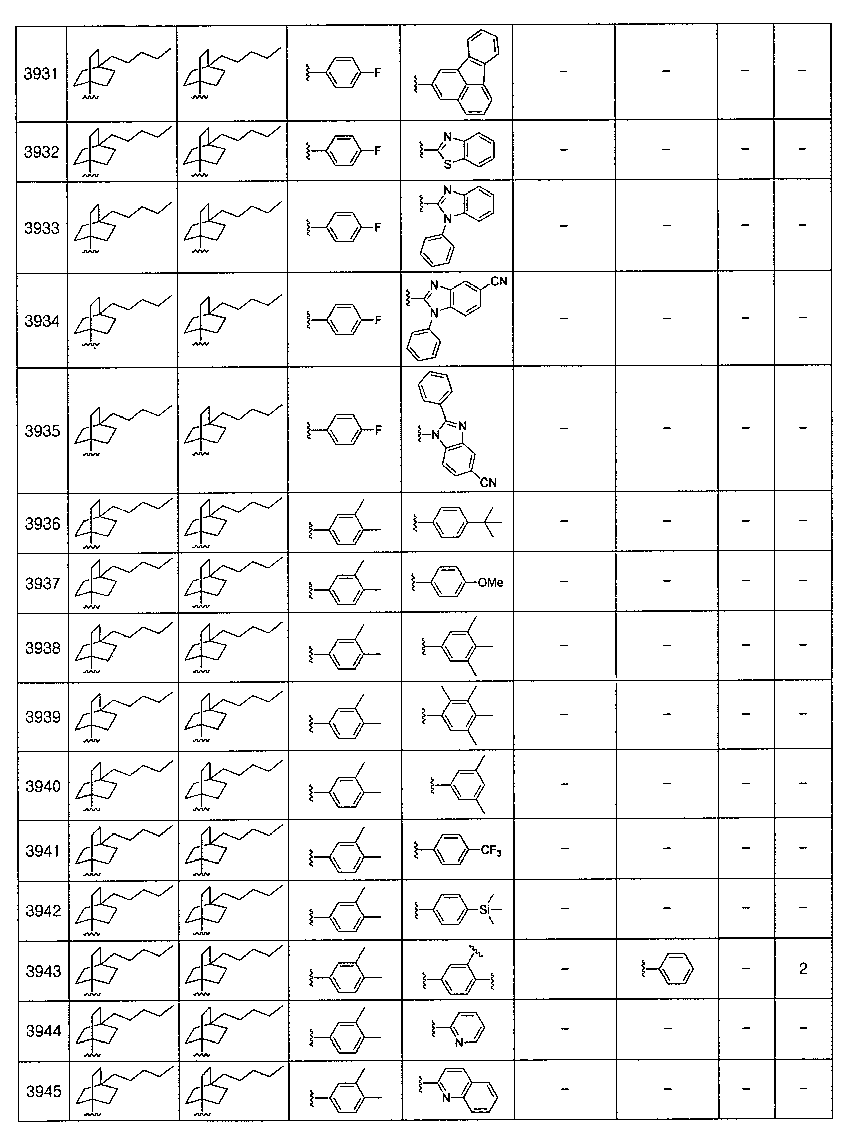

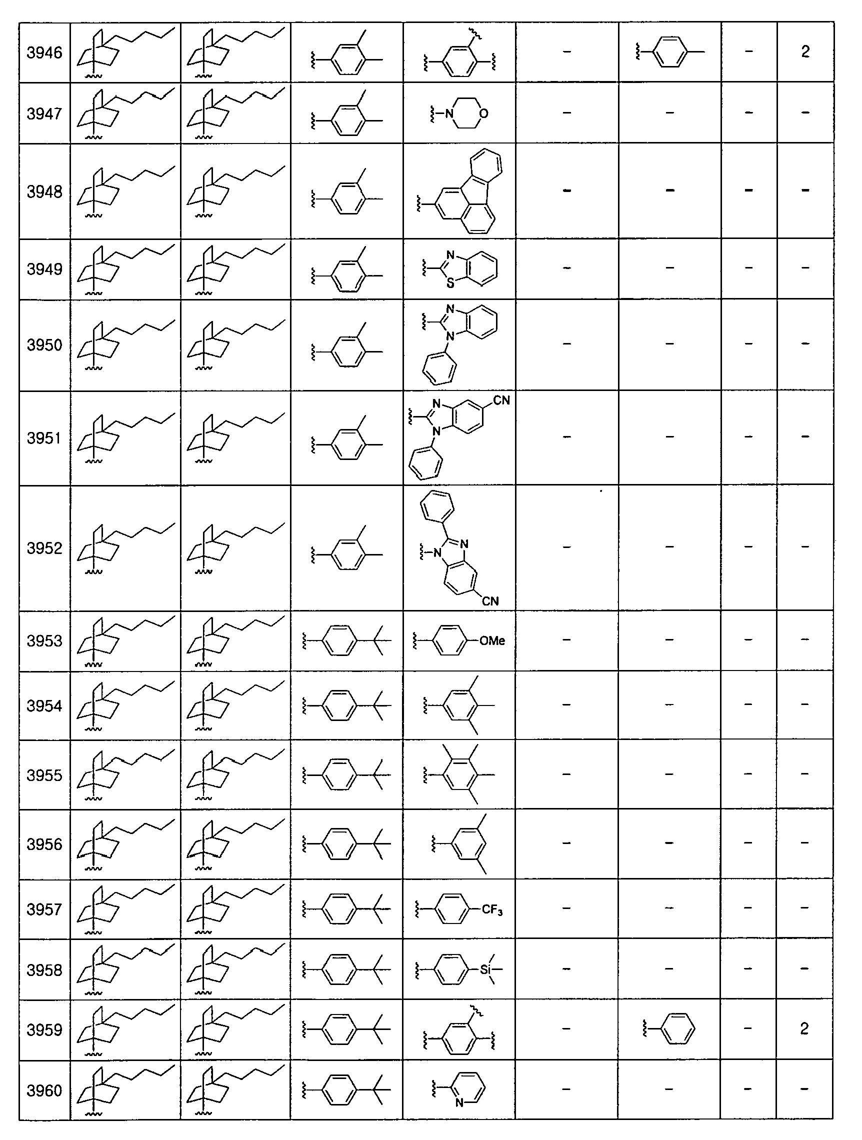

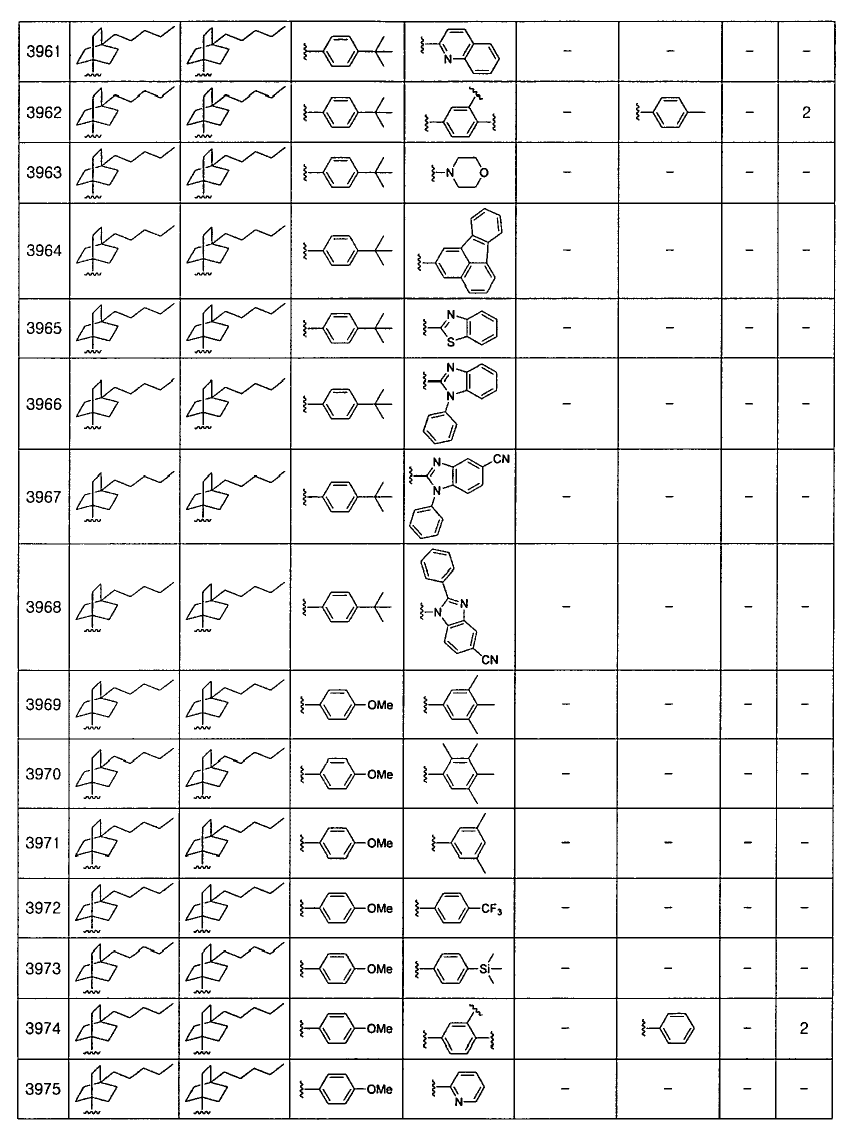

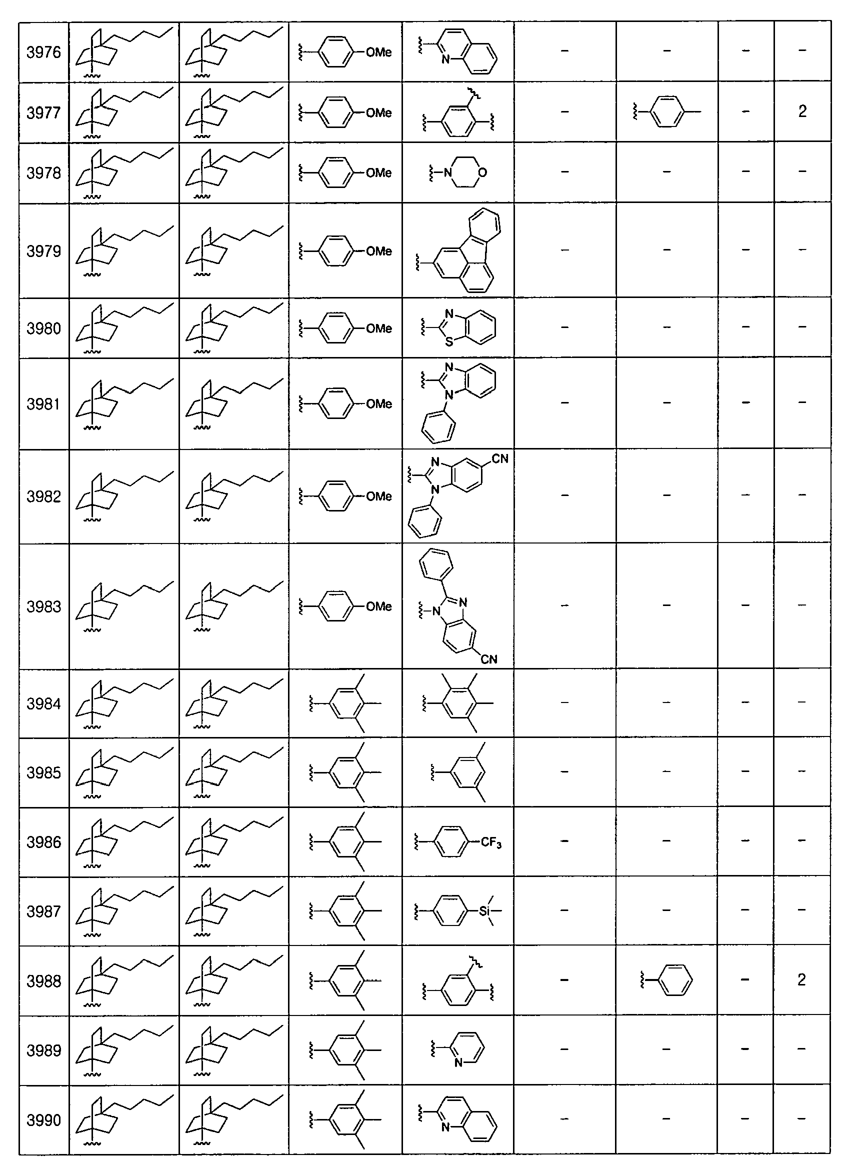

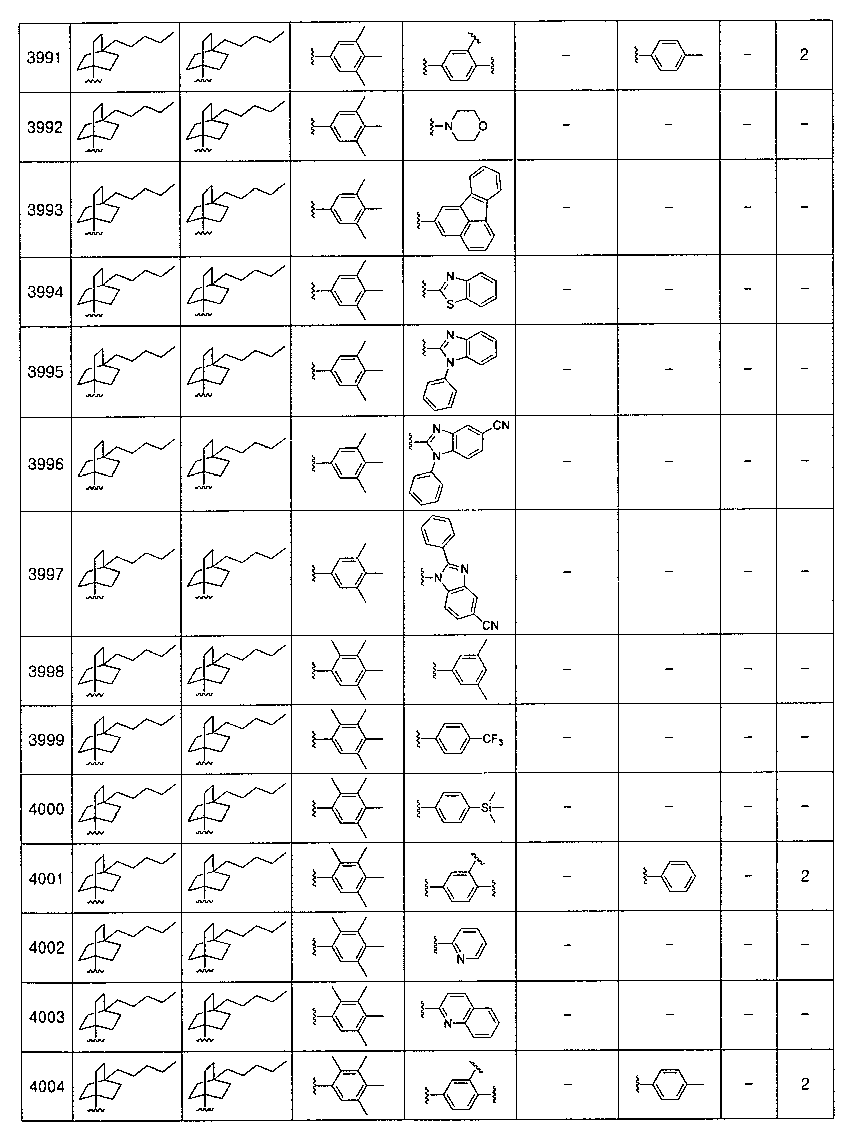

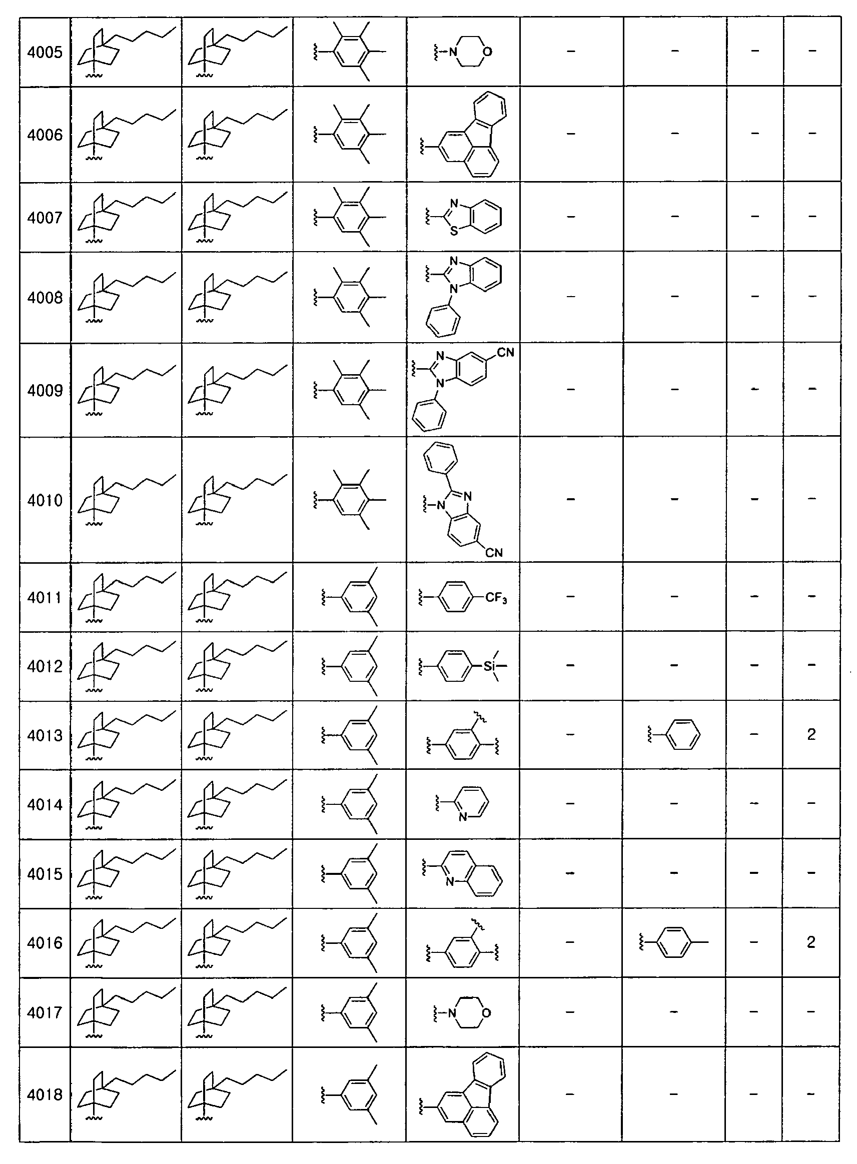

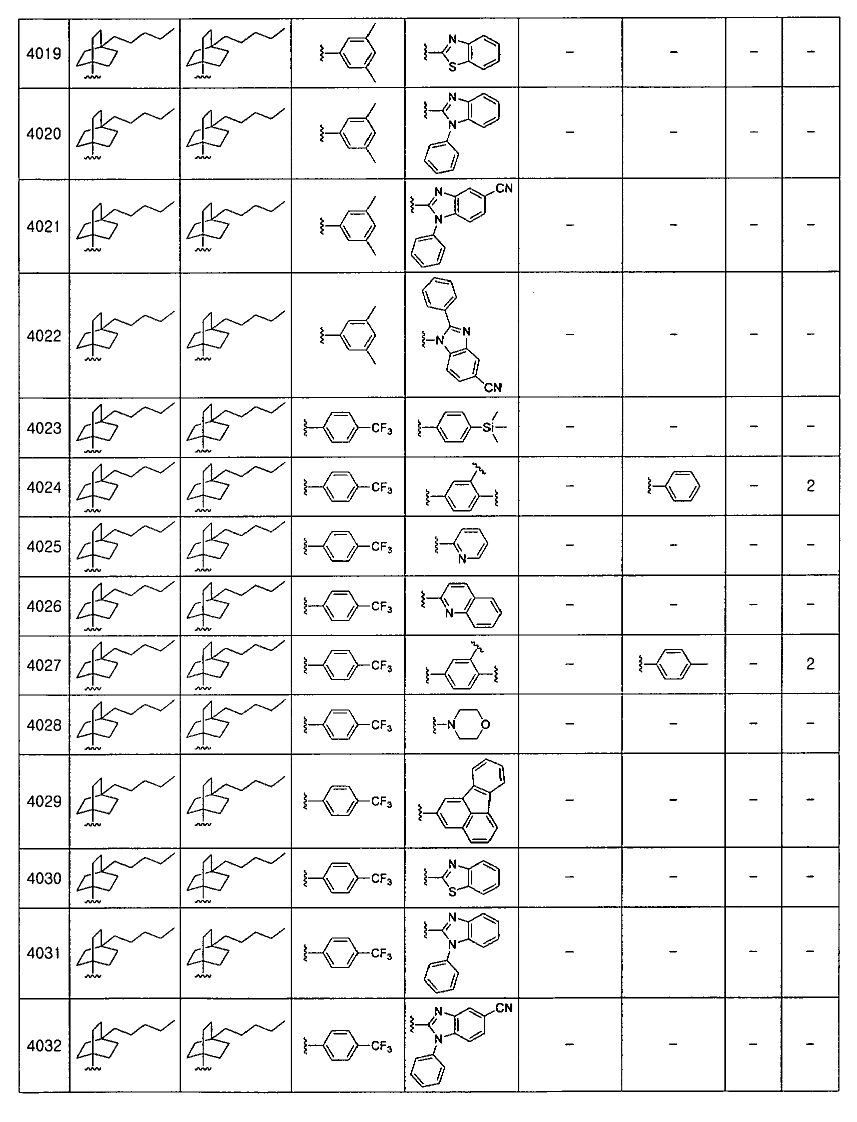

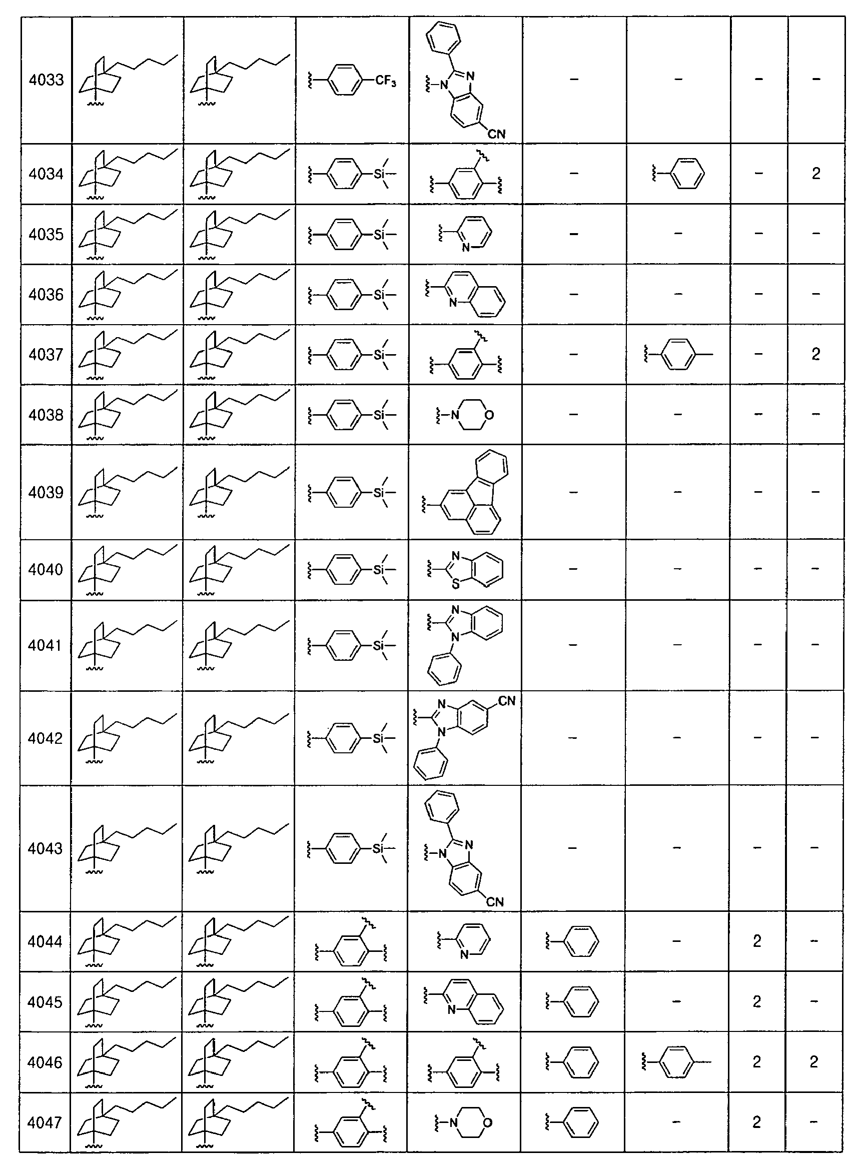

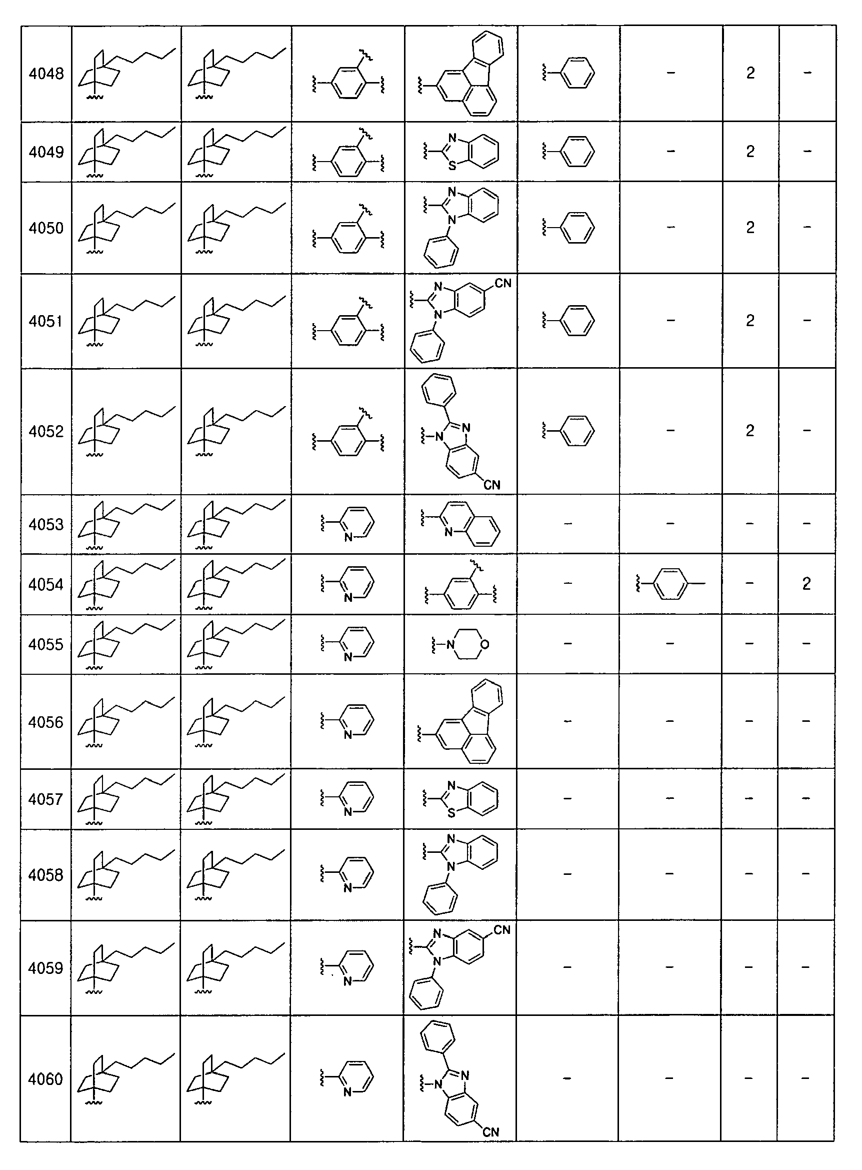

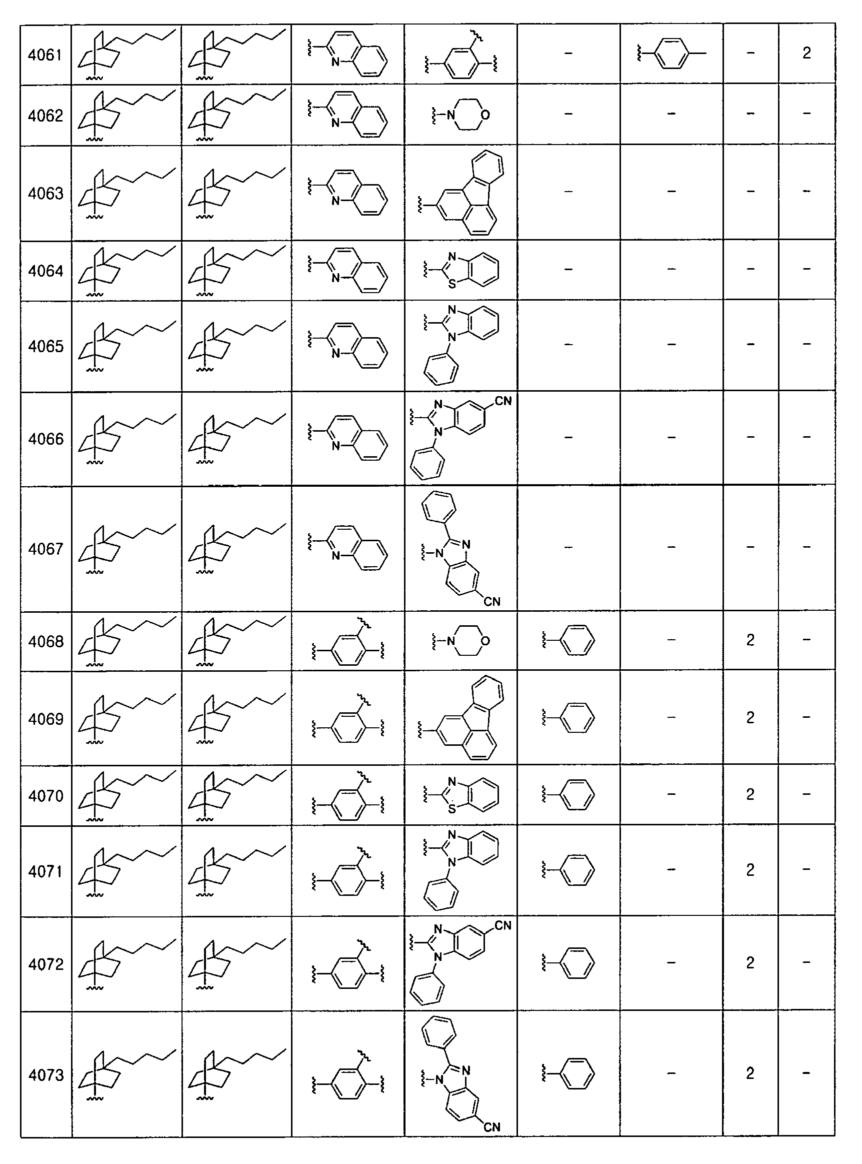

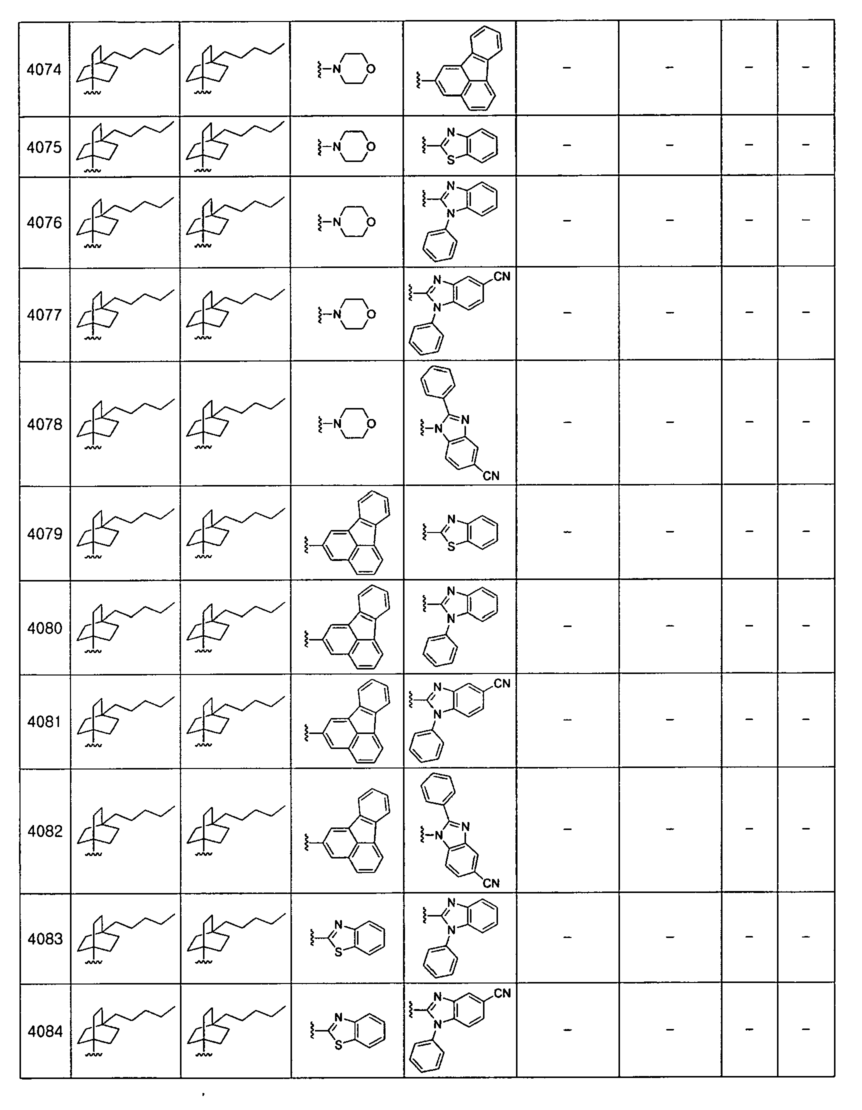

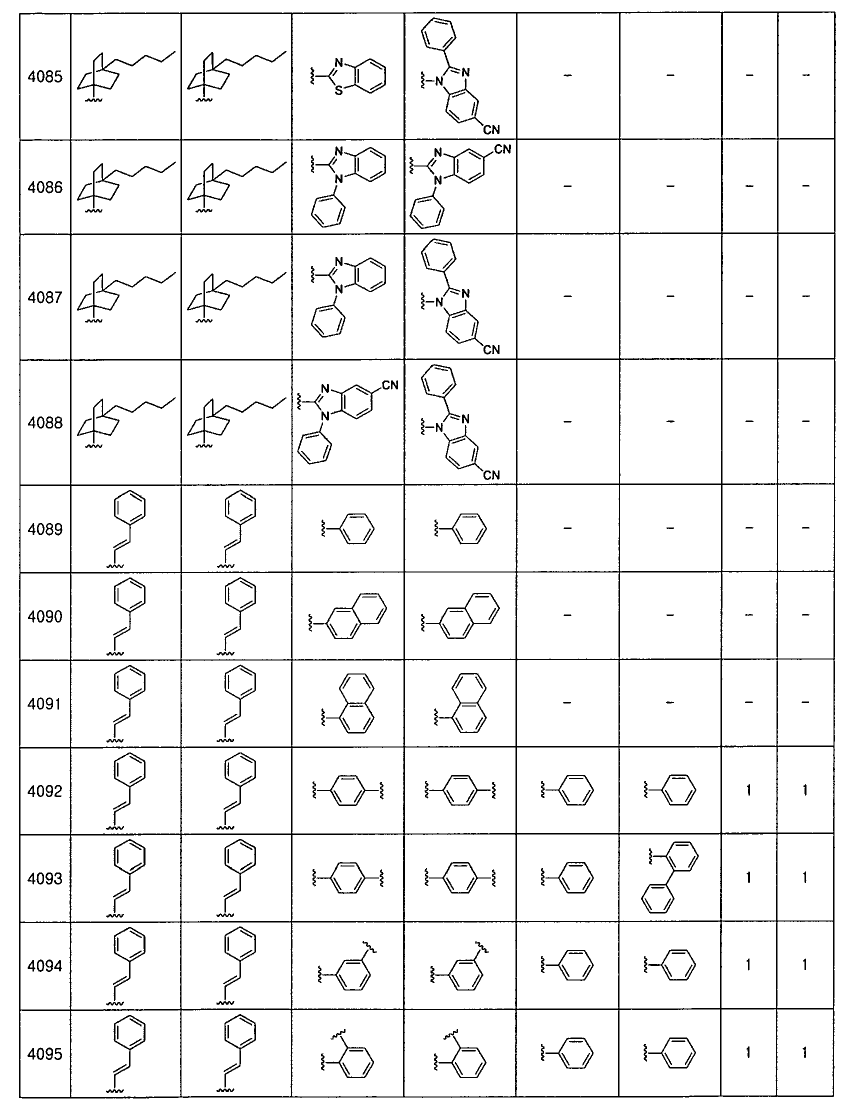

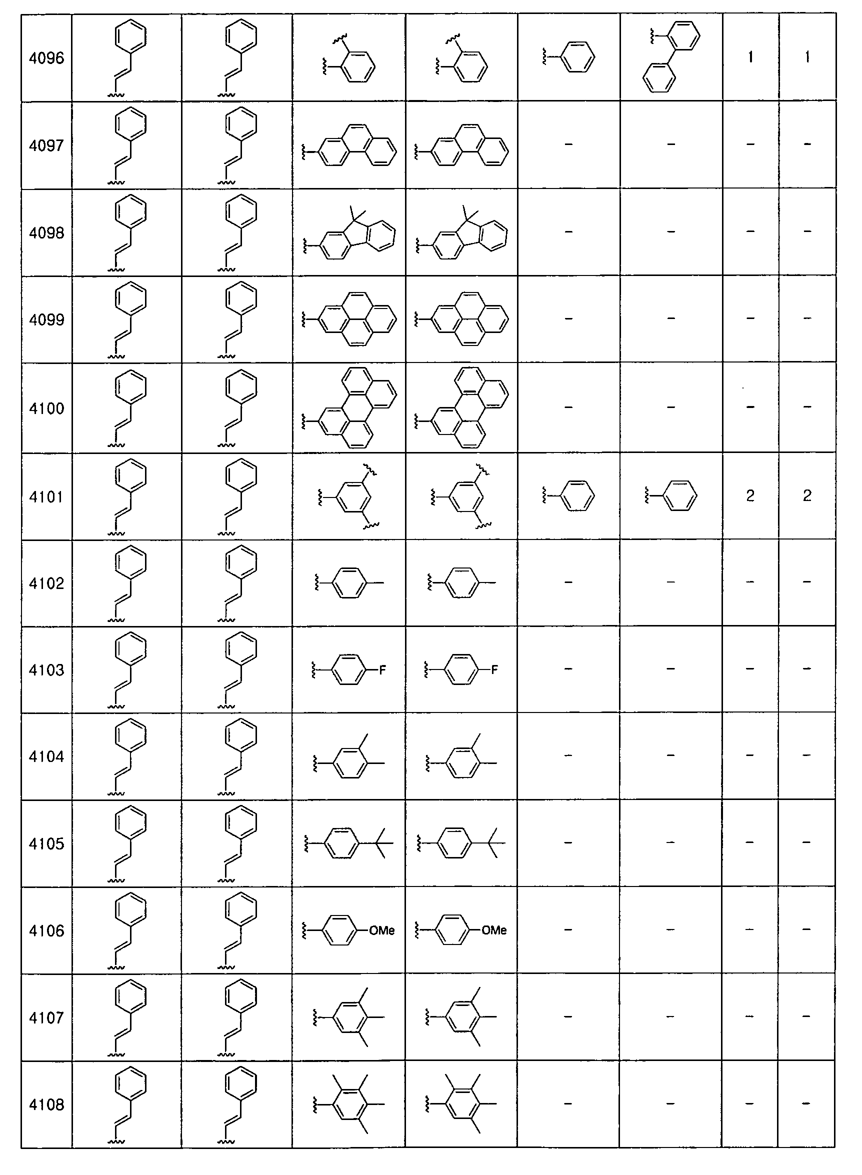

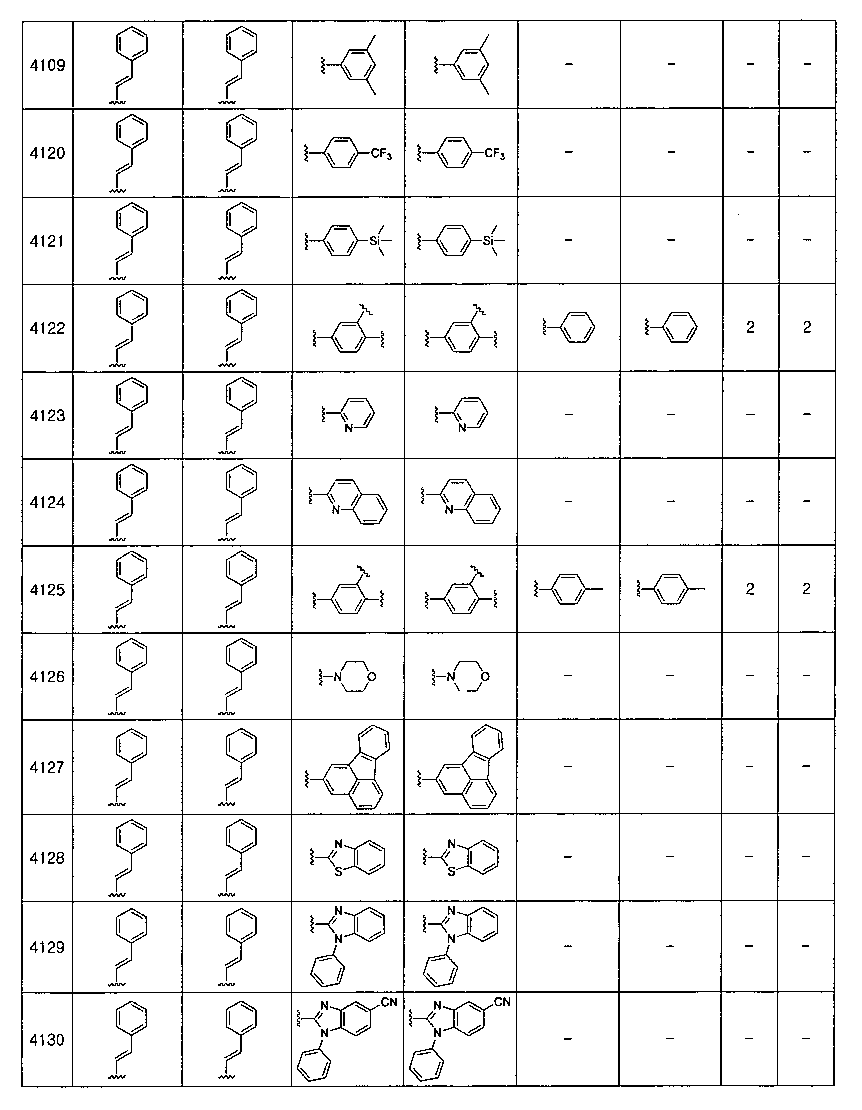

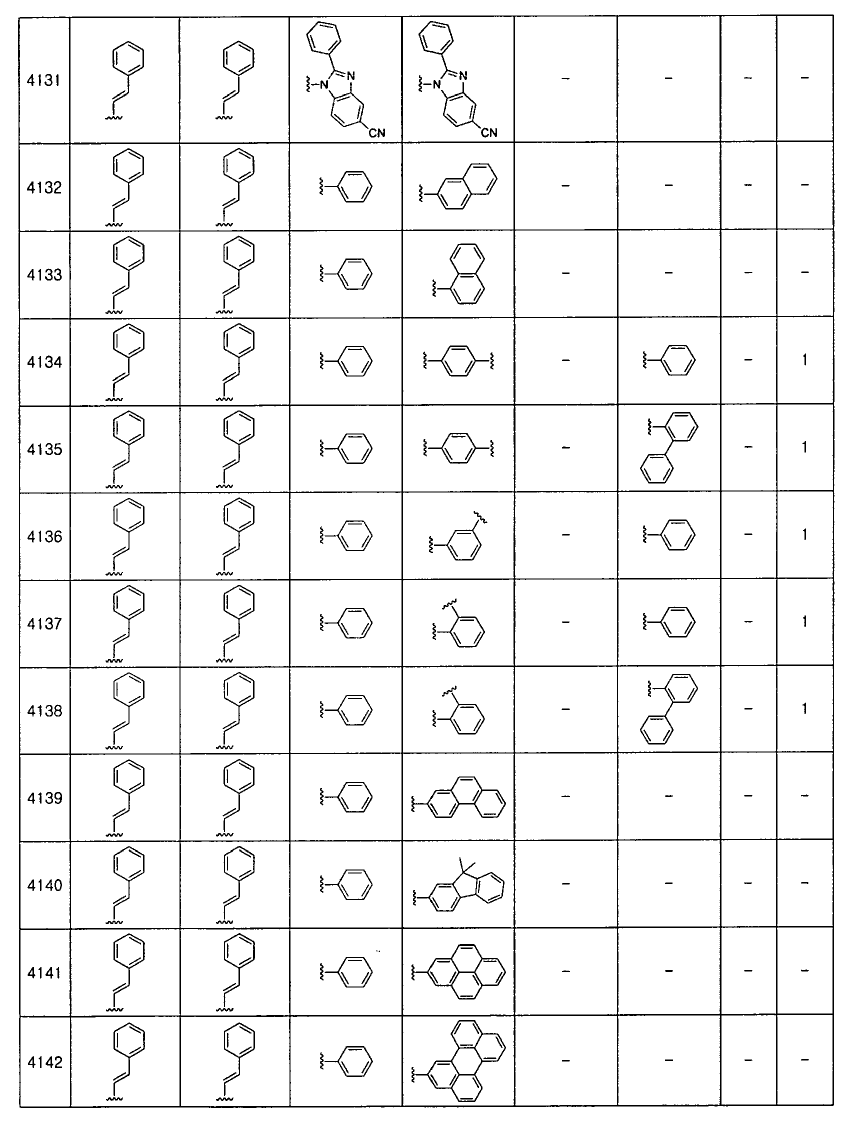

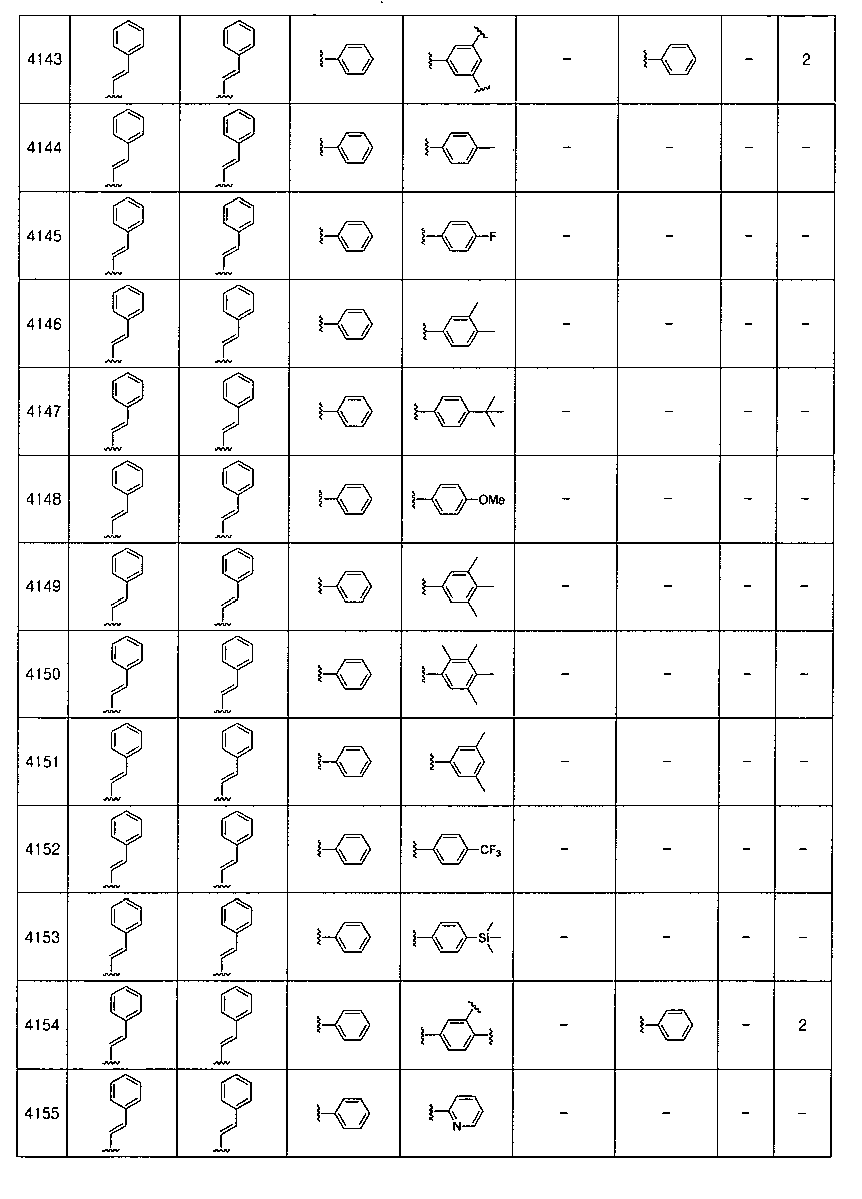

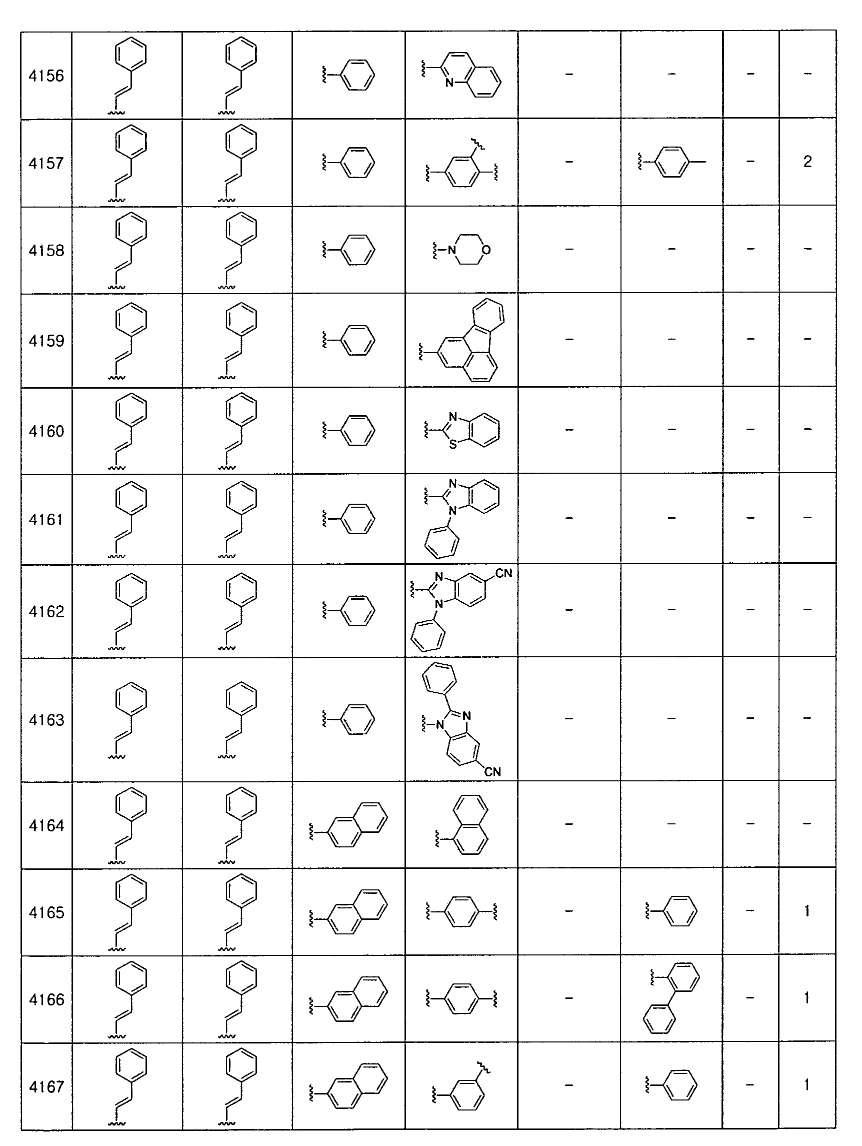

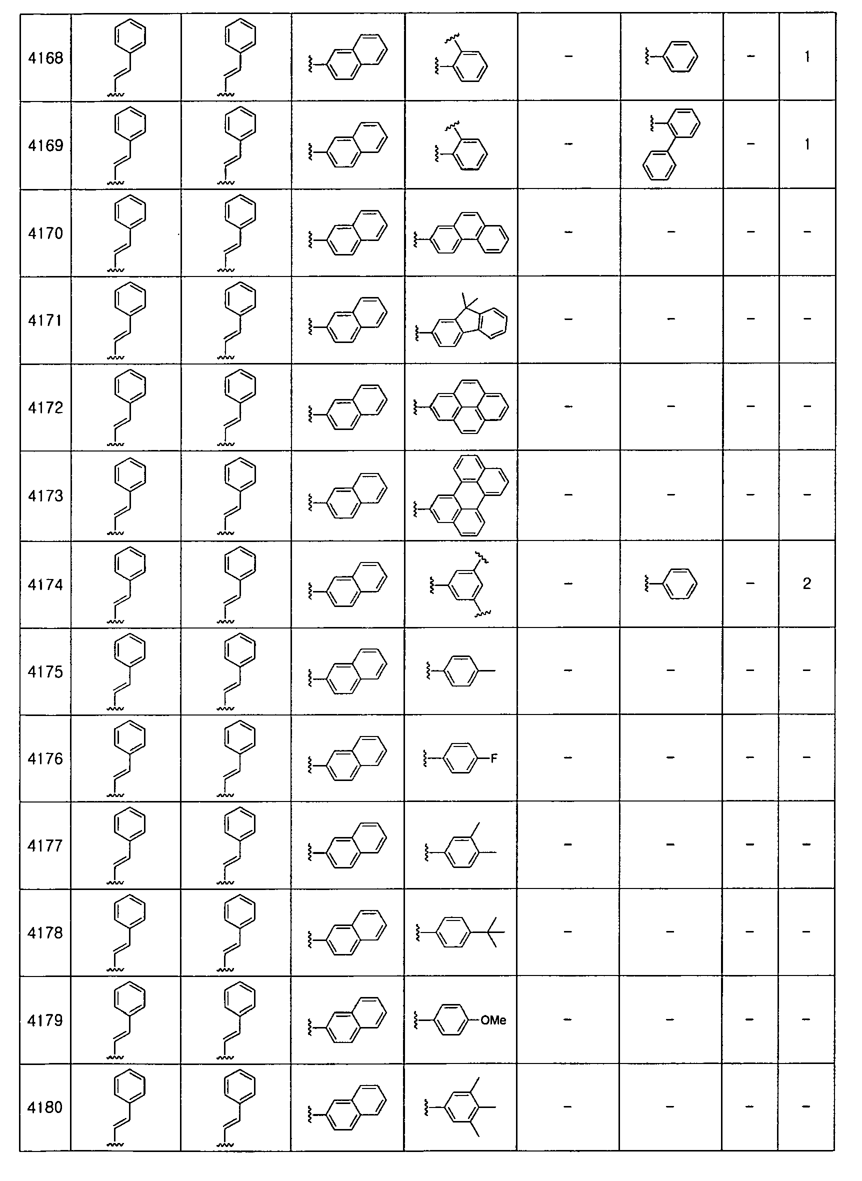

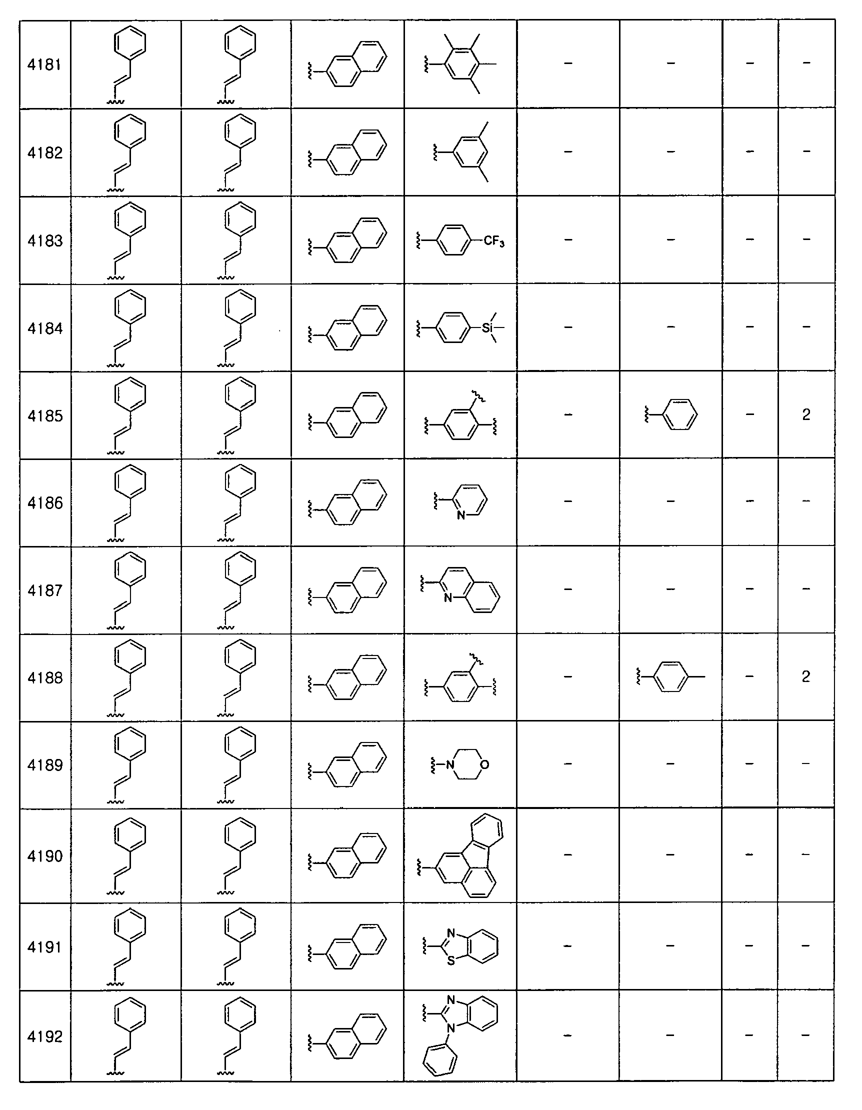

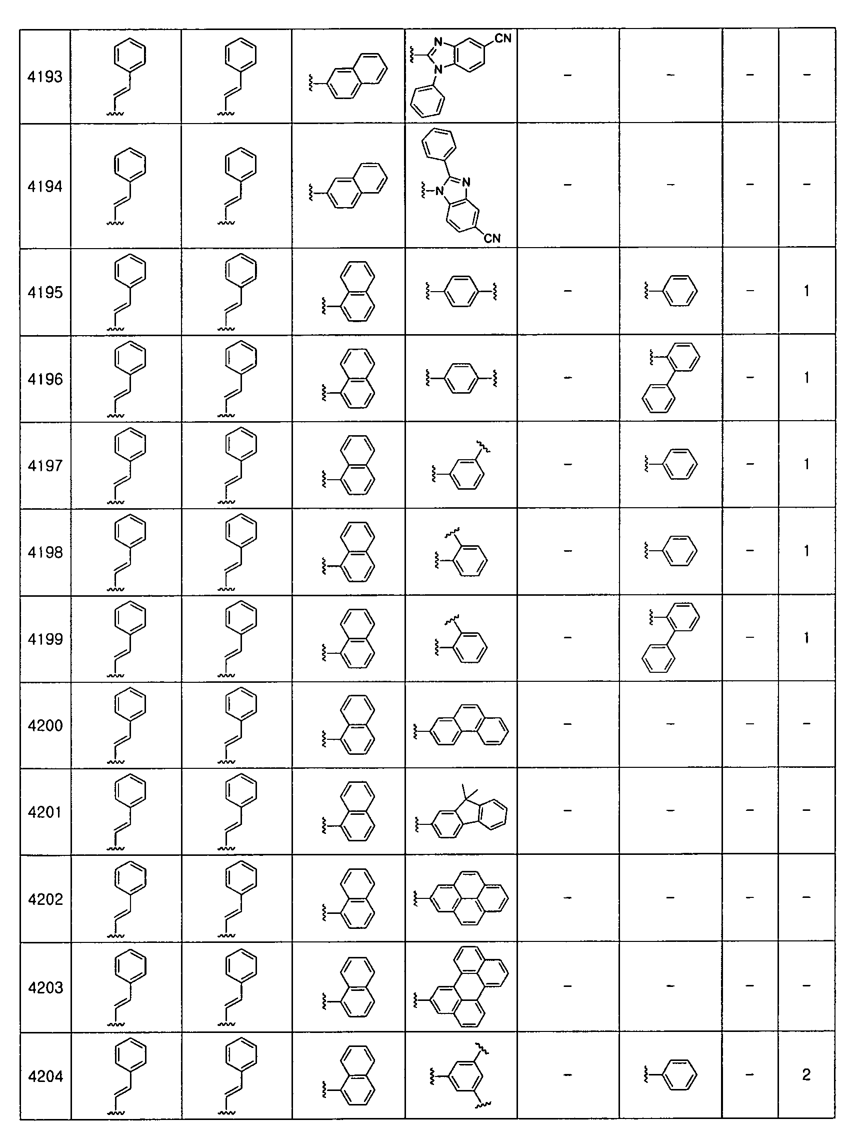

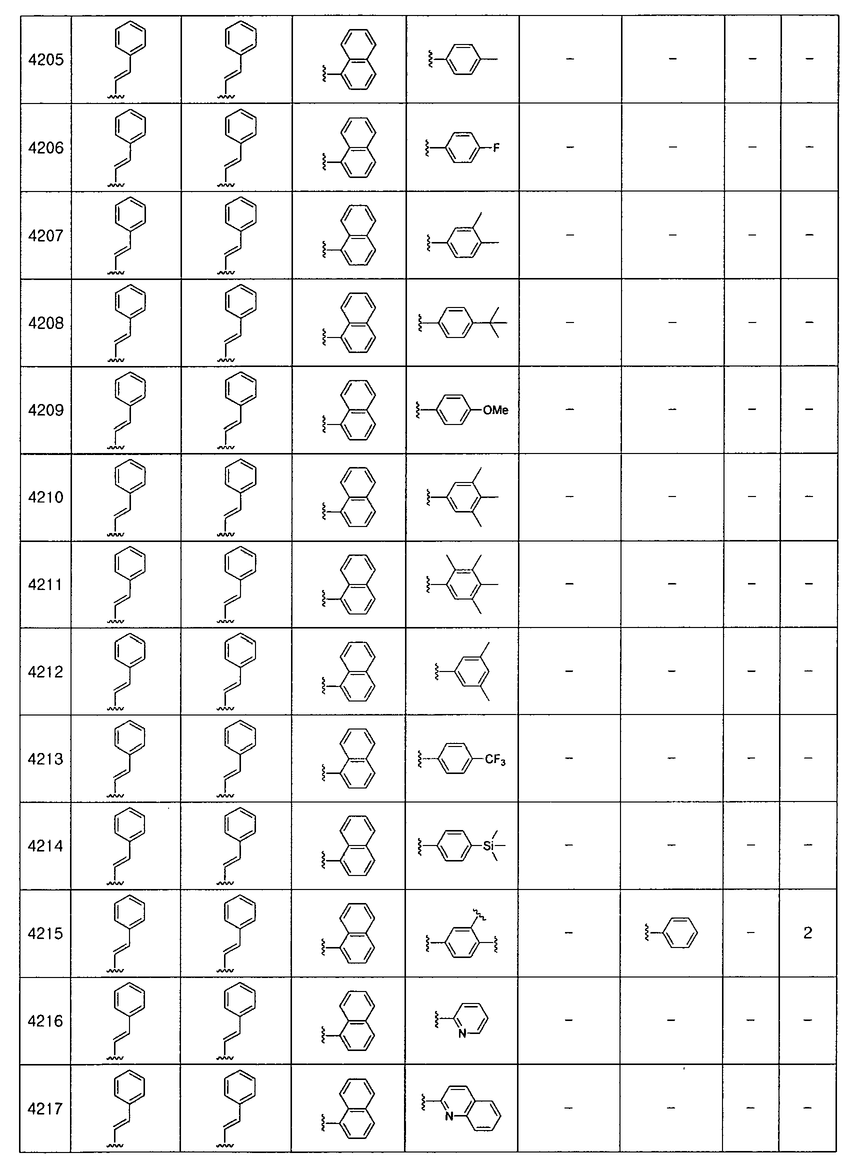

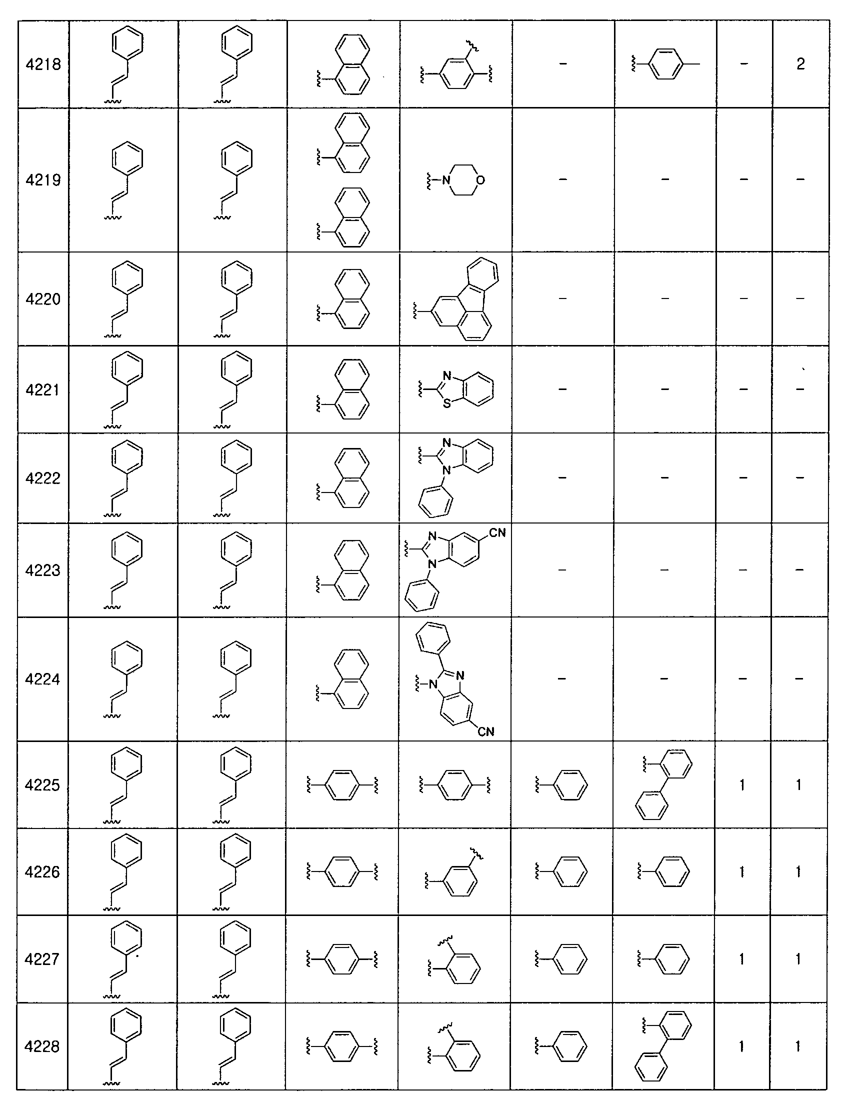

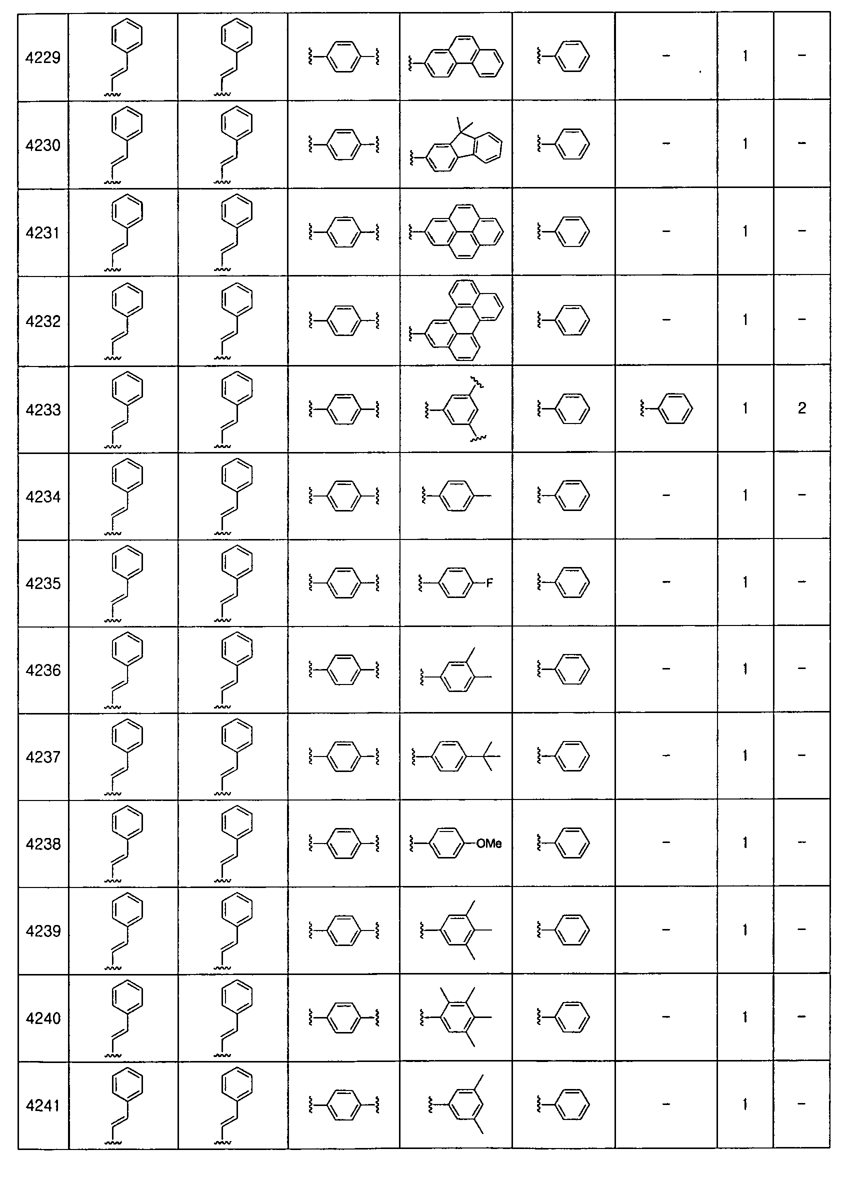

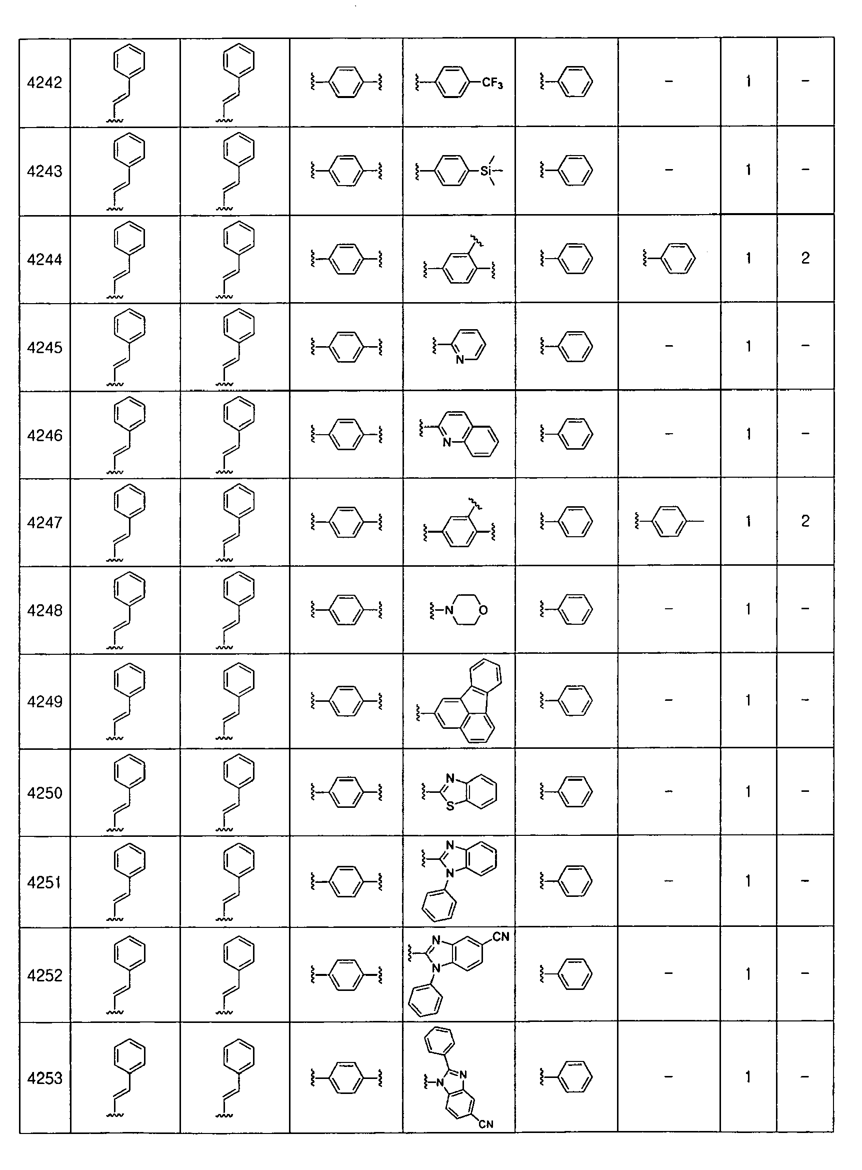

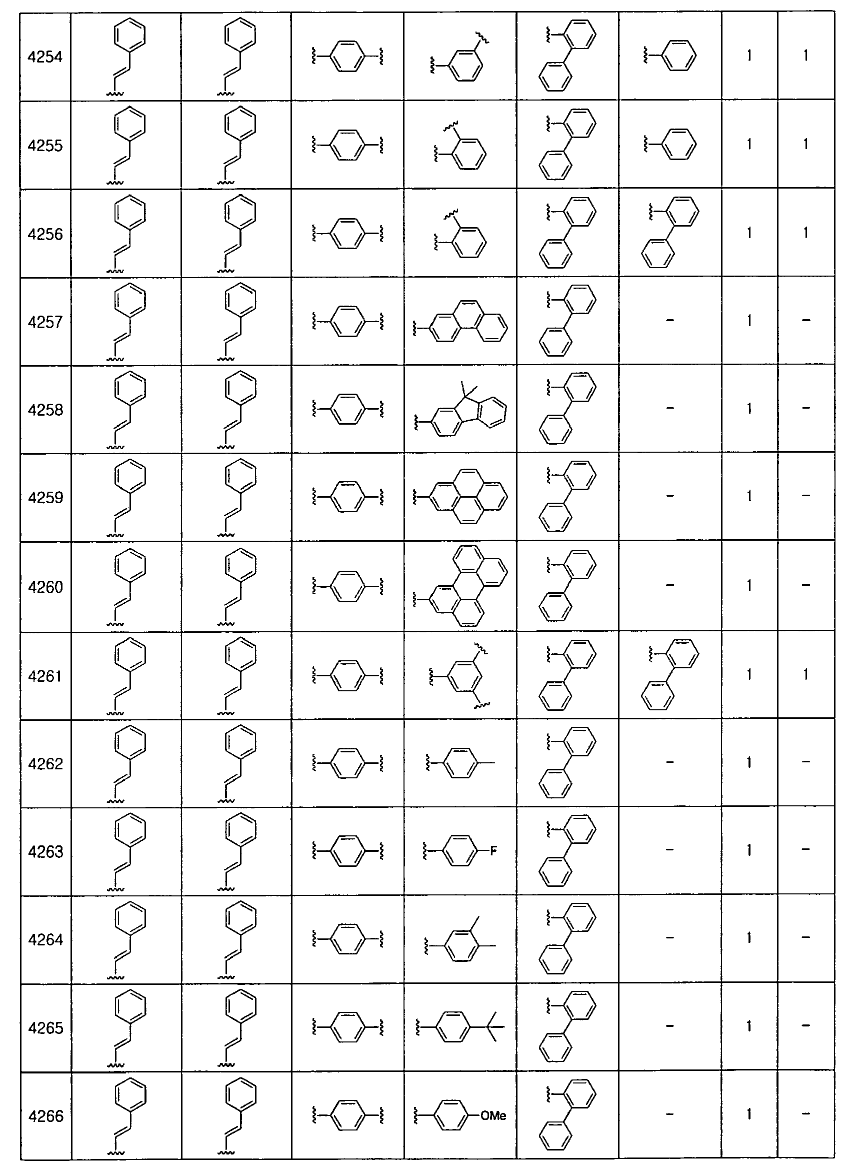

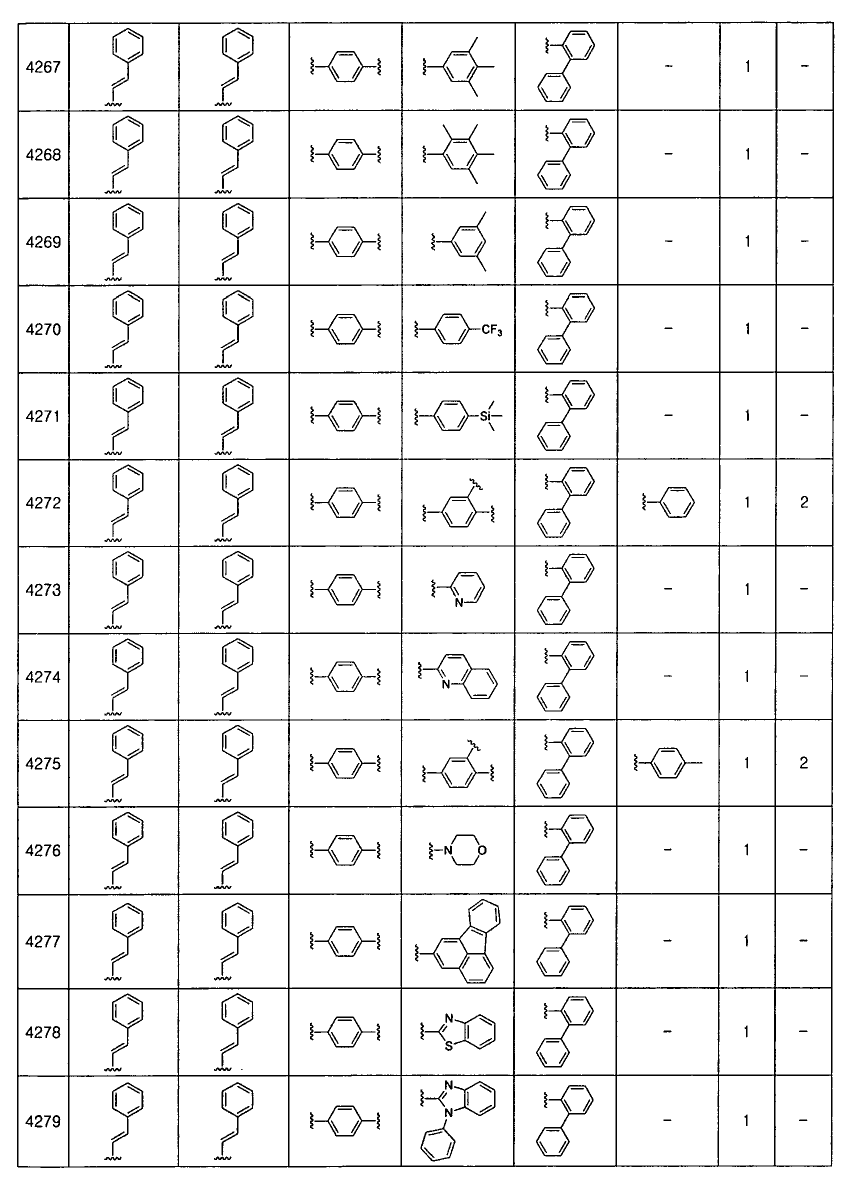

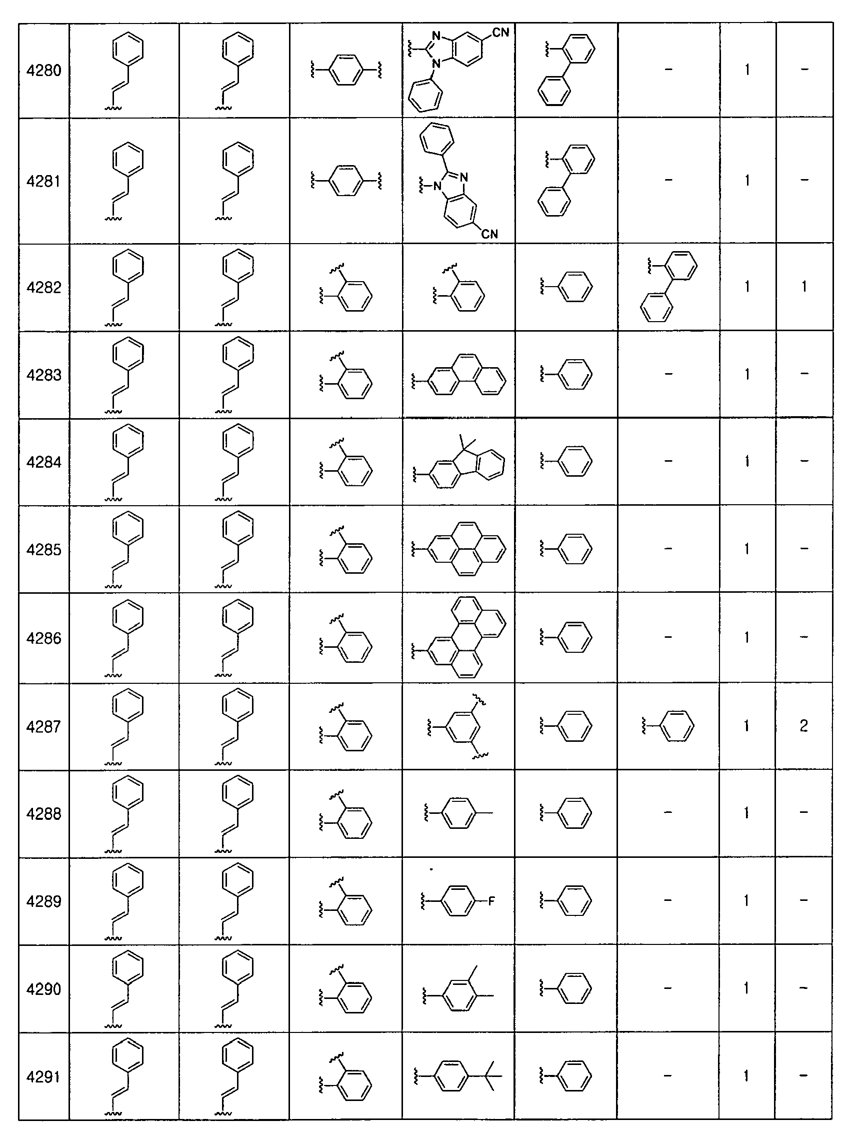

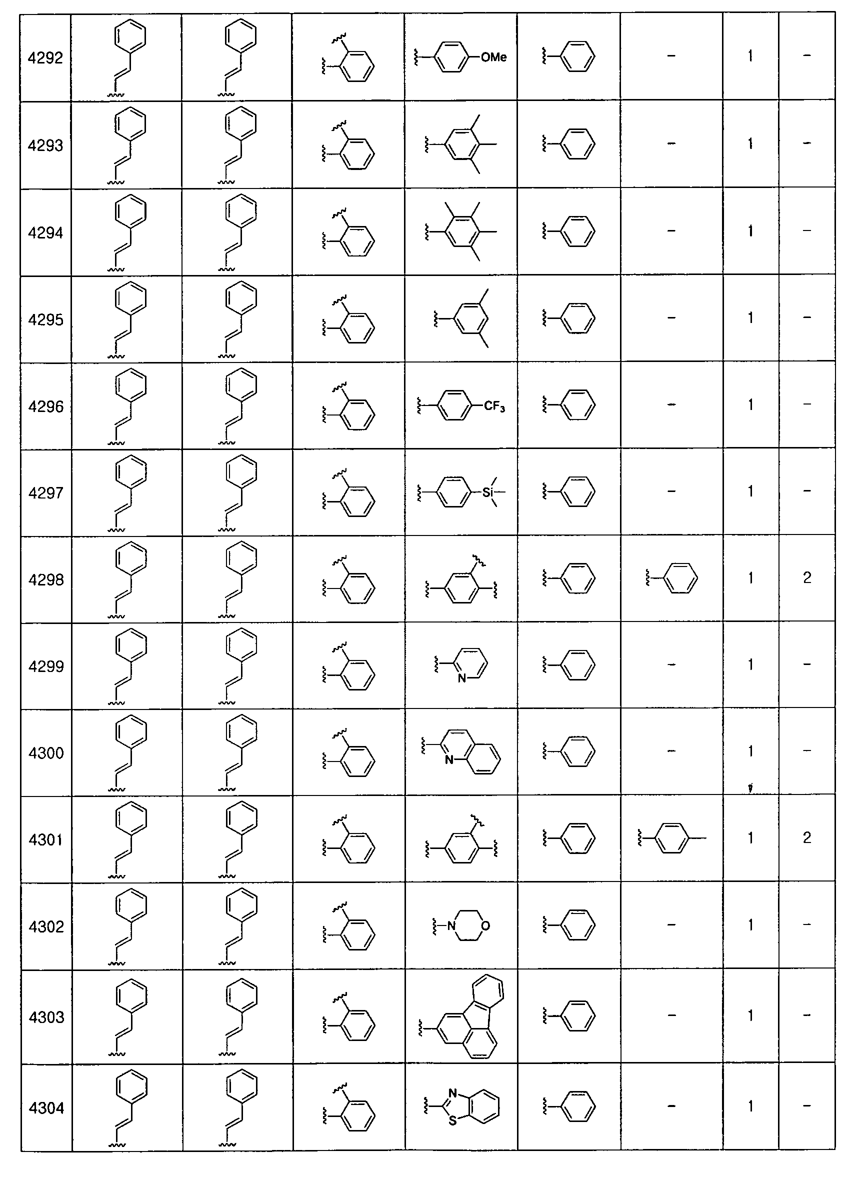

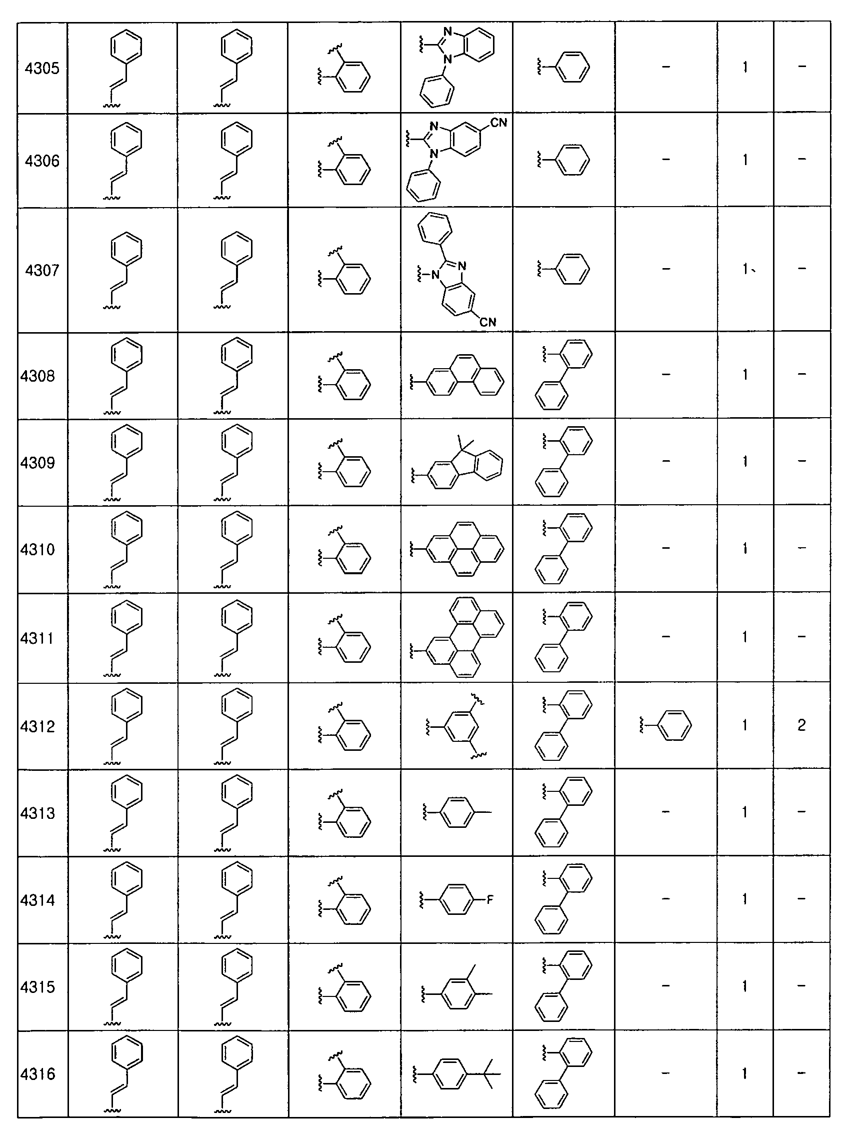

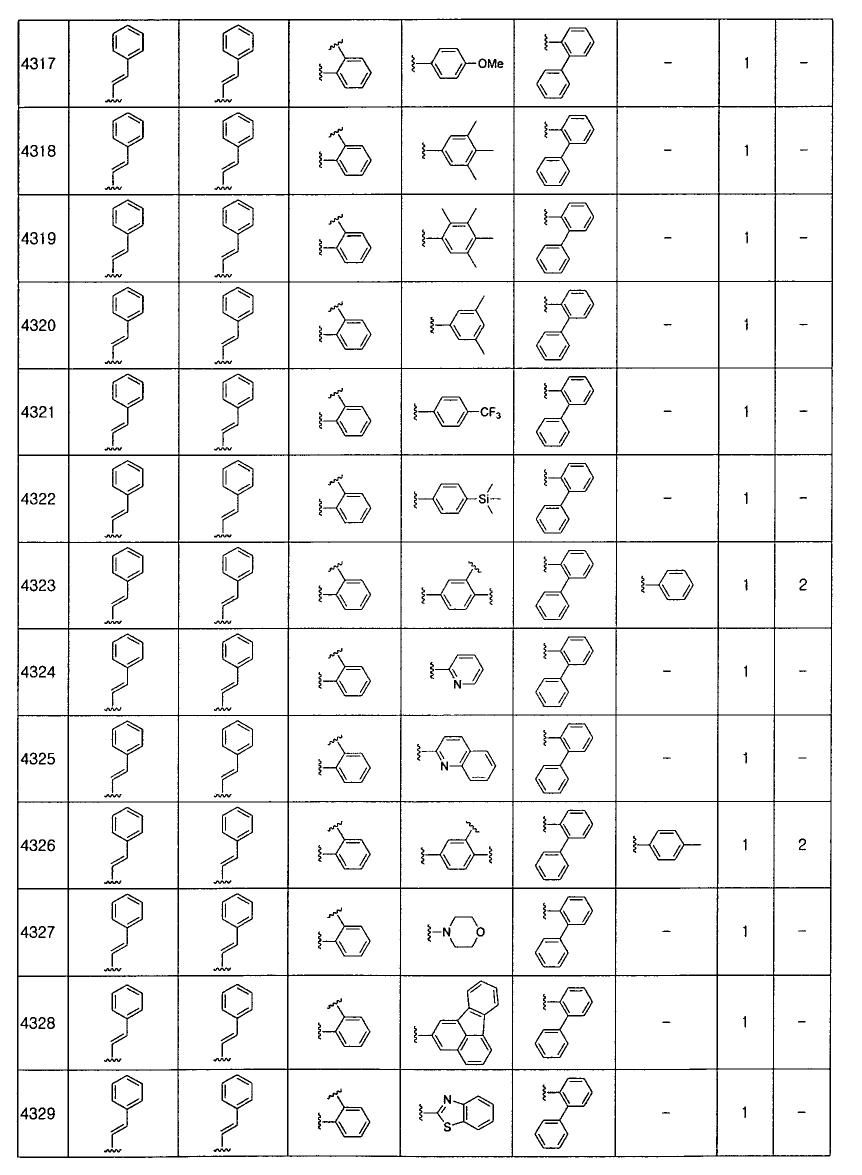

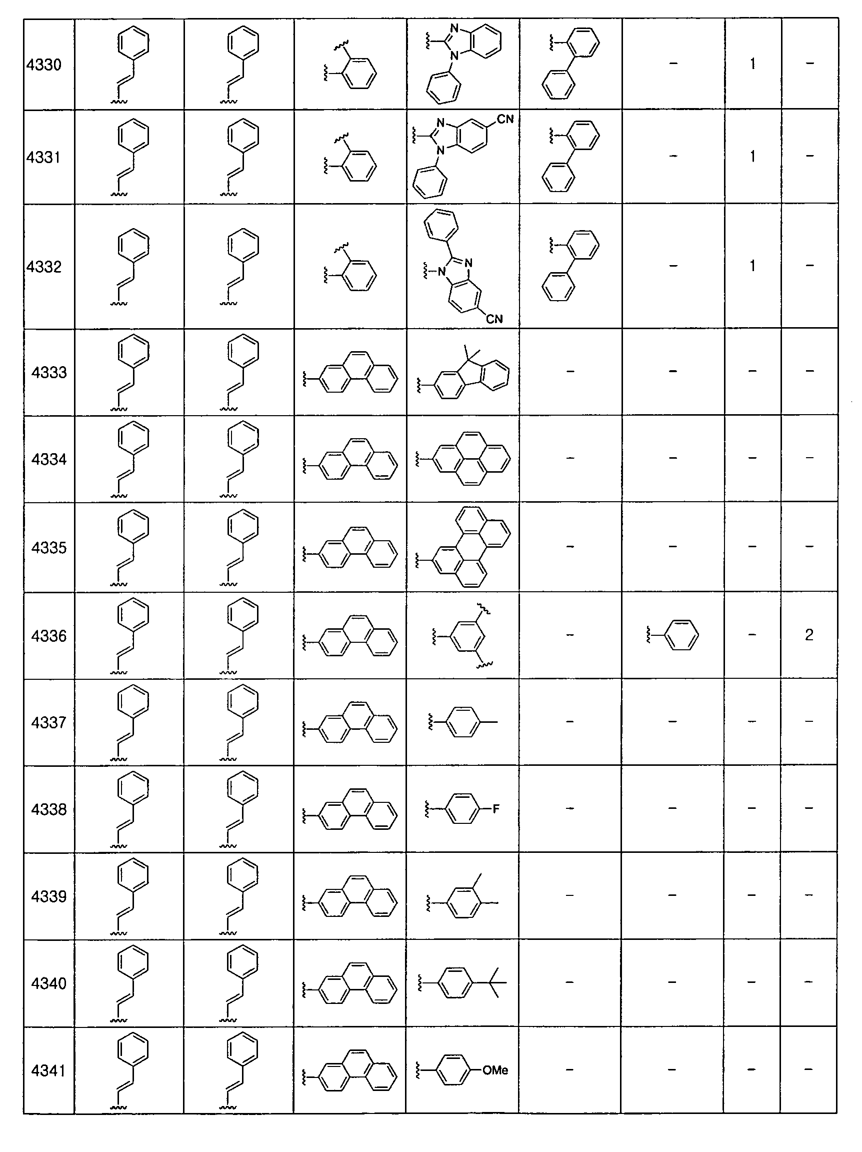

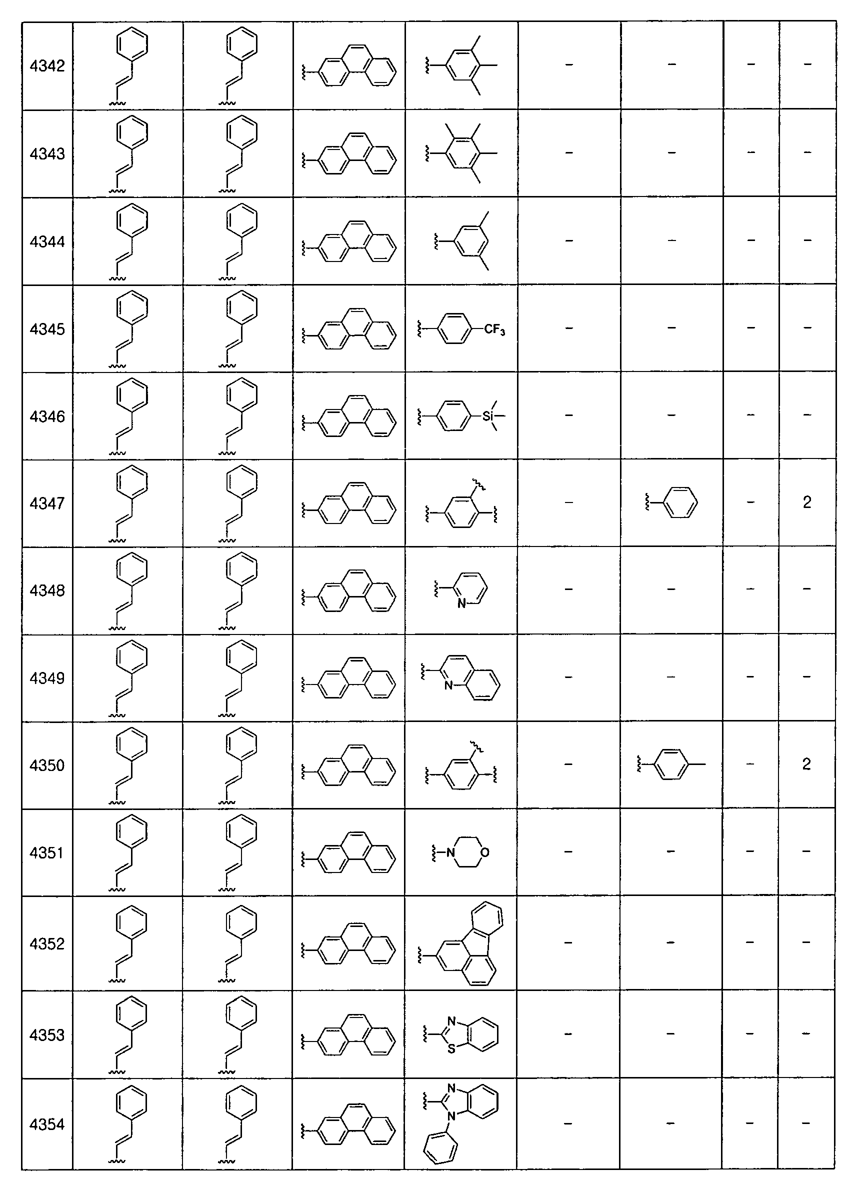

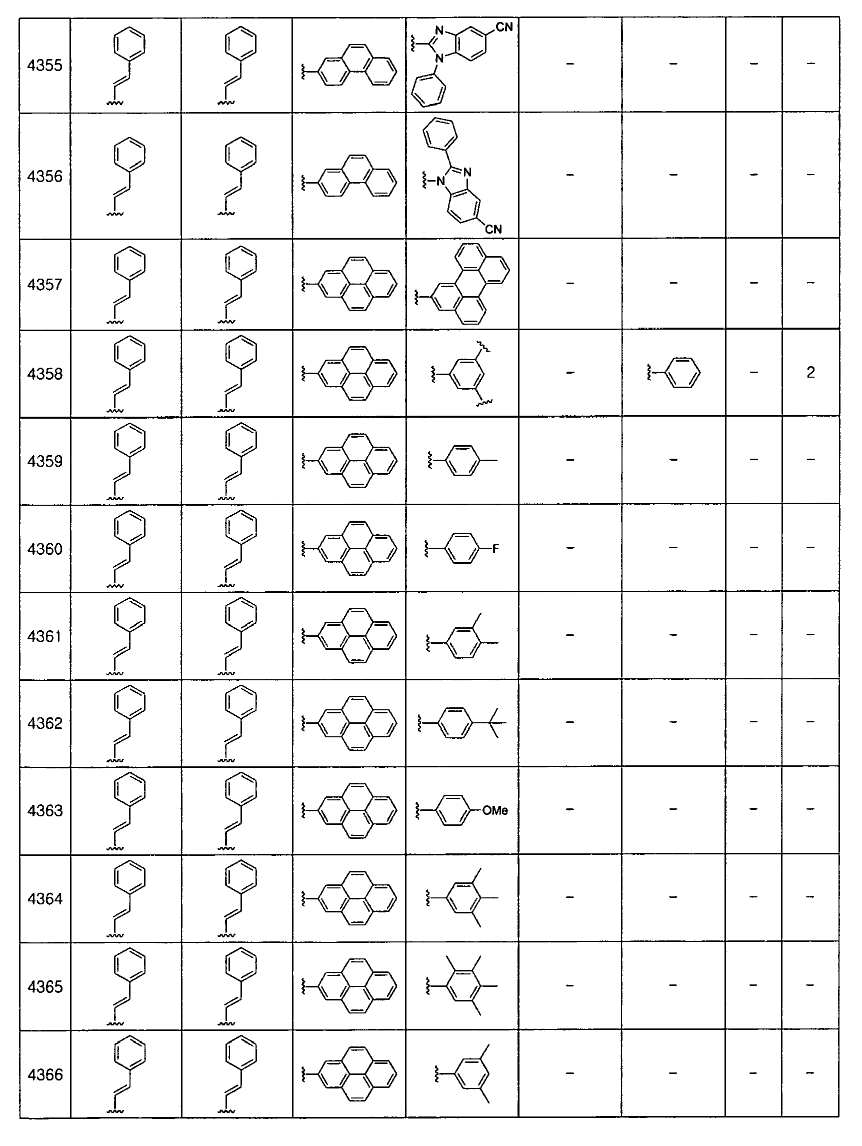

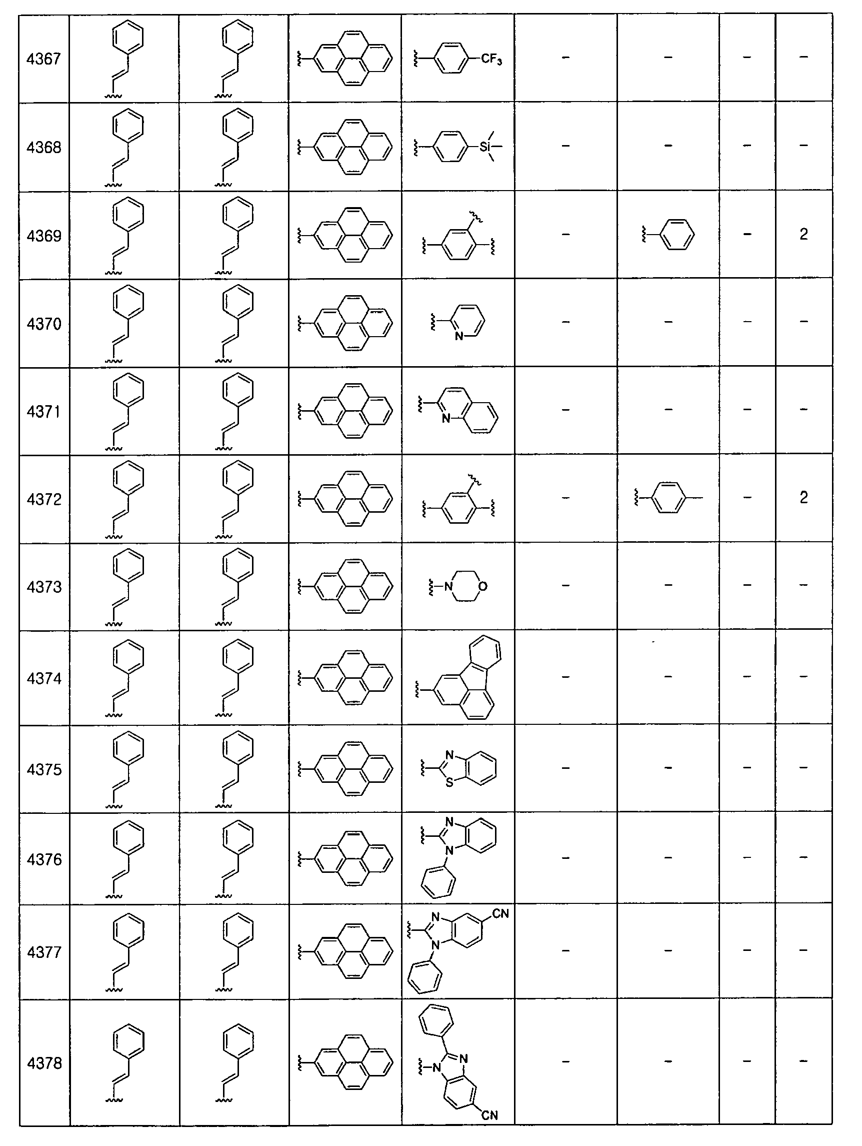

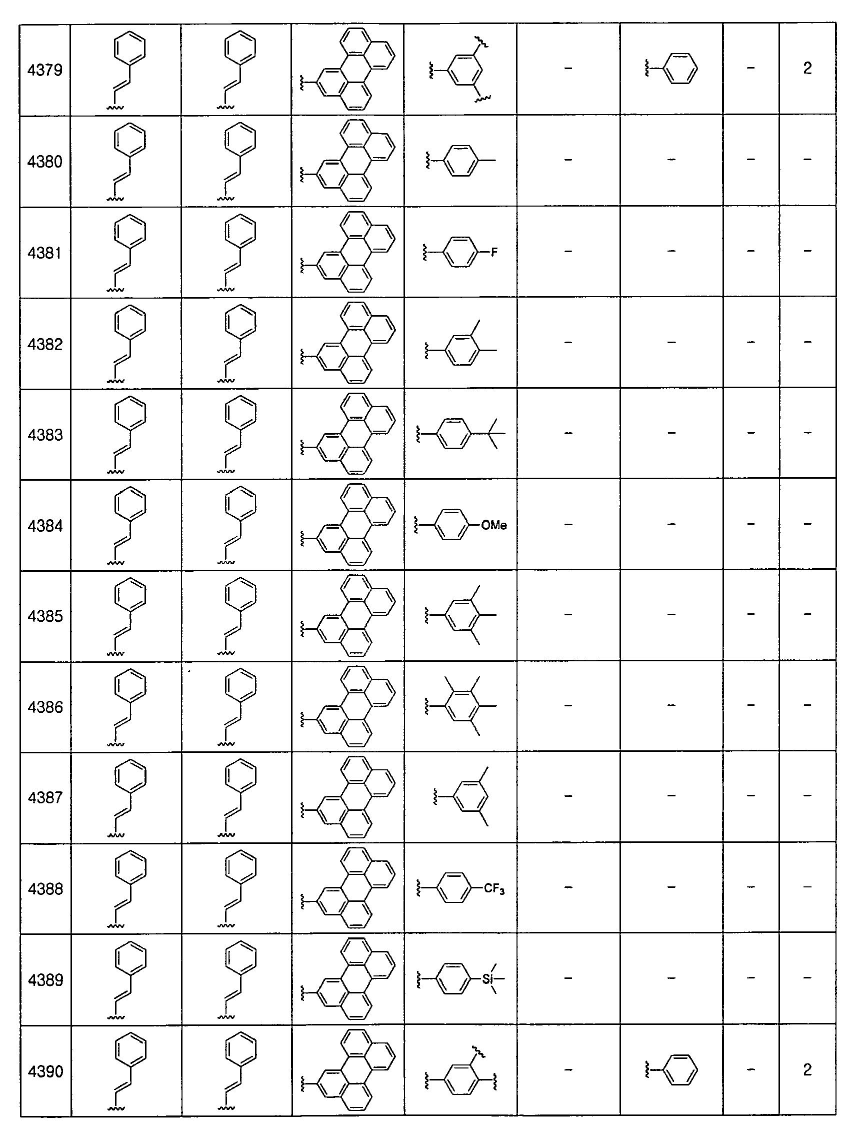

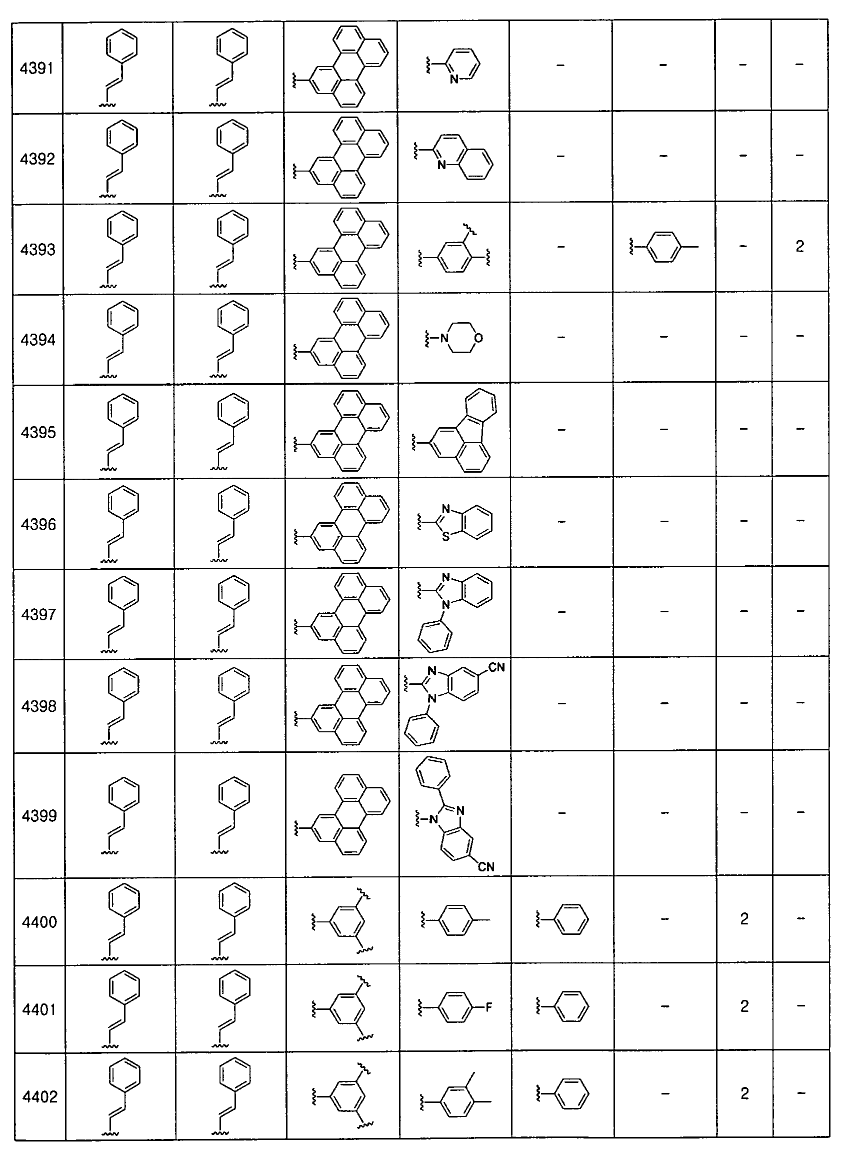

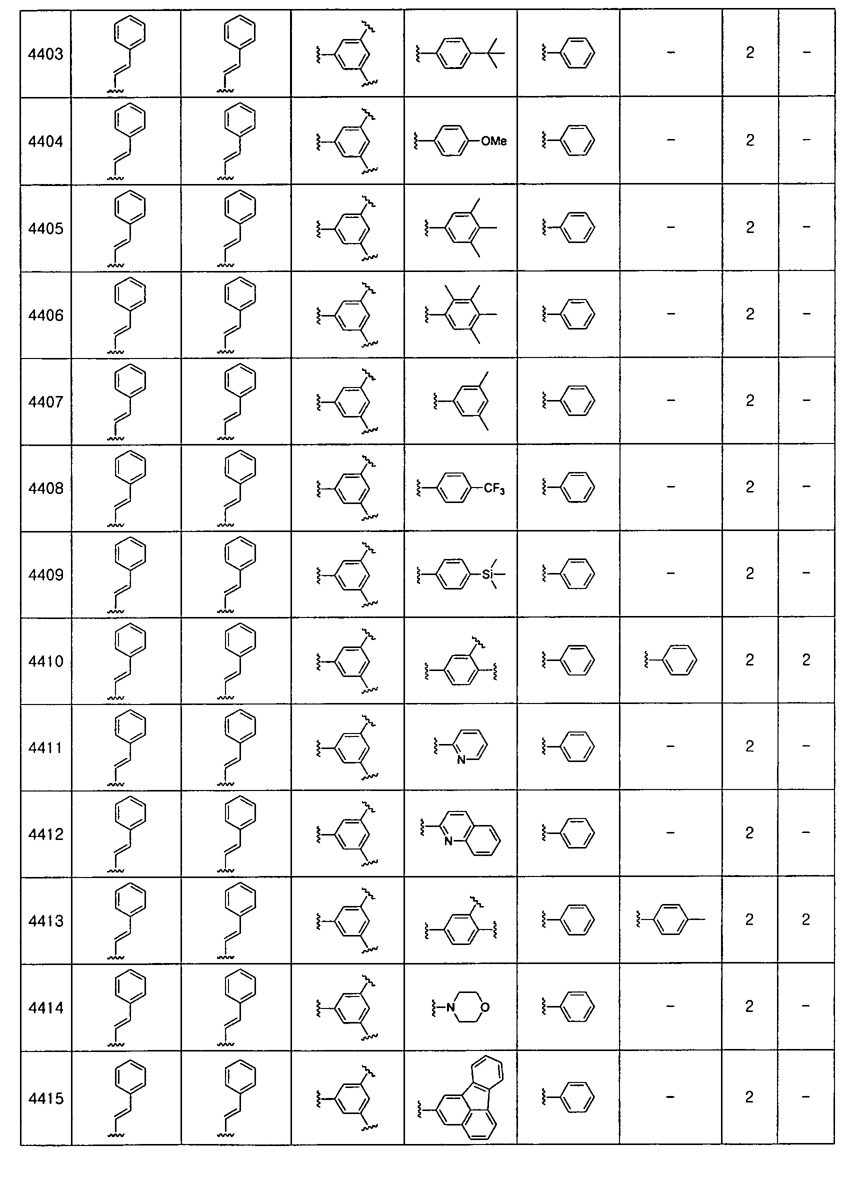

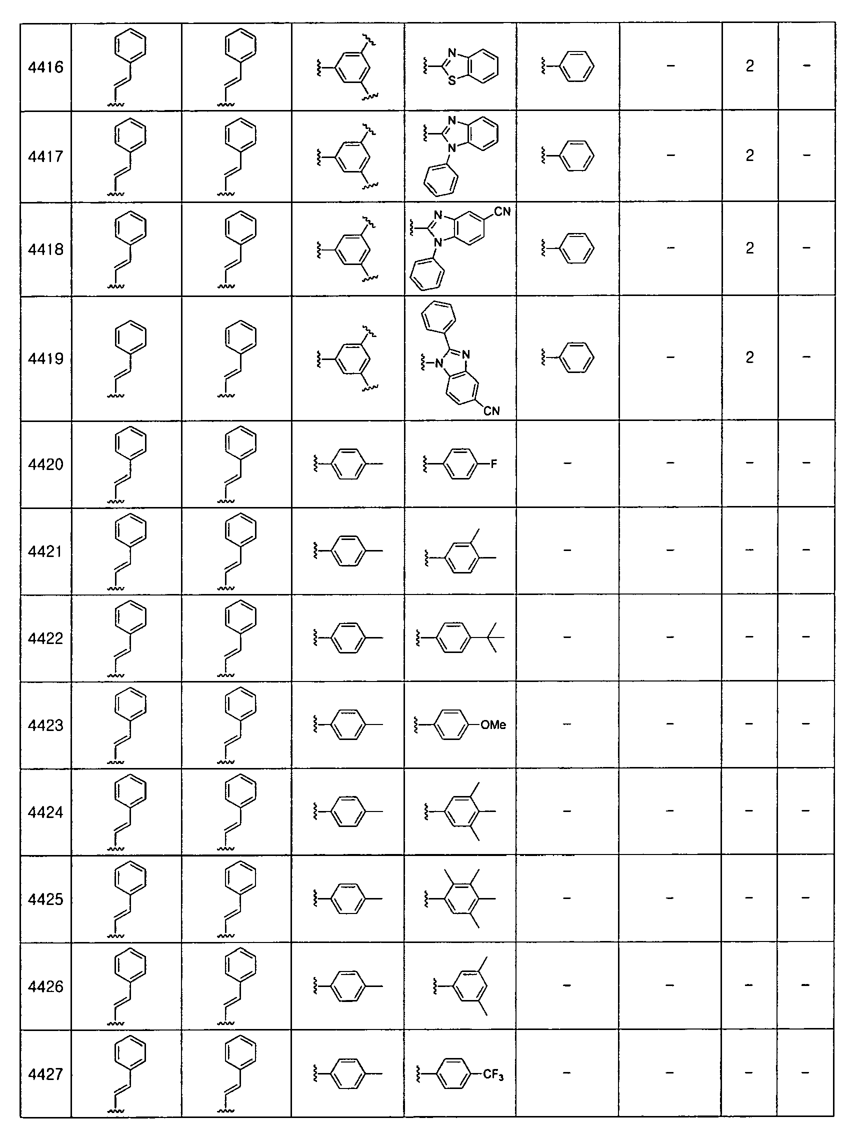

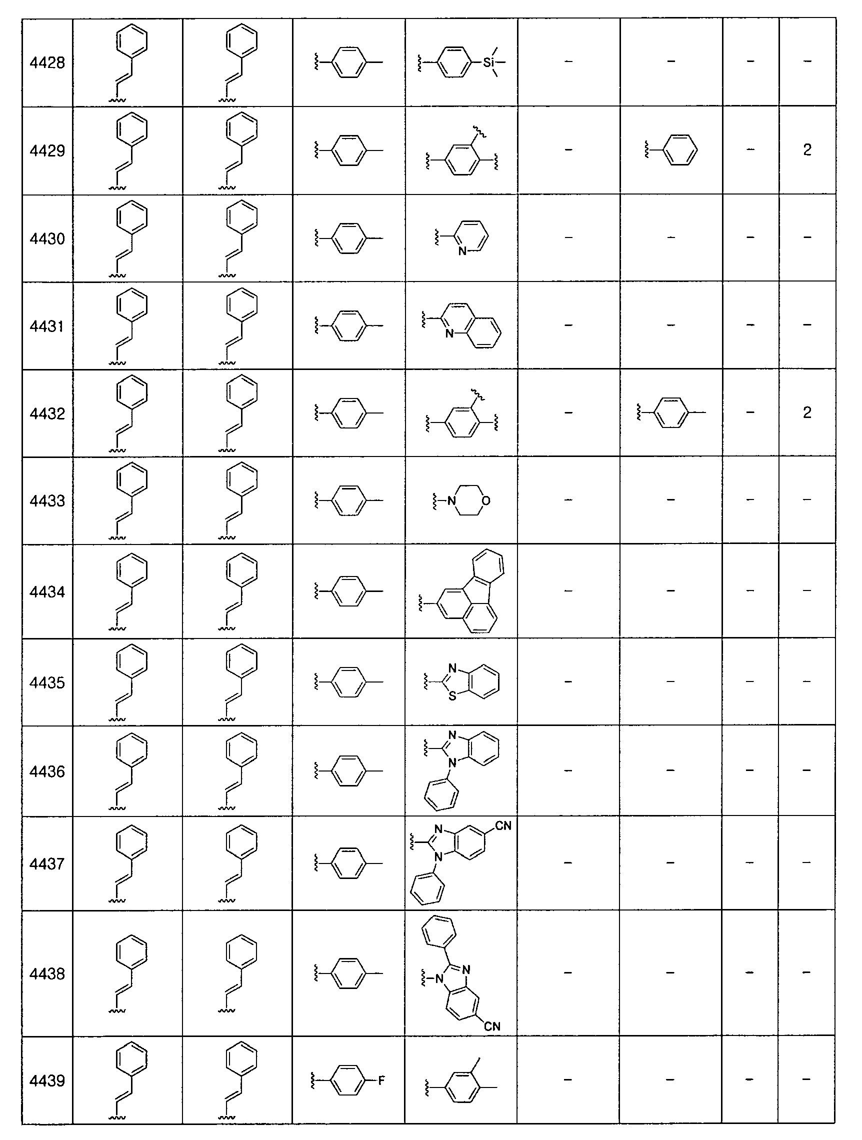

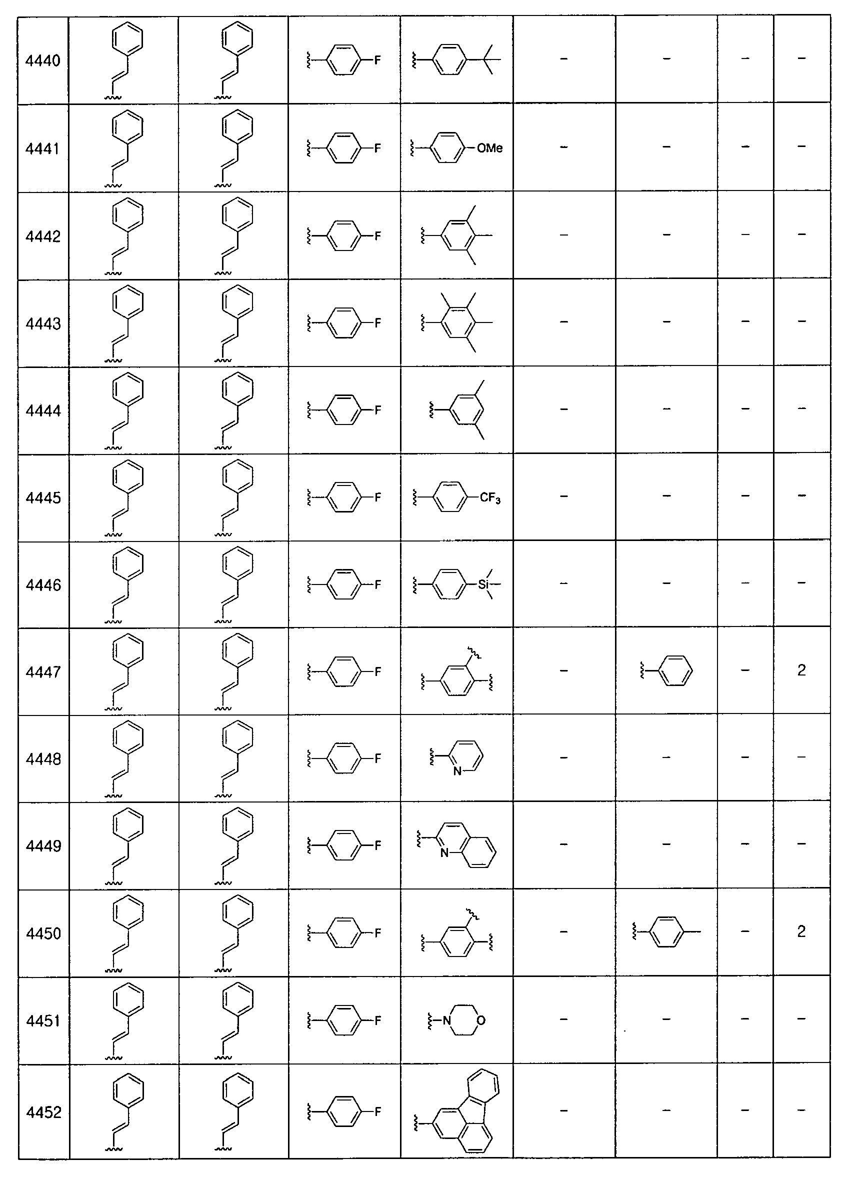

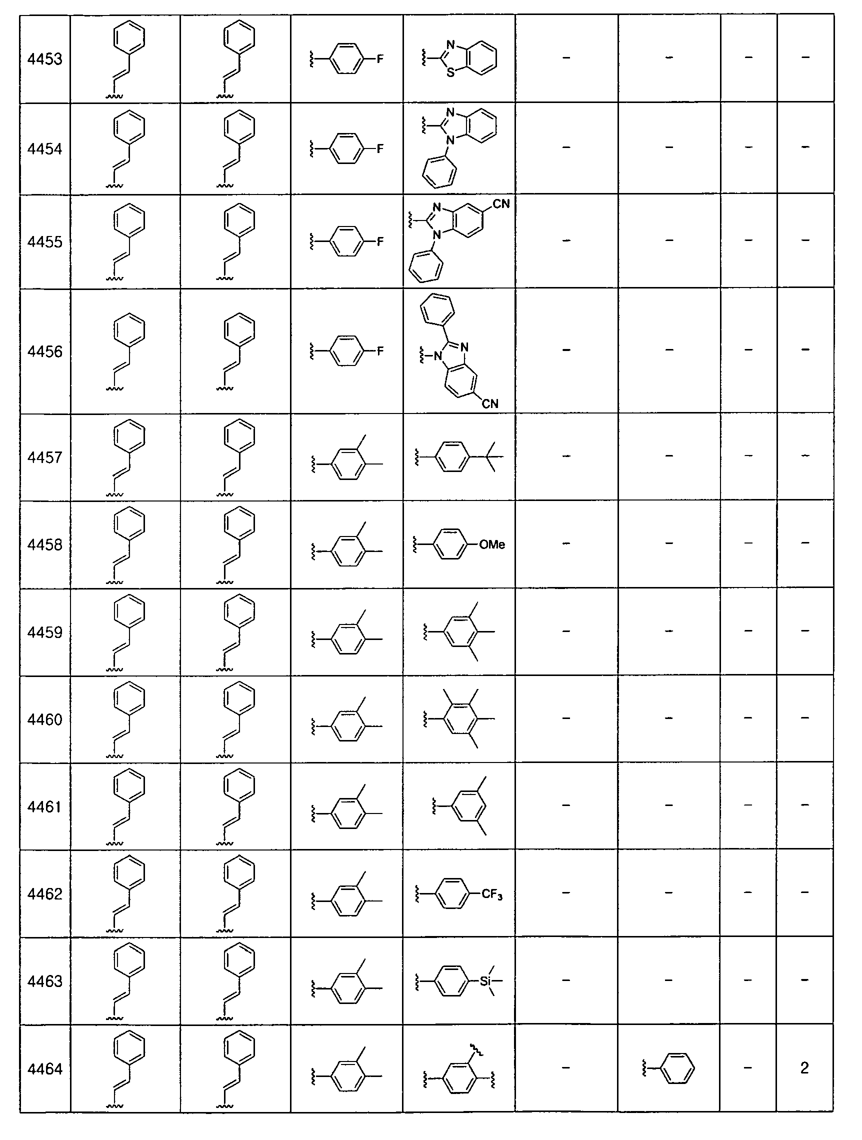

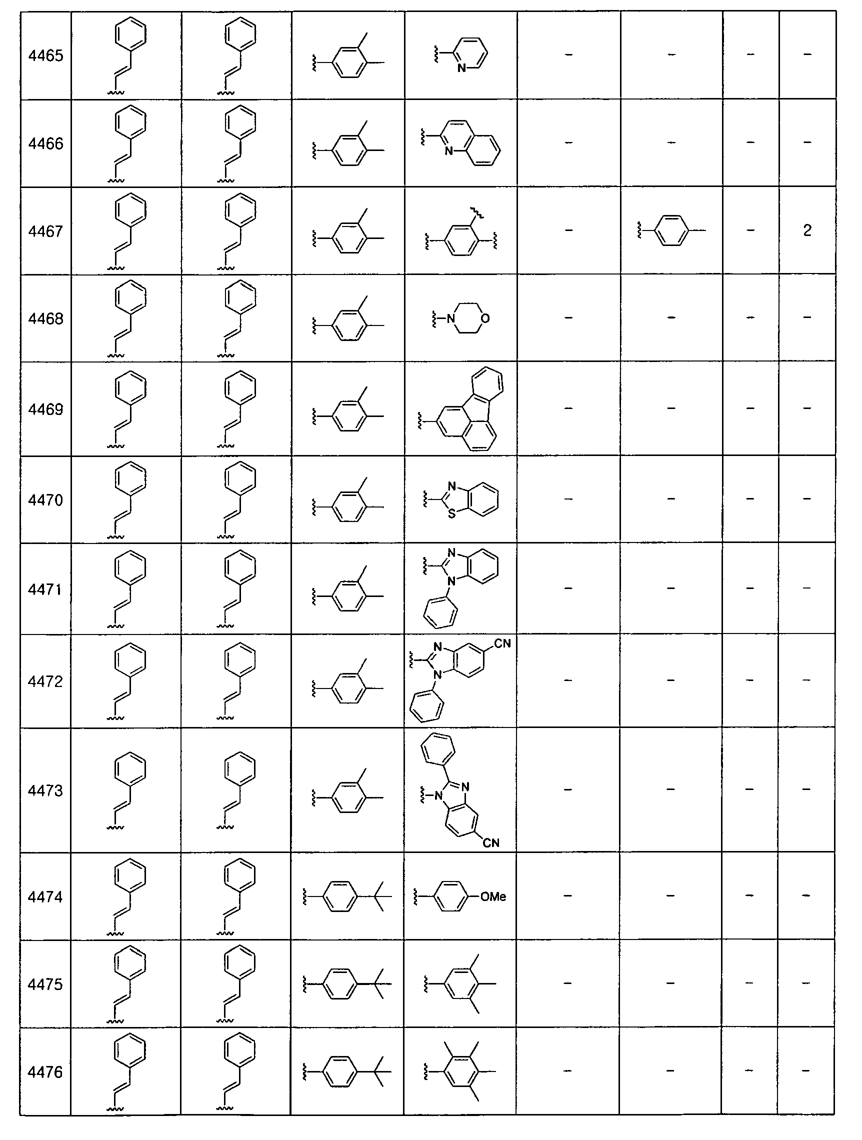

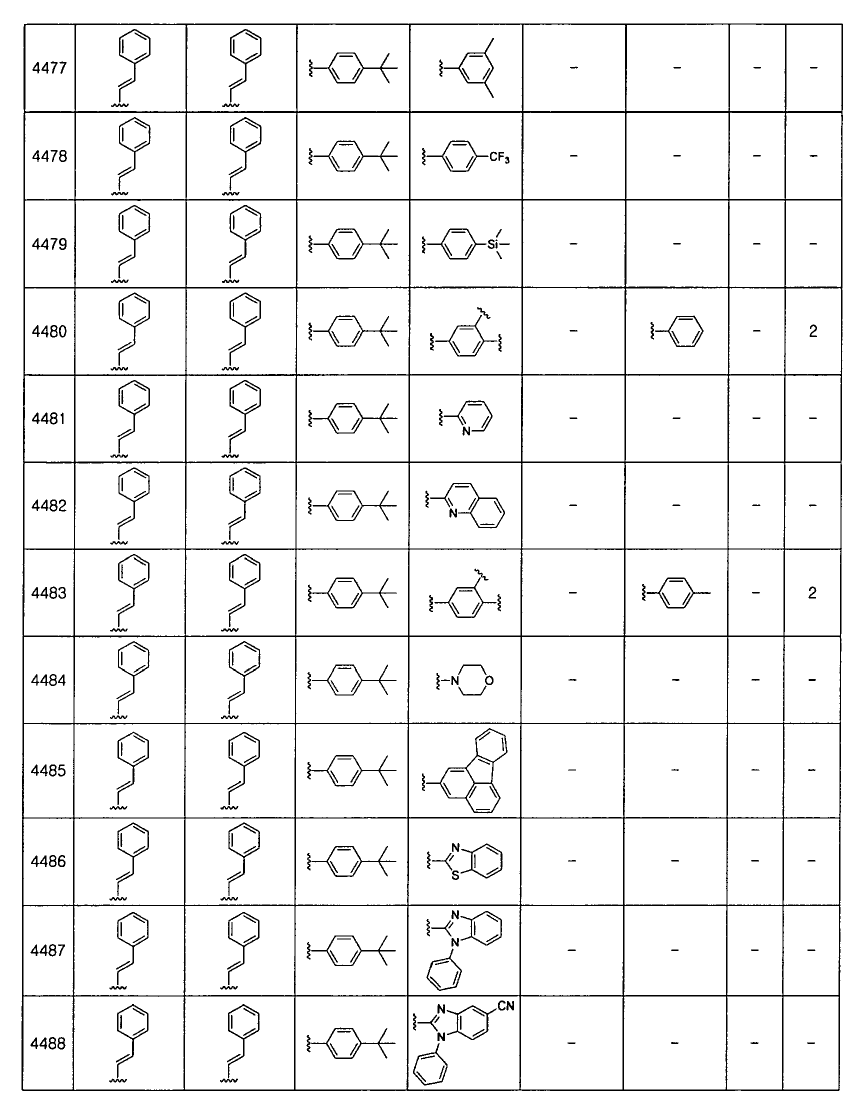

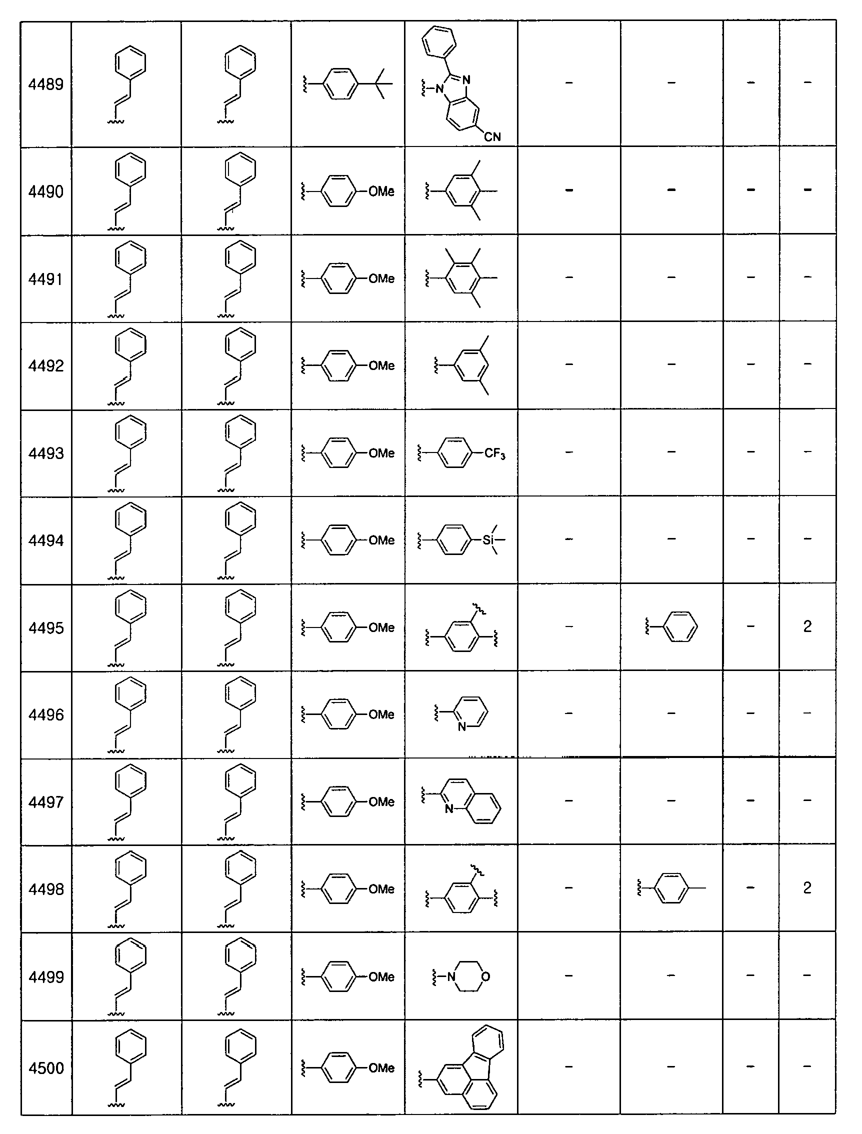

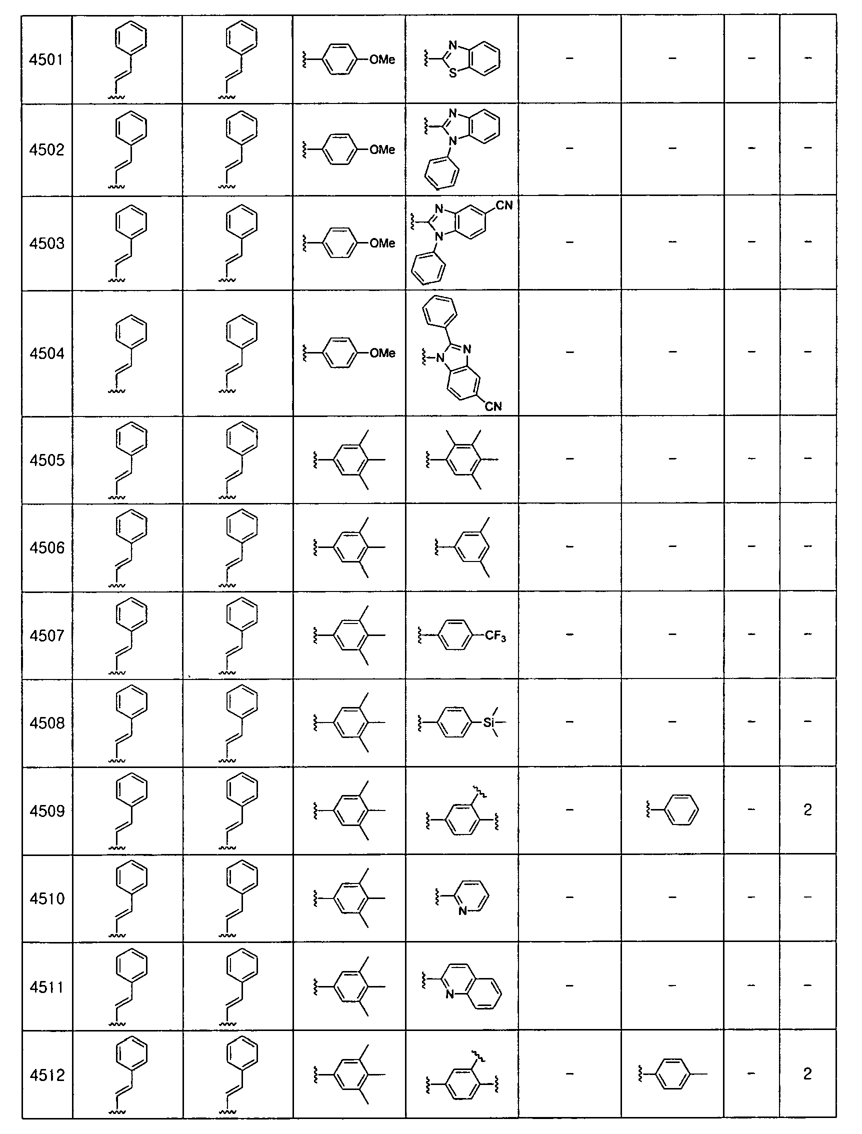

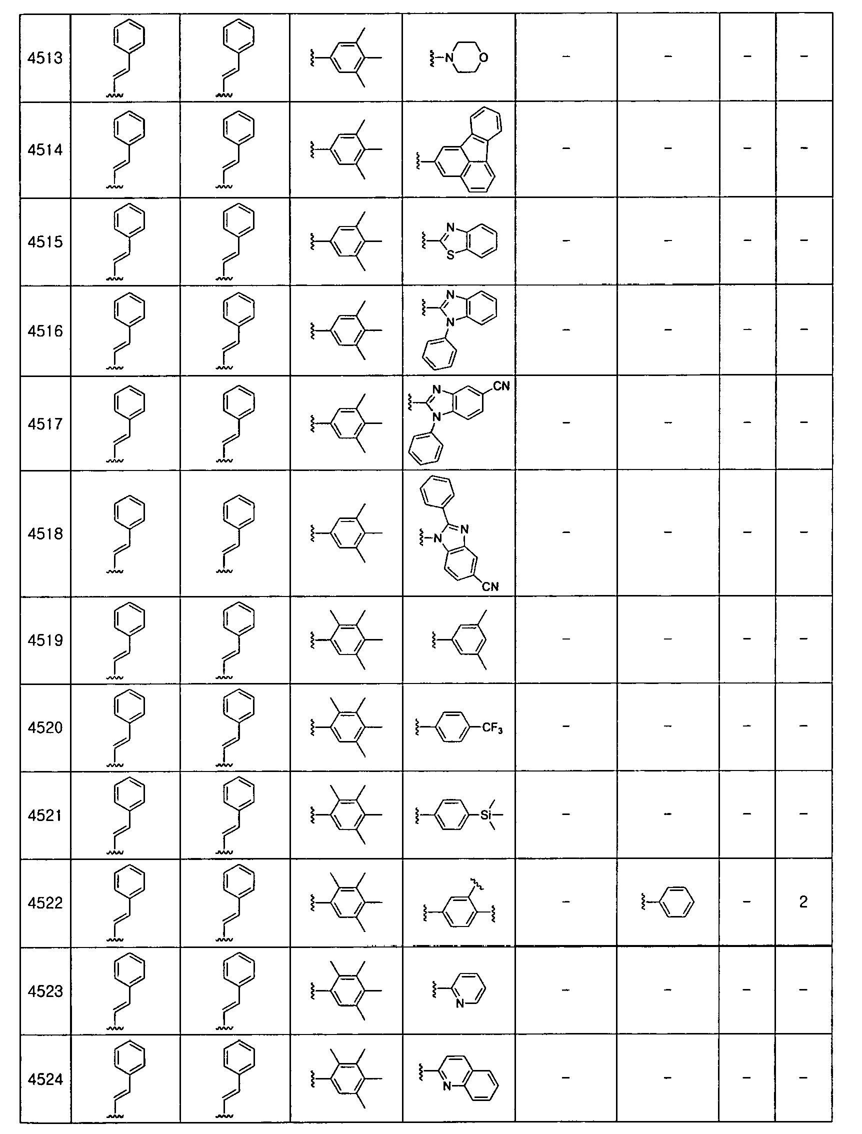

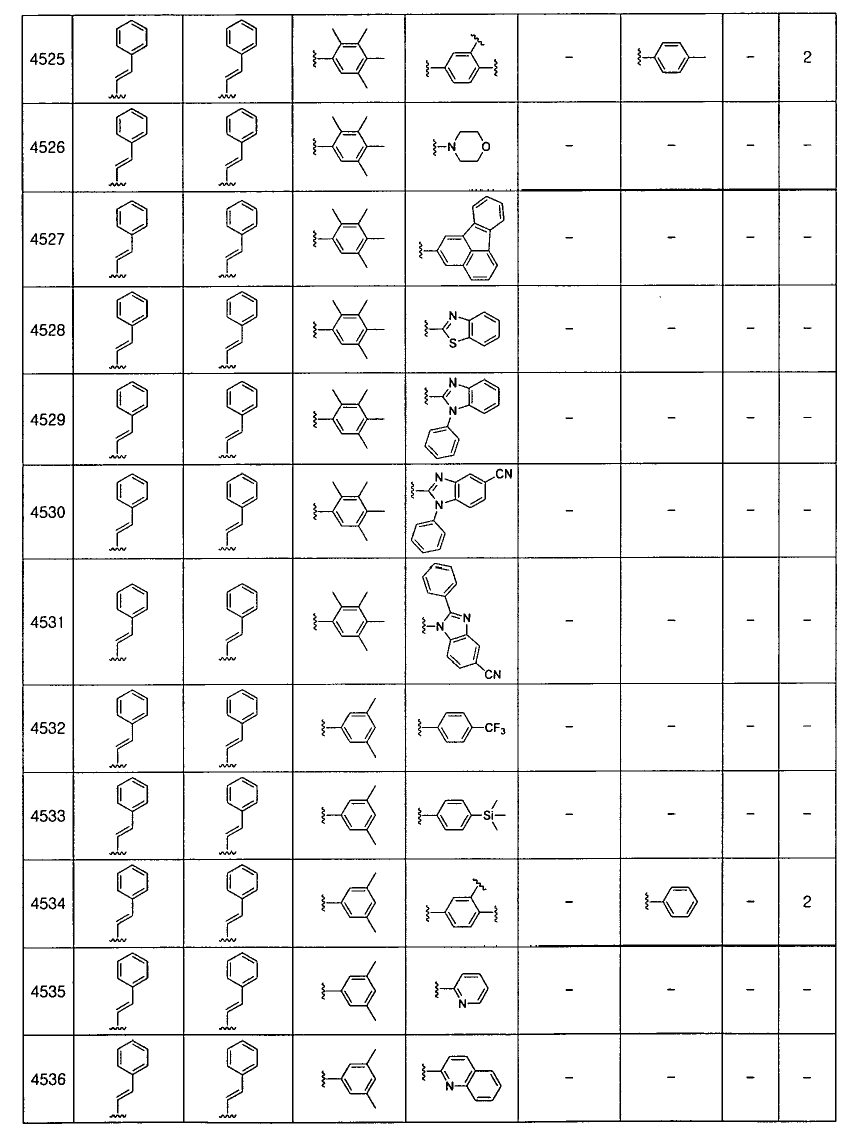

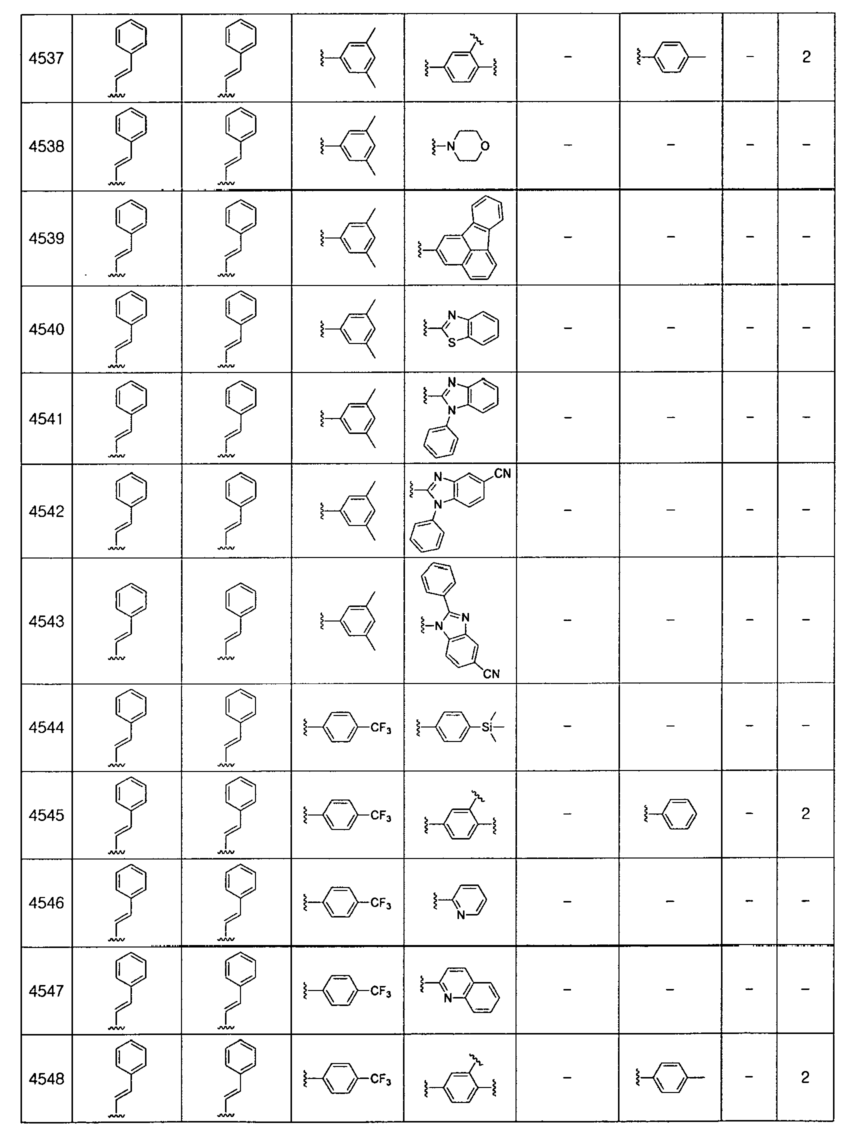

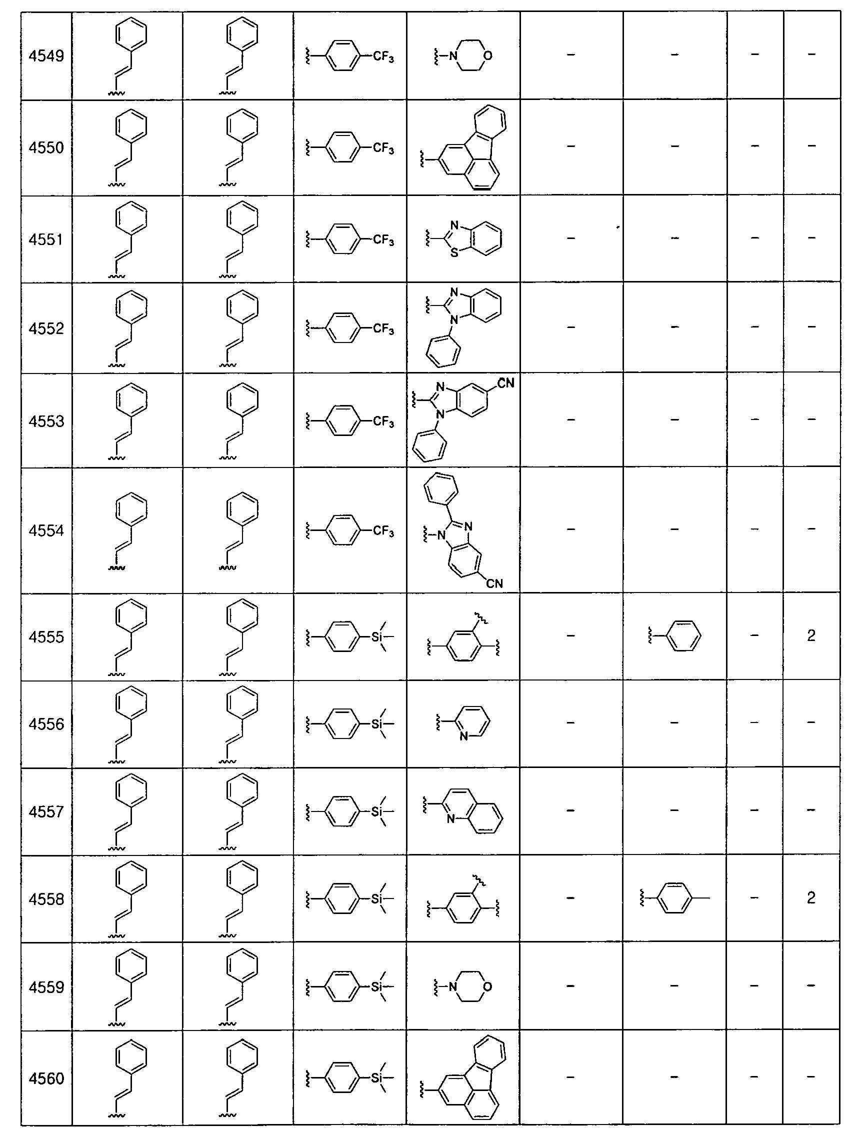

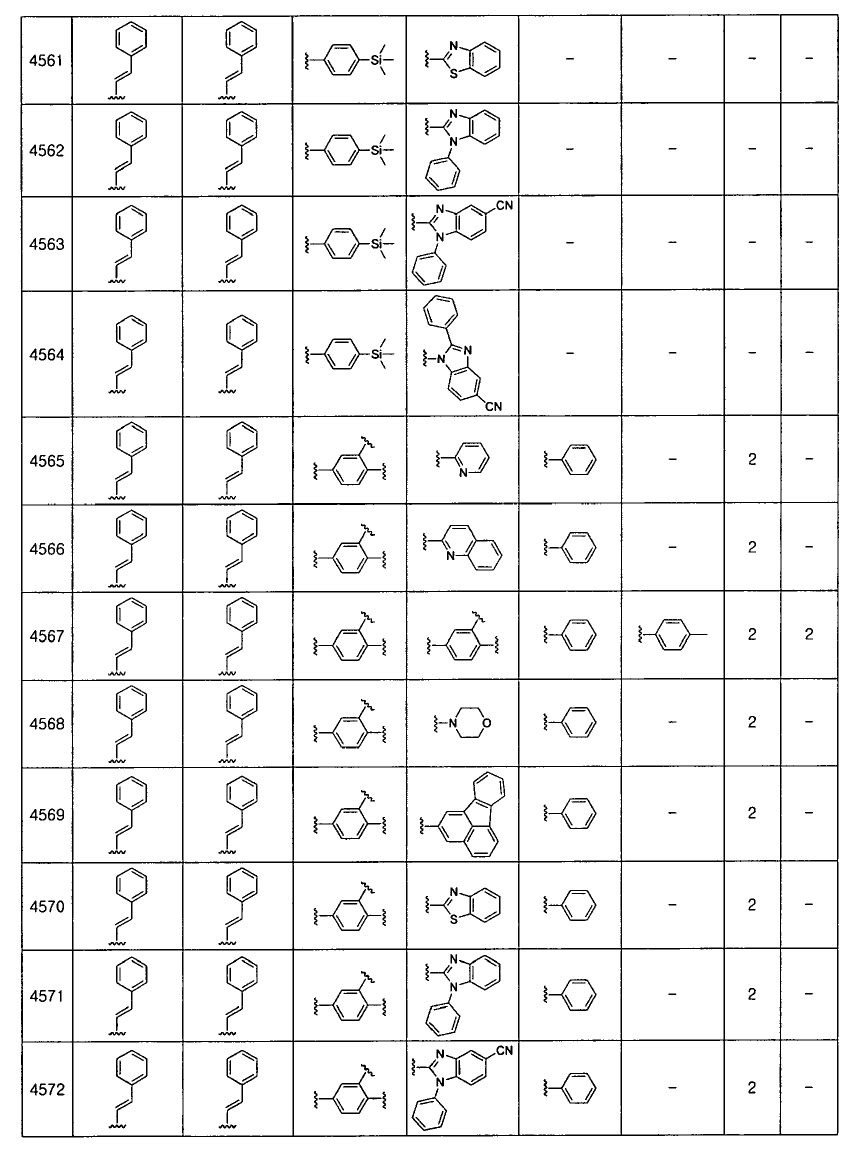

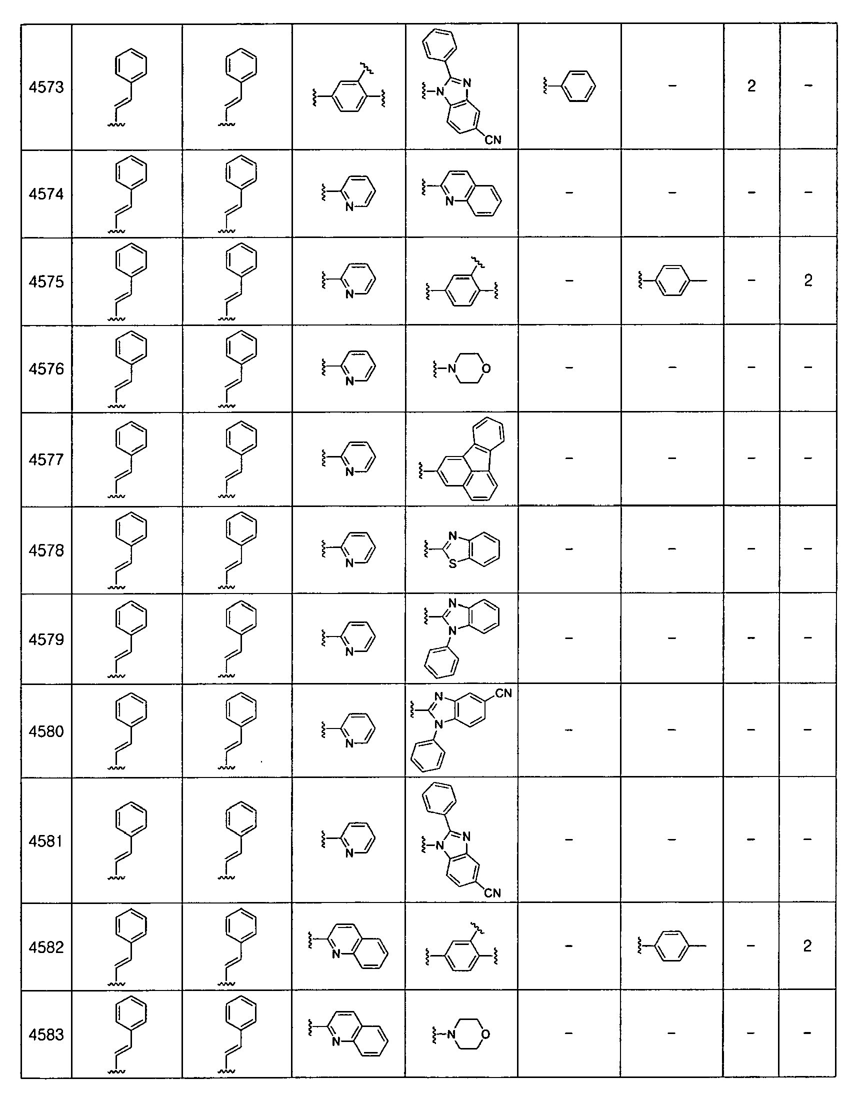

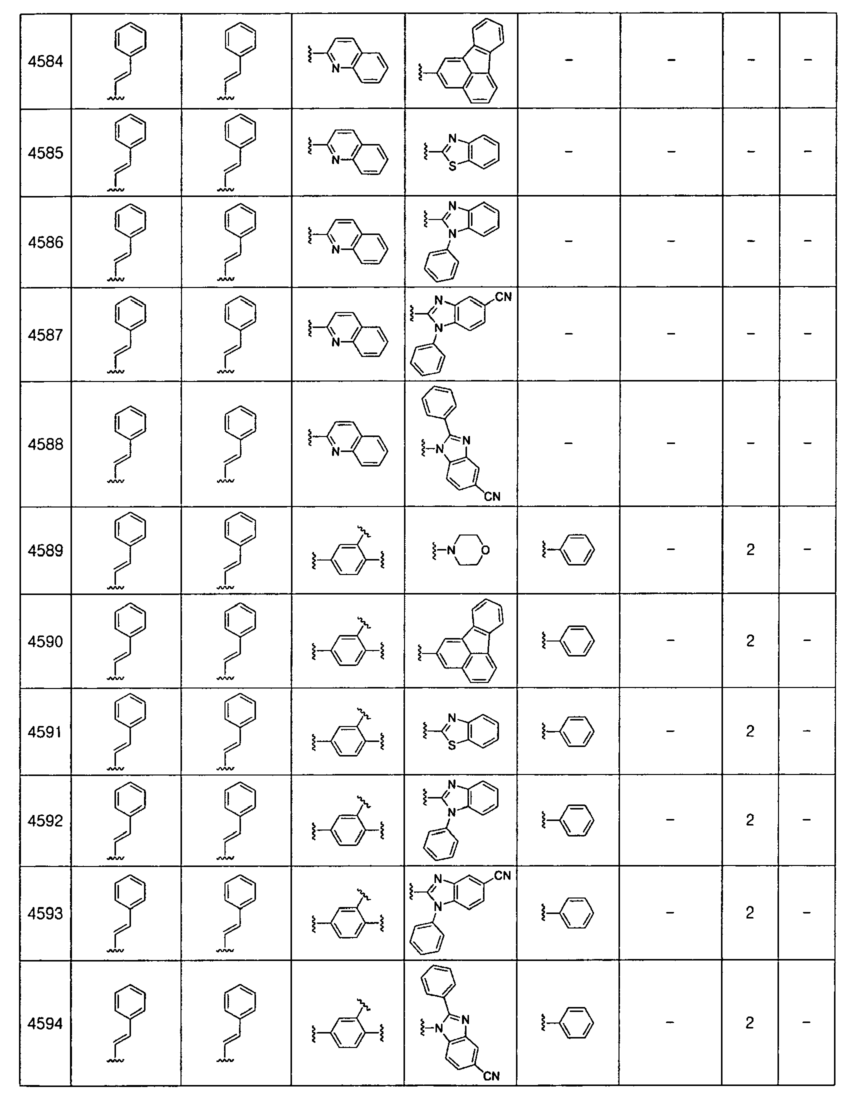

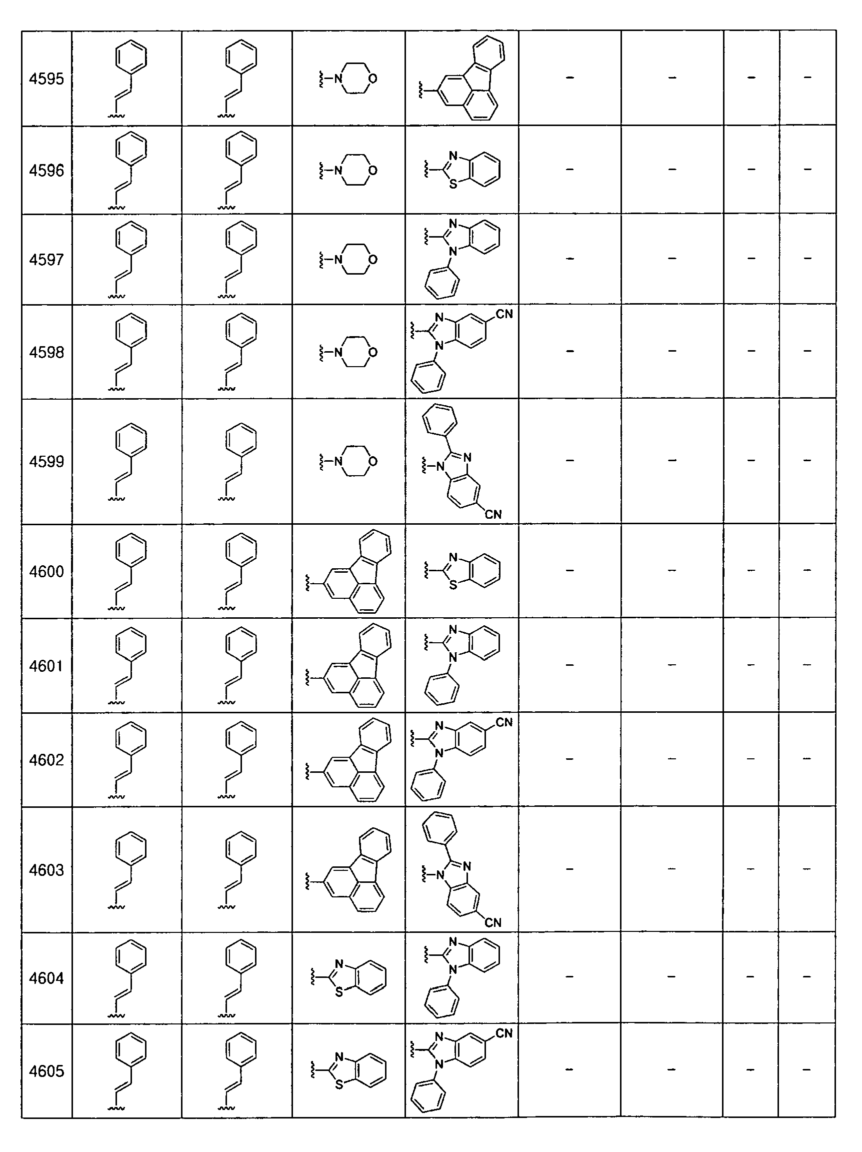

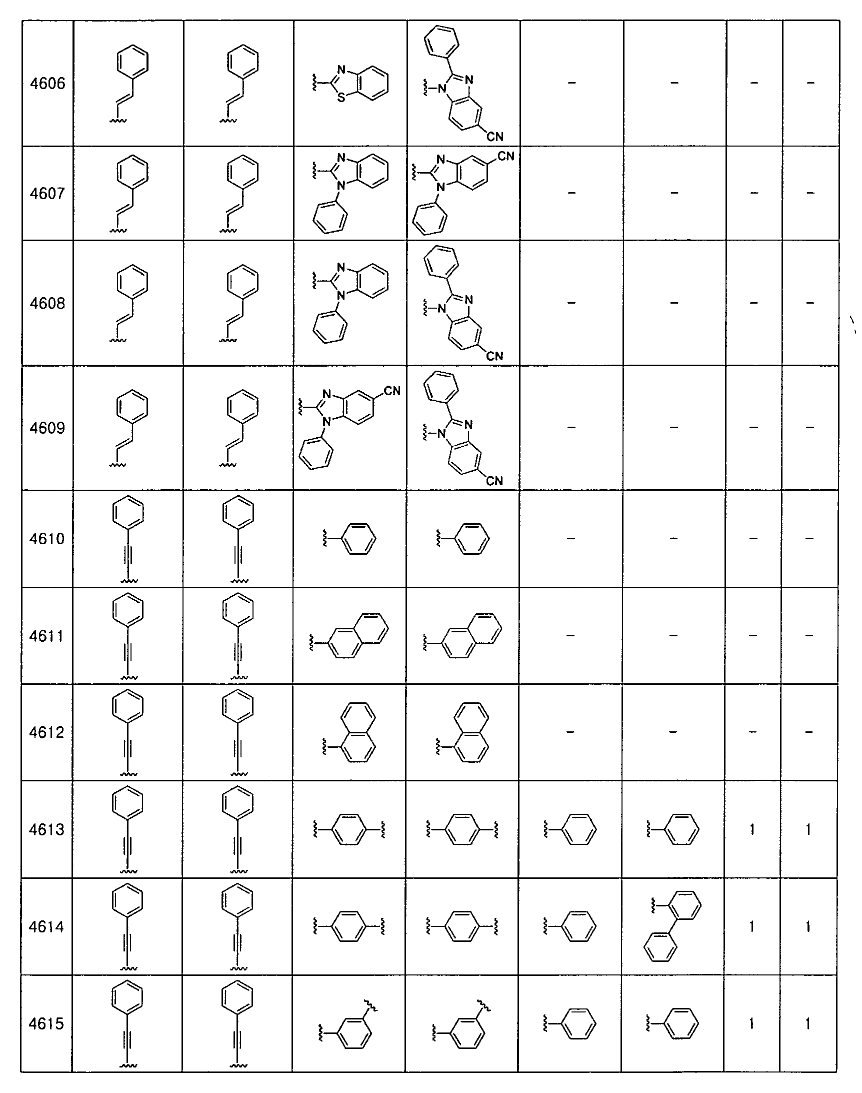

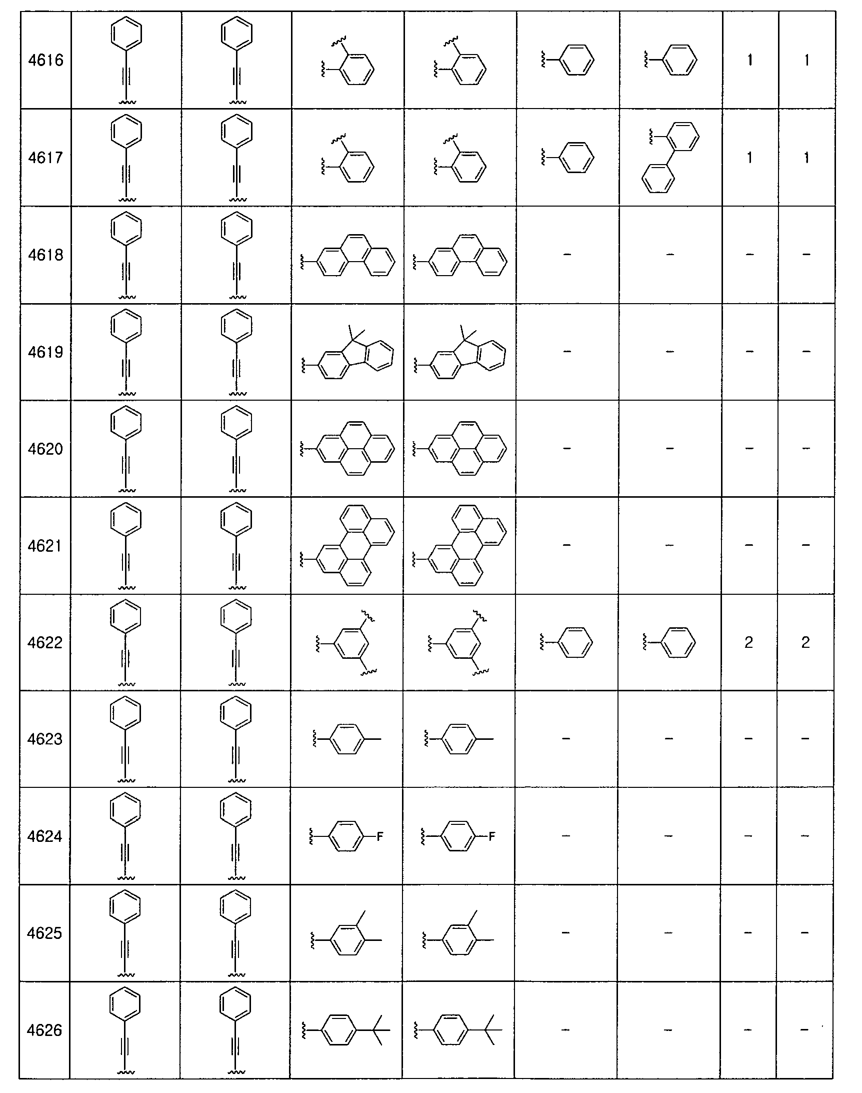

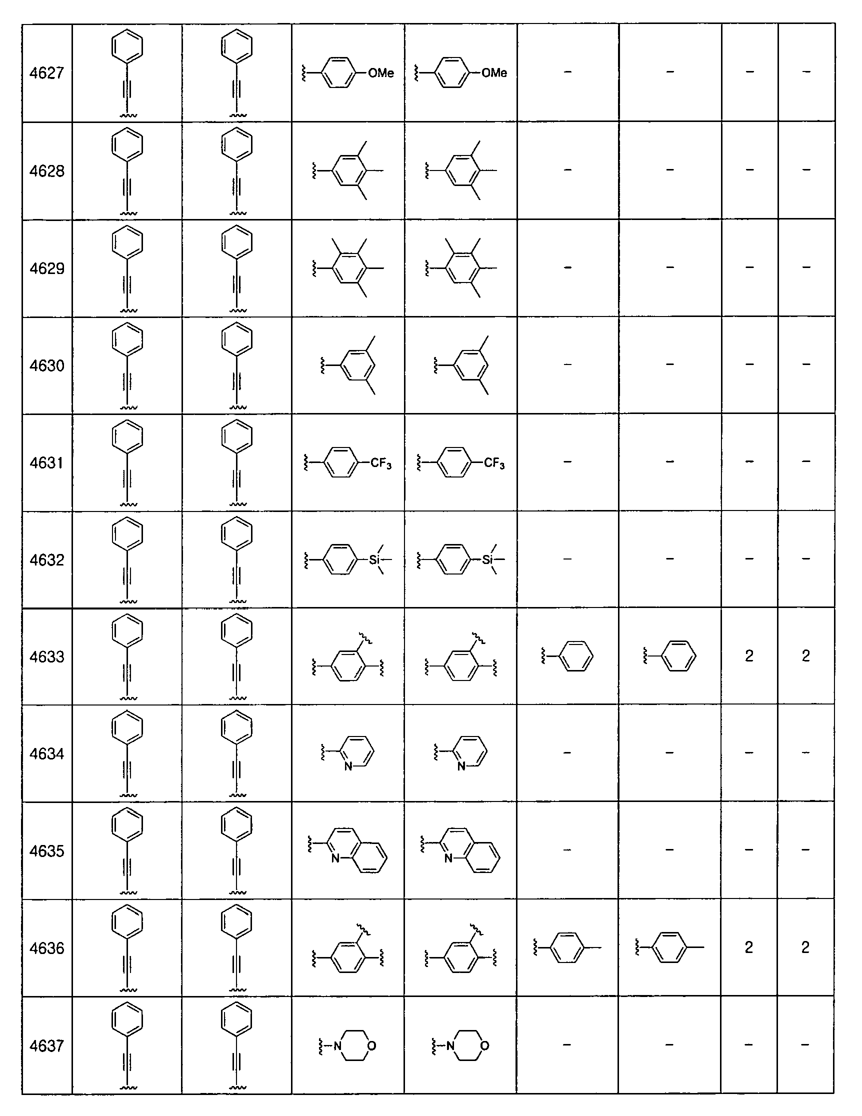

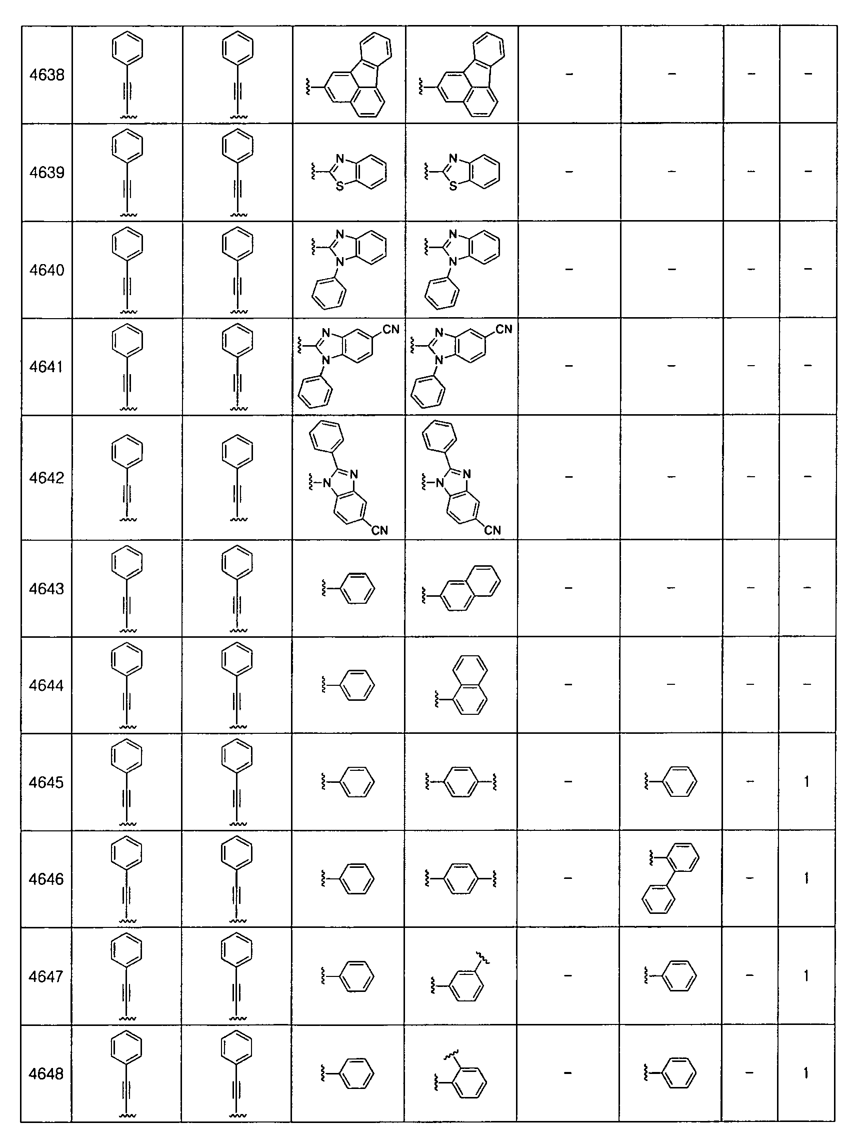

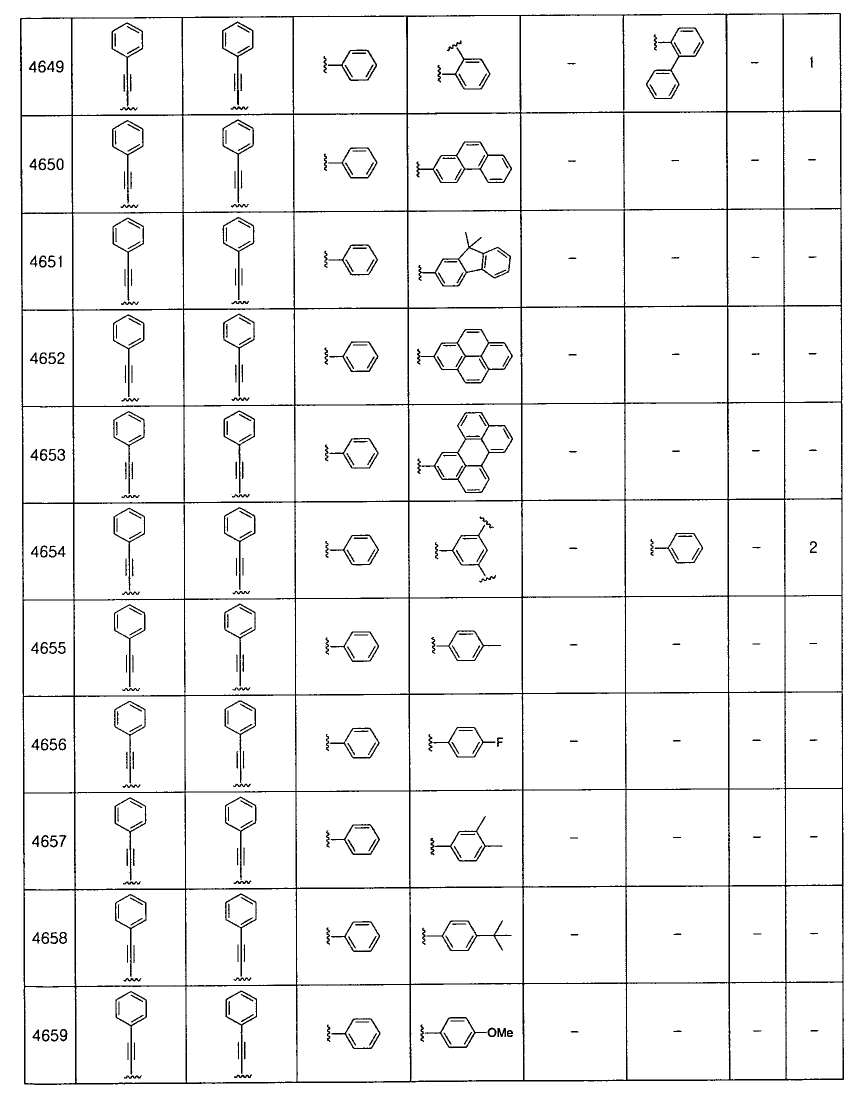

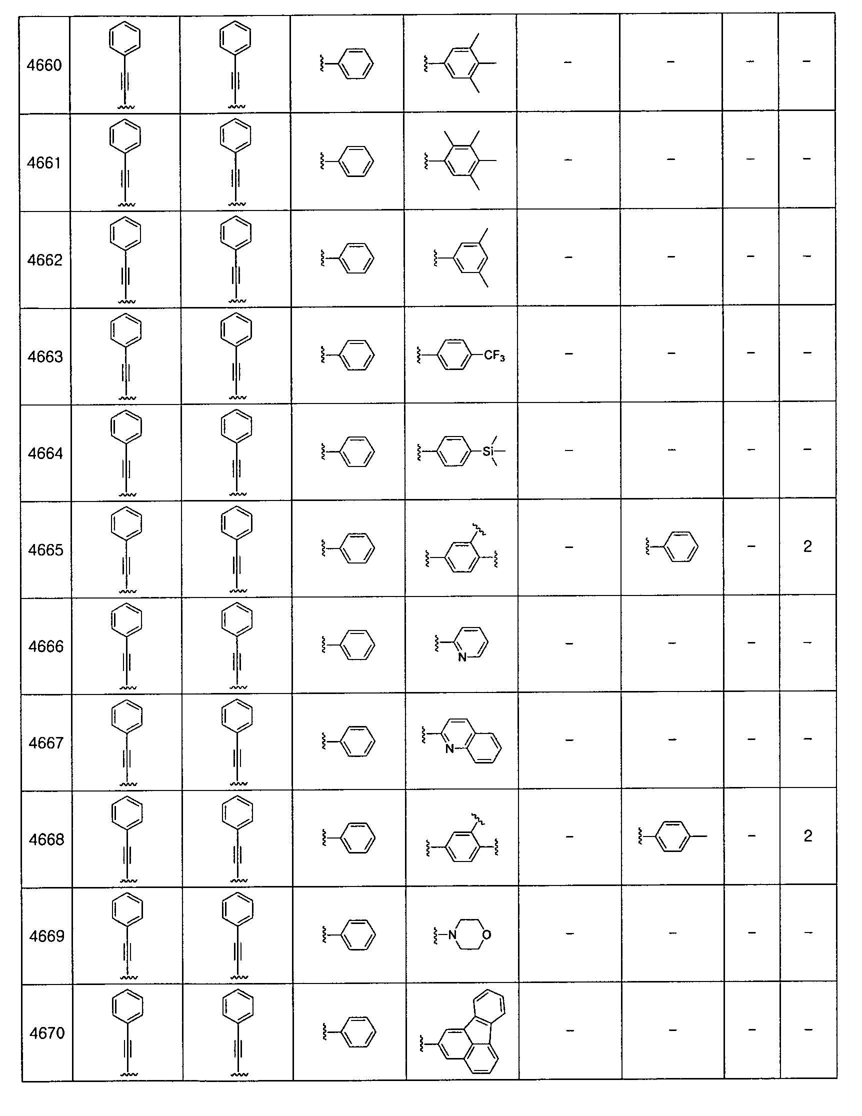

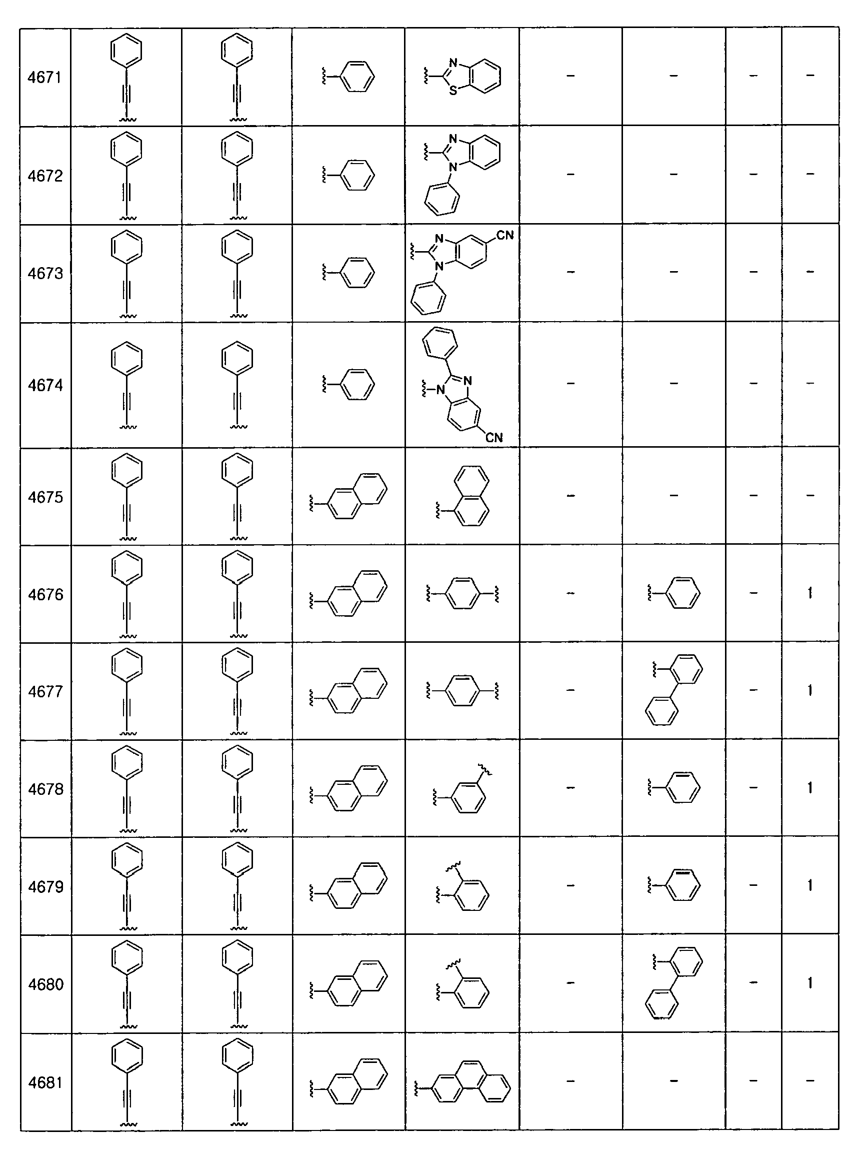

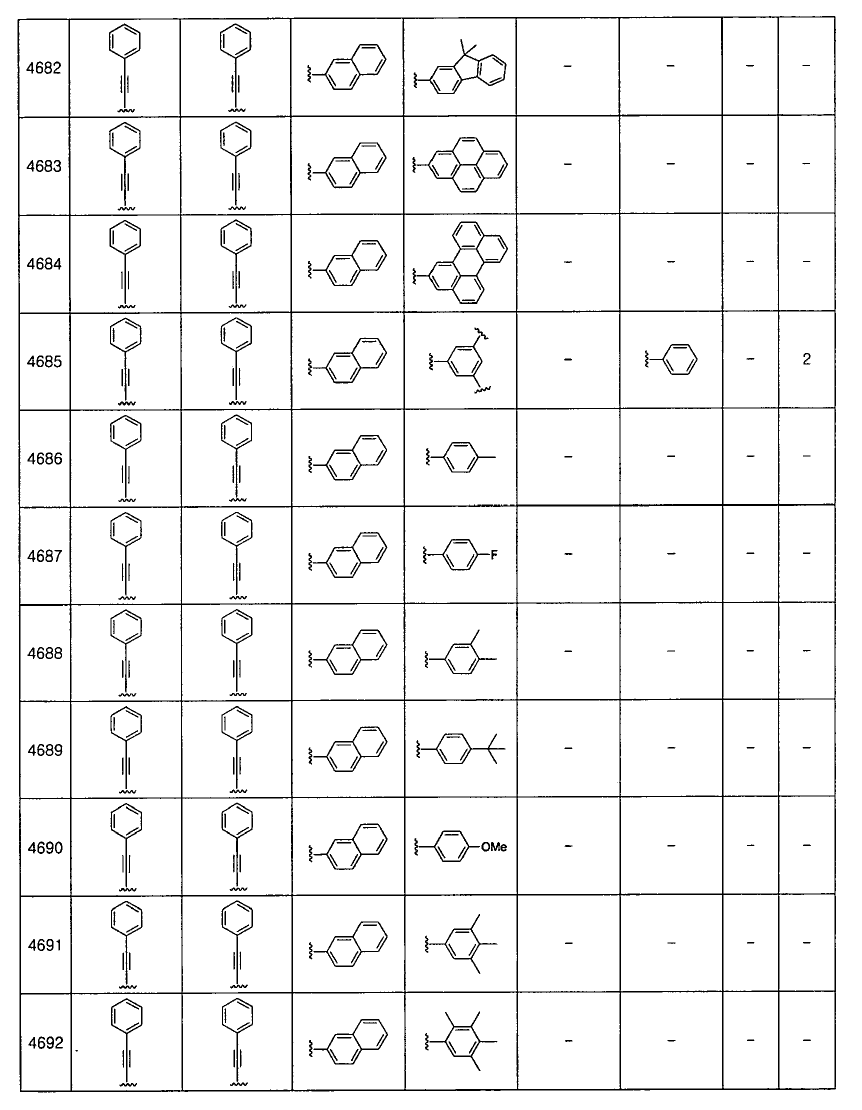

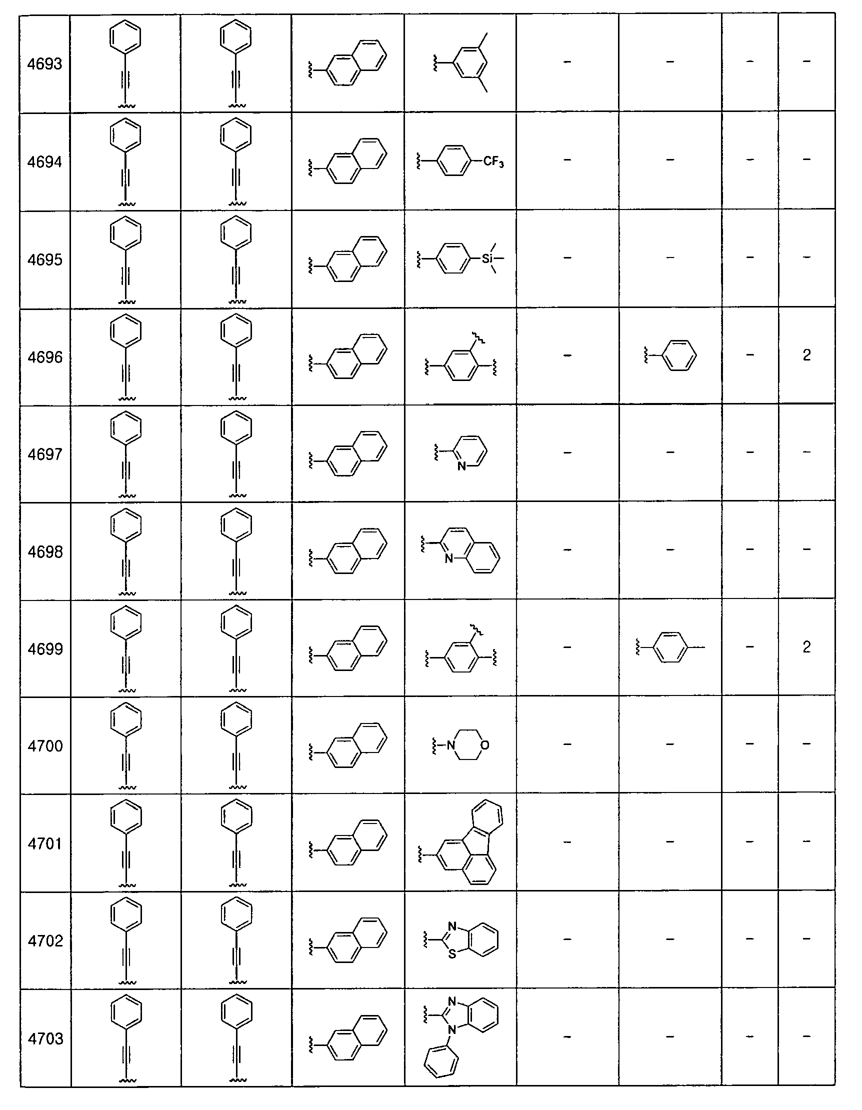

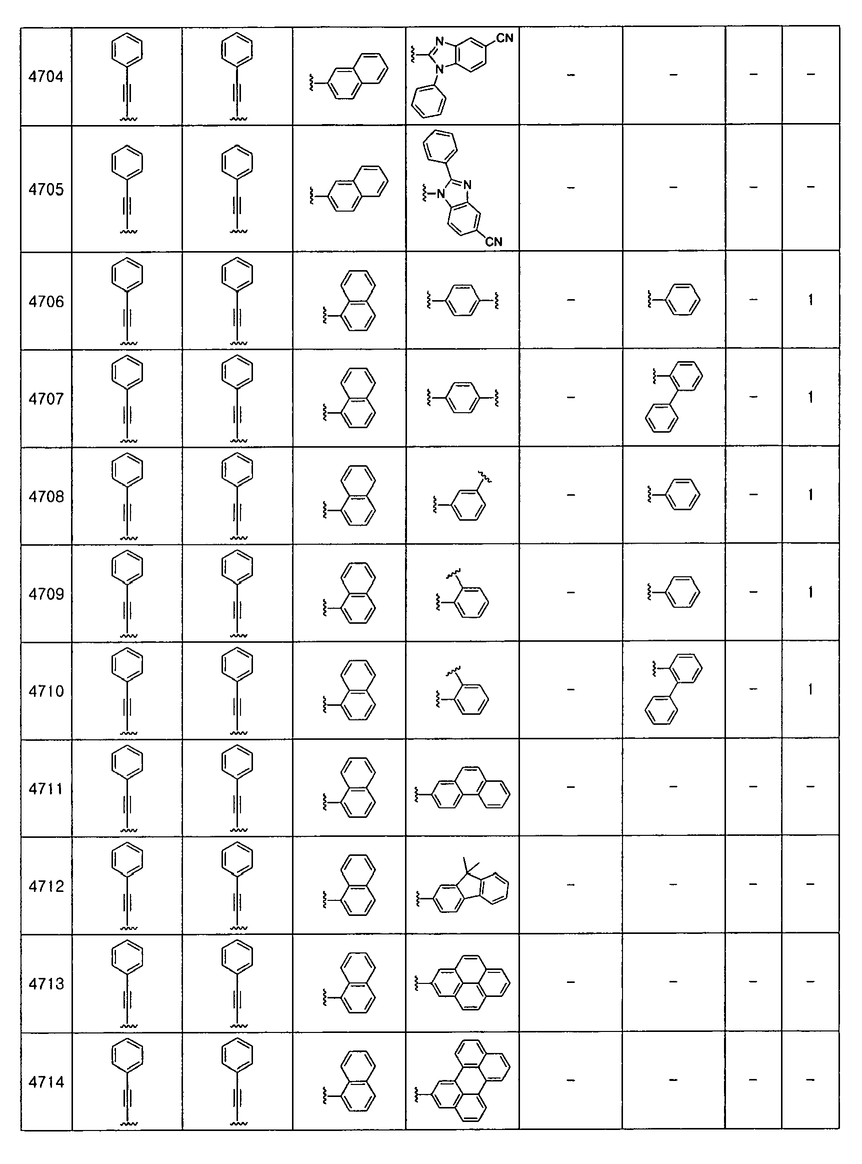

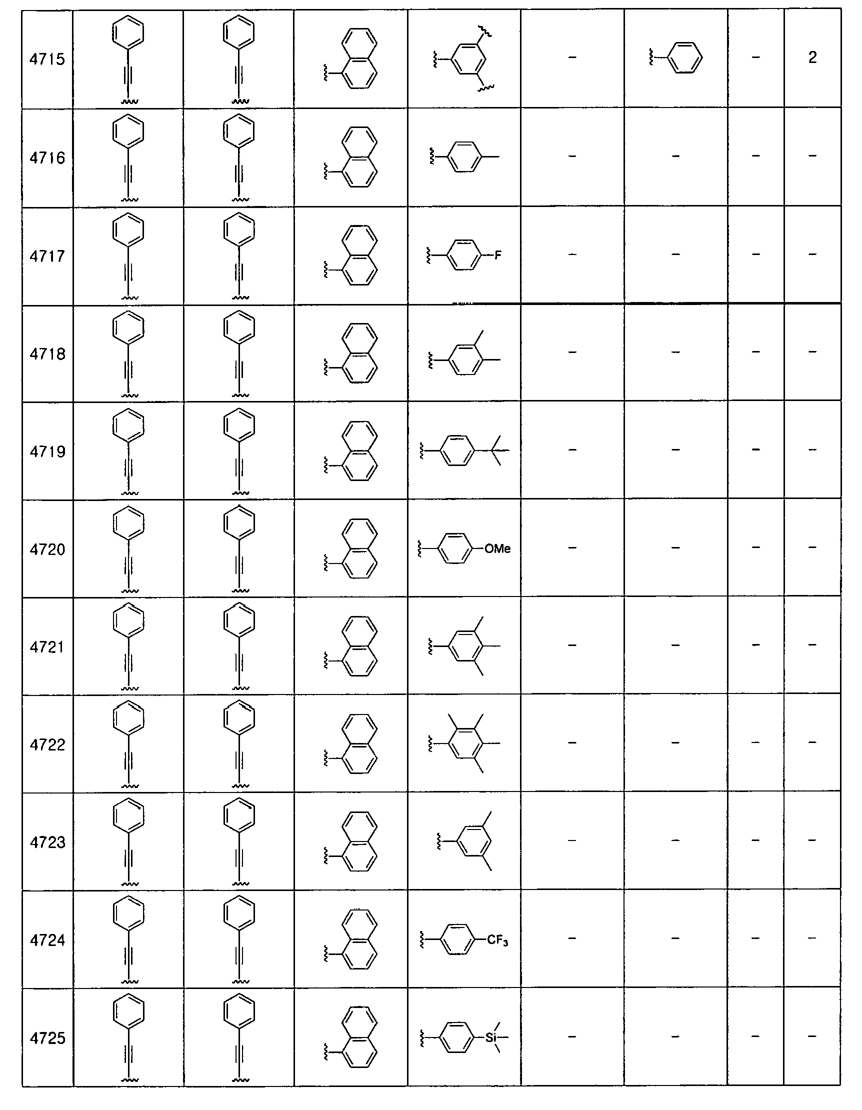

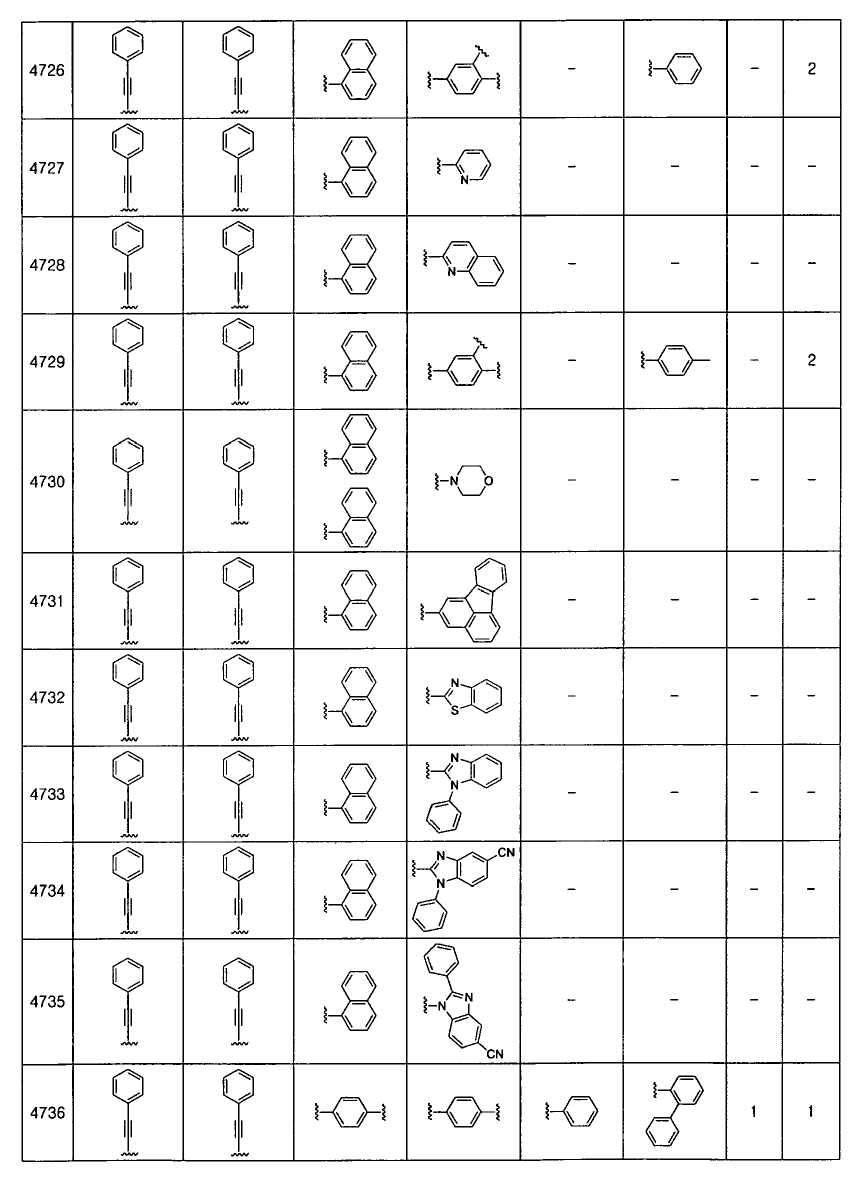

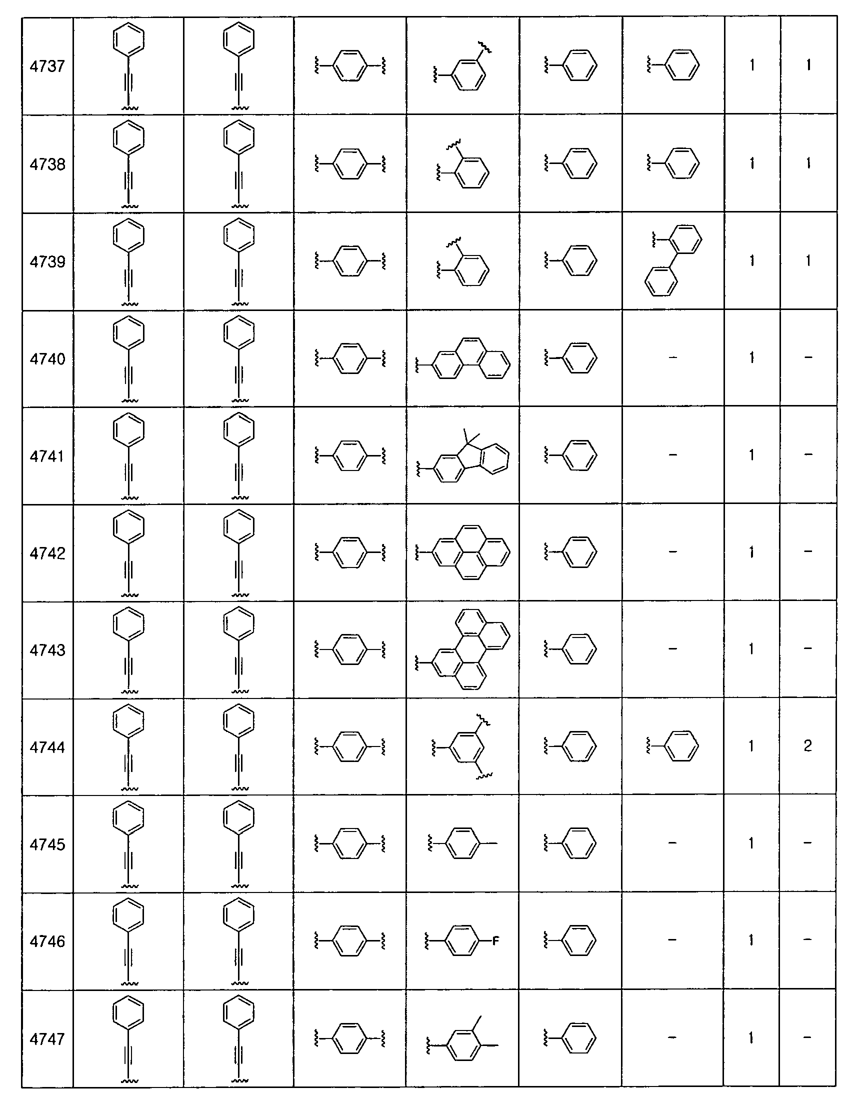

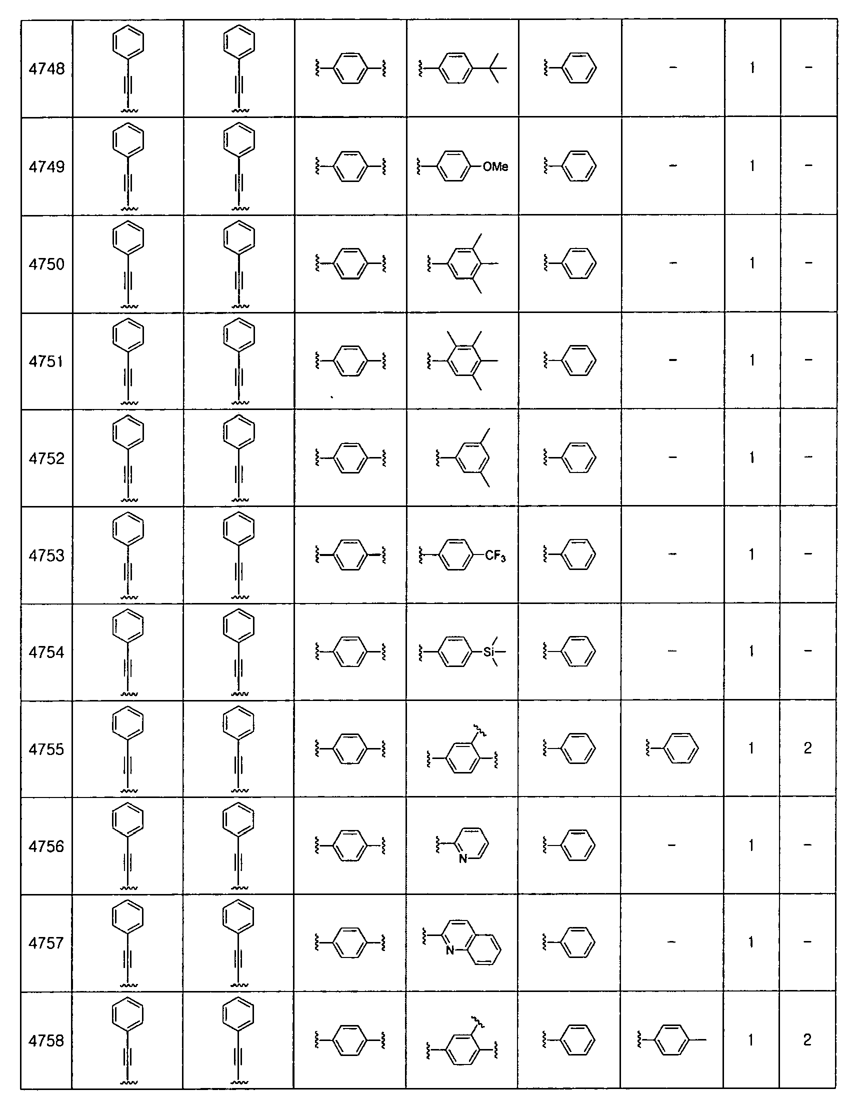

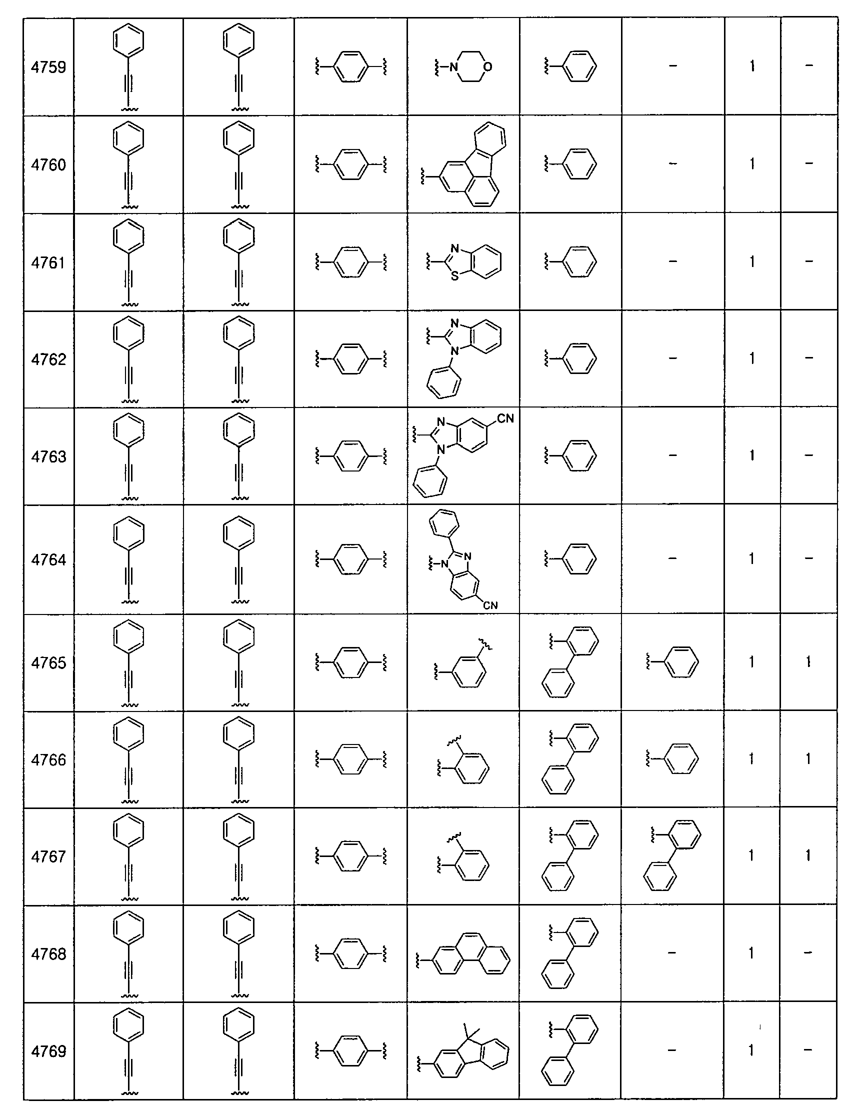

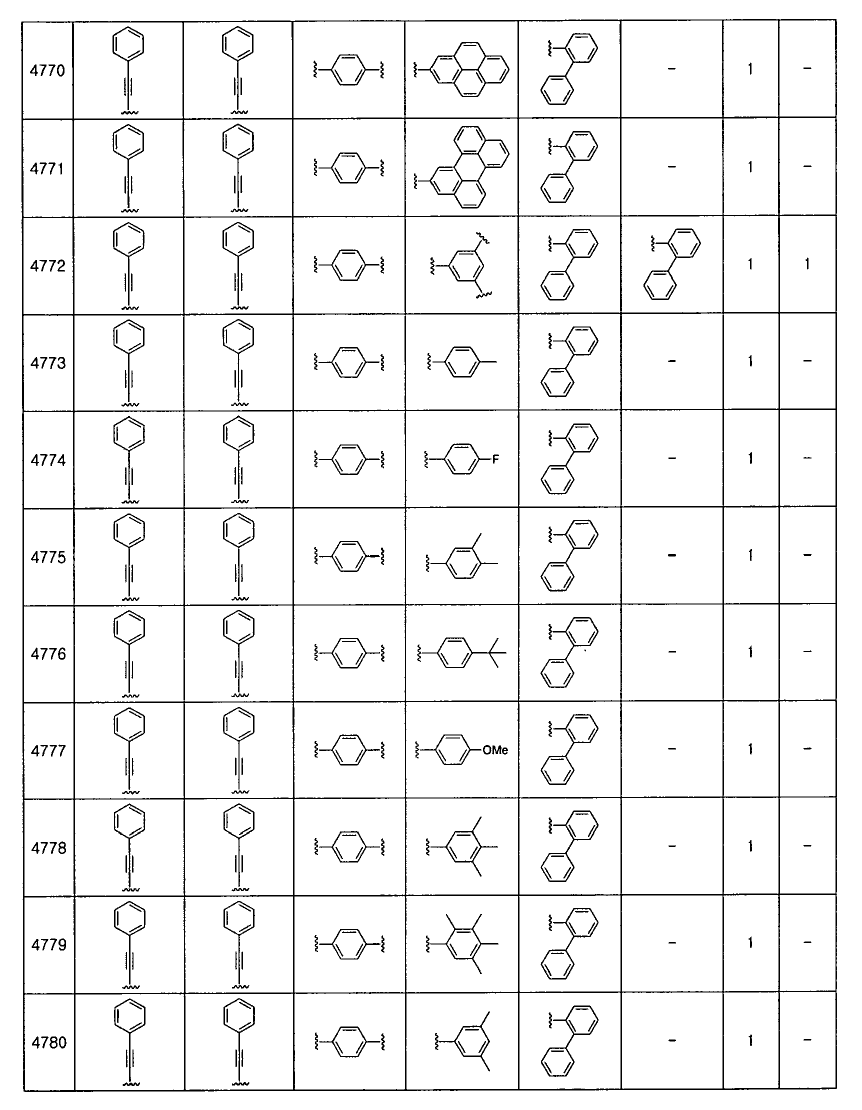

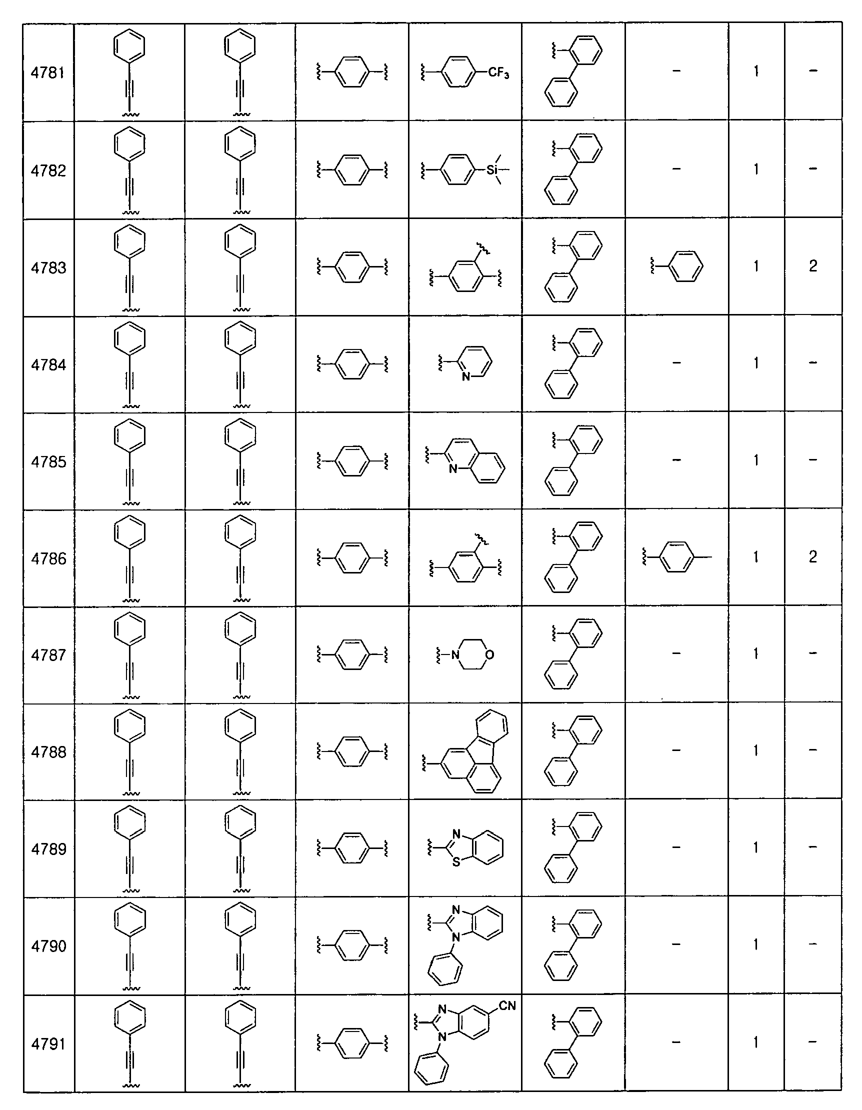

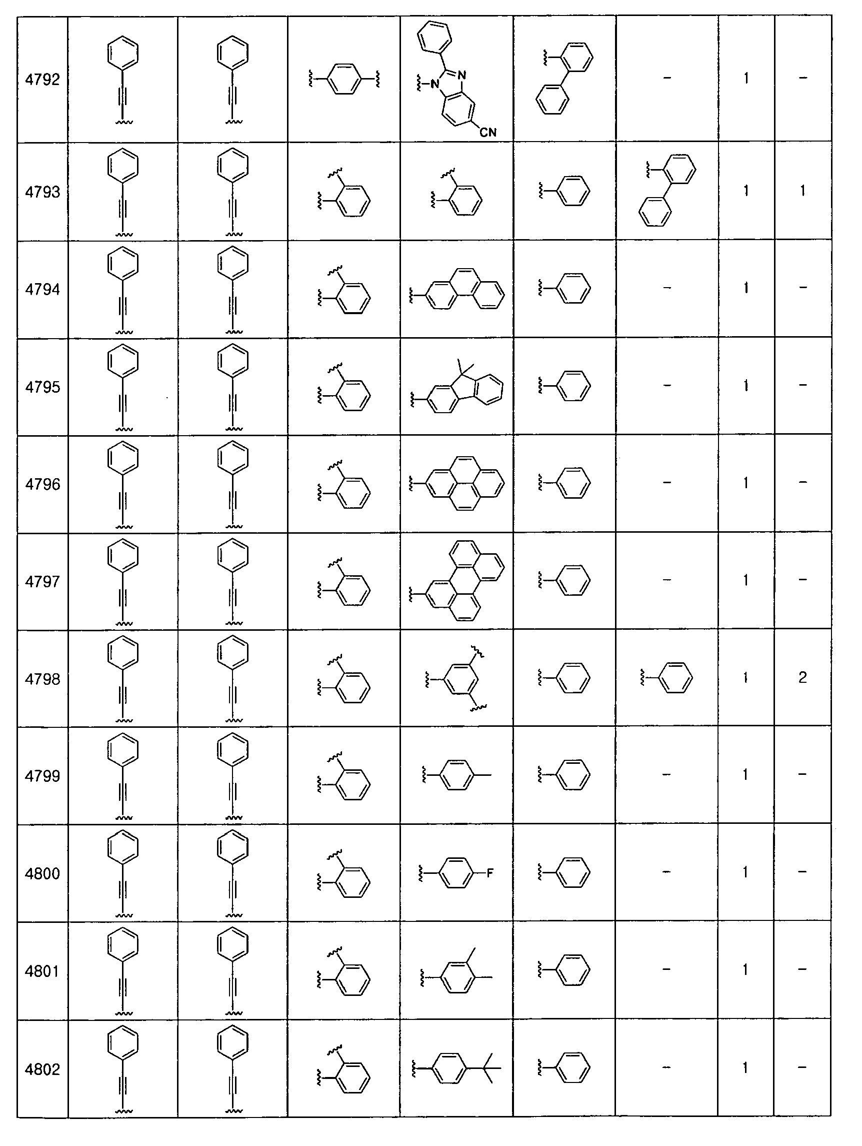

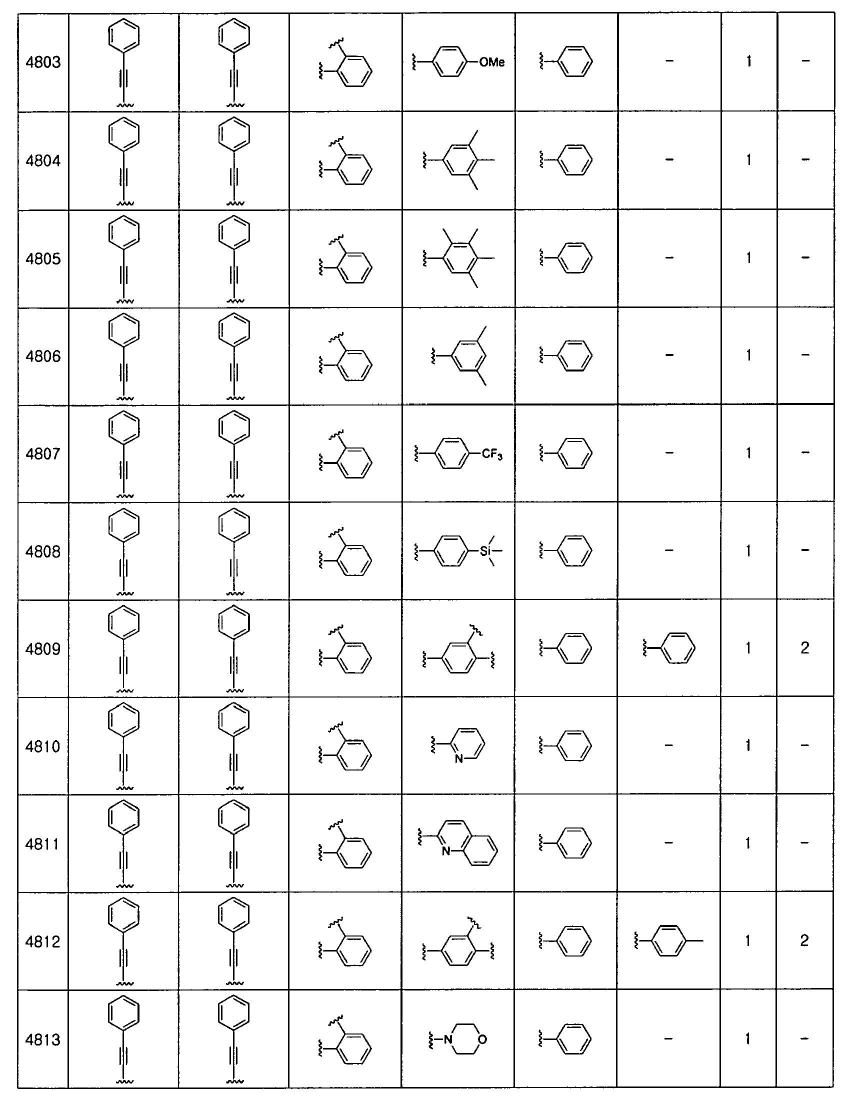

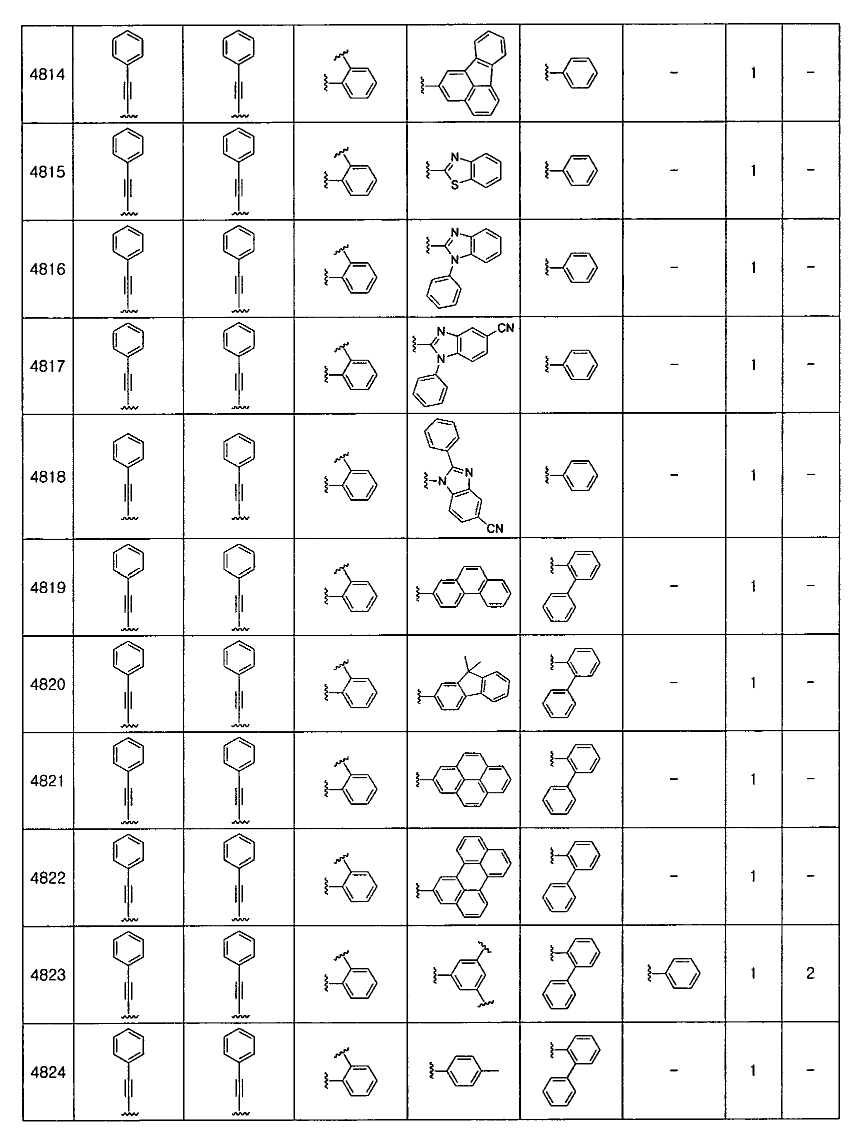

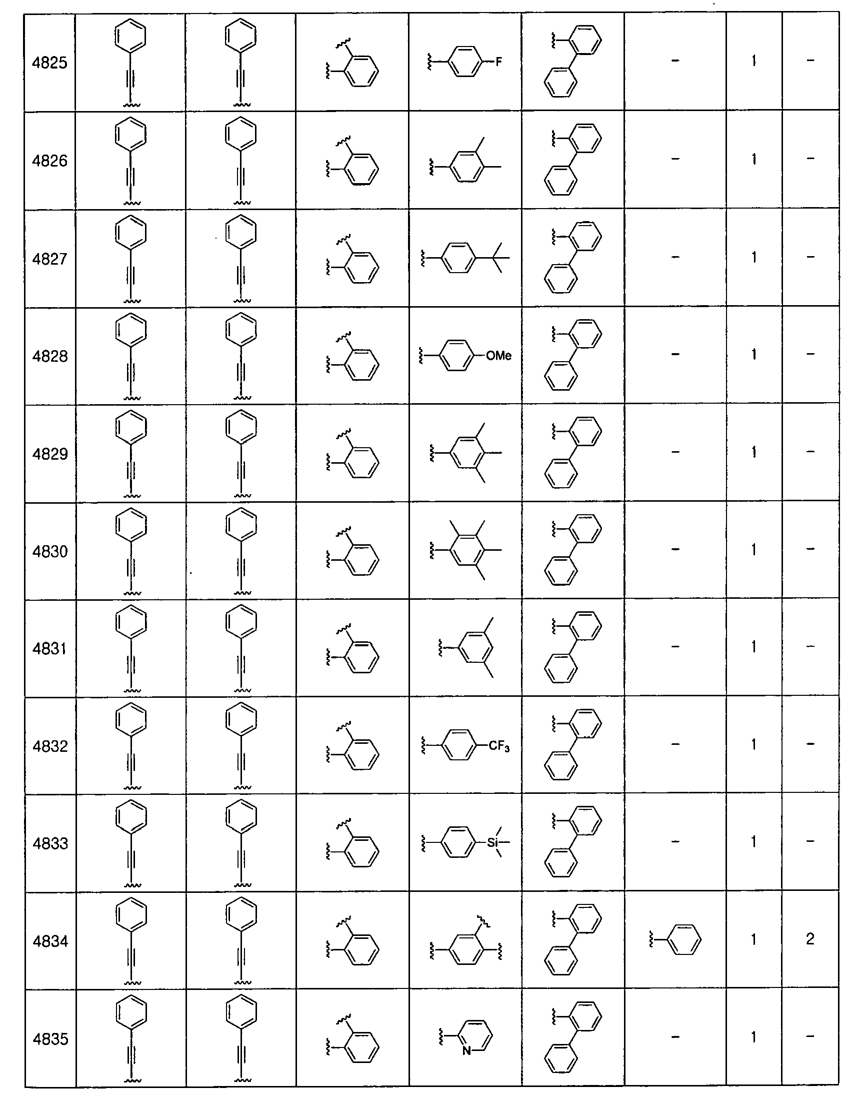

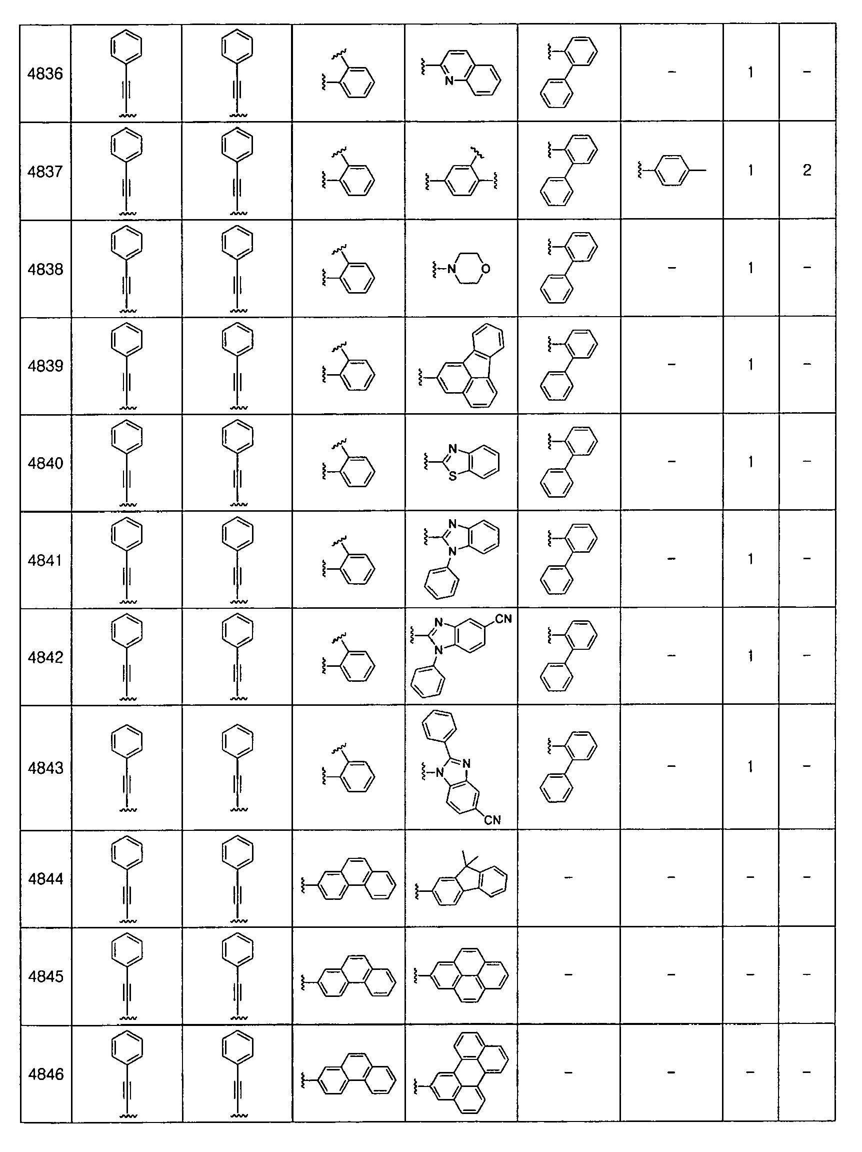

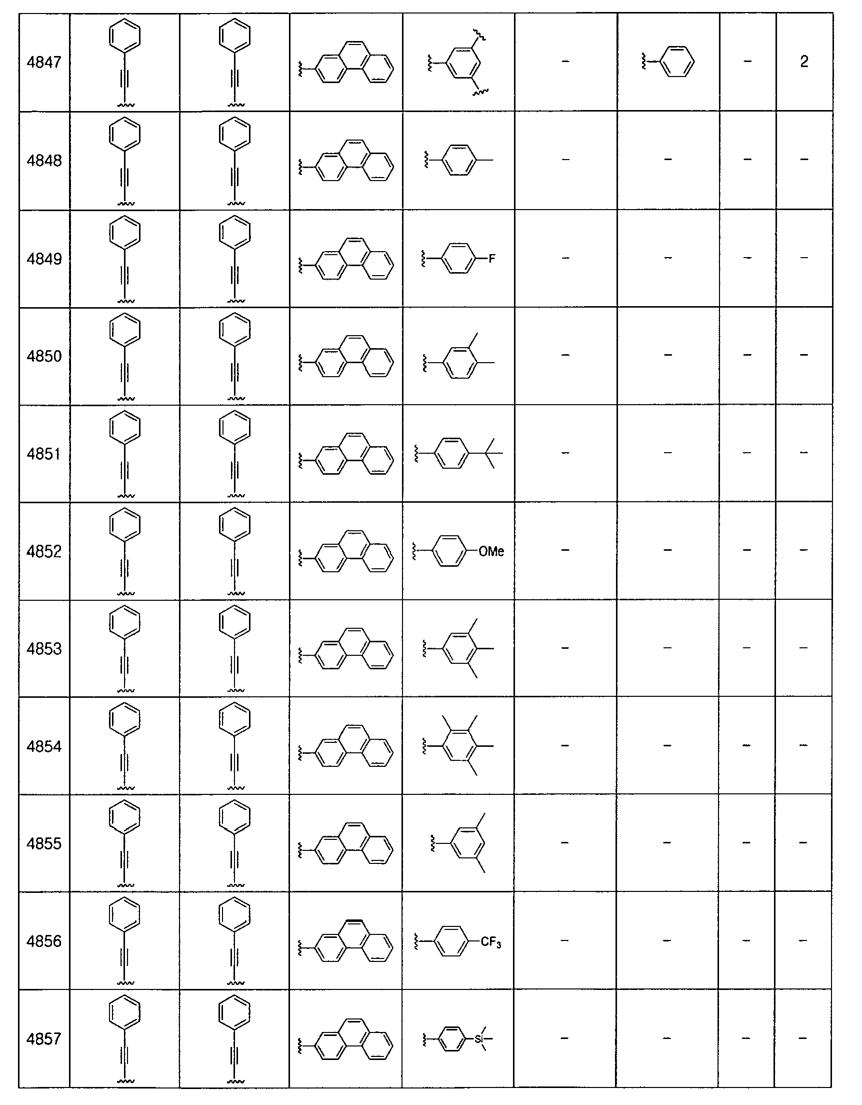

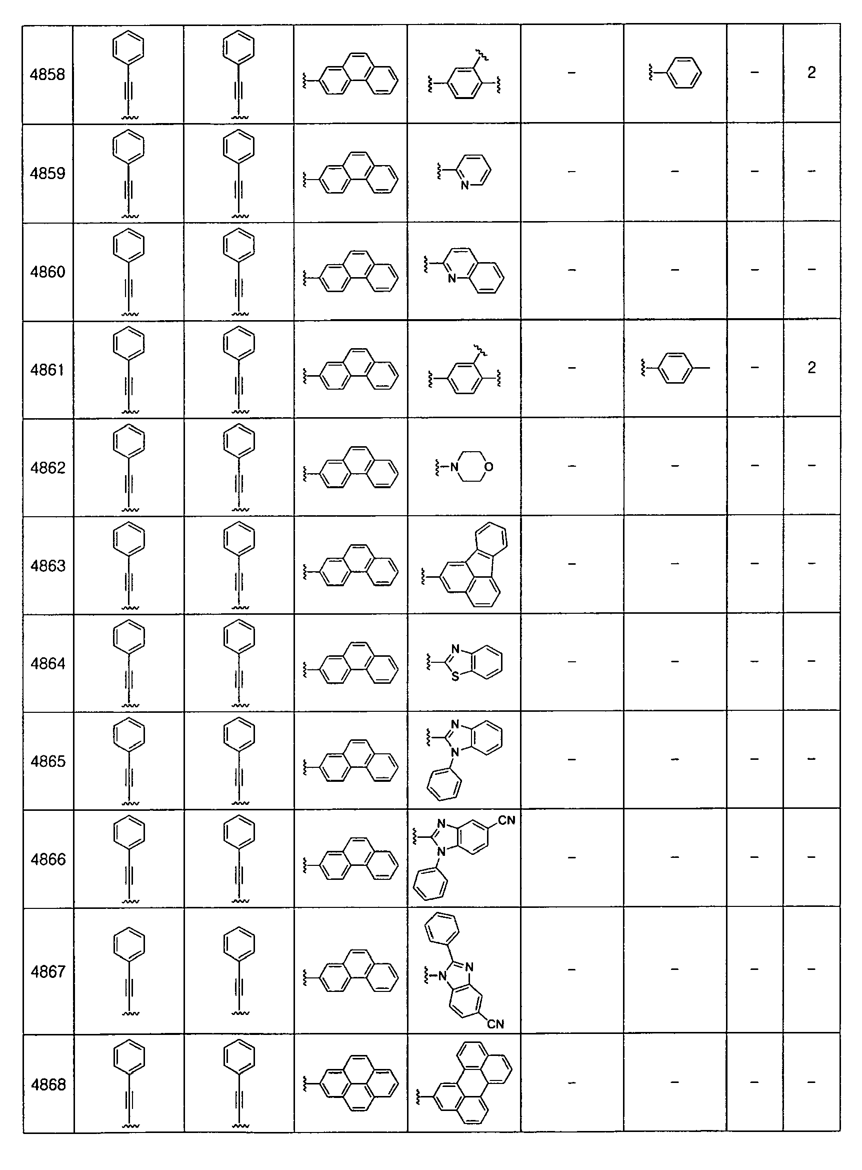

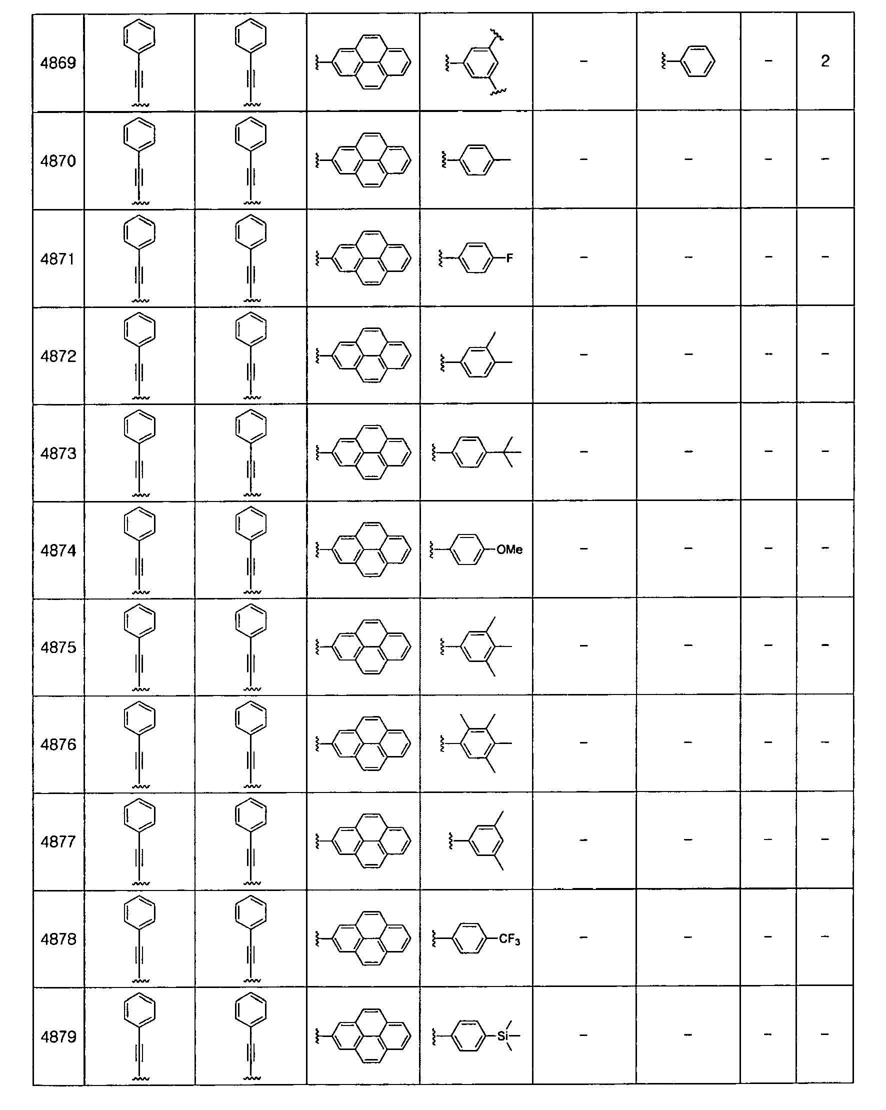

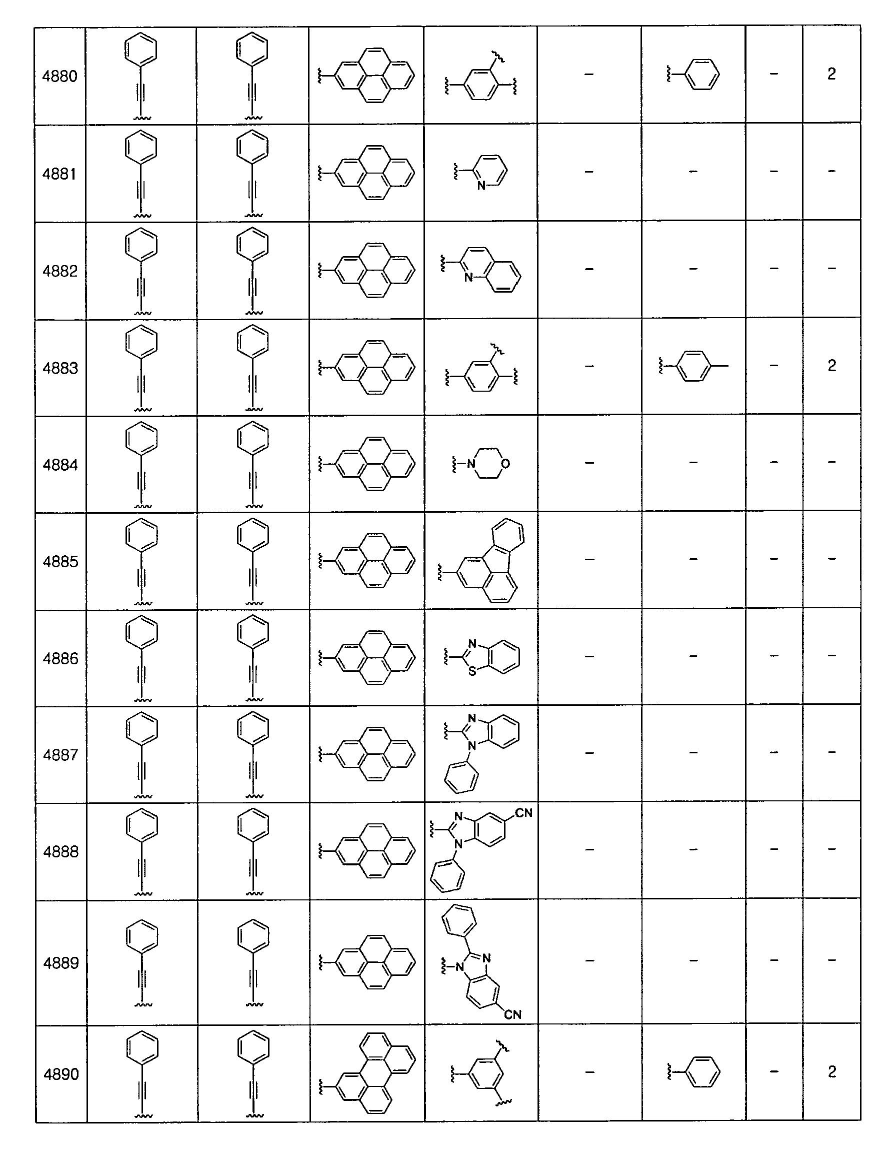

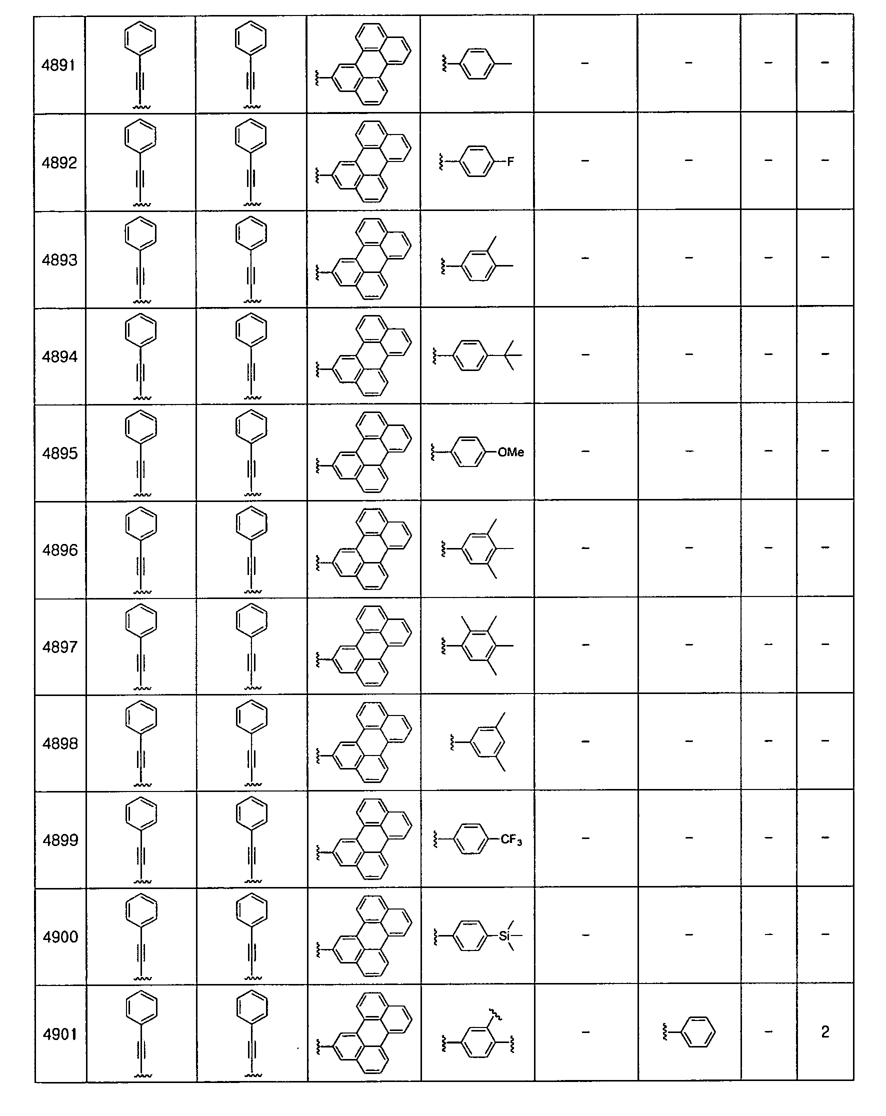

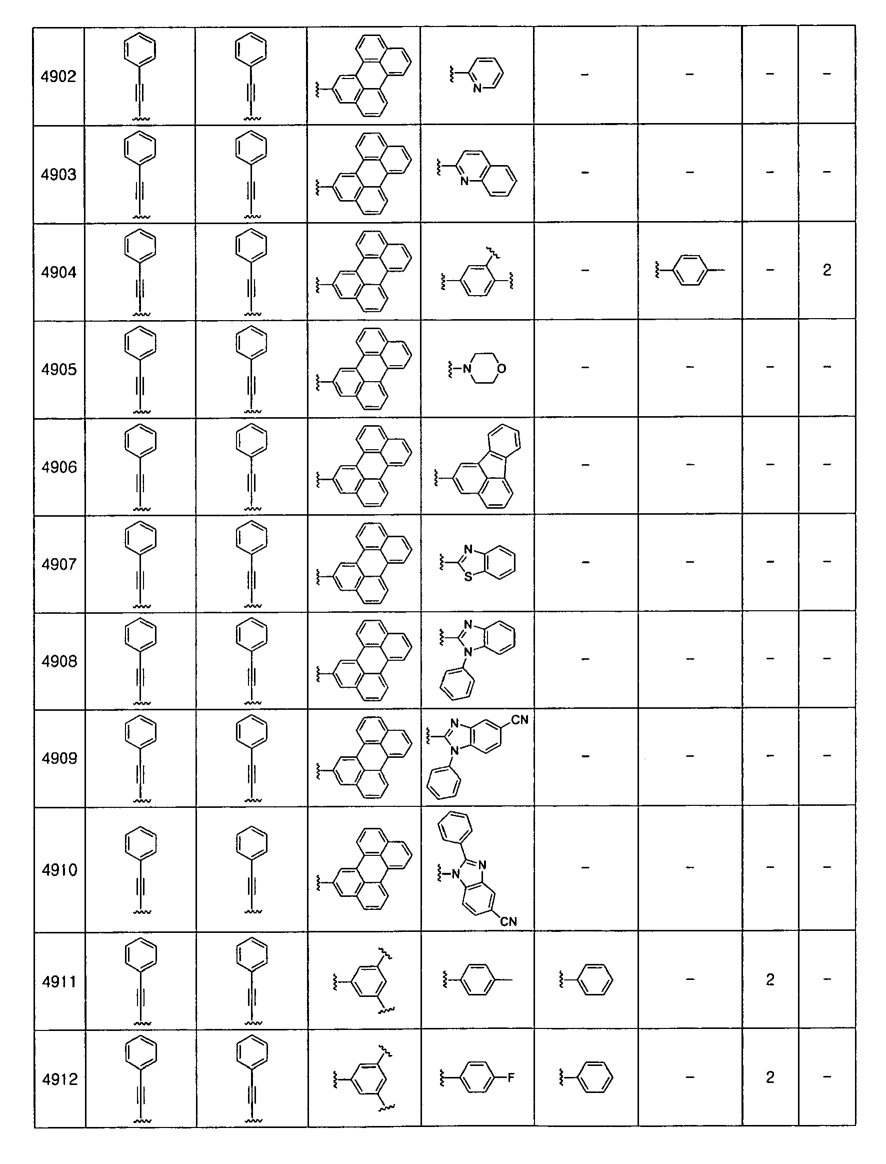

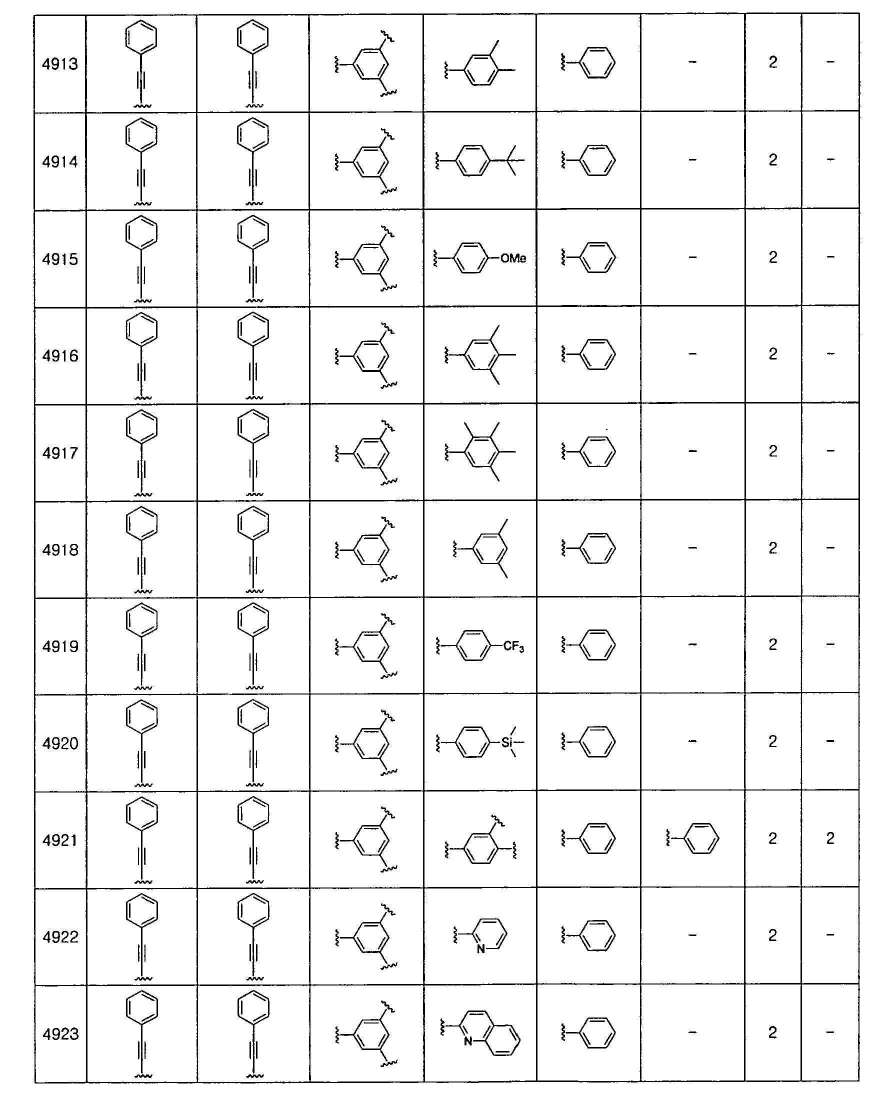

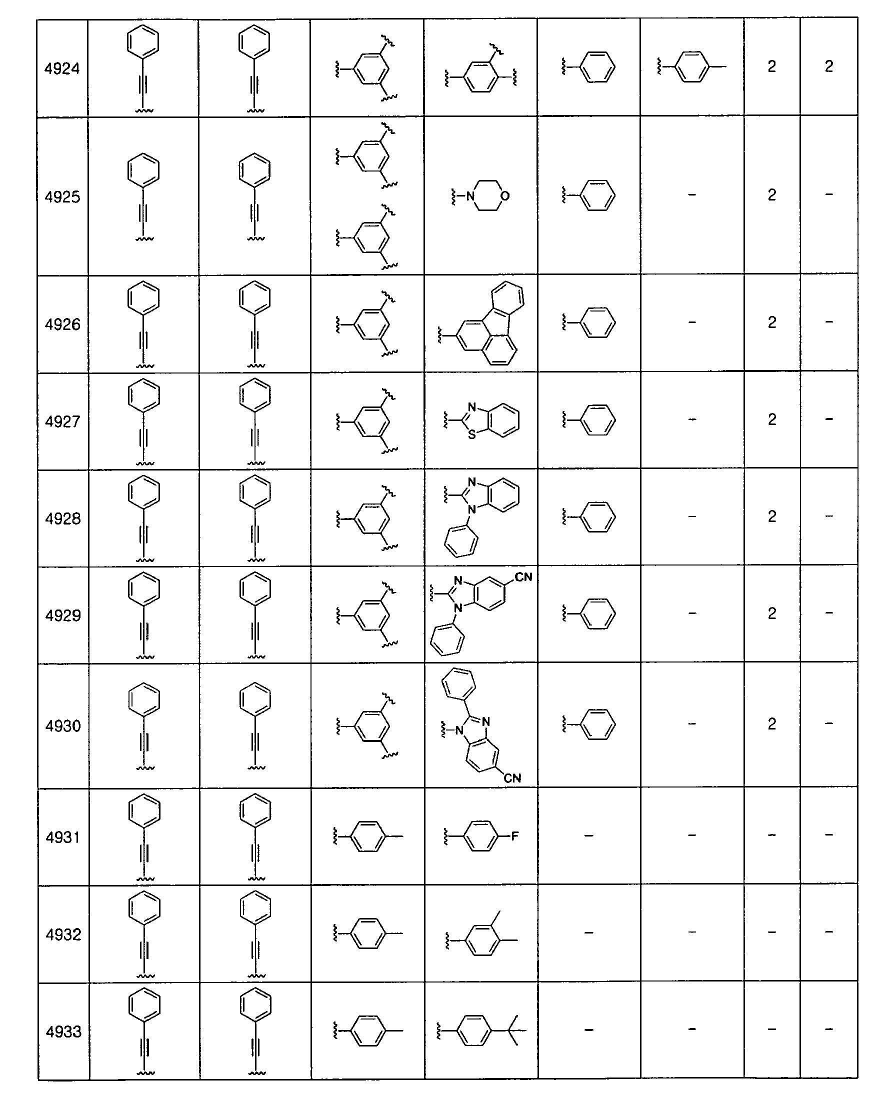

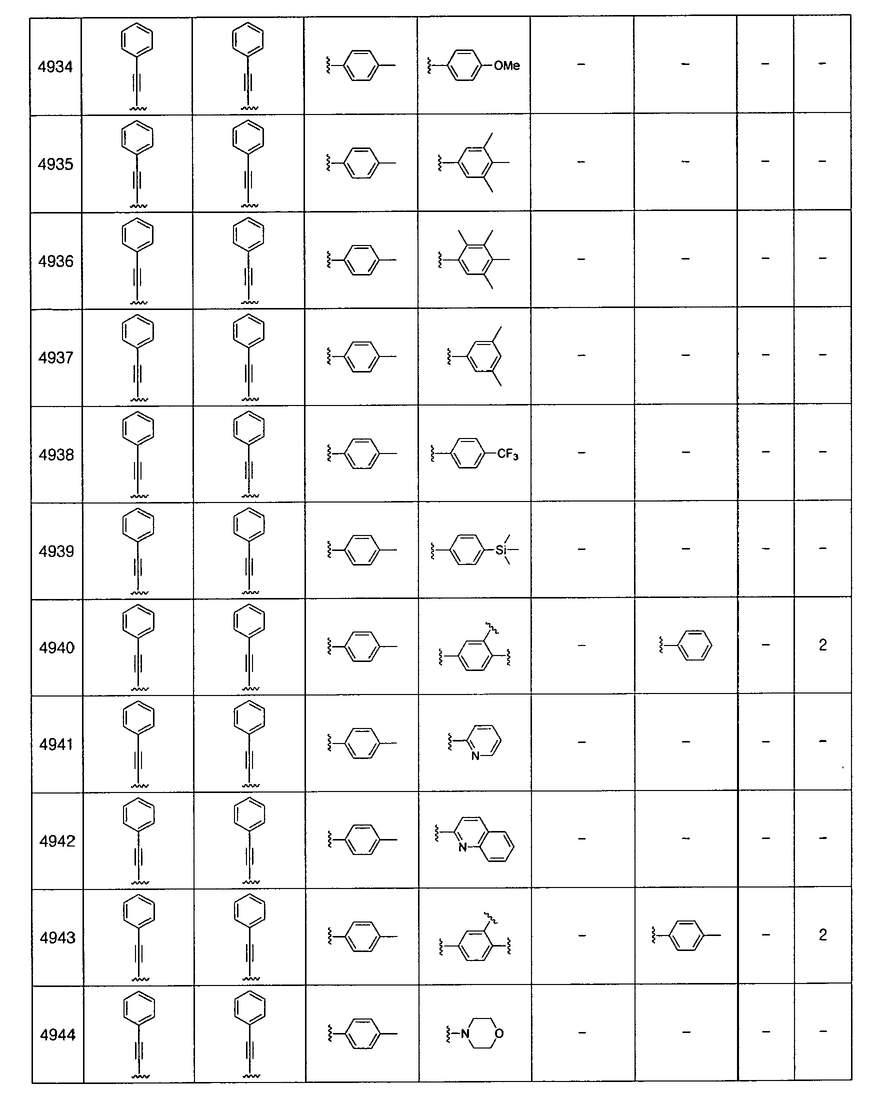

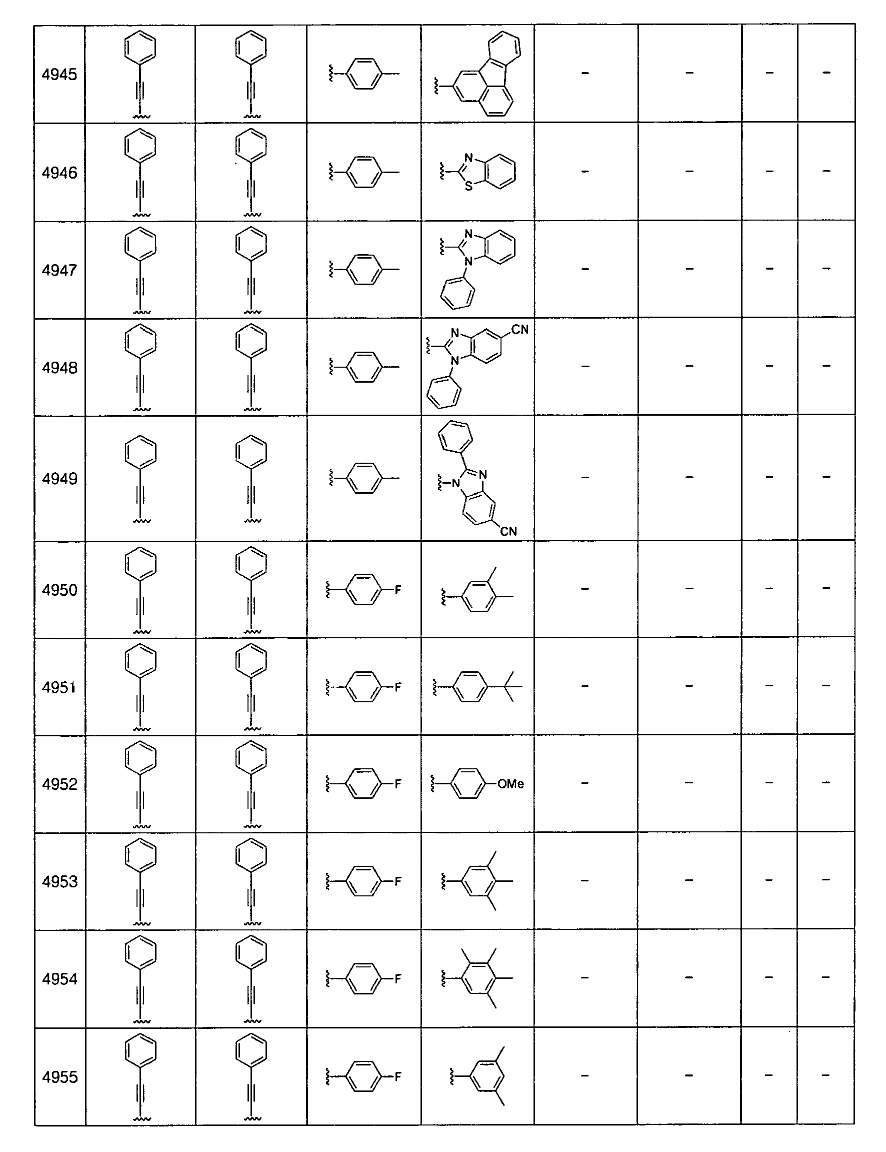

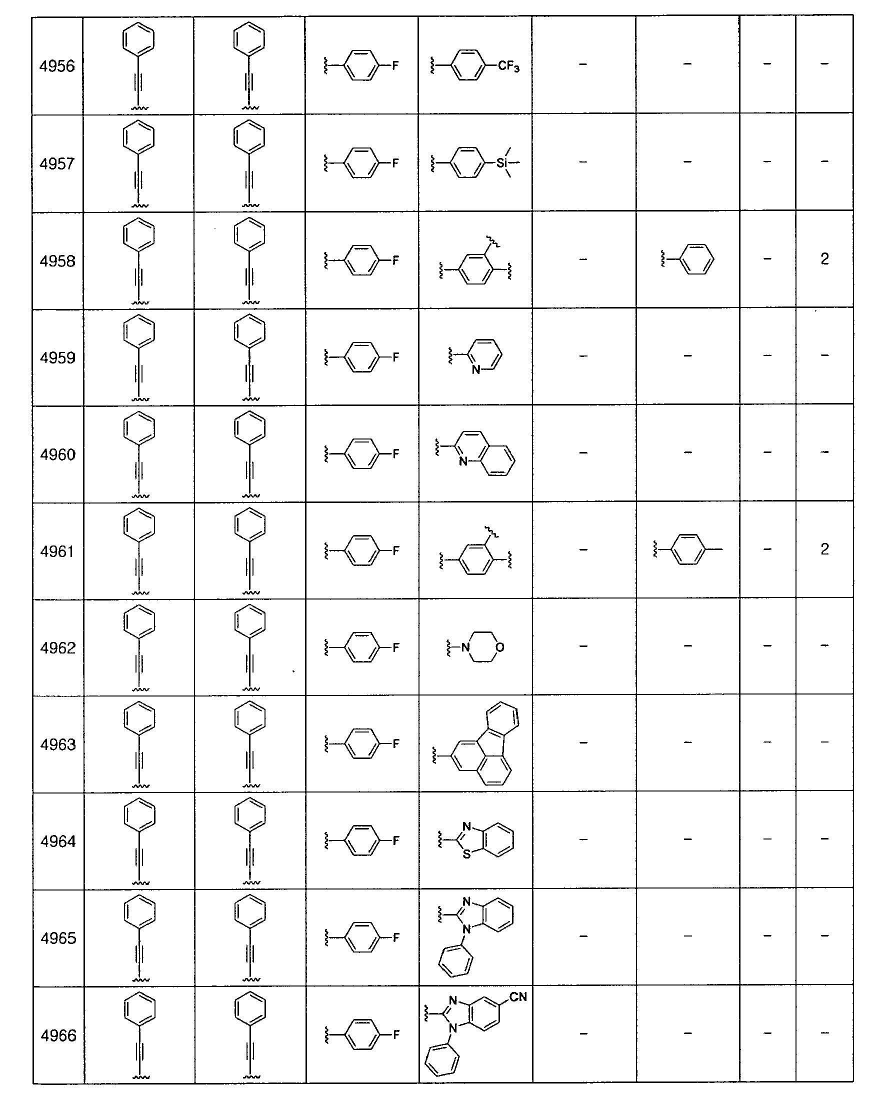

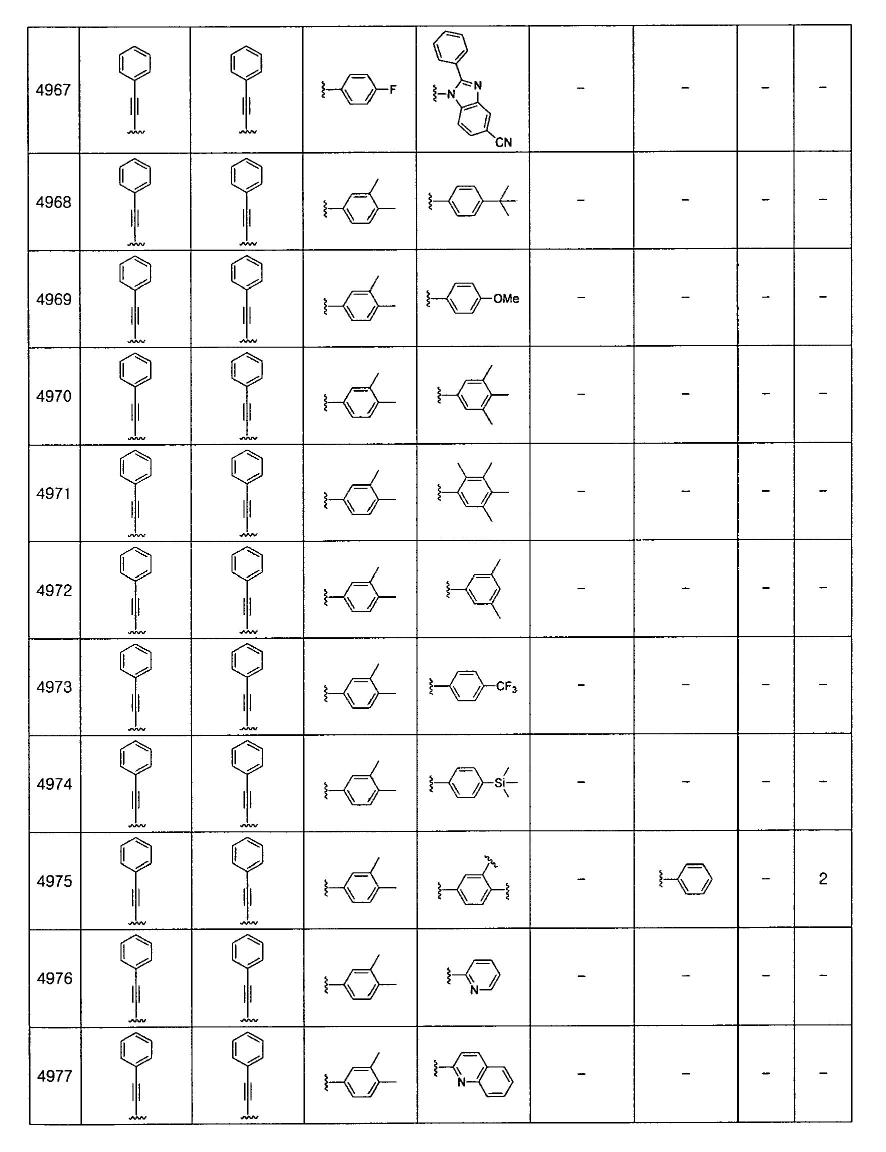

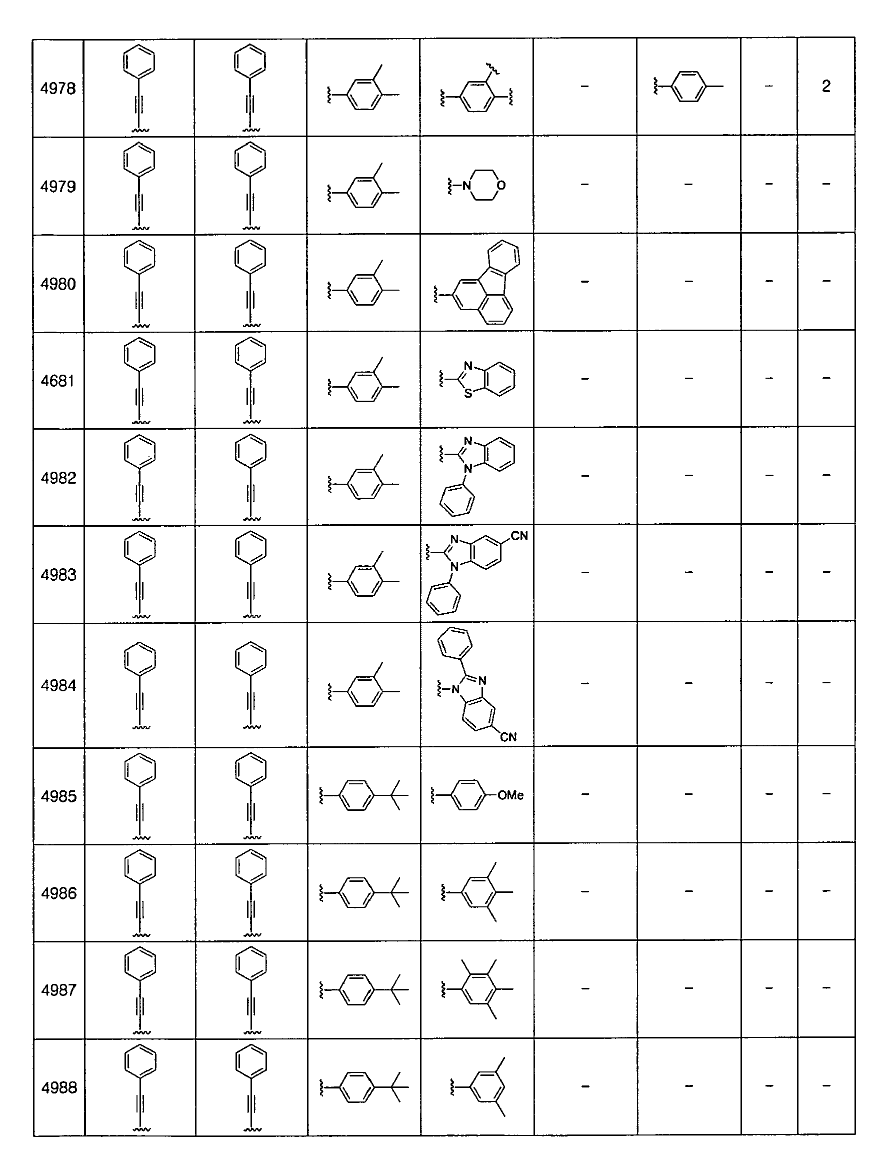

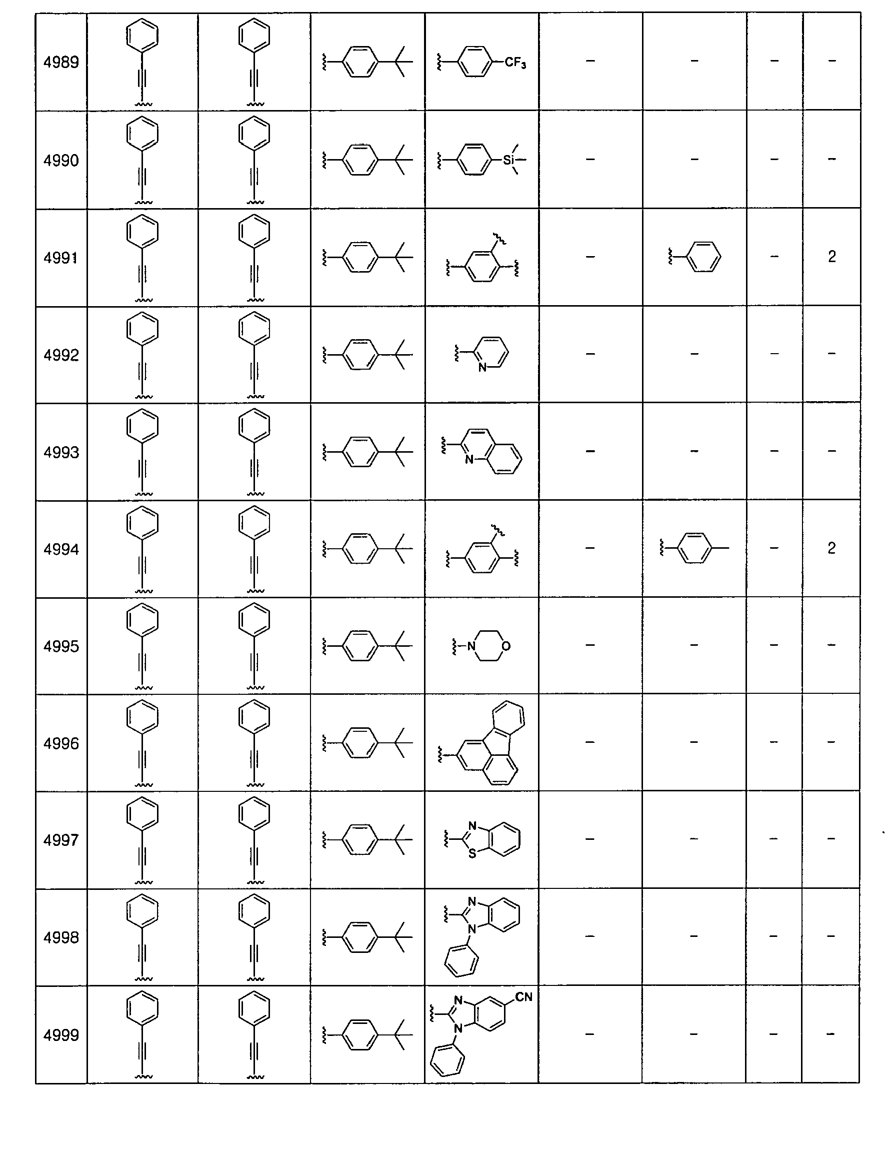

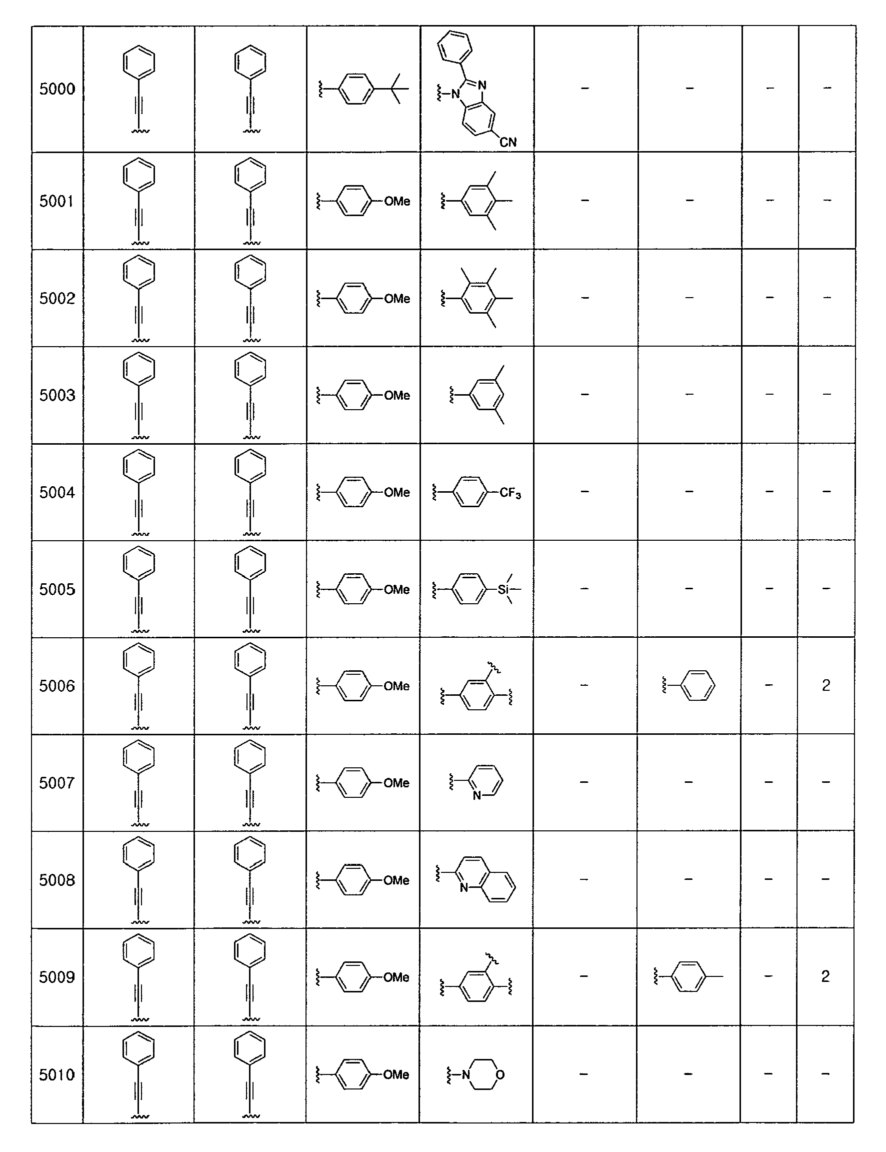

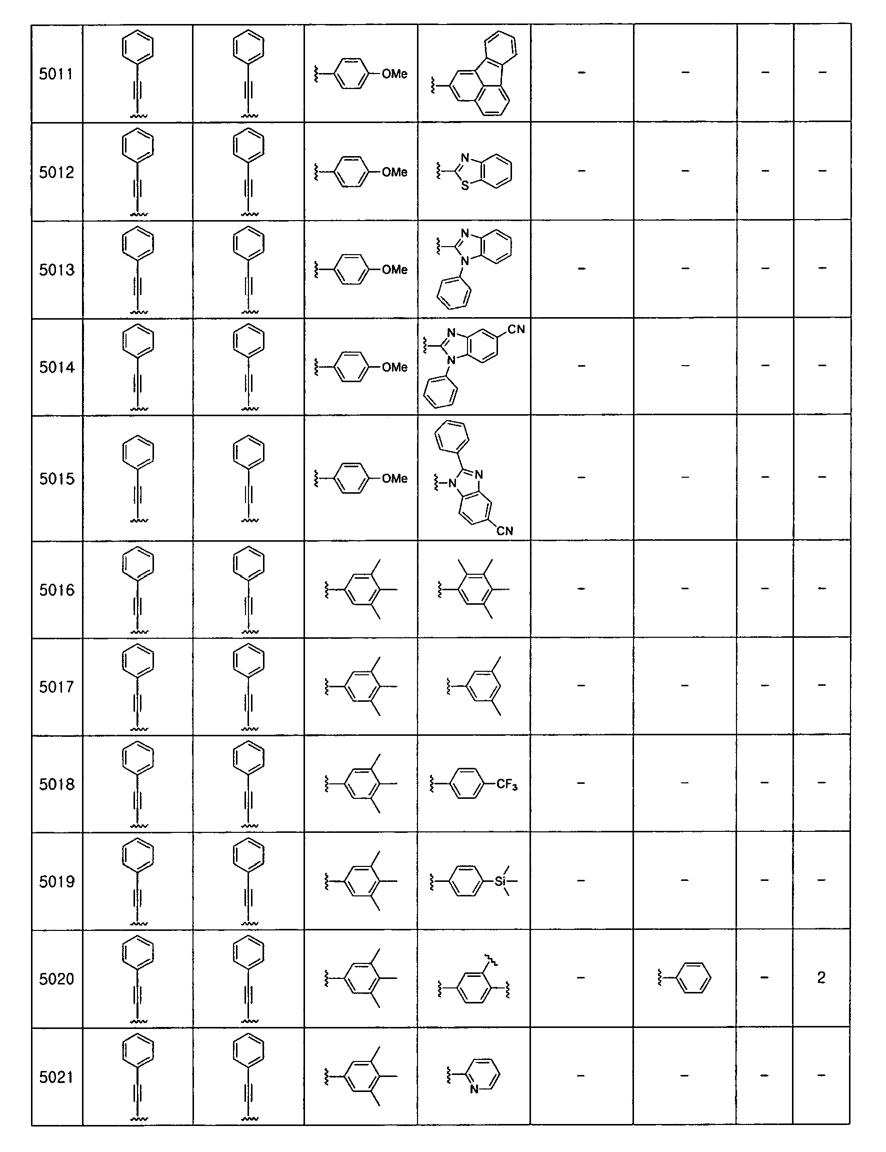

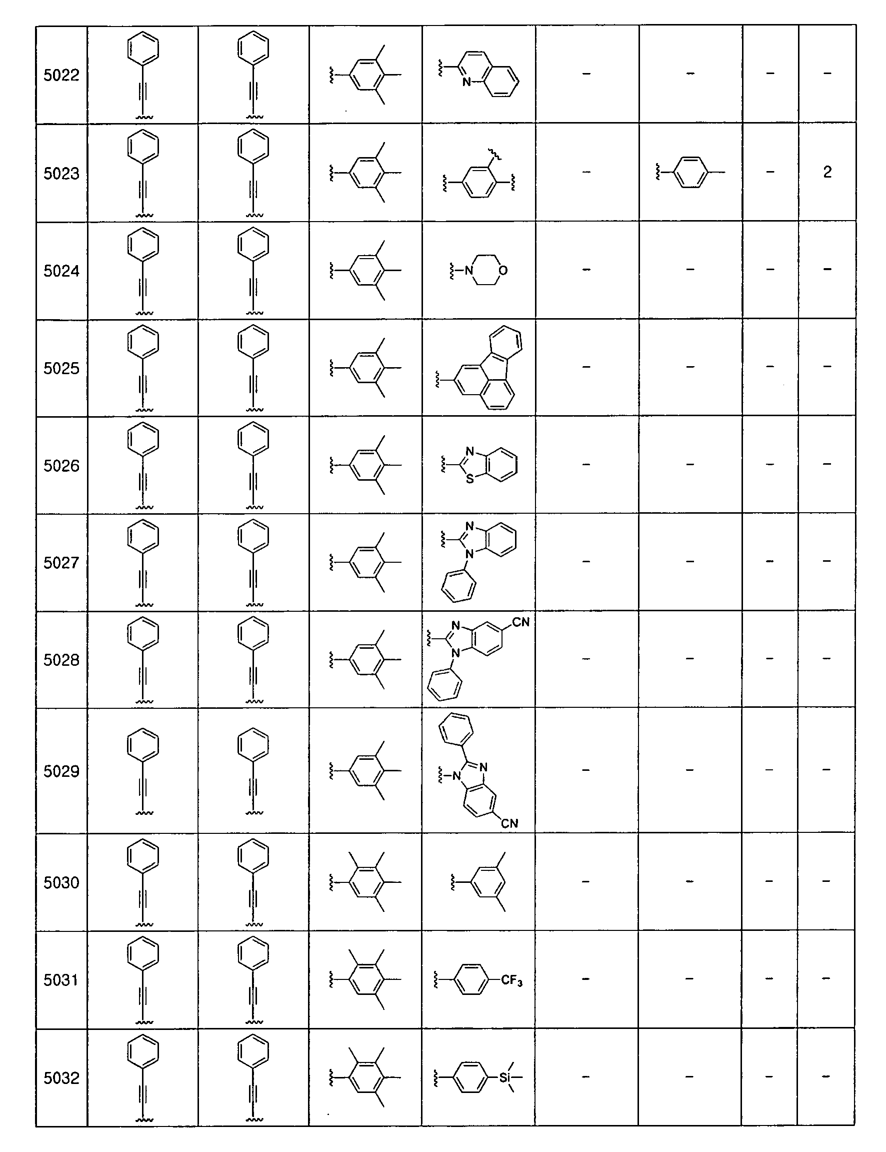

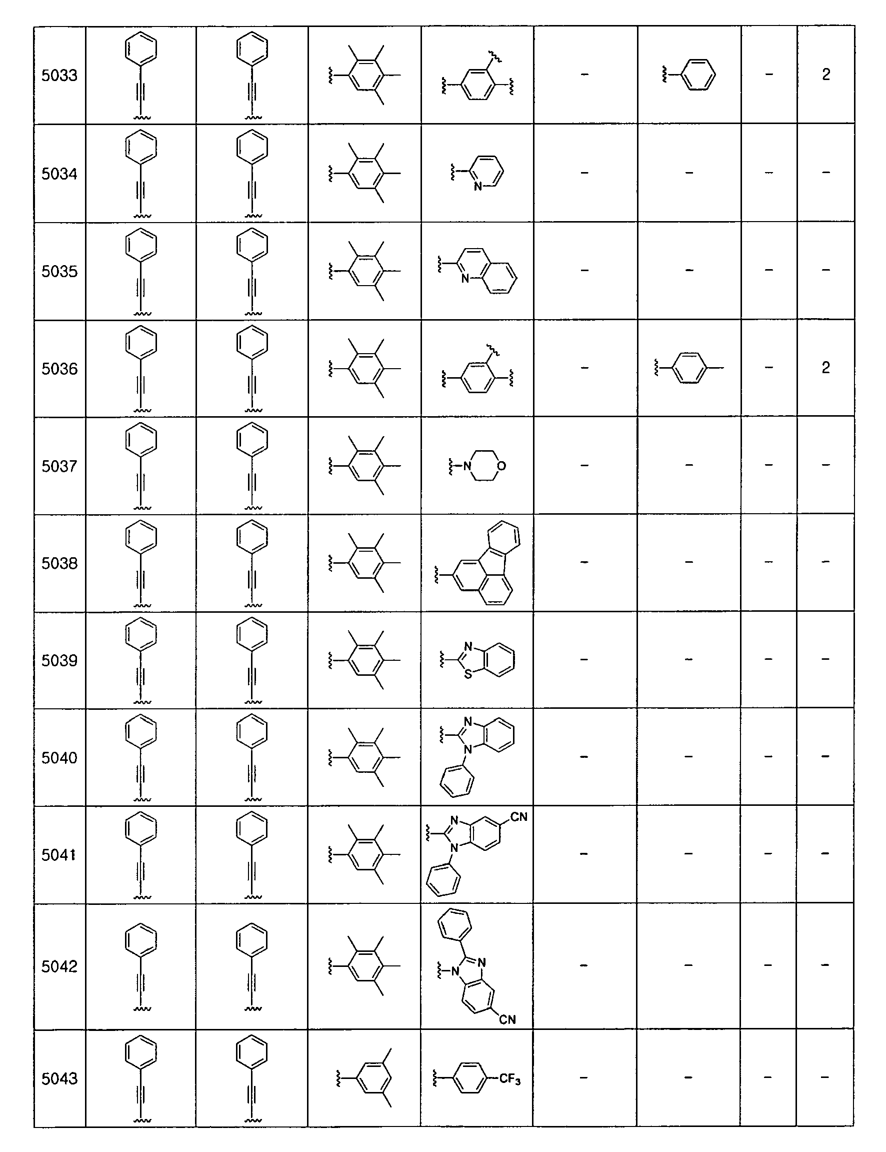

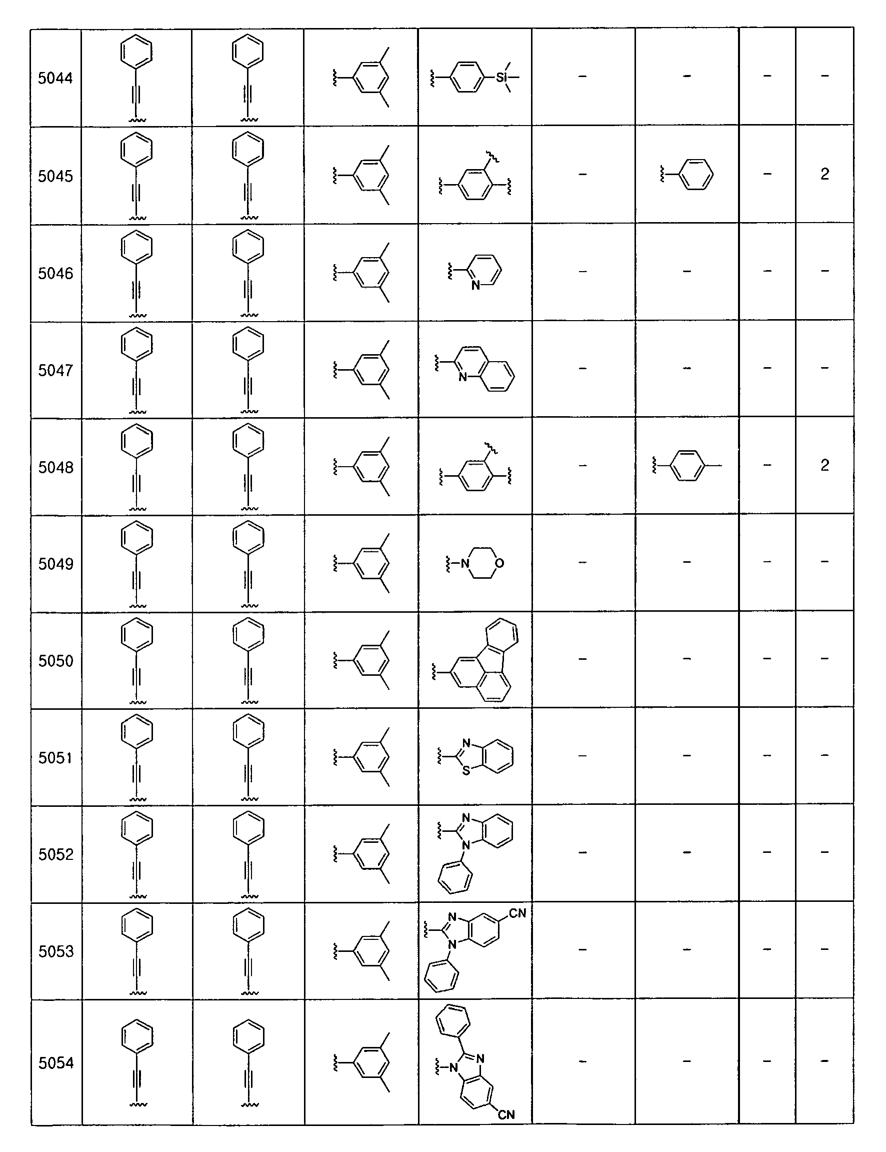

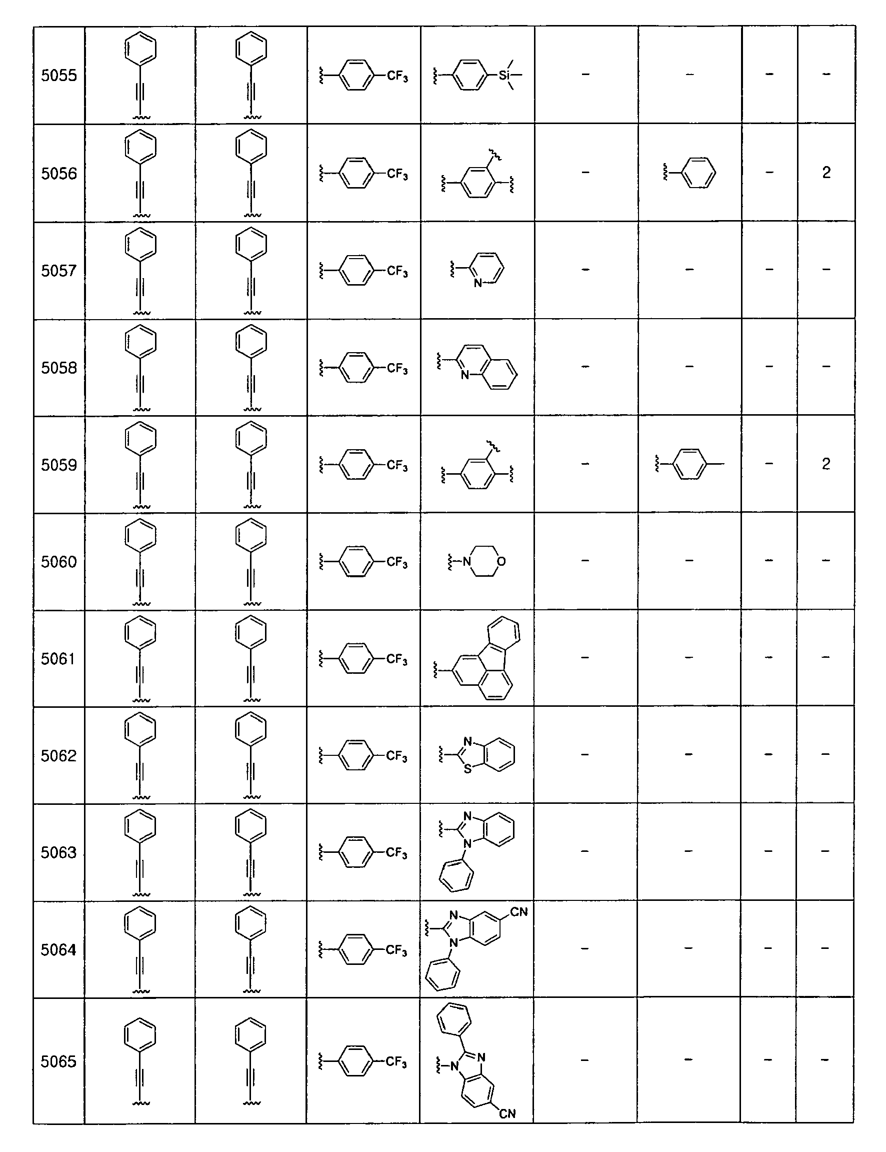

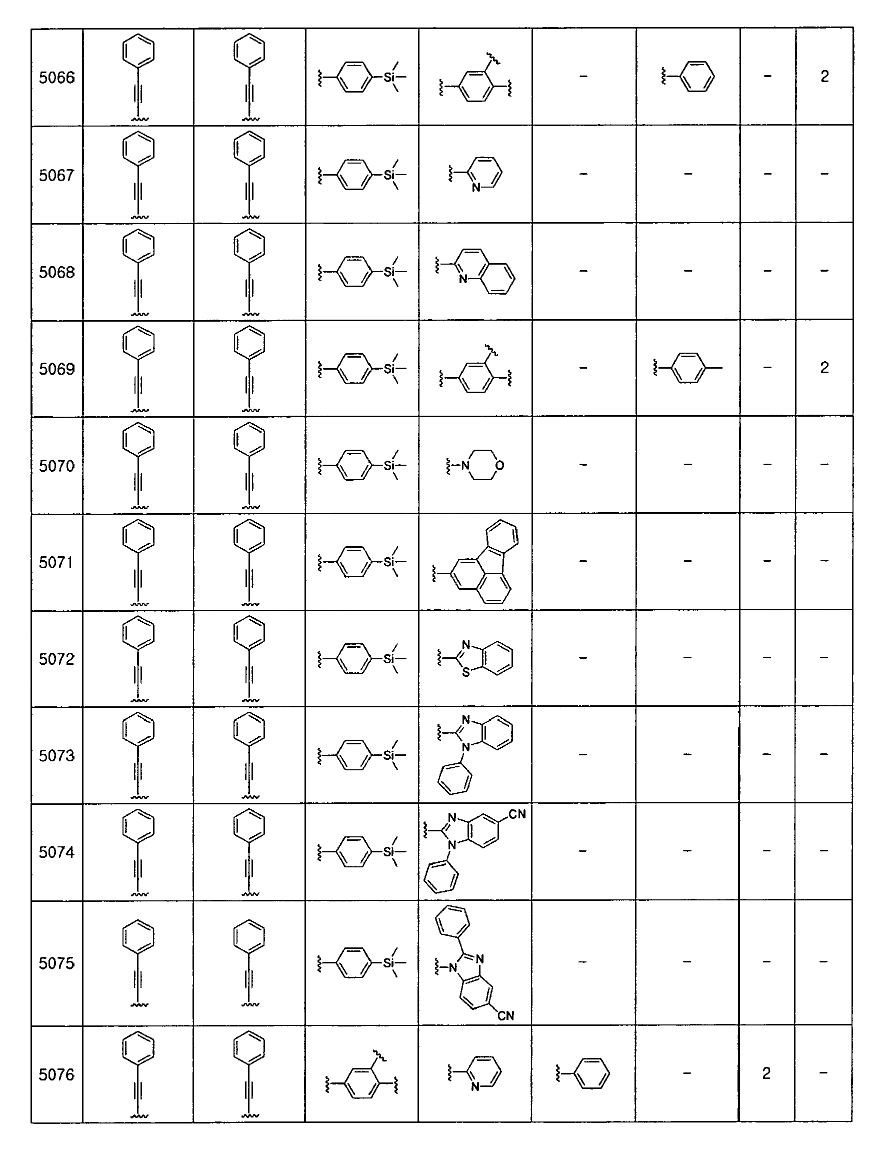

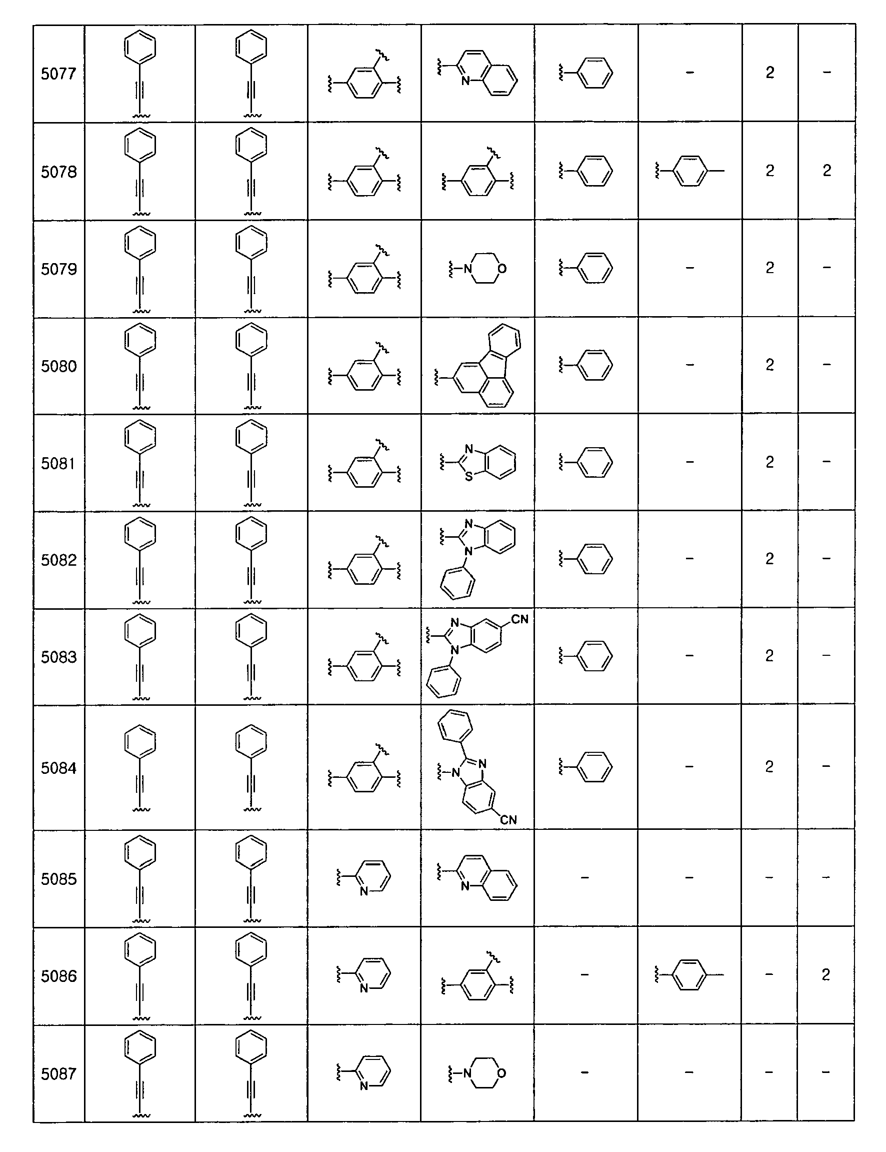

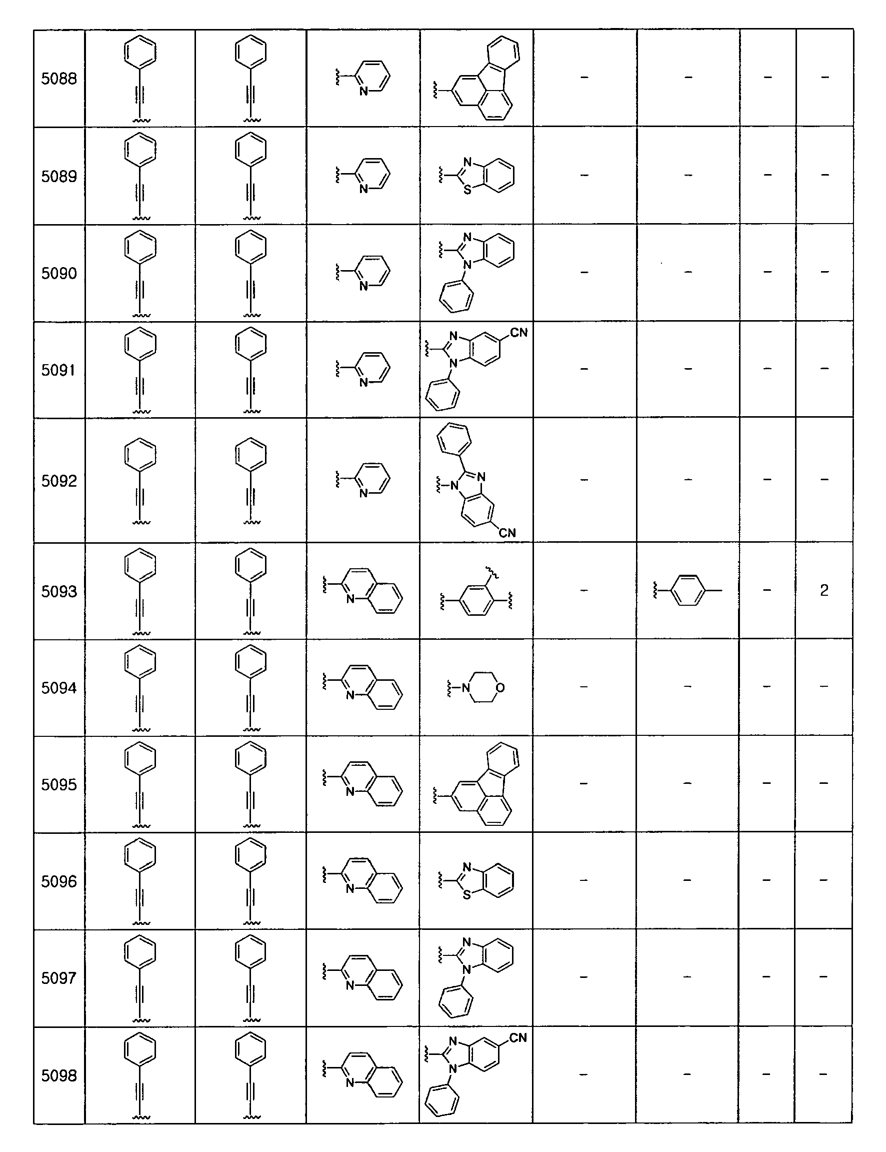

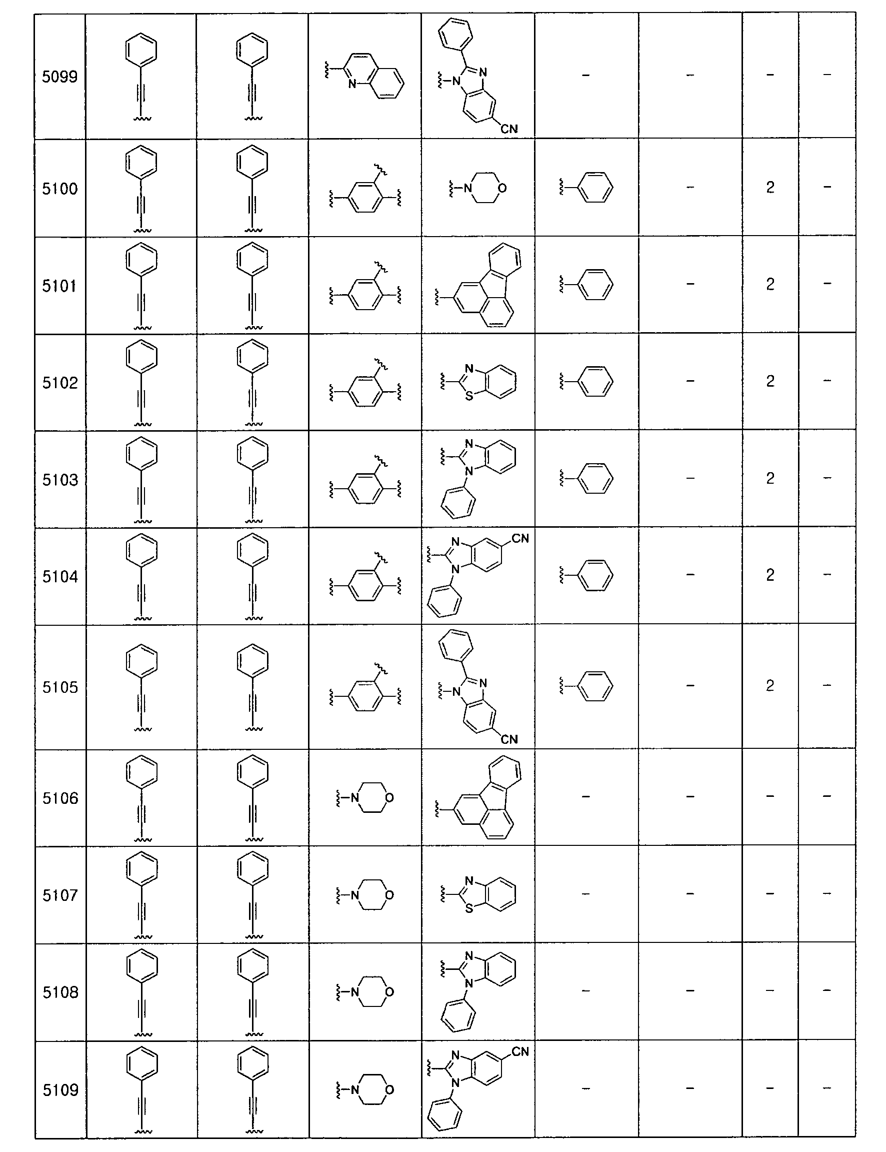

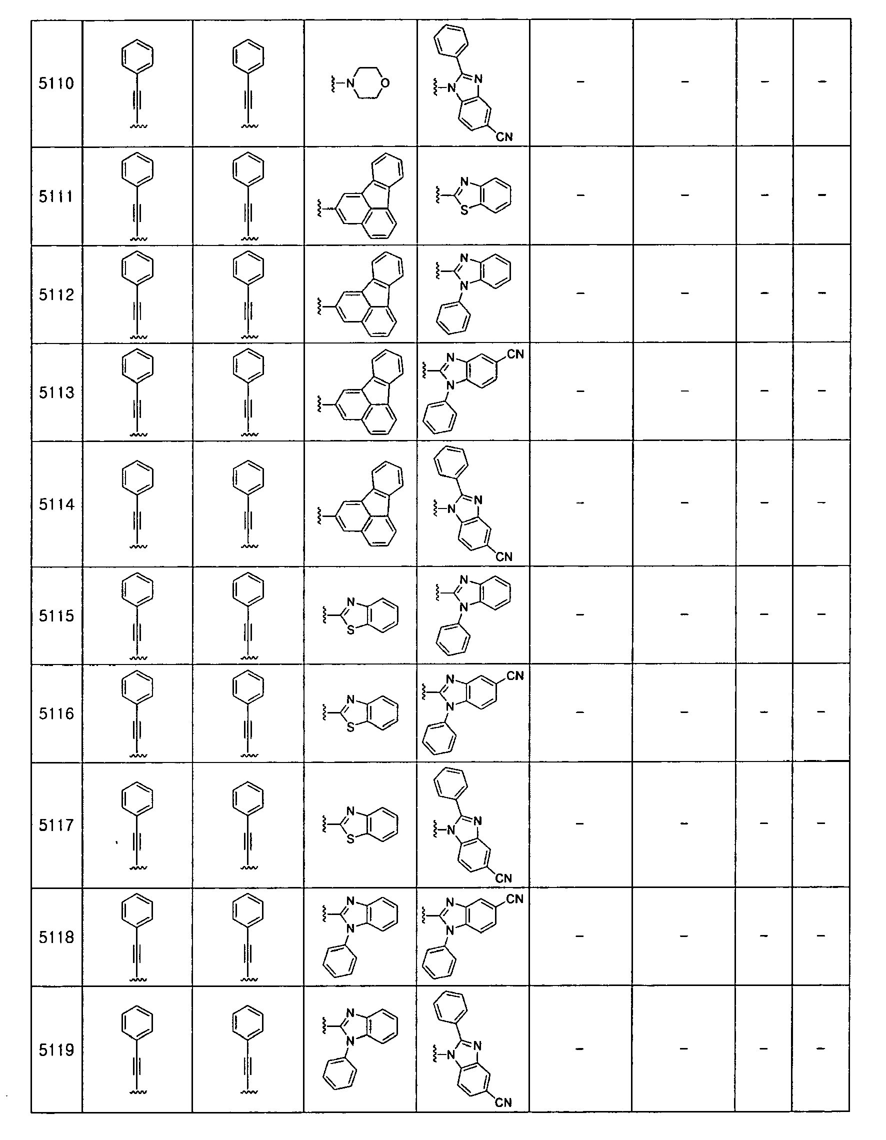

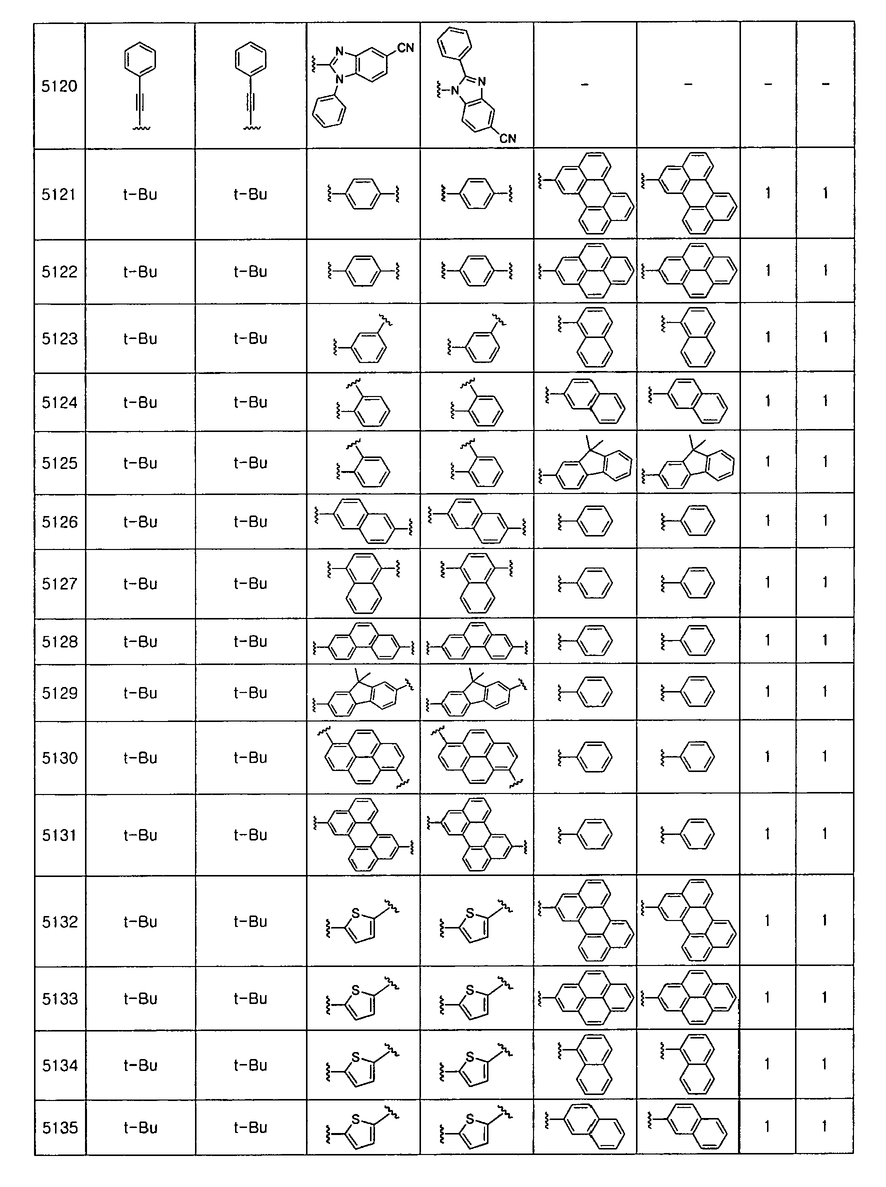

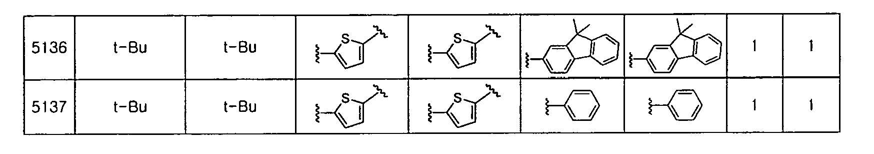

**Table 2**

| Comp. | ¹H NMR(CDCl₃, 200 MHz) | MS/FAB | |
|---|---|---|---|
| | | found | calculated |
| 1 | δ = 7.56(m, 2H), 7.01(m. 8H), 6.77-6.75(m, 4H), 6.62(m, 4H), 6.46(m, 8H), 1.47(s, 18H) | 624 | 624.85 |
| 2 | δ = 7.56-7.51(m, 10H), 7.44(m, 4H), 7.23(m, 4H), 7.09(m, 4H), 6.79-6.75(m, 12H), 1.47(s, 18H) | 824 | 825.09 |
| 3 | δ = 7.66-7.56(m, 10H), 7.31-7.30(m, 8H), 7.16-7.15(m, 8H), 6.77(m, 4H), 6.55(m, 4H), 1.47(s, 18H) | 824 | 825.09 |
| 9 | δ = 8.93-8.68(m, 8H), 8.12-8.10(m, 4H), 7.88-7.71(m 16H), 7.54-7.32(m, 6H), 7.08-6.74(m, 8H), 1.47(s, 18H) | 1025 | 1025.32 |
| 13 | δ = 7.56(m, 2H), 7.48(m, 16H), 7.32-7.22(m, 24H), 7.06(m, 4H), 6.77-6.75(m, 4H), 6.64(m, 8H), 1.48(s, 18H) | 1233 | 1233.62 |
| 28 | δ = 7.55-7.54(m, 2H), 6.82-6.74(m, 4H), 3.68-6.67(m, 16H), 3.0-2.9(m, 16H), 1.47(s, 18H) | 660 | 660.89 |
| 34 | δ = 7.56-7.51 (m, 6H), 7.44(m, 2H), 7.23(m, 2H), 7.09-7.01(m, 6H), 6.79-6.75(m, 8H), 6.62(m, 2H), 6.46(m, 4H), 1.48(s, 18H) | 724 | 724.97 |
| 35 | δ = 7.66-7.56(m, 6H), 7.31-7.30(m, 4H), 7.16-7.15(m, 4H), 7.01(m, 4H), 6.77-6.75(m, 4H), 6.62-6.55(m, 4H), 6.46(m, 4H), 1.45(s, 18H) | 724 | 724.97 |
| 42 | δ = 7.84(m, 2H), 7.59-7.56(m, 6H), 7.38(m, 2H), 7.28(m, 2H), 7.01(m, 4H), 6.77-6.75(m, 6H), 6.62-6.58(m, 4H), 6.46(m, 4H), 1.67(s, 12H), 1.48(s, 18H) | 856 | 857.17 |
| 47 | δ = 7.55-7.54(m, 2H), 7.01-6.82(m, 6H), 6.74-6.62(m, 8H), 6.46-6.44(m, 8H), 1.47(s, 18H) | 660 | 660.84 |
| 50 | δ = 7.54-7.01(m, 6H), 6.82-6.74(m, 4H), 6.62-6.52(m, 6H), 6.46-6.35(m, 8H), 3.73(s, 6H), 1.48(s, 18H) | 684 | 684.91 |
| 85 | δ = 7.55-7.44(m, 8H), 7.23-7.09(m, 8H), 6.82-6.39(m, 12H), 1.47(s, 18H) | 860 | 860.97 |
| 104 | δ = 8.00-7.82(m, 6H), 7.71-7.54(m, 14H), 7.31-7.15(m, 12H), 6.82-6.55(m, 6H), 1.48(s, 18H) | 973 | 973.25 |
| 132 | δ = 7.84-7.54(m, 8H), 7.38-7.28(m, 4H), 6.82-6.81(m, 6H), 6.75-66.34(m, 10H), 2.35(s, 6H), 1.67(s, 12H), 1.47(s. 18H) | 885 | 885.23 |
| 137 | δ = 7.54-7.22(m, 16H), 6.82-6.72(m, 8H), 6.52-6.44(m, 8H), | 813 | 813.03 |
| | 1.47(s, 18H) | | |
| 175 | δ = 8.11(m, 2H), 7.54-7.44(m, 12H), 7.32-7.22(m, 14H), 6.82-6.70(m, 6H), 6.60-6.52(m, 6H), 1.48(s, 18H) | 931 | 931.21 |
| 205 | δ = 7.67-7.54(m. 6H), 7.49-7.48(m, 6H), 7.34-7.32(m, 6H), 7.26-7.22(m, 12H), 6.97-6.82(m, 6H), 6.74-6.52(m, 6H), 1.48(s, 18H) | 1025 | 1025.32 |
| 214 | δ = 7.54-7.48(m, 18H), 7.32-7.28(m, 16H), 7.23-7.22(m, 8H), 7.06-6.82(m, 6H), 6.74-6.52(10H), 1.47(s, 18H) | 1233 | 1233.62 |
| 235 | δ = 8.93-8.68(m, 4H), 8.12-7.82(m, 8H), 7.71-7.51(m, 8H), 7.38-7.28(m, 6H), 7.20-7.08(m, 6H), 6.79-6.48(m, 6H), 1.67(s, 12H), 1.47(s, 18H) | 1057 | 1057.41 |
| 246 | δ = 8.93-8.12(m, 6H), 7.88-7.71 (m, 8H), 7.54-7.32(m, 4H), 7.08-6.74(m, 6H), 6.22-6.07(m, 6H), 2.35(s, 12H), 1.47(s, 18H) | 881 | 881.2 |
| 292 | δ = 8.93(m, 6H), 8.13-7.82(m, 14H), 7.54-7.48(m, 10H), 7.32-7.22(m, 14H), 7.02-6.48(m, 10H), 1.47(s, 18H) | 1277 | 1277.63 |
| 313 | δ = 8.11(m, 2H), 7.54-7.44(m, 12H), 7.32-7.06(m, 14H), 6.82-6.60(m, 12H), 1.47(s, 18H) | 931 | 931.21 |
| 322 | δ = 7.56(m, 2H), 6.81-6.72(m, 12H), 6.44(m, 4H), 6.34(m, 4H), 2.35(s, 6H), 1.45(s, 18H) | 688 | 688.89 |
| 394 | δ = 7.54-6.74(m, 6H), 6.52-6.35(m, 8H), 6.22-6.07(m, 6H), 3.73(s, 6H), 2.35(s, 12H), 1.47(s, 18H) | 741 | 741.01 |
| 434 | δ = 7.54-7.20(m, 6H), 6.82-6.74(m, 4H), 6.39-6.07(m, 10H), 2.35(s, 12H), 1.47(s, 18H) | 816 | 816.96 |
| 484 | δ = 7.84-7.82(m, 4H), 7.56-7.52(m, 6H), 7.36(m, 8H), 7.20-7.12(m, 10H), 7.03(m, 2H), 6.90(m, 2H), 6.77-6.70(m, 6H), 6.48(m, 2H), 2.35(s, 12H), 1.48(s, 18H) | 1087 | 1087.44 |
| 512 | δ = 7.54-7.01(m, 10H), 6.82-6.62(8H), 6.46(m, 8H), 2.65(s, | 540 | 540.7 |
| 553 | δ = 7.84(m, 2H), 7.59-7.55(m, 6H), 7.38(m, 2H), 7.28(m, 2H), 7.01(m, 4H), 6.77-6.75(m, 6H), 6.62-6.58(m, 4H), 6.46(m, 4H), 2.65(s, 6H), 1.67(s, 12H) | 772 | 773.02 |
| 567 | δ = 7.54-7.48(m, 10H), 7.32-7.22(m, 14H), 7.01-6.82(m, 6H), 6.74-6.46(m, 12H), 2.65(s, 6H) | 845 | 845.08 |
| 629 | δ = 8.11(m, 2H), 7.61-7.56(m, 6H), 7.44(m, 2H), 7.31-7.30(m, 4H), 7.16-7.15(m, 4H), 6.77-6.70(m, 6H), 6.60-6.55(m, 4H), 2.65(s, 6H) | 642 | 642.79 |
| 685 | δ = 7.54-7.48(m, 18H), 7.32-7.28(m, 18H), 7.23-6.82(m, 12H), 6.74-6.48(m, 10H), 2.65(s, 6H) | 1149 | 1149.46 |
| 797 | δ = 8.93(m, 6H), 8.13-7.82(m, 14H), 7.54-6.74(m, 8H), 6.52-6.35(m, 8H), 3.73(s, 6H), 2.65(s, 6H) | 949 | 949.14 |
| 855 | δ = 7.54-6.72(m, 10H), 6.44-5.95(m, 8H), 2.65(s, 6H), 2.35(s, 18H) | 660 | 660.84 |
| 921 | δ = 7.54-7.23(m, 6H), 6.82-6.44m, (8H), 5.95(m, 4H), 2.65(s, 6H), 2.35(s, 18H), 0.66(s, 18H) | 769 | 769.22 |
| 1023 | δ = 7.56(m, 2H), 7.01(m, 8H), 6.77-6.75(m, 4H), 6.62(m, 4H), 6.46(m, 8H), 2.72(m, 2H), 1.88(m, 4H), 1.63(m, 4H), 1.49-1.39(m, 12H) | 676 | 676.93 |
| 1032 | δ = 7.84(m, 4H), 7.59-7.55(m, 8H), 7.38(m, 4H), 7.28(m, 4H), 7.20-7.13(m, 4H), 6.77-6.75(m, 6H), 6.58(m, 2H), 6.48(m, 2H), 2.72(m, 2H), 1.88(m, 4H), 1.67(s, 24H), 1.63(m, 4H), 1.49-1.39(m, 12H) | 1141 | 1141.57 |
| 1468 | δ = 7.54-7.20(m, 10H), 6.82-6.74(m, 4H), 6.44-6.39(m, 8H), 2.72(m, 2H), 1.88-1.39(m, 20H), 0.66(s, 18H) | 957 | 957.29 |
| 1555 | δ = 7.51(m, 2H), 7.20(m, 8H), 6.79(m, 4H), 6.39(m, 8H), 0.66(s, 18H) | 929 | 929 |
| 1666 | δ = 8.00(m, 4H), 7.82-7.71(m, 10H), 7.51-7.48(m, 6H), 7.32-7.20(m, 14H), 6.79-6.52(m, 8H), 0.66(s, 18H) | 1057 | 1057.47 |
| 1820 | δ = 8.93(m, 6H), 8.13(m, 2H), 7.88-7.51(m, 14H), 7.02-6.76(m, 6H), 5.95(m, 4H), 2.35(s, 18H), 0.66(s, 18H) | 1089 | 1089.56 |
| 2088 | δ = 8.93(m, 6H), 8.13(m, 2H), 7.88-7.82(m, 12H), 7.54-7.51(m, 14H), 7.37-7.35(m, 18H), 7.02-7.01 (m, 6H), 6.79-6.62(m, 6H), 6.47-6.46(m, 4H) | 1377 | 1377.82 |
| 2194 | δ = 7.75-7.71(m, 6H), 7.55-7.37(m, 40H), 7.03(m, 2H), 6.89-6.83(m, 6H), 3.65(m, 8H), 3.11(m, 8H) | 1198 | 1199.63 |
| 2572 | δ = 7.78(m, 2H), 7.06-7.01(m, 10H), 6.82(m, 2H), 6.55(m, 8H), 2.02-2.01(m, 18H), 1.71(m, 12H), 1.35(s, 36H) | 1005 | 1005.5 |
| 3067 | δ = 8.23(m, 2H), 8.12(m, 2H), 7.55-7.51 (m, 6H), 7.01 (m, 8H), 6.79-6.76(m, 4H), 6.62(m, 4H), 6.46(m, 8H) | 778 | 778.98 |
| 3163 | δ = 8.23-8.12(m, 4H), 7.66-7.51(m, 10H), 7.48-7.30(m, 12H), 7.23-7.15(m, 10H), 6.79-6.52(m, 10H) | 1031 | 1031.29 |
| 3578 | δ = 7.56(m, 2H), 7.01(m, 8H), 6.77-6.75(m, 4H), 6.62(m, 4H), 6.46(m, 8H), 1.84(m, 6H), 1.59(m, 6H), 1.49(m, 6H), 1.33-1.21(m, 22H), 0.96(m, 6H) | 868 | 869.27 |
| 4089 | δ = 7.51(m, 2H), 7.42(m, 4H), 7.26(m, 4H), 7.11(m, 2H), 7.01-6.99(m, 12H), 6.79-6.76(m, 4H), 6.62(m, 4H), 6.46(m, 8H) | 716 | 716.91 |
| 4272 | δ = 7.54-7.48(m, 18H), 7.42-7.26(m, 26H), 7.23-7.11(m, 12H), 6.99-6.48(m, 16H) | 1325 | 1325.68 |
| 4610 | δ = 7.51-7.46(m, 6H), 7.22-7.19(m, 6H), 7.01(m, 8H), 6.79-6.76(m, 4H), 6.62(m, 4H), 6.46(m, 8H) | 712 | 712.88 |
| 4661 | δ = 7.51-7.46(m, 6H), 7.22-7.01 (m, 10H), 6.79-6.62(m, 6H), 6.46-5.83(m, 6H), 2.35(s, 24H) | 825 | 825.09 |
| 5127 | δ = 7.66-7.61(m, 8H), 7.54-7.48(m, 10H), 7.37-7.30(m, 20H), 7.22-6.61 (m, 12H), 1.47(s, 18H) | 1129 | 1129.47 |
| 5135 | δ = 8.34(m, 4H), 8.00-7.87(m, 16H), 7.78(m, 2H), 7.59(m, 8H), 7.28(m, 4H), 7.06(m, 2H), 6.81(m, 2H), 6.30(m, 4H), 1.48(s, 18H) | 1153 | 1153.58 |

### [Example 1] Manufacture of an OLED by using an organic EL compound according to the invention

An OLED device was manufactured by using EL material according to the invention.

First, a transparent electrode ITO thin film (15 Ω/□) (2) prepared from glass for OLED (produced by Samsung Corning) (1) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injection layer (3) having 60 nm of thickness on the ITO substrate.

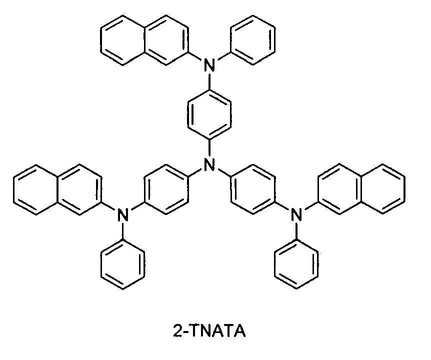

Then, to another cell of the vacuum vapor-deposit device, charged was N,N'-bis(α-naphthyl)-N,N'-diphenyl-4,4'-diamine (NPB), and electric current was applied to the cell to evaporate NPB, thereby providing vapor-deposit of a hole transport layer (4) of 20 nm of thickness on the hole injection layer.

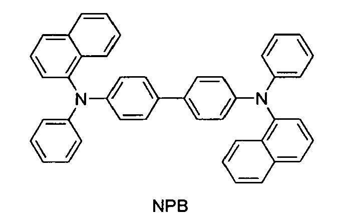

After forming the hole injection layer and hole transport layer, an EL layer was vapor-deposited thereon as follows. To one cell of said vacuum vapor-deposit device, charged was the host (H-6) (having the chemical structure shown below), and Compound (1031) according to the present invention was charged as a dopant to another cell. The two materials were evaporated at different rates to carry out doping at a concentration of 2 to 5 mol% on the basis of the host, to vapor-deposit an electroluminescent layer (5) having 30 nm of thickness on the hole transport layer.

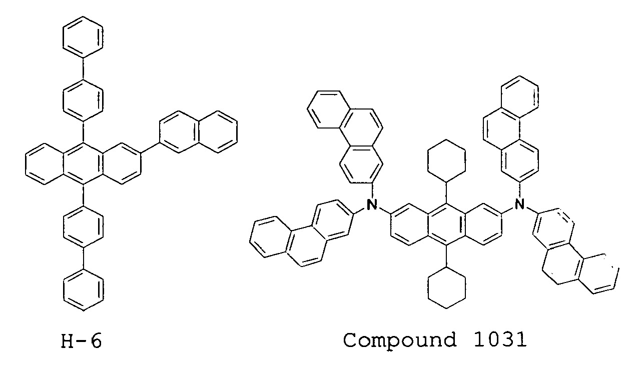

Then, tris(8-hydroxyquinoline)aluminum (III) (Alq) was vapor-deposited as an electron transport layer (6) with a thickness of 20 nm, and lithium quinolate (Liq) (of which the structure is shown below) was vapor-deposited as an electron injection layer (7) with a thickness of 1 to 2 nm. Thereafter, an Al cathode (8) was vapor-deposited with a thickness of 150 nm by using another vacuum vapor-deposit device to manufacture an OLED.

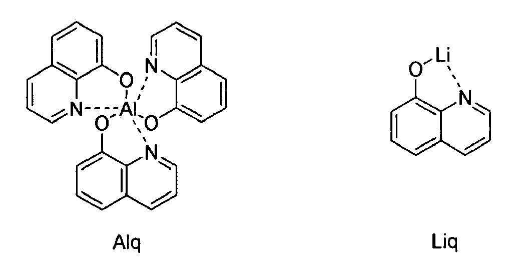

Each compound was used as an EL material for an OLED, after purifying via vacuum sublimation under 10⁻⁶ torr.

### [Comparative Example 1] Manufacture of an OLED by using a conventional EL material

After formation of a hole injection layer and a hole transport layer as described in Example 1, another cell of the vacuum deposition device was charged with tris(8-hydroxyquinoline)aluminum (III) (Alq) as an electroluminescent host material, and still another cell was charged with Coumarin 545T (C545T) having the structure shown below. Two substances were doped by evaporation at different rates to vapor-deposit an electroluminescent layer with a thickness of 30 nm on the hole transport layer. Preferable doping concentration is from 1 to 3 mol% on the basis of Alq.

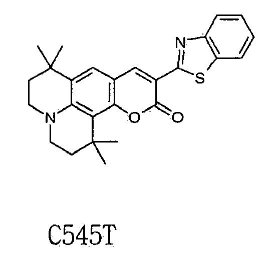

Then, an electron transport layer and an electron injection layer were vapor-deposited according to the same procedure as described in Example 1, and an Al cathode was vapor-deposited by using another vacuum vapor-deposit device with a thickness of 150 nm, to manufacture an OLED.

### [Comparative Example 2] Manufacture of an OLED by using a conventional EL material

After formation of a hole injection layer and a hole transport layer as described in Example 1, another cell of the vacuum deposition device was charged with H-6 as an electroluminescent host material, and still another cell was charged with Compound G. Two substances were doped by evaporating at different rates (with a doping concentration of 2 to 5% on the basis of the host) to vapor-deposit an electroluminescent layer with a thickness of 30 nm on the hole transport layer.

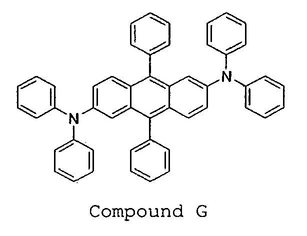

Then, an electron transport layer and an electron injection layer were vapor-deposited according to the same procedure as described in Example 1, and an Al cathode was vapor-deposited by using another vacuum vapor-deposit device with a thickness of 150 nm, to manufacture an OLED.

### [Example 2] EL properties of OLED's manufactured

The luminous efficiencies of OLED's manufactured from Example 1 and Comparative Examples 1-2, comprising an organic EL compound according to the invention and conventional electroluminescent compounds, respectively, were measured at 5,000 cd/m² and 20,000 cd/m², individually, of which the results are shown in Table 3. Since the electroluminescent properties in high luminance region are very important, particularly in case of green electroluminescent materials, the data at high luminance (about 20,000 cd/m²) are attached.

**Table 3**

| No. | | Host | Dopant | Doping Conc. (mol%) | Efficiency (cd/A) | | Color |
|---|---|---|---|---|---|---|---|
| | | | | | @5,000 cd/m² | @20,000 cd/m² | |
| Ex. 1 | 1 | H-6 | Compound 2 | 3.0 | 18.1 | 17.5 | 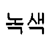 |
| | 2 | H-6 | Compound 124 | 3.0 | 18.7 | 17.8 | 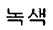 |
| | 3 | H-6 | Compound 1031 | 3.0 | 20.2 | 19.8 | 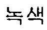 |
| | 4 | H-10 | Compound 586 | 3.0 | 18.3 | 17.7 | 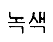 |
| | 5 | H-10 | Compound 1136 | 3.0 | 18.1 | 18.0 | 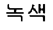 |
| | 6 | H-16 | Compound 1691 | 3.0 | 16.5 | 15.1 | 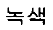 |
| | 7 | H-16 | Compound 1820 | 3.0 | 16.2 | 15.9 | 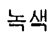 |
| | 8 | H-24 | Compound 1944 | 3.0 | 19.8 | 19.0 | 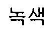 |
| | 9 | H-24 | Compound 2088 | 3.0 | 17.3 | 16.7 | 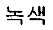 |
| | 10 | H-34 | Compound 3392 | 3.0 | 19.7 | 19.5 | 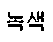 |
| | 11 | H-34 | Compound 3682 | 5.0 | 19.8 | 19.6 | 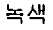 |
| | 12 | H-45 | Compound 4320 | 5.0 | 19.2 | 19.7 | 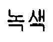 |
| Comp. Ex. 1 | | Alq | Compound C545T | 1.0 | 10.3 | 9.1 | 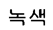 |
| Comp. Ex. 2 | | H-6 | Compound G | 3.0 | 11.3 | 9.0 | 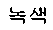 |

As can be seen from Table 3, when the organic EL compound according to the present invention was employed in a green electroluminescent device, the device exhibited significantly improved luminous efficiency, while maintaining at least comparable color purity as compared to Comparative Example 1.

Particularly, the fact that decrease in the efficiency of the electroluminescent materials of high performance according to the present invention is not more than 1 cd/A, even at a high luminance (20,000 cd/m²), implies excellent property as an EL material maintained even at a high luminance, not only at a low luminance. This demonstrates that the compounds can be advantageously employed in both passive and active organic OLED's.

This feature is contrary to that of conventional EL material having excellent electron conductivity, and the result shows the most noticeable advantage of the EL material according to the present invention.

## Claims

1. An organic electroluminescent compound represented by Chemical Formula (1): 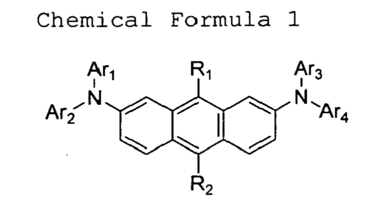 wherein, R₁ and R₂ independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R₁ and R₂ may be further substituted by one or more substituent(s) selected from deuterium, linear or branched (C1-C20)alkyl, (C1-C20)alkenyl, (C1-C20)alkynyl, halogen, phenyl, fluorenyl, naphthyl and anthryl; and
Ar₁ through Ar₄ independently represent (C6-C20)aryl, (C4-C20)heteroaryl, morpholino or thiomorpholino, and the aryl or heteroaryl of Ar₁ through Ar₄ may be further substituted by one or more substituent(s) selected from a group consisting of (C6-C20)aryl or (C4-C20)heteroaryl containing one or more substituent(s) selected from a group consisting of deuterium, linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl; linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl.

2. The organic electroluminescent compound according to claim 1, which is represented by Chemical Formula (2): 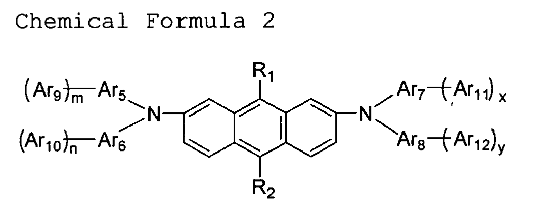 wherein, R₁ and R₂ are defined as in Claim 1;
Ar₅ through Ar₈ independently represent (C6-C20)aryl, (C4-C20)heteroaryl, morpholino, thiomorpholino, (C6-C20)arylene or (C4-C20)heteroarylene, and the aryl or heteroaryl of Ar₅ through Ar₈ may be further substituted by one or more substituent(s) selected from deuterium, linear or branched (C1-C20)alkyl with or without halogen substituent(s), (C1-C20)alkoxy, (C3-C15)cycloalkyl, halogen, cyano, (C6-C20)aryl, tri(C1-C20)alkylsilyl, di(C1-C20)alkyl(C6-C20)arylsilyl and tri(C6-C20)arylsilyl;
Ar₉ through Ar₁₂ independently represent (C6-C20)aryl or (C4-C20)heteroaryl, and the aryl or heteroaryl of Ar₉ through Ar₁₂ may be further substituted by one or more substituent(s) selected from deuterium, linear or branched (C1-C20)alkyl, (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl;
provided that m is 0 when Ar₅ represents (C6-C20)aryl, (C4-C20)heteroaryl, morpholino or thiomorpholino, while m is an integer from 1 to 4 when Ar₅ represents (C6-C20)arylene or (C4-C20)heteroarylene;
x is 0 when Ar₁₁ represents (C6-C20)aryl, (C4-C20)heteroaryl, morpholino or thiomorpholino, while x is an integer from 1 to 4 when Ar₁₁ represents (C6-C20)arylene or (C4-C20)heteroarylene;
n is 0 when Ar₆ represents (C6-C20)aryl, (C4-C20)heteroaryl, morpholino or thiomorpholino, while n is an integer from 1 to 4 when Ar₆ represents (C6-C20)arylene or (C4-C20)heteroarylene; and
y is 0 when Ar₁₂ represents (C6-C20)aryl, (C4-C20)heteroaryl, morpholino or thiomorpholino, while y is an integer from 1 to 4 when Ar₁₂ represents (C6-C20)arylene or (C4-C20)heteroarylene.

3. An organic electroluminescent device according to claim 4, which is comprised of a first electrode; a second electrode; and at least one organic layer(s) interposed between the first electrode and the second electrode; wherein the organic layer comprises an organic electroluminescent compound represented by Chemical Formula (1): 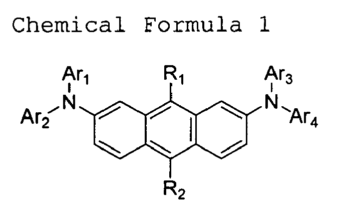 wherein, R1 and R2 independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R1 and R2 may be further substituted by one or more substituent(s) selected from deuterium, linear or branched (C1-C20)alkyl, (C1-C20)alkenyl, (C1-C20)alkynyl, halogen, phenyl, fluorenyl, naphthyl and anthryl; and Ar1 through Ar4 independently represent (C6-C20)aryl, (C4-C20)heteroaryl, morpholino or thiomorpholino, and the aryl or heteroaryl of Ar1 through Ar4 may be further substituted by one or more substituent(s) selected from a group consisting of (C6-C20)aryl or (C4-C20)heteroaryl containing one or more substituent(s) selected from a group consisting of deuterium, linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl; linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl, and one or more host(s) selected from the compounds represented by Chemical Formula (3) or (4):
Chemical Formula 3 (Ar₂₁)ₑ-L₁-(Ar₂₂)_{f}
Chemical Formula 4 (Ar₂₃)_{g}-L₂-(Ar₂₄)ₕ
wherein, L₁ represents (C6-C60)arylene or (C4-C60)heteroarylene;
L₂ represents anthracenylene;
Ar₂₁ through Ar₂₄ are independently selected from hydrogen, (C1-C60)alkyl, (C1-C60)alkoxy, halogen, (C4-C60)heteroaryl, (C5-C60)cycloalkyl and (C6-C60)aryl; and the cycloaryl, aryl or heteroaryl of Ar₂₁ through Ar₂₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; and
e, f, g and h independently represent an integer from 0 to 4.

4. The organic electroluminescent device according to claim 3, wherein the organic layer comprises one or more compound(s) selected from a group consisting of arylamine compounds and styrylarylamine compounds.

5. The organic electroluminescent device according to claim 3, wherein the organic layer comprises one or more metal(s) selected from a group consisting of organic metals of Group 1, Group 2, 4^{th} period and 5^{th} period transition metals, lanthanide metals and d-transition elements.

6. The organic electroluminescent device according to claim 3, which is an organic display comprising an organic electroluminescent compound having the electroluminescent peak with wavelength of not more than 500 nm, and an organic electroluminescent compound having the electroluminescent peak with wavelength of not less than 560 nm, at the same time.

7. The organic electroluminescent device according to claim 3, wherein the organic layer comprises an electroluminescent layer and a charge generating layer.

8. The organic electroluminescent device according to claim 3, wherein a mixed region of reductive dopant and organic substance, or a mixed region of oxidative dopant and organic substance is placed on the inner surface of one or both electrode(s) among the pair of electrodes.

9. An organic solar cell which comprises an organic electroluminescent compound represented by Chemical Formula (1): 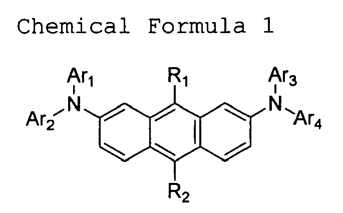 wherein, R₁ and R₂ independently represent hydrogen, deuterium, linear or branched (C1-C20)alkyl, (C2-C20)alkenyl, (C2-C20)alkynyl, (C3-C15)cycloalkyl, tri(C1-C20)alkylsilyl, di(C1-C20)alkyl(C6-C20)arylsilyl, tri(C6-C20)arylsilyl, adamantyl, (C7-C15)bicycloalkyl or (C4-C20)heteroaryl, and the alkyl, alkenyl, alkynyl, cycloalkyl, trialkylsilyl, dialkylarylsilyl, triarylsilyl, adamantyl, bicycloalkyl or heteroaryl of R₁ and R₂ may be further substituted by one or more substituent(s) selected from deuterium, linear or branched (C1-C20)alkyl, (C1-C20)alkenyl, (C1-C20)alkynyl, halogen, phenyl, fluorenyl, naphthyl and anthryl; and Ar₁ through Ar₄ independently represent (C6-C20)aryl, (C4-C20)heteroaryl, morpholino or thiomorpholino, and the aryl or heteroaryl of Ar₁ through Ar₄ may be further substituted by one or more substituent(s) selected from a group consisting of (C6-C20)aryl or (C4-C20)heteroaryl containing one or more substituent(s) selected from a group consisting of deuterium, linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl; linear or branched (C1-C20)alkyl with or without halogen substituent(s), (C1-C20)alkoxy, (C3-C15)cycloalkyl, halogen, cyano, tri(C1-C20)alkylsilyl, di(C1-C20)alkyl(C6-C20)arylsilyl and tri(C6-C20)arylsilyl.
